(19) Europäisches Patentamt
European Patent Office
Office européen des brevets

(11) EP 2 044 016 B1

(12) # EUROPÄISCHE PATENTSCHRIFT

(45) Veröffentlichungstag und Bekanntmachung des Hinweises auf die Patenterteilung:
**05.12.2012 Patentblatt 2012/49**

(21) Anmeldenummer: **07801431.3**

(22) Anmeldetag: **17.07.2007**

(51) Int Cl.:
***C07D 209/14*** (2006.01) ***C07D 209/20*** (2006.01)
***C07D 409/08*** (2006.01) ***C07D 409/12*** (2006.01)
***C07D 471/04*** (2006.01) ***C07D 403/06*** (2006.01)
***C07D 401/06*** (2006.01) ***C07D 307/81*** (2006.01)
***C07D 401/12*** (2006.01) ***C07D 333/58*** (2006.01)
***C07D 401/04*** (2006.01) ***A61K 31/404*** (2006.01)
***A61K 31/4178*** (2006.01) ***A61K 31/4439*** (2006.01)
***A61K 31/4184*** (2006.01)

(86) Internationale Anmeldenummer:
**PCT/EP2007/006325**

(87) Internationale Veröffentlichungsnummer:
**WO 2008/009415 (24.01.2008 Gazette 2008/04)**

(54) **4-HETEROARYL-SUBSTITUIERTE 1-AMINOCYCLOHEXAN-1- UND CYCLOHEXEN-1-DERIVATE MIT WIRKUNG AUF DAS OPIOD REZEPTOR SYSTEM**

4-HETEROARYL-SUBSTITUTED 1-AMINOCYCLOHEXANE-1-AND CYCLOHEXENE-1-DERIVATIVES HAVING EFFECTS ON THE OPIOD RECEPTOR SYSTEM

DERIVES DE 1-AMINOCYCLOHEXANE ET 1-CYCLOHEXENE SUBSTITUES EN POSITION 4 PAR UN HETEROARYLE PRESENTANT UNE ACTIVITEE SUR LE SYSTEME DES RECEPTEURS OPIOIQUES

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**HR**

(30) Priorität: **18.07.2006 DE 102006033109**

(43) Veröffentlichungstag der Anmeldung:
**08.04.2009 Patentblatt 2009/15**

(73) Patentinhaber: **Grünenthal GmbH**
**52078 Aachen (DE)**

(72) Erfinder:
- **ZEMOLKA, Saskia**
**52066 Aachen (DE)**
- **SCHUNK, Stefan**
**52080 Aachen (DE)**
- **ENGLBERGER, Werner**
**52223 Stolberg (DE)**
- **KÖGEL, Babette-Yvonne**
**52379 Langerwehe-Hamich (DE)**
- **LINZ, Klaus**
**53343 Wachtberg (DE)**
- **SCHICK, Hans**
**13086 Berlin (DE)**
- **SONNENSCHEIN, Helmut**
**10245 Berlin (DE)**
- **GRAUBAUM, Heinz**
**12557 Berlin (DE)**
- **HINZE, Claudia**
**D-53639 Königswinter (DE)**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
**WO-A-99/51576 WO-A-2004/043967**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 044 016 B1

## Beschreibung

Die vorliegende Erfindung betrifft substituierte Heteroaryl-Derivate, Verfahren zu deren Herstellung, Arzneimittel enthaltend diese Verbindungen und die Verwendung von substituierten Heteroaryl-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische μ-Opioide wie Morphin sind bei der Therapie starker bis stärkster Schmerzen gut wirksam und von größter Bedeutung für die Schmerztherapie. Es kann jedoch von Vorteil sein, wenn neben dem μ-Opioid-Rezeptor auch andere Opioid-Rezeptoren, insbesondere der ORL-1-Rezeptor, beeinflusst werden, da die reinen μ-Opioide auch unerwünschte Nebenwirkungen wie Obstipation und Atemdepression aufweisen, aber auch zu Abhängigkeit führen können. Auch die Opioid-Rezeptoren δ, κ und ORL-1 sind am Schmerzgeschehen beteiligt (Opioids: Introduction, S. 127-150, Further Opioid Receptors, 455-476 in: Analgesics - From Chemistry and Pharmacology to Clinical Application. Wiley VCH, 2002).

Der ORL1-Rezeptor ist außerdem noch an der Regulation weiterer physiologischer und pathophysiologischer Prozesse beteiligt. Hierzu gehören unter anderem Lemen und Gedächtnisbildung (Manabe et al., Nature, 394, 1997, S. 577-581), Hörvermögen (Nishi et al., EMBO J., 16, 1997, S. 1858-1864) sowie zahlreiche weitere.Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese. Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Aus dem Stand der Technik (WO 02090317) sind strukturell verwandte Verbindungen bekannt, die eine Affinität zum ORL-1-Rezeptor, besitzen, wobei jedoch keine Affinität zum μ-Opioid-Rezeptor beschrieben ist. Bei diesen Verbindungen ist jedoch der Heteroarylring über einen Stickstoff mit dem Cyclohexanring verknüpft.

Aus WO 99/51576 sind N-Aryloxyethylamin-Derivate und aus WO 2004/043967 sind spirocyclische Cyclohexan-Derivate bekannt.

Aufgabe der vorliegenden Erfindung war es, weitere Arzneimittel zur Verfügung zu stellen, die auf das Opioid-Rezeptor-System wirken und damit für Arzneimittel insbesondere zur Behandlung der verschiedenen mit diesem System in Verbindung stehenden Krankeiten bzw. zum Einsatz in den damit zusammenhängenden Indikationen geeignet sind. Gegenstand der Erfindung sind daher substituierte Heteroaryl-Derivate der allgemeinen Formel I,

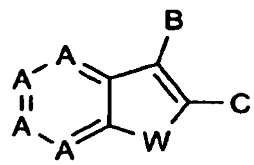

I

worin

A für N oder $CR^{7-10}$ steht, wobei A höchstens zweimal für N steht
W für O, S oder $NR^4$ steht
mit der Maßgabe, dass wenn W für O oder S steht, A $CR^{7-10}$ bedeutet;

einer der Reste B oder C für H; $C_{1-8}$-Alkyl jeweils gesättigt oder ungesättigt, verzweigt oder unverzweigt, einfach oder mehrfach substituiert oder unsubstituiert, $COR^{12}$; $SO_2R^{12}$; über $C_{1-3}$-Alkyl gebundenes Aryl, $C_{3-8}$-Cycloalkyl oder He-

teroaryl, jeweils einfach oder mehrfach substituiert oder unsubstituiert; Aryl-, oder Heteroaryl, jeweils einfach oder mehrfach substituiert oder unsubstituiert; $C_{3-4}$-Cycloalkyl, jeweils einfach oder mehrfach substituiert oder unsubstituiert; steht, und der jeweils andere Rest B oder C für

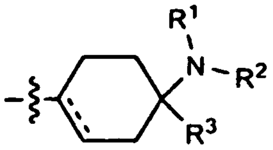

steht
wobei

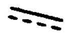

für eine Einfachbindung oder eine Doppelbindung steht,

$R^1$ und $R^2$, unabhängig voneinander für H; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils einfach oder mehrfach substituiert oder unsubstituiert; Aryl oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;
oder die Reste $R^1$ und $R^2$ zusammen für $CH_2CH_2OCH_2CH_2$, $CH_2CH_2NR^{11}CH_2CH_2$ oder $(CH_2)_{3-6}$ stehen,

wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; C(O)Phenyl, C(O)Heteroaryl, $C(O)C_{1-5}$-Alkyl, jeweils substituiert oder unsubstituiert, bedeutet;

und

$R^3$ für $C_{1-8}$-Alkyl, jeweils gesättigt oder ungesättigt, verzweigt oder unverzweigt, einfach oder mehrfach substituiert oder unsubstituiert; $C_{3-8}$-Cycloalkyl, jeweils einfach oder mehrfach substituiert oder unsubstituiert; Aryl oder Heteroaryl, jeweils unsubstituiert oder einfach oder mehrfach substituiert; über $C_{1-3}$-Alkyl-Gruppe gebundenes Aryl, Heteroaryl oder $C_{3-8}$-Cycloalkyl, jeweils unsubstituiert oder einfach oder mehrfach substituiert, steht;

$R^4$ für H; $C_{1-5}$-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$-Alkyl-Gruppe gebundenes Aryl, Heteroaryl oder Cycloalkyl, jeweils einfach oder mehrfach substituiert oder unsubstituiert; $COR^{12}$; $SO_2R^{12}$ steht, wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $OR^{13}$; $NR^{14}R^{15}$ bedeutet;

$R^7$, $R^8$, $R^9$ und $R^{10}$ unabhängig voneinander für H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$, $NHC(O)NHR^{13}$, $NHC(O)R^{13}$, $NH(CNR^{13})NHR^{13}$, $SO_2NHR^{13}$, $C_{1-5}$-Alkyl, $C_{3-8}$-Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, stehen;
wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeutet;
oder $R^7$, $R^8$ und $R^9$ die oben angegebene Bedeutung haben und $R^{10}$ mit B zusammen für - $CH_2CH_2CH_2$- steht und $R^{10}$ und B somit einen sechsgliedrigen Ring bilden,

$R^{14}$ und $R^{15}$ unabhängig voneinander H; $C_{1-5}$-Alkyl , jeweils gesättigt oder ungesättigt, verzweigt oder unverzweigt, unsubstituiert oder einfach oder mehrfach substituiert; oder $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeuten;

oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$-Alkyl gesättigt oder ungesättigt, verzweigt oder unverzweigt, unsubstituiert oder einfach oder mehrfach substituiert, bedeutet;

worin der Ausdruck "Cycloalkyl" oder "$C_{3-8}$-Cycloalkyl" für cyclische Kohlenwasserstoffe mit 3, 4, 5, 6, 7 oder 8 Kohlenstoffatomen steht, wobei die Kohlenwasserstoffe gesättigt oder ungesättigt (aber nicht aromatisch), unsubstituiert oder ein- oder mehrfach substituiert sein können, und wobei auch gesättigte oder ungesättigte (aber nicht aromatische) Cycloalkyle, in denen ein oder zwei Kohlenstoffatome durch ein Heteroatom S, N oder O ersetzt sind, umfasst sind, und wobei ein Cycloalkyl-Ring auch mit einem weiteren Ring kondensiert sein kann, der gesättigt oder ungesättigt (auch aromatisch) sein kann,

worin der Ausdruck "Aryl" für aromatische Kohlenwasserstoffe steht, diese Aryl-Reste auch mit weiteren gesättigten, (partiell) ungesättigten oder aromatischen Ringsystemen kondensiert sein können, und jeder dieser Aryl-Reste unsubstituiert oder einfach oder mehrfach substituiert sein kann, wobei die Aryl-Substituenten gleich oder verschieden und in jeder beliebigen und möglichen Position des Aryls sein können,

worin der Ausdruck "Heteroaryl" für einen 5-, 6- oder 7-gliedrigen cyclischen aromatischen Rest steht, der mindestens 1, ggf. auch 2, 3, 4 oder 5 Heteroatome ausgewählt aus der Gruppe bestehend aus Stickstoff, Sauerstoff und Schwefel enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein; und wobei der Heterocyclus auch Teil eines bi- oder polycyclischen Systems sein kann,

worin die vorstehend genannten $C_{1-8}$-Alkyle. $C_{1-5}$-Alkyle, $C_{1-3}$-Alkyle bzw. $C_{1-3}$-Alkylene bzw. $C_{3-8}$-Cycloalkylreste jeweils einfach oder mehrfach mit F, Cl, Br, I, -CN, $NH_2$, NH-$C_{1-6}$-Alkyl, NH-$C_{1-6}$-Alkyl-OH, N($C_{1-6}$-Alkyl)$_2$, N($C_{1-6}$-Alkyl-OH)$_2$, $NO_2$, SH, S-$C_{1-6}$-Alkyl, S-Benzyl, $OCF_3$, O-$C_{1-6}$-Alkyl, OH, O-$C_{1-6}$-Alkyl-OH, =O, $C_{1-6}$-Alkyl. Benzyl, O-Benzyl, O-Phenyl, C(=O)$C_{1-6}$-Alkyl, $CO_2H$, NHC(=O)$C_{1-6}$-Alkyl, OC(=O)$C_{1-6}$Alkyl, $CO_2$-$C_{1-6}$-Alkyl, substituiert sein können,

worin die vorstehend genannten Aryl- oder Heteroaryl-Reste jeweils einfach oder mehrfach mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, $CO_2H$, $CO_2$-$C_{1-6}$-Alkyl, $CF_3$, $OCF_3$, $C_{1-6}$-Alkyl oder Phenoxy substituiert sein können,

in Form des Razemats; der Enantiomere, Diastereomere. Mischungen der Enantiomere oder Diastereomere oder eines einzelnen Enantiomers oder Diastereomers; der Basen und/oder Salze physiologisch verträglicher Säuren.

Wenn ein Rest, beispielsweise $R^{13}$, innerhalb einer Verbindung mehr als einmal vorkommt, z. B. in NH(CN$R^{13}$)N$HR^{13}$, kann der Rest innerhalb desselben Moleküls unterschiedliche Bedeutungen annehmen. So kann NH(CN$R^{13}$)N$HR^{13}$ beispielsweise

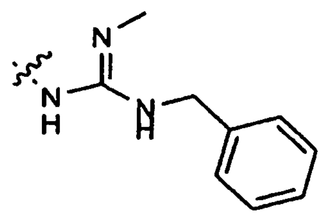

bedeuten.

Die Ausdrücke "$C_{1-8}$-Alkyl", $C_{1-3}$-Alkyl"," und "$C_{1-5}$-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 8 bzw. 1 bis 3 C-Atomen bzw. 1-5 C-Atomen, d.h. $C_{1-8}$-Alkanyle, $C_{2-8}$-Alkenyle und $C_{2-8}$-Alkinyle bzw. $C_{1-3}$-Alkanyle, $C_{2-3}$-Alkenyle und $C_{2-3}$-Alkinyle bzw $C_{1-5}$-Alkanyle, $C_{2-5}$-Alkenyle und $C_{2-5}$-Alkinyle. Dabei weisen Alkenyle mindestens eine C-C-Doppelbindung und Alkinyle mindestens eine GC-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, n-Hexyl, 2-Hexyl, n-Heptyl, n-Octyl, 1,1,3,3-Tetramethylbutyl; Ethylenyl (Vinyl), Ethinyl, Propenyl ($-CH_2CH=CH_2$, $-CH=CH-CH_3$, $-C(=CH_2)-CH_3$), Propinyl ($-CH-C\equiv CH$, $-C\equiv C-CH_3$), Butenyl, Butinyl, Pentenyl, Pentinyl, Hexenyl, Hexinyl, Heptenyl, Heptinyl, Octenyl und Octinyl umfasst. Besonders vorteihaft sind Methyl, Ethyl, n-Propyl, n-Butyl, sec-Butyl, iso-Butyl.

Der Ausdruck "Cycloalkyl" oder "$C_{3-8}$-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Cycloalkyl-Ringe, die ein Heteroatom enthalten, werden in der Literatur manchmal als "Heterocyclyl-Ringe" bezeichnet; im Sinne dieser Erfindung sind diese "Heterocyclyl-Ringe" durch den Begriff $C_{3-8}$-Cycloalkyl ausdrücklich abgedeckt. Daher steht der Begriff "$C_{3-8}$-Cycloalkyl" in der Beschreibung und den Ansprüchen jeweils für "$C_{3-8}$-Cycloalkyl (ohne Heteroatome im Ring)" und gleichzeitig auch für "einen drei bis achtgliedrigen Heterocyclyl-Ring". Der Cycloalkyl-Ring kann auch mit einem weiteren Ring kondensiert sein, der gesättigt, ungesättigt (auch aromatisch) sein kann. Vorteilhaft ist $C_{3-8}$-Cycloalkyl aus der Gruppe, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Tetrahydrochinolinyl, Tetrahydroisochinolinyl, Isoindolinyl, Thiazolidinyl, Morpholinyl, Piperidinyl, Piperazinyl, Pyrazolinonyl und Pyrrolidinyl enthält. Besonders bevorzugt ist Cyclopropyl, Cyclopentyl, Cyclohexyl, Pyrrolidinyl, Tetrahydrochinolinyl, Piperidyl, Tetrahydroisochinolinyl, Isoindolinyl, Piperazinyl, Morpholinyl und Thiazolinyl.

Der Ausdruck "Aryl" bedeutet im Sinne dieser Erfindung aromatische Kohlenwasserstoffe, u.a. Phenyle und Naphthyle. Die Aryl-Reste können auch mit weiteren gesättigten, (partiell) ungesättigten oder aromatischen Ringsystemen kondensiert sein, sodass der Arylrest ein aromatisches Ringsystem von höchstens 20 C-Atomen bildet. Jeder dieser $C_{6-20}$-Aryl-Reste kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl. 2-Naphthyl, welche jeweils unsubstituiert oder ein- oder mehrfach substituiert sein können, enthält. Besonders vorteilhaft ist der Phenyl-Rest.

Der Ausdruch "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bicyclischen oder polycyclischen Systems mit insgesamt bis zu 20 Ringgliedern sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Benzimidazolyl, Phtalazinyl, Pyrazolyl, Imidazolyl, Triazolyl, Tetrazolyl, Imidazolyl, Thiazolyl, Oxazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann. Besonders bevorzugt sind Pyridyl, Imidazolyl, Thienyl, Benzimidazolyl, Pyrrolyl, Triazolyl, Pyrazolyl und Tetrazolyl.

Der Ausdruck "über $C_{1-3}$-Alkyl gebundenes Aryl oder Heteroaryl" bedeuten für die Zwecke der vorliegende Erfindung, daß $C_{1-3}$-Alkyl und Aryl bzw. Heteroaryl die oben definierten Bedeutungen haben und der Aryl- bzw. Heteroaryl-Rest über eine $C_{1-3}$-Alkyl-Gruppe an die Verbindung der allgemeinen Struktur I gebunden ist. Besonders vorteilhaft im Sinne dieser Erfindung sind Benzyl. Methylpyridyl und Phenethyl.

Im Zusammenhang mit "Alkyl" oder "Cycloalkyl" versteht man unter dem Begriff "substituiert" im Sinne dieser Erfindung die Substitution eines Wasserstoffrestes durch F, Cl, Br, I, -CN, $NH_2$, NH-$C_{1-6}$-Alkyl, NH-$C_{1-6}$-Alkyl-OH, N ($C_{1-6}$-Alkyl)$_2$, N($C_{1-6}$-Alkyl-OH)$_2$, $NO_2$, SH, S-$C_{1-6}$-Alkyl, OC(=O)$C_{1-6}$-Alkyl, S-Benzyl, $OCF_3$, O-$C_{1-6}$-Alkyl, OH, O-$C_{1-6}$-Alkyl-OH, =O, $C_{1-6}$-Alkyl, Benzyl, O-Benzyl, O-Phenyl, C(=O)$C_{1-6}$-Alkyl, $CO_2H$, NHC(=O)$C_{1-6}$-Alkyl, $CO_2$-$C_{1-6}$,-Alkyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von $CF_3$ oder -$CH_2CF_3$ oder an verschiedenen Stellen wie im Falle von -CH(OHyCH=CH-$CHCl_2$. Die Mehrfachsubstitution kann mit dem gleichen oder mit verschiedenen Substituenten erfolgen. Besonders bevorzugt für die Zwecke der vorliegenden Erfindung bedeutet "einfach oder mehrfach substituiert" im Zusammenhang mit Alkyl F, Cl, $NH_2$, SH, S-$C_{1-6}$-Alkyl, OC(=O)$C_{1-6}$-Alkyl. O-$C_{1-6}$-Alkyl, OH, =O, C(=O)$C_{1-6}$-Alkyl, $CO_2H$, NHC(=O)$C_{1-6}$-Alkyl und $CO_2$-$C_{1-6}$-Alkyl.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl. OH, O-$C_{1-6}$-Alkyl, O-$C_{1-6}$Alkyl-OH, C(=O)$C_{1-6}$-Alkyl, $CO_2H$, $CO_2C_{1-6}$-Alkyl. $CF_3$, $OCF_3$, $C_{1-6}$-Alkyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" und "Heteroaryl" sind dabei bevorzugte Substituenten F, Cl, CN, $NH_2$, $NO_2$, SH, OH, O-$C_{1-6}$-Alkyl, C(=O)$C_{1-6}$-Alkyl, $CO_2H$, $CO_2$-$C_{1-6}$-Alkyl, $CF_3$, $OCF_3$ und $C_{1-6}$-Alkyl. Besonders bevorzugt sind -F und -Cl.

Erfindungsgemäße Verbindungen, bei denen $R^{10}$ mit B zusammen für -$CH_2CH_2CH_2$- steht und $R^{10}$ und B somit

einen sechsgliedrigen Ring bilden, haben die folgende allgemeine Formel:

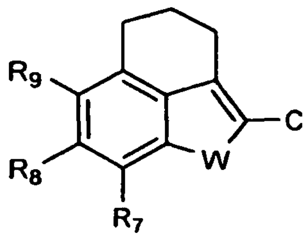

wobei die übrigen Reste die oben angegebene Bedeutung haben.

Unter dem Begriff des mit einer physiologisch verträglichen Säure gebildeten Salzes versteht man im Sinne dieser Erfindung Salze des jeweiligen Wrkstoffes mit anorganischen bzw. organischen Säuren, die physiologisch - insbesondere bei Anwendung im Menschen und/oder Säugetier - verträglich sind. Bevorzugt ist das Hydrochlorid, das Citrat, das Hemicitrat und das Methansulfonat. Besonders bevorzugt ist das Methansulfonat. Beispiele für physiologisch verträgliche Säuren sind: Salzsäure, Bromwasserstoffsäure, Schwefelsäure, Methansulfonsäure, Ameisensäure. Essigsäure. Oxalsäure, Bernsteinsäure, Weinsäure, Mandelsäure, Fumarsäure, Milchsäure, Äpfelsäure, Maleinsäure, Zitronensäure, Glutaminsäure, 1,1-Dioxo-1,2-dihydro1$\lambda^6$-benzo[*d*]isothiazol-3-on (Saccharinsäure), Monomethylsebacinsäure. 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Hippursäure, Phosphorsäure und/oder Asparaginsäure. Bevorzugt sind Citronensäure, Methansulfonsäure und Salzsäure. Besonders bevorzugt ist die Methansulfonsäure.

Unter dem Begriff $(CH_2)_{3-6}$ bzw $(CH_2)_{4-5}$ ist $-CH_2CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ bzw. $-CH_2-CH_2-CH_2-CH_2-$ und $-CH_2-CH_2-CH_2-CH_2-CH_2-$zu verstehen.

Es ist bevorzugt, dass $R^1$ und $R^2$ nicht gleichzeitig H bedeuten.

Bevorzugt sind Verbindungen, worin C für

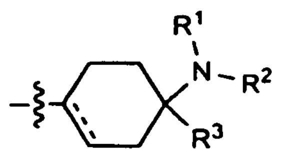

steht und B H; $C_{1-8}$-Alkyl jeweils gesättigt oder ungesättigt, verzweigt oder unverzweigt, einfach oder mehrfach substituiert oder unsubstituiert, $COR^{12}$ ; $SO_2R^{12}$; über $C_{1-3}$-Alkyl gebundenes Aryl, $C_{3-8}$-Cycloalkyl oder Heteroaryl, jeweils einfach oder mehrfach substituiert oder unsubstituiert, Aryl-, oder Heteroaryl, jeweils einfach oder mehrfach substituiert oder unsubstituiert; $C_{3-8}$-Cycloalkyl, jeweils einfach oder mehrfach substituiert oder unsubstituiert bedeutet.

Weiterhin bevorzugt sind Verbindungen, worin 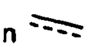 für eine Einfachbindung steht.

Für eine bevorzugte Ausführungsform der erfindungsgemäßen Verbindungen gilt, dass
die vorstehend genannten $C_{1-8}$-Alkyle, $C_{1-5}$-Alkyle, $C_{1-3}$-Alkyle bzw. $C_{1-3}$-Alkylene bzw. $C_{3-8}$-Cycloalkylreste jeweils einfach oder mehrfach mit F, Cl, Br, I, -CN, $NH_2$, NH-$C_{1-6}$-Alkyl, NH-$C_{1-6}$-Alkyl-OH, N($C_{1-6}$-Alkyl)$_2$, N($C_{1-6}$-Alkyl-OH)$_2$, $NO_2$, SH, S-$C_{1-6}$-Alkyl, S-Benzyl, $OCF_3$, O-$C_{1-6}$-Alkyl, OH, O-$C_{1-6}$-Alkyl-OH, =O, $C_{1-6}$-Alkyl, Benzyl, O-Benzyl, O-Phenyl, C(=O)$C_{1-6}$-Alkyl, $CO_2H$, NHC(=O)$C_{1-6}$-Alkyl, OC(=O)$C_{1-6}$-Alkyl, $CO_2$-$C_{1-6}$-Alkyl, substituiert sein können,
die vorstehend genannten Aryl- oder Heteroaryl-Reste jeweils einfach oder mehrfach mit F, Cl. Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, $CO_2H$, $CO_2$-$C_{1-6}$-Alkyl, $CF_3$, $OCF_3$, $C_{1-6}$-Alkyl oder Phenoxy substituiert sein können,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die nachfolgend als bevorzugt beschriebenen Reste und Gruppen bzw. Substituenten können in den erfindungsgemäßen Verbindungen mit der breitesten Bedeutung der übrigen Reste, aber auch mit bevorzugten Bedeutungen anderer Reste und Gruppen bzw. Substituenten kombiniert werden.

Bevorzugt sind substituierte Heteroaryl-Derivate, worin W für $NR^4$ steht.

Weiterhin bevorzugt sind auch Heteroaryl-Derivate, worin A für $CR^{7-10}$ steht oder die allgemeine Formel I die Bedeutungen der allgemeinen Formeln Ia und Ib annimmt:

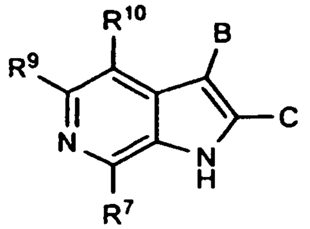

Ia

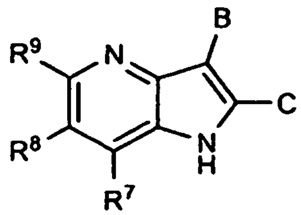

Ib

Bevorzugt sind weiterhin substituierte Heteroaryl-Derivate, worin
$R^1$ und $R^2$ unabhängig voneinander für H; $C_{1-5}$-Alkyl, gesättigt oder ungesättigt, verzweigt oder unverzweigt, einfach oder mehrfach substituiert oder unsubstituiert, stehen;
oder die Reste $R^1$ und $R^2$ zusammen einen Ring bilden und $CH_2CH_2OCH_2CH_2$, $CH_2CH_2NR^{11}H_2CH_2$ oder $(CH_2)_{3-6}$ bedeuten,

Besonders bevorzugt sind substituierte Heteroaryl-Derivate, worin $R^1$ und $R^2$ unabhängig voneinander für $CH_3$ oder H stehen, wobei $R^1$ und $R^2$ nicht gleichzeitig H bedeuten, oder $R^1$ und $R^2$ einen Ring bilden und $CH_2CH_2OCH_2CH_2$, $CH_2CH_2N(CH_3)CH_2CH_2$, $CH_2CH_2CH_2$, $CH_2CH_2CH_2CH_2$ oder $CH_2CH_2CH_2CH_2CH_2$ bedeuten.

Ganz besonders bevorzugt sind substituierte Heteroaryl-Derivate, worin $R^1$ und $R^2$ für $CH_3$ oder H stehen, wobei $R^1$ und $R^2$ nicht gleichzeitig H bedeuten.

Weiterhin bevorzugt sind substituierte Heteroaryl-Derivate, worin
$R^3$ für $C_{1-6}$-Alkyl, Cyclopentyl, Cyclohexyl, Phenyl, Benzyl, Naphthyl, Thiazolyl, Thiophenyl, Triazolyl, Benzimidazolyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte $C_{1-3}$-Alkyl-Gruppe gebundenes Cyclopentyl, Cyclohexyl, Phenyl, Naphthyl, Thiophenyl, Benzothiophenyl, Pyridyl. Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
insbesondere
$R^3$ Butyl, Phenyl, Thiophenyl, Thiazolyl, Cyclopentyl, Cyclohexyl, Naphthyl, Benzyl, Benzofuranyl, 1,2,4-Triazolyl, Benzimidazolyl, Benzodioxanyl, Benzodioxolanyl, Pyridyl oder Benzothiophenyl, jeweils unsubstituiert oder einfach oder mehrfach substituiert; über eine gesättigte, unverzweigte $C_{1-3}$-Alkyl-Gruppe gebundenen Phenyl, Furyl oder Thiophenyl, jeweils unsubstituiert oder einfach oder mehrfach substituiert, bedeutet.

Besonders bevorzugt sind substituierte Heteroaryl-Derivate, worin $R^3$ Phenyl, 4-Fluorphenyl, Benzyl, Butyl oder Benzothiophenyl bedeutet.

Bevorzugt sind auch substituierte Heteroaryl-Derivate, worin B oder C
für $C_{1-8}$-Alkyl jeweils gesättigt oder ungesättigt, verzweigt oder unverzweigt, einfach oder mehrfach substituiert oder unsubstituiert, stehen; vorzugsweise für $C_{1-4}$-Alkyl, einfach oder mehrfach substituiert oder unsubstituiert.

Weiterhin bevorzugt sind substituierte Heteroaryl-Derivate, worin B oder C für

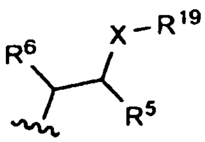

stehen, wobei

X für O, $NR^{20}$, S oder $CH_2$ steht;

$R^5$ für =O; H; $COOR^{13}$, $CONR^{13}$, $OR^{13}$, $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}$ $R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl,

gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;
oder $R^5$ und $R^6$ gemeinsam $(CH_2)_n$ mit n = 2, 3, 4, 5 oder 6 bedeuten, wobei einzelne Wasserstoffatome auch durch F, Cl. Br, I, $NO_2$, $CF_3$, $OR^{13}$, CN oder $C_{1-5}$-Alkyl ersetzt sein können;

$R^{19}$ H; Aryl; Benzyl; $C(=O)C_{1-5}$-Alkyl; $C_{1-5}$-Alkyl bedeutet und

$R^{20}$ H oder $C_{1-5}$-Alkyl bedeutet.

Es ist bevorzugt, dass $R^5$ für H, $CH_3$, $CH_2OH$, COOH oder $COOCH_3$ steht, vorzugsweise für H.
Bevorzugt sind auch substituierte Heteroaryl-Derivate, worin $R^6$ für H, $C_{1-5}$-Alkyl, Aryl oder über eine $C_{1-3}$-Alkylgruppe verknüpftes Aryl steht, vorzugsweise H.
Bevorzugt sind darüber hinaus auch substituierte Heteroaryl-Derivate, worin B oder C für $(CH_2)_{1-4}$-$R^{21}$ steht, wobei $R^{21}$ für H, OH, SH, $COOC_{1-6}$-Alkyl, COOH, $OC(=O)C_{1-6}$-Alkyl, $NH_2$, $NHC(=O)C_{1-6}$-Alkyl; oder $C_{3-8}$Cycloalkyl, Aryl oder Heteroaryl, jeweils unsubstituiert oder einfach oder mehrfach substituiert, vorzugsweise für Phenyl, Benzimidazol, Pyridyl, Triazolyl, Phenyl, Pyrazolyl, Tetrazolyl oder Imidazolyl, Pyrrolidinyl, Tetrahydrochinolinyl, Tetrahydroisochinolinyl, Thiazolidinyl, Isoindolinyl, Piperazinyl, Morpholinyl, Cyclohexyl, Piperidyl, Pyrrolidinyl oder Cyclopropyl, jeweils unsubstituiert oder einfach oder mehrfach substituiert, steht.
Besonders bevorzugt sind substituierte Heteroaryl-Derivate, worin $R^{21}$ für OH, SH, $COOCH_3$, COOH, $OC(=O)CH_3$, $NH_2$, $NHC(=O)CH_3$, $NHC(=O)CH_2C(CH_3)_3$; oder Phenyl, Benzimidazol, Pyridyl, Triazolyl, Phenyl, Pyrazolyl, Tetrazolyl oder Imidazolyl, jeweils unsubstituiert oder substituiert mit $COOCH_3$. $CH_3$; oder Cyclopropyl, Cyclohexyl, Pyrrolidinyl, Tetrahydrochinolinyl, Pyrrolidinyl, Piperidyl, Tetrahydroisochinolinyl, Isoindolinyl, Piperazinyl, Morpholinyl oder Thiazolinyl jeweils unsubstituiert oder substituiert mit =O oder $CH_3$, steht.
Bevorzugt sind auch substituierte Heteroaryl-Derivate, worin B $C_{3-8}$-Cycloalkyl bedeutet, insbesondere Cyclopropyl.
Darüber hinaus sind auch substituierte Heteroaryl-Derivate bevorzugt, worin $R^7$, $R^8$, $R^9$ und $R^{10}$ unabhängig voneinander für H; Methyl; Ethyl; Propyl; Butyl; Pyridyl, O-Benzyl, F, Cl. Br, I, CN, $CF_3$, $OCF_3$, OH, $OCH_3$, $NH_2$, COOH, $COOCH_3$, $NHCH_3$ oder $N(CH_3)_2$ oder $NO_2$ stehen. Besonders bevorzugt sind substituierte Heteroaryl-Derivate, worin $R^7$. $R^8$. $R^9$ und $R^{10}$ unabhängig voneinander für H, F, Cl, $NO_2$, CN, $CF_3$. $OCH_3$, $OCF_3$ oder OH stehen.
Ganz besonders bevorzugt sind substituierte Heteroaryl-Derivate, worin $R^7$. $R^8$, $R^9$ und $R^{10}$ für H stehen.
Bevorzugt sind auch substituierte Heteroaryl-Derivate, worin $R^4$ H; $C_{1-3}$-Alkyl-$C_{3-8}$-Cycloalkyl. $C_{1-6}$-Alkyl, unsubstituiert oder einfach oder mehrfach substituiert; oder $SO_2$-Phenyl oder CO-Phenyl, jeweils unsubstituiert oder einfach oder mehrfach substituiert, insbesondere $R^4$ Methyloxiran, $CH_2CH(OH)CH_2N(CH_3)_2$, $SO_2$-Phenyl, $CH_2CH_2OH$, $CH_2CH(OH)CH_2NHCH_3$ oder $CH_3$ bedeutet
Besonders bevorzugt sind weiterhin substituierte Heteroaryl-Derivate, worin $R^4$ für H steht.
Am meisten bevorzugt sind substituierte Heteroaryl-Derivate aus der Gruppe

(1) 2-(2-(4-(Dimethylamino)4-phenylcyclohexyl)-1H-indol-3-yl)ethanol, Citrat
(3) (±) 2-(2-(4-(Dimethylamino)-4-phenylcyclohex-1-enyl)-1H-indol-3-yl)ethyl acetat Hydrochlorid
(4) (±) 2-(4-(Dimethylamino)-4-phenylcyclohex-1-enyl)-3-(3-aminopropyl)-1H-indol, Citrat
(6) (±) 3-(2-(4-(Dimethylamino)4-phenylcyclohex-1-enyl)-1H-indol-3-yl)propan-1-ol Hydrochlorid
(7) (±) 2-(5,6-Dichlor-2-(4-(dimethylamino)-4-phenylcyclohex-1-enyl)-1H-indol-3-yl)ethanol, Citrat
(8) (±) 2-(2-(4-Morpholino-4-phenylcyclohex-1-enyl)-1H-indol-3-yl)ethanol, Citrat
(9) (±) 2-(4,6-Dichlor-2-(4-(dimethylamino)-4-phenylcyclohex-1-enyl)-1H-indol-3-yl)ethanol, Citrat
(10) (±) 2-(2-(4-(Dimethylamino)-4-phenylcyclohex-1-enyl)-5-fluor-1H-indol-3-yl)ethanol, Citrat
(11) (±) 2-(2-(4-(Dimethylamino)-4-phenylcyclohex-1-enyl)-5-(pyridin-3-yl)-1H-indol-3-yl)ethanol, Citrat
(13) (±) 2-(2-(4-(Dimethylamino)-4-phenylcyclohex-1-enyl)-5-nitro-l H-indol-3-yl)ethanol, Citrat
(14) (±) 2-(2-(4-(Benzo[b]thiophen-2-yl)-4-(dimethylamino)cyclohex-1-enyl)-1H-indol-3-yl)ethanol, Citrat
(15) (±) 2-(2-(4-(Benzo[b]thiophen-2-yl)-4-(dimethylamino)cyclohex-1-enyl)-5-fluor-1H-indol-3-yl)ethanol, Citrat
(16) (±) 2-(4-(Dimethylamino)-4-phenylcyclohex-1-enyl)-3-methyl-1H-indol, Citrat
(17) N,N-Dimethyl-4-(3-methyl-1H-indol-2-yl)-1-phenylcyclohexanamin, Citrat
(18) N,N-Dimethyl-4-(3-methyl-1H-indol-2-yl)-1-phenylcyclohexanamin, Citrat
(19) 2-(2-(2-(4-(Dimethylamino)-4-phenylcyclohexyl)-1H-indol-3-yl)ethyl)isoindolin-1,3-dione, Citrat
(20) N-(2-(2-(4-(Dimethylamino)-4-phenylcyclohexyl)-1H-indol-3-yl)ethyl)acetamid, Citrat
(21) N-(2-(2-(4-(Dimethylamino)-4-phenylcyclohexyl)-1H-indol-3-yl)ethyl)acetamid, Citrat
(22) (±)-2-(4-Benzyl-4-(dimethylamino)cyclohex-1-enyl)-3-methyl-1H-indol-5-carbonitril
(23) (±)-2-(4-(Dimethylamino)4-phenylcyclohex-1-enyl)-3-methyl-1H-indol-5-carbonitril

(24) (±)-2-(4-(Dimethylamino)-4-butylcyclohex-1-enyl)-3-methyl-5-trifluormethyl-1H-indol
(25) (±)-2-(4-(Dimethylamino)-4-phenylcyclohex-l-enyl)-3-methyl-5-trifluormethyl-1H-indol. Citrat
(26) (±)-2-(4-(Dimethylamino)-4-benzylcyclohex-1-enyl)-3-methyl-5-fluor-1H-indol, Citrat
(27) (±)-2-(4-(Dimethy)amino)-4-butytcyclohex-1-eny))-3-methyl-5-fluor-1H-indol, Citrat
(28) (±)-2-(4-(Dimethylamino)-4-phenylcyclohex-1-enyl)-3-methyl-5-fluor-1H-indol, Citrat
(29) (±)-2-(4-(Dimethylamino)-4-phenylcyclohex-1-enyl)-3-methyl-5-methoxy-1H-indol, Citrat
(30) (±)-2-(4-(Dimethylamino)-4-benzylcyclohex-1-enyl)-3-methyl-1H-indol, Citrat
(31) (±)-2-(4-(Dimethylamino)-4-butylcyclohex-1-enyl)-3-methyl-1H-indol, Citrat
(32) (±)-2-(4-(Dimethylamino)-4-phenylcyclohex-1-enyl)-3-methyl-1H-pyrrolo[2,3-b]pyridin, Citrat
(33) (±)-2-(4-(Dimethylamino)-4-benzylcyclohex-1-enyl)-3-cyclopropyl-1H-indol Hydrochlorid
(34) (±)-2-(4-(Dimethylamino)-4-butylcyclohex-1-enyl)-3-cyclopropyl-1H-indol
(35) (±)-2-(4-(Dimethylamino)-4-phenylcyclohex-1-enyl)-3-cyclopropyl-1H-indol
(36) (±)-2-(4-(Dimethylamino)-4-benzylcyclohex-1-enyl)-3-(cyclohexylmethyl)-1H-indol
(37) (±)-2-(4-(Dimethylamino)-4-benzylcyclohex-1-enyl)-3-benzyl-1H-indol Hydrochlorid
(38) (±)-2-(4-(Dimethylamino)-4-butylcyclohex-1-enyl)-3-(cyclohexylmethyl)-1H-indol Hydrochlorid
(39) (±)-2-(4-(Dimethylamino)-4-butylcyclohex-1-enyl)-3-benzyl-1H-indol
(40) (t)-2-(4-(Dimethytamino)-4-phenylcyclohex-1-enyl)-3-(cyclohexytmethyl)-1H-indol Hydrochlorid
(41) (±)-2-(4-(Dimethylamino)-4-phenylcyclohex-1-enyl)-3-benzyl-1H-indol Hydrochlorid
(42) (±)-2-(4-(Dimethylamino)-4-benzylcyclohex-1-enyl)-3-propyl-1H-indol
(43) (±)-2-(4-(Dimethylamino)-4-butylcyclohex-1-enyl)-3-propyl-1H-indol
(44) (±)-2-(4-(Dimethylamino)-4-phenylcyclohex-1-enyl)-3-propyl-1H-indol
(45) (±) 2-(4-(dimethylamino)-4-benzylcyclohex-1-enyl)-3-(2-(pyridin-4-yl)ethyl)-1H-indol
(46) (±) 2-(4-(dimethylamino)-4-butylcyclohex-1-enyl)-3-(2-(pyridin-4-yl)ethyl)-1H-indol
(47) (±) 2-(4-(dimethylamino)-4-phenylcyclohex-1-enyl)-3-(2-(pyridin-4-yl)ethyl)-1H-indol
(48) (±)-3-(2-(4-Benzyl-4-(dimethylamino)cyclohex-1-enyl)-1H-indol-3-yl)propan-1-ol, Citrat
(49) (±)-3-(2-(4-Butyl-4-(dimethylamino)cyctohex-1-enyl)-1H-indol-3-yl)propan-1-ol, Citrat (51) (±) 2-(2-(2-(4-(Dimethylamino)-4-phenylcyclohex-1-enyl)-1H-indol-3-yl)ethyl)isoindolin-1,3-dione
(52) (±) 2-(4-(Dimethylamino)-4-benzylcyclohex-1-enyl)-3-(2-(1H-benzo[d]imidazol-1-yl)ethyl)-5-fluor-1H-Indol
(53) (±) 2-(4-(Dimethylamino)-4-butylcyclohex-1-enyl)-3-(2-(1H-benzo[d]imidazol-1-yl)ethyl)-5-fluor-1H-indol
(54) (±) 2-(4-(Dimethylamino)-4-phenylcyclohex-1-enyl)-3-(2-(1H-benzo[d]imidazol-1-yl)ethyl)-5-fluor-1H-indol
(55) (±) 2-(4-(Dimethylamino)-4-benzylcyclohex-1-enyl)3-(2-(piperidin-1-yl)ethyl)-5-fluor-1H-indol
(56) (±) 2-(4-(Dimethylamino)-4-butylcyclohex-1-enyl)-3-(2-(piperidin-1-yl)ethyl)-5-fluor-1H-indol
(57) (±) 2-(4-(Dimethylamino)-4-phenylcyclohex-1-enyl)-3-(2-(piperidin-1-yl)ethyl)-5-fluor-1H-indol
(58) (±) 2-(4-(Dimethylamino)-4-phenylcyclohex-1-enyl)-3-(2-(piperidin-1-yl)ethyl)-1H-indol, Citrat
(59) (±) 2-(4-(Dimethylamino)-4-benzylcyclohex-1-enyl)-3-(2-(1H-1,2,3-triazol-1-yl)ethyl)-5-fluor-1H-indol
(60) (±) 2-(4-(Dimethylamino)-4-butylcyclohex-l-enyl)-3-(2-(1H-1,2,3-triazol-1-yl)ethyl)-5-fluor-1H-indol
(61) (±) 2-(4-(Dimethylamino)-4-phenylcyclohex-1-enyl)-3-(2-(1H-1,2,3-triazol-1-yl)ethyl)-5-fluor-1H-indol
(62) N-(2-(2-(4-Butyl-4-(dimethylamino)cyclohexyl)-1H-indol-3-yl)ethyl)-3,3-dimethylbutanamid, Citrat
(63) (±) N-(2-(2-(4-(Dimethylamino)-4-phenylcyclohex-1-enyl)-1H-indol-3-yl)ethyl)acetamid
(64) (±) N-(2-(2-(4-Butyl-4-(dimethylamino)cyclohex-1-enyl)-1H-indol-3-yl)ethyl)-3,3-dimethylbutanamid
(65) (±)-2-(2-(4-(Dimethylamino)-4-phenylcyclohex-1-enyl)-5-fluor-6-methoxy-1H-indol-3-yl)ethanol, Citrat
(66) (±)-2-(2-(4-Benzyl-4-(4-methylpiperazin-1-yl)cyclohex-1-enyl)-5-fluor-1H-indol-3-yl)ethanol
(67) (±)-2-(5-fluor-2-(4-phenyl-4-(pyrrolidin-1-yl)cyclohex-1-enyl)-1H-indol-3-yl)ethanol
(68) 2-(4-Benzyl-4-(dimethylamino)cyctohexyl)-3-methyl-1H-indol-5-carbonitril, Citrat
(69) 2-(4-Benzyl-4-(dimethylamino)cyclohexyl)-3-methyl-1H-indol-5-carbonitril, Citrat
(70) 2-(4-Butyl-4-(dimethylamino)cyclohexyl)-3-methyl-1H-indol-5-carbonitril, Citrat
(71) 2-(4-Butyl-4-(dimethylamino)cyctohexyl)-3-methyl-1H-indol-5-carbonitril. Citrat
(72) 2-(4-(Dimethylamino)-4-phenylcyclohexyl)-3-methyl-1H-indol-5-carbonittil, Citrat
(73) 1-Benzyl-N,N-dimethyl-4-(3-methyl-5-(trifluormethyl)-1H-indol-2-yl)cyclohexanamin, Citrat
(74) 1-Benzyl-N,N-dimethyl-4-(3-methyl-5-(trifluormethyl)-1H-indol-2-yl)cyctohexanamin, Citrat
(75) 1-Butyl-N,N-dimethyl-4-(3-methyl-5-(trifluormethyl)-1H-indol-2-yl)cyclohexanamin, Citrat
(76) N,N-Dimethyl-4-(3-methyl-5-(trifluormethyl)-1H-indol-2-yl)-1-phenylcyclohexanamin, Citrat
(77) 1-Benzyl-4-(5-fluor-3-methyl-1H-indol-2-yl)-N,N-dimethylcyclohexanamin, Citrat
(78) 1-Benzyl-4-(5-fluor-3-methyl-1H-indol-2-yl)-N,N-dimethylcyclohexanamin, Citrat
(79) 1-Butyl-4-(5-fluor-3-methyl-1H-indol-2-yl)-N,N-dimethylcyclohexanamin Hydrochlorid
(80) 4-(5-Fluor-3-methyl-1H-indol-2-yl)-N,N-dimethyl-1-phenylcyclohexanamin, Citrat
(81) 4-(5-Fluor-3-methyl-1H-indol-2-yl)-N,N-dimethyl-1-phenylcyclohexanamin, Citrat
(82) 4-(3-(2-(1H-Benzo[d]imidazol-1-yl)ethyl)-5-fluor-1H-indol-2-yl)-1-butyl-N,N-dimethylcyclohexanamin, Citrat

(83) 4-(3-(2-(1 H-Benzo[d]imidazol-1-yl)ethyl)-5-fluor-1H-indol-2-yl)-N,N-dimethyl-1-phenylcyclohexanamin, Citrat
(84) 4-(3-(2-(1H-Benzo[d]imidazol-1-yl)ethyl)-5-fluor-1H-indol-2-yl)-N,N-dimethyl-1-phenylcyclohexanamin, Citrat
(85) 1-Benzyl-4-(5-fluor-3-(2-(pyrrolidin-1-yl)ethyl)-1H-indol-2-yl)-N,N-dimethylcyclohexanamin, Citrat
(86) 1-Benzyl-4-(5-fluor-3-(2-(pyrrolidin-1-yl)ethyl)-1H-indol-2-yl)-N,N-dimethylcyclohexanamin, Citrat
(87) 1-Butyl-4-(5-fluor-3-(2-(pyrrolidin-1-yl)ethyl)-1H-indol-2-yl)-N,N-dimethylcyclohexanamin, Citrat
(88) 1-Butyl-4-(5-fluor-3-(2-(pyrrolidin-1-yl)ethyl)-1H-indol-2-yl)-N,N-dimethylcyclohexanamin, Citrat
(89)4-(5-Fluor-3-(2-(pyrrolidin-1-yl)ethyl)-1H-indol-2-yl)-N,N-dimethyl-1-phenylcyclohexanamin, Citrat
(90) 4-(5-Fluor-3-(2-(pyrrolidin-1-yl)ethyl)-1H-indol-2-yl)-N,N-dimethyl-1-phenylcyclohexanamin, Citrat
(91) 1-Benzyl-4-(5-fluor-3-(2-(piperidin-1-yl)ethyl)-1H-indol-2-yl)-N,N-dimethylcyclohexanamin, Citrat
(92) 1-Benzyl-4-(5-fluor-3-(2-(piperidin-1-yl)ethyl)-1H-indol-2-yl)-N,N-dimethylcyclohexanamin, Citrat (2:3)
(93) 1-Butyl-4-(5-fluor-3-(2-(piperidin-1-yl)ethyl)-1H-indol-2-yl)-N,N-dimethylcyclohexanamin, Citrat (4:3)
(94) 1-Butyl-4-(5-fluor-3-(2-(piperidin-1-yl)ethyly)-1H-indol-2-yl)-N,N-dimethylcyclohexanamin, Citrat
(95) 4-(5-Fluor-3-(2-(piperidin-1-yl)ethyl)-1H-indol-2-yl)-N,N-dimethyl-1-phenylcyclohexanamin, Citrat
(96) 4-(5-Fluor-3-(2-(piperidin-1-yl)ethyl)-1H-indol-2-yl)-N,N-dimethyl-1-phenylcyclohexanamin, Citrat
(97) 4-(3-(2-(1H-pyrazol-1-yl)ethyl)-5-fluor-1H-indol-2-yl)-1-benzyl-N,N-dimethylcyclohexanamin, Citrat (2:3)
(98) 4-(3-(2-(1H-Pyrazol-1-yl)ethyl)-5-fluor-1H-indol-2-yl)-1-benzyl-N,N-dimethylcyclohexanamin, Citrat
(99) 4-(3-(2-(1H-Pyrazol-1-yl)ethyl)-5-fluor-1H-indol-2-yl)-1-butyl-N,N-dimethylcyclohexanamin. Citrat
(100) 4-(3-(2-(1H-Pyrazol-1-yl)ethyl)-5-fluor-1H-indol-2-yl)-1-butyl-N,N-dimethylcyclohexanamin, Citrat
(101) 4-(3-(2-(1H-Pyrazol-1-yl)ethyl)-5-fluor-1H-indol-2-yl)-N,N-dimethyl-1-phenylcyclohexanamin, Citrat
(102) 4-(3-(2-(1H-Pyrazol-1-yl)ethyl)-5-fluor-1H-indol-2-yl)-N,N-dimethyl-1-phenylcyclohexanamin, Citrat
(103) 4-(3-(2-(1H-Imidazol-1-yl)ethyl)-5-fluor-1H-indol-2-yl)-1-benzyl-N,N-dimethylcyclohexanamin, Citrat
(104) 4-(3-(2-(1H-Imidazol-1-yl)ethyl)-5-fluor-1H-indol-2-yl)-1-benzyl-N,N-dimethylcyclohexanamin, Citrat
(105) 4-(3-(2-(1H-Imidazol-1-yl)ethyl)-5-fluor-1H-indol-2-yl)-1-butyl-N,N-dimethylcyclohexanamin, Citrat (2:3)
(106) 4-(3-(2-(1H-Imidazol-1-yl)ethyl)-5-fluor-1H-indol-2-yl)-1-butyl-N,N-dimethylcyclohexanamin, Citrat
(107) 4-(3-(2-(1H-Imidazol-1-yl)ethyl)-5-fluor-1H-indol-2-yl)-N,N-dimethyl-1-phenylcyclohexanamin. Citrat
(108) 4-(3-(2-(1H-Imidazol-1-yl)ethyl)-5-fluor-1H-indol-2-yl)-N,N-dimethyl-1-phenylcyclohexanamin, Citrat (2:3)
(109) 4-(3-(2-(1H-Benzo[d]imidazol-1-yl)ethyl)-5-fluor-1H-indol-2-yl)-1-benzyl-N,N-dimethylcyclohexanamin, Citrat (4:3)
(110) 4-(3-(2-(1H-Benzo[d]imidazol-1-yl)ethyl)-5-fluor-1H-indol-2-yl)-1-benzyl-N,N-dimethylcyclohexanamin, Citrat (2:3)
(111) 4-(3-(2-(1H-Benzo[d]imidazo)-1-yl)ethyl)-5-fluor-1H-indol-2-yl)-1-butyl-N,N-dimethylcyclohexanamin, Citrat (2:3)
(112) 4-(3-(2-(1H-1,2,3-Triazol-1-yl)ethyl)-5-fluor-1H-indol-2-yl)-1-benzyl-N,N-dimethylcyclohexanamin, Citrat (4:1)
(113) 4-(3-(2-(1H-1,2,3-Triazol-1-yl)ethyl)-5-fluor-1H-indol-2-yl)-1-benzyl-N,N-dimethylcyclohexanamin, Citrat
(114) 4-(3-(2-(1H-1,2,3-Triazol-1-yl)ethyl)-5-fluor-1H-indol-2-yl)-1-butyl-N,N-dimethylcyclohexanamin, Citrat
(115) 4-(3-(2-(1H-1,2,3-Triazol-1-yl)ethyl)-5-fluor-1H-indol-2-yl)-butyl-N,N-dimethylcyclohexanamin, Citrat
(116) 4-(3-(2-(1H-1,2,3-Triazol-1-yl)ethyl)-5-fluor-1H-indo)-2-yl)-N,N-dimethyl-1-phenylcyclohexanamin, Citrat
(117) 4-(3-(2-(1H-1,2,3-Triazol-1-yl)ethyl)-5-fluor-1H-indol-2-yl)-N,N-dimethyl-1-phenylcyclohexanamin, Citrat
(118) 2-(4-(Dimethylamino)-4-phenylcyclohexyl)-3-methyl-1H-indol-5-ol
(119) 2-(4-(Dimethylamino)-4-phenylcyclohexyl)-3-methyl-1H-indol-5-ol
120) 1-Benzyl-N,N-dimethyl-4-(3-methyl-5-(trifluormethoxy)-1H-indol-2-yl)cyclohexanamin, Citrat
(121) 1-Butyl-N,N-dimethyl-4-(3-methyl-5-(trifluormethoxy)-1H-indol-2-yl)cyclohexanamin, Citrat
(122) 1-Butyl-N,N-dimethyl-4-(3-methyl-5-(trifluormethoxy)-1H-indol-2-yl)cyclohexanamin, Citrat
(123) N,N-Dimethyl-4-(3-methyl-5-(trifluormethoxy)-1H-indol-2-yl)-1-phenylcyclohexanamin, Citrat
(124) N,N-Dimethyl-4-(3-methyl-5-(trifluormethoxy)-1H-indol-2-yl)-1-phenylcyclohexanamin, Citrat
(125) 1-Butyl-4-(5-methoxy-3-methyl-1H-indol-2-yl)-N,N-dimethylcyclohexanamin, Citrat
(126) 1-Butyl-4-(5-methoxy-3-methyl-1H-indol-2-yl)-N,N-dimethylcyclohexanamin, Citrat
(127) 4-(5-Methoxy-3-methyl-1H-indol-2-yl)-N,N-dimethyl-1-phenylcyclohexanamin, Citrat
(128) 4-(5-Methoxy-3-methyl-1H-indol-2-yl)-N,N-dimethyl-1-phenylcyclohexanamin, Citrat (4:3)
(129) 1-Benzyl-N,N-dimethyl-4-(3-methyl-1H-indol-2-yl)cyclohexanamin. Citrat
(130) 1-Benzyl-N,N-dimethyl-4-(3-methyl-1H-indol-2-yl)cyclohexanamin, Citrat
(131) 1-Butyl-N,N-dimethyl-4-(3-methyl-1H-indol-2-yl)cyclohexanamin, Citrat
(132) 1-Butyl-N,N-dimethyl-4-(3-methyl-1H-indol-2-yl)cyclohexanamin, Citrat
(133) 1-Benzyl-4-(3-cyclopropyl-1H-indol-2-yl)-N,N-dimethylcyclohexanamin, Citrat
(134) 1-Benzyl-4-(3-cyclopropyl-1H-indol-2-yl)-N,N-dimethylcyclohexanamin, Citrat
(135) 1-Butyl-4-(3-cyclopropyl-1H-indol-2-yl)-N,N-dimethylcyclohexanamin Hydrochlorid
(136) 4-(3-Cyclopropyl-1H-indol-2-yl)-N,N-dimethyl-1-phenylcyclohexanamin, Citrat
(137) Methyl 2-(2-(4-(dimethylamino)-4-phenylcyclohexyl)-1H-indol-3-yl)acetate, Citrat

(138) Methyl 2-(2-(4-(dimethylamino)-4-phenylcyctohexyl)-1H-indol-3-yl)acetate. Citrat
(139) 1-Benzyl-4-(3-(cyclohexylmethyl)-1H-indol-2-yl)-N,N-dimethylcyclohexanamin, Citrat
(140) 1-Benzyl-4-(3-(cyclohexylmethyl)-1H-indol-2-yl)-N,N-dimethylcyclohexanamin, Citrat
(141) 1-Benzyl-4-(3-Benzyl-1H-indol-2-yl)-N,N-dimethylcyclohexanamin, Citrat
(142) 1-Benzyl-4-(3-Benzyl-1H-indol-2-yl)-N,N-dimethylcyclohexanamin, Citrat
(143) 1-Butyl-4-(3-(cyclohexylmethyl)-1H-indol-2-yl)-N,N-dimethylcyclohexanamin, Citrat
(144) 1-Butyl-4-(3-(cyclohexylmethyl)-1H-indol-2-yl)-N,N-dimethylcyclohexanamin Hydrochlorid
(145) 4-(3-Benzyl-1H-indol-2-yl)-1-butyl-N,N-dimethylcyclohexanamin, Citrat
(146) 4-(3-Benzyl-1H-indol-2-yl)-1-butyl-N,N-dimethylcyclohexanamin, Citrat
(147) 4-(3-(Cyclohexylmethyl)-1H-indol-2-yl)-N,N-dimethyl-1-phenylcyclohexanamin, Citrat
(148) 4-(3-(Cyclohexylmethyl)-1H-indol-2-yl)-N,N-dimethyl-1-phenylcyclohexanamin, Citrat
(149) 4-(3-Benzyl-1H-indol-2-yl)-N,N-dimethyl-1-phenylcyclohexanamin, Citrat
(150) 4-(3-Benzyl-1H-indol-2-yl)-N,N-dimethyl-1-phenylcyclohexanamin, Citrat
(151) N,N-Dimethyl-1-phenyl-4-(3-(pyridin-2-ylmethyl)-1H-indol-2-yl)cyclohexanamin
(152) N,N-Dimethyl-1-phenyl-4-(3-(pyridin-2-ylmethyl)-1H-indot-2-yl)cyclohexanamin
(153) 3-(2-(4-(Dimethylamino)-4-phenylcyclohexyl)-1H-indol-3-yl)propanoic acid Hydrochlorid
(156) 1-Benzyl-N,N-dimethyl-4-(3-propyl-1H-indol-2-yl)cyclohexanamin, Citrat
(157) 1-Butyl-N,N-dimethyl-4-(3-propyl-1H-indol-2-yl)cyclohexanamin Hydrochlorid
(158) 1-Butyl-N,N-dimethyl-4-(3-propyl-1H-indol-2-yl)cyclohexanamin, Citrat
(159) N,N-Dimethyl-1-phenyl-4-(3-propyl-1H-indol-2-yl)cyclohexanamin, Citrat
(160) N,N-Dimethyl-1-phenyl-4-(3-propyl-1H-indol-2-yl)cyclohexanamin, Citrat
(161) 1-Benzyl-N,N-dimethyl-4-(3-(2-(pyridin-4-yl)ethyl)-1H-indol-2-yl)cyclohexanamin, Citrat
(162) 1-Benzyl-N,N-dimethyl-4-(3-(2-(pyridin-4-yl)ethyl)-1H-indol-2-yl)cyclohexanamin, Citrat
(163) 1-Butyl-N,N-dimethyl-4-(3-(2-(pyridin-4-yl)ethyl)-1H-indol-2-yl)cyclohexanamin, Citrat
(164) 1-Butyl-N,N-dimethyl-4-(3-(2-(pyridin-4-yl)ethyl)-1H-indol-2-yl)cyclohexanamin, Citrat
(165) N,N-Dimethyl-1-phenyl-4-(3-(2-(pyridin-4-yl)ethyl)-1H-indol-2-yl)cyclohexanamin, Citrat (1:4)
(166) N,N-Dimethyl-1-phenyl-4-(3-(2-(pyridin-4-yl)ethyl)-1H-indol-2-yl)cyclohexanamin, Citrat
(167) N,N-Dimethyl-4-(3-(2-(1-methyl-1H-benzo[d]imidazol-2-yl)ethyl)-1H-indol-2-yl)-1-phenylcyclohexanamin, Citrat
(168) N,N-Dimethyl-4-(3-(2-(1-methyl-1H-benzo[d]imidazol-2-yl)ethyl)-1H-indol-2-yl)-1-phenylcyclohexanamin, Citrat
(169) N,N-Dimethyl-1-phenyl-4-(3-(2-(pyridin-2-yl)ethyl)-1H-indol-2-yl)cyclohexanamin, Citrat
(170) N,N-Dimethyl-1-phenyl-4-(3-(2-(pyridin-2-yl)ethyl)-1H-indot-2-yl)cyclohexanamin, Citrat (2:3)
(171) 4-(2-(4-Benzyl-4-(dimethylamino)cyclohexyl)-1H-indol-3-yl)butanoic acid Hydrochlorid
(172) 4-(2-(4-(Dimethylamino)-4-phenylcyclohexyl)-1H-indol-3-yl)butanoic acid Hydrochlorid
(173) 4-(2-(4-(Dimethylamino)-4-phenylcyclohexyl)-1H-indol-3-yl)butan-1-ol Hydrochlorid
(174) 4-(2-(4-Benzyl-4-(dimethylamino)cyclohexyl)-1H-indol-3-yl)butyl acetate Hydrochlorid
(175) 4-(2-(4-Benzyl-4-(dimethylamino)cyclohexyl)-1H-indol-3-yl)butyl acetate Hydrochlorid
(176) 4-(2-(4-Butyl-4-(dimethylamino)cyclohexyl)-1H-indol-3-yl)butyl acetate Hydrochlorid
(177) 4-(2-(4-Butyl-4-(dimethylamino)cyclohexyl)-1H-indol-3-yl)butyl acetate Hydrochlorid
(178) 4-(2-(4-(Dimethylamino)-4-phenylcyclohexyl)-1H-indol-3-yl)butyl acetate Hydrochlorid
(179) 4-(2-(4-(Dimethylamino)-4-phenylcyclohexyl)-1H-indol-3-yl)butyl acetate Hydrochlorid
(180) 3-(2-(4-Benzyl-4-(dimethylamino)cyclohexyl)-1H-indol-3-yl)propan-1-ol, Citrat
(181) 3-(2-(4-Butyl-4-(dimethylamino)cyclohexyl)-1H-indol-3-yl)propan-1-ol Hydrochlorid
(182) 3-(2-(4-Butyl-4-(dimethylamino)cyclohexyl)-1H-indol-3-yl)propan-1-ol Hydrochlorid
(183) 3-(2-(4-(Dimethylamino)-4-phenylcyclohexyl)-1H-indol-3-yl)propan-1-ol Hydrochlorid
(184) 3-(2-(4-(Dimethylamino)-4-phenylcyclohexyl)-1H-indol-3-yl)propan-1-ol Hydrochlorid
(185) 3-(2-(4-(Dimethylamino)-4-phenylcyclohexyl)-1H-indol-3-yl)propyl acetate Hydrochlorid
(186) 3-(2-(4-(Dimethylamino)-4-phenylcyclohexyl)-1H-indol-3-yl)propyl acetate Hydrochlorid
(187) 1-(2-(2-(4-(Dimethylamino)-4-phenylcyclohexyt)-1H-indol-3-yl)ethyl)pyrrolidine-2,5-dione
(188) 1-(2-(2-(4-(Dimethylamino)-4-phenylcyclohexyl)-1H-indol-3-yl)ethyl)pyrrolidine-2,5-dione
(189) 4-(3-(2-(3,4-Dihydrochinolin-1(2H)-yl)ethyl)-1H-indol-2-yl)-N,N-dimethyl-1-phenylcyclohexanamin, Citrat (4:3)
(190) 4-(3-(2-(3,4-Dihydrochinolin-1(2H)-yl)ethyl)-1H-indol-2-yl)-N,N-dimethyl-1-phenylcyclohexanamin, Citrat
(191) Methyl 1-(2-(2-(4-(dimethylamino)-4-phenylcyclohexyl)-1H-indol-3-yl)ethyl)-1H-1,2,3-triazole-4-carboxylat, Citrat
(192) Methyl 1-(2-(2-(4-(dimethylamino)-4-phenylcyclohexyl)-1H-indol-3-yl)ethyl)-1H-1,2,3-triazole-4-carboxylate, Citrat
(193) 4-(3-(2-(Isoindolin-2-yl)ethyl)-1H-indol-2-yl)-N,N-dimethyl-1-phenylcyclohexanamin, Citrat

(194) 4-(3-(2-(Isoindolin-2-yl)ethyl)-1H-indol-2-yl)-N,N-dimethyl-1-phenylcyclohexanamin. Citrat
(195) 4-(3-(2-(3,4-Dihydroisochinolin-2(1H)-yl)ethyl)-1H-indol-2-yl)-N,N-dimethyl-1-phenylcyclohexanamin, Citrat
(196) 4-(3-(2-(3,4-Dihydroisochinolin-2(1H)-yl)ethyl)-1H-indol-2-yl)-N,N-dimethyl-1-phenylcyclohexanamin, Citrat (2:3)
(197) N,N-Dimethyl-1-phenyl-4-(3-(2-(pyrrolidin-1-yl)ethyl)-1H-indol-2-yl)cyclohexanamin, Citrat
(198) N,N-Dimethyl-l-phenyl-4-(3-(2-(pyrrolidin-1-yl)ethyl)-1H-indol-2-yl)cyclohexanamin, Citrat
(199) N,N-Dimethyl-1-phenyl-4-(3-(2-(piperidin-1-yl)ethyl)-1H-indol-2-yl)cyclohexanamin, Citrat
(200) N,N-Dimethyl-4-(3-(2-(4-methylpiperazin-1-yl)ethyl)-1H-indol-2-yl)-1-phenylcyclohexanamin, Citrat
(201) N,N-Dimethyl-4-(3-(2-morpholinoethyl)-1H-indol-2-yl)-1-phenylcyclohexanamin, Citrat
(202) N,N-Dimethyl-4-(3-(2-morpholinoethyl)-1H-indol-2-yl)-1-phenylcyclohexanamin, Citrat
(203) 4-(3-(2-(1H-Benzo[d]imidazol-1-yl)ethyl)-1H-indol-2-yl)-N,N-dimethyl-1-phenylcyclohexanamin, Citrat
(204) 4-(3-(2-(1H-Benzo[d]imidazol-1-yl)ethyl)-1H-indol-2-yl)-N,N-dimethyl-1-phenylcyclohexanamin, Citrat
(205) 4-(3-(2-(1H-Imidazol-1-yl)ethyl)-1H-indol-2-yl)-N,N-dimethyl-1-phenylcyclohexanamin, Citrat
(206) 4-(3-(2-(1H-Imidazol-1-yl)ethyl)-1H-indol-2-yl)-N,N-dimethyl-1-phenylcyclohexanamin, Citrat
(207) 4-(3-(2-(1H-1,2,4-Triazol-1-yl)ethyl)-1H-indol-2-yl)-N,N-dimethyl-1-phenylcyclohexanamin, Citrat
(208) 4-(3-(2-(1H-1,2,4-Triazol-1-yl)ethyl)-1H-indol-2-yl)-N,N-dimethyl-1-phenylcyclohexanamin, Citrat
(209) N,N-Dimethyl-1-phenyl-4-(3-(2-(thiazolidin-3-yl)ethyl)-1H-indol-2-yl)cyclohexanamin, Citrat
(210) N,N-Dimethyl-1-phenyl-4-(3-(2-(thiazolidin-3-yl)ethyl)-1H-indot-2-yl)cyclohexanamin, Citrat
(211) N,N-Dimethyl-4-(3-(2-(5-methyl-2H-tetrazol-2-yl)ethyl)-1H-indol-2-yl)-1-phenylcyclohexanamin, Citrat
(212) N,N-Dimethyl-4-(3-(2-(5-methyl-2H-tetrazol-2-yl)ethyl)-1H-indol-2-yl)-1-phenylcyclohexanamin, Citrat
(213) 4-(3-(2-(1H-pyrazol-1-yl)ethyl)-1H-indol-2-yl)-N,N-dimethyl-1-phenylcyclohexanamin, Citrat
(214) 4-(3-(2-(1H-Pyrazol-1-yl)ethyl)-1H-indol-2-yl)-N,N-dimethyl-1-phenylcyclohexanamin, Citrat
(215) 4-(3-(2-(1H-1,2,3-Triazol-1-yl)ethyl)-1H-indol-2-yl)-N,N-dimethyl-1-phenylcyclohexanamin, Citrat
(216) 4-(3-(2-(1H-1,2,3-Triazol-1-yl)ethyl)-1H-indol-2-yl)-N,N-dimethyl-1-phenylcyclohexanamin, Citrat
(217) N,N-Dimethyl-4-(3-(2-(5-methyl-1H-tetrazol-1-yl)ethyl)-1H-indol-2-yl)-1-phenylcyclohexanamin, Citrat
(218) N,N-Dimethyl-4-(3-(2-(5-methyl-1H-tetrazol-1-yl)ethyl)-1H-indol-2-yl)-1-phenylcyclohexanamin, Citrat
(219) 2-(2-(4-(Dimethylamino)-4-phenylcyclohexyl)-1H-indol-3-yl)ethanol
(220) 2-(2-(4-(Dimethylamino)-4-phenylcyclohexyl)-1H-indol-3-yl)ethyl acetate
(221) N,N-Dimethyl-4-(3-methyl-1H-pyrrolo[3,2-c]pyridin-2-yl)-1-phenylcyclohexanamin, Citrat
(222) N,N-Dimethyl-4-(3-methyl-1H-pyrrolo[3,2-c]pyridin-2-yl)-1-phenylcyclohexanamin, Citrat
(223) N,N-Dimethyl-4-(3-methyl-1H-pyrrolo[2,3-b]pyridin-2-yl)-1-phenylcyclohexanamin, Citrat
(224) N,N-Dimethyl-4-(3-methyl-1H-pyrrolo[2,3-b]pyridin-2-yl)-1-phenylcyclohexanamin, Citrat
(225) N,N-Dimethyl-4-(3-methylbenzofuran-2-yl)-1-phenylcyclohexanamin, Citrat
(226) N,N-Dimethyl-4-(3-methylbenzofuran-2-yl)-1-phenylcyclohexanamin, Citrat
(227) 4-(1H-Indol-3-yl)-N,N-dimethyl-1-phenylcyclohexanamin, Citrat
(228) (±)-3-(4-(Dimethylamino)-4-benzylcyclohex-1-enyl)-1H-pyrrolo[2,3-b]pyridin
(229) (±)-3-(4-(Dimethylamino)-4-butylcyclohex-1-enyl)-1H-pyrrolo[2,3-b]pyridin
(230) (±)-3-(4-(Dimethylamino)-4-phenylcyclohex-1-enyl)-1H-pyrrolo[2,3-b]pyridin, Citrat
(231) (±)-4-(1H-Indol-3-yl)-N,N-dimethyl-1-phenylcyclohex-3-enamin, Citrat
(232) (±)-4-(1H-Indot-3-yl)-N,N-dimethyl-1-phenylcyclohex-3-enamin
(233) (±)-2-(3-(4-(Dimethylamino)-4-phenylcyclohex-1-enyl)benzo[b]thiophen-2-yl)ethanol Hydrochlorid
(234) (±)-2-(3-(4-(Dimethylamino)-4-(pyridin-2-yl)cyclohex-1-enyl)benzo[b]thiophen-2-yl)ethanol Hydrochlorid
(235) 4-(1H-Indol-2-yl)-N,N-dimethyl-1-phenylcyclohexanamin, Citrat
(236) 4-(1H-Indol-2-yl)-N,N-dimethyl-1-phenylcyclohexanamin, Citrat
(237) 1-Benzyl-N,N-dimethyl-4-(1H-pyrrolo[2,3-b]pyridin-3-yl)cyclohexanamin Hydrochlorid
(238) 1-Butyl-N,N-dimethyl-4-(1H-pyrrolo[2,3-b]pyridin-3-yl)cyclohexanamin Hydrochlorid
(239) (±)-4-(Benzofuran-2-yl)-1-benzyl-N,N-dimethylcyclohex-3-enamin Hydrochlorid
(240) (±)-N,N-Dimethyl-4-(3-methylbenzofuran-2-yl)-1-phenylcyclohex-3-enamin, Citrat
(241) (±)-2-(2-(4-(Dimethylamino)-4-phenylcyclohex-1-enyl)benzofuran-3-yl)ethanethiol, Citrat
(242) (±)-N,N-Dimethyl-4-(3-methylbenzo[b]thiophen-2-yl)-1-phenylcyclohex-3-enamin, Citrat
(243) N,N-Dimethyl-4-(3-methylbenzo[b]thiophen-2-yl)-1-phenylcyclohexanamin, Citrat
(244) N,N-Dimethyl-1-phenyl-4-(1H-pyrrolo[2,3-b]pyridin-3-yl)cyclohexanamin, Citrat
(246) 1-(Dimethylamino)-3-(2-(4-(Dimethylamino)-4-phenylcyclohexyl)-3-methyl-1H-indol-1-yl)propan-2-ol
(247) N,N-Dimethyl-4-(3-methyl-1-(oxiran-2-ylmethyl)-1H-indol-2-yl)-1-phenylcyclohexanamin
(248) 4-(1,3-Dimethyl-1H-indol-2-yl)-N,N-dimethyl-1-phenylcyclohexanamin
(249) 2-(4-Butyl-4-(dimethylamino)cyclohexyl)-3-methyl-1H-indol-5-ol, Citrat
(250) 2-(4-Butyl-4-(dimethylamino)cyclohexyl)-3-methyl-1H-indol-5-ol, Citrat
(251) (±)-2-(4-(Dimethylamino)-4-(thiophen-2-yl)-cyclohex-1-enyl)-3-(2-(pyridin-4-yl)ethyl)-1H-indol

(252) (±)-2-(4-(Dimethylamino)-4-(3-fluorphenyl)-cyclohex-1-enyl)-3-(2-(pyridin-4-yl)ethyl)-1H-indol
(253) (±)-2-(4-Butyl-4-(pyrrolidin-1-yl)cyclohex-1-enyl)-3-(2-(pyridin-4-yl)ethyl)-1H-indol
(254) (±)-2-(4-(Methylamino)-4-phenylcyclohex-1-enyl)-3-(2-(pyridin-4-yl)ethyl)-1H-indol
(255) (±)-2-(4-(Dimethylamino)-4-(thiophen-2-yl)-cyclohex-1-enyl)-3-methyl-1H-indol, Citrat
(256) N,N-Dimethyl-4-(3-methyl-1H-indol-2-yl)-1-(thiophen-2-yl)cyclohexanamin, Citrat
(257) N,N-Dimethyl-4-(3-methyl-1H-indol-2-yl)-1-(thiophen-2-yl)cyclohexanamin, Citrat
(258) 2-(4-Butyl-4-(pyrrolidin-1-yl)cyclohex-1-enyl)-3-methyl-1H-indol
(259) N-Methyl-4-(3-methyl-1H-indol-2-yl)-1-phenylcyclohexanaminhydrobromid
(260) N-Methyl-4-(3-methyl-1H-indol-2-yl)-1-phenylcyclohexanamin, Citrat
(261) 1-(3-Fluorphenyl)-N,N-dimethyl-4-(3-methyl-1H-indol-2-yl)cyclohexanamin, Citrat
(262) 1-(3-Fluorphenyl)-N,N-dimethyl-4-(3-methyl-1H-indol-2-yl)cyclohexanamin, Citrat
(263) (±)-2-(4-(Dimethylamino)-4-(3-fluorphenyl)-cyclohex-1-enyl)-3-methyl-1H-indol
(264) 2-(4-Benzyl-4-(dimethylamino)cyclohexyl)-3-methyl-1H-indol-5-ol, Citrat
(265) 2-(4-Benzyl-4-(dimethylamino)cyclohexyl)-3-methyl-1H-indol-5-ol, Citrat
(266) 2-(4-Butyl-4-(pyrrolidin-1-yl)cyclohexyl)-3-(2-(pyridin-4-yl)ethyl)-1H-indol, Citrat
(267) 2-(4-Butyl-4-(pyrrolidin-1-yl)cyclohexyl)-3-(2-(pyridin-4-yl)ethyl)-1H-indol, Citrat
(268) 2-(4-(Azetidin-1-yl)-4-phenylcyclohexyl)-3-methyl-1H-indol, Citrat
(269) 2-(4-(Azetidin-1-yl)-4-phenylcyclohexyl)-3-methyl-1H-indol, Citrat
(270) 2-(4-(Azetidin-1-yl)-4-phenylcyclohexyl)-3-(2-(pyridin-4-yl)ethyl)-1H-indol Hydrochlorid
(271) 2-(4-(Azetidin-1-yl)-4-phenylcyclohexyl)-3-(2-(pyridin-4-yl)ethyl)-1H-indol Hydrochlorid
(272) 2-(4-Butyl-4-(pyrrolidin-1-yl)cyclohexyl)-3-methyl-1H-indol, Citrat
(273) 3-(2-(4-(Dimethylamino)-4-phenylcyclohexyl)-1H-pyrrolo[2,3-b]pyridin-3-yl)propan-1-ol, Citrat
(274) 1-(2-(4-(Dimethylamino)-4-phenylcyclohexyl)-3-methyl-1H-indol-1-yl)-3-(methylamino)propan-2-ol, Citrat
(275) 1-Benzyl-3-(2-(2-(4-(dimethylamino)-4-phenylcyclohexyl)-1H-indol-3-yl)ethyl)harnstoff, Citrat
(276) 1-Benzyl-3-(2-(2-(4-(dimethylamino)-4-phenylcyclohexyl)-1H-indol-3-yl)ethyl)harnstoff, Citrat
(277) 1-(2-(2-(4-(Dimethylamino)-4-phenylcyclohexyl)-1H-indol-3-yl)ethyl)-3-phenylhamstoff, Citrat
(278) 1-(2-(2-(4-(Dimethylamino)-4-phenylcyclohexyl)-1H-indol-3-yl)ethyl)-3-phenylharnstoff, Citrat
(279) 1-Cyclopentyl-3-(2-(2-(4-(dimethylamino)-4-phenylcyclohexyl)-1H-indol-3-yl)ethyl)hamstoff, Citrat
(280) 1-Cyclopentyl-3-(2-(2-(4-(dimethylamino)-4-phenylcyclohexyl)-1H-indol-3-yl)ethyl)harnstoff, Citrat
(281) N-(2-(2-(4-(Dimethylamino)-4-phenylcyclohexyl)-1H-indol-3-yl)ethyl) cyclopentansulfonamid, Citrat
(282) N-(2-(2-(4-(Dimethylamino)-4-phenylcyclohexyl)-1H-indol-3-yl)ethyl)cyclopentanesulfonamid, Citrat
(283) N-(2-(2-(4-(Dimethylamino)-4-phenylcyclohexyl)-1H-indol-3-yl)ethyl)benzenesulfonamid, Citrat
(284) N-(2-(2-(4-(Dimethylamino)-4-phenylcyclohexyl)-1H-indol-3-yl)ethyl)thiophen-2-sulfonamid, Citrat
(285) N-(2-(2-(4-(Dimethylamino)-4-phenylcyclohexyl)-1H-indol-3-yl)ethyl)thiophen-2-sulfonamid, Citrat
(286) N-(2-(2-(4-(Dimethylamino)-4-phenylcyclohexyl)-1H-indol-3-yl)ethyl)nicotinamid, Citrat
(287) N-(2-(2-(4-(Dimethylamino)-4-phenylcyclohexyl)-1H-indol-3-yl)ethyl)nicotinamid, Citrat
(288) N-(2-(2-(4-(Dimethylamino)-4-phenylcyclohexyl)-1H-indol-3-yl)ethyl)benzamid, Citrat
(289) 1-(3-Fluorphenyl)-N,N-dimethyl-4-(3-(2-(pyridin-4-yl)ethyl)-1H-indol-2-yl)cyclohexanamin
(290) 1-(3-Fluorphenyl)-N,N-dimethyl-4-(3-(2-(pyridin-4-yl)ethyl)-1H-indol-2-yl)cyclohexanamin, Citrat
(291) N-Methyl-1-phenyl-4-(3-(2-(pyridin-4-yl)ethyl)-1H-indol-2-yl)cyclohexanamin, Citrat
(292) N-Methyl-1-phenyl-4-(3-(2-(pyridin-4-yl)ethyl)-1H-indol-2-yl)cyclohexanamin, Citrat
(293) 2-(4-Butyl-4-(pyrrolidin-1-y1)cyclohexyl)-3-methyl-1H-indol, Citrat
(294) N,N-Dimethyl-1-phenyl-4-(1-(phenylsulfonyl)-1H-indol-2-yl)cyclohexanamin
(295) N,N-Dimethyl-1-phenyl-4-(1-(phenylsulfonyl)-1H-indol-2-yl)cyclohexanamin
(296) N,N-Dimethyl-4-(3-methyl-1-(oxiran-2-ylmethyl)-1H-indol-2-yl)-1-phenylcyclohex-3-enamin
(297) N,N-Dimethyl-4-(3-methyl-1-(oxiran-2-ylmethyl)-1H-indol-2-yl)-1-phenylcyclohexanamin, Citrat
(298) 1-(Dimethylamino)-3-(2-(4-(Dimethylamino)-4-phenylcyclohex-1-enyl)-3-methyl-1H-indol-1-yl)propan-2-ol, Citrat
(299) 1-(Dimethylamino)-3-(2-(4-(Dimethylamino)-4-phenylcyclohexyl)-3-methyl-1H-indol-1-yl)propan-2-ol, Citrat
(300) 2-(3-(4-(Dimethylamino)-4-phenylcyclohex-1-enyl)-1H-indol-1-yl)ethanol Hydrochlorid
(301) (±) 3-(4-(Dimethylamino)-4-phenylcyclohex-1-enyl)-(1-(phenylsulfonyl)-1H-indol) Hydrochlorid
(302) 1-Benzyl-N,N-dimethyl-4-(1-methyl-1H-indol-2-yl)cyclohex-3-enamin; Hydrochlorid
(303) N,N-dimethyl-4-(3-(2-(pyridin-4-yl)ethyl)-1H-indol-2-yl)-1-(thiophen-2-yl)cyclohexanamin Hydrochlorid
(304) N,N-dimethyl-4-(3-(2-(pyridin-4-yl)ethyl)-1H-indol-2-yl)-1-(thiophen-2-yl)cyclohexanamin Hydrochlorid
(305) N-Methyl-4-(3-methyl-1H-indol-2-yl)-1-(4-methylthiazol-2-yl)cyclohexanamin
(306) N-Methyl-4-(3-methyl-1H-indol-2-yl)-1-(4-methylthiazol-2-yl)cyclohexanamin
(307) 2-(2-(4-(Dimethylamino)-4-phenylcyclohexyl)-1H-indol-3-yl)ethanol in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die erfindungsgemäßen Substanzen wirken beispielsweise auf den im Zusammenhang mit verschiedenen Erkrankungen relevanten μ-Opioid-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes substituiertes Heteroaryl-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Heteroaryl-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln. Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Heteroaryl-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierte Heteroaryl-Derivate verzögert freisetzen. Die erfindungsgemäßen substituierten Heteroaryl-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Heteroaryl-Derivats appliziert.

Der ORL-1-Rezeptor und der μ-Opioid-Rezeptor wurden insbesondere im Schmerzgeschehen identifiziert. Entsprechend können erfindungsgemäße substituierte Heteroaryl-Derivate zur Herstellung eines Arzneimittels zur Behandlung von Schmerz, insbesondere von akutem, neuropathischem, chronischem Schmerz oder Entzündungsschmerz, verwendet werden.

Ein weiterer Gegenstand der Erfindung ist daher die Verwendung eines erfindungsgemäßen substituierten Heteroaryl-Derivats zur Herstellung eines Arzneimittels zur Behandlung von Schmerz, insbesondere von akutem, viszeralem, neuropathischem, chronischem Schmerz oder Entzündungsschmerz.

Ein weiterer Gegenstand der Erfindung ist die Verwendung eines erfindungsgemäßen substituierten Heteroaryl-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinnitus, Pruritus, Migräne, Schwerhörigkeit, mangelnder Darmmotilität, gestörter Nahrungsaufnahme. Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes substituiertes Heteroaryl-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis eines erfindungsgemäßen substituierten Heteroaryl-Derivats, oder eines erfindungsgemäßen Arzneimittels.

Ein weiterer Gegenstand der Erfindung sind Verfahren zur Herstellung der erfindungsgemäßen substituierten Heteroaryl-Derivate wie in der folgenden Beschreibung und Beispielen ausgeführt.

## Verfahren 1

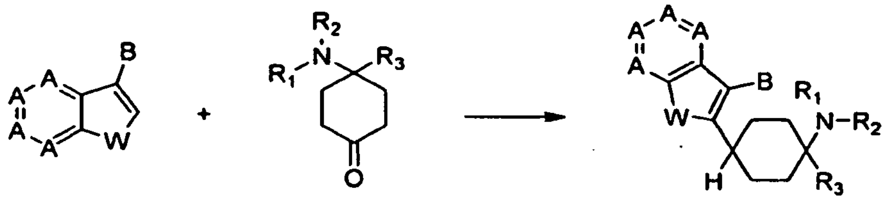

A B Ic

Zur Herstellung von Aminen der allgemeinen Formel **Ic** werden Ketone **B** mit Heteroaromaten **A** in organischen Lösungsmitteln oder Lösungsmittelgemischen, beispielsweise Ethylacetat, Chloroform, Dichlormethan (DCM), Dichlorethan (DCE), Diethylether ($Et_2O$), Acetonitril (MeCN) oder Nitromethan unter Zusatz einer organischen oder anorganischen Säure, beispielsweise HCl, HBr, Trifluormethansulfonsäure, Methansulfonsäure, Essigsäure, Trifluoressigsäure oder lösungsmittelfrei in einer organischen oder anorganischen Säure oder Säuregemischen bei Temperaturen zwischen 0°C und 150°C ggf. unter Verwendung von Mikrowelleneinstrahlung umgesetzt. Anschließend wird ein organisches oder anorganisches Reduktionsmittel z.B. Triethylsilan od. Zinn-Pulver gegeben und bei Temperaturen zwischen 0°C und 150°C ggf. unter Verwendung von Mikrowelleneinstrahlung reagiert.

## Verfahren 2

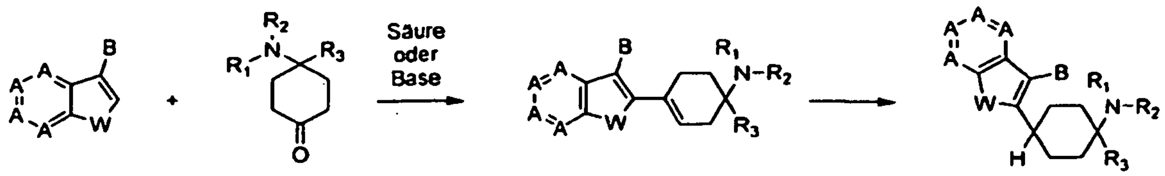


A B Id Ic

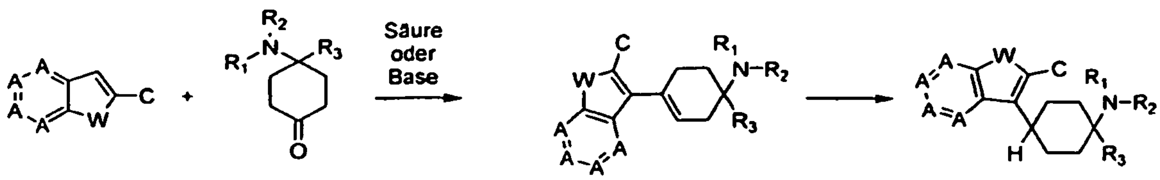


A' B Ie If

Die Umsetzung von Heteroaromaten der allgemeinen Formel **A** oder **A'** mit Cyclohexanonen der allgemeinen Formel **B** zu Cyclohexen-substituierten Hetereoaromaten der allgemeinen Formel **Id** oder **Ie** kann in organischen Lösungsmitteln oder Lösungsmittelgemischen, beispielsweise Chloroform, Dichlormethan (DCM), Dichlorethan (DCE), Diethylether ($Et_2O$), Acetonitril (MeCN) oder Nitromethan unter Zusatz einer organischen oder anorganischen Säure, beispielsweise HCl, HBr, Trifluormethansulfonsäure, Methansulfonsäure, Essigsäure, Trifluoressigsäure bei Temperaturen zwischen 0°C und 150°C ggf. unter Verwendung von Mikrowelleneinstrahlung erfolgen. Die Umsetzung kann auch z.B. unter Zusatz Trifluormethansulfonsäure-trimethylsilylester erfolgen. Bevorzugt erfolgt die Umsetzung unter Zusatz von Brönsted-Säuren.

Alternativ können Verbindungen der allgemeinen Formeln Id oder Ie auch unter basischen Bedingungen erhalten werden. Dabei wird eine Base, beispielsweise KOH oder NaOH, in einem organischen Lösungsmittel, beispielsweise Methanol, gelöst. In diesem Medium werden der Heteroaromat der allgemeinen Formel A/A' und das Keton der allgemeinen Formel B bei Temperaturen zwischen 20 und 100°C umgesetzt. Das Produkt wird ggf. durch Säulenchromatographie gereinigt.

Die Doppelbindung kann mittels Wasserstoff in Form von HBr/Eisessig//Sn oder HCl/Sn (nascierender Wasserstoff) oder $H_2$ in Gegenwart eines Metallkatalysators wie z.B. Palladium auf Kohle, Platin auf Kohle, Platinoxid, Raney-Nickel, Rhodium oder Rutheniumkomplexen in einem geeigneten Lösungsmittel oder Lösungsmittelgemisch wie beispielsweise Methanol (MeOH), Ethanol (EtOH), Aceton, Essigsäureethylester (AcOEt), HBr oder Essigsäure (AcOH) bei Temperaturen zwischen 0°C und 150°C reduziert werden. Dabei entstehen die Verbindungen der allgemeinen Formeln **Ic** und **If.** Wasserstoff kann auch aus Wasserstoffüberträgern wie z.B. Cyclohexen in situ generiert werden.

**Verfahren 3**

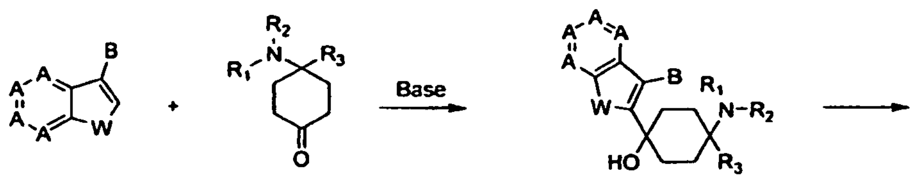


A B E

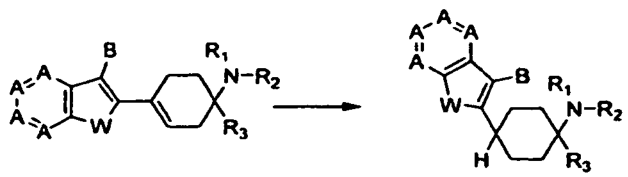

Id Ic

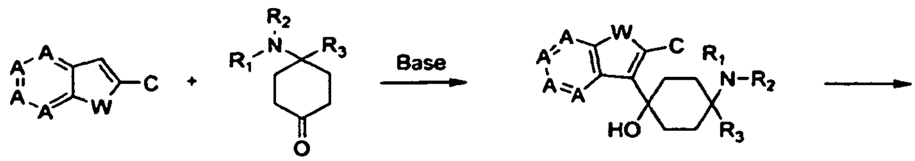


A' B E'

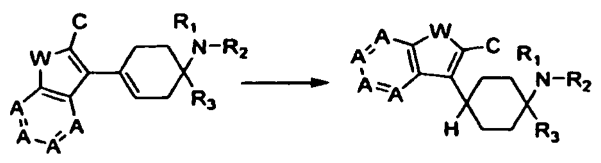

Ie If

Die Umsetzung von **A/A'** + **B** nach **E/E'** kann unter Einsatz geeigneter basischer Reagenzien, z.B. Organollithiumverbindungen, ggf. unter Zusatz von sekundären Aminen oder Hexaalkyldisilazanen, in aprotischen Lösungsmitteln, z.B. Butyllithium in Hexanen/THF, LDA in Hexan/THF oder Heptan/THF/Ethylbenzol oder LiHMDS in Hexanen/THF bei Temperaturen von -100 °C bis 50 °C, vorzugsweise bei -78 °C bis 0°C erfolgen. Im Falle von Indolen wird der Indol-Stickstoff zuvor mit geeigenten, dem Fachmann bekannten Schutzgruppen versehen, z.B. der Benzolsulfonylgruppe, subst. oder unsubst. Benzylgruppen oder Alkoxymethylgruppen oder substituierten Oxycarbonylgruppen.

Die Umsetzung von **E** nach **Id** oder **Ie** kann unter sauren oder wasserentziehenden Bedingungen oder nach Überführung der Hydroxygruppe in eine Abgangsgruppe erfolgen. Z.B. können dabei Mineralsäuren, z.B. HCl oder

$H_2SO_4$ oder wasserentziehendes $P_4O_{10}$ zum Einsatz kommen oder die Hydroxygruppe mittels $SOCl_2$ / Pyridin in situ in das Chlorid übergeführt werden. Die Doppelbindung kann gegebenenfalls unter den oben beschriebenen Bedingungen reduziert werden.

**Synthese über Larock Reaktion:**

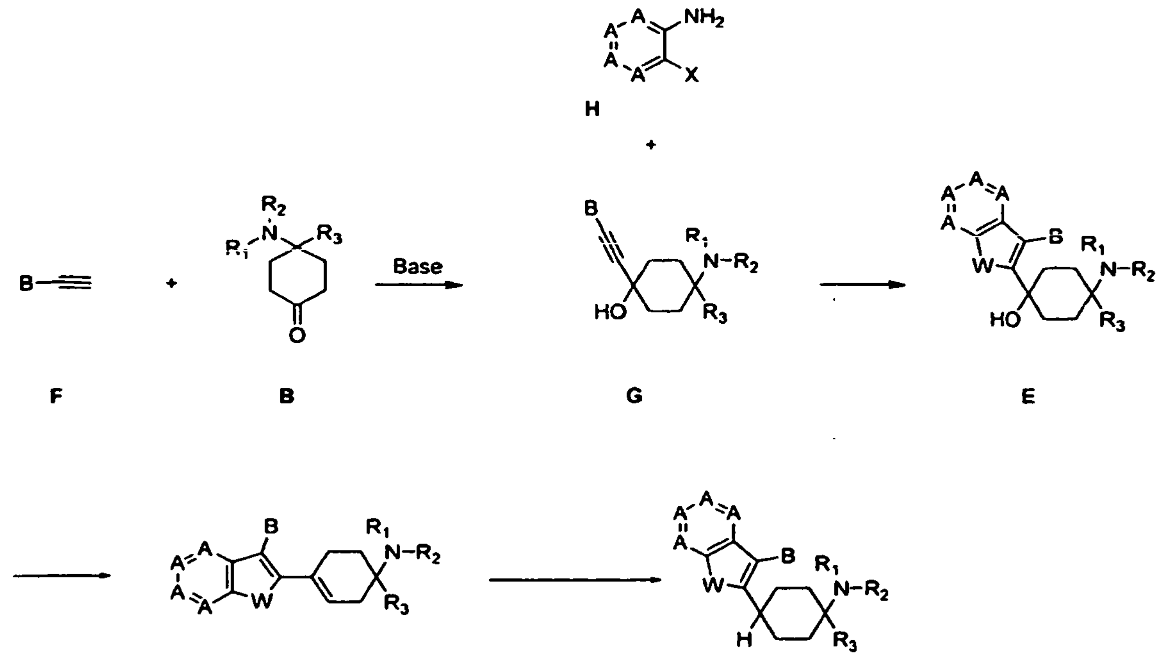


H

F B G E

Id Ic

Die Umsetzung von F + B nach G kann unter Einsatz geeigenter basischer Reagenzien, z.B. Organollithiumverbindungen, ggf. unter Zusatz von sekundären Aminen oder Hexaalkyldisilazanen, in aprotischen Lösungsmitteln, z.B. Butyllithium in Hexanen/THF, LDA in Hexan/THF oder Heptan/THF/Ethylbenzol oder LiHMDS in Hexanen/THF bei Temperaturen von -100 °C bis 50 °C, vorzugsweise bei -78 °C bis 0°C erfolgen. Weitere geeignete Bedingungen sind z. B. Grignardreagentien in aprotischen Lösungsmitteln, z.B. Ethylmagnesiumbromid in THF oder fein gepulvertes Alkalihydroxid, vorzugsweise KOH, in trockenem Ether, mit oder ohne Eiskühlung; Alkalialkoholate, z.B. MeOK EtOK in THF bei 0°C, KOtBu in THF bei -10 °C

In Stufe 1 werden Verbindungen der vorstehend angegebenen allgemeinen Formel **H,** worin X für einen Halogen-Rest oder einen Sulfonsäureester, besonders bevorzugt für Iod, Brom oder Trifluormethansulfonat, steht, im Sinne einer Indolsynthese nach Larock mit Alkinen der allgemeinen Formel **G** in einem Reaktionsmedium, vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen, vorzugsweise unter Zusatz wenigstens eines Palladiumkatalysators, vorzugsweise ausgewählt aus der Gruppe bestehend aus Palladium(II)-dichlorid [$PdCl_2$], Bis(triphenylphosphin)-palladium(II)-acetat [$Pd(PPh_3)_2(OAc)_2$], Bis(triphenylphosphin)-palladium(II)-chlorid [$PdCl_2(PPh_3)_2$], Palladium(II)-acetat [$Pd(OAc)_2$; Ac = Acetat], Bis(acetonitril)-palladium(II)-chlorid [$(CH_3CN)_2)PdCl_2$], Bis(benzonitril)-palladium(II)-chlorid [$(PhCN)_2PdCl_2$] und Tetrakis(triphenylphosphin)palladium [$(PPh_3)_4Pd$], besonders bevorzugt ausgewählt aus der Gruppe bestehend aus $Pd(PPh_3)_2(OAc)_2$, $(PPh_3)_4Pd$ und $PdCl_2(PPh_3)_2$, ggf. in Gegenwart wenigstens eines Phosphins, vorzugsweise eines Phosphins ausgewählt aus der Gruppe bestehend aus Triphenylphosphin, Tri-(tert-butyl)-phosphin, Triphenylarsin und Tri-(ortho-toluyl)-phosphin, besonders bevorzugt in Gegenwart von Triphenylphosphin, ggf. unter Zusatz wenigstens eines anorganischen Salzes, bevorzugt unter Zusatz von Lithiumchlorid oder Tetrabutylammoniumchlorid, ggf. unter Zusatz wenigstens einer anorganischen Base, vorzugsweise ausgewählt aus der Gruppe bestehend aus Kaliumcarbonat, Natriumcarbonat, Kaliumacetat, Natriumhydrogencarbonat und Cäsiumcarbonat, und/oder Zusatz wenigstens einer organischen Base ausgewählt aus der Gruppe bestehend aus Triethylamin, Diisopropylamin, Diisopropylethylamin und [1,4]-Diazabicyclo-[2.2.2]octan bei Temperaturen von vorzugsweise -70 °C bis 300 °C, besonders bevorzugt von -70°C bis 150°C, zu Verbindungen der allgemeinen Formel E und/oder Ihren Regioisomeren umgesetzt.

Verbindungen der allgemeinen Formel H sind kommerziell erhältlich oder aus der Literatur bekannt. Exemplarisch sind Synthesen zu Verbindungen der allgemeinen Formel A im Beispielteil beschrieben.

In Stufe 2 werden für den Fall dass Rest **B** in Vebindung **G** einer Silylschutzgruppe entspricht die Verbindungen der allgemeinen Formel **E** in einem Reaktionsmedium vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Acetonitril, Tetrahydrofuran, Methanol, Ethanol, Ethylacetat, Pyridin, Wasser und entsprechenden Mischungen, in Gegenwart von Fluorid, ausgewählt aus der Gruppe bestehend aus Tetra-n-butylammoniumfluorid, Flusssäure (HF, HF-Pyridin), Kalium- und/oder Natriumfluorid, Cäsiumfluorid oder in Gegenwart einer organischen oder anorganischen Säure, vorzugsweise HCl, Essigsäure, Trifluoressigsäure, Bortrifluorid bei Temperaturen von vorzugsweise -70 °C bis 300 °C. besonders bevorzugt von -70°C bis 150°C, zu Verbindungen der allgemeinen Formel **E** mit B = H umgesetzt.

Die Umsetzung von **E** nach **Id** oder **Ic** kann unter den oben angegebenen Bedingungen erfolgen.

Die Synthesen der Cyclohexanonderivate mit der allgemeinen Formel **B** sind in der Literatur bekannt (WO04043967, WO0290317, US 4065573, Lednicer et al., J.Med.Chem., 23, 1980, 424-430). Derivate der allgemeinen Formeln **A** oder **A'** sind kommerziell erhältlich oder in der Literatur beschrieben.

**Beispiele**

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

In Fällen, in denen Isomere angefallen sind, wird das Isomer als "unpolares Isomer" bezeichnet, das im Vergleich zum "polaren Isomer" auf Kieselgel-Dünnschichtplatten (Normalphase) weiter läuft. Als Laufmittel wird z. B. DCM/Methanol 9:1, ggf. unter Zugabe von 5% Triethylamin, verwendet.

Alle Temperaturen sind unkorrigiert.

**Abkürzungen:**

| | |
|---|---|
| d | Tage |
| DCM | Dichlormethan |
| DMF | N,N-Dimethylformamid |
| Ether | Diethylether |
| EtOAc | Essigsäureethylester |
| $H_2O$ | Wasser |
| MeOH | Methanol |
| $NEt_3$ | Triethylamin |
| RT | Raumtemperatur |
| TBAF | Tetrabutylammoniumfluorid |
| THF | Tetrahydrofuran |
| TMEDA | N,N,N',N'-Tetramethylethylendiamin |

**<u>Ketonbausteine</u>**

**<u>Vorstufen:</u>**

**8-Dimethylamino-1,4-dioxa-spiro[4.5]decan-8-carbonitril**

Zu einem Gemisch aus 4 N Salzsäure (50 ml) und Methanol (30 ml) wurde unter Eiskühlung 40-proz. wässrige Dimethylamin-Lösung (116 ml, 0.92 mol), Cyclohexan-1,4-dion-monoethylenketal (30.0 g, 0.192 mol) und Kaliumcyanid (30.0 g, 0.46 mol) hinzugefügt. Die Mischung wurde 72 h bei Raumtemperatur gerührt und anschließend nach der Zugabe von Wasser (80 ml) mit Ether (4 x 100 ml) extrahiert. Nach dem Einengen der Lösung wurde der Rückstand in Dichlormethan (200 ml) aufgenommen und mit Magnesiumsulfat über Nacht getrocknet. Die organische Phase wurde eingeengt und das Ketal als weißer Feststoff erhalten.
*Ausbeute:* 38.9 g (96 %); *Schmelzpunkt:* 86-88 °C
*$^1$H-NMR (DMSO-$d_6$):* 1.57 (2 H, m); 1.72 (2 H; m); 1.85 (2 H, m); 1.99 (2 H, m); 2.25 (6 H, s); 3.87 (4 H, m).

*$^{13}C$-NMR (DMSO-$d_6$):* 30.02; 31.32; 60.66; 63.77; 106.31; 118.40.

**8-Methylamino-1,4-dioxa-spiro[4.5]decan-8-carbonitril** Zu einem Gemisch aus 4N Salzsäure (12.5 ml) und Methanol (7.5 ml) wurde unter Eiskühlung 40%-ige wässrige Methylamin-Lösung (29.0 ml, 0.23 mol), Cyclohexan-1,4-dion-monoethylenketal (7.50 g, 0.048 mol) und Kaliumcyanid (7.50 g) hinzugefügt. Die Mischung wurde bei Raumtemperatur 7 d gerührt. Nach Zugabe von Wasser (20 ml) wurde mit Ether (4 x 25 ml) extrahiert. Nach dem Einengen der Lösung wurde der Rückstand in Dichlormethan (50 ml) aufgenommen und mit $MgSO_4$ über Nacht getrocknet. Die organische Phase wurde eingeengt und das Ketal als Öl erhalten, welches durchkristallisierte.

*Ausbeute:* 7.05 g (80 %)

*$^1H$-NMR (DMSO-$d_6$):* 1.54 (2 H, m); 1.71 (4 H, m); 1.95 (2 H, m); 2.30 (3 H, d); 2.72 (1H, q); 3.86 (4 H, s).

**Ketonbaustein Ket-2:**

**1-Methyl-4-(8-[1,2,3]triazol-1-yl-1,4-dioxaspiro[4.5]dec-8-yl)piperazin** In einem ausgeheizten Kolben wurde eine Lösung von N-Methylpiperazin (2.60 g, 2.88 ml, 26 mmol), 1,4-Dioxaspiro[4.5]decan-8-on (3.90 g, 25 mmol) und 1,2,3-Triazol (1.87 g, 27 mmol) in Toluol (25 ml) 6 h am Wasserabscheider unter Rückfluss erhitzt.
Anschließend wurde die Reaktionslösung in einen verschließbaren Messzylinder überführt und das Rohprodukt weiter umgesetzt.

**1-(8-Benzyl-1,4-dioxaspiro[4.5]dec-8-yl)-4-methylpiperazin** Zu einer Lösung von 1-Methyl-4-(8-[1,2,3]triazol-1-yl-1,4-dioxaspiro[4.5]dec-8-yl)piperazin (12.5 mmol) in Toluol (12 ml) wurde unter Argon eine 2 M Benzylmagnesiumchlorid-Lösung in Tetrahydrofuran (15 ml, 30 mmol) so getropft, dass die Innentemperatur unter 24 °C blieb. Nach beendeter Zugabe wurde das Reaktionsgemisch 2 h bei Raumtemperatur gerührt und dann auf 0 °C gekühlt und zu einer 20 % Ammoniumchloridlösung (50 ml) getropft, die wässrige Phase mit Diethylether (3 × 40 ml) extrahiert, die vereinigten organischen Phasen wurden mit 2 N Natronlauge (70 ml) und Wasser (70 ml) gewaschen, mit Natriumsulfat getrocknet und i. Vak. eingeengt. Das Rohprodukt (4.28 g) wurde weiter umgesetzt.

**4-Benzyl-4-(4-methylpiperazin-1-yl)cyclohexanon (Ket-2)**Eine Lösung von 1-(8-Benzyl-1,4-dioxaspiro[4.5]dec-8-yl)-4-methylpiperazin (4.28 g, 13.0 mmol) in Aceton (15 ml) wurde zuerst mit Wasser (2.5 ml) und dann mit konzentrierter Salzsäure (2.5 ml) versetzt und über Nacht bei Raumtemperatur gerührt. Anschließend wurde das Reaktionsgemisch mit 2 M Kaliumcarbonatlösung alkalisch (pH 10) gestellt, mit Diethylether (3 × 40 ml) extrahiert, die vereinigten organischen Phasen wurden mit Natriumsulfat getrocknet und i. Vak. eingeengt. Das Rohprodukt wurde mittels Flashchromatographie (200 g, 20 × 5.7 cm) mit Methanol gereinigt.

*Ausbeute:* 2.63 g (71 %), gelblicher Feststoff **(Ket-2)**; *Schmelzpunkt:* 113-132 °C

*$^1H$-NMR(DMSO-$d_6$):* 1.36 (dt, 2H, J = 13.8, 4.6 Hz); 1.92 (dd, 2H, J = 14.9, 4.1 Hz), 2.05-2.14 (m. 2H); 2.17 (s, 3H); 2.34-2.47 (m, 6H); 2.61-2.69 (m, 4H); 2.72 (s, 2H); 7.12-7.28 (m, 5H).

*$^{13}C$-NMR:* 30.9 (2C); 35.8 (2C); 37.3; 43.7 (2C); 45.6; 55.6 (2C); 56.7; 125.8; 127.7 (2C); 130.5 (2C); 138.3; 210.0.

**Ketonbaustein Ket-3:**

**Dimethyl-(8-benzyl-1,4-dioxa-spiro[4.5]dec-8-yl)amin Hydrochlorid**

Zu einer Lösung von 8-Dimethylamino-1,4-dioxa-spiro[4.5]decan-8-carbonitril (39,8 g, 190 mmol) in THF (300 ml) wurde unter Argon und Eiskühlung innerhalb von 15 min 2M Benzylmagnesiumchlorid-Lösung in THF (3, 285 ml, 570 mmol) hinzugefügt. Anschließend wurde der Ansatz 16 h bei RT gerührt. Zur Aufarbeitung des Reaktionsgemisches wurde unter Eiskühlung gesättigte Ammoniumchloridlösung (200 ml) und Wasser (100 ml) zugegeben und mit Diethylether (3 × 100 ml) extrahiert. Die organische Phase wurde mit Wasser (50 ml) und gesättigter NaCl-Lösung (50 ml) ausgeschüttelt und dann eingeengt. Es blieb ein weißer Feststoff (68,34 g) zurück, der außer des Produktes noch das Edukt enthielt. Das Rohprodukt wurde in Ethylmethylketon (250 ml) gelöst und unter Eiskühlung mit $ClSiMe_3$ (30 ml, 238 mmol) versetzt. Nach 16 h wurde Dimethyl-(8-benzyl-1,4-dioxa-spiro[4.5]dec-8-yl)amin Hydrochlorid in einer Ausbeute von 52 % (40.33 g) als weißer Feststoff mit einem Schmelzpunkt von 276-280 °C isoliert.

**4-Dimethylamino-4-benzylcyclohexanon (Ket-3)**

Das Hydrochlorid (40,33 g, 130 mmol) wurde in Wasser (55 ml) gelöst, mit konzentrierter Salzsäure (100 ml, 1,21 mol) versetzt und 4 d bei Raumtemperatur gerührt. Nach beendeter Hydrolyse wurde die Reaktionsmischung mit Diethylether extrahiert (2 × 100 ml) und die wäßrige Phase unter Eiskühlung mit 5N Natronlauge alkalisch gemacht, wobei das Produkt ausfiel. Der Feststoff wurde abgesaugt, mit $H_2O$ (3 × 20 ml) gewaschen und getrocknet. 4-Dimethylamino-4-benzylcyclohexanon **(Ket-3)** konnte so in einer Ausbeute von 87 % (26 g) als gelber Feststoff mit einem Schmelzpunkt von 80-84 °C isoliert werden.

**Ketonbaustein Ket-4:**

**(8-Butyl-1,4-dioxa-spiro[4.5]dec-8-yl)-dimethyl-amin Hydrochlorid** 8-Dimethylamino-1,4-dioxa-spiro[4.5]decan-8-carbonitril (10.5 g, 50 mmol) wurde in THF (150 ml) unter Eiskühlung und Argon vorgelegt. Innerhalb von 15 min wurde 2M Butylmagnesiumchlorid in THF (62.5 ml, 125 mmol) zugetropft und 16 h bei RT gerührt.
Der Ansatz wurde unter Eiskühlung mit 20 %-iger Ammoniumchlorid-Lösung (37 ml) und Wasser (50 ml) versetzt und mit Ether (3 x 50 ml) extrahiert. Die org. Phase wurde mit Wasser (1 x 50 ml) und gesättigte Natriumchlorid-Lösung (1 x 50 ml) gewaschen, die organische Phase mit $Na_2SO_4$ getrocknet und i. Vak. eingeengt.
Das Rohprodukt (2.05 g) wurde in Ethylmethylketon (75 ml) gelöst, unter Eiskühlung mit $ClSiMe_3$ (9.5 ml, 75 mmol) versetzt und 6 h bei RT gerührt. Der ausgefallene weiße Niederschlag wurde abgesaugt und i. Vak. getrocknet. *Ausbeute:* 3.1 g (22 %)
*$^1$H-NMR (DMSO-$d_6$):* 0.91 (3 H, t); 1.31 (4 H, m); 1.56 (2 H, m); 1.75 (8 H, m); 2.64 (6 H, s); 3.87 (4 H, s); 9.87 (1 H, s).

**4-Butyl-4-dimethylamino-cyclohexanon (Ket-4)**

8-Butyl-1,4-dioxa-spiro[4.5]dec-8-yl)-dimethyl-amin Hydrochlorid (3.10 g, 11.1 mmol) wurde in $H_2O$ (4.7 ml) und konz. HCl (7 ml) vorgelegt und 24 h bei RT gerührt. Der Ansatz wurde mit Ether (1 x 15 ml) extrahiert, die wässrige Phase unter Eiskühlung mit 5N NaOH alkalisch eingestellt und mit Dichlormethan (3 x 20 ml) extrahiert. Die org. Phase wurde über $Na_2SO_4$ getrocknet und i. Vak. eingeengt. *Ausbeute:* 1.96 g (89 %), Öl
*$^1$H-NMR (DMSO-$d_6$):* 0.88 (3 H, t); 1.23 (4 H, m); 1.40 (2 H, m); 1.68 (2 H, m); 1.91 (2 H, m); 2.31 (2 H, m); 2.22 (6 H, s); 2.42 (2 H, m).
*$^{13}$C-NMR* (DMSO-$d_6$): 13.91; 23.21; 26.06; 29.53; 31.07; 37.04; 38.88; 55.36; 210.37.

**Ketonbaustein Ket-6:**

**2-Iod-benzo[b]thiophen**

In einem 500 ml Dreihalskolben wurde unter Argonatmosphäre Butyllithium 1.6M in Hexan (112.5 ml, 180 mmol) und abs. Ether (70 ml) vorgelegt und im Eisbad auf 0 °C abgekühlt. Anschließend Benzothiophen (20.1 g, 150 mmol) in abs. Ether (40 ml) gelöst und tropfenweise unter Eiskühlung innerhalb von 30 Minuten zugetropft und 2.5 h im Eisbad nachgerührt. Der Reaktionsansatz stand über Nacht im Kühlschrank. In einem 500 ml Dreihalskolben wurden unter Argonatmosphäre Iod (75.0 g) und abs. Ether (50 ml) vorgelegt und Die Kösung der Lithium-Verbindung unter Eiskühlung zugetropft. Der Ansatz wurde langsam auf Raumtemperatur erwärmt, mit Wasser hydrolysiert, mit Natriumthiosulfat-Lösung gewaschen und die organische Phase über Natriumsulfat getrocknet. Anschließend wurde die Reaktionslösung i. Vak. eingeengt und mittels Flash-Chromatographie mit Cyclohexan gereinigt. *Ausbeute:* 24.1 g (62 %), halbfeste, hellbraune Kristalle
*$^1$H-NMR (DMSO-$d_6$):* 7.32 (2 H, m); 7.75 (1 H, s); 7.81 (1 H, m); 7.93 (1 H, m).

**(8-Benzo[b]thiophen-2-yl-1,4-dioxa-spiro[4.5]dec-8-yl)-dimethyl-amin Hydrochlorid**

In einem 100 ml Dreihalskolben wurde Mg (238 mg) in abs. Ether (2 ml) unter Argon vorgelegt, dazu wurde langsam 2-Iod-benzo[b]thiophen (2.51 g, 9.6 mmol) in abs. Ether (8 ml) zugetropft. Nach Zugabe von abs. Ether (10 ml) wurde der Ansatz 5 h unter Rückfluss gekocht. Die Reaktionslösung wurde im Eisbad abgekühlt und bei 10 °C tropfenweise das 8-Dimethylamino-1,4-dioxa-spiro[4.5]decan-8-carbonitril (1.03 g, 4.9 mmol) in THF (10 ml) zugegeben. Der Ansatz rührte bei Raumtemperatur über Nacht, das Reaktionsgemisch wurde unter Eiskühlung mit $NH_4Cl$-Lösung (5 ml) und Wasser (7 ml) versetzt und mit Ether (3 x 30 ml) extrahiert. Die org. Phase wurde mit Wasser (30 ml) und anschließend mit gesättigter NaCl-Lösung (20 ml) gewaschen, über $Na_2SO_4$ getrocknet und i. Vak. eingeengt. *Ausbeute:* 1.99 g (66 %)

Das Rohprodukt wurde in Ethylmethylketon (19 ml) gelöst, unter Eiskühlung mit Trimethylchlorsilan (1.63 ml, 12.8 mmol) versetzt und bei Raumtemperatur 5 h gerührt. Der entstandene Niederschlag wurde abgesaugt und i. Vak. getrocknet.
*Ausbeute:* 600 mg (35 %)
*$^1$H-NMR (DMSO-$d_6$):* 1.46 (2 H, m); 1.79 (2 H, m); 2.37 (2 H, m); 2.63 (6 H, s); 2.75 (2 H, m); 7.47 (2 H, m); 7.91 (1 H, s); 7.95 (1 H, m); 8.06 (1 H, m); 11.40 (1 H, s).
*$^{13}$C-NMR (DMSO-$d_6$):* 30.43; 31.13; 37.84; 63.88; 66.42; 105.84; 122.48; 124.55; 124.89; 125.71; 128.99; 135.00; 138.91; 139.58.

**4-Benzo[b]thiophen-2-yl-4-dimethylamino-cyclohexanon (Ket-6)**

Das (8-Benzo[b]thiophen-2-yl-1,4-dioxa-spiro[4.5]dec-8-yl)-dimethyl-amin Hydrochlorid (0.60 g, 1.7 mmol) wurde in Wasser (0.8 ml) gelöst, mit konzentrierter Salzsäure (1.04 ml, 151 mmol) versetzt und 3 d bei Raumtemperatur gerührt. Nach beendeter Hydrolyse wurde die Reaktionsmischung mit Diethylether (2 x 25 ml) extrahiert und die wässrige Phase mit 5N Natronlauge alkalisch gemacht, mit Dichlormethan (3 x 25 ml) extrahiert, über Natriumsulfat getrocknet und i. Vak. eingeengt. *Ausbeute:* 0.44 g (95 %) **Ket-6**

*$^1$H-NMR (DMSO-$d_6$):* 2.19 (10 H, m); 2.52 (4 H, m); 7.35 (3 H, m); 7.84 (1 H, m); 7.91 (1 H, m).

*$^{13}$C-NMR (DMSO-$d_6$):* 33.74; 36.51; 38.05; 58.60; 121.87; 121.94; 123.35; 124.02; 124.16; 138.19; 139.17; 144.28; 209.50.

**Ketonbaustein Ket-8:**

**8-Pyrrolidin-1-yl-1,4-dioxaspiro[4.5]decan-8-carbonitril**

Zu einem Gemisch aus 4N Salzsäure (17 ml) und Methanol (10 ml) wurde unter Eiskühlung Pyrrolidin (22.5 ml, 0,306 mol), Cyclohexan-1,4-dion-monoethylenketal (10,0 g, 0,064 mol) und Kaliumcyanid (10,0 g, 0,15 mol) hinzugefügt. Die Mischung wurde 74 h bei Raumtemperatur gerührt und anschließend nach der Zugabe von Wasser (80 ml) mit Diethylether (4 × 70 ml) extrahiert. Nach dem Einengen wurde der Rückstand in Dichlormethan (70 ml) aufgenommen und mit Magnesiumsulfat über Nacht getrocknet. Die organische Phase wurde eingeengt und das 8-Pyrrolidin-1-yl-1,4-dioxaspiro[4.5]decan-8-carbonitril als weißer Feststoff mit einem Schmelzpunkt von 65-67 °C in einer Ausbeute von 68 % (10,2 g) erhalten.

**4-(8-Phenyl-1,4 dioxaspiro[4.5]dec-8-yl)pyrrolidin Hydrochlorid**

Zu einer 1,82M Phenylmagnesiumchlorid-Lösung in THF (70 ml, 0,127 mol) wurde unter Argon und Eiskühlung innerhalb von 15 min8-Pyrrolidin-1-yl-1,4-dioxaspiro[4.5]decan-8-carbonitril (10,0 g, 42,6 mmol), gelöst in THF (90 ml), hinzugefügt und 16 h bei Raumtemperatur gerührt. Zur Aufarbeitung des Reaktionsgemisches wurde unter Eiskühlung gesättigte Ammoniumchloridlösung (100 ml) zugegeben und dann mit Diethylether (3 × 100 ml) extrahiert. Die organische Phase wurde mit Wasser (70 ml) und gesättigter NaCl-Lösung (70 ml) ausgeschüttelt und eingeengt. Es blieb ein gelber Kristallbrei (11,8 g) zurück, der außer der dem gewünschten Produkt noch Edukt enthielt. Das Rohprodukt wurde in Ethylmethylketon (70 ml) gelöst und unter Eiskühlung mit $ClSiMe_3$ (8 ml, 0,063 mol) versetzt. Nach einer Reaktionszeit von 6 h konnte das 4-(8-Phenyl-1,4-dioxaspiro[4.5]dec-8-yl)pyrrolidin Hydrochlorid in einer Ausbeute von 43 % (5,9 g) als weißer Feststoff isoliert werden.

**4-Pyrrolidin-4-yl-4-phenylcyclohexanon (Ket-8)**

4-(8-Phenyl-1,4-dioxaspiro[4.5]dec-8-yl)pyrrolidin Hydrochlorid (5,8 g, 17,9 mmol) wurde in 7,5N Salzsäure (16 ml) gelöst und 24 h bei Raumtemperatur gerührt. Nach beendeter Hydrolyse wurde die Reaktionsmischung mit Diethylether extrahiert (2 × 50 ml), die wäßrige Phase unter Eiskühlung mit 5N Natronlauge alkalisch gemacht, mit Dichlormethan (3 × 50 ml) extrahiert und eingeengt. Das Keton Ket-8 konnte als gelber Feststoff mit einem Schmelzpunkt von 75-79 °C und einer Ausbeute von 96 % (4.1 g) isoliert werden.

**Ketonbaustein Ket-9:**

**4-(8-Phenyl-1,4-dioxaspiro[4.5]dec-8-yl)morpholin Hydrochlorid**

Unter Argon wurde zu einem Gemisch aus Magnesium (2,9 g, 0,119 mol) und wasserfreiem THF (15 ml) Brombenzol (3,0 g, 0,019 mol) und etwas Iod gegeben. Nach 30 min wurde der Ansatz auf 50 °C erwärmt, wobei die Grignard-Reaktion unter Sieden ansprang. Innerhalb von 20 min wurde weiteres Brombenzol (15,7 g, 0,1 mol), gelöst in THF (50 ml), hinzugefügt und 1,5 h unter Rückfluß gekocht. Unter Eiskühlung wurde das 8-Pyrrolidin-1-yl-1,4-dioxaspiro [4.5]decan-8-carbonitril (10,0 g, 0,0396 mol), gelöst in THF (60 ml), innerhalb von 20 min zum Ansatz gegeben. Anschließend wurde die Reaktionsmischung 4 h auf 70 °C erwärmt. Der Abbruch der Reaktion erfolgte nach einer weiteren Reaktionszeit von 16 h bei Raumtemperatur durch Zugabe von $NH_4Cl$-Lösung (60 ml) unter Eiskühlung. Die wäßrige Phase wurde mit Diethylether (2 × 70 ml) extrahiert, die organische Phase mit Wasser (50 ml) und gesättigter NaCl-Lösung (50 ml) ausgeschüttelt und eingeengt. Es blieb ein gelber Kristallbrei (11 g) zurück, der außer der gewünschten Phenylverbindung auch das nicht umgesetzte Aminonitril Edukt enthielt. Das erhaltene Rohprodukt wurde in Methylethylketon (140 ml) gelöst und unter Eiskühlung mit Trimethylchlorsilan (7,5 ml, 0,059 mol) versetzt. Nach 15 min begann

ein weißer Niederschlag auszufallen, der nach 6 h abgesaugt wurde. Es konnten 6,5 g (49 %) des Hydrochlorids 5 mit einem. Schmelzpunkt von 250-252 °C erhalten werden.

**4-Morpholin-4-yl-4-phenylcyclohexanon (Ket-9)**

Das soeben erhaltene Hydrochlorid (6,5 g, 19,1mmol) wurde in 7,5N Salzsäure (22 ml) gelöst und 24 h bei Raumtemperatur gerührt. Nach beendeter Hydrolyse wurde die Reaktionsmischung mit $Et_2O$ (2 × 50 ml) ausgeschüttelt. Die wäßrige Phase wurde unter Eiskühlung mit 5N Natronlauge alkalisch gemacht, mit Dichlormethan (3 × 50 ml) extrahiert und eingeengt. Das Keton **Ket-9** konnte so als beigefarbener Feststoff mit einem Schmelzpunkt von 116-119 °C und einer Ausbeute von 84 % (4,1 g) isoliert werden.

**<u>Ketonbaustein Ket-10:</u>**

**Dimethyl-(8-phenyl-1,4-dioxaspiro[4.5]dec-8-yl)amin Hydrochlorid**

Zu einer 1,82 M Phenylmagnesiumchlorid-Lösung in THF (109 ml, 0,198 mol) wurde unter Argon und Eiskühlung innerhalb von 15 min 8-Dimethylamino-1,4-dioxa-spiro[4.5]decan-8-carbonitril (21 g, 0,1 mol), gelöst in THF (210 ml), hinzugefügt und dann 16 h bei Raumtemperatur gerührt. Zur Aufarbeitung des Reaktionsgemisches wurde unter Eiskühlung gesättigte Ammoniumchloridlösung (150 ml) zugegeben und mit Diethylether (3 × 100 ml) extrahiert. Die organische Phase wurde mit Wasser (100 ml) und gesättigter NaCl-Lösung (100 ml) ausgeschüttet und eingeengt. Es blieb ein gelbes Öl (25,2 g) zurück. Das Rohprodukt wurde in Ethylmethylketon (280 ml) gelöst und unter Eiskühlung mit $ClSiMe_3$ (18,8 ml, 0.15 mol) versetzt. Nach einer Reaktionszeit von 6 h konnte Dimethyl-(8-phenyl-1,4-dioxaspiro [4.5]dec-8-yl)amin Hydrochlorid in einer Ausbeute von 35 % (10,5 g) als weißer Feststoff isoliert werden.

**4-Dimethylamino-4-phenylcyclohexanon (Ket-10)**

Dimethyl-(8-phenyl-1,4-dioxaspiro[4.5]dec-8-yl)amin Hydrochlorid (10,5 g, 35,2 mmol) wurde in 7,5N Salzsäure (36 ml) gelöst und 96 h bei Raumtemperatur gerührt. Nach beendeter Hydrolyse wurde die Reaktionsmischung mit Diethylether extrahiert (2 × 50 ml). Die wäßrige Phase wurde unter Eiskühlung mit 5N Natronlauge alkalisch gemacht, mit Dichlormethan (3 × 50 ml) extrahiert und eingeengt. 4-Dimethylamino-4-phenylcyclohexanon **(Ket-10)** konnte so als gelber Feststoff mit einem Schmelzpunkt von 104-108 °C in einer Ausbeute von 97 % (7,4 g) isoliert werden.

**<u>Ketonbaustein Ket-11:</u>**

Eine Lösung aus 4,5 g 8-Dimethylamino-1,4-dioxa-spiro[4.5]decan-8-carbonitril, 50 mg Cyclopentadienyl-cycloocta-1,5-diene-cobalt(I) [cpCo(cod)] und 100 ml Toluol wurde im Schutzgas-/Acetylengegenstrom in das Reaktionsgefäß überführt. Nach Sättigung mit Acetylen wurde die Reaktionslösung unter kräftigem Rühren bei einer Temperatur von 25°C über einen Zeitraum von 6 Stunden bestrahlt. Die Reaktion wurde durch Abschalten der Lampen und Luftzufuhr unterbrochen und die Reaktionslösung eingeengt. Das erhaltene Rohprodukt (5,47 g) wurde in einem Gemisch aus Wasser (8,7 ml) und konz. Salzsäure (15 ml) aufgenommen und über Nacht bei RT gerührt. Zur Aufarbeitung wurde mit Diethylether (3 × 100 ml) gewaschen, die Phasen getrennt, die wäßrige Phase mit 32 massenprozentiger Natronlauge alkalisch gestellt, mit Dichlormethan extrahiert (3 × 100 ml), die vereinigten Extrakte getrocknet ($Na_2SO_4$), filtriert und eingeengt. Es wurden 3,72 g 4-Dimethylamino-4-pyridin-2-ylcyclohexanon (Ket-11) erhalten.

**<u>Ketonbaustein Ket-12:</u>**

**Dimethyl-(8-thiophen-2-yl-1,4-dioxaspiro[4.5]dec-8-yl)amin Hydrochlorid**

2-Iodthiophen (22,9 g, 109 mmol) wurde unter Argon in THF (80 ml) gelöst und innerhalb von 30 min bei 0 °C mit 2M Isopropylmagnesiumchlorid (35,7 ml, 72 mmol) in THF versetzt. Nach einer Reaktionszeit von 1 h bei 3-5 °C wurde 8-Dimethylamino-1,4-dioxa-spiro[4.5]decan-8-carbonitril (10 g, 47,6 mmol), gelöst in Tetrahydrofuran (20 ml), hinzugefügt und 20 h bei Raumtemperatur gerührt. Die Aufarbeitung des Ansatzes erfolgte durch Zugabe von gesättigter $NH_4Cl$-Lösung (85 ml) und Extraktion mit Diethylether (3x100 ml). Die organische Phase wurde mit Wasser (50 ml) und gesättigter NaCl-Lösung (50 ml) ausgeschüttelt und eingeengt. Es wurde ein dunkelbraunes Öl (21,3 g) erhalten. Das Rohprodukt wurde in Ethylmethylketon (140 ml) gelöst und mit $ClSiMe_3$ (9,1 ml, 71,4 mmol) versetzt. Nach einer Reaktionszeit von 6 h wurde Dimethyl-(8-thiophen-2-yl-1,4-dioxaspiro[4.5]dec-8-yl)amin Hydrochlorid als weiße, kristalline Verbindung in einer Ausbeute von 60 % (8,74 g) isoliert.

**4-Dimethylamino-4-thiophen-2-ylcyclohexanon (Ket-12)**

Dimethyl-(8-thiophen-2-yl-1,4-dioxaspiro[4.5]dec-8-yl)amin Hydrochlorid (8,68 g, 28,6 h beendeter Hydrolyse wurde die Reaktionsmischung mit Diethylether extrahiert (2 × 50 ml). Die wäßrige Phase wurde unter Eiskühlung mit 5N Natronlauge alkalisch gemacht, mit Dichlormethan (3 × 50 ml) extrahiert und eingeengt. **Ket-12** wurde so als gelber Feststoff mit einem Schmelzpunkt von 108-110 °C in einer Ausbeute von 89 % (5,66 g) erhalten.

**Ketonbaustein Ket-13:**

*Variante 1:* **[8-(3-Fluorphenyl)-1,4-dioxaspiro[4.5]dec-8-yl]dimethylamin Hydrochlorid**

Zu einer Lösung von 8-Dimethylamino-1,4-dioxa-spiro[4.5]decan-8-carbonitril (19,8 g, 94 mmol) in THF (100 ml) wurde unter Argon und Eiskühlung innerhalb von 15 min 0,5M 3-Fluorphenylmagnesiumbromid-Lösung in THF (3, 750 ml, 375 mmol) hinzugefügt und dann 16 h bei Raumtemperatur gerührt. Zur Aufarbeitung des Reaktionsgemisches wurde unter Eiskühlung gesättigte Ammoniumchloridlösung (150 ml) und Wasser (60 ml) zugegeben und mit Diethylether (3 × 100 ml) extrahiert. Die organische Phase wurde mit Wasser (50 ml) und gesättigter NaCl-Lösung (50 ml) ausgeschüttelt und eingeengt. Es blieb ein braunes Öl (26,5 g) zurück, das außer der Phenylverbindung 4 noch das Ketal 2 enthielt. Das Rohprodukt wurde in Ethylmethylketon (156 ml) gelöst und unter Eiskühlung mit ClSiMe3 (17,8 ml, 141 mmol) versetzt. Nach einer Reaktionszeit von 6 h konnte das Hydrochlorid in einer Ausbeute von 55 % (16,3 g) als weißer Feststoff mit einem Schmelzpunkt von 275-278 °C isoliert werden.

*Variante 2:* **[8-(3-Fluor-phenyl)-1,4-dioxa-spiro[4.5]dec-8-yl]-dimethyl-amin Hydrochlorid**

Eine Lösung von 1-Brom-3-fluorbenzol (5.00 g, 28.6 mmol) in abs. Ether (15 ml) wurde zu einer Suspension von Magnesium (694 mg, 28.6 mmol) in abs. Ether (10 ml) so getropft, dass der Ether siedete. Nach beendeter Zugabe wurde 10 min bei RT nachgerührt, das Magnesium war danach vollständig gelöst. Die Reaktionslösung wurde im Eisbad abgekühlt und bei 10 °C tropfenweise 8-Dimethylamino-1,4-dioxa-spiro[4.5]decan-8-carbonitril (3.00 g, 14.3 mmol) in abs. THF (30 ml) zugegeben. Der Ansatz rührte bei Raumtemperatur über Nacht, das Reaktionsgemisch wurde unter Eiskühlung mit 20 %iger $NH_4Cl$-Lösung (20 ml) und Wasser (30 ml) versetzt und mit Ether (3 x 50 ml) extrahiert. Die org. Phase wurde mit Wasser (50 ml) und anschließend mit gesättigter NaCl-Lösung (50 ml) gewaschen, über $Na_2SO_4$ getrocknet und i. Vak. eingeengt.
Das Rohprodukt wurde in Ethylmethylketon (25 ml) gelöst, unter Eiskühlung mit $ClSiMe_3$ (3.2 ml, 25 mmol) versetzt und bei Raumtemperatur 5 h gerührt. Der entstandene Niederschlag wurde abfiltriert und i. Vak. getrocknet. *Ausbeute:* 2.8 g (62 %)
*$^1$H-NMR (DMSO-$d_6$):* 1.91 (8 H, m); 2.54 (6 H, s); 3.91 (4 H, d); 7.37 (1 H, m); 7.61 (3 H, m).

*Variante 1:* **4-Dimethylamino-4-(3-fluor-phenyl)-cyclohexanon (Ket-13)**

[8-(3-Fluor-phenyl)-1,4-dioxa-spiro[4.5]dec-8-yl]-dimethyl-amin Hydrochlorid (7,2 g, 22,75 mmol) wurde in Wasser (9,6 ml) gelöst, mit konzentrierter Salzsäure (14 ml, 455 mmol) versetzt und 4 d bei Raumtemperatur gerührt. Nach beendeter Hydrolyse wurde die **Reaktionsmischung** mit Diethylether extrahiert (2 × 50 ml), die wäßrige Phase unter Eiskühlung mit 5N Natronlauge alkalisch gemacht, wobei das Produkt ausfiel. Das Keton **Ket-13** konnte als gelber Feststoff mit einem Schmelzpunkt von 83-88 °C und einer Ausbeute von 50 % (6,05 g) isoliert werden.

*Variante 2:*

**4-Dimethylamino-4-(3-fluor-phenyl)-cyclohexanon (Ket-13)** [8-(3-Fluor-phenyl)-1,4-dioxa-spiro[4.5]dec-8-yl]-dimethyl-amin Hydrochlorid (2.80 g, 8.86 mmol) wurde in Wasser (3.7 ml) gelöst, mit konzentrierter Salzsäure (5.5 ml) versetzt und bei RT 4 d gerührt. Nach beendeter Hydrolyse wurde die Reaktionsmischung mit Ether (2 x 10 ml) extrahiert, die wässrige Lösung unter Eiskühlung mit 5N Natronlauge alkalisch gestellt, die Reaktionsmischung mit Dichlormethan (3 x 50 ml) extrahiert, die organische Phase über Natriumsulfat getrocknet und i. Vak. eingeengt. Das Rohprodukt wurde durch Flash-Chromatographie mit $CHCl_3$/MeOH (20:1) gereinigt.
Ausbeute **Ket-13:** 676 mg (32 %), farbloser Feststoff; *Schmelzpunkt:* 62-67 °C
*$^1$H-NMR (DMSO-$d_6$):* 2.02 (6 H, s); 2.12 (5 H, m); 2.45 (3 H, m); 7.24 (3 H, m); 7.43 (1 H, m).

**Ketonbaustein Ket-14:**

**1-(8-Pyrrolidin-1-yl-1,4-dioxaspiro[4.5]dec-8-yl)-1H-[1,2,3]triazol** Zu einer Lösung von 1,4 Dioxaspiro[4,5]decan-8 on (3.9 g, 25 mmol) in Toluol (40 ml) wurden Pyrrolidin (1.95 g, 2.29 ml, 27.5 mmol), 1,2,3-Triazol (2.07 g, 30 mmol) und Molsieb 4 Å (7.14 g) gegeben. Das Gemisch wurde 7 h bei 90 °C gerührt. Anschließend wurde die Lösung dekantiert und sofort weiter umgesetzt.

**1-(8-Butyl-1,4-dioxaspiro[4.5]dec-8-yl)pyrrolidin** Zu einer 2 M Lösung von n-Butylmagnesiumchlorid (25 ml, 50 mmol) in Tetrahydrofuran wurde unter Eiskühlung und Argon die Reaktionslösung von 1-(8-Pyrrolidin-1-yl-1,4-dioxaspiro[4.5]dec-8-yl)-1H-[1,2,3]triazol (ca. 6.9 g, 25 mmol) in Toluol (38 ml) getropft. Das Reaktionsgemisch wurde über Nacht bei Raumtemperatur gerührt und anschließend in gesättigte Ammoniumchloridlösung (60 ml) gegossen. Die Phasen wurden getrennt und die wässrige mit Diethylether (3 × 70 ml) extrahiert. Die vereinigten organischen Phasen wurden mit Natriumsulfat getrocknet, i. Vak. eingeengt und der Rückstand (12 g) durch Flashchromatographie (400 g, 20 × 7.6 cm) mit Ethylacetat / Methanol (9:1) gereinigt.
*Ausbeute:* 2.70 g (40 % über zwei Stufen)
*$^1$H-NMR (DMSO-$d_6$):* 0.87 (t, 3H, J = 7.1 Hz); 1.12-1-29 (m, 4H); 1.30-1.45 (m, 4H); 1.46-1.60 (m, 4H); 1.61-1.75 (m, 6H); 1.93 (t, 1 H, J = 7.1 Hz); 2.36 (t, 1 H, J = 7.0 Hz), 2.58 (br s, 2H), 3.83 (s, 4H).

**4-Butyl-4-pyrrolidin-1-yl-cyclohexanon (Ket-14)**

Eine Lösung von 1-(8-Butyl-1,4-dioxaspiro[4.5]dec-8-yl)pyrrolidin (2.70 g, 10.1 mmol) in Aceton (100 ml) wurde mit Wasser (10.0 ml) und 37 % Salzsäure (14.0 ml) versetzt und über Nacht bei Raumtemperatur gerührt. Zum Gemisch wurde anschließend langsam 4 M Natronlauge getropft, bis pH 10 erreicht war. Das Gemisch wurde mit Diethylether (4 × 40 ml) extrahiert, die vereinigten organischen Phasen wurden mit Natriumsulfat getrocknet und i. Vak. eingeengt. Das Rohprodukt (2.6 g) wurde durch Flashchromatographie (260 g, 30 × 5.6 cm) mit Ethylacetat / Methanol (9: 1) gereinigt.
*Ausbeute:* 1.06 g (47 %), braunes Öl **Ket-14**
*$^1$H-NMR (DMSO-$d_6$)*: 0.88 (t, 3H, J = 6.7 Hz); 1.14-1.34 (m, 4H); 1.40-1.50 (m, 2H); 1.62-1.88 (m, 8H); 2.04 (dt, 2H, J = 15.0, 3.9 Hz); 2.42 (ddd, 2H, J = 6.3. 11.8, 15.5 Hz); 2.63 (t, 4H, J = 6.0 Hz).

**<u>Ketonbaustein Ket-15:</u>**

**4-Cyano-4-phenylheptandisäure-dimethylester**

Phenylacetonitril (11,7 g, 0,1 mol) und Methylacrylat (47 ml, 0,5 mol) wurden in tert-Butanol (60 ml) vorgelegt und bis zum Sieden erhitzt. Anschließend wurde die Heizquelle entfernt. Das in *tert*-Butanol (23 ml) gelöste Triton B (Benzyltrimethylammoniumhydroxid, 40-proz. in Methanol, 15,2 ml) wurde erst langsam, später zügig zugetropft. Nach dem Zutropfen wurde der Ansatz 4 h unter Rückfluß gekocht. Über Nacht wurde die Reaktionsmischung auf Raumtemperatur abgekühlt. - Zur Aufarbeitung wurde der Ansatz mit Toluol (100 ml) und Wasser (70 ml) versetzt. Die organische Phase wurde abgetrennt und mit Wasser (1 × 70 ml) und gesättigter Natriumchloridlösung (1 × 50 ml) gewaschen. Nach dem Trocknen mit $Na_2SO_4$ wurde das Lösungsmittel wegen der starken Geruchsbelästigung im Abzug destilliert. Die Reinigung erfolgte durch eine Kugelrohrdestillation bei einem Druck von $7{,}8 \times 10^{-2}$ mbar und einer Temperatur von 235 °C. Der gewünschte 4-Cyano-4-phenylheptandisäure-dimethylester konnte in einer Ausbeute von 21,45 g (72 %) als farblose, zähflüssige Substanz isoliert werden.

**5-Cyano-2-oxo-5-phenylcyclohexancarbonsäure-methylester**

4-Cyano-4-phehylheptandisäure-dimethylester (14,45 g, 0,05 mol) wurde in trockenem Tetrahydrofuran (350 ml) gelöst. Anschließend wurde portionsweise Natrium-tert-butylat (9,6 g, 0,1 mol) zugegeben. Bei dieser Zugabe verfärbte sich die Reaktionsmischung orange. Danach wurde der Ansatz 5 h unter Rückfluß gekocht. Während des Kochens entstand eine beigefarbende, breiartige Suspension. Über Nacht wurde die Reaktionsmischung auf Raumtemperatur abgekühlt. Unter Eiskühlung wurde 2,5N Essigsäure (170 ml) langsam zum Reaktionsgemisch zugetropft. Anschließend wurde der Ansatz mit Toluol (100 ml) versetzt. Die organische Phase wurde abgetrennt und mit gesättigter Natriumhydrogencarbonat-Lösung (3 × 70 ml), Wasser (3 × 50 ml) und Natriumchloridlösung (1 × 70 ml) gewaschen. Nach dem Trocknen mit $Na_2SO_4$ wurde das Lösungsmittel am Rotationsverdampfer abdestilliert und der Rückstand aus Methanol umkristallisiert. Das gewünschte Produkt konnte in einer Ausbeute von 10,7 g (83 %) als gelber Feststoff mit einem Schmelzpunkt von 75-80 °C gewonnen werden.

**4-Cyano-4-phenylcyclohexanon**

5-Cyano-2-oxo-5-phenylcyclohexancarbonsäure-methylester (7,71 g, 0,03 mol) wurde in 10-proz. $H_2SO_4$ und konzentrierter Essigsäure (240 ml) gelöst. Die Reaktionsmischung wurde 24 h bei 100°C gerührt. Der Verlauf der

Reaktion wurde durch DC verfolgt. Zur Aufarbeitung wurde der Ansatz unter Eiskühlung mit Wasser (400 ml) verdünnt und mit Ethylacetat (3 × 100 ml) extrahiert. Anschließend wurde die organische Phase gründlich mit Wasser (6 × 100 ml), gesättigter Natriumhydrogencarbonatlösung (10 × 100 ml) und gesättigter Natriumchloridlösung (1 × 100 ml) gewaschen. Nach dem Trocknen mit $Na_2SO_4$ wurde das Lösungsmittel am Rotationsverdampfer abdestilliert. Das gewünschte Produkt konnte in einer Ausbeute von 5,46 g (92 %) mit einem Schmelzpunkt von 106-107 °C isoliert werden.

**8-Cyano-8-phenyl-1,4-dioxaspiro[4.5]decan**

4-Cyano-4-phenylcyclohexanon (5,97 g, 30 mmol) wurde in Toluol (200 ml) aufgenommen und mit Ethylenglykol (4 ml, 71,6 mmol) versetzt. Nach Zugabe von *p*-Toluotsulfonsäure (86 mg, 0,5 mmol) wurde der Ansatz am Wasserabscheider zum Sieden erhitzt. Der Verlauf der Reaktion wurde DC-chromatographisch verfolgt. Nach 20 h war im DC kein Ausgangsprodukt mehr nachweisbar. Nach Abkühlung wurde die Toluol-Lösung mit Wasser (5 x 30 ml) und gesättigter wäßriger NaCl-Lösung (3 x 20 ml) ausgeschüttelt und über $Na_2SO_4$ getrocknet. Nach Entfernung des Lösungsmittels am Rotationsverdampfer fällt das gewünschte Ketal in einer Ausbeute von 6,8 g (94 %) als weißer Feststoff mit einem Schmelzpunkt von 108-110 °C an.

**8-Phenyl-1,4-dioxaspiro[4.5]decan-8-carbonsäure** (Schneider, Woldemar; Krombholz, Gottfried; ARPMAS; Arch.Pharm.(Weinheim Ger.); 313; 6; 1980; 487-498) 8-Cyano-8-phenyl-1,4-dioxaspiro[4.5]decan (4,86 g, 20 mmol) wurde in Ethylenglykol (40 ml) gelöst, mit NaOH (4 g, 100 mmol) versetzt und anschließend unter Rückfluß zum Sieden erhitzt. Der Reaktionsverlauf wurde mittels DC verfolgt. Nach 20 h war kein Nitril mehr nachweisbar. Zur Aufarbeitung wurde der Ansatz mit Eis (ca. 100 g) versetzt, mit Ether (40 ml) überschichtet und durch langsame Zugabe von halbkonzentrierter HCl (50 ml) angesäuert. Die wäßrige Phase wurde mit Ether extrahiert (3 x 30 ml). Die vereinigten organischen Extrakte wurden mit gesättigter $NH_4Cl$-Lösung gewaschen (2 × 30 ml), über $Na_2SO_4$ getrocknet und am Rotationsverdampfer zur Trockne gebracht. Durch Umkristallisation des anfallenden Feststoffs aus Toluol wurde die gewünschte Carbonsäure als kristalliner Feststoff mit einem Schmelzpunkt von 134-139 °C in einer Ausbeute von 3,1 g (59 %) gewonnen.

**8-Isocyanato-8-phenyl-1,4-dioxaspiro[4.5]decan**

8-Phenyl-1,4-dioxaspiro[4.5]decan-8-carbonsäure (3 g, 11,5 mmol) wurde in Anisol (30 ml) vorgelegt. Die erhaltene Suspension wurde im Eis-Kochsalzbad auf eine Temperatur von 0 °C gekühlt und mit Triethylamin (2,25 ml, 16 mmol) versetzt. Es entstand eine klare Lösung, die weitere 15 min bei 0 °C gerührt wurde. Anschließend wurde das Gemisch innerhalb von 5 min mit Phosphorsäurediphenylesterazid (2,5 ml, 11,5 mmol) versetzt. Das Reaktionsgemisch wurde 20 min bei 0 °C gerührt, innerhalb weiterer 20 min auf RT kommen gelassen und anschließend in einem Ölbad 2 h lang auf 100°C (Badtemperatur) erhitzt. Zur Aufarbeitung wurde das Anisol im Ölpumpenvakuum abdestilliert. Die chromatographische Reinigung erfolgte an Kieselgel mit Toluol. Das gewünschte Produkt wurde als kristalliner Feststoff mit einem Schmelzpunkt von 38-41 °C in einer Ausbeute von 2,7 g (91 %) erhalten.

**Methyl-(8-phenyl-1,4-dioxaspiro[4.5]dec-8-yl)amin**

$LiAlH_4$ (535 mg, 14,08 mmol) wurde unter Ausschluß von Luftfeuchtigkeit in trockenem THF (4 ml) suspendiert. Das 8-Isocyanato-8-phenyl-1,4-dioxaspiro[4.5]decan (2,29 g, 8,8 mmol, gelöst in 40 ml trockenem THF) wurde innerhalb von 20 min zugetropft. Nach vollständiger Zugabe wurde das Reaktionsgemisch 4 h unter Rückfluß zum Sieden erhitzt. Nach Abkühlung wurde das Reaktionsgemisch unter Eiskühlung zunächst vorsichtig mit wäßrigem THF (1 ml $H_2O$ in 3 ml), dann mit 1,7 ml 15-proz. Natronlauge und schließlich mit 5 ml $H_2O$ versetzt. Der Ansatz wurde 20 min gerührt und anschließend über Kieselgur filtriert. Das nach mehrmaligen Waschen des Filterkuchens mit Ethylacetat erhaltene Lösungsmittelgemisch wurde am Rotationsverdampfer zur Trockene eingeengt. Das gewünschte Produkt wurde in einer Ausbeute von 2,1 g (97 %) als viskoses Öl erhalten.

**4-Methylamino-4-phenylcyclohexanon (Ket-15)** (Upjohn_Lednicer, US4065573A1, 1977**)** Methyl-(8-phenyl-1,4-dioxaspiro[4.5]dec-8-yl)amin (2,1 g, 8,4 mmol) wurde mit einem Gemisch aus konz. HCl (15 ml) und Wasser (8 ml) übergossen und 5 Tage bei RT gerührt. Zur Aufarbeitung wurde das Reaktionsgemisch mit Wasser (20 ml) verdünnt und mit Ether (3 x 30 ml) extrahiert. Die etherische Phase wurde verworfen. Die wäßrige Phase wurde anschließend mit 2N NaOH basisch gemacht und mit Dichlormethan (3 × 30 ml) extrahiert. Die so erhaltene organische Phase wurde mit $Na_2SO_4$ getrocknet und anschließend am Rotationsverdampfer eingeengt. Das Keton Ket-15 konnte durch chromatographische Reinigung an Kieselgel mit Ethylacetat/Ethanol (4 : 1) in einer Ausbeute von 1,38 g (81 %) als Feststoff mit einem Schmelzpunkt von 32-38°C erhalten werden.

**Ketonbaustein Ket-16:**

**8-Azetidin-1-yl-1,4-dioxaspiro[4.5]decan-8-carbonitril**

Zu einer Mischung von 4 *N* Salzsäure (8.1 ml), Methanol (4.9 ml) und Azetidin (8.5 g, 10 ml. 149 mmol) wurde unter Eiskühlung zuerst 1,4-Dioxaspiro[4,5]decan-8-on (4.84 g, 31 mmol) und danach Kaliumcyanid (4.85 g, 74.4 mmol) in Wasser (15 ml) gegeben. Die Mischung wurde 5 d bei Raumtemperatur gerührt, dann mit Wasser (50 ml) versetzt und mit Diethylether (3 × 50 ml) extrahiert. Die vereinigten organischen Phasen wurden mit Natriumsulfat getrocknet und i. Vak. Eingeengt; *Ausbeute:* 6.77 g (98 %), Öl
$^1$*H-NMR (DMSO-*$d_6$*)*: 1.45-1.63 (m, 4H); 1.67-1.82 (m, 4H); 1.99 (q, 2H, J = 7.1 Hz); 3.21 (t, 4H, J = 7.1 Hz); 3.86 (s, 4H).

**1-(8-Phenyl-1,4-dioxaspiro[4.5]dec-8-yl)azetidin**

Eine 2 M Lösung von Phenylmagnesiumchlorid in Tetrahydrofuran (12 ml, 24 mmol) wurde unter Argon und Eiskühlung tropfenweise mit einer Lösung des soeben hergestellten Nitrils (2.20 g, 9.9 mmol) in wasserfreiem Tetrahydrofuran (25 ml) versetzt und danach bei Raumtemperatur über Nacht gerührt. Nach Zugabe von gesättigter Ammoniumchloridlösung (5 ml) und Wasser (5 ml) wurden die Phasen getrennt und die wässrige mit Diethylether (3 × 50 ml) extrahiert. Die vereinigten organischen Phasen wurden mit Natriumsulfat getrocknet und i. Vak. eingeengt. Das Rohprodukt wurde durch Flashchromatographie (100 g, 20 × 4.0 cm) mit Ethylacetat / Cyclohexan (1:1) gereinigt; *Ausbeute:* 670 mg (25 %), farbloses Öl $^1$*H-NMR* (*DMSO-*$d_6$):1.27-1.40 (m, 2H); 1.55-2.00 (m, 8H); 2.86 (t, 4H, J = 6.8 Hz); 3.76-3.89 (m, 4H); 7.24-7.45 (m, 5H).

**4-Azetidin-1-yl-4-phenylcyclohexanon (Ket-16)**

Eine Lösung des soeben hergestellten Acetals (370 mg, 1.3 mmol) in Aceton (30 ml) wurde mit 6 N Salzsäure (2 ml) versetzt und über Nacht bei Raumtemperatur gerührt. Durch Zugabe von 5 N Natronlauge wurde pH 10 eingestellt und die wässrige Phase mit Dichlormethan (3 × 20 ml) extrahiert. Die vereinigten organischen Phasen wurden mit Natriumsulfat getrocknet und i. Vak. eingeengt.
*Ausbeute:* 274 mg (92 %), weißer Feststoff **(Ket-16)**
$^1$*H-NMR (DMSO-*$d_6$*)*: 1.67 (td, 2H, J = 13.8, 6.9 Hz); 1.95-2.13 (m, 4H); 2.20-2.33 (m, 2H); 2.40-2.47 (m, 1H); 2.52-2.57 (m, 1H); 2.94 (t, 4H; J = 6.9 Hz); 7.28-7.47 (m, 5H).

**Ketonbaustein Ket-17:**

**1-(8-Pyrrolidin-1-yl-1,4-dioxaspiro[4.5]dec-8-yl)-1*H*-[1,2,3]triazol**

Zu einer Lösung von 1,4 Dioxaspiro[4,5]decan-8 on (3.9 g, 25 mmol) in Toluol (40 ml) wurden Pyrrolidin (1.95 g, 2.29 ml, 27.5 mmol), 1,2,3-Triazol (2.07 g, 30 mmol) und Molsieb 4 A (7.14 g) gegeben. Das Gemisch wurde 7 h bei 90°C gerührt. Anschließend wurde die Lösung dekantiert und sofort weiter umgesetzt.

**1-(8-Butyl-1,4-dioxaspiro[4.5]dec-8-yl)pyrrolidin**

Zu einer 2 *M* Lösung von n-Butylmagnesiumchlorid (25 ml, 50 mmol) in Tetrahydrofuran wurde unter Eiskühlung und Argon die Reaktionslösung soeben hergestellten Triazol
Derivates (ca. 6.9 g, 25 mmol) in Toluol (38 ml) getropft. Das Reaktionsgemisch wurde über Nacht bei Raumtemperatur gerührt und anschließend in gesättigte Ammoniumchloridlösung (60 ml) gegossen. Die Phasen wurden getrennt und die wässrige mit Diethylether (3 × 70 ml) extrahiert. Die vereinigten organischen Phasen wurden mit Natriumsulfat getrocknet, i. Vak. eingeengt und der Rückstand (12 g) durch Flashchromatographie (400 g, 20 × 7.6 cm) mit Ethylacetat / Methanol (9:1) gereinigt.
*Ausbeute:* 2.70 g (40 % über zwei Stufen), braunes Öl
$^1$*H-NMR (DMSO-*$d_6$*)*: 0.87 (t, 3H, J = 7.1 Hz); 1.12-1-29 (m, 4H); 1.30-1.45 (m, 4H); 1.46-1.60 (m, 4H); 1.61-1.75 (m, 6H); 1.93 (t, 1H, J = 7.1 Hz); 2.36 (t, 1H, J = 7.0 Hz), 2.58 (br s, 2H), 3.83 (s, 4H).

**4-Butyl-4-pyrrolidin-1-yl-cyclohexanon (Ket-17)**

Eine Lösung des soeben erhaltenen Acetals (2.70 g, 10.1 mmol) in Aceton (100 ml) wurde mit Wasser (10.0 ml) und 37 % Salzsäure (14.0 ml) versetzt und über Nacht bei Raumtemperatur gerührt. Zum Gemisch wurde anschließend langsam 4 *M* Natronlauge getropft, bis pH 10 erreicht war. Das Gemisch wurde mit Diethylether (4 x 40 ml)

extrahiert, die vereinigten organischen Phasen wurden mit Natriumsulfat getrocknet und i. Vak. eingeengt. Das Rohprodukt (2.6 g) wurde durch Flashchromatographie (260 g, 30 × 5.6 cm) mit Ethylacetat / Methanol (9:1) gereinigt.
*Ausbeute:* 1.06 g (47 %), braunes Öl (Ket-17)
$^1$*H-NMR (DMSO-$d_6$)*: 0.88 (t, 3H, J = 6.7 Hz); 1.14-1.34 (m, 4H); 1.40-1.50 (m, 2H); 1.62-1.88 (m, 8H); 2.04 (dt, 2H, J = 15.0, 3.9 Hz); 2.42 (ddd, 2H, J = 6.3, 11.8, 15.5 Hz); 2.63 (t, 4H, J = 6.0 Hz).

**Ketonbaustein Ket-18:**

***Variante 1:***

**Methyl-[8-(4-methyl-thiazol.2-yl)-1,4-dioxa-spiro[4.5]dec-8-yl]-amin Unter** Argonatmosphäre wurde Butyllithium (2.5 M in Hexan, 9.2 ml, 23.0 mmol) vorgelegt und im Kältebad auf -78°C abgekühlt. 4-Methyl-thiazol (2.09 ml, 23 mmol) wurde in abs. Tetrahydrofuran (60.0 ml) gelöst und tropfenweise unter Eiskühlung bei -78°C addiert und bei dieser Temperatur 10 min nachgerührt.
Zu dieser Lösung wurde 8-Methylamino-1,4-dioxa-spiro[4.5]decan-8-carbonitril (2.12 g, 10.8 mmol) in abs. Tetrahydrofuran (15 ml) bei -78°C schnell zugetropft. Nach der Zugabe wurde die Reaktionslösung 1 h im Kältebad nachgerührt und anschließend langsam auf 0 °C erwärmt. Das Reaktionsgemisch wurde bei Raumtemperatur über Nacht gerührt. Dann bei 0 °C mit Wasser (10 ml) hydrolysiert, die wässrige Phase mit Chloroform (3 x 50 ml) extrahiert, die organische Phase wurde mit Wasser (1 x 50 ml) und gesättigter NaCl-Lösung (50 ml) gewaschen, über $Na_2SO_4$ getrocknet und i. Vak. eingeengt.
Das Produkt wurde mittels Flash-Chromatographie mit Cyclohexan/Essigester (1:4) und Essigester gereinigt. *Ausbeute:* 2.71 g (94 %)
$^1$*H-NMR (DMSO-$d_6$)*: 1.54 (2 H, m); 1.82 (4 H, m); 2.02 (2 H, m); 2.08 (3 H, d); 2.32 (3 H, s); 2.38 (1 H, q); 3.86 (4 H, s); 7.09 (1 H, s).
$^{13}$*C-NMR (DMSO-$d_6$)*: 16.96; 28.92; 30.04; 33.03; 58.29; 63.48; 63.59; 107.67; 113.69; 151.07; 178.75.

**4-(Methylamino)-4-(4-methylthiazol-2-yl)cyclohexanon (Ket-18)** Methyl-[8-(4-methyl-thiazol-2-yl)-1,4-dioxa-spiro[4.5]dec-8-yl]-amin (100 mg, 0.37 mmol) wurde mit 5%-iger Schwefelsäure (6 ml) versetzt und bei RT über Nacht gerührt. Nach beendeter Hydrolyse wurde die Reaktionsmischung mit Ether (1 x 5 ml) extrahiert, die wässrige Lösung unter Eiskühlung mit 5N NaOH alkalisch gestellt, die Reaktionsmischung mit Dichlormethan (3 x 10 ml) extrahiert, die organische Phase über $Na_2SO_4$ getrocknet und i. Vak. eingeengt. *Ausbeute:* 74 mg (88 %), Öl.
$^{13}$*C-NMR (DMSO-$d_6$)*: 17.19; 29.52; 35.48; 37.00; 58.58; 113.06; 152.54; 176.03; 210.85.

*Variante 2:*

**Ethyl 5-cyano-5-(4-methylthiazol-2-yl)-2-oxocyclohexancarboxylat Eine Lösung von (4-**Methyl-thiazol-2-yl)-acetonitril (24.2 g, 0.175 mol) und Brom-propionsäure-ethylester (51.6 ml, 0.4 mol) in abs. Toluol (700 ml) wurde bei 0 - 5°C portionsweise mit Natriumamid (58.0 g, 1.48 mol) versetzt und anschließend 3 h unter Rückfluss gekocht. Die Reaktionslösung wurde auf 0 °C abgekühlt und mit Essigsäure/ Wasser (2/1, 240 ml) langsam hydrolysiert. Die organische Phase wurde abgetrennt, mit ges. $NaHCO_3$-Lösung (2 x 400 ml) und Wasser (2 x 400 ml) gewaschen, über Natriumsulfat getrocknet und i. Vak. eingeengt.
*Ausbeute:* 45.0 g (88 %), braunes Öl
$^1$*H-NMR (DMSO-$d_6$)*: 1.26 (3 H, t); 2.30-2.62 (4 H, m); 2.38 (3 H, s); 2.84 (1 H, d); 2.93 (1 H, d); 4.22 (2 H, q); 7.39 (1 H, s); 12.21 (1 H, s).

**1-(4-Methyl-thiazol-2-yl)-4-oxo-cyclohexancarbonitril** Ethyl 5-cyano-5-(4-methylthiazol-2-yl)-2-oxocyclohexancarboxylat (45.0 g, 0.153 mol) wurde in Essigsäure (1.23 L) und 10%-iger Schwefelsäure (540 ml) gelöst und 4 d unter Rückfluss gekocht.
Zur Aufarbeitung wurde der Ansatz unter Eiskühlung mit Wasser (850 ml) versetzt und mit Essigsäureethylester (dreimal 300 ml) extrahiert (zur besseren Phasentrennung wurde ges. NaCl-Lösung zugesetzt). Die organische Phase wurde mit Wasser (6 x 100 ml) gewaschen, mit ges. $NaHCO_3$-Lösung (1 L) 20 min durchgerührt, anschließend wurde die organische Phase ein weiteres mal mit ges. $NaHCO_3$-Lösung und ges. NaCl-Lösung gewaschen, über Natriumsulfat getrocknet und i. Vak. eingeengt.
*Ausbeute:* 13.3 g (39 %), hellbrauner Feststoff
$^1$*H-NMR (DMSO-$d_6$)*: 2.38-2.69 (11 H, m); 7.39 (1 H, s).
$^{13}$*C-NMR (DMSO-$d_6$)*: 16.68; 34.88; 37.32; 41.43; 115.76; 120.09; 152.37; 166.26: 206.05.

**8-(4-Methyl-thiazol-2-yl)-1,4-dioxa-spiro[4.5]decan-8-carbonitril**1-(4-Methyl-thiazol-2-yl)-4-oxo-cyclohexancarbonitril (13.3 g, 61 mmol) und Ethylenglykol (6.8 ml, 122 mmol) wurden in Toluol (250 ml) gelöst, mit einer katalytischen Menge an p-Toluolsulfonsäure versetzt und am Wasserabscheider 3 h gekocht.
Zur Aufarbeitung wurde die organische Lösung mit Wasser (125 ml), ges. $NaHCO_3$-Lösung, Wasser und ges. NaCl-Lösung gewaschen, über Natriumsulfat getrocknet und i. Vak. eingeengt. *Ausbeute:* 15.8 g (99 %), hellbraunes Öl
$^1$*H-NMR (DMSO-$d_6$)*: 1.80 (4 H, m); 2.11-2.42 (7 H, m); 4.22 (4 H, s); 7.35 (1 H, s).

**8-(4-Methyl-thiazol-2-yl)-1,4-dioxa-spiro[4.5]decan-8-carbonsäharnstoffmid8**-(4-Methyl-thiazol-2-yl)-1,4-dioxa-spiro[4.5]decan-8-carbonitril (15.2 g, 58 mmol), gelöst in Ethanol (200 ml), wurde mit einer Lösung von KOH (12.9 g, 230 mmol) in Wasser (200 ml) versetzt und bei RT 48 h gerührt. Da laut DC (EE/Cyclohexan 4:1) noch Edukt vorhanden war, wurde der Ansatz bei 80 °C noch 3 h nachgerührt.
Zur Aufarbeitung wurde i. Vak. Ethanol entfernt, aus der wässrigen Lösung fiel ein Feststoff aus, dieser wurde abgetrennt und mit halbkonzentrierter Essigsäure kurz aufgekocht, abgekühlt und abgesaugt.
*Ausbeute:* 6.5 g (40%), farblose Kristalle; *Schmelzpunkt:* 167-170 °C
$^1$*H-NMR (DMSO-$d_6$)*: 1.61 (4 H, m); 2.10 (2 H, m); 2.38 (5 H, m); 3.91 (4 H, s); 7.12 (3 H, m).
$^{13}$*C-NMR (DMSO-$d_6$)*: 16.84; 31.57; 31.92; 34.12; 50.93; 66.65; 107.17; 114.11; 151.12; 172.27; 173.14.

**Methyl 8-(4-methylthiazol-2-yl)-1,4-dioxaspiro[4.5]decan-8-ylcarbamat** 8-(4-Methyl-thiazol-2-yl)-1,4-dioxa-spiro[4.5]decan-8-carbonsäharnstoffmid (2.82 g, 10 mmol) und Quecksilber(II)-acetat (4.58 g, 12 mmol) wurden in absol. DMF (50 ml) gelöst. Bei RT wurden Methanol (12 ml, 300 mmol) und eine Lösung von N-Bromsuccinimid (1.96 g, 11 mmol) in absol. DMF (15 ml) zugegeben und 18 h gerührt.
Zur Aufarbeitung wurde i. Vak. das Lösungsmittel entfernt und der feste Rückstand mit Ether (4 x 50 ml) extrahiert. Die organische Phase wurde eingeengt und das Produktgemisch durch Flash-Chromatographie mit EE/Cyclohexan (1:2) getrennt. *Ausbeute:* 541 mg (19 %)
$^1$*H-NMR (DMSO-$d_6$)*: 1.66 (2 H, m); 1.72 (2 H, m); 2.03 (2 H, m); 2.25 (5 H, m); 3.57 (3 H, s); 3.87 (4 H, s); 7.09 (1 H, s).
$^{13}$*C-NMR (DMSO-$d_6$)*: 16.84; 31.57; 31.92; 34.12; 50.93; 66.65; 107.17; 114.11; 151.12; 172.27; 173.14.

**Methyl-[8-(4-methyl-thiazol-2-yl)-1,4-dioxa-spiro[4.5]dec-8-yl]-amin** Eine Lösung von Methyl 8-(4-methyl-thiazol-2-yl)-1,4-dioxaspiro[4.5]decan-8-ylcarbamat (520 mg, 1.7 mmol) in absol. THF (20 ml) wurde portionsweise mit $LiAlH_4$ (125 mg, 3.3 mmol) versetzt und 5 h unter Rückfluss gekocht. Das Reaktionsgemisch wurde bei 0 °C mit ges. $Na_2SO_4$-Lösung hydrolysiert, über Celite filtriert, mit THF gewaschen und i. Vak. eingeengt. Der Rückstand wurde in EE aufgenommen, mit Wasser gewaschen und über Natriumsulfat getrocknet. Die Reinigung erfolgte durch Flash-Chromatographie mit EE/Cyclohexan (4:1). *Ausbeute:* 106 mg (24 %), Öl
$^1$*H-NMR (DMSO-$d_6$)*: 1.55 (2 H, m); 1.82 (4 H, m); 1.98 (2 H, m); 2.05 (3 H, s); 2.32 (3 H, s); 3.86 (4 H, s); 7.10 (1 H, s).

**<u>Indolbausteine</u>**

**<u>Indol Building Block Vorstufen:</u>**

**3-(2-Brom-ethyl)-5-fluor-1*H*-indol**

2-(5-Fluor-1 H-indol-3-yl)-ethanol (20.0 g, 112 mmol) wurde in abs. $CH_2Cl_2$ (250 ml) gelöst und bei RT mit Tetrabrommethan (56.0 g, 170 mmol) versetzt. Anschließend wurde unter Wasserkühlung bei RT portionsweise Triphenylphosphin (44.0 g, 165 mmol) zugegeben. Die Lösung wurde 2.5 h bei RT gerührt und anschließend i. Vak. eingeengt. Der Rückstand wurde auf Kieselgel aufgezogen und in zwei gleiche Teile geteilt. Das Rohprodukt wurde durch Flash-Chromatographie über 2 Säulen mit je 500 g Kieselgel und Cyclohexan/Essigester (9:1→4:1) gereinigt. *Ausbeute:* 25.4 g (93 %), roter Feststoff $^1$*H-NMR (DMSO-$d_6$)*: 3.18 (2 H, t); 3.69 (2 H, t); 6.91 (1 H, m); 7.32 (3 H, m); 11.03 (1 H, s).
$^{13}$*C-NMR (DMSO-$d_6$)*: 28.36; 34.22; 102.83: 108.96; 111.96; 125.55; 126.95; 132.74; 155.60; 157.90.

**<u>Indolbaustein Ind-1:</u>**

**2-(2-(1H-Indol-3-yl)ethyl)isoindolin-1,3-dion (Ind-1)**

Tryptamin (3.04 g, 19.0 mmol) und Phthalsäharnstoffnhydrid (3.00 g, 20.2 mmol) wurden 12 h im Wasserabscheider gekocht. Das Lösungsmittel wurde i. Vak. abdestilliert und der Rückstand in Methylenchlorid gelöst. Durch Zugabe von Cyclohexan wurde Produkt ausgefällt, der Niederschlag abgesaugt und getrocknet. *Ausbeute:* 4.46 g **(Ind-1;** 87 %)
$^1$*H-NMR (DMSO-$d_6$)*: 3.03 (2 H, t); 3.85 (2 H, t); 7.04 (2 H, m); 7.18 (1 H, s); 7.34 (1 H, d); 7.54 (1 H, d); 7.83 (4 H, m); 10.83 (1 H, s).

**<u>Indolbaustein Ind-2:</u>**

**N-(2-(1 H-Indol-3-yl)ethyl)acetamid (Ind-2)**

Tryptamin (192 mg/ 1.2 mmol) wurde in abs. THF (5 ml) vorgelegt und mit Triethylamin (179 µL/ 1.3 mmol)

versetzt. Anschließend wurde Essigsäharnstoffnhydrid (132 mg/ 1.3 mmol) zugegeben und 4 h bei RT gerührt. Der Ansatz wurde i. Vak. bis zur Trockene eingeengt. Der Rückstand wurde in Essigester aufgenommen und diese Lösung mit gesättigter $NaHCO_3$-Lösung (zweimal 20 ml) und mit NaCl-Lösung (zweimal 20 ml) gewaschen. Die organische Phase wurde über $Na_2SO_4$ getrocknet und i. Vak. eingeengt.
*Ausbeute:* 236 mg (Ind-2; 97 %)
$^1$*H-NMR (DMSO-$d_6$)*: 1.81 (3 H, s); 2.83 (2 H, m); 3.33 (2 H, m); 6.96 (1 H, m); 7.05 (1 H, m); 7.14 (1 H, s); 7.35 (1 H, m); 7.53 (1 H, d); 7.92 (1 H, t, NH); 10.79 (1 H, s).
$^{13}$*C-NMR (DMSO-d*6): 22.65; 25.19; 33.11; 39.50; 111.31; 111.86; 118.16; 120.84; 122.52; 127.21; 136.21; 168.99.

**N-(2-(1H-Indol-3-yl)ethyl)acetamid (Ind-2)**

Tryptamin (192 mg/ 1.2 mmol) wurde in abs. THF (5 ml) vorgelegt und mit Triethylamin (179 µL/ 1.3 mmol) versetzt. Anschließend wurde Essigsäharnstoffnhydrid (132 mg/ 1.3 mmol) zugegeben und 4 h bei RT gerührt. Der Ansatz wurde i. Vak. bis zur Trockene eingeengt. Der Rückstand wurde in Essigester aufgenommen und diese Lösung mit gesättigter $NaHCO_3$-Lösung (zweimal 20 ml) und mit NaCl-Lösung (zweimal 20 ml) gewaschen. Die organische Phase wurde über $Na_2SO_4$ getrocknet und i. Vak. eingeengt.
*Ausbeute:* 236 mg (Ind-2, 97 %)
$^1$*H-NMR (DMSO-$d_6$):* 1.81 (3 H, s); 2.83 (2 H, m); 3.33 (2 H, m); 6.96 (1 H, m); 7.05 (1 H, m); 7.14 (1 H, s); 7.35 (1 H, m); 7.53 (1 H, d); 7.92 (1 H, t, NH); 10.79 (1 H, s).
$^{13}$*C-NMR (DMSO-$d_6$):* 22.65; 25.19; 33.11; 39.50; 111.31; 111.86; 118.16; 120.84; 122.52; 127.21; 136.21; 168.99.

**Indolbaustein Ind-4:**

**(5-Fluor-3-hydroxy-2-oxo-2,3-dihydro-1H-indol-3-yl)essigsäure-ethylester 5-**Fluorindolin-2,3-dion (10 mmol) wurde in einem Gemisch aus Ethanol/Pyridin/Essigsäure (50 ml, 15 : 5 : 2) gelöst, mit Ethylkaliummalonat (1,87 g, 11 mmol) versetzt und entsprechend 14 h unter Rückfluß erhitzt. Der Verlauf der Reaktion wurde mittels DC (Eluent: Ethylacetat/Hexan 1 : 1) kontrolliert. Zur Aufarbeitung wurde das Lösungsmittelgemisch im Vakuum abdestilliert. Der Rückstand wurde in Ethylacetat (50 ml) aufgenommen und mit Wasser (50 ml) ausgeschüttelt. Nach Phasentrennung wurde die wäßrige Phase zweimal mit Ethylacetat (je 30 ml) extrahiert. Die kombinierten organischen Phasen wurden mit 2N HCl (50 ml) gewaschen, über $Na_2SO_4$ getrocknet und im Vakuum auf 20 ml eingeengt. Zu der Lösung wurde solange Hexan zugegeben, bis die Kristallisation einsetzte. Zur Vervollständigung der Kristallisation wurde der Ansatz 12 h auf 10°C gekühlt. Der Feststoff wurde abgesaugt und im Vakuum getrocknet (S. J. Garden, R. B. da Silva, A. C. Pinto, Tetrahedron 2002, 58, 8399-8412 (speziell Seite 8406)).
*Ausbeute* (5-Fluor-3-hydroxy-2-oxo-2,3-dihydro-1H-indol-3-yl)essigsäure-ethylester: 89 % *Schmelzpunkt:* 133-135°C

**2-(5-Fluor-1 H-indol-3-yl)ethanol (Ind-4)[1]**

(5-Fluor-3-hydroxy-2-oxo-2.3-dihydro-1 H-indol-3-yl)essigsäure-ethylester (10 mmol) wurde unter Argon-Atmosphäre in absolutem THF (20 ml) gelöst. Der Ansatz wurde anschließend unter Wasserbadkühlung mit $BH_3 \times THF$ (40 ml, 1 M Lösung, 40 mmol) versetzt und bei Raumtemperatur 14 h gerührt. Der Reaktionsverlauf wurde mittels DC überwacht. Nach Beendigung der Reaktion wurde die Reaktionslösung zu einer Mischung aus Ethylacetat (50 ml) und $H_2O$ (50 ml) gegeben. Nach Phasentrennung wurde die wäßrige Phase zweimal mit Ethylacetat (je 30 ml) extrahiert. Die kombinierten organischen Phasen wurden über $Na_2SO_4$ getrocknet und im Vakuum eingeengt. Der Rückstand wurde über Kieselgel mit Ethylacetat filtriert. Das nach Entfernen des Lösungsmittels erhaltene Produkt lag als ausreichend reines Öl vor und kristallisierte spontan.
Ausbeute (Ind-4): 95 %; *Schmelzpunkt:* 47-50°C.

**Indolbaustein Ind-5: 2-(1H-indol-3-yl)ethanol (Ind-5)**

*Kommerziell erhältlich unter CAS 526-55-6 z.B. bei Sigma-Aldrich*

**Indolbaustein Ind-6:**

**3-Methyl-2-trimethylsilanyl-1*H*-indol-5-carbonitril**

1-(Trimethylsilyl)propin (2,23 ml, 1,68 g, 15,0 mmol), 4-Amino-3-iodbenzonitril (3,33 g, 13,65 mmol), Lithiumchlorid (606 mg, 14,3 mmol) und Natriumcarbonat (4,35 g, 40,95 mmol) wurden unter Argon nacheinander in abs. Dimethylformamid (60 ml) gegeben. Zu dieser Mischung wurde Pd(dppf)$Cl_2$ (1,116 g, 1,365 mmol) hinzugefügt, die

Reaktionsmischung 6 h unter Feuchtigkeitsausschluß auf 100°C erwärmt und anschließend 16 h bei Raumtemperatur gerührt. Zur Aufarbeitung des Ansatzes wurde Wasser (150 ml) und Ethylacetat (300 ml) hinzugefügt und 10 min gerührt. Nach Phasentrennung wurde die wäßrige Phase mit Ethylacetat (3 × 100 ml) extrahiert. Die vereinigten organischen Phasen wurden mit gesättigter Natriumchlorid-Lösung (3 × 100 ml) gewaschen, getrocknet und eingeengt. Der Rückstand war ein braunes Öl (5,5 g), das außer dem 3-Methyl-2-trimethylsilanyl-1*H*-indol-5-carbonitril Spuren des Isomers 2-Methyl-3-trimethytsilanyl-1*H*-indol-5-carbonitril und DMF enthielt.

**3-Methyl-1*H*-indol-5-carbonitril (Ind-6)**

Die Abspaltung der Trimethylsilylgruppe wurde in zwei Stufen durchgeführt. Das Gemisch der Nitrile aus der vorherigen Stufe (5,15 g, Rohprodukt) wurde in Tetrahydrofuran (60 ml) gelöst, mit Tetrabutylammoniumfluorid × 3 $H_2O$ (5,58 g, 17,7 mmol) versetzt und 4 h bei Raumtemperatur gerührt. Die Reaktionsmischung wurde mit Wasser (150 ml) versetzt und 10 min gerührt. Nach der Zugabe von Diethylether (50 ml) wurden die Phasen getrennt. Die wäßrige Phase wurde mit Diethylether (3 × 150 ml) extrahiert. Die vereinigten organischen Phasen wurden getrocknet und eingeengt. Der Rückstand war ein braunes Öl (3,4 g) das chromatographisch aufgetrennt wurde [Kieselgel 60 (170 g); Ethylacetat/ Cyclohexan (1 :10 ,1600 ml), Ethylacetat/Cyclohexan (1 : 4, 500 ml)]. Dabei wurde 3-Methyl-2-trimethylsilanyl-1*H*-indol-5-carbonitril als beigefarbener Feststoff in einer Ausbeute von 60 % (1,92 g) mit einem Schmelzpunkt von 128-131 °C gewonnen. Es wurde weiterhin ein Produktgemisch der Indol 3-Methyl-1 H-indol-5-carbonitril und 2-Methyl-1 H-indol-5-carbonitril (264 mg) isoliert, die nahezu in einem Verhältnis von 1 : 1 vorlagen. Die Silylverbindung 3-Methyl-2-trimethylsilanyl-1H-indol-5-carbonitril (1,9 g, 8,32 mmol) wurde in Tetrahydrofuran (60 ml) gelöst und 2 h auf 60°C erwärmt. Anschließend wurde der Ansatz eingeengt, mit Wasser (150 ml) versetzt und 10 min gerührt. Nach Zugabe von Diethylether (100 ml) wurden die Phasen getrennt. Die wäßrige Phase wurde mit Diethylether (2 × 50 ml) extrahiert. Die organischen Phasen wurden getrocknet und eingeengt. Das 3-Methyl-1H-indol-5-carbonitril (Ind-6) wurde dabei als beigefarbener Feststoff in einer Ausbeute von 99 % (1,33 g) mit einem Schmelzpunkt von 110-112 °C erhalten.

**<u>Indolbaustein Ind-7:</u>**

**3-Methyl-5-trifluormethyl-2-trimethylsilanyl-1*H*-indol2-Iod-4-trifluormethylanilin** (1,15 g, 4 mmol), Trimethylsilylpropin (494 mg, 0,656 ml, 4,4 mmol,), Lithiumchlorid (178 mg, 4,2 mmol) und Natriumcarbonat (1,27 g, 12 mmol) wurden in abs. Dimethylformamid (20 ml) in einer Argon-Atmosphäre vereinigt. Anschließend wurde (Pd(dppf)$Cl_2$ × $CH_2Cl_2$); 327 mg, 0,4 mmol) hinzugegeben. Die Reaktionsmischung wurde 6 h bei 100°C (Ölbadtemperatur) und 18 h bei Raumtemperatur gerührt. Anschließend wurde die Reaktionsmischung mit Eiswasser gekühlt, mit Wasser (50 ml) und Ethylacetat (100 ml) versetzt und 30 min gerührt. Um den Katalysator abzutrennen, wurde die dunkelbraune Mischung über Celite filtriert. Die Phasen des Filtrats wurden getrennt. Die wäßrige Phase wurde mit Ethylacetat (5 × 35 ml) extrahiert.

Die vereinigten organischen Phasen wurden mit gesättigter Natriumchlorid-Lösung (3 × 35 ml) gewaschen, anschließend mit Natriumsulfat getrocknet, filtriert und im Vakuum eingeengt. Das erhaltene dunkelbraune Öl(1,8 g), das außer neben 3-Methyl-5-trifluormethyl-2-trimethylsilanyl-1*H*-indol Spuren von DMF und auch das Isomer 2-Methyl-5-trifluormethyl-3-trimethylsilanyl-1*H*-indol enthielt, wurde als Rohprodukt für die nächste Stufe eingesetzt.

**3-Methyl-5-trifluormethyl-1*H*-indol (Ind-7)**

Die Abspaltung der Trimethylsilylgruppe erfolgte in zwei Stufen. Das Rohprodukt aus der vorherigen Stufe (1,8 g, 4 mmol, bezogen auf die Vorstufe) wurde mit THF (20 ml) und Tetrabutylammoniumfluorid (1,64 g, 5,5 mmol) 5 h bei RT gerührt. Dann wurde der Ansatz aufgearbeitet. Nach Zugabe von Wasser (20 ml) wurde die Mischung 15 min gerührt. Anschließend wurden die Phasen getrennt. Die wäßrige Phase wurde mit Diethylether (3 × 30 ml) extrahiert. Die vereinigten organischen Phasen wurden mit Natriumsulfat getrocknet, filtriert und im Vakuum eingeengt. Es wurden 1,8 g eines zähen dunkelbraunen Öl erhalten, das durch Chromatographie [Kieselgel 60 (60 g); Cyclohexan/Ethylacetat (15 : 1; 500 ml, 10 : 1; 500 ml)] in seine Bestandteile aufgetrennt wurde. Es wurden 763 mg (2,8 mmol, 70 % bezogen auf die vorherige Stufe) 3-Methyl-5-trifluormethyl-2-trimethylsilanyl-1H-indolerhalten. Diese wurde erneut mit THF (20 ml) und Tetrabutylammoniumfluorid (1,15 g, 3,6 mmol) versetzt und 2 h zum Rückfluß erhitzt. Nach zuvor beschriebener Aufarbeitung wurden 550 mg (69 %) 3-Methyl-5-trifluormethyl-1*H*-indol **(Ind-7)** als beigefarbener Feststoff mit einem Schmelzpunkt von 63-65 °C erhalten.

**<u>Indolbaustein Ind-8:</u> 5-Fluor-3-methylindol (Ind-8)**

*Kommerziell erhältlich unter CAS-Nr.* 392-13-2 *bei z.B. Chempur*

**<u>Indolbaustein Ind-9:</u>**

**5-Methoxy-3-methyl-1*H*-indol (5-Methoxyskatol)(Ind-9) 4-Methoxyphenylhydrazin**

Hydrochlorid (3,5 g, 20 mmol) wurde in $H_2O$ (100 ml) gelöst und mit Natriumcarbonat (2,1 g, 20 mmol) versetzt. Der Ansatz wurde gerührt, wobei nach vorrübergehendem Ausfallen der freien Base innerhalb weniger Minuten eine klare Lösung entstand. Zu dem Ansatz wurde Propionaldehyd (0,98 g, 16,8 mmol) und Ethanol (5 ml als Lösungsvermittler) gegeben. Die Mischung wurde 14 h bei RT gerührt, wobei aus der Lösung ein öliger Niederschlag ausfiel. Zur Aufarbeitung wurde der Ansatz mit Ethylacetat (3 x 20 ml) extrahiert. Die vereinigten organischen Phasen wurden über $MgSO_4$ getrocknet und anschließend am Rotationsverdampfer bis zur Trockene eingeengt. Der erhaltene braune Rückstand (3,2 g) wurde ohne weitere Reinigung in DMF (20 ml) gelöst, auf 110°C (Badtemperatur) erwärmt und mit 12-proz. Schwefelsäure (10 ml) versetzt. Es fand eine sofortige Entfärbung statt. Zur Vervollständigung der Reaktion wurde der Ansatz weitere 3 h bei 110 °C gerührt und anschließend über Nacht bei RT stehen gelassen - Zur Aufarbeitung wurde die Reaktionslösung auf Eis (100 g) gegeben. Die entstandene wäßrige Mischung wurde mit Ethylacetat (4 x 20 ml) extrahiert, die vereinigten organischen Phasen wurden über $MgSO_4$ getrocknet. Der nach Abdampfen des Lösungsmittels erhaltene Rückstand (3,3 g) wurde chromatographisch [Kieselgel 60 (100 g); Cyclohexan/EtOAc 4 : 1 (300 ml)] gereinigt. 5-Methoxy-3-methyl-1H-indol (Ind-9) wurde so in einer Ausbeute von 2,3 g (85 %) als klebriger Feststoff erhalten.

**<u>Indolbaustein Ind-10:</u> 3-Methyl-1H-indol (Ind-10)**

*Kommerziell erhältlich unter CAS 83-34-1 bei z.B. Sigma Aldrich*

**<u>Indolbaustein Ind-11:</u>**

**3-Cyclopropyl-2-trimethylsilanyl-1*H*-indolCyclopropylethinyl)trimethylsilan (1,38 g, 10,0** mmol), 2-Iodanilin (1,94 g, 9,1 mmol), Lithiumchlorid (404 mg, 9,54 mmol) und Natriumcarbonat (2,9 g, 27,3 mmol) wurden unter Argon nacheinander in abs. Dimethylformamid (40 ml) gegeben. Zu dieser Mischung wurde der Pd(dppf)$Cl_2$ (744 mg, 0,91 mmol) hinzugefügt. Die Reaktionsmischung wurde 6 h unter Feuchtigkeitsausschluß auf 100 °C erwärmt und anschließend 16 h bei Raumtemperatur gerührt. Zur Aufarbeitung des Ansatzes wurden Wasser (100 ml) und Ethylacetat (200 ml) hinzugefügt. Dann wurde 10 min gerührt. Die Mischung wurde über Celite filtriert. Nach Phasentrennung wurde die wäßrige Phase mit Ethylacetat (3 $\times$ 70 ml) extrahiert. Die vereinigten organischen Phasen wurden mit gesättigter Natriumchlorid-Lösung (3 $\times$ 70 ml) gewaschen, mit Natriumsulfat getrocknet und eingeengt. Der Rückstand war ein braunes Öl (3,6 g), das noch Spuren von Nebenprodukten und Dimethylformamid enthielt. Nach chromatographischer Reinigung [Kieselgel 60 (120 g); Ethylacetat/Cyclohexan 1 : 20 (700 ml)] wurde das 3-Cyclopropyl-2-trimethylsilanyl-1H-indol als beigefarbener Feststoff in einer Ausbeute von 75 % (1.56 g) mit einem Schmelzpunkt von 78-81 °C gewonnen.

**3-Cyclopropyl-1*H*-indol (Ind-11)** 3-Cyclopropyl-2-trimethylsilanyl-1H-indol (1,56 g, 6,8 mmol) wurde in Tetrahydrofuran (40 ml) gelöst, mit Tetrabutylammoniumfluorid $\times$ 3 $H_2O$ (2,79 g, 8,84 mmol) versetzt und 2 h bei 70 °C gerührt. Die Reaktionsmischung wurde eingeengt, mit Wasser (90 ml) und mit Diethylether (70 ml) versetzt und 10 min gerührt. Die Phasen wurden getrennt. Die wäßrige Phase wurde mit Diethylether (2 $\times$ 50 ml) extrahiert. Die vereinigten organischen Phasen wurden mit Natriumsulfat getrocknet und eingeengt. Ind-11 wurde als braunes Öl in einer Ausbeute von 99 % (1,04 g) isoliert.

**<u>Indolbaustein Ind-12:</u>**

**(3-Cyclohexylprop-1-inyl)trimethylsilanProp-2-ynylcyclohexan** (5,00 g, 40,9 mmol) wurde in Tetrahydrofuran (60 ml) bei -25°C vorgelegt und n-Butyllithium (17,2 ml, 43,0 mmol; 2,5M in Hexan) hinzugetropft. Die Temperatur wurde bei -15 bis -20°C gehalten (ca. 5 Minuten). Anschließend wurde die Reaktionsmischung bei 0 bis -5°C 30 min gerührt. Danach wurde bei 0 bis -5°C Triethylchlorsilan (6,6 g, 43,8 mmol) zugetropft (ca. 5 min) und anschließend über Nacht bei Raumtemperatur nachgerührt. Die Reaktionsmischung wurde im Vakuum eingeengt und der Rückstand mit Wasser (50 ml) versetzt. Die Mischung wurde mit Cyclohexan extrahiert (3 $\times$ 30 ml). Die vereinigten organischen Phasen wurden mit Natriumsulfat getrocknet. Die flüchtigen Bestandteile wurden im Vakuum vollständig entfernt. (3-Cyclohexylprop-1-inyl)trimethyisilan wurde als gelbes Öl gewonnen (9,61 g, 99 %).

**3-Cyclohexylmethyl-2-triethylsilanyl-1H-indol2**-Iodanilin (5,48 g, 25,02 mmol), (3-Cyclohexylprop-1-inyl)trimethylsilan (6,50 g, 27,45 mmol), Lithiumchlorid (1,11 g, 26,19 mmol) und Natriumcarbonat (7,95 g, 75,01 mmol) wurden in Dimethylformamid (absolut, 70 ml) in einer Argonatmosphäre vereinigt. Anschließend wurde der Katalysator ([Pd (dppf)$Cl_2$ x $CH_2Cl_2$)], 2,05 g, 2,51 mmol) hinzugegeben. Die Lösung wurde 6 h bei 100-106°C gerührt. Zu der auf Raumtemperatur abgekühlten schwarzen Reaktionsmischung wurden nacheinander Wasser (300 ml) und Ethylacetat (150 ml) gegeben. Nach einstündigem Rühren wurde die Mischung über Celite filtriert. Die Phasen wurden getrennt. Die wäßrige Phase wurde mit Ethylacetat (3 × 50 ml) extrahiert. Die vereinigten organischen Phasen wurden mit Natriumsulfat getrocknet. Nach Filtration wurden die flüchtigen Bestandteile vollständig im Vakuum entfernt. Der Rückstand (10,8 g, braunes Öl) wurde chromatographisch getrennt [Kieselgel 60 (300 g); Cyclohexan/Ethylacetat 20 : 1 (1050 ml), Cyclohexan/Ethylacetat 10: 1 (500 ml), Cyclohexan/Ethylacetat 3 : 1 (750 ml)]. 3-Cyclohexylmethyl-2-triethylsilanyl-1*H*-indol wurde als braunes Öl (5,79 g, 71 %) isoliert.

**3-Cyclohexylmethyl-1*H*-indol (Ind-12)** Zu einer Lösung von 3-Cyclohexylmethyl-2-triethylsilanyl-1*H*-indol (5,70 g, 17,40 mmol) in MeOH (106 ml) wurde 5N Salzsäure (20 ml, 100 mmol) zudosiert. Das Reaktionsgemisch wurde über Nacht bei Raumtemperatur gerührt. Methanol wurde abdestilliert, der wäßrige Rückstand wurde mit Dichlormethan (3 × 20 ml) extrahiert. Die vereinigten organischen Phasen wurden mit Natriumsulfat getrocknet. Nach Filtration wurden die flüchtigen Bestandteile vollständig im Vakuum entfernt. Der feste Rückstand (brauner Feststoff, 4,50 g) wurde aus Hexan (15 ml) umkristallisiert. Es wurden 2,70 g (73 %) 3-Cyclohexylmethyl-1*H*-indol (Ind-12)*(Schmelzpunkt:* 71-73 °C) erhalten.

__Indolbaustein Ind-13:__

**3-Propyl-2-trimethylsilanyl-1*H*-indol**

2-lodanilin (4,65 g, 21,2 mmol), Ttrimethyl(pent-1-inyl)silan (3,27 g, 23,3 mmol). Lithiumchlorid (0,96 g, 22,6 mmol) und Natriumcarbonat (6,75 g, 63,7 mmol wurden in Dimethylformamid (absolut, 64 ml) in einer Argonatmosphäre vereinigt. Anschließend wurde der Katalysator ([Pd(dppf)$Cl_2$ x $CH_2Cl_2$], 1,78 g, 2,2 mmol) hinzugegeben. Die Lösung wurde 7 h bei 105-112 °C (Ölbadtemperatur) gerührt. Zu der auf Raumtemperatur abgekühlten schwarzen Reaktionsmischung wurden nacheinander Wasser (200 ml) und Ethylacetat (200 ml) gegeben. Nach einstündigem Rühren wurde die Mischung über Celite filtriert. Die Phasen wurden getrennt. Die organische Phase wurde mit 10-proz.Citronensäurenlösung (100 ml) und mit gesättigter Natriumchlorid-Lösung (100 ml) gewaschen. Die organische Phase wurde mit Natriumsulfat getrocknet. Nach Filtration wurden die flüchtigen Bestandteile vollständig im Vakuum entfernt. Der Rückstand (7,03 g braunes Öl) wurde chromatographisch getrennt [Kieselgel 60 (150 g); Cyclohexan/Trichlormethan 10: 1 (550 ml); Cyclohexan/Trichlormethan 5 : 1 (1650 ml)]. Es wurde 2,35 g (50 %) 3-Propyl-2-trimethylsilanyl-1 H-indol als braunes Öl isoliert (verunreinigt)..

**3-Propyl-1*H*-indol (Ind-13)** 3-Propyl-2-trimethylsilanyl-1*H*-indol (3,76 g, 16,25 mmol) wurde in MeOH (70 ml) gelöst und 2N Salzsäure (45 ml, 90 mmol) zudosiert. Das Reaktionsgemisch wurde über Nacht bei Raumtemperatur gerührt. Methanol wurde abdestilliert, der wäßrige Rückstand wurde mit Dichlormethan (3 × 20 ml) extrahiert. Die vereinigten organischen Phasen wurden mit Natriumsulfat getrocknet. Nach Filtration wurden die flüchtigen Bestandteile vollständig im Vakuum entfernt. Es wurden 2,66 g (140 %) 3-Propyl-1*H*-indol **(Ind-13)** als braunes Öl erhalten.

__Indolbaustein Ind-14:__

**3-(2-(Pyridin-4-yl)ethyl)-1H-indol (Ind-14)**

Indol (5,85 g, 50 mmol) wurde zusammen mit 4-Vinylpyridin (5,80 g, 55 mmol) in Eisessig (25 ml) gelöst. Das Reaktionsgemisch wurde 6 h unter Rückfluß gekocht. Zur Aufarbeitung wurde der Eisessig abdestilliert. Anschließend wurde der Ansatz mit gesättigter $NaHCO_3$-Lösung (75 ml) und Wasser (15 ml) versetzt. Das Gemisch wurde mit Ethylacetat (1 ×
100 ml, 3 x15 ml) extrahiert. (Das zwischen den Phasen bleibende unlösliche Öl enthielt nur polare Verunreinigungen.) Die organische Phase wurde über $Na_2SO_4$ getrocknet und anschließend eingedampft. Der Rückstand wurde zweimal aus Ethylacetat (je 30 ml) umkristallisiert. Das Produkt **Ind-14** wurde als hellgelber Feststoff in einer Ausbeute von 7,06 g (31,8 mmol, 63 %, Schmp.: 154-158 °C) erhalten.

__Indolbaustein Ind-15:__

**3-(1*H*-Indol-3-yl)propansäurecyanmethylester**

Die 3-(1 H-indol-3-yl)propansäure (5 g, 26 mmol) wurde in Aceton (50 ml) gelöst und nacheinander mit Caesiumcarbonat (4,2 g, 13,0 mmol), Chloracetonitril (1,8 ml, 28,6 mmol) und Kaliumjodid (20 mg) versetzt. Nach einer

Reaktionszeit von 3 d bei Raumtemperatur unter Feuchtigkeitsausschluß wurden die festen Rückstände durch Filtration abgetrennt und das Filtrat eingeengt. Dabei wurde das Rohprodukt des Esters nur in einer Ausbeute von 3,6 g erhalten. Das Filtrat wurde nochmals in Aceton (25 ml) aufgenommen und zur besseren Löslichkeit, des als Zwischenprodukt gebildeten Caesiumsalzes, mit DMF (25 ml) versetzt. Zu dieser Lösung wurde der zuvor abgetrennte Rückstand, Chloracetonitril (1,8 ml, 18,6 mmol) und Kaliumjodid (20 mg) hinzugefügt. Die Reaktionsmischung wurde 3 h bei 60 °C und 16 h bei Raumtemperatur unter Wasserausschluß gerührt. Die festen Rückstände wurde durch Filtration abgetrennt und das Filtrat eingeengt. Es konnte weiteres Rohprodukt des Esters erhalten werden, das noch DMF enthielt. Die beiden Rohprodukte wurden vereinigt und an Kieselgel mit Ethylacetat/Cyclohexan (1 : 3) chromatographisch gereinigt. Der gewünschte Cyanmethylester wurde als beigefarbene Verbindung mit einem Schmelzpunkt von 72 °C in einer Ausbeute von 91 % (5,36 g) erhalten.

**3-(Indol-3-yl)propionsäharnstoffmid**

Der soeben hergestellte Cyanmethylesters (5,1 g, 22,3 mmol) in Tetrahydrofuran (100 ml) wurde unter Rühren zu einer 25-proz. Ammoniaklösung (125 ml) gegeben und 20 h bei Raumtemperatur gerührt. Nach dieser Reaktionszeit war die Umsetzung vollständig. Die Aufarbeitung des Ansatzes erfolgte durch Phasentrennung und Extraktion der wäßrigen Phase mit Tetrahydrofuran (2 × 30 ml). Die organischen Phasen wurden vereinigt, getrocknet und eingeengt. Der Rückstand wurde mit Wasser (3 × 10 ml) und Diethylether (3 × 10 ml) gewaschen und getrocknet. Das gewünschte Amid blieb als weißer Feststoff in einer Ausbeute von 76 % (3.2 g) mit einem Schmelzpunkt von 140°C zurück.

**3-(Indol-3-yl)propylamin (Ind-15)**

Lithiumaluminiumhydrid (1,42 g, 34 mmol) wurde unter Argon portionsweise unter Rühren in abs. THF (70 ml) gegeben. Eine Lösung des soeben hergestellten Amids (3,2 g, 17 mmol) in abs. THF (60 ml) wurde unter Rühren innerhalb von 30 min bei 60 °C der $LiAlH_4$-Suspension hinzugefügt. Nach einer Reaktionszeit von 12 h bei 60°C unter Argon wurde der Ansatz mit THF (30 ml) versetzt und unter Eiskühlung langsam Wasser (35 ml) zugegeben. Die dabei erhaltenen Aluminiumverbindungen wurden durch Filtration abgetrennt und mit THF (3 × 10 ml) gewaschen. Das Filtrat wurde eingeengt, bis ein Öl ausfiel. Nach der Zugabe von Wasser (30 ml) wurde das Amin mit Ethylacetat (3 × 40 ml) extrahiert, die Extrakte vereinigt und mit Wasser (40 ml) gewaschen. Nach dem Trocknen und Einengen der organischen Phase wurde das 3-(Indol-3-yl)propylamin (Ind-15) als farbloser Feststoff in einer Ausbeute von 94 % (2,77 g) mit einem Schmelzpunkt von 65 °C erhalten.

<u>**Indolbaustein Ind-16:**</u>

**3-(1H-Indol-3-yl)propanol**

$LiAlH_4$ (1,21 mg, 31,71 mmol) wurde in trockenem THF (50 ml) vorgelegt. Zu der Suspension wurde eine Lösung von 3-Indolpropionsäure (2,5 g, 13,21 mmol) in trockenem THF (80 ml) innerhalb von 30 min zugetropft. Im Anschluss wurde das Reaktionsgemisch 3h unter Rückfluss erhitzt, dann 18 bei RT gerührt. Anschließend wurde $H_2O$ (60 ml), dann ein Gemisch aus konz. $H_2SO_4$ (10 ml) und $H_2O$ (30 ml) hinzugefügt. Die Mischung wurde 20 min gerührt und schließlich mit Ether (50 ml) versetzt. Die organische Phase wurde abgetrennt und die wässrige Phase mit Ether (3 × 40 ml) extrahiert. Die vereinigten etherischen Extrakte wurden über $Na_2SO_4$ getrocknet und nach Filtration des Trockenmittels am Rotationsverdampfer zur Trockne eingeengt. 3-(1*H*-Indol-3-yl)propanol wurde als viskoses Öl (2,28 g, 99 %) erhalten.

**3-(3-Trimethylsilanyloxypropyl)-1H-indol (Ind-16)**

3-(1*H*-Indol-3-yl)propanol (1,75 g, 10 mmol) wurde in trockenem THF (30 ml) vorgelegt und bei RT zunächst mit Hexamethyldisilazan (10 ml, 47 mmol) gefolgt von Trimethylchlorsilan (2 ml, 15.7 mmol) versetzt. Es wurde für 20 h bei RT gerührt, dann das Lösungsmittel am Rotationsverdampfer entfernt und der Rückstand mit gesättigter $NaHCO_3$-Lösung basisch gestellt. Die wässrige Lösung wurde mit Ether (3 × 30 ml) extrahiert. Die vereinigten organischen Phasen wurden mit $H_2O$ (2 × 20 ml) gewaschen und über $Na_2SO_4$ getrocknet.

Entfernung des Lösungsmittels am Rotationsverdampfer lieferte 3-(3-Trimethylsilanyloxypropyl)-1H-indol (2,46 g, 100 %, Smp: 34-38 °C) als kristallinen Feststoff.

**Indolbaustein Ind-18:**

**2-(2-(1H-Indol-3-yl)ethyl)isoindolin-1,3-dion**

Eine Lösung von Tryptamin (3.04 g, 19.0 mmol) und Phthalsähamstoffnhydrid (3.0 g, 20.2 mmol) in Toluol (300 ml) wurde 12 h unter Rückfluss am Wasserabscheider gekocht. Das Lösungsmittel wurde i. Vak. entfernt und der Rückstand aus Dichlormethan/Cyclohexan umkristallisiert. *Ausbeute:* 4.46 g (81 %)

**Indolbaustein Ind-19:**

**1-(2-(5-Fluor-1H-indol-3-yl)ethyl)-1H-benzo[d]imidazol (Ind-19)**

Eine Lösung von 3-(2-Brom-ethyl)-5-fluor-1H-indol (7.26 g, 30 mmol), Benz-imidazol (3.54 g, 30 mmol) und Ethyl-diisopropylamin (5.1 ml, 30 mmol) in abs. Chloroform (80 ml) wurde 20 h unter Rückfluss gekocht. Anschließend wurde die Reaktionslösung mit Wasser zweimal gewaschen, über $Na_2SO_4$ getrocknet, i. Vak. eingeengt und der verbliebene Rückstand durch Flash-Chromatographie mit $CHCl_3$/MeOH (50:1) gereinigt.
Im Wasser setzte sich ein unlöslicher Feststoff ab, der abgesaugt und ebenfalls über eine Kieselgelsäule gereinigt wurde. Dabei handelte es sich ebenfalls um das gewünschte Produkt.
*Ausbeute:* 2.95 g (35 %)
*$^1$H-NMR (DMSO-$d_6$):* 3.21 (2 H, t); 4.51 (2 H, t); 6.90 (1 H, m); 7.21 (5 H, m); 7.62 (1 H, d); 8.09 (1 H, s); 10.95 (1 H, s).

**Indolbaustein Ind-20:**

**5-Fluor-3-(2-(piperidin-1-yl)ethyl)-1H-indol (Ind-20)**

Piperidin (3.52 g, 4.08 ml, 41.3 mmol) wurde in abs. Dioxan (100 ml) gelöst, bei RT mit 3-(2-Brom-ethyl)-5-fluor-1 H-indol (5.00 g, 20.7 mmol) versetzt und 16 h bei 70 °C gerührt. Die Lösung wurde eingeengt, in $CHCl_3$ (150 ml) aufgenommen und mit Wasser (2 x 50 ml) gewaschen. Die organische Phase wurde über $Na_2SO_4$ getrocknet, filtriert und i. Vak. eingeengt. Der erhaltene Rückstand wurde durch Flash-Chromatographie mit 200 g Kieselgel und Essigester/ Ethanol (9:1→1:2) gereinigt.
*Ausbeute:* 3.80 g (75 %), farbloser Feststoff
*$^1$H-NMR (DMSO-$d_6$):* 1.43 (2 H, m); 1.59 (4 H, m); 2.67 (6 H, m); 2.92 (2 H, t); 6.89 (1 H, m); 7.24 (1 H, s); 7.31 (2 H, m); 11.05 (1 H, s).

**Indolbaustein Ind-21:**

**3-(2-(Piperidin-1-yl)ethyl)-1H-indol (Ind-21)**

Zu einer Lösung aus 3-(2-Brom-ethyl)-indol (5.00 g, 22.31 mmol) in trockenem Chloroform (25 ml) wurde bei Raumtemperatur Piperidin (11.2 ml, 113 mmol) hinzugefügt und anschließend der Ansatz 5 h zum Sieden erhitzt. Nach dem Abkühlen wurde die organische Phase mit verdünnter Schwefelsäure (2 x 50 ml) extrahiert.
Die wässrige Phase wurde mit 5N NaOH unter Eiskühlung alkalisch gestellt und mit Ether (3 x 50 ml) extrahiert. Die organische Phase wurde über $Na_2SO_4$ getrocknet und i. Vak. eingeengt. Zur weiteren Reinigung wurde der Rückstand aus Toluol (100 ml) umkristallisiert. *Ausbeute:* 2.00 g (40 %)
*$^1$H-NMR (DMSO-$d_6$):* 1.38 (2 H, m); 1.52 (4 H, m); 2.42 (4 H, m); 2.55 (2 H; t); 3.31 (2 H, t); 7.03 (3 H, m); 7.31 (1 H, d); 7.50 (1 H, d); 10.73 (1 H, s).

**Indolbaustein Ind-22:**

**3-(2-(1H-1,2,3-Triazol-1-yl)ethyl)-5-fluor-1H-indol (Ind-22)**

Eine Lösung von 3-(2-Brom-ethyl)-5-fluor-1*H*-indol (7.26 g, 30 mmol), 1,2,3-Triazol (2.07 g, 30 mmol) und Ethyl-diisopropylamin (5.1 ml, 30 mmol) in abs. Chloroform (70 ml) wurde 24 h unter Rückfluss gekocht. Anschließend wurde die Reaktionslösung mit Wasser zweimal gewaschen, über $Na_2SO_4$ getrocknet, i. Vak. eingeengt und der verbliebene Rückstand durch Flash-Chromatographie mit EE/Cyclohexan (1:1→4:1) gereinigt.
*Ausbeute:* 2.35 g (34 %)
*$^1$H-NMR (DMSO-$d_6$):* 3.24 (2 H, t); 4.64 (2 H, t); 6.89 (1 H, m); 7.12 (1 H, s); 7.30 (2 H, m); 7.67 (1 H, s); 8.07 (1 H, s); 10.96 (1 H, s).

**Indolbaustein Ind-26:**

**(4-Fluor-3-methoxyphenyl)hydrazinhydrochlorid** Alle Arbeitsschritte wurden bei 0 °C durchgeführt. 4-Fluor-3-methoxy-anilin (4.92 g. 34.8 mmol) wurde unter Rühren in konzentrierte Salzsäure (30 ml) gegeben. Nach 10 min wurde zur Suspension eine wässrige Natriumnitrit-Lösung (10 ml, 2.41 g, 34.8 mmol) getropft. Nach weiteren 10 min wurde eine Zinn(II)-chlorid-Lösung (10 ml, 13.6 g, 73 mmol) in konzentrierter Salzsäure zugefügt. Nach Bildung eines gelblichen Niederschlags wurde weitere konzentrierte Salzsäure (30 ml) zugegeben. Der Niederschlag wurde abfiltriert, mit wenig Salzsäure gewaschen und i. Vak. durch wiederholte Zugabe von Toluol getrocknet.
*Ausbeute:* 9.81 g (146 %), farbloser Feststoff. *Schmelzpunkt:* 123-127 °C
$^1$H-NMR (300 MHz, $d_6$-*DMSO*): 3.80 (s, 3H), 6.53 (ddd, *J* = 11.2, 5.2, 2.4 Hz, 1H), 6.99 (dd, *J* = 7.5, 1.6 Hz, 1H), 7.11 (dd, *J* = 11.4, 8.8 Hz, 1 H), 8.20 (s, 1H), 10.28 (s, 3H).
$^{13}$C-NMR (100 MHz, $d_6$-*DMSO*): 55.9; 101.9; 106.3 (*J* = 6 Hz); 115.7 (*J* = 19 Hz); 142.5; 146.2; 147.2 (*J* = 237 Hz); 147.3.
**2-(5-Fluor-6-methoxy-1*H*-indol-3-yl)ethanol (Ind-26)** Zu einer Lösung von (4-Fluor-3-methoxyphenyl)hydrazinhydrochlorid [1.00 g, 3.46 mmol (bezogen auf eine Reinheit des Eduktes von 68 %)] in Acetonitril (30 ml) und 4 % wässriger Schwefelsäure (30 ml) wurde bei Raumtemperatur eine Lösung von Dihydrofuran (242 mg, 3.46 mmol) in Acetonitril (10 ml) getropft. Danach wurde die Temperatur auf 80°C erhöht und das Reaktionsgemisch 2 h bei dieser Temperatur gerührt, anschließend auf Raumtemperatur abgekühlt und das Lösungsmittel i. Vak. entfernt. Der Rückstand wurde mit 5 % Natriumhydrogencarbonat-Lösung versetzt und mit Ethylacetat (3 × 50 ml) extrahiert. Die vereinigten organischen Phasen wurden mit Natriumsulfat getrocknet und i. Vak. eingeengt.
*Ausbeute:* 570 mg (78 %)

**Indolbaustein stein Ind-27 & 28:**

**(3-Hydroxy-2-oxo-2,3-dihydro-1H-indol-3-yl)essigsäureethylester** (S. **J.** Garden, R. **B.** da Silva, A. C. Pinto, Tetrahedron 2002. 58, 8399-8412 (speziell Seite 8406))

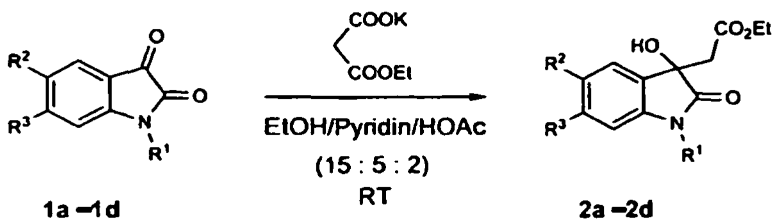


**1a–1d** **2a–2d**

**Tabelle 1:**

| | Produkt | Reaktionszeit | Ausbeute Schmelzpunkt |
|---|---|---|---|
| **2a** | R1 = H, R2 = Br, R3 = H.<br>(5-Brom-3-hydroxy-2-oxo-2,3-dihydro-1H-indol-3-yl) essigsäure-ethylester | 15 h | 80 %<br>168-170 °C |
| **2b** | R1 = H, R2 = F, R3 = H,<br>(5-Fluor-3-hydroxy-2-oxo-2,3-dihydro-1H-indol-3-yl) essigsäure-ethylester | 14 h | 89%<br>133-135 °C |
| **2c** | R1 = Me, R2 = H, R3 = H,<br>(3-Hydroxy-1-methyl-2-oxo-2,3-dihydro-1H-indol-3-yl) essigsäure-ethylester | 36 h | 73 %<br>97-98 °C |
| **2d** | R1 = H, R2 = N02, R3 = H,<br>(3-Hydroxy-5-nitro-2-oxo-2,3-dihydro-1H-indol-3-yl) essigsäure-ethylester | 26 h | 86%<br>194-197 °C |

Das entsprechende Isatin **1a-1d** (10 mmol) wurde in einem Gemisch aus Ethanol/Pyridin/Essigsäure (50 ml, 15 : 5 : 2) gelöst, mit Ethyl-kaliummalonat (1.87 g, 11 mmol) versetzt und entsprechend den in der Tabelle angegebenen

Zeiten unter Rückfluß erhitzt. Der Verlauf der Reaktion wurde mittels DC (Eluent: Ethylacetat/Hexan 1:1) kontrolliert. Zur Aufarbeitung wurde das Lösungsmittelgemisch im Vakuum abdestilliert. Der Rückstand wurde in Ethylacetat (50 ml) aufgenommen und mit Wasser (50 ml) ausgeschüttelt. Nach Phasentrennung wurde die wäßrige Phase zweimal mit Ethylacetat (je 30 ml) extrahiert. Die kombinierten organischen Phasen wurden mit 2N HCl (50 ml) gewaschen, über $Na_2SO_4$ getrocknet und im Vakuum auf 20 ml eingeengt. Zu der Lösung wurde solange Hexan zugegeben, bis Kristallisation des entsprechenden 3-Hydroxy-2-oxo-2,3-dihydro-1H-indol-3-yl)essigsäureethylester **(2a-2d)** einsetzte. Zur Vervollständigung der Kristallisation wurde der Ansatz 12 h auf 10°C gekühlt. Der Feststoff wurde abgesaugt und im Vakuum getrocknet.

**(1H-Indol-3-yl)ethanol**

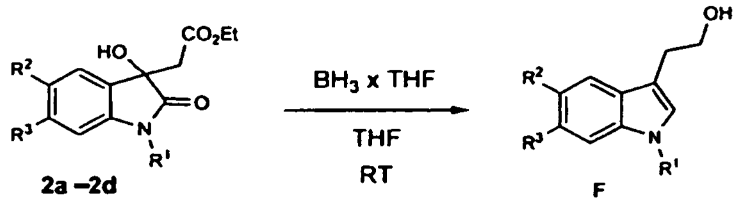


2a –2d

F

**Tabelle 2:**

| | Produkt | Reaktionszeit | Ausbeute Schmelzpunkt |
|---|---|---|---|
| **5-Bromtryptophol** | R1 = H, R2 = Br, R3 = H, 2-(5-Brom-1H-indol-3-yl)ethanol | 12 h | 75% 78-79 °C |
| | R1 = H, R2 = F, R3 = H, 2-(5-Fluor-1 H-indol-3-yl)ethanol | 14 h | 95% 47-50 °C |
| | R1 = Me, R2 = H, R3 = H, 2-(1-Methyl-1H-indol-3-yl)ethanol | 20 h | 98% Öl |
| **Ind-27** | R1 = H, R2 = N02, R3 = H, 2-(5-Nitro-1H-indol-3-yl)ethanol | 24 h | 70 % ** 78-81 °C |
| ** Säulenchromatographische Reinigung: Kieselgel; Eluent: Ethylacetat/Cyclohexan (1 : 4) | | | |

Das Aldolprodukt **2a-2d** (10 mmol) wurde unter Ar-Atmosphäre in absolutem THF (20 ml) gelöst. Der Ansatz wurde anschließend unter Wasserbadkühlung mit $BH_3 \times THF$ (40 ml, 1 M Lösung, 40 mmol) versetzt und bei Raumtemperatur entsprechend den in der Tabelle angegebenen Zeiten gerührt. Der Reaktionsverlauf wurde mittels DC überwacht. Nach Beendigung der Reaktion wurde die Reaktionslösung zu einer Mischung aus Ethylacetat (50 ml) und H2O (50 ml) gegeben. Nach Phasentrennung wurde die wäßrige Phase zweimal mit Ethylacetat (je 30 ml) extrahiert. Die kombinierten organischen Phasen wurden über $Na_2SO_4$ getrocknet und im Vakuum eingeengt. Der Rückstand wurde über Kieselgel mit Ethylacetat filtriert. Das nach Entfernen des Lösungsmittels erhaltene Produkt lag als ausreichend reines Öl vor und kristallisierte in der Regel spontan. Gegebenenfalls wurde eine säulenchromatische Reinigung an Kieselgel mit den in der Tabelle angegebenen Laufmitteln durchgeführt.

**2-(5-Pyridin-4-yl-1H-indol-3-yl)ethanol (Ind-28)**

5-Bromtryptophol (1,05 g, 4.39 mmol; s. Tabelle 1 und 2) und Pyridin-4-boronsäure (10 mg, 6,59 mmol) wurden in Tetrahydrofuran (65 ml) suspendiert und mit $Na_2CO_3$-Lösung (4,65 g, 43,9 mmol in 44 ml $H_2O$) versetzt. Dann wurde Bistriphenylphosphinpalladiumdichlorid (456,2 mg, 0,65 mmol) zugegeben. Das in der Wärme klare Reaktionsgemisch wurde unter Rühren bei 65°C 14 h gekocht. Der Verlauf der Reaktion wurde durch DC verfolgt. Zur Aufarbeitung wurde die Reaktionslösung filtriert. Das Filtrat war zweiphasig. Die organische Phase wurde abgetrennt. Das Lösungsmittel wurde am Rotationsverdampfer abdestilliert. Der viskose Rückstand wurde durch Flash-Chromatographie gereinigt [Kieselgel 60 (45 g); Eluent: EtOAc (800 ml)].
*Ausbeute:* 282 mg (27 %), **Ind-28,** hellgelber Feststoff; *Schmelzpunkt:* 169-171 °C.

**Indolbaustein Ind-31:**

**N-(3,5-Dichlorphenyl)-2-hydroxyiminoacetamid**

Zu einer Lösung von Chloralhydrat (11 g, 0,066 mol) und Natriumsulfat (70 g) in Wasser (240 ml) wurde eine Suspension des 3,5-Dichloroanilins (10g, 0,0617 mol) in Wasser (40 ml) und 37-proz. Salzsäure (5,3 ml, 0,066 mol) gegeben. Zu dieser Mischung wurde eine Lösung von Hydroxylamin Hydrochlorid (13,5 g, 0,195 mol) in Wasser (60 ml) hinzugefügt. Das Reaktionsgemisch wurde 1 h unter Rückfluß gekocht, wobei eine klare Reaktionslösung entstand, aus der schon in der Wärme ein Reaktionsprodukt ausfiel. Der Ansatz wurde 16 h bei Raumtemperatur gerührt und das gewünschte Oxim nach Filtration und Waschen mit Wasser (3 × 50 ml) in einer Ausbeute von 84 % (12,1 g) mit einem Schmelzpunkt von 179 °C als gelber Feststoff erhalten.

**4,6-Dichlor-1 H-indol-2,3-dion**

Das soeben hergestellte N-(3,5-Dichlorphenyl)-2-hydroxyiminoacetamid (12,1 g, 0,052 mol) wurde innerhalb von 15 min bei 50-75 °C in 96-proz. Schwefelsäure (56 ml) gegeben. Anschließend wurde die Reaktionsmischung 15 min unter Rühren auf 90°C erwärmt. Nach dem Abkühlen wurde der Ansatz langsam auf Eis (500 g) gegossen. Der entstandene Feststoff wurde nach 30 min abgesaugt. Das gewünschte Isatin wurde in einer Ausbeute von 81 % (9,12 g) als orangefarbener Feststoff isoliert. Ein Schmelzpunkt konnte nicht bestimmt werden.

(4,6-Dichlor-3-hydroxy-2-oxo-2,3-dihydro-1H-indol-3-yl)essigsäure-ethylester Eine Lösung des 4,6-Dichlor-1H-indol-2,3-dions (4,5 g, 20,8 mmol) in einem Gemisch von Ethanol /Pyridin/Essigsäure [(15: 5 : 2), 100 ml] wurde mit Malonsäure-monoethylester-Kaliumsalz (3,9 g, 22,88 mmol) versetzt und 7 h unter Rückfluß gekocht. Das Reaktionsgemisch wurde eingeengt, in Ethylacetat (50 ml) aufgenommen und mit Wasser (50 ml) gewaschen. Die wäßrige Phase wurde mit Ethylacetat (2 × 30 ml) extrahiert. Die organischen Phasen wurde vereinigt und mit 2N Salzsäure (50 ml) gewaschen, getrocknet und eingeengt. Der gewünschte Hydroxyester wurde als gelbes Öl in einer Ausbeute von 82 % (5,19 g) erhalten.

**2-(4,6-Dichlor-1H-indol-3-yl)ethanol (Ind-31)**

Die Lösung des soeben hergestellten (4,6-Dichlor-3-hydroxy-2-oxo-2,3-dihydro-1H-indol-3-yl)essigsäure-ethylester (5,19 g, 17,0 mmol) in abs. Tetrahydrofuran (50 ml) wurde im Eisbad innerhalb von 20 min mit einer 1M Boran/THF-Lösung (68 ml, 68 mmol) versetzt. Die Reaktionsmischung wurde 48 h bei Raumtemperatur gerührt und zur Aufarbeitung unter Rühren in eine Mischung aus Ethylacetat (100 ml) und Wasser (100 ml) gegeben. Die Phasen wurden getrennt und die wäßrige Phase mit Ethylacetat (2 × 50 ml) extrahiert. Die organischen Phasen wurden vereinigt, getrocknet und eingeengt. Der Rückstand wurde nochmals in einem Gemisch aus Wasser (50 ml) und Ethylacetat (50 ml) aufgenommen. Die organische Phase wurde mit Wasser (3 × 50 ml) gewaschen, getrocknet und eingeengt. Dabei wurde das rohe Indolbaustein Ind-31 als gelbes Öl erhalten (3,74 g). Eine chromatographische Reinigung [Kieselgel G (120 g); Ethylacetat/Cyclohexan 1 : 2 (3,5 l)] lieferte 2-(4,6-Dichlor-1H-indol-3-yl)ethanol als beigefarbenes Öl in einer Ausbeute von 69 % (2,4 g).

**Indolbaustein Ind-36:**

**5-Fluor-3-(2-(pyrrolidin-1-yl)ethyl)-1H-indol (Ind-36)**

Pyrrolidin (2.94 g, 3.4 ml, 41.3 mmol) wurde in abs. Dioxan (100 ml) gelöst, bei RT mit 3-(2-Brom-ethyl)-5-fluor-1H-indol (5.00 g, 20.7 mmol) versetzt und 8 h bei 70 °C gerührt. Die Lösung wurde eingeengt, in $CHCl_3$ (150 ml) aufgenommen und mit Wasser (2 x 50 ml) gewaschen. Die organische Phase wurde über $Na_2SO_4$ getrocknet, filtriert und i. Vak. eingeengt. Der erhaltene Rückstand wurde durch Flash-Chromatographie mit 500 g Kieselgel und Chloroform/Methanol (20:1 → 9:1 →4:1 → Methanol gereinigt. Erst mit Methanol wurde ein Salz der Verbindung erhalten, dieses konnte anschließend durch Rühren mit 2N NaOH und $CHCl_3$ freigesetzt werden. Die Phasen wurden getrennt, die wässrige Phase wurde zweimal mit $CHCl_3$ extrahiert und die vereinigten organischen Phasen über $Na_2SO_4$ getrocknet, filtriert und i. Vak. eingeengt. Wahrscheinlich war das verwendete Chloroform mit HCl verunreinigt.
*Ausbeute:* 3.27 g (**Ind-36**, 68 %), farbloser Feststoff
*$^1$H-NMR (DMSO-$d_6$):* 1.72 (4 H, m); 2.62 (4 H, m); 2.83 (4 H, m); 6.89 (1 H, m); 7.25 (3 H, m); 10.93 (1 H, s).
*$^{13}$C-NMR (DMSO-$d_6$):* 23.05; 23.90; 53.39; 56.07; 102.77: 108.72; 112.25; 124.68; 127.29; 132.84; 155.46; 157.75.

**Indolbaustein Ind-38:**

**3-(2-(1H-Pyrazol-1-yl)ethyl)-5-fluor-1H-indol (Ind-38)**

Eine Lösung von 3-(2-Brom-ethyl)-5-fluor-1*H*-indol (7.26 g, 30 mmol), Pyrazol (2.04 g, 30 mmol) und Ethyl-diisopropylamin (5.1 ml, 30 mmol) in abs. Chloroform (80 ml) wurde 12 h bei 90 °C gerührt. Anschließend wurde die Reaktionslösung mit Wasser zweimal gewaschen, über $Na_2SO_4$ getrocknet, i. Vak. eingeengt und der verbliebene Rückstand durch Flash-Chromatographie mit $CHCl_3$/MeOH (20:1) gereinigt.
*Ausbeute:* 1.91 g (Ind-38, 28 %)
*$^1$H-NMR (DMSO-$d_6$):* 3.16 (2 H, t); 4.35 (2 H, t); 6.17 (1 H, s); 6.86 (1 H, m), 7.24 (3 H, d); 7.44 (1 H, s); 7.64 (1 H, s); 10.93 (1 H, s).

**Indolbaustein Ind-39:**

**3-(2-(1H-Imidazol-1-yl)ethyl)-5-fluor-1H-indol (Ind-39;)**

Eine Lösung von 3-(2-Brom-ethyl)-5-fluor-1*H*-indol (4.84 g, 20 mmol), Imidazol (1.36 g, 20 mmol) und Ethyl-diisopropylamin (3.4 ml, 20 mmol) in abs. Dioxan (50 ml) wurde 8 h bei 90 °C gerührt, bis lt. DC kein Edukt mehr vorhanden war. Anschließend wurde das Lösungsmittel i. Vak. entfernt, der Rückstand mit $CHCl_3$ (100 ml) versetzt, und die organische Phase mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/MeOH (9:1) gereinigt.
*Ausbeute:* 1.29 g (**Ind-39**; 28 %)
*$^1$H-NMR (DMSO-$d_6$):* 3.10 (2 H, t); 4.20 (2 H, t); 6.90 (2 H, m); 7.12 (1 H, s); 7.20 (1 H, s); 7.33 (2 H, m); 7.55 (1 H, s); 10.9 (1 H, s).

**Indolbaustein Ind-40:**

**1-(2-(5-Fluor-1H-indol-3-yl)ethyl)-1H-benzo[d]imidazol (Ind-40)**

Eine Lösung von 3-(2-Brom-ethyl)-5-fluor-1H-indol (7.26 g, 30 mmol), Benz-imidazol (3.54 g, 30 mmol) und Ethyl-diisopropylamin (5.1 ml, 30 mmol) in abs. Chloroform (80 ml) wurde 20 h unter Rückfluss gekocht. Anschließend wurde die Reaktionslösung mit Wasser zweimal gewaschen, über $Na_2SO_4$ getrocknet, i. Vak. eingeengt und der verbliebene Rückstand durch Flash-Chromatographie mit $CHCl_3$/MeOH (50:1) gereinigt.
Im Wasser setzte sich ein unlöslicher Feststoff ab, der abgesaugt und ebenfalls über eine Kieselgelsäule gereinigt wurde. Dabei handelte es sich ebenfalls um das gewünschte Produkt. *Ausbeute:* 2.95 g (35 %)
*$^1$H-NMR (DMSO-$d_6$):* 3.21 (2 H, t); 4.51 (2 H, t); 6.90 (1 H, m); 7.21 (5 H, m); 7.62 (1 H, d); 8.09 (1H, s); 10.95 (1 H, s).

**Indolbaustein Ind-43:**

**3-Methyl-5-trifluormethoxy-2-trimethylsilanyl-1H-indol**

2-Iod-4-trifluormethoxyanilin (1, 2,42 g, 8 mmol), Trimethylsilylpropin (2, 988 mg, 1,31 ml, 8,8 mmol), Lithiumchlorid (356 mg, 8,4 mmol) und Natriumcarbonat (2,54 g, 24 mmol) wurden in abs. Dimethylformamid (20 ml) in einer Argon-Atmosphäre vereinigt. Anschließend wurde der Katalysator ([Pd(dppf)Cl2 × $CH_2C_{l2}$]; 654 mg, 0,8 mmol) hinzugegeben. Die Reaktionsmischung wurde 6 h bei 100°C (Ölbadtemperatur) und 18 h bei Raumtemperatur gerührt. Anschließend wurde die Reaktionsmischung mit Eiswasser gekühlt, mit Wasser (100 ml) und Ethylacetat (200 ml) versetzt und 30 min gerührt. Um den Katalysator abzutrennen, wurde die dunkelbraune Mischung über Celite filtriert. Die Phasen des Filtrats wurden getrennt. Die wäßrige Phase wurde mit Ethylacetat (5 × 35 ml) extrahiert. Die vereinigten organischen Phasen wurden mit gesättigter Natriumchlorid-Lösung (3 × 35 ml) gewaschen, anschließend mit Natriumsulfat getrocknet, filtriert und im Vakuum eingeengt. Das erhaltene dunkelbraune Öl (2,95 g), das außer 3-Methyl-5-trifluormethoxy-2-trimethylsilanyl-1H-indol auch das isomere 2-Methyl-5-(Trifluormethoxy)-3-(trimethylsilyl)-1H-indol enthielt, wurde als Rohprodukt für die nächste Stufe eingesetzt.

**3-Methyl-5-trifluormethoxy-1H-indol (Ind-43)**

Die Abspaltung der Trimethylsilylgruppe erfolgte in zwei Stufen. Das Rohprodukt der Silylverbindungen (3-Methyl-5-trifluormethoxy-2-trimethylsilanyl-1H-indol und 2-Methyl-5-(Trifluormethoxy)-3-(trimethylsilyl)-1H-indol) (2,95 g, 8 mmol, bezogen auf die Vorstufe) wurde mit THF (40 ml) und Tetrabutylammoniumfluorid (3,28 g, 11 mmol) 4 h bei

RT gerührt. Dann wurde der Ansatz aufgearbeitet. Nach Zugabe von Wasser (40 ml) wurde die Mischung 15 min gerührt. Anschließend wurden die Phasen getrennt. Die wäßrige Phase wurde mit Diethylether (3 × 60 ml) extrahiert. Die vereinigten organischen Phasen wurden mit Natriumsulfat getrocknet, filtriert und im Vakuum eingeengt. Es wurde ein zähes dunkelbraunes Öl erhalten, das durch Chromatographie [Kieselgel 60 (100 g); Cyclohexan/Ethylacetat (15 : 1; 1000 ml; 10 : 1; 400 ml)] in seine Bestandteile aufgetrennt wurde. Es wurden 1,44 g (5 mmol, 63 % bezogen auf die Vorstufe) des reinen 3-Methyl-5-trifluormethoxy-2-trimethylsilanyl-1H-indols erhalten. Diese wurde erneut mit THF (30 ml) und Tetrabutylammoniumfluorid (2,05 g, 6,5 mmol) versetzt und 2 h zum Rückfluß erhitzt. Nach zuvor beschriebener Aufarbeitung wurden 1,018 g (59 %, bezogen auf die 1. Stufe) 3-Methyl-5-trifluormethoxy-1H-indol (Ind-43) als braunes Öl erhalten.

**Indolbaustein Ind-47: Methyl 2-(1H-indol-3-yl)acetat (Ind-47)**

*CAS-Nr.1912-33-0, kommerziell erhältlich z.B. bei Fluka*

**Indolbaustein Ind-49:**

**3-Hydroxy-3-pyridin-2-ylmethyl-1,3-dihydroindol-2-on** Ein Gemisch aus Isatin (12.0 g, 82 mmol) und 2-Picolin (25.1 g, 24 ml, 0.27 mol) wurde 5 h unter Rückfluss erhitzt. Das Reaktionsgemisch wurde anschließend i. Vak. eingeengt, der Rückstand wiederholt mit Toluol versetzt und jeweils wieder i. Vak. eingeengt. Noch vorhandenes 2-Picolin wurde durch Kugelrohrdestillation bei 60 °C abdestilliert. Der Rückstand wurde mit Chloroform (100 ml) und 1 M Salzsäure (80 ml) versetzt, die wässrige Phase abgetrennt und die Chloroformphase emeut mit 1 M Salzsäure (40 ml) versetzt und extrahiert. Die vereinigten sauren wässrigen Phasen wurden mit 25 % Ammoniaklösung auf pH 10 gestellt und das ausgefallene Produkt wurde abfiltriert, mit Wasser gewaschen und bei 100°C getrocknet.
*Ausbeute:* 21.0 g (100 %), gelber Feststoff; *Schmelzpunkt:* 165-170 °C
*1H-NMR (DMSO-d6):* 3.17 (d, 1H, J = 13.0 Hz); 3.30 (d, 1H, J = 13.1 Hz); 6.29 (s, 1H); 6,64 (d, 1H. J = 7.6 Hz); 6.82 (t, 1H. J = 7.3 Hz); 6.91 (d, 1H. J = 6.9 Hz); 7.04-7.17 (m, 3H); 7.57 (dt, 1H, J = 6.9 und 1.4 Hz); 8.29 (d, 1H, J = 4.3 Hz); 10.13 (s, 1H).

**3-Pyridin-2-ylmethyl-1H-indol (Ind-49)** Eine Lösung von 3-Hydroxy-3-pyridin-2-ylmethyl-1,3-dihydroindol-2-on (4.80 g, 20 mmol) in wasserfreiem Tetrahydrofuran (250 ml) wurde mit einer 2 M Lösung des Boran-Dimethylsulfid-Komplexes (20 ml, 40 mmol) versetzt und über Nacht bei Raumtemperatur gerührt. Das Reaktionsgemisch wurde vorsichtig mit Methanol (10 ml) versetzt und i. Vak. eingeengt. Der Rückstand wurde wiederholt mit Methanol versetzt, jeweils wieder i. Vak. eingeengt und in 1 N Salzsäure (40 ml) aufgenommen. Die wässrige Suspension wurde mit Ethylacetat (2 × 40 ml) extrahiert. Die wässrige Phase wurde mit gesättigter Kaliumcarbonat-Lösung auf pH 10 gestellt und mit Ethylacetat (3 × 30 ml) extrahiert. Die vereinigten organischen Phasen wurden mit Natriumsulfat getrocknet und i. Vak. eingeengt. Das Rohprodukt (4.0 g) wurde durch Flashchromatographie (400 g, 20 x 7.6 cm) zuerst mit Ethylacetat / Cyclohexan (2:1) und dann Chloroform gereinigt.
*Ausbeute* **(Ind-49):** 2.00 g (48 %), rötlicher Feststoff; *Schmelzpunkt:* 80-82 °C
*1H-NMR (DMSO-d6):* 4.18 (s, 2H); 6.92 (ddd, 1H, J = 8.0 ,7.0 und 1.1 Hz); 7.05 (ddd, 1H, J = 8.2, 7.1 und 1.2 Hz); 7.15 (m, 1H); 7.18-7.24 (m, 2H); 7.34 (dt, 1H, J = 8.1 und 0.9 Hz); 7.45 (br d, 1H, J = 7.8 Hz); 7.64 (dt, 1H, J = 7.7 und 1.9 Hz); 8.47 (ddd, 1H, J = 4.9, 1.8 und 0.9 Hz); 10.86 (s, 1H).

**Indolbaustein Ind-50: 3-(1H-Indol-3-yl)propionsäure (Ind-50)**

*CAS-Nr: 830-96-6, kommerziell erhältlich z.B. bei Fluka*

**Indolbaustein Ind-54:**

**2-(2-(1H-Indol-3-yl)ethyl)-1-methyl-1H-benzo[d]imidazol (Ind-54;)**

Ein Gemisch von 3-Indolpropionsäure (2.85 g, 15 mmol) und N-Methyl-1,2-phenylendiamin (611 mg, 5.0 mmol) wurde bei 130 °C 5 h gerührt, dabei entsteht eine dunkelbraune, zähe Masse, die in Chloroform (100 ml) gelöst wurde. Anschließend wurde die organische Lösung mit 10 %-iger $Na_2CO_3$-Lösung (2 × 30 ml) und Wasser gewaschen, über $Na_2SO_4$ getrocknet, i. Vak. eingeengt und der Rückstand durch Flash-Chromatographie mit Cyclohexan/EE (1:1) gereinigt. *Ausbeute:* 863 mg (**Ind-54**, 63 %), farbloser Feststoff
*$^1H$-NMR (DMSO-$d_6$):* 3.22 (4 H, t); 3.65 (3 H, s); 6.95-7.56 (5 H, m); 7.35 (1 H, d); 7.45 (1 H, d); 7.59 (2 H, m); 10.81 (1 H, s).

**Indolbaustein Ind-55: 2-(2-(1H-Indol-3-yl)ethyl)-1-methyl-1H-benzo[d]imidazol (Ind-61)**

*CAS-Nr: 16571-51-0 , kommerziell erhältlich z.B. bei Sigma-Aldrich*

**Indolbaustein Ind-56: 4-(1H-Indol-3-yl)butansäure (Ind-56)**

*CAS-Nr: 133-32-4, kommerziell erhältlich z.B. bei ACROS*

**Indolbaustein Ind-57:**

**4-(1H-Indol-3-yl)-butan-1-ol (Ind-57)**

$LiAlH_4$ (1,14 g, 30 mmol) wurde unter Ausschluß von Sauerstoff in trockenem THF (100 ml) vorgelegt. Zu der Suspension wurde 4-(1H-Indol-3-yl)butansäure (2,03 g, 10 mmol, gelöst in 80 ml trockenem THF) innerhalb von 30 min zugetropft. Im Anschluß wurde der Ansatz 3 h unter Rückfluß zum Sieden erhitzt. Das Reaktionsgemisch wurde über Nacht bei Raumtemperatur gerührt. Anschließend wurde der Ansatz vorsichtig mit Wasser (30 ml) versetzt. Die Mischung wurde 20 min gerührt und mit 2N NaOH (10 ml) versetzt. Die organische Phase wurde abgetrennt und die verbliebene wäßrige Lösung mit Diethylether (3 x 40 ml) extrahiert. Die etherische Lösung wurde über $Na_2SO_4$ getrocknet und am Rotationsverdampfer zur Trockne eingeengt. Ind-57 wurde so in einer Ausbeute von 1,8 g (95 %) als farbloses Öl erhalten.

**Indolbaustein Ind-61:**

1-(2-(1H-Indol-3-yl)ethyl)pyrrolidin-2,5-dion (Ind-61)

Zur heißen Lösung von Tryptamin (3.30 g, 20.6 mmol) und Kaliumacetat (2.23 g, 22.7 mmol) in Essigsäure (10 ml) wurde portionsweise Bernsteinsäharnstoffnhydrid (2.27 g, 22.7 mmol) gegeben. Die Reaktionslösung wurde 3 h zum Sieden erhitzt, über Nacht bei RT nachgerührt, der ausgefallene Niederschlag abfiltriert und mit Essigsäure und EtOH gewaschen. *Ausbeute:* 3.00 g (**Ind-61**, 60 %)
*$^1$H-NMR (DMSO-$d_6$):* 2.61 (4 H, s); 2.88 (2 H, t); 3.61 (2 H, t); 7.01 (2 H, m); 7.20 (1 H, s); 7.36 (1 H, d); 7.53 (1 H, t); 10.86 (1 H, bs).
*$^{13}$C-NMR (DMSO-$d_6$):* 23.06; 27.98; 38.60; 110.60; 111.41: 117.86; 118.33; 120.95; 122.83; 127.01; 136.19; 177.54.

**Indolbaustein Ind-62:**

**1-(2-(1H-Indol-3-yl)ethyl)-1,2,3,4-tetrahydrochinolin (Ind-62)**

Zu einer Lösung aus 3-(2-Bromethyl)-indol (5.00 g, 22.3 mmol) in trockenem Chloroform (25 ml) wurde bei Raumtemperatur 1,2,3,4-Tetrahydrochinolin (5.94 g, 5.6 ml, 44.6 mmol) hinzugefügt und anschließend unter Rückfluss 5 h gerührt. Der Reaktionsansatz kühlte über Nacht ab. Die organische Phase wurde mit verd. Schwefelsäure (2x50 ml) extrahiert. Die wässrige Phase wurde mit 5N NaOH unter Eiskühlung basisch gestellt und mit Ether (3 x 50 ml) extrahiert. Die organische Phase wurde über $Na_2SO_4$ getrocknet und i. Vak. eingeengt. *Ausbeute:* 5.35 g (Ind-62, 88 %)
*$^1$H-NMR (DMSO-$d_6$):* 1.83 (2 H, m); 2.67 (2 H, m); 2.92 (2 H, m); 3.50 (2 H; m); 6.47 (1 H, m); 6.66 (1 H, m); 6.87 (1 H, m); 7.08 (3 H, m); 7.20 (1 H, m); 7.36 (1 H, m); 7.55 (1 H, m); 10.82 (1 H, s).

**Indolbaustein Ind-63:**

**Methyl 1-(2-(1H-indol-3-yl)ethyl)-1H-1,2,3-triazol-4-carboxylat (Ind-63)**

Das Harz (4-(Brommethyl)-phenoxyethyl-polystyren HL) (5.00 g, 5.5 mmol) wurde in *N,N*-Dimethylacetamid (DMA) (30 ml) suspendiert, mit Natriumazid (1.78 g, 27.5 mmol, 5 equ.) versetzt und 48 h bei RT gerührt. Das Harz wurde abfiltriert, mit Methanol gewaschen und getrocknet. Das Harz wurde wieder in DMA (100 ml) suspendiert, 3-(2-Bromethyl)indol (0.60 g, 2.67 mmol) zugegeben und 5 d bei RT gerührt. Anschließend wurde mit Propiolsäuremethylester (0.24 ml, 2.67 mmol) versetzt, 20 h bei 80 °C gerührt und i. Vak. das Lösungsmittel entfernt. Der Rückstand wurde durch Flash-Chromatographie mit $CHCl_3$/MeOH (9:1) getrennt. Neben dem gewünschten Produkt wurde 3-(2-Bromethyl)indol (103 mg, 17 %) zurückgewonnen. *Ausbeute:* 342 mg (**Ind-63**, 47 %)
*$^1$H-NMR (DMSO-$d_6$):* 3.36 (2 H, m); 3.80 (3 H; m); 4.73 (2 H, m); 7.03 (3 H, m); 7.33 (1 H, d); 7.53 (1 H, d); 8.73 (1 H, s); 10.85 (1 H, s).
*$^{13}$C-NMR (DMSO-$d_6$):* 25.65; 50.29; 51.63; 109.60; 111.39; 118.09; 121.05; 123.18; 126.80; 129.00; 136.11; 137.34;

138.30; 160.73.

**Indolbaustein Ind-64:**

**3-(2-(Isoindolin-2-yl)ethyl)-1H-indol (Ind-64)**

Eine Lösung von 3-(2-Brom-ethyl)-1*H*-indol (4.48 g, 20 mmol) und Isoindol (4.76 g, 40 mmol) in abs. Dioxan (50 ml) wurde 6 h bei 80 °C gerührt. Anschließend wurde das Lösungsmittel i. Vak. entfernt, der Rückstand mit $CHCl_3$ (100 ml) versetzt und mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/MeOH (20:1) gereinigt. *Ausbeute:* 3.01 g (**Ind-64**, 57 %), brauner Feststoff
*$^1$H-NMR (DMSO-$d_6$):* 2.95 (4 H, m); 3.93 (4 H, m); 7.18 (2 H, m); 7.20 (5 H, m); 7.35 (1 H, d); 7.57 (1 H, d); 10.79 (1 H, s).

**Indolbaustein Ind-65:**

**2-(2-(1H-Indol-3-yl)ethyl)-1,2,3,4-tetrahydroisochinolin (Ind-65)**

Eine Lösung von 3-(2-Brom-ethyl)-1 *H*-indol (4.48 g, 20 mmol) und Isochinolin (5.33 g, 40 mmol) in abs. Dioxan (50 ml) wurde 6 h bei 80 °C gerührt. Anschließend wurde das Lösungsmittel i. Vak. entfernt, der Rückstand mit $CHCl_3$ (100 ml) versetzt und mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/MeOH (50:1) gereinigt. *Ausbeute:* 4.78 g (**Ind-65**, 86 %), weißer Feststoff
*$^1$H-NMR (DMSO-$d_6$):* 2.76 (6 H, m); 2.95 (2 H, m); 3.66 (2 H, s); 7.06 (6 H, m); 7.18 (1 H, s); 7.34 (1 H, d); 7.56 (1 H, d); 10.77 (1 H, s).

**Indolbaustein Ind-66:**

**3-(2-(Pyrrolidin-1-yl)ethyl)-1H-indol (Ind-66)**

Pyrrolidin (3.17 g, 3.7 ml, 44.6 mmol) wurde in abs. Dioxan (100 ml) gelöst, bei RT mit 3-(2-Brom-ethyl)-1*H*-indol (5 g, 22.3 mmol) versetzt und 8 h bei 70 °C gerührt. Die Lösung wurde eingeengt, in $CHCl_3$ (150 ml) aufgenommen und mit Wasser (2 x 50 ml) gewaschen. Die organische Phase wurde über $Na_2SO_4$ getrocknet, filtriert und i. Vak. eingeengt. *Ausbeute:* 3.00 g (**Ind-66**, 63 %)
*$^1$H-NMR (DMSO-$d_6$):* 1.73 (4 H, m); 2.54 (4 H, m); 2.74 (2 H, t); 2.61 (2 H, t); 6.99 (2 H, m); 7.15 (1 H, s); 7.35 (1 H, d); 7.52 (1 H, d); 10.79 (1 H, s).

**Indolbaustein Ind-68:**

**3-(2-(4-Methylpiperazin-1-yl)ethyl)-1H-indol (Ind-68)**

3-(2-Brom-ethyl)-indol (3.00 g, 13.39 mmol) wurde in abs. $CHCl_3$ (25 ml) vorgelegt und mit 1-Methylpiperazin (2.68 g, 26. 8 mmol) versetzt. Bei 75 °C Badtemperatur wurde 5 h und über Nacht bei RT gerührt. Der Ansatz wurde mit verdünnter Schwefelsäure (2 x 30 ml) extrahiert, die saure wässrige Phase mit 5N NaOH unter Eiskühlung alkalisch gestellt und mit Ether (3 x 30 ml) extrahiert. Die organische Phase wurde über $Na_2SO_4$ getrocknet und i. Vak. eingeengt. *Ausbeute:* 1.45 g (**Ind-68**, 45 %)
*$^1$H-NMR (DMSO-$d_6$):* 2.18 (3 H, s); 2.38 (4 H, m); 2.58 (4 H, m); 2.83 (2 H, t); 3.43 (2 H, t); 6.98 (2 H, m); 7.13 (1 H, s); 7.34 (1 H, m); 7.49 (1 H, m); 10.77 (1 H, s).

**Indolbaustein Ind-69:**

**4-(2-(1H-Indol-3-yl)ethyl)morpholin (Ind-69)**

Morpholin (2.33 g, 2.33 ml, 26.8 mmol) wurde in abs. Dioxan (50 ml) gelöst und bei RT mit 3-(2-Brom-ethyl)-1H-indol (3.00 g, 13.4 mmol) versetzt. Die Lösung wurde 14 h bei 70 °C gerührt, i. Vak. eingeengt, in $CHCl_3$ (100 ml) aufgenommen und mit Wasser (2 x 30 ml) gewaschen. Die organische Phase wurde über $Na_2SO_4$ getrocknet, filtriert und i. Vak. eingeengt. Der erhaltene Rückstand wurde aus wässrigem Methanol umkristallisiert. *Ausbeute:* 1.09 g (35 %)
*$^1$H-NMR (DMSO-$d_6$):* 2.45 (4 H, m); 2.58 (2 H, t); 2.83 (2 H, t); 3.59 (4 H, m); 7.04 (2 H, m); 7.15 (1 H, s); 7.32 (1 H, d); 7.50 (1 H, d); 10.77 (1 H, s).

**Indolbaustein Ind-70:**

1-(2-(1H-Indol-3-yl)ethyl)-1H-benzo[d]imidazol (Ind-70)

Eine Lösung von 3-(2-Brom-ethyl)-1H-indol (4.48 g, 20 mmol) und Benzimidazol (4.72 g, 40 mmol) in abs. Dioxan (50 ml) wurde 13 h bei 90 °C gerührt. Anschließend wurde das Lösungsmittel i. Vak. entfernt, der Rückstand mit $CHCl_3$ (200 ml) versetzt und mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/MeOH (40:1) gereinigt. *Ausbeute:* 1.32 g (25 %), hellbrauner Feststoff
*$^1$H-NMR (DMSO-$d_6$):* 3.24 (2 H, t); 4.53 (2 H, t); 7.10 (3 H, m); 7.27 (3 H, m); 7.62 (3 H, m); 8.09 (1 H, s); 10.85 (1 H, s).

**Indolbaustein Ind-71:**

**3-(2-(1H-Imidazol-1-yl)ethyl)-1H-indol (Ind-71)**

Eine Lösung von 3-(2-Brom-ethyl)-1H-indol (1.12g, 5 mmol) und Imidazol (0.68 g, 10 mmol) in abs. Dioxan (10 ml) wurde 4 h bei 80 °C gerührt, bis lt. DC kein Edukt mehr vorhanden war. Anschließend wurde das Lösungsmittel i. Vak. entfernt, der Rückstand mit $CHCl_3$ (100 ml) versetzt und mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/MeOH (20:1) gereinigt. *Ausbeute:* 507 mg (Ind-71, 48 %), farbloser Feststoff
*$^1$H-NMR (DMSO-$d_6$):* 3.16 (2 H, t); 4.23 (2 H, t); 6.86 (1 H, s); 7.05 (3 H, m); 7.19 (1 H, s); 7.32 (1 H, m); 7.55 (2 H, m); 10.86 (1 H, s).

**Indolbaustein Ind-72:**

**3-(2-(1H-1,2,4-Triazol-1-yl)ethyl)-1H-indol (Ind-72)**

Eine Lösung von 3-(2-Brom-ethyl)-1 -indol (4.92 g, 22 mmol) und 1.2.4-Triazol (3.03 g, 44 mmol) in abs. Dioxan (50 ml) wurde 24 h bei 80 °C gerührt. Anschließend wurde das Lösungsmittel i. Vak. entfernt, der Rückstand mit $CHCl_3$ (100 ml) versetzt und mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/MeOH (50:1) gereinigt. *Ausbeute:* 0.792 g (Ind-72, 17 %), farbloses Öl
*$^1$H-NMR (DMSO-$d_6$):* 3.20 (2 H, t); 4.44 (2 H, t); 7.05 (3 H, m); 7.35 (1 H, d); 7.49 (1 H, s); 7.96 (1 H, s); 8.37 (1 H, s); 10.83 (1 H, s).

**Indolbaustein Ind-73:**

**3-(2-(1H-Indol-3-yl)ethyl)thiazolidin (Ind-73)**

3-(2-Brom-ethyl)-indol (3.00 g, 13.4 mmol) und Thiazolidin (2.38 g, 26. 8 mmol) wurden in abs. $CHCl_3$ (25 ml) 5 h bei 75 °C Badtemperatur gerührt. Der Ansatz wurde auf RT abgekühlt und mit verdünnter Schwefelsäure (2 x 30 ml) extrahiert. Die saure wässrige Phase wurde mit 5N NaOH unter Eiskühlung alkalisch gestellt und mit Ether (3 x 30 ml) extrahiert. Die organische Phase wurde über $Na_2SO_4$ getrocknet, i. Vak. eingeengt und der Rückstand durch Flash-Chromatographie mit $CHCl_3$/MeOH (20:1 → 4:1 → MeOH) gereinigt. *Ausbeute:* 564 mg (**Ind-73**, 18 %)
*$^1$H-NMR (DMSO-$d_6$):* 2.68-3.05 (10 H, m); 6.97 (2 H, m); 7.18 (1 H, m); 7.32 (1 H, m); 7.57 (1 H, m); 10.84 (1 H, s).

**Indolbaustein Ind-74:**

**3-(2-(5-Methyl-2H-tetrazol-2-yl)ethyl)-1H-indol (Ind-74)**

Eine Lösung von 3-(2-Brom-ethyl)-1H-indol (2.24 g, 10 mmol), 5-Methyl-1,2,3,4-tetrazol (0.84 g, 10 mmol) und Ethyl-diisopropylamin (1.7 ml, 10 mmol) in abs. Dioxan (25 ml) wurde 8 h bei 90 °C gerührt, bis lt. DC kein Edukt mehr vorhanden war. Anschließend wurde das Lösungsmittel i. Vak. entfernt, der Rückstand mit $CHCl_3$ (100 ml) versetzt und die organische Phase mit Wasser zweimal gewaschen. Die organische Phase wurde über $Na_2SO_4$ getrocknet, i. Vak. eingeengt und der verbliebene Rückstand durch Flash-Chromatographie mit EE/Cyclohexan (1:4→1:1→MeOH) gereinigt.
*Ausbeute:* 843 mg (37 %), 3-[2-(5-Methyl-tetrazol-2-yl)-ethyl]-1H indol (**Ind-74**)
936 mg (41 %), 3-[2-(5-Methyl-tetrazol-1-yl)-ethyl]-1H-indol (**Ind-77**)

**Ind-74:**

$^{1}$*H-NMR (DMSO-d$_6$)*: 2.44 (3 H, s); 3.36 (2 H, t); 4.87 (2 H, t); 7.05 (3 H, m); 7.35 (1 H, m); 7.49 (1 H, m); 10.85 (1 H, s).

**Ind-77:**

$^{1}$*H-NMR (DMSO-d$_6$)*: 2.16 (3 H, s); 3.25 (2 H, t); 4.58 (2 H, t); 7.07 (3 H, m); 7.36 (1 H, m); 7.43 (1 H, m); 10.87 (1 H, s).

**Indolbaustein Ind-75:**

**3-(2-(1H-Pyrazol-1-yl)ethyl)-1H-indol (Ind-75)**

Eine Lösung von 3-(2-Brom-ethyl)-1*H*-indol (2.24 g, 10 mmol) und Pyrazol (1.36 g, 20 mmol) in abs. Dioxan (20 ml) wurde 16 h bei 80 °C gerührt. Anschließend wurde das Lösungsmittel i. Vak. entfernt, der Rückstand mit $CHCl_3$ (100 ml) versetzt und mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/MeOH (50:1) gereinigt. *Ausbeute:* 0.62 g (**Ind-75**, 29 %), braunes Öl
$^{1}$*H-NMR (DMSO-d$_6$):* 3.20 (2 H, t); 4.37 (2 H, t); 6.18 (1 H, s); 7.04 (3 H, m); 7.32 (1 H, d); 7.44 (1 H, s); 7.52 (1 H, d); 7.66 (1 H, s); 10.80 (1 H, s).

**Indolbaustein Ind-76:**

**3-(2-(1H-1,2,3-Triazol-1-yl)ethyl)-1H-indol (Ind-76)**

Eine Lösung von 3-(2-Brom-ethyl)-1H-indol (4.92 g, 22 mmol) und 1.2.3-Triazol (3.03 g, 44 mmol) in abs. Dioxan (50 ml) wurde 22 h bei 80 °C gerührt. Anschließend wurde das Lösungsmittel i. Vak. entfernt, der Rückstand mit $CHCl_3$ (100 ml) versetzt und mit Wasser zweimal gewaschen. Die organische Phase wurde über $Na_2SO_4$ getrocknet, i. Vak. eingeengt und der verbliebene Rückstand durch Flash-Chromatographie mit $CHCl_3$/meOH (50:1) gereinigt. *Ausbeute:* 0.50 g (Ind-76, 11 %), braunes Öl
$^{1}$*H-NMR (DMSO-d$_6$):* 3.31 (2 H, t); 4.67 (2 H, t); 6.97 (1 H, m); 7.08 (2 H, m); 7.34 (1 H, d); 7.51 (1 H, d); 7.65 (1 H, s); 8.01 (1 H, s); 10.72 (1 H, s).

**Indolbaustein Ind-77:**

**3-(2-(5-Methyl-1H-tetrazol-1-yl)ethyl)-1H-indol (Ind-77)**

Die Synthese des Indols **Ind-77** wurde unter im Rahmen Synthese von **Indolbaustein 74** beschrieben.

**Indolbaustein Ind-83:**

**N-(3,4-Dichlorphenyl)-2-hydroxyiminoacetamid**

Zu einer Lösung von Chloralhydrat (11 g, 0,066 mol) und Natriumsulfat (70 g) in Wasser (240 ml) wurde eine Suspension des 3,4-Dichloroanilins (10 g, 0,0617 mol) in Wasser (40 ml) und 37-proz. Salzsäure (5,3 ml, 0,064 mol) gegeben. Zu dieser Mischung wurde eine Lösung von Hydroxylamin Hydrochlorid (13,5 g, 0,195 mol) in Wasser (60 ml) hinzugefügt. Das Reaktionsgemisch wurde 1 h unter Rückfluß gekocht, wobei eine klare Reaktionslösung entstand, aus der schon in der Wärme ein Reaktionsprodukt ausfiel. Der Ansatz wurde 16 h bei Raumtemperatur gerührt und das gewünschte Oxim nach Filtration und Waschen mit Wasser ($3 \times 50$ ml) in einer Ausbeute von 91 % (13,1 g) mit einem Schmelzpunkt von 179 °C als gelber Feststoff erhalten.

**5,6-Dichlor-1H-indol-2,3-dion und 4,5-Dichlor-1H-indol-2,3-dion**

Das soeben erhaltene Oxim (13 g, 0,055 mol) wurde innerhalb von 15 min bei 50-60 °C in 96-proz. Schwefelsäure (60 ml) gegeben. Anschließend wurde die Reaktionsmischung 15 min unter Rühren auf 80 °C erwärmt. Nach dem Abkühlen wurde der Ansatz langsam auf Eis (500 g) gegossen. Der entstandene Feststoff wurde nach 30 min abgesaugt. Es wurde ein Gemisch der aus 5,6-Dichlor-1H-indol-2,3-dion und 4,5-Dichlor-1H-indol-2,3-dion erhalten (10,6 g, 90 %).

Die beiden Isatine lagen in einem Verhältnis von 1 : 4 vor. Durch chromatographische Trennung des Isomerengemisches [Kieselgel G (500 g); Ethylacetat/Cyclohexan 1 : 2 (4,8 l), 1 :1 (2,0 l), Ethylacetat (1,8 l)] wurde das 5,6-Dichlor-1H-indol-2,3-dion in einer Ausbeute von 14 % (1,7 g) und das 4,5-Dichlor-1H-indol-2,3-dion in einer Ausbeute von 22 % (2,55 g, Fp. 252-253 °C) gewonnen. Beide Verbindungen waren orangefarbene Feststoffe.

**(5,6-Dichlor-3-hydroxy-2-oxo-2,3-dihydro-1H-indol-3-yl)essigsäure-ethylester**

Eine Lösung von 5,6-Dichlor-1H-indol-2,3-dion (1,7 g, 7,9 mmol) in einem Gemisch von Ethanol/Pyridin/Essigsäure [(15 : 5 : 2), 39,5 ml] wurde mit Malonsäure-monoethylester-Kaliumsalz (1,49 g, 8,74 mmol) versetzt und 23 h unter Rückfluß gekocht. Das Reaktionsgemisch wurde eingeengt und mit Toluol codestilliert (3 × 10 ml). Es wurde ein fester roter Rückstand erhalten, der in einem Gemisch aus Wasser (30 ml) und Ethylacetat (30 ml) 20 min gerührt wurde. Die wäßrige Phase wurde mit Ethylacetat (2 × 20 ml) extrahiert. Die organischen Phasen wurden vereinigt, mit 2N Salzsäure gewaschen, getrocknet und eingeengt. Der gewünschte Hydroxyester wurde dabei als roter Feststoff in einer Ausbeute von 89 % (2,13 g) mit einem Schmelzpunkt von 204-208 °C erhalten.

**2-(5,6-Dichlor-1H-indol-3-yl)ethanol (Ind-83)**

Die Lösung des soeben hergestellten (5,6-Dichlor-3-hydroxy-2-oxo-2,3-dihydro-1H-indol-3-yl)essigsäure-ethylesters (2,13 g, 7,0 mmol) in abs. Tetrahydrofuran (15 ml) wurde im Eisbad innerhalb von 15 min mit einer 1 M Boran/THF-Lösung (28,0 ml, 28,0 mmol) versetzt. Die Reaktionsmischung wurde 57 h bei Raumtemperatur gerührt und zur Aufarbeitung unter Rühren in eine Mischung aus Ethylacetat (50 ml) und Wasser (50 ml) gegeben. Die Phasen wurden getrennt und die wäßrige Phase mit Ethylacetat (2 × 30 ml) extrahiert. Die organischen Phasen wurden vereinigt, getrocknet und eingeengt. Dabei wurde des rohe Indol als gelbes Öl erhalten (1,9 g). Eine chromatographischer Reinigung [Kieselgel G (80 g); Ethylacetat/Cyclohexan 1 : 2 (2 l)] lieferte das gewünschte 2-(5,6-Dichlor-1H-indol-3-yl)ethanol (**Ind-83**) als beigefarbenen Feststoff in einer Ausbeute von 60 % (0,96 g) (Fp. 65-67 °C).

<u>**Indolbaustein Ind-85:**</u> **1H-Indol (Ind-85)**

*Indol, CAS-Nr: 120-72-9, kommerziell erhältlich z.B. bei Sigma-Aldrich.*

<u>**Indolbaustein Ind-86:**</u>**1H-Pyrrolo[2,3-b]pyridin (Ind-86)**

*7-Azaindol, CAS-Nr:* 271-63-6, *kommerziell erhältlich z. B. bei Sigma-Aldrich.*

<u>**Indolbaustein Ind-89:**</u>

**2-(Benzo[b]thiophen-2-yl)ethanol (Ind-89)**

Zu einer Lösung von Benzothiophen (5 g, 37,2 mmol) in Diethylether (40 ml) wurde unter Argon innerhalb von 10 min eine 2,5M Lösung von *n*-Buthyllithium in Hexan (18 ml, 45 mmol) bei -70 °C hinzugegeben. Nach 30 min wurde die Reaktionsmischung langsam auf -15 °C erwärmt (30 min). Zu dieser Mischung wurde eine Lösung von Ethylenoxid (4,88 g, 112 mmol) in Diethylether (20 ml) bei -10 °C innerhalb von 20 min hinzugefügt. Die Ethylenoxidlösung wurde durch Kondensation des gasförmigen Ethylenoxids bei -40 °C und Aufnahme in Diethylether hergestellt. Die Reaktionsmischung wurde langsam auf Raumtemperatur erwärmt und 16 h gerührt. Die Aufarbeitung des Ansatzes erfolgte durch Zugabe von gesättigter $NH_4Cl$-Lösung (30 ml) unter Argon und Eiskühlung. Die leicht trübe Lösung wurde filtriert und dann mit Wasser (10 ml) und Diethylether (10 ml) versetzt. Die Phasen wurden getrennt. Die wäßrige Phase wurde mit Diethylether (2 × 30 ml) extrahiert. Die organischen Phasen wurden vereinigt und mit 2N HCl (30 ml) und gesättigter NaCl-Lösung (30 ml) gewaschen. Die organische Phase wurde nach dem Trocknen eingeengt, wobei das Rohprodukt des Alkohols als gelber Feststoff erhalten wurde. Nach chromatographischer Reinigung an Kieselgel (160 g) mit Ethylacetat/Cyclohexan (1 : 5) wurde der Alkohol als weißer Feststoff mit einem Schmelzpunkt von 82-84 °C in einer Ausbeute von 59 % gewonnen.

<u>**Indolbaustein Ind-90:**</u> **1-Phenylsulfonyl-1H-indol (Ind-90)**

*Kommerziell erhältlich CAS : 40899-71-6; z.B. Sigma-Aldrich.*

**<u>Indolbaustein Ind-92:</u> Benzofuran (Ind-92)**

*Kommerziell erhältlich CAS :* 271-89-6 *z.B. bei Sigma-Aldrich.*

**<u>Indolbaustein Ind-94:</u>**

**Benzofuran-3-ylessigsäure-methylester**

KOtBu (0.673 g, 6 mmol) wurde unter Ausschluss von Sauerstoff in trockenem DMF (10 ml) gelöst. Der Ansatz wurde anschließend mit Phosphonoessigsäure-triethylester (0,87 ml, 6 mmol) versetzt. Nach 20 min wurde zu diesem Gemisch eine Lösung von Benzofuran-3(2H)-on (0,536 g, 4 mmol) in trockenem DMF (10 ml, Argon-Atmosphäre) gegeben. Die Reaktionsmischung wurde 1 h bei Raumtemperatur gerührt und danach zur Aufarbeitung auf Eis (50 ml) gegossen. Das erhaltene Gemisch wurde mit Diethylether (4 x 20 ml) extrahiert. Die organische Phase wurde mit Wasser (4 x 20 ml) gewaschen, mit $Na_2SO_4$ getrocknet und anschließend eingeengt. Das entstandene Produkt wurde durch Säulenchromätographie [Kieselgel 60 (50 g); Cyclohexan, Ethylacetat (4 : 1)] gereinigt und in einer Ausbeute von 0,372 g (48 %) als gelbliches Öl erhalten.

**2-(Benzofuran-3-yl)ethanol**

$LiAlH_4$ (1,025 g, 37,95 mmol) wurde unter Ausschluss von Sauerstoff in Diethylether suspendiert. Anschließend wurde der Ansatz langsam mit einer Lösung des soeben hergestellten Benzofuran-3-ylessigsäure-methylesters (2,546 g, 13,4 mmol) in Diethylether (15 ml) versetzt und 30 min bei Raumtemperatur gerührt. Der Verlauf der Reaktion wurde durch DC verfolgt. Zur vollständigen Hydrolyse des überschüssigen Hydrids wurde zu dem Ansatz eine Mischung aus Wasser (2 ml) und Diethylether (5 ml) vorsichtig zugetropft. Die erhaltene etherische Lösung wurde über Kieselgur filtriert und der Filterkuchen mit Diethylether gespült. Nach Entfernung des Lösungsmittels wurde der gewünschte Alkohol in einer Ausbeute von 1,93 g (89 %) als hellgelbes Öl erhalten und ohne weitere Reinigung zur weiteren Synthese eingesetzt.

**3-(2-Bromethyl)benzofuran**

Triphenylphosphandibromid (5,52 g, 14,41 mmol) wurde in abs. Acetonitril (15 ml) unter Argon suspendiert, im Wasserbad auf 19 °C gebracht und innerhalb von 15 min mit 2-(Benzofuran-3-yl)ethanol (2,11 g, 13,1 mmol), gelöst in abs. Acetonitril (7 ml), versetzt. Während der Zugabe wurde die Temperatur des Reaktionsgemisches zwischen 19 und 21 °C gehalten. Anschließend wurde der Ansatz 12 h ohne weitere Kühlung stehen gelassen. Das während dieser Zeit ausgefallene Triphenylphosphan wurde durch Filtration aus dem Reaktionsgemisch entfernt. Das erhaltene Filtrat wurde eingeengt. Zur vollständigen Entfernung des Phosphans wurde der erhaltene Rückstand in Cyclohexan (20 ml) aufgenommen und über eine etwa 3 cm dicke Kieselgelschicht (15 g) filtriert. Das Kieselgel wurde mit Cyclohexan ($5 \times 20$ ml) gewaschen Die so erhaltene Lösung, die das gewünschte Bromid in reiner Form enthält, wurde am Rotationsverdampfer eingeengt. Dieses wurde in einer Ausbeute von 2,47 g (87 %) als gelbliches Öl isoliert.

**Thioschwefelsäure-S-[2-(benzofuran-3-yl)ethyl]ester-natriumsalz**

Natriumthiosulfat (5,44 g, Pentahydrat, 21,9 mmol) wurde in Wasser (22 ml) gelöst und innerhalb von 10 min unter Rühren mit dem in Ethanol (40 ml) gelösten 3-(2-Bromethyl)benzofuran (2,90 g, 12,9 mmol) versetzt. Die Reaktionsmischung wurde anschließend unter Rückfluß gekocht. Nach 4 h war die Umsetzung beendet (DC-Kontrolle). Zur Aufarbeitung wurde das im Lösungsmittelgemisch enthaltene Ethanol im Vakuum abdestilliert. Der wäßrige Rückstand wurde mit Diethylether ($3 \times 20$ ml) extrahiert, die organische Phase mit Wasser ($2 \times 20$ ml) gewaschen. Die vereinigten wäßrigen Phasen wurden am Rotationsverdampfer eingedampft. Der so erhaltene weiß-gelbliche Rückstand (3,63 g) enthält x mol Wasser. Die Umsetzung zum Thiol erfolgte ohne weitere Reinigung.

**2-(Benzofuran-3-yl)ethanthiol (Ind-94)**

Unter Sauerstoffausschluß (Argonatmosphäre) wurde das soeben hergestellte Thioschwefelsäure-S-[2-(benzofuran-3-yl)ethyl]ester-natriumsalz (3,63 g, enthält x mol Wasser) in 50-proz. Phosphorsäure (60 ml) suspendiert. Das erhaltene Reaktionsgemisch wurde anschließend mit Diethylether (75 ml) überschichtet und unter kräftigem Rühren am Rückfluß (7 h) erhitzt, bis in der wäßrigen Phase kein Feststoff mehr zu beobachten war. Nach Abkühlung wurden die beiden Phasen getrennt und die wäßrige Phase mit Diethylether ($4 \times 15$ ml) extrahiert. Die vereinigten etherischen Phasen wurden mit Wasser ($2 \times 10$ ml) gewaschen und über Natriumsulfat getrocknet. Der nach Entfernung des Diethylethers erhaltene Rückstand (gelbliches Öl, 1,71 g) enthielt laut NMR ca. 80 % des gewünschten Thiols Ind-94.

Einfache Reinigungs- und Löseversuche zeigten, daß Ind-94 relativ unbeständig ist und schon beim Stehen in einer etherischen Lösung Nebenprodukte entstehen (Nachweis durch NMR-Spektroskopie). Deshalb wurde das erhaltene Rohprodukt ohne weitere Aufarbeitung zur Thioethersynthese eingesetzt.

**<u>Indolbaustein Ind-95:</u> 3-Methylbenzo[b]thiophen (Ind-95)**

Kommerziell erhältlich CAS 1455-18-1 bei z.B. Acros Organics.

**<u>Indolbaustein Ind-100:</u>**

**1-Benzyl-3-[2-(1*H*-indol-3-yl)-ethyl]-harnstoff (Ind-100)**

Tryptamin (1.04 g, 6.5 mmol) wurde unter Stickstoffatmosphäre in einem Gemisch aus Aceton (20 ml) und Triethylamin (1 ml) gelöst. Bei 0 °C wurde Benzylisocyanat (952 mg, 7.15 mmol, 0.88 ml) zügig zugetropft, anschließend wurde der Ansatz 2 h unter Eiskühlung und 2 h bei RT nachgerührt. Ein anschließendes DC in Chloroform/Methanol 20:1 zeigte nur noch geringe Mengen an Tryptamin. Der Ansatz wurde i. Vak. eingeengt. Der erhaltene Rückstand wurde durch Flashchromatographie mit 100 g Kieselgel und Chloroform/Methanol 20:1→9:1→4:1 gereinigt.
*Ausbeute:* 1.72 g (90 %)

**<u>Indolbaustein Ind-101:</u>**

**1-(2-(1H-Indol-3-yl)ethyl)-3-phenyl-harnstoff (Ind-101)**

Tryptamin (1.04 g, 6.5 mmol) wurde unter Stickstoffatmosphäre in einem Gemisch aus Aceton (20 ml) und Triethylamin (1 ml) gelöst. Bei 0 °C wurde Phenylisocyanat (852 mg, 7.15 mmol, 0.78 ml) zügig zugetropft, anschließend wurde der Ansatz 2 h unter Eiskühlung und über Nacht bei RT nachgerührt. Ein anschließendes DC in Chloroform/Methanol 20:1 zeigte nur noch geringe Mengen an Tryptamin. Der Ansatz wurde i. Vak. eingeengt. Der erhaltene Rückstand wurde durch Flashchromatographie mit 100 g Kieselgel und Chloroform/Methanol 50:1→9:1 gereinigt. *Ausbeute:* 928 mg (51 %)

**<u>Indolbaustein Ind-102:</u>**

**1-Cyclopentyl-3-[2-(1*H*-indol-3-yl)-ethyl]-harnstoff (Ind-102)**

Tryptamin (1.04 g, 6.5 mmol) wurde unter Stickstoffatmosphäre in einem Gemisch aus Aceton (20 ml) und Triethylamin (1 ml) gelöst. Bei 0 °C wurde Cyclopentylisocyanat (795 mg, 7.15 mmol, 0.81 ml) zügig zugetropft, anschließend wurde der Ansatz 2 h unter Eiskühlung und über Nacht bei RT nachgerührt. Aus der Lösung war ein weißer Feststoff ausgefallen, der abgesaugt und mit Aceton nachgewaschen wurde. Es handelte sich dabei um 641 mg reines Produkt. Das Filtrat wurde i. Vak. eingeengt und anschließend mit Aceton (10 ml) durchgerührt. Der nicht gelöste weiße Feststoff wurde abgesaugt und mit Aceton nachgewaschen, so wurden weitere 612 mg sauberes Produkt erhalten.
*Ausbeute:* 1.25 g (71 %)

**<u>Indolbaustein Ind-103:</u>**

**Cyclopentansulfonsäure [2-(1*H*-indol-3-yl)-ethyl]-amid (Ind-103)**

Tryptamin (950 mg, 5.93 mmol) wurde in abs. THF (30 ml) vorgelegt und bei RT mit Triethylamin (0.82 ml, 5.93 mmol) versetzt. Anschließend wurde bei RT Cyclopentansulfonylchlorid (1.00 g, 5.93 mmol) zügig zugetropft und der Ansatz 1 d bei RT gerührt. Ein anschließendes DC in Chloroform/Methanol 20:1 zeigte nur noch geringe Mengen an Tryptamin. Der Ansatz wurde i. Vak. eingeengt, der erhaltene Rückstand wurde in Essigester (20 ml) aufgenommen und mit gesättigter $NaHCO_3$-Lösung (2x 20 ml) gewaschen. Ein Niederschlag fiel aus der Lösung aus, dieser wurde abgesaugt, es handelte sich jedoch laut DC nicht um das Produkt. Die organische Phase wurde mit gesättigter NaCl-Lösung (2x 20 ml) gewaschen, über $Na_2SO_4$ getrocknet und i. Vak. eingeengt.
*Ausbeute:* 749 mg (43 %)

**Indolbaustein Ind-104:**

**N-[2-(1*H*-Indol-3-yl)-ethyl]-benzensulfonamid [2-(1*H*-indol-3-yl)-ethyl]-amid (Ind-104)**

Tryptamin (955 mg, 5.96 mmol) wurde in abs. THF (30 ml) vorgelegt. Anschließend wurde TEA (888 μL, 6.45 mmol) und Benzosulfonsäurechlorid (826 μL, 6.45 mmol) zugegeben und 16 h bei RT gerührt. Der Ansatz wurde i. Vak. bis zur Trockene eingeengt. Der Rückstand wurde in EE (20 ml) aufgenommen und mit gesättigter $NaHCO_3$- Lösung (2 x 20 ml) und mit NaCl-Lösung (2 x 20 ml) gewaschen. Die organische Phase wurde über $Na_2SO_4$ getrocknet und i. Vak. eingeengt. *Ausbeute:* 1.80 g (100 %)

**Indolbaustein Ind-105:**

**Thiophen-2-sulfonsäure [2-(1*H*-)ndo)-3-yl)-ethyl]-amid [2-(1*H*-indol-3-yl)-ethyl]-amid (Ind-105)**

Tryptamin (640 mg, 4.0 mmol) wurde in abs. THF (30 ml) vorgelegt. Anschließend wurde TEA (596 μL. 4.3 mmol) und 2-Thiophensulfonylchlorid (785 mg, 4.3 mmol) zugegeben und 5 h bei RT gerührt. Der Ansatz wurde i. Vak. bis zur Trockene eingeengt. Der Rückstand wurde in EE (20 ml) aufgenommen und mit gesättigter $NaHCO_3$- Lösung (2 x 20 ml) und mit NaCl-Lösung (2 x 20 ml) gewaschen. Die organische Phase wurde über $Na_2SO_4$ getrocknet und i. Vak. eingeengt. *Ausbeute:* 1.38 g (100% )

**Indolbaustein Ind-106:**

***N*-[2-(1*H*-Indol-3-yl)-ethyl]-nicotinamid (Ind-106)**

Tryptamin (640 mg, 4.0 mmol) wurde in abs. THF (30 ml) vorgelegt. Anschliessend wurde TEA (596 μL, 4.3 mmol) und Nicotinsäurechlorid Hydrochlorid (770 mg, 4.3 mmol) zugegeben und 5 h bei RT gerührt. Der Ansatz wurde i. Vak. bis zur Trockene eingeengt. Der Rückstand wurde in EE (20 ml) aufgenommen und mit gesättigter $NaHCO_3$-Lösung (2 x 20 ml) und mit NaCl-Lösung (2 x 20 ml) gewaschen. Die organische Phase wurde über $Na_2SO_4$ getrocknet und i. Vak. eingeengt. *Ausbeute:* 482 mg (45 %)

**Indolbaustein Ind-107:**

**2-(1-Benzolsulfonyl-1H-indol-2-yl)ethanol**

In einem Sulfierkolben mit Magnetrührer, Innenthermometer, Tropftrichter, Gasein- und Ableitungsrohr und Septumkappe wurde unter Argon bei -5°C LDA aus Diisopropylamin (6,7 ml, 48 mmol) und 2,5M *n*-BuLi-Lösung in Hexan (17,6 ml, 44 mmol) in trockenem THF (100 ml) hergestellt. Es wurde 20 min bei -5 °C nachgerührt, dann auf -75°C abgekühlt und 1-(Phenylsulfonyl)indol (10,3 g, 40 mmol) in trockenem THF (80 ml) über einen Zeitraum von 2 h so zugetropft, daß die Innentemperatur -70 °C nicht überstieg. Nach beendeter Zugabe wurde 90 min bei dieser Temperatur nachgerührt. Anschließend wurde bei -15°C Ethylenoxid (6 ml, 120 mmol) in trockenem THF (25 ml) zugetropft. Die Reaktionsmischung wurde über Nacht im Kältebad belassen. Die klare rotbraune Lösung wurde in eine gesättigte $NH_4Cl$-Lösung (100 ml) gegossen. Nach Zugabe von Wasser (30 ml) kam es zur Phasentrennung. Die wäßrige Phase wurde mit Diethylether (2 × 50 ml) extrahiert und die organische Phase mit 2N HCl (30 ml) und gesättigter NaCl-Lösung (30 ml) gewaschen. Die organische Phase wurde nach dem Trocknen eingeengt, wobei das Rohprodukt ein Gemisch aus 2-(1-(Phenylsulfonyl)-1H-indol-2-yl)ethanol, 2-(1H-indol-1-yl)ethanol und dem Ausgangsprodukt darstellte. Durch Chromatographie [Kieselgel G (300 g); Cyclohexan/EtOAc (7 : 1)] konnte das Ausgangsprodukt abgetrennt werden. Das Gemisch aus den beiden Alkoholen (4,7 g) wurde so für die nächste Stufe eingesetzt.

**2-(1H-indol-1-yl)ethanol (Ind-107)**

Zur Abspaltung des Phenylsulfonylrestes aus 2-(1-(Phenylsulfonyl)-1H-indol-2-yl)ethanol wurde das soeben erhaltene Gemisch (4,7 g) in Ethanol (80 ml) und 2M Natronlauge (80 ml) gelöst und unter Rühren 32 h zum Rückfluß erhitzt. Am Rotationsverdampfer wurde das Ethanol abgezogen und der Rückstand mit Wasser (20 ml) verdünnt. Die wäßrige Lösung wurde mit Ether (3 × 70 ml) extrahiert. Die vereinigten organischen Phasen wurden mit Wasser (30 ml) und gesättigter NaCl-Lösung (30 ml) gewaschen. Die organische Phase wurde nach dem Trocknen eingeengt. Es wurde ein dunkelbraunes Öl (3,08 g) erhalten, das an Kieselgel G (200 g); Cyclohexan/EtOAc (3 : 1) in das Nebenprodukt (1,23 g, 16 %. bezogen auf die 1. Stufe) und den gewünschten Alkohol 2-(1H-indot-1-yl)ethanol (1,12 g, 14 %) getrennt wurde.

**Indolbaustein Ind-108:**

**Triethyl-(3-phenylprop-1-inyl)silan** Zu einer Lösung von Prop-2-inylbenzol (5,00 g, 43,0 mmol) in Tetrahydrofuran (60 ml) wurde bei -25 °C *n*-Butyllithium (18,1 ml, 45,3 mmol, 2,5M in Hexan) getropft. Die Temperatur wurde bei -15 bis -20°C gehalten (ca. 5 Minuten). Anschließend wurde die Reaktionsmischung bei 0 bis -5°C 30 min gerührt. Danach wurde bei 0 bis -5°C Triethylchlorsilan (6,9 g, 45,8 mmol) zugetropft (ca. 5 min) und anschließend über Nacht bei Raumtemperatur gerührt. Die Reaktionsmischung wurde im Vakuum eingeengt und der Rückstand mit Wasser (50 ml) versetzt. Die Mischung wurde mit Cyclohexan extrahiert (3 × 30 ml). Die vereinigten organischen Phasen wurden mit Natriumsulfat getrocknet. Die flüchtigen Bestandteile wurden im Vakuum vollständig entfernt. Triethyl-(3-phenylprop-1-inyl)silan wurde als gelbes Öl gewonnen (9,46 g, 95 %, AS 11024).

3-Benzyl-2-triethylsilanyl-1*H*-indol2-Iodanilin (5,48 g, 25,02 mmol), Triethyl-(3-phenylprop-1-inyl)silan (6,34 g, 27,51 mmol), Lithiumchlorid (1,11 g, 26,19 mmol) und Natriumcarbonat (7,95 g, 75,01 mmol wurden in Dimethylformamid (absolut, 70 ml) in einer Argonatmosphäre vereinigt. Anschließend wurde der Katalysator ([Pd(dppf)$Cl_2$ x $CH_2Cl_2$]. 2,05 g, 2,51 mmol) hinzugegeben. Die Lösung wurde 6 h bei 100-106 °C gerührt. Zu der auf Raumtemperatur abgekühlten schwarzen Reaktionsmischung wurden nacheinander Wasser (300 ml) und Ethylacetat (150 ml) gegeben. Nach einstündigem Rühren wurde die Mischung über Celite filtriert. Die Phasen wurden getrennt. Die wäßrige Phase wurde mit Ethylacetat (3 × 50 ml) extrahiert. Die vereinigten organischen Phasen wurden mit Natriumsulfat getrocknet. Nach Filtration wurden die flüchtigen Bestandteile vollständig im Vakuum entfernt. Der Rückstand (10,5 g braunes Öl) wurde chromatographisch getrennt [Kieselgel 60 (300 g); Cyclohexan/Ethylacetat 10 : 1 (2200 ml)]. 3-Benzyl-2-triethylsilanyl-1*H*-indol wurde als braunes Öl (6,44 g, (80 %) isoliert .

**3-Benzyl-1*H*-indol (Ind-108)** 3-Benzyl-2-triethylsilanyl-1*H*-indol (6,37 g, 19,81 mmol) wurde in MeOH (119 ml) gelöst und Salzsäure (5N, 22 ml, 110 mmol) zudosiert. Das Reaktionsgemisch wurde über Nacht bei Raumtemperatur gerührt. Methanol wurde abdestilliert, der wäßrige Rückstand wurde mit Dichlormethan (3 × 20 ml) extrahiert. Die vereinigten organischen Phasen wurden mit Natriumsulfat getrocknet. Nach Filtration wurden die flüchtigen Bestandteile vollständig im Vakuum entfernt. Der Rückstand (brauner Feststoff, 4,97 g) wurde aus Toluol/Hexan (5 + 30 ml) umkristallisiert. Es wurden 3,53 g (86 %) 3-Benzyl-1*H*-indol (*Schmelzpunkt:* 108-110 °C) erhalten.

**Beispiele**

**Beispiel 1: 2-(2-(4-(Dimethylamino)-4-phenylcyclohexyl)-1H-indol-3-yl)ethanol, Citrat**

**(1:1) Diastereomerengemisch**Zu einer Suspension von N,N-Dimethyl-N-{4-phenyl-2',3',4',9'-tetrahydrospiro [cyclohexan-1,1'-pyrano[3,4-b]indol]-4-yl}-amin (unpolares Diastereomer, vgl. W02004043967) (0,72 g, 2,00 mmol) in konz. HCl (60 ml) wurde innerhalb von 2 h sukzessive Sn-Pulver (3,00 g, 25,40 mmol) gegeben. Während der Zugabe entstand eine klare Lösung, die weitere 2 h bei RT gerührt wurde. Zur Aufarbeitung wurde der Ansatz mit gesättigter $Na_2CO_3$-Lösung basisch gestellt und das entstandene Gemisch mit EtOAc (50 ml) versetzt. Da die Phasentrennung unvollständig war, wurden die sowohl in $H_2O$ als auch in EtOAc unlöslichen Bestandteile mittels Filtration abgetrennt. Der Filterkuchen wurde mit EtOAc (5 x 20 ml) gewaschen, die wäßrige Phase wurde mit den jeweiligen Ethylacetatfraktionen extrahiert (5 x). Die vereinigten organischen. Extrakte wurden über $Na_2SO_4$ getrocknet. Nach Filtration des Trockenmittels wurde das Lösungsmittel am Rotationsverdampfer entfernt und der Rückstand (600 mg) aus Toluol (100 ml) umkristallisiert. Es wurde 2-(2-(4-(Dirnethylamino)-4-phenylcyclohexyl)-1H-indol-3-yl)ethanol als Diastereomerengemisch (0,22 g, 30 %) erhalten.

Der Feststoff (0,22 g, 0,60 mmol) wurde in siedendem EtOH (30 ml) gelöst und mit Citronensäure (0,13 mg, 0,67 mmol), gelöst in heißem EtOH (5 ml), versetzt. Die ethanolische Lösung wurde eingeengt (auf ca. 10 ml) und mit Ether (10 ml) versetzt. Der entstandene Niederschlag wurde mittels einer Fritte abgetrennt und getrocknet. Das Citrat (Bsp 1) (0,20 g, 60%, Smp: ab 114°C) wurde als weisser Feststoff erhalten.

**Beispiel 3: (±)-2-(2-(4-(Dimethylarnino)-4-phenylcyclohex-1-enyl)-1H-indol-3-yl)ethyl acetat Hydrochlorid**

Unter Argon wurden Tryptophol (Ind-5, 161 mg, 1 mmol) und Keton (Ket-10, 217 mg, 1 mmol) bei 0°C in HBr/Eisessig (33 %) (5 ml) gelöst. Das Gemisch wurde über Nacht bei RT aufbewahrt. Unter Rühren wurde portionsweise $NaHCO_3$zugesetzt und die flüchtigen Bestandteile im Vakuum abdestilliert. Der Rückstand wurde in EtOAc (20 ml) gelöst und mit gesättigter wässriger $NaHCO_3$-Lösung (3 x 10 ml) gewaschen. Nach Entfernung des Lösungsmittels am Rotationsverdampfer wurde der Rückstand durch Umkristallisation aus MeOH (15 ml) gereinigt. Die freie Base des gewünschten Produktes wurde als weißer Feststoff (289 mg, 72 %) erhalten. Dieser wurde in Ethylmethylketon (5 ml) suspendiert und mit Chlortrimethylsilan (137 µl, 1.1 mmol) versetzt. Das gewünschte Produkt (Bsp 3) fiel als ein Feststoff aus, der abgesaugt und getrocknet wurde (315 mg, 100 %, Smp: 120-122°C)).

**Beispiel 4: (±)-2-(4-(Dimethylamino)-4-phenylcyclohex-1-enyl)-3-(3-aminopropyl)-1H-indol, Citrat (1:1)**

Keton (Ket-10, 435 mg, 2 mmol) und 3-(1H-Indol-3-yl)propan-1-amin (Ind-15, 348 mg, 2 mmol) wurden in DCE (20 ml) gelöst. Anschließend erfolgte eine schnelle Zugabe von Methansulfonsäure (4 ml). Der Ansatz wurde 1 h bei RT gerührt. Das klare, rote Reaktionsgemisch wurde mit $H_2O$ (10 ml) verdünnt und mit 2N NaOH auf pH 11 gestellt. Nach Phasentrennung wurde die wässrige Phase mit DCE (3 x 20 ml) extrahiert. Die organischen Extrakte wurden vereinigt, über $Na_2SO_4$ getrocknet und das Lösungsmittel am Rotationsverdampfer entfernt. Der Rückstand wurde mittels Säulenchromatographie (MeOH) gereinigt und die freie Base des gewünschten Produktes als gelber Feststoff erhalten (400 mg, 54 %).
Zur Herstellung des Citrates wurde das soeben erhaltene Olefin (380 mg, 1,02 mmol) in heißem EtOH (10 ml) gelöst und mit einer heißen Lösung von Citronensäure (196 mg, 1,02 mmol) in EtOH (2 ml) versetzt. Das Gemisch wurde anschließend 16 h bei. 5°C aufbewahrt. Ethanol wurde am Rotationsverdampfer entfernt und das gewünschte Citrat (Bsp 4) als gelber Feststoff erhalten (576 mg, 100 %, Smp: 150-155 °C).
*$^1H$ NMR (400 MHz, DMSO-$d_6$) $\delta$ ppm:* 1.33 (s, broad, 2H), 1.43-1.57 (m, 2H), 1.65-1.82 (m, 2H), 1.99-2.17 (m, 8H), 2.37-2.47 (m, 1H), 2.47-2.54 (m, 3H), 2.54-2.68 (m, 3H), 2.68-2.79 (m, 1H). 6.11-6.19 (m, 1H), 6.85-7.02 (m, 1H). 7.16-7.25 (m, 2H), 7.25-7.35 (m, 2H), 7.37-7.43 (m, 1H), ), 7.43-7.52 (m, 2H),10.52 (s, 1H)
*$^{13}C$ NMR (101 MHz, DMSO-$d_6$) $\delta$ ppm:* 21.8, 26.6,27.0,32.7, 35.2. 38.5. 41.74. 60.1. 110.4. 110.8, 117.8 117.9, 120.5, 124.6, 126.0, 126.8, 127.2, 128.3, 129.7, 134.8, 134.9, 142.5

**Beispiel 6: (±)-3-(2-(4-(Dimethylamino)-4-phenylcyclohex-1-enyl)-1H-indol-3-yl)propan-1-ol Hydrochlorid**

Unter Argon wurde eine Lösung von Keton **(Ket-10,** 1,1 g, 5,07 mmol) und Indol **(Ind-16,** 1,48 g, 6,0 mmol) in DCM (30 ml) bei -78 °C innerhalb von 5 min mit Triftuormethansulfonsäuretrimethylsilylester (1 ml, 5 mmol) versetzt. Das Reaktionsgemisch wurde 60 min bei -78 °C gerührt und schließlich mit Triethylsilan (0.9 ml, 5,6 mmol) versetzt. Das Gemisch wurde über einen Zeitraum von ca. 4 h auf RT erwärmt und weitere 10 h bei RT gerührt. Es wurde 1 N NaOH (40 ml) hinzugegeben und für 60 min gerührt. Es bildete sich ein Niederschlag, der sich bei Zugabe von DCM (30 ml) teilweise löste. Nach Phasentrennung wurde die wässrige Phase mit DCM (3 x 30 ml) extrahiert, die organischen Extrakte vereinigt und mit 1N NaOH (1 x 30 ml) und $H_2O$ (2 x 30 ml) gewaschen. Nach Trocknung über $Na_2SO_4$ wurde das Lösungsmittel am Rotationsverdampfer entfernt und der Rückstand säulenchromatographisch (EtOAc, dann EtOAc/ EtOH (8 : 2)) gereinigt und das gewünschte Olefin (134 mg, 7 %. Smp: 163-167 °C) erhalten.Zur Überführung in das Hydrochlorid wurde das Olefin (120 mg, 0,31 mmol) in Ethylmethylketon (10 ml) gelöst, mit $Me_3SiCl$ (76 $\mu$l. 0,6 mmol) versetzt und 3 h bei RT gerührt. Dabei fiel das Hydrochlorid (Bsp 6) als weißer Feststoff aus (67 mg (52 %, Smp: 212-216 °C).

**Beispiel 7: (±)-2-(5,6-Dichlor-2-(4-(dimethylamino)-4-phenylcyclohex-1-enyl)-1H-indol-3-yl)ethanol, Citrat (1:1)**

Unter Argon wurden Keton **(Ket-10**, 217 mg, 1 mmol) und Indol **(Ind-83,** 230 mg, 1 mmol) in absolutem DCM (50 ml) mit Trifluormethansulfonsäure (0,1 ml, 1,1 mmol) versetzt. Das Reaktionsgemisch wurde 21 h bei RT gerührt. Es fiel ein brauner Feststoff aus. Die Reaktionsmischung wurde mit 1N NaOH (20 ml) versetzt und bei RT 1 h gerührt. Die Phasen wurden getrennt und die wässrige Phase mit DCM (20 ml) extrahiert. Die organischen Phasen wurden vereinigt, über $Na_2SO_4$ getrocknet und das Lösungsmittel nach Filtration des Trockenmittels am Rotationsverdampfer entfernt. Der Rückstand wurde mit 2-Propanol (3 ml) 10 min gerührt, der Rückstand durch Filtration abgetrennt und mit 2-Propanol (3 $\times$ 2 ml) gewaschen. Das Filtrat wurde am Rotationsverdampfer eingeengt (312 mg) und chromatographisch (MeOH=10: 1 (1,0 l), 4:1 (0,5 l). MeOH (0,51) gereinigt. Dabei wurde das gewünschte Olefin als gelber Feststoff (87 mg, 20 %, Smp: 116-119 °C) gewonnen. Dieser wurde zur Herstellung des Citrates mit EtOH (15 ml) versetzt und auf 50 °C erwärmt. Die trübe Lösung wurde mit Citronensäure (42 mg, 0,22 mmol), gelöst in warmem EtOH (4 ml), versetzt. Es resultierte eine klare Lösung, aus der beim Abkühlen auf RT ein Feststoff ausfiel. Es wurde 16 h bei RT gerührt und die Mischung 2 h bei 5 °C aufbewahrt. Das gewünschte Citrat (Bsp 7) wurde abgesaugt und mit EtOH (2 $\times$ 5 ml) gewaschen (59 mg, 48%. Smp: 210-212 °C).
*$^1H$ NMR (300 MHz, DMSO-$d_6$) $\delta$ ppm:* 1.64-1.78 (m, 1H), 2.16-2.05 (m, 2H), 2.21 (s, 6H), 2.37-2.48, (m, 2H), 2.60 (dd, 4H), 2.73-2.88 (m, 3H), 6.16 (t, 1H). 7.17-7.23 (m, 1H). 7.23-7.31 (m, 2H), 7.38 (d, 1H). 7.43 (d, 2H), 7.66 (d, 1H). 10.97 (s, 1H)

**Beispiel 8: (±)-2-(2-(4-Morpholino-4-phenylcyclohex-1-enyl)-1H-indo)-3-yl)ethanol, Citrat (1:1)**

Keton **(Ket-9,** 259 mg, 1 mmol) und Tryptophol **(Ind-5,** 1 mg, 1 mmol) wurden in absolutem DCM (50 ml) vorgelegt und mit Trifluormethansulfonsäure (0,1 ml, 1,1 mmol) versetzt. Es wurde 15 h bei RT gerührt. Es bildete sich ein hellbrauner Niederschlag. Das Reaktionsgemisch wurde mit 1N NaOH (20 ml) versetzt und 16 h bei RT gerührt.

Nach Phasentrennung wurde die wässrige Phase mit DCM (2 × 20 ml) extrahiert. Die organischen Phasen wurden vereinigt und über $Na_2SO_4$ getrocknet. Entfernung des Lösungsmittels am Rotationsverdampfer lieferte das gewünschte Olefin als beigefarbenen Feststoff (401 mg, 99 %). Dieser wurde in EtOH (5 ml) gelöst und mit Citronensäure (211 mg, 1.1 mmol), gelöst in EtOH (5 ml), versetzt. Es wurde 16 h bei RT gerührt und die Mischung 2 h bei 5 °C aufbewahrt. Der Feststoff wurde abfiltriert, das Filtrat auf 5 ml eingeengt und mit Ether (50 ml) versetzt. Der ausgefallene Feststoff wurde abgesaugt und mit EtOH (2 × 10 ml) gewaschen. Das gewünschte Citrat (Bsp 8) konnte als gelber Feststoff (269 mg, 45 %, Smp: 125-137 °C) isoliert werden.

**Beispiel 9: (±)-2-(4,6-Dichlor-2-(4-(dimethylamino)-4-phenylcyclohex-1-enyl)-1H-indol-3-yl)ethanol, Citrat (1:1)**

Keton (Ket-10, 217,3 mg, 1 mmol) wurde mit Indol (Ind-31, 230,1 mg, 1 mmol) in absolutem DCM (40 ml) vorgelegt. Anschließend erfolgte die Zugabe von Trifluormethansulfonsäure (0,1 ml, 1,1 mmol). Der Ansatz wurde 20 h bei RT gerührt. Die klare hellbraune Lösung wurde mit 1 N NaOH (20 ml) versetzt und eine weitere Stunde kräftig gerührt. Zwischen den Phasen setzte sich ein weißer Feststoff ab. Dieser wurde abgesaugt, mit DCM (20 ml) gewaschen und auf diese Weise das gewünschte Olefin (416 mg, 97 %, Smp: 255-258 °C) erhalten. Zur Herstellung des Citrates wurde ein Teil des Feststoffes (341 mg, 0,79 mmol) in EtOH (60 ml) unter Erwärmung gelöst und mit Citronensäure (168 mg, 0,87 mmol), gelöst in EtOH (5 ml), versetzt. Es wurde 6 h bei RT gerührt und die Mischung 20 h bei 5 °C aufbewahrt. Die Lösung wurde am Rotationsverdampfer auf ca. 10 ml eingeengt, mit Ether (20 ml) versetzt und 1h gerührt. Der ausgefallene Feststoff wurde abgesaugt und mit Ether (10 ml) gewaschen. Das Citrat (9) fiel als weißer Feststoff (444 mg, 91%, Smp: 151-154 °C) an.

**Beispiel 10: (±)-2-(2-(4-(Dimethylamino)-4-phenytcyctohex-1-enyl)-5-fluor-1H-indol-3-yl)ethanol, Citrat (1:1)**

Eine Suspension von N,N-Dimethyl-N-{4-phenyl-6'-fluor-1',3',4',9'-tetrahydrospiro[cyclohexan-1,1'-pyrano [3,4-b]indol]-4-yl}amin (unpolares Diastereomer, vgl. WO2004043967) (400 mg, 1,06 mmol) (20 ml) wurde in konz. HCL 18 h bei RT gerührt. Die anfangs trübe Lösung klarte im Laufe der Zeit auf. Anschließend wurde mit gesättigter $Na_2CO_3$-Lösung basisch gestellt. Der ausgefallene Feststoff wurde über eine Fritte abgetrennt und so das gewünschte Olefin (340 mg, 84 %, Smp: 216-221 °C) erhalten. Dieses (340 mg, 0,89 mmol) wurde in 30 ml siedendem Isopropanol gelöst und mit Citronensäure (170 mg, 0,88 mmol), gelöst in heißem Isopropanol (5 ml), versetzt. Das Reaktionsgemisch wurde auf RT abgekühlt und das Gemisch am Rotationsverdampfer auf ca. 10 ml eingeengt. Hierbei fiel ein Niederschlag aus, welcher mittels einer Fritte abgetrennt wurde. Trocknung im Vakuum lieferte Beispiel 10 (180 mg, 42 %).

**Beispiel 11: 2',3',4',9'-Tetrahydro-N,N-dimethyl-4-(phenyl)-6'-(pyridin-4-yl)-spiro[cyclohexan-1,1'(1'H)-pyrido [3,4-b]indol]-4-amin (Diastereomerengemisch)**

Das Keton (Ket-10, 245,4 mg, 1,13 mmol) wurde mit dem Indol **(Ind-28,** 270,0 mg, 1,13 mmol) in absolutem 1,2-Dichlorethan (35 ml) vorgelegt. Anschließend erfolgte die tropfenweise Zugabe der Methansulfonsäure (220,6 μl, 3,39 mmol). Der Ansatz wurde 16 h bei RT gerührt. Die Reaktionsmischung wurde nun auf 75°C erwärmt und 7 h bei dieser Temperatur gerührt. Dabei fiel ein hellgelber Niederschlag aus. Dieser wurde bei Raumtemperatur abgesaugt und mit 1,2-Dichlorethan (3 × 2 ml) sowie mit Diethylether (2 × 2 ml) gewaschen und anschließend getrocknet. Ausbeute (Diastereomerengemisch): 660 mg (93 %); *Schmelzpunkt:* 190-197 °C.

**(±)-2-(2-(4-(Dimethylamino)-4-phenylcyclohex-1-enyl)-5-(pyridin-3-yl)-1H-indol-3-yl)ethanol, Citrat (1:1)**

Das soeben hergestellte Sulfonat (763 mg, 1,21 mmol) war in Wasser (28 ml) klar löslich. Die Lösung wurde mit 1 N Natronlauge (pH 11) versetzt und eine Stunde kräftig gerührt. Der voluminöse Niederschlag löste sich in Dichlormethan (100 ml). Die klaren Phasen wurden getrennt. Die wäßrige Phase wurde mit Dichlormethan (3 × 10 ml) extrahiert. Die organischen Extrakte wurden vereinigt, getrocknet und dann eingeengt. Der Rückstand war ein hellgelber Feststoff (477,5 mg), der im DC dem Gemisch des Spiroethers und dem gewünschten Olefin entsprach. Die Trennung des Gemisches wurde durch zweimalige Flash-Chromatographie [Kieselgel 60 je (50 g); 1. Säule Eluent: MeOH/EtOAc (1 : 7; 400 ml), MeOH/EtOAc (1 : 1; 1000 ml); 2. Säule Eluent: $CH_2Cl_2$/MeOH/EtOAc (10 : 1 : 1; 480 ml), $CH_2Cl_2$MeOH/EtOAC (2 : 1 : 1; (800 ml)] erreicht. Das Olefin wurde so in einer Ausbeute von (20 mg, Fp. 202-207 °C, 4 %) gewonnen.
*$^1$H NMR (300 MHz, $CDCl_3$-$d_6$) δ ppm:* 1.61-1.78 (m, 2H), 2.15-2.18 (m, 2H), 2.21 (s, 6H), 2.32-2.46 (m, 1H), 2.58-2.85 (m, 2H), 3.03 (t, J = 6.93, 6.93 Hz, 2H), 3.78 (t, J = 6.75, 6.75 Hz, 2H), 6.21-6.34 (m,1H), 7.23-7.28 (m, 1H), 7.29-7.40 (m,4H), 7.42-7.51 (m, 2H), 7.71 (s,1H), 7.88-7.94 (m, 1H), 7.94-7.99 (m, 1H), 8.53 (dd, J = 4.78, 1.56 Hz, 1H), 8.86 (d, J = 1.64 Hz, 1H)
$^{13}$C NMR (101 MHz,$CDCl_3$) *δ ppm:* 27.1, 27.5, 28.2, 33.2, 38.5, 60.9, 63.2, 108,4, 111.1, 117.3, 121.5, 123.4, 126.7, 127.1, 127.2, 127.8, 130.1, 134.4, 137.8, 142.6, 147.5, 148.5 Das Racemat des soeben erhaltenen Olefins I (20 mg,

0,046 mmol) wurde in Ethanol (5 ml) unter Erwärmen gelöst und mit Citronensäure (19,3 mg, 0,101 mmol), gelöst in Ethanol (1 ml), versetzt. Die Mischung wurde 1 h bei RT gerührt. Auch bei Kühlung fiel kein Niederschlag aus. Die Lösung wurde bis auf ca. 1 ml eingeengt, mit Diethylether (3 ml) versetzt und 2 h gerührt. Die Mutterlauge über dem abgesetzten Feststoff wurde vorsichtig dekantiert. Der Feststoff zweimal mit Diethylether (2 ml) gespült und die überstehende Lösung wieder abpipettiert. Der Rückstand wurde im Vakuum getrocknet. Das Citrat des Olefins (Bsp 11) wurde als gelber Feststoff in einer Ausbeute von 98 % (28,2 mg, Schmelzpunkt nicht bestimmbar) erhalten.

**Beispiel 13: (±)-2-(2-(4-(Dimethylamino)-4-phenylcyclohex-1-enyl)-5-nitro-1H-indol-3-yl)ethanol**

Das Keton (Ket-10, 1,305 g, 6,0 mmol) wurde zusammen mit dem Indol (Ind-27, 1,24 g, 6 mmol) in trockenem Dichlormethan (60 ml) gelöst. Bei RT wurde Trifluormethansulfonsäure (1,19 ml, 6 mmol) schnell zugegeben, wobei sich die Lösung braun färbte. Der Ansatz wurde weitere 48 h bei RT gerührt. Die Reaktion wurde durch DC kontrolliert. Zur Aufarbeitung wurde der Ansatz mit 5N NaOH (50 ml) versetzt und 30 min gerührt. Die organische Phase wurde abgetrennt. Die wäßrige Phase wurde mit Dichlormethan (5 × 30 ml) extrahiert. Die organische Phase wurde über $Na_2SO_4$ getrocknet und anschließend eingedampft. Das zurückgebliebene gelbe Öl war ein Gemisch aus dem gewünschten Olefin und einem Nebenprodukt. Das Gemisch konnte durch Säulenchromatographie [Kieselgel 60 (200 g); MeOH (2000 ml)] getrennt und gereinigt werden. Das Olefin wurde als gelber Feststoff in einer Ausbeute von 1,23 g (51 %) erhalten.

*$^1H$ NMR (400 MHz, DMSO-$d_6$) δ* ppm: 1.58-1.83 (m, 1H), 1.94-2.18 (m, 8H), 2.32-2.46 (m, 1H). 2.52-2.70 (m, 2H), 2.85-3.03 (m, 2H), 3.34-3.50 (m, 2H), 6.18-6.27 (m, 1H), 7.09-7.17 (m, 1H), 7.18-7.26 (m, 2H), 7.32-7.41 (m, 3H), 7.92 (dd, J = 8.92, 2.27 Hz, 1H), 8.45 (t, J = 2.01 Hz, 1H), 11.43 (s, 1H)

*$^{13}C$ NMR (101 MHz, DMSO-$d_6$) δ ppm:* 24.9, 26.3, 26.9, 32.5, 60.0, 70.6, 110.0, 111.9, 115.5, 1164. 126.3, 126.8, 127.4, 127.7, 128.0, 128.8, 138.5, 139.7, 140.2, 142.5

**(±)-2-(2-(4-(Dimethylamino)-4-phenylcyclohex-1-enyl)-5-nitro-1H-indol-3-yl)ethanol, Citrat (2:1)**

Zur Herstellung des Citrates wurde das Olefin (360 mg, 1 mmol) in heißem Ethanol (50 ml) gelöst und mit einer ebenfalls heißen Lösung von Citronensäure (194 mg, 1 mmol) in Ethanol (5 ml) versetzt. Nach dem Abkühlen auf 5°C wurde der Ansatz 16 h stehen gelassen. Der entstandene Feststoff wurde abgesaugt und getrocknet. Das gewünschte Hemicitrat wurde so in einer Ausbeute von 276 mg (50 %) als weißer Feststoff (*Schmelzpunkt:* 199-203 °C) erhalten.

*$^1H$ NMR (300 MHz, DMSO-$d_6$) δ* ppm: 1.58-1.82 (m, 1H), 2.00-2.21 (m, 8H), 2.33-2.82 (m), 2.82-3.02 (m, 2H), 3.25-3.48 (m, 2H), 6.14-6.29 (m, 1H), 7.10-7.21 (m, 1H), 7.21-7.31 (m, 2H), 7.32-7.47 (m, 3H), 7.93 (dd. J = 8.95, 2.22 Hz, 1H), 8.41-8.51 (m, 1H), 11.47 (s, 1H)

**Beispiel 14: (±)-2-(2-(4-(Benzo[b]thiophen-2-yl)-4-(dimethylamino)cyclohex-1-enyl)-1H-indol-3-yl)ethanol, Citrat (1:1)**

Keton **(Ket-6,** 0.220 g, 0.804 mmol) und Tryptophol (Ind-5, 0.130 g, 0.804 mmol) wurden in abs. Dichlormethan (10 ml) unter Argon vorgelegt, anschließend mit Methansulfonsäure (0.078 ml, 0.881 mmol) versetzt und bei Raumtemperatur über Nacht gerührt. Der Ansatz wurde mit 1N NaOH versetzt und mit Dichlormethan (3 x 15 ml) extrahiert, die org. Phase über $Na_2SO_4$ getrocknet und i. Vak. eingeengt, anschließend das Produkt mittels Flashchromatographie mit Chloroform/Methanol (9:1) gereinigt.

*Ausbeute:* 0.07 g, 21.2 %

*$^1H$-NMR (DMSO-$d_6$):* 2.01 (2 H, m); 2.19 (1 H, m); 2.24 (6 H, s); 2.73 (2 H, s); 2.91 (2 H, t); 3.34 (1 H, m); 3.53 (2 H, m); 4.71 (1 H, t, OH); 6.25 (1 H, bs); 6.96 (2 H, m); 7.27 (4 H, m); 7.41 (1 H, m); 7.73 (1 H, m); 7.88 (1 H, m); 10.66 (1 H, bs).

*$^{13}C$-NMR (DMSO-$d_6$):* 26.44; 28.74; 28.97; 33.81; 38.32; 59.98; 61.84; 79.12; 107.53; 110.65; 118.09; 118.26; 120.85; 121.46; 122.07; 123.12; 123.84; 124.39; 128.72; 129.29; 135.18; 135.81; 138.87; 139.17; 150.93.

Das soeben erhaltene Olefin (0.07 g, 0.168 mmol) wurde in heißem Ethanol (2.5 ml) gelöst und bei Raumtemperatur mit Citronensäure (0.033 g, 0.168 mmol), gelöst in heißem Ethanol (1 ml), versetzt. Dann wurde die Reaktionslösung i. Vak. eingeengt und es verblieb ein bräunlicher Feststoff.

*Ausbeute:* 99 mg (97 %) Beispiel 14; *Schmelzpunkt:* 95-97°C

**Beispiel 15: (±)-2-(2-(4-(Benzo[b]thiophen-2-yl)-4-(dimethylamino)cyclohex-1-enyl)-5-fluor-1H-indol-3-yl)ethanol, Citrat (1:1)**

Keton (Ket-6, 0.220 g, 0.804 mmol) und Indol (Ind-4, 0.194 g, 0.804 mmol) wurden in abs. Dichlormethan (10 ml) unter Argon vorgelegt, anschließend mit Methansulfonsäure (0.078 ml, 0.881 mmol) versetzt und bei Raumtemperatur über Nacht gerührt. Der Ansatz wurde mit 1 N NaOH versetzt und mit Dichlormethan (3 x 15 ml) extrahiert, die org.

Phase über $Na_2SO_4$ getrocknet und i. Vak. eingeengt, anschließend das Produkt mittels Flash-Chromatographie mit Chloroform/Methanol (9:1) gereinigt. *Ausbeute:* 0.130 g (37%) *$^1$H-NMR (DMSO-$d_6$):* 2.01 (2 H, m); 2.25 (7 H, m); 2.74 (2 H, s); 2.87 (2 H, t); 3.29 (1 H, m); 3.53 (2 H, m); 4.63 (1 H, t, OH); 6.26 (1 H, bs); 6.82 (2 H, m); 7.20 (3 H, m); 7.73 (1 H, m); 7.86 (1 H, m); 10.72 (1 H, bs).
*$^{13}$C-NMR (DMSO-$d_6$):* 26.38; 28.61; 28.97; 33.77; 38.31; 59.94; 61.70; 79.12; 102.72; 102.95; 108.08; 108,65; 108.90; 111.54; 121.48; 122.07; 123.13; 123.85; 125.09; 129.10; 131.78; 137.91; 138.88; 139.15; 150.83; 157.79.

Das soeben erhaltene Olefin (0.130 g, 0.298 mmol) wurde in heißem Ethanol (2.5 ml) gelöst und mit Citronensäure (0.058 g, 0.298 mmol), gelöst in heißem Ethanol (1.5 ml), versetzt. Dann wurde die Reaktionslösung i. Vak. eingeengt und es verblieb ein bräunlicher Feststoff.
*Ausbeute:* 0.151 g (83 %) Beispiel 15; *Schmelzpunkt:* 92-104 °C

**Beispiel 16: (±)-2-(4-(Dimethylamino)-4-phenylcyclohex-1-enyl)-3-methyl-1H-indol, Citrat (1:1)**

Skatol (Ind-10, 262 mg, 2 mmol) wurde zusammen mit dem Keton (Ket-10, 434 mg, 2 mmol) in Dichlormethan (20 ml) gelöst und mit Trifluormethansulfonsäure (0,2 ml, 2,3 mmol) versetzt. Der Ansatz wurde 3 Tage bei RT gerührt. - Zur Aufarbeitung wurde das Reaktionsgemisch mit 2N NaOH (10 ml) versetzt. Nach Trennung der Phasen wurde die wässrige Phase mit Dichlormethan (2 x 20 ml) extrahiert. Die vereinigten organischen Extrakte wurden über $Na_2SO_4$ getrocknet und anschließend eingeengt. Das erhaltene Rohprodukt (720 mg) wurde aus Methanol (20 ml) umkristallisiert. Das gewünschte Olefin (Bsp 16) wurde so in einer Ausbeute von 412 mg (62 %) mit einem Schmelzpunkt von 168 - 180°C erhalten.
Die Citratfällung erfolgte analog Beispiel 15.

Beispiel 17: N,N-Dimethyl-4-(3-methyl-1H-indol-2-yl)-1-phenylcyclohexanamin, Citrat (2:1)

**Beispiel 18: N,N-Dimethyl-4-(3-methyl-1H-indol-2-yl)-1-phenylcyclohexanamin, Citrat (2:1)**

***Variante 1:***

**(±)-*N,N*-Dimethyl-*N*-[4-(3-methyl-1*H*-indol-2-yl)-1-phenylcyclohex-3-enyl]amin**

3-Methylindol (Ind-10, 262 mg, 2 mmol) wurde zusammen mit Ket-10 (434 mg, 2 mmol) in Dichlormethan (20 ml) gelöst und mit Trifluormethansulfonsäure (0,2 ml, 2,3 mmol) versetzt. Der Ansatz wurde 3 Tage bei RT gerührt. - Zur Aufarbeitung wurde das Reaktionsgemisch mit 2N NaOH (10 ml) versetzt. Nach Trennung der Phasen wurde die wässrige Phase mit Dichlormethan (2 x 20 ml) extrahiert. Die vereinigten organischen Extrakte wurden über $Na_2SO_4$ getrocknet und anschließend eingeengt. Das erhaltene Rohprodukt (720 mg) wurde aus Methanol (20 ml) umkristallisiert. (±)-*N,N*-Dimethyl-*N*-[4-(3-methyl-1*H*-indol-2-yl)-1-phenylcyclohex-3-enyl]amin wurde so in einer Ausbeute von 412 mg (62 %) mit einem Schmelzpunkt von 168-180 °C erhalten.

***N,N*-Dimethyl-*N*-[4-(3-methyl-1*H*-indol-2-yl)-1-phenylcyclohexyl]amin (polares und unpolares Diastereomer)**

(±)-*N,N*-Dimethyl-*N*-[4-(3-methyl-1*H*-indol-2-yl)-1-phenylcyclohex-3-enyl]amin (550 mg, 1,66 mmol) wurden in HBr/Eisessig (33 % HBr, 20 ml) gelöst. Anschließend wurde zu dem Ansatz bei RT Sn-Pulver (1 g, 8,5 mmol) innerhalb von 30 min portionsweise gegeben. Nach Beendigung der Zugabe wurde das Reaktionsgemisch weitere 30 min gerührt. - Zur Aufarbeitung wurde das Gemisch am Rotationsverdampfer bis zur Trockene eingeengt. Der verbliebene Rückstand wurde durch Zugabe von 2N NaOH (20 ml) basisch gestellt. Das erhaltene wässrige Gemisch wurde mit Ethylacetat extrahiert (4 x 20 ml). Die vereinigten organischen Phasen wurden mit $Na_2SO_4$ getrocknet und anschließend eingeengt. Der erhaltene Rückstand (530 mg) wurde aus Methanol (60 ml) umkristallisiert. *N,N*-Dimethyl-*N*-[4-(3-methyl-1*H*-indol-2-yl)-1-phenylcyclohexyl]amin (unpolareres Isomer) wurde so in einer Ausbeute von 222 mg (40 %, *Schmelzpunkt:* ab 204 °C) erhalten.

Die methanolische Mutterlauge wurde eingeengt. NMR-Untersuchungen des verbliebenen Rückstandes (305 mg, 55 % Ausbeute) zeigten, daß es sich überwiegend um das zweite, polarere Diastereoisomer handelte. Die Verbindung wurde ohne weitere Reinigung zur Citratbildung eingesetzt.

***Variante 2:***

***N,N*-Dimethyl-*N*-[4-(3-methyt-1*H*-indol-2-yl)-1-phenylcyclohexyl]amin (unpolares Diastereomer)**

3-Methylindol (**Ind-10,** 262 mg, 2 mmol) wurde zusammen mit Ket-10 (434 mg, 2 mmol) mit HBr/Eisessig (33

% HBr, 20 ml) versetzt und 22 h bei RT gerührt*). Anschließend wurde zu dem Ansatz bei RT Sn-Pulver (0,5 g) innerhalb von 30 min portionsweise gegeben. Nach beendeter Zugabe wurde die Mischung weitere 30 min gerührt. - Zur Aufarbeitung wurde das Reaktionsgemisch am Rotationsverdampfer bis zur Trockene eingeengt. Der Ansatz wurde mit 2N NaOH basisch gemacht. Die wässrige Mischung wurde mit Ethylacetat versetzt (20 ml). Die unlöslichen Bestandteile des Gemisches wurden mittels einer Fritte abgetrennt. Der Filterkuchen wurde mit Ethylacetat (3 x 20 ml) gewaschen. Die Phasen der Mutterlauge wurden getrennt, die wässrige Phase wurde mit Ethylacetat (4 x 20 ml) extrahiert. Die vereinigten organischen Phasen wurden über $MgSO_4$ getrocknet und anschließend eingeengt. Der erhaltene Rückstand (550 mg) wurde aus Methanol (70 ml, Feststoff weitgehend gelöst) umkristallisiert. Auf diese Weise wurden 110 mg Produkt isoliert. Aus der methanolischen Mutterlauge wurden durch Flash-Chromatographie (Eluent: Ethylacetat) weitere 40 mg Dirnethyl-[4-(3-methyl-1*H*-indol-2-yl)-1-phenylcyclohexyl]amin isoliert (Ausbeute: 150 mg, 28 %, *Schmelzpunkt:* ab 204 °C).

**N,N-Dimethyl-4-(3-methyl-1H-indol-2-yl)-1-phenylcyclohexanamin, Citrat (2:1), unpolares Diastereomer (Bsp 17)**

*N,N*--Dimethyl-[4-(3-methyl-1*H*-indol-2-yl)-1-phenylcyclohexyl]amin (unpolares Isomer, 222 mg, 0,68 mmol) wurde in der Siedehitze in 2-Propanol (50 ml) gelöst und mit Citronensäure (192 mg, 1 mmol), gelöst in heißem Isopropanol (2 ml), versetzt. Die Lösung wurde auf 5 °C abgekühlt (Kühlschrank) und 12 h stehen gelassen. Der entstandene Niederschlag wurde mittels einer Fritte abgetrennt. Beispiel 17 wurde so in einer Ausbeute von 283 mg (99 %, *Schmelzpunkt:* 244 - 252 °C) erhalten.

**N,N-Dimethyl-4-(3-methyl-1H-indol-2-yl)-1-phenylcyctohexanamin, Citrat (2:1), polares Diastereomer**

*N,N*-Dimethyl-[4-(3-methyl-1*H*-indol-2-yl)-1-phenylcyclohexyl]amin (polares Isomer 290 mg, 0,87 mmol) wurde in der Siedehitze in Isopropanol (150 ml) gelöst und mit Citronensäure (254 mg, 1,32 mmol, gelöst in 5 ml Isopropanol) versetzt. Die Reaktionsmischung wurde für 10 min gerührt. Nach Abkühlung auf RT wurde die Reaktionsmischung auf ca. 80 ml eingeengt. Die Lösung wurde 1 h bei Raumtemperatur und anschließend über Nacht bei 5 °C gelagert. Der entstandene Feststoff wurde abgesaugt und verworfen. Die Mutterlauge wurde bis zur Trockene eingeengt. Der Rückstand wurde mit Wasser (7 ml) versetzt und 30 min bei RT kräftig gerührt. Der entstandene gelbe Feststoff wurde abgesaugt und getrocknet. Beispiel 18 wurde so in einer Ausbeute von 185 mg (49 %) als gelber Feststoff mit einem Schmelzpunkt von 224-236 °C erhalten.

**Beispiel 19: (±)-2-(2-(2-(4-(Dimethylamino)-4-phenylcyclohex-1-enyl)-1H-indol-3-yl)ethyl)isoindofin-1,3-dion 2-hydroxypropan-1,2,3-tricarboxylat**

Keton **(Ket-10,** 898 mg, 4.13 mmol) und Indol (Ind-1, 1.20 g, 4.13 mmol) wurden in abs. Dichlormethan (50 ml) unter Argon gelöst. Anschließend wurde Trifluormethansulfonsäure (480 μL/ 5.5 mmol) schnell zugegeben und über Nacht bei RT gerührt. Der Ansatz wurde mit 1 N NaOH alkalisch gestellt und 15 min bei RT nachgerührt. Die Phasen wurden getrennt. Die wässrige Phase wurde mit Dichlormethan (dreimal 20 ml) extrahiert. Die organische Phase wurde über $Na_2SO_4$ getrocknet und i. Vak. eingeengt. Der Rückstand wurde durch Flash-Chromatographie mit $CHCl_3$/MeOH (9:1) gereinigt.
*Ausbeute:* 1.10 g (55 %)
*$^1H$-NMR (DMSO-$d_6$):* 1.63 (2 H, m); 2.09 (6 H, m); 2.48 (2 H, m); 2.64 (2 H, m); 3.00 (2 H, m); 3.69 (2 H, m); 6.24 (1 H, s); 6.97 (2 H, m); 7.20-7.47 (5 H, m); 7.68 (4 H, m); 10.66 (1 H, s).

**2-(2-(2-(4-(Dimethylamino)-4-phenylcyclohexyl)-1H-indol-3-yl)ethyl)isoindolin-1,3-dion, Citrat (1:1) Eines von 2 möglichen Diatereomeren**

Das soeben hergestellte Olefin (1.10 g, 2.24 mmol) wurde in HBr/Eisessig (55 ml) gelöst. Innerhalb von 30 min wurde Zinn (2.60 g, 2.24 mmol) zugegeben und 4 h bei RT gerührt. Der Ansatz wurde mit Ethanol versetzt und über Nacht bei RT gerührt. Anschließend wurde der Ansatz i. Vak. eingeengt, mit 5N NaOH versetzt und mit Dichlormethan (dreimal 20 ml) extrahiert. Die organische Phase wurde über $Na_2SO_4$ getrocknet und i. Vak. eingeengt. Der Rückstand wurde durch Flash-Chromatographie EE/EtOH (1:2 → MeOH + 1 % TEA) gereinigt.

*Ausbeute:* 177 mg (16 %) (Mischfraktion)
432 mg (39 %) polares Diastereomer

Das soeben erhaltene polare Diastereomer (80 mg, 0.162 mmol) wurde in heißem Ethanol (5 ml) gelöst.

Citronensäure (30 mg, 0.162 mmol) wurde in heißen Ethanol (1 ml) gelöst und zugegeben. Der Ansatz wurde abgekühlt, auf die Hälfte eingeengt, der ausgefallene Niederschlag abgesaugt und i. Vak. getrocknet.
*Ausbeute:* 111 mg (Bsp 19; 100 %)
*Schmelzpunkt:* 108-110 °C
*$^1$H-NMR (DMSO-$d_6$):* 1.46 (2 H, m); 1.74 (2 H, m); 1.89 (2 H, m); 2.37 (6 H, s); 2.45-2.65 (4 H, m); 2.97 (2 H, m); 3.75 (2 H, t); 6.89 (2 H, m); 7.13 (1 H, d); 7.46 (6 H, m); 7.64 (4 H, s); 10.44 (1 H, s), Citrat.

**Beispiel 20: (±)-N-(2-(2-(4-(Dimethylamino)-4-phenytcyclohex-1-enyl)-1H-indol-3-yl)ethyl)acetamid**

Keton **(Ket-10,** 234 mg, 1.08 mmol) und Indol (Ind-2, 219 mg, 1.08 mmol) wurden in abs. Dichlormethan (10 ml) unter Argon gelöst, mit Trifluormethansulfonsäure (188 μL, 2.16 mmol) schnell versetzt und 16 h bei RT gerührt. Anschließend wurde mit 1 N NaOH alkalisch gestellt und 15 min bei RT nachgerührt. Die Phasen wurden getrennt. Die wässrige Phase wurde mit Dichlormethan (dreimal 20 ml) extrahiert. Die organische Phase wurde über $Na_2SO_4$ getrocknet und i. Vak. eingeengt. Der Rückstand wurde durch Flash-Chromatographie mit CH/EE (1:1 →1:4), EE/EtOH (4:1 → 1:1), EtOH, (Methanol + 1 % TEA) gereinigt. Die Fraktionen wurden mit 1 N NaOH versetzt und mit Dichlormethan (zweimal 10 ml) extrahiert. Die organische Phase wurde über $Na_2SO_4$ getrocknet und i. Vak. eingeengt. *Ausbeute:* 86 mg (19 %)

**N-(2-(2-(4-(Dimethylamino)-4-phenylcyclohexyl)-1H-indol-3-yl)ethyl)acetamid, Citrat (1:1) Unpolares Diastereomer**

Das soeben erhaltene Olefin (436 mg/1.08 mmol) wurde in HBr/Eisessig (25 ml) gelöst, innerhalb von 30 min mit Zinn (1.25 g, 1.08 mmol) versetzt und 4 h bei RT gerührt. Der Ansatz wurde mit Ethanol versetzt und über Nacht bei RT gerührt. Anschließend wurde der Ansatz i. Vak. zur Trockene eingeengt. Der Rückstand wurde mit 5N NaOH versetzt und mit Dichlormethan (dreimal 20 ml) extrahiert. Die organische Phase wurde über $Na_2SO_4$ getrocknet und i. Vak. eingeengt. Der noch in der wässrigen Phase befindliche Feststoff wurde abgesaugt und i. Vak. getrocknet (unpolares Diastereomer). Die Mutterlauge wurde durch Flash-Chromatographie mit $CHCl_3$/MeOH (4:1→ MeOH → MeOH + 1% TEA) gereinigt.

*Ausbeute:* 185 mg (42 %) unpolares Diastereomer
250 mg (57 %) polares Diastereomer

Das soeben erhaltene unpolare Diastereomer (76 mg, 0.188 mmol) wurde in heißem Ethanol (5 ml) gelöst. Citronensäure (36 mg/ 0.188 mmol) wurde in heißem Ethanol (1 ml) gelöst und zugegeben. Der Ansatz wurde abgekühlt und mit Ether versetzt. Der dabei ausgefallene Niederschlag wurde abgesaugt und i Vak. getrocknet.
*Ausbeute:* 43 mg (Bsp 20, 38 %)
*Schmelzpunkt:* 245-247 °C
*$^1$H-NMR (DMSO-$d_6$):* 1.77 (5 H, m); 2.35 (6 H, s); 2.65-2.80 (6 H, m); 2.97 (2 H, m); 3.18 (2 H, m); 6.96 (2 H, m); 7.30-7.58 (6 H, m); 7.89 (1 H, s); 10.91 (1 H, s), Citrat.

**Beispiel 21: N-(2-(2-(4-(Dimethylamino)-4-phenylcyclohexyl)-1H-indol-3-yl)ethyl)acetamid, Citrat (1:1) Polares Diastereomer**

Das unter Beispiel 20 erhaltene polare Diastereomer (63 mg/ 0.155 mmol) wurde in heißen Ethanol (5 ml)/ Dioxan (5 ml) gelöst. Citronensäure (30 mg/ 0.155 mmol) wurde in heißen Ethanol (1 ml) gelöst und zugegeben. Der Ansatz wurde abgekühlt und mit Ether versetzt, dabei fiel ein Niederschlag aus. Der Niederschlag wurde abgesaugt und i Vak. getrocknet. *Ausbeute:* 52 mg (Bsp 21, 56 %)
*$^1$H-NMR (DMSO-$d_6$):* 1.47 (2 H, m); 1.81 (5 H, m); 2.23 (2 H, m); 2.43 (6 H, s); 2.58-2.71 (4 H, m); 2.92 (1 H, t); 3.11 (2 H, m); 3.43 (2 H, m); 6.90 (2 H, m); 7.14 (1 H, m); 7.39 (1 H, d); 7.57 (3 H, m); 7.74 (2 H, m); 8.02 (1 H, s); 10.40 (1 H, s).

**Beispiel 22: (±)-2-(4-Benzyl-4-(dimethylamino)cyclohex-1-enyl)-3-methyl-1H-indol-5-carbonitril**

Das Keton Ket-3 (606 mg, 2,62 mmol) und **Ind-6** (410 mg, 2,62 mmol) wurden in abs. Dichlormethan (40 ml) gelöst, mit Trifluormethansulfonsäure (0,256 ml, 432 mg, 2,88 mmol) versetzt und 3 Tage bei Raumtemperatur gerührt. Im Reaktionsgemisch bildete sich eine helle Fällung. Der Ansatz wurde mit Wasser (10 ml) und 1N Natronlauge (10 ml) versetzt und 1 h gerührt. Die Phasen wurden getrennt. Die wässrige Phase wurde mit Dichlormethan (40 ml) extrahiert. Die organischen Phasen wurden vereinigt, mit Wasser (20 ml) gewaschen, getrocknet und eingeengt. Der Rückstand

war ein braunes Öl (950 mg), das chromatographisch aufgetrennt wurde [Kieselgel 60 (80 g); Ethylacetat (500 ml), Ethylacetat/Methanol (4 : 1, 500 ml)]. Beispiel 22 wurde als farbloser Feststoff in einer Ausbeute von 34 % (318 mg) mit einem Schmelzpunkt von 120-123 °C gewonnen

**Beispiel 23: (±)-2-(4-(Dimethylamino)-4-phenylcyclohex-1-enyl)-3-Methyl-1H-indol-5-carbonitril**

Das Keton Ket-10 (568 mg, 2,62 mmol) und das Indol Ind-6 (410 mg, 2,62 mmol) wurden in abs. Dichlormethan (40 ml) gelöst, mit Trifluormethansulfonsäure (0,256 ml, 432 mg, 2,88 mmol) versetzt und 3 Tage bei Raumtemperatur gerührt. Im Reaktionsgemisch bildete sich eine helle Fällung. Der Ansatz wurde mit Wasser (20 ml) und 1N Natronlauge (15 ml) versetzt und 30 min gerührt. Die Phasen wurden getrennt. Die wässrige Phase wurde mit Dichlormethan (30 ml) extrahiert. Die organischen Phasen wurden vereinigt, mit Wasser (20 ml) gewaschen, getrocknet und eingeengt. Der Rückstand war ein braunes Öl (983 mg), das chromatographisch aufgetrennt wurde [Kieselgel 60 (120 g); Trichlormethan/Methanol (40 : 1,1600 ml), Trichlormethan/Methanol (20 : 1, 400 ml), Trichlormethan/Methanol (10 : 1, 700 ml)]. Beispiel 23 wurde als farbloser Feststoff in einer Ausbeute von 84 % (824 mg) mit einem Schmelzpunkt von 180-185 °C gewonnen .

**Beispiel 24: (±)-2-(4-(Dimethylamino)-4-butylcyclohex-1-enyl)-3-methyl-5-trifluonnethyl-1H-indol**

Das Keton Ket-4 (395 mg, 2 mmol) wurde zusammen mit **Ind-7** (398 mg, 2 mmol) in Dichlormethan (20 ml) gelöst. Anschließend erfolgte die Zugabe von Trifluormethansulfonsäure (0,2 ml, 338 mg, 2,25 mmol), wobei sich der Ansatz dunkel färbte. Es wurde 3 d bei RT gerührt. Zur Aufarbeitung wurde das Reaktionsgemisch mit 1 N NaOH (10 ml) versetzt und 10 min gerührt. Die Farbe wechselte dabei von dunkelrot nach hellbraun. Nach Phasentrennung wurde die wässrige Phase mit Dichlormethan (2 × 10 ml) extrahiert. Die vereinigten organischen Phasen wurden mit Natriumsulfat getrocknet, filtriert und im Vakuum eingeengt. Es wurden 741 mg hellbrauner Feststoff erhalten, der chromatographisch getrennt wurde [Kieselgel 60 (80 g); Ethylacetat/Methanol (15 : 1; 1,5 l); (10 : 1; 500 ml); (1 : 1; 500 ml)]. Es wurde Beispiel 24 (140 mg, 18 %, Fp : 118-120 °C) erhalten. )

**Beispiel 25: (±)-2-(4-Dimethylamino)-4-phenylcyclohex-l-enyl)-3-methyl-5-trifluormethyl-1H-indol, Citrat (1:1)**

Das Keton **Ket-10** (599 mg, 2,76 mmol) wurde zusammen mit **Ind-7** (550 mg, 2,76 mmol) in Dichlormethan (20 ml) gelöst. Anschließend erfolgte die Zugabe von Trifluormethansulfonsäure (0,276 ml, 3,1 mmol), wobei sich der Ansatz dunkel färbte. Es wurde 3 d bei RT gerührt. Der Verlauf der Reaktion wurde mittels DC verfolgt. Zur Aufarbeitung wurde das Reaktionsgemisch mit 1 N NaOH (10 ml) versetzt und 10 min gerührt. Die Farbe wechselte dabei von dunkelrot nach hellbraun. Nach Phasentrennung wurde die wässrige Phase mit Dichlormethan (2 × 10 ml) extrahiert. Die vereinigten organischen Phasen wurden mit Natriumsulfat getrocknet, filtriert und im Vakuum eingeengt. Es wurden 1,1 g hellbrauner Feststoff erhalten, der chromatographisch gereinigt wurde [Kieselgel 60 (80 g); Ethylacetat/Methanol (15 : 1; 900 ml)]. Es wurden 740 mg (67 %) N,N-dimethyl-4-(3-methyl-5-(trifluormethyl)-1H-indol-2-yl)-1-phenylcyclohex-3-enamin gewonnen, das jedoch noch ca. 10 % **Ket-10** enthielt.

Das in Ethanol (5 ml) gelöste (±)-2-(4-(Dimethylamino)-4-phenylcyclohex-1-enyl)-3-methyl-5-trifluormethyl-1H-indol (150 mg, 0,375 mmol) wurde mit Citronensäure (80 mg, 0,413 mmol), gelöst in Ethanol (2 ml), versetzt. Die klare grüne Lösung wurde 20 h bei RT gerührt, dann bis auf ca. 0,5 ml eingeengt und anschließend mit Diethylether (5 ml) bis zur Kristallisation versetzt. Beispiel 25 wurde nach dem Absaugen in einer Ausbeute von 56 % (124 mg) mit einem nicht bestimmbaren Schmelzpunkt gewonnen Das bereits in der Vorstufe noch vorhandene Keton Ket-10 konnte bei der Citratherstellung nicht abgetrennt werden.

**Beispiel 26: (±)-2-(4-(Dimethylamino)-4-benzylcyclohex-1-enyl)-3-methyl-5-fluor-1H-indol, Citrat (1:1)**

5-Fluor-3-methylindol (Ind-8) (596 mg, 4 mmol) wurde zusammen mit Ket-3 (932 mg, 4 mmol) in Dichlormethan (20 ml) gelöst und mit Trifluormethansulfonsäure (0,4 ml, 4,6 mmol) versetzt. Der Ansatz wurde 22 h bei RT gerührt. - Zur Aufarbeitung wurde das Reaktionsgemisch mit 2N NaOH (10 ml) versetzt. Das Gemisch wurde weitere 20 min gerührt. Nach Trennung der Phasen wurde die wässrige Phase mit Dichlormethan (2 x 20 ml) extrahiert. Die vereinigten organischen Extrakte wurden über $MgSO_4$ getrocknet und anschließend eingeengt. Das erhaltene Rohprodukt (1,50 g) wurde unter Erwärmen in Ethanol (10 ml) aufgenommen. Die klare Lösung wurde abgekühlt und 14 h bei 5 °C belassen. Der entstandene Kristallbrei wurde mit weiterem EtOH (10 ml) versetzt und mittels einer Fritte abgetrennt. (±)-2-(4-(Dimethylamino)-4-benzylcyclohex-1-enyl)-3-methyl-5-fluor-1H-indol wurde so in einer Ausbeute von 558 mg (38 %, Schmp.: 62-65 °C) in kristalliner Form erhalten. Durch säulenchromatische Auftrennung (Laufmittel: Ethylacetat) wurde weiteres Produkt (109 mg) erhalten, so dass die Gesamtausbeute 667 mg (46 %) betrug.
(±)-2-(4-(Dimethylamino)-4-benzylcyclohex-1-enyl)-3-methyl-5-fluor-1H-indol (150 mg, 0,41 mmol) wurde in Methanol

(10 ml) unter leichter Erwärmung gelöst und mit Citronensäure (80 mg, 0,42 mmol), gelöst in Methanol (2 ml), versetzt. Anschließend wurde das Lösungsmittel am Rotationsverdampfer eingeengt. Der erhaltene Rückstand wurde mit $H_2O$ (ca. 5 ml) angerieben. Es entstand ein klebriges Öl, welches bei der Trocknung in Vakuum zu einem glasigen Feststoff erstarrte. Beispiel 26 wurde so in einer Ausbeute von 161 mg (70 %.) erhalten.

**Beispiel 27: (±)-2-(4-(Dimethylamino)-4-butylcyclohex-1-enyl)-3-methyl-5-fluor-1H-indol, Citrat (1 :1)**

5-Fluor-3-methylindol (Ind-8) (596 mg, 4 mmol) wurde zusammen mit dem Keton Ket-4 (788 mg, 4 mmol) in Dichlormethan (30 ml) gelöst und mit Trifluormethansulfonsäure (400 µl, 4,6 mmol) versetzt. Der Ansatz wurde 24 h bei RT gerührt. Zur Aufarbeitung wurde das Reaktionsgemisch mit 2N NaOH (30 ml) versetzt und 20 min bei RT gerührt. Nach Abtrennung der organischen Phase wurde die wässrige Phase mit Dichlormethan (3 x 15 ml) extrahiert. Die vereinigten organischen Extrakte wurden über $Na_2SO_4$ getrocknet und anschließend eingeengt. Das erhaltene Rohprodukt (1,4 g) wurde durch Säulenchromatographie [Kieselgel 60 (50 g); Ethylacetat (500 ml)] gereinigt. (±)-2-(4-(Dimethylamino)-4-butylcyclohex-1-enyl)-3-methyl-5-fluor-1H-indol wurde so in einer Ausbeute von 160 mg (13 %) als weißer Feststoff erhalten.
(±)-2-(4-(Dimethylamino)-4-butylcyclohex-1-enyl)-3-methyl-5-fluor-1H-indol (160 mg, 0,49 mmol) wurde in der Siedehitze in Isopropanol (10 ml) gelöst und mit Citronensäure (94 mg, 0,49 mmol), gelöst in heißem Isopropanol (2 ml), versetzt. Die Lösung wurde auf 5 **°C** abgekühlt und 16 h im Kühlschrank aufbewahrt. Da dabei kein Feststoff ausfiel, wurde Isopropanol am Rotationsverdampfer abdestilliert. Beispiel 27 () wurde in einer Ausbeute von 254 mg (100 %, *Schmelzpunkt:* 31-35 °C) erhalten.

**Beispiel28: (±)-2-(4-(Dimethylamino)-4-phenylcyclohex-1-enyl)-3-methyl-5-fluor-1H-indol, Citrat (1:1)**

5-Fluor-3-methylindol (**Ind-8)** (498 mg, 2 mmol) wurde zusammen mit dem Keton Ket-10 (434 mg, 2 mmol) in Dichlormethan (20 ml) gelöst und mit Trifluormethansulfonsäure (0,2 ml, 2,3 mmol) versetzt. Der Ansatz wurde 22 h bei RT gerührt. - Zur Aufarbeitung wurde das Reaktionsgemisch mit 2N NaOH (10 ml) versetzt. Das Gemisch wurde weitere 20 min gerührt. Nach Trennung der Phasen wurde die wässrige Phase mit Dichlormethan (2 x 20 ml) extrahiert. Die vereinigten organischen Extrakte wurden über $MgSO_4$ getrocknet und anschließend eingeengt. Das erhaltene Rohprodukt (710 mg) wurde in siedenden Ethanol (18 ml) aufgenommen. Die klare Lösung wurde abgekühlt und 14 h bei 5 °C belassen. Der entstandene Niederschlag wurde mittels einer Fritte abgetrennt. (±)-2-(4-(Dimethylamino)-4-phenylcyclohex-1-enyl)-3-methyl-5-fluor-1H-indol wurde so in einer Ausbeute von 399 mg (57 %, Schmp.: 171-175 °C) in kristalliner Form erhalten.

(±)-2-(4-(Dimethylamino)-4-phenylcyclohex-1-enyl)-3-methyl-5-fluor-1H-indol (150 mg, 0,43 mmol) wurde in der Siedehitze in Isopropanol (10 ml) gelöst und mit Citronensäure (100 mg, 0,52 mmol), gelöst in heißem Isopropanol (5 ml), versetzt. Das Lösungsmittelvolumen wurde auf ca. 6 ml reduziert, anschließend wurde der Ansatz auf 5 °C abgekühlt (Kühlschrank) und 12 h stehen gelassen. Der Niederschlag wurde mittels einer Fritte abgetrennt und anschließend getrocknet. Beispiel 28 wurde so in einer Ausbeute von 151 mg (79 %, *Schmelzpunkt:* 88-93 °C) () erhalten.

**Beispiel 29: (±)-2-(4-(Dimethylamino)-4-phenylcyclohex-1-enyl)-3-methyl-5-methoxy-1H-indol, Citrat (2:1)**

5-Methoxyskatol (Ind-9) (644 mg, 4 mmol) wurde zusammen mit dem Keton Ket-10 (868 mg, 4 mmol) in Dichlormethan (40 ml) gelöst und mit Trifluormethansulfonsäure (0,4 ml, 4,5 mmol) versetzt. Der Ansatz wurde 2,5 d bei RT gerührt. - Zur Aufarbeitung wurde das Reaktionsgemisch mit 2N NaOH (20 ml) versetzt und 60 min bei RT gerührt. Nach Abtrennung der organischen Phase wurde die wässrige Phase mit Dichlormethan (2 x 20 ml) extrahiert. Die vereinigten organischen Extrakte wurden über $MgSO_4$ getrocknet und anschließend eingeengt. Das erhaltene Rohprodukt (1,2 g) konnte durch Säulenchromatographie [Kieselgel 60 G (10 g); Cyclohexan/EtOAc 1 : 1 (100 ml)] gereinigt werden. (±)-2-(4-(Dimethylamino)-4-phenylcyclohex-1-enyl)-3-methyl-5-methoxy-1H-indol wurde in einer Ausbeute von 400 mg (27 %) als Feststoff *(Schmelzpunkt:* 175-185 °C) erhalten.
(±)-2-(4-(Dimethylamino)-4-phenylcyclohex-1-enyl)-3-methyl-5-methoxy-1H-indol (150 mg, 0,41 mmol) wurde in der Siedehitze in Isopropanol (15 ml) gelöst und mit Citronensäure (80 mg, 0,42 mmol), gelöst in heißem Isopropanol (2 ml), versetzt. Nach dem Abkühlen der Lösung fiel ein Niederschlag aus. Zur Vervollständigung der Fällung wurde der Ansatz auf 5 °C abgekühlt (Kühlschrank) und 17 h bei dieser Temperatur belassen. Der Niederschlag wurde.mittels einer Fritte abgetrennt und anschließend getrocknet. Beispiel 29 wurde so in einer Ausbeute von 124 mg (65 %) als rosa Feststoff mit einem Schmelzpunkt von 204-209 °C erhalten.

**Beispiel 30: (±)-2-(4-(Dimethylamino)-4-benzylcyclohex-1-enyl)-3-methyl-1H-indol, Citrat (2:1)**

3-Methylindol (Ind-10, 524 mg, 4 mmol) wurde zusammen mit dem Keton Ket-3 (932 mg, 4 mmol) in Dichlormethan (20 ml) gelöst und mit Trifluormethansulfonsäure (0,4 ml, 4,6 mmol) versetzt. Der Ansatz wurde 22 h bei RT gerührt. - Zur Aufarbeitung wurde das Reaktionsgemisch mit 2N NaOH (10 ml) versetzt. Das Gemisch wurde weitere 10 min gerührt. Nach Trennung der Phasen wurde die wässrige Phase mit Dichlormethan (2 x 20 ml) extrahiert. Die vereinigten organischen Extrakte wurden über $MgSO_4$ getrocknet und anschließend eingeengt. Das erhaltene Rohprodukt (1,28 g) wurde mit Methanol (7 ml) angerieben, das entstandene Gemisch wurde 14 h bei 5 °C belassen. Der entstandene Feststoff, der beim Trocknen glasig wurde, wurde mittels einer Fritte abgetrennt. (±)-2-(4-(Dimethylamino)-4-benzylcyclohex-1-enyl)-3-methyl-1H-indol wurde so in einer Ausbeute von 510 mg (37 %) in ausreichender Reinheit erhalten.

(±)-2-(4-(Dimethylamino)-4-benzylcyclohex-1-enyl)-3-methyl-1H-indol (100 mg, 0,29 mmol) wurde in Methanol (10 ml) unter leichter Erwärmung gelöst und mit Citronensäure (58 mg, 0,3 mmol), gelöst in Methanol (1 ml), versetzt. Anschließend wurde das Lösungsmittel am Rotationsverdampfer eingeengt. Der erhaltene Rückstand wurde mit $H_2O$ (ca. 5 ml) angerieben. Es entstand ein weißer Feststoff, der mittels einer Fritte isoliert wurde. Beispiel 30 wurde so in einer Ausbeute von 78 mg (61 %, ) erhalten.

**Beispiel 31: (±)-2-(4-(Dimethylamino)-4-butylcyclohex-1-enyl)-3-methyl-1H-indol, Citrat (1:1)**

3-Methylindol (Ind-10, 524 mg, 4 mmol) wurde zusammen mit dem Keton Ket-4 (788 mg, 4 mmol) in Dichlormethan (30 ml) gelöst und mit Trifluormethansulfonsäure (0,4 ml, 4,6 mmol) versetzt. Der Ansatz wurde 22 h bei RT gerührt. - Zur Aufarbeitung wurde das Reaktionsgemisch mit 2N NaOH (10 ml) versetzt. Das Gemisch wurde weitere 20 min gerührt. Nach Trennung der Phasen wurde die wässrige Phase mit Dichlormethan (2 x 20 ml) extrahiert. Die vereinigten organischen Extrakte wurden über $MgSO_4$ getrocknet und anschließend eingeengt. Das erhaltene Rohprodukt (1,21 g) wurde säulenchromatisch (Laufmittel: Ethylacetat) gereinigt. (±)-2-(4-(Dimethylamino)-4-butylcyclohex-1-enyl)-3-methyl-1H-indol wurde als eine mit etwa 10 % Ket-4 verunreinigte Fraktion in einer Ausbeute von 315 mg (23 %) als halbfeste Substanz erhalten.

(±)-2-(4-(Dimethylamino)-4-butylcyclohex-1-enyl)-3-methyl-1H-indol (120 mg, 0,39 mmol) wurde in Methanol (10 ml) unter leichter Erwärmung gelöst und mit Citronensäure (80 mg, 0,42 mmol), gelöst in Methanol (1 ml), versetzt. Da nach Abkühlung der Lösung (5 °C) kein Niederschlag entstand, wurde das Lösungsmittel am Rotationsverdampfer eingeengt. Der erhaltene Rückstand wurde in 4 ml heißem Isopropanol aufgenommen. Nach Abkühlung fiel ein klebriger Niederschlag aus, der bei der Trocknung in Vakuum zu einem glasigen Feststoff erstarrte. Beispiel 31, das beim Stehen an der Luft wieder klebrig wurde, wurde so in einer Ausbeute von 105 mg (53 %) als Citrat erhalten.

**Beispiel 32: (±)-2-(4-(Dimethylamino)-4-phenylcyclohex-1-enyl)-3-methyl-1H-pyrrolo[2,3-b]pyridin, Citrat (1:1)**

**4-(Dimethylamino)-4-phenyl-1-(prop-1-ynyl)cyclohexanol**

Das Keton Ket-10 (3000 mg 13,81 mmol) wurde in Tetrahydrofuran (absolut, 50 ml) bei - 78 °C vorgelegt. Unter Argon wurde Prop-1-inyl Magnesiumbromid (31,8 ml, 15,88 mmol; 0,5 M in Tetrahydrofuran) hinzugetropft. Anschließend wurde die Reaktionsmischung bei -78 °C 15 min gerührt. Danach wurde auf Raumtemperatur erwärmt und 1 h bei dieser Temperatur gerührt. Anschließend wurde Ammoniumchlorid-Lösung (50 ml; 1,0 M) hinzugegeben. Die Phasen wurden getrennt. Die wässrige Phase wurde mit Tetrahydrofuran (3 × 50 ml) extrahiert. Die vereinigten organischen Phasen wurden mit Natriumsulfat getrocknet und anschließend die flüchtigen Bestandteile vollständig im Vakuum entfernt. Es verblieb ein hellbraunes Öl, welches mit Diethylether (20 ml) versetzt wurde. Es fiel ein weißer Feststoff aus (990 mg, 4-Dimethylamino-4-phenyl-1-(prop-1-inyl)cyclohexanol, unpolares Diastereomer). Die Waschlösung wurde im Vakuum bis auf 5 ml eingeengt. Es fiel abermals ein weißer Feststoff aus (1350 mg; Diastereomerengemisch). Die Waschlösung wurde langsam auf 3 ml eingedampft. Es fiel erneut eine Produktfraktion aus (410 mg; beide Diastereoisomere). *Ausbeute:* 2750 mg (10,68 mmol; 77 %, Diastereomerengemisch)

**4-Dimethylamino-1-(3-methyl-1*H*-pyrrolo[2,3-b]pyridin-2-yl)-4-phenylcyclohexanol**

2-Amino-3-iodpyridin (3108 mg, 14,13 mmol), 4-(Dimethylamino)-4-phenyl-1-(prop-1-ynyl)cyclohexanol (4000 mg, 15.54 mmol), Lithiumchlorid (630 mg, 14,83 mmol) und Natriumcarbonat (4,49 g, 42,38 mmol wurden in Dimethylformamid (absolut, 60 ml) in einer Argonatmosphäre vereinigt. Anschließend wurde der Katalysator ([Pd(dppf)$Cl_2$ × $CH_2Cl_2$], 1154 mg, 1,41 mmol) hinzugegeben. Die rote Lösung wurde für 5 h auf 79 °C (Olbadtemperatur) erwärmt. Zur Vervollständigung der Reaktion wurden noch 0,3 Aquivalente 2-Amino-3-iodpyridin (932 mg, 4,24 mmol) und 0,05 Aquivalente Katalysator (577 mg, 0,71 mmol) hinzugegeben. Danach wurde weitere 2 h bei 99 °C (Ölbadtemperatur) gerührt. Zu der auf Raumtemperatur abgekühlten schwarzen Reaktionsmischung wurden nacheinander Wasser (50 ml; 10 min Rühren) und Dichlormethan (50 ml) gegeben. Die Phasen wurden getrennt (das Gemisch wurde über Kieselgur filtriert) und die wässrige Phase mit Dichlormethan (3 × 20 ml) extrahiert. Die vereinigten organischen Phasen wurden

mit gesättigter NaCl-Lösung (3 × 20 ml) gewaschen und über Natriumsulfat getrocknet. Nach Filtration wurden die flüchtigen Bestandteile vollständig im Vakuum entfernt. Der Rückstand wurde auf Kieselgur aufgezogen und chromatographisch getrennt (Kieselgel [200 g]; Chloroform/Ethanol [9 : 1, 1000 ml]). Es wurden 1200 mg (3,43 mmol; 22 % des unpolaren Diastereoisomers als farbloser Feststoff isoliert.

**(±)-2-(4-(Dimethylamino)-4-phenylcyclohex-1-enyl)-3-methyl-1H-pyrrolo[2,3-b]pyridin, Citrat (1:1)**

4-Dimethylamino-1-(3-methyl-1*H*-pyrrolo[2,3-b]pyridin-2-yl)-4-phenylcyclohexanol (900 mg, 2,58 mmol) wurde in Methansulfonsäure (20 ml) gelöst, $P_4O_{10}$ (ca. 1 g) hinzugegeben und die leicht gefärbte (hellbraune) Lösung für 3 h bei 77 °C (Ölbadtemperatur) gerührt. Die Reaktionsmischung wurde mit 5M Natriumhydroxid-Lösung basisch gestellt. Anschließend wurde Dichlormethan (30 ml) hinzugegeben und 10 min gerührt. Die Phasen wurden getrennt. Die wässrige Phase wurde mit Dichlormethan (3 × 35 ml) extrahiert. Die vereinigten organischen Phasen wurden mit Natriumsulfat getrocknet und anschließend die flüchtigen Bestandteile vollständig im Vakuum entfernt. Es verblieben 805 mg (2,43 mmol; 94 %) eines dunkelbraunen Feststoffes ((±)-2-(4-(Dimethylamino)-4-phenylcyclohex-1-enyl)-3-methyl-1H-pyrrolo [2,3-b]pyridin).

(±)-*N*,*N*-Dimethyl-*N*-[4-(3-methyl-1*H*-pyrrolo[2,3-b]pyridin-2-yl)-1-phenylcyclohex-3-enyl]amin (43 mg, 0,13 mmol) wurde in Ethanol (10 ml) gelöst. Die dunkle Lösung wurde mit Citronensäure (27 mg, 0,14 mmol) versetzt. Es wurde 1 h in der Siedehitze gerührt. Die Reaktionsmischung wurde auf Raumtemperatur abgekühlt und im Vakuum auf ca. 3 ml eingeengt. Der Kolben wurde bei Raumtemperatur und bei 0 °C gelagert. Die Lösung wurde mit ca. 5 ml Diethylether überschichtet und für 3 Tage bei Raumtemperatur stehen gelassen. Es fiel ein beigefarbener Feststoff aus. Die überstehende Lösung wurde abgehebert und verworfen. Der pulvrige Feststoff wurde im Vakuum getrocknet. Es wurden 30 mg (0,057 mmol; 44 %) der Zielverbindung Beispiel 32 (*Schmelzpunkt:* 107 °C) erhalten.

*$^1$H NMR (400 MHz, RT, $CD_3OD$) δ ppm:* 2.08 (s, br, 1 ), 2.19 (s, 3H), 2.46 (s, br, 1H), 2.72 (s, 6H), 2.79 (dd, 4H), 2.60-2.85 (darunter, br, weitere 2 H) 2.97 (d, br, 1H), 3.68 (d, br, 1H), 6.14 (s, 1H), 7.01 (m, 1H), 7.40-7.60 (m, 2H), 7.73 (d, 2H), 7.84 (dd, 1H), 8.08 (s, br, 1H).

**Beispiel 33: (±)-2-(4-(Dimethylamino)-4-benzylcyclohex-1-enyl)-3-cyclopropyl-1H-indol Hydrochlorid**

Das Keton Ket-3 (693 mg, 3 mmol) und Ind-11 (472 mg, 3,0 mmol) wurden in abs. Dichlormethan (40 ml) gelöst, mit Trifluormethansulfonsäure (0,293 ml, 495 mg, 3,3 mmol) versetzt und 67 h bei Raumtemperatur gerührt. Da die Umsetzung nicht vollständig war wurde nochmals Trifluormethansulfonsäure (0,586 ml, 990 mg, 6,6 mmol) hinzugefügt und 5 h bei Raumtemperatur gerührt. Der Ansatz wurde mit Wasser (20 ml) und 1 N Natronlauge (15 ml) versetzt und 30 min gerührt. Die Phasen wurden getrennt. Die wässrige Phase wurde mit Dichlormethan (30 ml) extrahiert. Die organischen Phasen wurden vereinigt, mit Wasser (20 ml) gewaschen, mit Natriumsulfat getrocknet und eingeengt. Der Rückstand war ein braunes Öl (1,17 g ), das chromatographisch aufgetrennt wurde [Kieselgel 60 (70 g); Ethylacetat (500 ml), Ethylacetat/Methanol 4 : 1 (400 ml)]. (±)-2-(4-(Dimethylamino)-4-benzylcyclohex-1-enyl)-3-cyclopropyl-1H-indol wurde als farbloser Feststoff (100 mg) gewonnen. Eine erhaltene Mischfraktion aus Keton Ket-3 und Produkt wurde nochmals chromatographisch [Kieselgel 60 (70 g); Ethylacetat (500 ml), Ethylacetat/Methanol 4 : 1 (300 ml)] getrennt. Neben dem Produkt (102 mg) wurde eine weitere Mischfraktion (490 mg) erhalten, die erneut chromatographisch getrennt wurde. [Kieselgel 60 (70 g); Ethylacetat/Cyclohexan 1 : 1 (800 ml), Methanol (500 ml)]. Es wurde nur eine geringe Menge (26 mg) reinen Produkts erhalten. Die Mischfraktion (286 mg) wurde in Ethylacetat (30 ml) gelöst, mit Wasser (30 ml) und 1N Salzsäure (5 ml) versetzt und 1 h bei Raumtemperatur gerührt. Ein farbloser Feststoff, der sich zwischen den Phasen bildete wurde durch Filtration abgetrennt, mit Wasser (2 × 10 ml) und mit Ethylacetat (2 × 10 ml) gewaschen. Das Hydrochlorid Beispiel 33 (221 mg, ) wurde so mit einem Schmelzpunkt von 222-224 °C erhalten Ausbeute insgesamt: 39 %).

**Beispiel 34: (±)-2-(4-(Dimethylamino)-4-butylcyclohex-1-enyl)-3-cyclopropyl-1H-indol**

Das Keton Ket-4 (592 mg, 3 mmol) und Ind-11 (472 mg, 3,0 mmol) wurden in abs. Dichlormethan (40 ml) gelöst, mit Trifluormethansulfonsäure (0,293 ml, 495 mg, 3,3 mmol) versetzt und 67 h bei Raumtemperatur gerührt. Da die Umsetzung nicht vollständig war wurde nochmals Trifluormethansulfonsäure (0,586 ml, 990 mg, 6,6 mmol) hinzugefügt und 5 h bei Raumtemperatur gerührt. Der Ansatz wurde mit Wasser (20 ml) und 1N Natronlauge (15 ml) versetzt und 30 min gerührt. Die Phasen wurden getrennt. Die wässrige Phase wurde mit Dichlormethan (30 ml) extrahiert. Die organischen Phasen wurden vereinigt, mit Wasser (20 ml) gewaschen, mit Natriumsulfat getrocknet und eingeengt. Der Rückstand war ein braunes Öl (1,06 g), das chromatographisch aufgetrennt wurde [Kieselgel 60 (80 g); Ethylacetat (800 ml), Ethylacetat/Methanol 4 : 1 (500 ml), Methanol (300 ml)]. Beispiel 34 wurde als beigefarbener Feststoff (226 mg) gewonnen. Eine Mischfraktion (236 mg) wurde mit n-Hexan (5 ml) versetzt und 10 min gerührt. Beispiel 34 blieb ungelöst zurück und wurde durch Filtration und Waschen mit n-Hexan (2 × 3 ml ) abgetrennt. Ausbeute: 348 mg (34 %)

Schmelzpunkt : 126-130 °C

**Beispiel 35: (±)-2-(4-(Dimethylamino)-4-phenylcyclohex-1-enyl)-3-cyclopropyl-1H-indol**

**Variante 1:** Keton Ket-10 (652 mg, 3,0 mmol) und Indol Ind-11 (472 mg, 3 mmol) wurden in abs. Dichlormethan (40 ml) gelöst, mit Trifluormethansulfonsäure (0,293 ml, 495 mg, 3,3 mmol) versetzt und 4 Tage bei Raumtemperatur gerührt. Es war kein Umsatz erkennbar. Der Ansatz wurde mit Wasser (20 ml) und 1N Natronlauge (15 ml) versetzt und 30 min gerührt. Die Phasen wurden getrennt. Die wässrige Phase wurde mit Dichlormethan (20 ml) extrahiert. Die organischen Phasen wurden vereinigt, mit Wasser (20 ml) gewaschen, mit Natriumsulfat getrocknet und eingeengt. Der Rückstand war ein braunes Öl (1,1 g ), das in abs. 1,2-Dichlorethan (40 ml) gelöst wurde. Ein Teil der Lösung (20 ml, 1,5 mmol Edukte) wurde mit Trifluormethansulfonsäure (0,44 ml, 743 mg, 4,95 mmol) versetzt und 23 h bei Raumtemperatur gerührt. Der Ansatz wurde mit Wasser (10 ml) und 1N Natronlauge (10 ml) versetzt und 30 min gerührt. Die Phasen wurden getrennt. Die wässrige Phase wurde mit Dichlormethan (20 ml) extrahiert. Die organischen Phasen wurden vereinigt, mit Wasser (20 ml) gewaschen, mit Natriumsulfat getrocknet und eingeengt. Der Rückstand war ein braunes Öl (593 mg ), das chromatographisch [Kieselgel 60 (50 g); Trichlormethan/Methanol 40 : 1 (600 ml), Trichlormethan/Methanol 20 : 1 (400 ml)] aufgetrennt wurde. Beispiel 35 wurde als beigefarbener Feststoff in einer Ausbeute von 60 % (302 mg) mit einem Schmelzpunkt von 180-187 °C gewonnen .

**Variante 2:** Eine Lösung von Ket-10 und Ind-11 in 1,2-Dichlorethan (1,5 mmol, 20 ml) wurde mit Trifluormethansulfonsäure (0,147 ml, 248 mg, 1,65 mmol) versetzt und 8 h auf 70 °C erwärmt. Es wurde weitere Trifluormethansulfonsäure (0,293 ml, 495 mg, 3,3 mmol) hinzugefügt und 24 h bei Raumtemperatur gerührt. Der Ansatz wurde mit Wasser (10 ml) und 1 N Natronlauge (10 ml) versetzt und 30 min gerührt. Die Phasen wurden getrennt. Die wässrige Phase wurde mit Dichlormethan (30 ml) extrahiert. Die organischen Phasen wurden vereinigt, mit Wasser (20 ml) gewaschen, mit Natriumsulfat getrocknet und eingeengt. Der Rückstand war ein braunes Öl (572 mg ), das chromatographisch [Kieselgel 60 (50 g); Trichlormethan/Methanol 40 : 1 (650 ml), Trichlormethan/Methanol 20 : 1 (400 ml)] aufgetrennt wurde. Beispiel 35 wurde als beigefarbener Feststoff in einer Ausbeute von 43 % (217 mg) gewonnen.

**Beispiel 36: (±)-2-(4-(Dimethylamino)-4-benzylcyclohex-1-enyl)-3-(cyclohexylmethyl)-1H-indol Hydrochlorid**

3-Cyclohexylmethyl-1*H*-indol (Ind-12, 640 mg, 3 mmol) wurde zusammen mit Keton Ket-3 (694 mg, 3 mmol) in abs. Dichlormethan (40 ml) gelöst und mit Trifluormethansulfonsäure (0,396 ml, 4,51 mmol) versetzt. Der Ansatz wurde 90 h bei Raumtemperatur gerührt. Zur Aufarbeitung wurde die Reaktionslösung mit 5N NaOH (10 ml) versetzt. Das Gemisch wurde weitere 10 min gerührt. Nach Trennung der Phasen wurde die wässrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,34 g braunes Öl) wurde in Diethylether (30 ml) gelöst. Bei Raumtemperatur wurde 2,5N HCl (20 ml) zugesetzt. Der Ansatz wurde 5 h bei Raumtemperatur gerührt. Der Niederschlag wurde abfiltriert, mit etwas Wasser und Diethylether gewaschen und getrocknet. Beispiel 36 (761 mg, 55 %, *Schmelzpunkt:* 152-160 °C,) wurde als beigefarbiger Feststoff erhalten.

**Beispiel 37: (±)-2-(4-(Dimethylamino)-4-benzylcyclohex-1-enyl)-3-benzyl-1H-indol Hydrochlorid**

3-Benzyl-1*H*-indol **(Ind-108)** (622 mg, 3 mmol) wurde zusammen mit dem Keton Ket-3 (694 mg, 3 mmol) in abs. Dichlormethan (40 ml) gelöst und mit Trifluormethansulfonsäure (0,396 ml, 4,51 mmol) versetzt. Der Ansatz wurde 81 h bei Raumtemperatur gerührt. Zur Aufarbeitung wurde die Reaktionslösung mit 5N NaOH (10 ml) versetzt. Das Gemisch wurde weitere 10 min gerührt. Nach Trennung der Phasen wurde die wässrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,30 g braunes Öl) wurde in Toluol (5 ml) gelöst. Bei Raumtemperatur wurde 1 N HCl (10 ml) zugesetzt. Danach wurde noch Diethylether (25 ml) zugegeben. Der Ansatz wurde 3 h bei Raumtemperatur gerührt. Der Niederschlag wurde abfiltriert, mit etwas Wasser und Diethylether gewaschen und getrocknet. Beispiel 37 ( 824 mg 60 %, *Schmelzpunkt:* 235-250 °C, beigefarbiger Feststoff.

**Beispiel 38: (±)-2-(4-(Dimethylamino)-4-butylcyclohex-1-enyl)-3-(cyclohexylmethyl)-1H-indol Hydrochlorid**

Ind-12 (640 mg, 3 mmol) wurde zusammen mit Keton Ket-4 (592 mg, 3 mmol) in abs. Dichlormethan (40 ml) gelöst und mit Trifluormethansulfonsäure (0,396 ml, 4,51 mmol) versetzt. Der Ansatz wurde 90 h bei Raumtemperatur gerührt. Zur Aufarbeitung wurde die Reaktionslösung mit 5N NaOH (10 ml) versetzt. Das Gemisch wurde weitere 10 min gerührt. Nach Trennung der Phasen wurde die wässrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,33 g, braunes Öl) wurde in Diethylether (30 ml) gelöst. Bei Raumtemperatur wurde 2,5N HCl (20 ml) zugesetzt.

Der Ansatz wurde 3 h bei Raumtemperatur gerührt. Der Niederschlag wurde abfiltriert, mit etwas Wasser und Diethylether gewaschen und getrocknet. Beispiel 38 wurde (836 mg, 65 %, *Schmelzpunkt:* 241-244 °C) ( als beigefarbiger Feststoff erhalten.

**Beispiel 39: (±)-2-(4-(Dimethylamino)-4-butylcyclohex-1-enyl)-3-benzyl-1H-indol ( 39)**

3-Benzyl-1*H*-indol **(Ind-108,** 622 mg, 3 mmol) wurde zusammen mit dem Keton Ket-4 (592 mg, 3 mmol) in abs. Dichlormethan (40 ml) gelöst und mit Trifluormethansulfonsäure (0,396 ml, 4,51 mmol) versetzt. Der Ansatz wurde 81 h bei Raumtemperatur gerührt. Zur Aufarbeitung wurde die Reaktionslösung mit 5N NaOH (10 ml) versetzt. Das Gemisch wurde weitere 10 min gerührt. Nach Trennung der Phasen wurde die wässrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,17 g, braunes Öl) konnte durch Säulenchromatographie [Kieselgel 60 (80 g): Ethylacetat/Methanol 5 : 1 (1800 ml)] gereinigt werden. Beispiel 39 wurde (464 mg, 40 %, *Schmelzpunkt:* 107-115 °C) (als orangefarbener Feststoff erhalten.

**Beispiel 40: (±)-2-(4-(Dimethylamino)-4-phenylcyclohex-1-enyl)-3-(cyclohexylmethyl)-1H-indol Hydrochlorid ( 40)**

3-Cyclohexylmethyl-1H-indol **(Ind-12)** (640 mg, 3 mmol) wurde zusammen mit dem Keton **Ket-10** (652 mg, 3 mmol) in abs. Dichlormethan (40 ml) gelöst und mit Trifluormethansulfonsäure (0,396 ml, 4,51 mmol) versetzt. Der Ansatz wurde 90 h bei Raumtemperatur gerührt. Zur Aufarbeitung wurde die Reaktionslösung mit 5N NaOH (10 ml) versetzt. Das Gemisch wurde weitere 10 min gerührt. Nach Trennung der Phasen wurde die wässrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,34 g) wurde in heißem Toluol (5 ml) gelöst. Bei Raumtemperatur wurde 2,5 N HCl (20 ml) und Diethylether (25 ml) zugesetzt. Der Ansatz wurde 3 h bei Raumtemperatur gerührt. Der Feststoff wurde abfiltriert, mit etwas Wasser und Diethylether gewaschen und getrocknet. Es wurden ( 968 mg, 72 %, *Schmelzpunkt:* 235-238°C) Beispiel 40 erhalten.

**Beispiel 41: (±)-2-(4-(Dimethylamino)-4-phenylcyclohex-1-enyl)-3-benzyl-1H-indol Hydrochlorid ( 41)**

3-Benzyl-1*H*-indol (Ind-108, 622 mg, 3 mmol) wurde zusammen mit Keton Ket-10 (652 mg, 3 mmol) in abs. Dichlormethan (40 ml) gelöst und mit Trifluormethansulfonsäure (0,396 ml, 4,51 mmol) versetzt. Der Ansatz wurde 81 h bei Raumtemperatur gerührt. Zur Aufarbeitung wurde die Reaktionslösung mit 5N NaOH (10 ml) versetzt. Das Gemisch wurde weitere 10 min gerührt. Nach Trennung der Phasen wurde die wässrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,24 g) wurde in Toluol (5 ml) gelöst. Bei Raumtemperatur wurden 1 N HCl (10 ml) und Diethylether (25 ml) zugesetzt. Der Ansatz wurde 3 h bei Raumtemperatur gerührt. Der ausgefallene Feststoff wurde abfiltriert, mit etwas Wasser und Diethylether gewaschen und getrocknet. Es wurden 840 mg ( 63 %) Beispiel 41 (*schmelzpunkt:* 163-166 °C) erhalten.

**Beispiel 42: (±)-2-(4-Dimethylamino)-4-benzylcyclohex-1-enyl)-3-propyl-1H-indol**

3-Propyl-1*H*-indol **(Ind-13**, 797 mg, 5,0 mmol) wurde zusammen mit Keton Ket-3 (1157 mg, 5,0 mmol) in abs. Dichlormethan (60 ml) gelöst und mit Trifluormethansulfonsäure (0,485 ml, 5,52 mmol) versetzt. Der Ansatz wurde 88 h bei Raumtemperatur gerührt. Zur Aufarbeitung wurde die Reaktionslösung mit Wasser (3 × 40 ml) extrahiert. Anschließend wurde die organische Phase mit 1 N NaOH-Lösung (40 ml) gewaschen, über $Na_2SO_4$ getrocknet und dann eingeengt. Das erhaltene Rohprodukt (1,68 g braunes Öl) wurde chromatographisch gereinigt [Kieselgel 60 (140 g); Ethylacetat/Methanol 20 : 1 (1260 ml)]. Es wurden 1031 mg (55 %) Beispiel **42** (*Schmelzpunkt:* 104-107 °C) erhalten.

**Beispiel 43: (±)-2-(4-(Dimethylamino)-4-butylcyclohex-1-enyl)-3-propyl-1H-indol**

3-Propyl-1*H*-indol **(Ind-13)** (797 mg, 5,0 mmol) wurde zusammen mit dem Keton **Ket-4** (987 mg, 5,0 mmol) in abs. Dichlormethan (60 ml) gelöst und mit Trifluormethansulfonsäure (0,485 ml, 5,52 mmol) versetzt. Der Ansatz wurde 88 h bei Raumtemperatur gerührt. Zur Aufarbeitung wurde die Reaktionslösung mit Wasser (3 × 40 ml) extrahiert. Die organische Phase wurde mit 1 N NaOH-Lösung (40 ml) gewaschen, über $Na_2SO_4$ getrocknet und dann eingeengt. Das erhaltene Rohprodukt (1,44 g braunes Öl) wurde chromatographisch gereinigt [Kieselgel 60 (140 g); Ethylacetat/Methanol 10 : 1 (550 ml), Ethylacetat/Methanol 5 : 1 (600 ml), Ethylacetat/Methanol 2 : 1 (1200 ml)]. Es wurden 808 mg (48 %) Beispiel 43 *(Schmelzpunkt:* 114-120 °C) erhalten.

**Beispiel 44: (±)-2-(4-(Dimethylamino)-4-phenylcyclohex-1-enyl)-3-propyl-1H-indol ( 44)**

3-Propyl-1*H*-indol (Ind-13) (1035 mg, 6,50 mmol) wurde zusammen mit Keton Ket-10 (1413 mg, 6,50 mmol) in abs. Dichlormethan (80 ml) gelöst und mit Trifluormethansulfonsäure (0,630 ml, 7,17 mmol) versetzt. Der Ansatz wurde 88 h bei Raumtemperatur gerührt. Zur Aufarbeitung wurde die Reaktionslösung mit Wasser (3 × 40 ml) extrahiert. Anschließend wurde die organische Phase mit 1 N NaOH-Lösung (40 ml) gewaschen, über $Na_2SO_4$ getrocknet und dann eingeengt. Das erhaltene Rohprodukt (1,90 g brauner Feststoff) wurde chromatographisch gereinigt [Kieselgel 60 (140 g); Trichlormethan/Methanol 10 : 1 (550 ml), Trichlormethan/Methanol 5 : 1 (600 ml)]. Es wurden 1130 mg (48 %) Beispiel 44 (*Schmelzpunkt:* 168-178 °C) erhalten.

**Beispiel 45: (±)-2-(4-(Dimethylamino)-4-benzylcyclohex-1-enyl)-3-(2-(pyridin-4-yl)ethyl)-1H-indol** 3-(2-Pyridin-4-ylethyl)-1*H*-indol (Ind-14, 667 mg, 3 mmol) wurde zusammen mit dem Keton (Ket-3, 652 mg, 3 mmol) in abs. Dichlormethan (45 ml) gelöst und mit Trifluormethansulfonsäure (0,553 ml, 6,3 mmol) versetzt. Der Ansatz wurde 67 h bei RT gerührt, wobei ein braunes Öl ausfiel. Zur Aufarbeitung wurde die Reaktionslösung mit 1 N NaOH (10 ml) und THF (10 ml) versetzt. Das Gemisch wurde weitere 60 min gerührt. Nach Trennung der Phasen wurde die wäßrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,22 g) wurde säulenchromatographisch gereinigt [Kieselgel 60 (100 g); Ethylacetat/Methanol (10 : 1, 1100 ml), Ethylacetat/Methanol (2 : 1, 500 ml), Ethylacetat/Methanol (1 : 2, 750 ml)]. Neben einer Bisindolverbindung (152 mg, Schmp.: 314-317 °C) wurde das gewünschte Produkt (45) als weißer Feststoff (379 mg, 29 %, Schmp.: 154-157 °C) erhalten.

**Beispiel 46: (±)-2-(4-(Dimethylamino)-4-butylcyclohex-1-enyl)-3-(2-(pyridin-4-yl)ethyl)-1H-indol**

3-(2-Pyridin-4-ylethyl)-1*H*-indol (Ind-14, 667 mg, 3 mmol) wurde zusammen mit dem Keton (Ket-4, 592 mg, 3 mmol) in Dichlormethan (45 ml) gelöst und mit Trifluormethansulfonsäure (0,553 ml, 6,3 mmol) versetzt. Der Ansatz wurde 67 h bei RT gerührt, wobei ein braunes Öl ausfiel. Zur Aufarbeitung wurde die Reaktionslösung mit 1 N NaOH (10 ml) und THF (10 ml) versetzt. Das Gemisch wurde weitere 60 min gerührt. Nach Trennung der Phasen wurde die wäßrige Phase mit Dichlormethan (3 x 10 ml) extrahiert. Die vereinigten organischen Extrakte wurden über $Na_2SO_4$ getrocknet und anschließend eingeengt. Das erhaltene Rohprodukt (1,24 g) wurde säulenchromatographisch gereinigt [Kieselgel 60 (100 g); Ethylacetat/Methanol (10 : 1, 1200 ml), Ethylacetat/Methanol (5 : 1, 600 ml), Ethylacetat/Methanol (2 : 1, 700 ml), Ethylacetat/Methanol (1 : 2, 750 ml), Methanol (800 ml)]. Neben einer Bisindolverbindung (121 mg, Schmp.: 274-282 °C) wurde das gewünschte Produkt 46 als weißer Feststoff (437 mg, 36 %, Schmp.: 145-149 °C) erhalten. $^1$H NMR (400 MHz, $CDCl_3$) *δ ppm:* 0.93 (t, J = 6.89, Hz, 3H), 1.16-1.60 (m, 6H), 1.63-1.87 (m, 2H), 1.92-2.06 (m, 1 H), 2.06-2.51 (m, 9H), 2.95 (t, J = 7.82 Hz, 2H), 3.12 (t, J = 7.82 Hz, 2H), 5.82 (m, 1H), 7.02-7.20 (m, 4H), 7.24-7.34 (m, 1H), 7.53 (d, J = 7.77 Hz, 1H), 8.05 (s, 1H), 8.46 (d, J = 5.00 Hz, 1H)

$^{13}$*C NMR (101 MHz, $CDCl_3$) δ ppm:* 14.2, 23.7, 25.6, 26.0, 26.9, 28.5, 30.5, 32.2, 36.4, 38.0, 55.9, 110.4, 110.6, 118.3, 119.4, 121.8, 124.0, 126.5, 128.7, 129.2, 135.1, 136.3, 149.5, 151.2

**Beispiel 47: (±)-2-(4-(Dimethylamino)-4-phenylcyclohex-1-enyl)-3-(2-(pyridin-4-yl)ethyl)-1H-indol**

3-(2-Pyridin-4-ylethyl)-1*H*-indol **(Ind-14**, 667 mg, 3 mmol) wurde zusammen mit dem Keton **(Ket-10**, 652 mg, 3 mmol) in abs. Dichlormethan (45 ml) gelöst und mit Trifluormethansulfonsäure (0,553 ml, 6,3 mmol) versetzt. Der Ansatz wurde 64 h bei RT gerührt, wobei ein braunes Öl ausfiel. Zur Aufarbeitung wurde die Reaktionslösung mit 1 N NaOH (10 ml) und THF (10 ml) versetzt. Das Gemisch wurde weitere 60 min gerührt. Nach Trennung der Phasen wurde die wäßrige Phase mit Dichlormethan (3 x 10 ml) extrahiert. Die vereinigten organischen Extrakte wurden über $Na_2SO_4$ getrocknet und anschließend eingeengt. Das erhaltene Rohprodukt (1,34 g) wurde säulenchromatographisch gereinigt [Kieselgel 60 (100 g); Ethylacetat/Methanol (20 : 1, 500 ml), Ethylacetat/Methanol (5: 1, 870 ml), Ethylacetat/Methanol (2 : 1, 320 ml), Ethylacetat/Methanol (1 : 2, 550 ml)]. Das gewünschte Produkt 47 wurde als farbloser Feststoff (339 mg, 27 %, Schmp.: 193-198 °C) erhalten.

$^1$*H NMR (300 MHz, DMSO-$d_6$) δ ppm:* 1.61-1.83 (m, 1H), 1.97-2.23 (m, 8H), 2.27-2.44 (m, 1 H), 2.50-2.82 (m, 4H), 2.82-3.06 (m, 2H), 6.10 (m, 1H), 6.86-6.96 (m, 1H), 6.96-7.06 (m, 1H), 7.06-7.15 (m, 2H), 7.15-7.35 (m, 4H), 7.41-7.54 (m, 3H), 8.41 (dd, J = 4.43, 1.51 Hz, 2H), 10.63 (s, 1H)

$^{13}$*C NMR (101 MHz, DMSO-$d_6$) δ ppm:* 25.5, 26.6, 27.1, 32.6, 35.9, 60.1, 109.3, 110.7, 117.9, 118.3, 120.8, 123.8, 125.6, 126.3, 127.0, 127.4, 128.1, 129.7, 135.1, 135.8, 142.6, 149.3, 150.5

**Beispiel 48: (±)-3-(2-(4-Benzyl-4-(dimethylamino)cyclohex-1-enyl)-1H-indol-3-yl)propan-1-ol, Citrat (1:1)**

Indol Ind-16 (350 mg, 2 mmol) wurde zusammen mit Keton Ket-3 (463 mg, 2 mmol) in Dichlormethan (40 ml) gelöst und mit Trifluormethansulfonsäure (270 µl, 3 mmol) versetzt. Der Ansatz wurde 24 h bei RT gerührt. Zur Aufarbeitung wurde das Reaktionsgemisch mit 2N NaOH (30 ml) versetzt und 20 min bei RT gerührt. Nach Abtrennung der organischen Phase wurde die wässrige Phase mit Dichlormethan (3 x 15 ml) extrahiert. Die vereinigten organischen Extrakte wurden über $Na_2SO_4$ getrocknet und anschließend eingeengt. Das erhaltene Rohprodukt (780 mg) konnte durch Säulenchromatographie [Kieselgel 60 (100 g); MeOH (500 ml)] gereinigt werden. (±)-3-(2-(4-Benzyl-4-(dimethylamino)cyclohex-1-enyl)-1H-indol-3-yl)propan-1-ol wurde in einer Ausbeute von 356 mg (46 %) als weißer Feststoff erhalten.
(±)-3-(2-(4-Benzyl-4-(dimethylamino)cyclohex-1-enyl)-1H-indol-3-yl)propan-1-ol (50 mg, 0,13 mmol) wurde in der Siedehitze in Isopropanol (4 ml) gelöst und mit Citronensäure (25 mg, 0,13 mmol), gelöst in heißem Isopropanol (1 ml), versetzt. Die Lösung wurde im Kühlschrank auf 5 °C abgekühlt und 16 h stehen gelassen. Der entstandene weiße Niederschlag wurde mittels einer Fritte abgetrennt. **Beispiel 48** wurde so in einer Ausbeute von 50 mg (67 %, *Schmelzpunkt:* 95-98 °C) erhalten.

**Beispiel 49: (±)-3-(2-(4-Butyl-4-(dimethylamino)cyclohex-1-enyl)-1H-indol-3-yl)propan-1-ol, Citrat (1:2) ( 49)**

Indol **Ind-16** (350 mg, 2 mmol) wurde mit Keton **Ket-4** (395 mg, 2 mmol) in Dichlormethan (40 ml) gelöst und mit Trifluormethansulfonsäure (270 µl, 3 mmol) versetzt. Der Ansatz wurde 24 h bei RT gerührt. Zur Aufarbeitung wurde das Reaktionsgemisch mit 2N NaOH (30 ml) versetzt und 20 min bei RT gerührt. Nach Abtrennung der organischen Phase wurde die wässrige Phase mit Dichlormethan (3 x 15 ml) extrahiert. Die vereinigten organischen Extrakte wurden über $Na_2SO_4$ getrocknet und anschließend eingeengt. Das erhaltene Rohprodukt (710 mg) wurde durch Säulenchromatographie [Kieselgel 60 (50 g); MeOH (500 ml)] gereinigt. (±)-3-(2-(4-Butyl-4-(dimethylamino)cyclohex-1-enyl)-1H-indol-3-yl)propan-1-ol wurde in einer Ausbeute von 344 mg (49 %) als gelber Feststoff erhalten.
Der Feststoff wurde in der Siedehitze in Isopropanol (4 ml) gelöst und mit Citronensäure (187 mg, 0,97 mmol), gelöst in heißem Isopropanol (2 ml), versetzt. Die Lösung wurde auf 5 °C abgekühlt (Kühlschrank) und 16 h stehen gelassen. Das Isopropanol wurde am Rotationsverdampfer abdestilliert und **Beispiel 49** so in einer Ausbeute von 531 mg (100 %, *Schmelzpunkt:* 50-54 °C) erhalten.

**Beispiel 51: (±)-2-(2-(2-(4-(Dimethylamino)-4-phenylcyclohex-1-enyl)-1H-indol-3-yl)ethyl)isoindolin-1,3-dion**

Keton **(Ket-10**, 461 mg, 2.12 mmol) und Indol (**Ind-18,** 615 mg, 2.12 mmol) wurden in abs. Dichlormethan (20 ml) unter Argon gelöst. Anschliessend wurde Trifluormethansulfonsäure (230 µL, 2.64 mmol) schnell zugegeben und 24 h bei RT gerührt. Zur Aufarbeitung wurde der Ansatz mit 1 N NaOH basisch gemacht und 15 min bei RT nachgerührt. Die Phasen wurden getrennt, die wässrige Phase wurde mit Dichlormethan (3 x 20 ml) extrahiert. Die organische Phase wurde über $Na_2SO_4$ getrocknet und i. Vak. eingeengt. Der Rückstand wurde durch Flash-Chromatographie mit $CHCl_3$/MeOH (9:1→4:1→1:1) gereinigt.
*Ausbeute:* 560 mg (54 %)

**Beispiel 52: (±)-2-(4-(Dimethylamino)-4-benzylcyclohex-1-enyl)-3-(2-(1H-benzo[d]imidazol-1-yl)ethyl)-5-fluor-1H-indol**

Indol (Ind-19, 668 mg, 2.39 mmol) und Keton **(Ket-3,** 553 mg, 2.39 mmol) wurden in abs. Dichlormethan (30 ml) gelöst und schnell mit Trifluormethansulfonsäure (0.64 ml, 7.2 mmol) versetzt. Der Ansatz wurde 72 h bei RT gerührt, dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit EE/EtOH (9:1→1:2) gereinigt.
*Ausbeute:* 395 mg ( 52; 33 %), poröser Feststoff
*$^1$H-NMR (DMSO-$d_6$):* 1.23 (2 H, m); 2.06 (2 H, m); 2.41 (6 H, bs); 3.22 (2 H, t); 3.33 (2 H, s); 4.43 (2 H, t); 5.58 (1 H, s); 6.84 (1 H, m); 7.08 (3 H, m); 7.29 (6 H, m); 7.40 (1 H, d); 7.56 (1 H, d); 7.86 (1 H, s); 10.92 (1 H, s).

**Beispiel 53: (±)-2-(4-(Dimethylamino)4-butylcyclohex-1-enyl)-3-(2-(1H-benzo[d]imidazol-1-yl)ethyl)-5-fluor-1H-indol**

Indol **(Ind-19**, 977 mg, 3.5 mmol) und Keton **(Ket-4**; 690 mg, 3.5 mmol) wurden in abs. Dichlormethan (30 ml) gelöst und schnell mit Trifluormethansulfonsäure (0.93 ml, 10.5 mmol) versetzt. Der Ansatz wurde 48 h bei RT gerührt, dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit EE/EtOH (1:4) gereinigt. *Ausbeute:* 522 mg (33 %), poröser Feststoff

*$^1$H-NMR (DMSO-$d_6$):* 0.88 (3 H, t); 1.23 (10 H, m); 2.11 (3 H, bs); 2.22 (6 H, bs); 3.22 (2 H, t); 4.43 (2 H, t); 5.62 (1 H, s); 6.86 (1 H, m); 7.20 (4 H, m); 7.41 (1 H, d); 7.61 (1 H, d); 7.86 (1 H, s); 10.83 (1 H, s).

**Beispiel 54: (±)-2-(4-(Dimethylamino)-4-phenylcyclohex-1-enyl)-3-(2-(1H-benzo[d]imidazol-1-yl)ethyl)-5-fluor-1H-indol**

Indol **(Ind-19**, 600 mg, 2.14 mmol) und Keton **(Ket-10,** 466 mg, 2.14 mmol) wurden in abs. Di-chlormethan (30 ml) gelöst und schnell mit Trifluormethansulfonsäure (0.57 ml, 6.4 mmol) versetzt. Der Ansatz wurde 72 h bei RT gerührt, dabei setzte sich ein dunkelbraunes Öl ab. Nach Zugabe von 1N NaOH (20 ml) und $CH_2Cl_2$ (20 ml) wurde noch 20 min nachgerührt, die Phasen getrennt, die wässrige Phase zweimal mit $CH_2Cl_2$ ex-trahiert, die vereinigten organischen Phasen mit Wasser gewaschen, getrocknet ($Na_2O_4$) und die Lösung i. Vak. eingeengt. Der verbliebene Rückstand wurde durch Flash-Chromatographie mit EE/EtOH (9:1→1:2) gereinigt. *Ausbeute:* 886 mg (87 %), poröser Feststoff
*$^1$H-NMR (DMSO-$d_6$):* 1.61 (2 H, m); 1.91 (2 H, m); 2.08 (6 H, bs); 3.13 (2 H, t); 4.31 (2 H, t); 5.85 (1 H, s); 6.82 (1 H, m); 7.20 (9 H, m); 7.64 (1 H, m); 7.85 (1 H, s); 10.79 (1 H, s).

**Beispiel 55: (±)-2-(4-(Dimethylamino)-4-benzylcyclohex-1-enyl)-3-(2-(piperidin-1-yl)ethyl)-5-fluor-1 H-indol**

Indol **(Ind-20**, 739 mg, 3.00 mmol) und Keton **(Ket-3**, 694 mg, 3.0 mmol) wurden bei RT in abs. $CH_2Cl_2$ (30 ml) gelöst und zügig mit Trifluormethansulfonsäure (1.35 g, 0.80 ml, 9.0 mmol) versetzt. Anschließend fiel ein schwarzes Öl aus. Die Mischung wurde 2 d bei RT gerührt. 1 N NaOH (30 ml) wurde zugegeben und das Gemisch 20 min gerührt. Die organische Phase wurde abgetrennt und die wässrige Phase mit $CH_2Cl_2$ (2 x 30 ml) extrahiert. Die vereinigten organischen Phasen wurden mit Wasser (15 ml) gewaschen, über $Na_2SO_4$ getrocknet, filtriert und i. Vak. eingeengt. Durch Flash-Chromatographie mit 50 g Kieselgel und Acetonitril/Methanol/wässrige 1 N Ammoniumchlorid-Lösung (9: 1:1) wurde als erste Fraktion Ind-20 und als zweite Fraktion das gereinigte Produkt erhalten. Die zweite Fraktion wurde i. Vak. eingeengt, mit 2N NaOH basisch gestellt, zweimal mit $CH_2Cl_2$ extrahiert, über $Na_2SO_4$ getrocknet und i. Vak. eingeengt.
*Ausbeute:* 764 mg ( 55, 55 %), farbloser Feststoff
*$^1$H-NMR (DMSO-$d_6$):* 1.37 (2 H, m); 1.49 (4 H, m); 1.95 (2 H, m); 2.31 (6 H, s); 2.41 (8 H, m); 2.80 (4 H, m); 5.91 (1 H, s); 6.83 (1 H, m); 7.20 (7 H, m); 10.73 (1 H, s).

**Beispiel 56: (±)-2-(4-(Dimethylamino)-4-butylcyclohex-1-enyl)-3-(2-(piperidin-1-yl)ethyl)-5-fluor-1 H-indol**

Indol (Ind-20, 1.06 g, 4.30 mmol) und Keton (Ket-4, 848 mg, 4.30 mmol) wurden bei RT in abs. $CH_2Cl_2$ (50 ml) gelöst und zügig mit Trifluormethansulfonsäure (1.96 g, 1.15 ml, 12.9 mmol) versetzt. Anschließend fiel ein schwarzes Öl aus. Die Mischung wurde 3 d bei RT gerührt. 1N NaOH (50 ml) wurde zugegeben und das Gemisch 20 min nachgerührt. Die organische Phase wurde abgetrennt und die wässrige Phase mit $CH_2Cl_2$ (2 x 50 ml) extrahiert. Die vereinigten organischen Phasen wurden mit Wasser (20 ml) gewaschen, über $Na_2SO_4$ getrocknet, filtriert und i. Vak. eingeengt. Durch Flash-Chromatographie mit 50 g Kieselgel und Acetonitril/Methanol/wässrige 1 N Ammoniumchlorid-Lösung (9: 1:1) wurde als erste Fraktion Indol (Ind-20) und als zweite Fraktion das gereinigte Produkt erhalten. Die zweite Fraktion wurde i. Vak. eingeengt, mit 2N NaOH basisch gestellt, zweimal mit $CH_2Cl_2$ extrahiert, über $Na_2SO_4$ getrocknet und i. Vak. eingeengt.
*Ausbeute:* 1.08 g ( 56, 59 %), farbloser Feststoff
*$^1$H-NMR (DMSO-$d_6$):* 0.89 (3 H, t); 1.25-1.61 (10 H, m); 1.79 (2 H, m); 1.96 (2 H, m); 2.21 (6 H, s); 2.39 (8 H, m); 2.83 (2 H, m); 5.97 (1 H, s); 6.81 (1 H, m); 7.16 (2 H, m); 10.79 (1 H, s).

**Beispiel 57: (±)-2-(4-(Dimethylamino)-4-phenylcyclohex-1-enyl)-3-(2-(piperidin-1-yl)ethyl)-5-fluor-1 H-indol**

Indol (Ind-20, 640 mg, 2.60 mmol) und Keton (Ket-10, 565 mg, 2.60 mmol) wurden bei RT in abs. $CH_2Cl_2$ (30 ml) gelöst und zügig mit Trifluormethansulfonsäure (1.17 g, 0.69 ml, 7.8 mmol) versetzt. Anschließend fiel ein schwarzes Öl aus. Die Mischung wurde 24 h bei RT gerührt. 1 N NaOH (30 ml) wurde zugegeben und das Gemisch 20 min nachgerührt. Die organische Phase wurde abgetrennt und die wässrige Phase zweimal mit $CH_2Cl_2$ (je 30 ml) extrahiert. Die vereinigten organischen Phasen wurden mit Wasser (15 ml) gewaschen, über $Na_2SO_4$ getrocknet, filtriert und i. Vak. eingeengt. Durch Flash-Chromatographie mit 50 g Kieselgel und Acetonitril/Methanol/wässrige 1N Ammoniumchlorid-Lösung (9:1:1) wurde als erste Fraktion Indol (Ind-20) und als zweite Fraktion das gereinigte Produkt erhalten. Die zweite Fraktion wurde i. Vak. eingeengt, mit 2N NaOH basisch gestellt, zweimal mit $CH_2Cl_2$ extrahiert, über $Na_2SO_4$ getrocknet und i. Vak. eingeengt.
*Ausbeute:* 404 mg (35 %), farbloser Feststoff
*$^1$H-NMR (DMSO-$d_6$):* 1.37-1.49 (6 H, m); 1.74 (2 H, m); 2.09 (6 H, s); 2.27 (6 H, m); 2.73 (4 H, m); 6.21 (1 H, s); 6.81

(1 H, m); 7.08-7.47 (7 H, m); 10.72 (1 H, s).

**Beispiel 58: (±)-2-(4-(Dimethylamino)-4-phenylcyclohex-1-enyl)-3-(2-(piperidin-1-yl)ethyl)-1H-indol, Citrat (1:1)**

Das Keton (Ket-10, 1.73 g, 8.0 mmol) und Indol (Ind-21, 1.82 g, 8.0 mmol) wurden in Dichlormethan (100 ml) gelöst. Dann erfolgte eine portionsweise Zugabe der Trifluormethansulfonsäure (insgesamt 3.12 ml, 36 mmol innerhalb von 3 d). Zur Aufarbeitung wurde der Ansatz mit 2N NaOH (150 ml) versetzt und bei Raumtemperatur 20 min gerührt. Die organische Phase wurde abgetrennt und die wässrige Phase mit Dichlormethan (2 x 2 5 ml) extrahiert. Die vereinigten organischen Phasen wurden über $Na_2SO_4$ getrocknet und i. Vak. eingeengt. Der Rückstand wurde durch Flash-Chromatographie mit Methanol gereinigt. *Ausbeute:* 0.209 g (7 %)
*$^1$H-NMR ($CDCl_3$):* 1.49-1.69 (6 H, m); 2.02-2.18 (10 H, m); 2.44 (4 H, m); 2.70 (2 H; m); 2.93 (2 H, t); 3.43 (2 H, t); 6.14 (1 H, m); 7.07 (2 H, m); 7.26 (5 H, m); 7.47 (2 H, m); 8.40 (1 H, s):

**Beispiel 59: (±)-2-(4-(Dimethylamino)-4-benzylcyclohex-1-enyl)-3-(2-(1H-1,2,3-triazol-1-yl)ethyl)-5-fluor-1 H-indol**

Indol (Ind-22, 750 mg, 3.25 mmol) und Keton (Ket-3, 752 mg, 3.25 mmol) wurden in abs. Dichlormethan (30 ml) gelöst und schnell mit Trifluormethansulfonsäure (0.87 ml, 9.8 mmol) versetzt. Der Ansatz wurde 48 h bei RT gerührt, dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit EE/EtOH (1:4) gereinigt.
*Ausbeute:* 387 mg (27 %), farbloser Feststoff; *Schmelzpunkt:* 175-183 °C
*$^1$H-NMR (DMSO-$d_6$):* 1.37 (1 H, m); 1.97 (3 H, m); 2.31 (6 H, bs); 2.72 (4 H, m); 3.24 (2 H, t); 4.56 (2 H, t); 6.86 (1 H, m); 7.22 (7 H, m); 7.62 (1 H, s); 8.00 (1 H, s); 10.91 (1 H, s).

**Beispiel 60: (±)-2-(4-(Dimethylamino)-4-butylcyclohex-1-enyl)-3-(2-(1H-1,2,3-triazol-1-yl)ethyl)-5-fluor-1H-indol**

Indol (Ind-22, 825 mg, 3.58 mmol) und Keton (Ket-4, 707 mg, 3.58 mmol) wurden in abs. Dichlormethan (25 ml) gelöst und schnell mit Trifluormethansulfonsäure (0.96 ml, 10.8 mmol) versetzt. Der Ansatz wurde 48 h bei RT gerührt, dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ ex-trahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit EE/EtOH (1:4) gereinigt.
*Ausbeute:* 514 mg (35 %), poröser Feststoff
*$^1$H-NMR (DMSO-$d_6$):* 0.89 (3 H, t); 1.33 (6 H, m); 1.77 (2 H, m); 1.99 (1 H, m); 2.28 (6 H, bs); 2.38 (2 H, m); 3.25 (2 H, t); 4.54 (2 H, t); 5.83 (1 H, s); 6.87 (1 H, m); 7.22 (2 H, m); 7.64 (1 H, s); 7.98 (1 H, s); 10.92 (1 H, s).

**Beispiel 61: (±)-2-(4-(Dimethylamino)-4-phenylcyclohex-1-enyl)-3-(2-(1H-1,2,3-triazol-1-yl)ethyl)-5-fluor-1H-indol**

Indol (Ind-22, 600 mg, 2.6 mmol) und Keton (Ket-10, 565 mg, 2.6 mmol) wurden in abs. Dichlormethan (20 ml) gelöst und schnell mit Trifluormethansulfonsäure (0.7 ml, 7.8 mmol) versetzt. Der Ansatz wurde 20 h bei RT gerührt, dabei setzte sich ein dunkelbraunes Öl ab. Nach Zugabe von 1N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit EE/EtOH (1:4) gereinigt.
*Ausbeute:* 803 mg (72 %), farbloser Feststoff, *Schmelzpunkt:* 90-97 °C
*$^1$H-NMR (DMSO-$d_6$):* 1.68 (1 H m); 2.10 (6 H, s); 2.39 (1 H, m); 2.62 (2 H, m); 3.20 (2 H, t); 4.47 (2 H, t); 6.06 (1 H, s); 6.81 (1 H, m); 7.16 (5 H, m); 7.46 (2 H, m); 7.66 (1 H, s); 7.95 (1 H, s); 10.82 (1 H, s).

**Beispiel 62: 2',3',4',9'-Tetrahydro-N,N-dimethyl-4-butyl-spiro[cyclohexanE-1,1'(1'H)-pyrido[3,4-b]indol]-4-amin Unpolareres Diastereomer**

Tryptamin (Ind-1, 2,43 g, 15,2 mmol) und das Keton (Ket-4, 3,0 g, 15,2 mmol) wurden in abs. Methanol (90 ml) gelöst und 25 h bei Raumtemperatur unter Argon gerührt. Die Reaktionsmischung wurde anschließend eingeengt. Der Rückstand wurde in abs. 1,2-Dichlorethan (150 ml) gelöst, schnell mit Trifluoressigsäure (10,4 ml, 15,5 g, 136 mmol) versetzt und 3 d bei Raumtemperatur gerührt. Die braune Lösung wurde unter Eiskühlung mit 1N Natronlauge (130 ml)

versetzt und 20 min bei RT gerührt. Die Phasen der Lösung wurden getrennt. Die wäßrige Phase wurde mit 1,2-Dichlorethan (2 x 70 ml) extrahiert. Die organischen Phasen wurden vereinigt, mit Wasser (50 ml) gewaschen, getrocknet und eingeengt. Der braune ölige Rückstand wurde mit Methanol (60 ml) versetzt, wodurch es zur Kristallisation kam. Die Suspension wurde noch 10 min gerührt. Die farblosen Kristalle wurden abgesaugt und mit Methanol (60 ml) gewaschen (1,28 g). Es handelte sich hierbei um das reine unpolarere Spiroamin. Das Filtrat wurde eingeengt und der erhaltene braune Feststoff wurde erneut mit Methanol (50 ml) versetzt und 1 h im Eisbad gerührt. Nach Absaugen und Waschen mit kaltem Methanol (20 ml) konnten 673 mg des unpolareren Spiroamins gewonnen werden. Das Filtrat wurde eingeengt und der Rückstand (2,4 g) chromatographisch aufgetrennt [Kieselgel 60 (130 g); Methanol (500 ml), Methanol/Triethylamin (100 : 1, 1,5 l)]. Das unpolarere Spiroamin wurde zusammen mit Verunreinigungen erhalten (1,02 g). Diese Fraktion wurde mit kaltem Methanol (10 ml) versetzt und abgesaugt. Der erhaltene Feststoff (332 mg) war reines unpolares Produkt. Das unpolarere Spiroamin wurde in einer Gesamtausbeute von 44 % (2,28 g) mit einem Schmelzpunkt von 180-182 °C erhalten. Das polarere Spiroamin wurde in einer weiteren Fraktion in einer Ausbeute von 12 % (622 mg) mit einem Schmelzpunkt von 93-96 °C gewonnen.

**(±)-N-(2-(2-(4-Butyl-4-(dimethylamino)cyclohex-1-enyl)-1H-indol-3-yl)ethyl)-3,3-dimethylbutanamid**

3,3-Dimethylbuttersäurechlorid (0,246 ml, 238 mg, 1,77 mmol) wurde unter Argon in abs. Dichlormethan (5 ml) gelöst und bei Raumtemperatur mit dem soeben hergestellten unpolareren Spiroamin (200 mg, 0,59 mmol), gelöst in Dichlormethan (15 ml), innerhalb von 30 min versetzt. Nach einer Reaktionszeit von 24 h wurde die gelbe Reaktionslösung mit Wasser (10 ml) und 1 N Natronlauge (5 ml) versetzt und 1 h gerührt. Die Phasen wurden getrennt. Die wäßrige Phase wurde mit Dichlormethan (20 ml) extrahiert. Die vereinigten organischen Phasen wurden mit Wasser (20 ml) gewaschen, getrocknet und eingeengt. Dabei wurde ein beigefarbenes Öl (322 mg) erhalten, das chromatographisch aufgetrennt wurde [Kieselgel 60 (40 g); Ethylacetat (250 ml), Ethylacetat/Methanol (4 : 1, 400 ml), Methanol (300 ml)]. Das gewünschte Olefin wurde in einer Ausbeute von 58 % (150 mg) mit einem Schmelzpunkt von 139-142 °C isoliert.

**N-(2-(2-(4-Butyl-4-(dimethylamino)cyclohexyl)-1H-indol-3-yl)ethyl)-3,3-dimethylbutanamid**

Das soeben hergestellte Olefin (82 mg, 0,187 mmol) wurde in Methanol (20 ml) gelöst und mit Palladium auf Kohle 5 % (16 mg) versetzt. Die Reaktionsmischung wurde bei Raumtemperatur 4,5 h bei 3 bar hydriert. Die Umsetzung erfolgte vollständig. Der Katalysator wurde über Celite abgetrennt und das Filtrat eingeengt. Der feste Rückstand (84 mg) wurde chromatographisch aufgetrennt [Kieselgel 60 (20 g); Ethylacetat/Methanol (4 : 1, 150 ml), Methanol (300 ml)]. Das polarere Amid in einer Ausbeute von 83 % (68 mg) gewonnen.

**N-(2-(2-(4-Butyl-4-(dimethylamino)cyclohexyl)-1H-indol-3-yl)ethyl)-3,3-dimethylbutanamide, Citrat (1:1) ( 62; : Polareres Diastereomer)**

Das soeben hergestellte polarere Amid (47 mg, 0,107 mmol) wurde in Ethanol (1 ml) gelöst und mit Citronensäure (23 mg), gelöst in Ethanol (1 ml), versetzt. Da nach 6 h keine Kristallisation erfolgte wurde die Mischung langsam mit Diethylether (15 ml) versetzt und bei Raumtemperatur 16 h gerührt. Das Lösungsmittel wurde dekantiert. Der zurückgebliebene farblose Feststoff wurde feucht in das Abgaberöhrchen überführt und getrocknet. Das Citrat 62 wurde in einer Ausbeute von 69 % (46 mg) erhalten.

**Beispiel 63: (±)-N-(2-(2-(4-(Dimethylamino)-4-phenylcyclohex-1-enyl)-1H-indol-3-yl)ethyl)acetamid**

Keton (Ket-10, 234 mg, 1.08 mmol) und Indol (Ind-2, 219 mg, 1.08 mmol) wurden in abs. Dichlormethan (10 ml) unter Argon gelöst, mit Trifluormethansulfonsäure (188 μL/ 2.16 mmol) schnell versetzt und 16 h bei RT gerührt. Anschließend wurde mit 1 N NaOH alkalisch gestellt und 15 min bei RT nachgerührt. Die Phasen wurden getrennt. Die wässrige Phase wurde mit Dichlormethan (dreimal 20 ml) extrahiert. Die organische Phase wurde über $Na_2SO_4$ getrocknet und i. Vak. eingeengt. Der Rückstand wurde durch Flash-Chromatographie mit CH/EE (1:1→1:4), EE/EtOH (4: 1→1:1), EtOH, (Methanol + 1 % TEA) gereinigt. Die Fraktionen wurden mit 1 N NaOH versetzt und mit Dichlormethan (zweimal 10 ml) extrahiert. Die organische Phase wurde über $Na_2SO_4$ getrocknet und i. Vak. eingeengt.
*Ausbeute:* 86 mg ( 63, 19 %)

**Beispiel 64: (±)-N-(2-(2-(4-Butyl-4-(dimethylamino)cyclohex-1-enyl)-1H-indol-3-yl)ethyl)-3,3-dimethylbutanamid**

Die unter Beispiel 62 beschriebene Substanz (±)-N-(2-(2-(4-butyl-4-(dimethylamino)cyclohex-1-enyl)-1H-indol-3-yl)ethyl)-3,3-dimethylbutanamide Wird im weiteren als Beispiel 64 geführt.

**Beispiel 65. (±)-2-(2-(4-(Dimethylamino)-4-phenylcyclohex-1-enyl)-5-fluor-6-methoxy-1H-indol-3-yl)ethanol, Citrat (1:1)**

Eine Lösung von Ind-26 (1.60 g, 7.63 mmol) und Ket-10 (1.65 g, 7.63 mmol) in wasserfreiem Dichlormethan (40 ml) wurde bei Raumtemperatur mit Trifluormethansulfonsäure (1.52 g 10.1 mmol) versetzt und 16 h bei Raumtemperatur gerührt. Die Reaktionslösung wurde anschließend mit 0.5 M Natronlauge (10 ml) versetzt und 2 h bei Raumtemperatur gerührt. Die Phasen wurden getrennt und die wässrige Phase mit Ethylacetat (3 × 30 ml) extrahiert. Die vereinigten organischen Phasen wurden mit Natriumsulfat getrocknet und i. Vak. eingeengt. Der Rückstand wurde durch Flashchromatographie (200 g, 20 × 5.6 cm) mit Chloroform / Methanol (10:1) gereinigt. Der Rückstand wurde in 1 N Natronlauge aufgenommen und mit Dichlormethan (3 × 40 ml) extrahiert. Die vereinigten organischen Phasen wurden mit Natriumsulfat getrocknet und i. Vak. eingeengt.
Der Rückstand (100 mg, 0.24 mmol) wurde zusammen mit DL-Methionin (107 ml, 0.72 mmol) in Dichlormethan (10 ml) mit Methansulfonsäure (69 mg, 0.72 mmol) versetzt und 2 h bei Raumtemperatur gerührt. Nach Zugabe weiterer Methansulfonsäure (69 mg, 0.72 mmol) wurde das Reaktionsgemisch über das Wochenende gerührt, anschließend mit 1 N Natronlauge (30 ml) versetzt und mit Dichlormethan (3 × 40 ml) extrahiert. Die vereinigten organischen Phasen wurden mit Natriumsulfat getrocknet und i. Vak. eingeengt.
Ausbeute (Bsp 65): 63 mg (66 %), farbloser Feststoff
$^1$H-NMR (300 MHz, *DMSO-d$_6$*): 1.57-1.80 (m, 2H), 2.09 (s, 6H), 2.41 (d, *J* = 17.1 Hz, 2H), 2.62 (t, *J* = 16.6 Hz, 2H), 2.77 (t, *J* = 7.3 Hz, 2H), 3.50 (dd, *J* = 12.3, 4.8 Hz, 2H), 3.79 (s, 3H), 4.60 (t, *J* = 5.4 Hz, 1H), 6.17 (s, 1H), 6.84 (d, *J* = 7.5 Hz, 1H), 7.15 (s, 1H), 7.17-7.26 (m, 1H), 7.31 (t, *J* = 7.5 Hz, 2H), 7.47 (d, *J* = 7.4 Hz, 2H), 10.52 (s, 1H).

**Beispiel 66. (±)-2-(2-(4-benzyl-4-(4-methylpiperazin-1-yl)cyclohex-1-enyl)-5-fluor-1H-indol-3-yl)ethanol**

Eine Lösung von Ket-2 (700 mg, 2.44 mmol) und 5-Fluortryptophol (Ind-4) (449 mg, 2.5 mmol) in wasserfreiem Dichlormethan (25 ml) wurde unter Eiskühlung mit Trifluormethansulfonsäure (450 mg, 265 μL, 3 mmol) versetzt und über Nacht bei Raumtemperatur gerührt. Zur Umsatzkontrolle wurde eine Probe (0.5 ml) entnommen, diese mit 0.5 N Natronlauge gewaschen und die organische Phase mit Natriumsulfat getrocknet. Unter Eiskühlung wurde Trifluormethansulfonsäure (450 mg, 265 μL, 3 mmol) zugegeben und das Gemisch über das Wochenende bei Raumtemperatur gerührt. Anschließend wurde das Reaktionsgemisch mit 0.5 N Natronlauge (10 ml) versetzt, 2 h bei Raumtemperatur gerührt, die wässrige Phase mit Dichlormethan (2 × 20 ml) extrahiert, die vereinigten organischen Phasen wurden mit Natriumsulfat getrocknet und i. Vak. eingeengt.
Das Rohprodukt wurde mittels Flashchromatographie (200 g, 20 × 5.7 cm) mit Methanol gereinigt.
***Ausbeute* ( 66):** 60 mg (0.1 %), gelber Feststoff, *Schmelzpunkt:* 91-97 °C
$^1$H-NMR($CDCl_3$): 1.48-1.75 (m, 2H); 1.93-2.21 (m, 2H); 2.28 (s, 3H); 2.38-2.50 (m, 4H); 2.69-2.87 (m, 7H); 3.03 (t, 2H, J = 6.8 Hz); 3.48 (s, 2H); 3.85 (t, 2H, J = 6.6 Hz); 5.92 (s, 1H); 6.82-6.95 (m, 1H); 7.16-7.32 (m, 7H); 7.92 (s, 1H).

**Beispiel 67. (±)-2-(5-fluor-2-(4-phenyl-4-(pyrrolidin-1-yl)cyclohex-1-enyl)-1H-indol-3-yl)ethanol (67)**

Eine Lösung von Ket-8 (486 mg, 2 mmol) und 5-Fluortryptophol (Ind-4) (358 mg, 2 mmol) in wasserfreiem Dichlormethan (20 ml) wurde bei 5-10 °C mit Trifluormethansulfonsäure (399 mg, 232 μL, 2.66 mmol) versetzt und über Nacht bei Raumtemperatur gerührt. Nach Zugabe von 0.5 M Natronlauge (10 ml) wurden die Phasen getrennt und die wässrige Phase mit Dichlormethan (3 × 10 ml) extrahiert. Die vereinigten organischen Phasen wurden mit Natriumsulfat getrocknet und i. Vak. eingeengt. Das Rohprodukt (596 mg) wurde durch Flashchromatographie (18 g, 20 × 1.5 cm) mit Ethylacetat / Cyclohexan (1:9→2:1) und jeweils 1 % Triethylamin gereinigt.
*Ausbeute.* 140 mg (17 %), weißer Feststoff, *Schmelzpunkt:* 188-191 **°C**
$^1$*H-NMR (DMSO-d$_6$):* 1.59 (br s, 4H); 1.76-1.88 (m, 1H); 2.08-2.20 (m, 2H); 2.34-2.48 (m, 3H); 2.52-2.60 (m, 2H); 2.66 (d, 1 H, J = 18.5 Hz); 2.80 (t, 3H, J = 7.3 Hz); 3.47 (dd, 2H, J = 13.1, 7.3 Hz); 4.58 (t, 1H, J = 5.3 Hz); 6.22 (s, 1H); 6.77-6.84 (m, 1H); 6.81 (dt, 1H, J = 8.8, 1.9 Hz); 7.12-7.24 (m, 3H); 7.32 (t, 2H, J = 7.6 Hz); 7.48 (d, 2H, J = 7.9 Hz); 10.07 (s, 1 H). $^{13}$*C-NMR (DMSO-d$_6$):* 22.9; 26.0; 28.6; 28.9; 33.4; 44.8; 58.2; 61.7; 102.7 (d, J = 24 Hz); 107.9 (d, J = 6 Hz); 108.7 (d, J = 26 Hz); 111.4 (d, J = 10 Hz); 125.7; 126.2; 126.8; 127.5; 129.1 (d, J = 10 Hz); 129.3; 131.7; 138.0; 142.2; 156.6 (d, J = 231 Hz).

**Beispiel 68: 2-(4-Benzyl-4-(dimethylamino)cyclohexyl)-3-methyl-1H-indol-5-carbonitril, Citrat (1:1), unpolares Diastereomer**

**Beispiel 69: 2-(4-Benzyl-4-(dimethylamino)cyclohexyl)-3-methyl-1H-indol-5-carbonitril, Citrat (1:1), polares Diastereomer**

**2-(4-Benzyl-4-(dimethylamino)cyclohexyl)-3-methyl-1H-indol-5-carbonitril) (unpolares und polares Diastereomer)**

Bsp. 22 (280 mg, 0,758 mmol) wurde in Methanol (100 ml) gelöst und mit Palladium auf Kohle (5-proz.; 110 mg) versetzt. Die Reaktionsmischung wurde 4,5 h bei 40°C und 3 bar hydriert. Die Umsetzung erfolgte vollständig. Der Katalysator wurde über Celite abgetrennt und das Filtrat eingeengt. Der feste Rückstand wurde chromatographisch aufgetrennt [Kieselgel 60 (30 g); Ethylacetat/Methanol (20 : 1, 200 ml), Ethylacetat/Methanol (10 : 1, 200 ml), Ethylacetat/ Methanol (4 : 1, 350 ml), Methanol (200 ml)]. Das unpolarere Diastereoisomer wurde in einer Ausbeute von 27 % (77 mg) mit einem Schmelzpunkt von 205-213 °C und das polarere in einer Ausbeute von 55 % (155 mg) mit einem Schmelzpunkt von 175-182 **°C** gewonnen.

**2-(4-Benzyl-4-(dimethylamino)cyclohexyl)-3-methyl-1H-indol-5-carbonitril, Citrat (1:1), unpolares Diastereomer (68)**

2-(4-Benzyl-4-(dimethylamino)cyclohexyl)-3-methyl-1H-indol-5-carbonitril **(unpolares Diastereomer)** (72 mg, 0,194 mmol) wurde bei 60 °C in Ethanol (9 ml) gelöst und mit einer ethanolischen Lösung (2 ml) von Citronensäure (41 mg, 0,213 mmol) versetzt. Eine Fällung begann sofort. Nach einer Reaktionszeit von 16 h bei Raumtemperatur wurde das farblose Citrat durch Filtration abgetrennt und mit Ethanol (2 ml) gewaschen. Beispiel 68 wurde in einer Ausbeute von 46 % (50 mg) mit einem Schmelzpunkt von 249-255 °C erhalten

**2-(4-Benzyl-4-(dimethylamino)cyclohexyl)-3-methyl-1H-indol-5-carbonitril, Citrat (1:1), polares Diastereomer (69)**

2-(4-Benzyl-4-(dimethylamino)cyclohexyl)-3-methyl-1H-indol-5-carbonitril (polares Diastereomer) (158 mg, 0,425 mmol) wurde bei 60°C in Ethanol (7 ml) gelöst und mit einer ethanolischen Lösung (2 ml) von Citronensäure (91 mg, 0,47 mmol) versetzt. Nach einer Reaktionszeit von 16 h bei Raumtemperatur wurde das farblose Citrat durch Filtration abgetrennt und mit Ethanol (3 ml) gewaschen. **Beispiel 69** wurde in einer Ausbeute von 51 % (122 mg) mit einem Schmelzpunkt von 193-195 °C erhalten

**Beispiel 70: 2-(4-Butyl-4-(dimethylamino)cyclohexyl)-3-methyl-1H-indol-5-carbonitril, Citrat (2:1), unpolares Diastereomer**

**Beispiel 71: 2-(4-Butyl-4-(dimethylamino)cyclohexyl)-3-methyl-1H-indol-5-carbonitril, Citrat (1:1), polares Diastereomer**

(±)-2-(4-Butyl-4-dimethylaminocyclohex-1-enyl)-3-methyl-1*H*-indol-5-carbonitril Das Keton **Ket-4** (517 mg, 2,62 mmol) und Indol Ind-6 (410 mg, 2,62 mmol) wurden in abs. Dichlormethan (40 ml) gelöst, mit Trifluormethansulfonsäure (0,256 ml, 432 mg, 2,88 mmol) versetzt und 5 Tage bei Raumtemperatur gerührt. Der klare Ansatz wurde mit Wasser (20 ml) und 1 N Natronlauge (15 ml) versetzt und 30 min gerührt. Die Phasen wurden getrennt. Die wässrige Phase wurde mit Dichlormethan (20 ml) extrahiert. Die organischen Phasen wurden vereinigt, mit Wasser (20 ml) gewaschen, getrocknet und eingeengt. Der Rückstand war ein braunes Öl (942 mg), das chromatographisch aufgetrennt wurde [Kieselgel 60 (70 g); Ethylacetat (700 ml), Ethylacetat/Methanol (10 : 1, 200 ml), Ethylacetat/Methanol (4:1. 750 ml), Methanol (200 ml)]. (±)-2-(4-Butyl-4-dimethylaminocyclohex-1-enyl)-3-methyl-1*H*-indol-5-carbonitril wurde als beigefarbenes Öl in einer Ausbeute von 33 % (289 mg) gewonnen.

**2-(4-Butyl-4-dimethylaminocyclohexyl)-3-methyl-1*H*-indol-5-carbonitril (unpolares und polares Diastereomer)**

(±)-2_(4-Butyl-4-dimethylaminocyclohex-1-enyl)-3-methy)-1*H*-indol-5-carbonitril (266 mg, 0,79 mmol) wurde in Methanol (85 ml) gelöst und mit Palladium auf Kohle (5 proz.; 110 mg) versetzt. Die Reaktionsmischung wurde bei 40 °C 2 h bei 3 bar hydriert. Die Umsetzung erfolgte vollständig. Der Katalysator wurde über Celite abgetrennt und das Filtrat eingeengt. Der feste Rückstand (239 mg) wurde chromatographisch aufgetrennt [Kieselgel 60 (40 g); Ethylacetat/ Methanol (10 : 1, 200 ml), Ethylacetat/Methanol (4 : 1, 250 ml), Methanol (400 ml)]. 2-(4-Butyl-4-dimethylaminocyclohexyl)-3-methyl-1*H*-indol-5-carbonitri (unpolareres Diastereoisomer) wurde in einer Ausbeute von 11 % (30 mg) mit einem Schmelzpunkt von 176-180 °C und das polarere Diastereoisomer in einer Ausbeute von 57 % (151 mg) mit einem Schmelzpunkt von 162-166 °C gewonnen.

**2-(4-Butyl-4-(dimethylamino)cyclohexyl)-3-methyl-1H-indol-5-carbonitril, Citrat (2:1), unpolares Diastereomer**

2-(4-Butyl-4-dimethylaminocyclohexyl)-3-methyl-1*H*-indol-5-carbonitri (unpolareres Diastereoisomer) (30 mg, 0,089 mmol) wurde bei 60 °C in Ethanol (3 ml) gelöst und mit einer ethanolischen Lösung (2 ml) von Citronensäure (19 mg, 0,098 mmol) versetzt. Nach einer Reaktionszeit von 20 h wurde die Lösung auf 1 ml eingeengt, mit Diethylether (20 ml) versetzt und 15 min gerührt. Das farblose Citrat wurde durch Filtration abgetrennt und mit Diethylether (2 ml) gewaschen. Beispiel 70 in einer Ausbeute von 45 % (21 mg) erhalten .

**2-(4-Butyl-4-(dimethylamino)cyclohexyl)-3-methyl-1H-indol-5-carbonitril, Citrat (1:1), polares Diastereomer**

2-(4-Butyl-4-dimethylaminocyclohexyl)-3-methyl-1*H*-indol-5-carbonitri (polareres Diastereoisomer) (144 mg, 0,426 mmol) wurde bei 60 °C in Ethanol (6 ml) gelöst und mit einer ethanolischen Lösung (3 ml) von Citronensäure (90 mg, 0,47 mmol) versetzt. Nach einer Reaktionszeit von 18 h wurde die trübe Mischung auf 1 ml eingeengt, mit Diethylether (20 ml) versetzt und 15 min gerührt. Das farblose Citrat wurde durch Filtration abgetrennt und mit Diethylether (3 ml) gewaschen. Beispiel 71 wurde in einer Ausbeute von 76 % (171 mg) erhalten.

**Beispiel 72: 2-(4-(Dimethylamino)-4-phenylcyclohexyl)-3-methyl-1H-indol-5-carbonitril, Citrat (2:1), polares Diastereomer**

**2-(4-Dimethylamino-4-phenylcyclohexyl)-3-methyl-1*H*-indol-5-carbonitril (polares Diastereomer)**

Bsp. 23 (200 mg, 0,562 mmol) wurde in Methanol (30 ml) gelöst und mit Palladium auf Kohle (5-proz.; 75 mg) versetzt. Die Reaktionsmischung wurde 20 h bei 3 bar hydriert. Die Umsetzung erfolgte vollständig. Der Katalysator wurde über Celite abgetrennt und das Filtrat eingeengt. Der feste Rückstand (150 mg) wurde chromatographisch aufgetrennt [Kieselgel 60 (30 g); Ethylacetat/Methanot (4 : 1,250 ml), Methanol (300 ml)]. 2-(4-Dimethylamino-4-phenylcyclohexyl)-3-methyl-1*H*-indol-5-carbonitril (polares Diastereomer) wurde in einer Ausbeute von 50 % (100 mg) mit einem Schmelzpunkt von 235-240 °C gewonnen.

**2-(4-(Dimethylamino)-4-phenylcyclohexyl)-3-methyl-1*H*-indol-5-carbonitril, Citrat (2:1), polares Diastereomer**

2-(4-Dimethylamino-4-phenylcyclohexyl)-3-methyl-1*H*-indol-5-carbonitril (polares Diastereomer) (95 mg, 0,265 mmol) wurde in Ethanol (7 ml) gelöst und mit Citronensäure (56 mg), gelöst in Ethanol (2 ml), versetzt. Da nach 16 h keine Kristallisation erfolgte wurde die Mischung auf 2 ml eingeengt, mit Diethylether (30 ml) versetzt und bei Raumtemperatur 10 min gerührt. Das Lösungsmittel wurde dekantiert. Der zurückgebliebene farblose Feststoff wurde feucht in das Abgaberöhrchen überführt und getrocknet. Beispiel 72 wurde in einer Ausbeute von 75 % (90 mg) mit einem Schmelzpunkt von 257-261 °C erhalten.

**Beispiel 73: 1-Benzyl-N,N-dimethyl-4-(3-methyl-5-(trifluormethyl)-1H-indol-2-yl)cyclohexanamin, Citrat (2:1), unpolares Diastereomer**

**Beispiel 74: 1-Benzyl-N,N-dimethyl-4-(3-methyl-5-(trifluormethyl)-1H-indol-2-yl)cyclohexanamin, Citrat (1:1), polares Diastereomer**

**(±)-[1-Benzyl-4-(3-methyl-5-trifluormethyl-1*H*-indol-2-yl)cyclohex-3-enyl]-dimethylamin** Keton Ket-3 (393 mg, 1,7 mmol) wurde zusammen mit Indol Ind-7 (340 mg, 1,7 mmol) in Dichlormethan (20 ml) gelöst. Anschließend erfolgte die Zugabe von Trifluormethansulfonsäure (0,17 ml, 287 mg, 1,9 mmol), wobei sich der Ansatz dunkel färbte. Es wurde 3 d bei RT gerührt. Der Verlauf der Reaktion wurde mittels DC verfolgt. Zur Aufarbeitung wurde das Reaktionsgemisch mit 1 N NaOH (10 ml) versetzt und 10 min gerührt. Die Farbe wechselte dabei von dunkelrot nach hellbraun. Nach Phasentrennung wurde die wässrige Phase mit Dichlormethan (2 × 10 ml) extrahiert. Die vereinigten organischen Phasen wurden mit Natriumsulfat getrocknet, filtriert und im Vakuum eingeengt. Es wurden 1,25 g braunes Öl erhalten, das chromatographisch getrennt wurde [Kieselgel 60 (80 g); Ethylacetat/Methanol (10 : 1; 1500 ml)]. (±)-[1-Benzyl-4-(3-methyl-5-trifluormethyl-1*H*-indol-2-yl)cyclohex-3-enyl]-dimethylamin wurde in einer Ausbeute von 31 % (220 mg) mit einem Schmelzpunkt von 119-120 °C als farbloser Feststoff erhalten.

***N*-[1-Benzyl-4-(3-methyl-5-trifluormethyl-1*H*-indol-2-yl)cyclohexyl]-*N,N*-dimethylamin (unpolares und polares Diastereomer)**

(±)-[1-Benzyl-4-(3-methyl-5-trifluormethyl-1*H*-indol-2-yl)cyclohex-3-enyl]-dimethylamin (220 mg, 0,53 mmol) wurde unter Erwärmen in Methanol (50 ml) gelöst und unter Argon mit Pd/C (5 %, 100 mg) versetzt. Bei 3 bar wurde 4 h bei 40 °C hydriert. Anschließend wurde der Katalysator über Celite abgesaugt und das Filtrat eingeengt. Der feste farblose Rückstand wurde chromatographisch getrennt [Kieselgel 60 (30 g); Ethylacetat/Methanol (20 : 1; 500 ml); (4 : 1; 300 ml); (2 : 1; 300 ml)]. *N*-[1-Benzyl-4-(3-methyl-5-trifluormethyl-1*H*-indol-2-yl)cyclohexyl]-*N,N*-dimethylamin (unpolarere Diastereoisomer) wurde in einer Ausbeute von 46 mg (21 %) als farbloser Feststoff mit einem Schmelzpunkt von 180-210 °C isoliert. Das polarere Diastereoisomer wurde in einer Ausbeute von 152 mg (69 %) als farbloser Feststoff mit einem Schmelzpunkt von 166-174 °C erhalten.

**1-Benzyl-N,N-dimethyl-4-(3-methyl-5-(trifluormethyl)-1H-indol-2-yl)cyclohexanamin, Citrat (2:1), unpolares Diastereomer (73)**

*N*-[1-Benzyl-4-(3-methyl-5-trifluormethyl-1*H*-indol-2-yl)cyclohexyl]-*N*,*N*-dimethylamin (unpolares Diastereoisomer) (46 mg, 0,11 mmol) wurde in Ethanol (10 ml) gelöst und mit Citronensäure (23 mg, 0,122 mmol), gelöst in heißem Ethanol (2 ml), versetzt. Nach 10 min Rühren bei RT begann ein farbloser Feststoff auszufallen. Das Reaktionsgemisch wurde mit Diethylether (5 ml) versetzt und 16 h gerührt. Danach wurde abgesaugt. Beispiel 73 wurde in einer Ausbeute von 42 % (28 mg) mit einem Schmelzpunkt von 201-203 °C gewonnen .

**1-Benzyl-N,N-dimethyl-4-(3-methyl-5-(trifluormethyl)-1H-indol-2-yl)cyclohexanamin, Citrat (1:1), polares Diastereomer**

*N*-[1-Benzyl-4-(3-methyl-5-trifluormethyl-1*H*-indol-2-yl)cyclohexyl]-*N,N*-dimethylamin (polares Diastereoisomer) 135 mg, 0,33 mmol) wurde in Ethanol (20 ml) gelöst und mit Citronensäure (70 mg, 0,36 mmol), gelöst in heißem Ethanol (5 ml), versetzt. Die klare farblose Lösung wurde 24 h gerührt und anschließend auf ca. die Hälfte eingeengt. Nach weiteren 2 h Rühren bei RT konnte der ausgefallene Niederschlag abgesaugt werden. Beispiel 74 wurde in einer Ausbeute von 76 % (151 mg) mit einem Schmelzpunkt von 158-165 °C gewonnen

**Beispiel 75: 1-Butyl-N,N-dimethyl-4-(3-methyt-5-(triftuormethyl)-1H-indol-2-yl)cyclohexanamin, Citrat (1:1), polares Diastereomer**

**1-Butyl-N,N-dimethyl-4-(3-nethyl-5-(trifluormethyl)-1H-indol-2-yl)cyclohexanamin (unpolares und polares Diastereomer)**

Bsp. 24 (110 mg, 0,29 mmol) wurde unter Argonatmosphäre in Methanol (25 ml) gelöst und mit Pd/C (5 %, 50 mg) versetzt. Bei 3 bar wurde 3h bei RT hydriert. Anschließend wurde der Katalysator über Celite abgesaugt und das Filtrat eingeengt. Der feste, farblose Rückstand wurde chromatographisch getrennt [Kieselgel 60 (20 g); Ethylacetat/Methanol (4 : 1; 500 ml); (1 : 1; 300 ml)]. 1-Butyl-N,N-dimethyl-4-(3-methyl-5-(trifluormethyl)-1H-indol-2-yl)cyclohexanamin **(polares Diastereomer)** wurde in einer Ausbeute von 83 mg (75 %) als farbloser Feststoff mit einem Schmelzpunkt von 141-146 **°C** erhalten.
Das unpolarere Diastereoisomer wurde in einer Ausbeute von 10 mg (9 %) als farbloses Öl isoliert.

**1-Butyl-N,N-dimethyl-4-(3-methyl-5-(trifluormethyl)-1H-indol-2-yl)cyclohexanamin, Citrat (1:1), polares Diastereomer**

Das polarere (1r,4s)-1-butyl-N,N-dimethyl-4-(3-methyl-5-(trifluormethyl)-1H-indol-2-yl)cyclohexanamin (82 mg, 0,21 mmol) wurde in Ethanol (10 ml) gelöst und mit Citronensäure (44 mg, 0,23 mmol), gelöst in heißem Ethanol (2 ml), versetzt. Die klare farblose Lösung wurde 24 h gerührt und anschließend eingeengt. Nach Zugabe von Diethylether (10 ml) wurde 2 h bei RT gerührt und dann der Niederschlag abgesaugt. **Beispiel 75** wurde in einer Ausbeute von 78 % (94 mg) mit einem Schmelzpunkt von 191-193 °C gewonnen

**Beispiel 76: N,N-Dimethyl-4-(3-methyl-5-(trifluormethyl)-1H-indol-2-yl)-1-phenylcyclohexanamin, Citrat (1:1), polares Diasteromer**

**N,N-Dimethyl-4-(3-methyl-5-(trifluormethyl)-1H-indol-2-yl)-1-phenylcyclohexanamin (unpolares und polares Diastereomer)**

Bsp. 25 (420 mg, 1,05 mmol) wurde mit HBr/Eisessig (33 % HBr, 22 ml) versetzt. Die Suspension wurde etwa 10 min bis zur vollständigen Lösung gerührt. Anschließend wurde zu dem Ansatz bei RT Sn-Pulver (1,22 g, 10,5 mmol) innerhalb von 40 min portionsweise gegeben. Nach Beendigung der Zugabe wurde das Reaktionsgemisch weitere 1,5 h gerührt und zur Aufarbeitung anschließend am Rotationsverdampfer bis zur Trockne eingeengt. Der verbliebene Rückstand wurde durch Zugabe von 5N NaOH (40 ml) basisch gemacht. Die erhaltene Lösung wurde mit Dichlormethan extrahiert (4 x 30 ml). Die vereinigten organischen Phasen wurden mit $Na_2SO_4$ getrocknet und anschließend eingeengt. Der Rückstand (420 mg) wurde säulenchromatographisch [Kieselgel 60 (30 g); Ethylacetat/Cyclohexan (1 : 1; 500 ml); Ethylacetat (500 ml); Ethylacetat/Methanol (2 : 1; 450 ml)] getrennt. N,N-Dimethyl-4-(3-methyl-5-(trifluormethyl-1H-indol-2-yl)-1-phenylcyclohexanamin (polares Diastereomer) wurde in einer Ausbeute von 176 mg (42 %) als farbloser Feststoff mit einem Schmelzpunkt von 244-251 °C erhalten. Das unpolarere Diastereoisomer wurde so in einer Ausbeute von 13 mg (3 %) als farbloser Feststoff mit einem Schmelzpunkt von 204-217 °C erhalten.

**N,N-Dimethyl-4-(3-methyl-5-(trifluormetlhyl)-1H-indol-2-yl)-1-phenylcyclohexanamin, Citrat (1:1), polares Diasteromer**

N,N-Dimethyl-4-(3-methyl-5-(trifluormethyl)-1H-indol-2-yl)-1-phenylcyclohexanamin (polares Diastereomer) (170 mg, 0,42 mmol) wurde in Ethanol (10 ml) gelöst und mit Citronensäure (89 mg, 0,46 mmol), gelöst in heißem Ethanol (2 ml), versetzt. Nach 30 min Rühren bei RT begann ein farbloser Feststoff auszufallen. Nach 1 h wurde abgesaugt. Beispiel 76 wurde in einer Ausbeute von 76 % (188 mg) mit einem Schmelzpunkt von 243-247 °C gewonnen

**Beispiel 77: 1-Benzyl-4-(5-fluor-3-methyl-1H-indol-2-yl)-N,N-dimethylcyclohexanamin, Citrat (2:1), unpolares Diastereomer**

**Beispiel 78: 1-Benzyl-4-(5-fluor-3-methyl-1H-indol-2-yl)-N,N-dimethylcyclohexanamin, Citrat (1:1), polares Diastereomer**

**1-Benzyl-4-(5-fluor-3-methyl-1H-indol-2-yl)-N,N-dimethylcyclohexanamin (unpolares und polares Diastereomer)**

**Hydrierung mit HBr/Sn**

Bsp. 26 (200 mg, 0,55 mmol) wurde in HBr/Eisessig (33 % HBr, 20 ml) gelöst. Anschließend wurde zu dem Ansatz bei RT Sn-Pulver (700 mg, 5,9 mmol) innerhalb von 60 min portionsweise gegeben. Nach Beendigung der Zugabe wurde das Reaktionsgemisch weitere 60 min gerührt. - Zur Aufarbeitung wurde das Gemisch mit Ethanol (20 ml) versetzt und das Lösungsmittelgemisch am Rotationsverdampfer bis zur Trockene eingeengt. Der verbliebene Rückstand wurde durch Zugabe von 5N NaOH (40 ml) basisch gestellt. Die erhalte Lösung wurde mit Dichlormethan versetzt und extrahiert (4 x 20 ml). Die vereinigten organischen Phasen wurden mit $MgSO_4$ getrocknet und anschließend eingeengt. Der erhaltene Rückstand (200 mg) wurde säulenchromatographisch (Laufmittel: unpolares Isomer EtOAc, polares Isomer EtOAc/EtOH 2 : 1)) gereinigt. 1-Benzyl-4-(5-fluor-3-methyl-1H-indol-2-yl)-N,N-dimethylcyclohexanamin (unpolareres Diastereoisomer) wurde so in einer Ausbeute von 87 mg (43 %) erhalten. Das polarere Diastereoisomer fiel in einer Ausbeute von 35 mg (17 %) an.

**Hydrierung mit $H_2$/Pd**

Bsp. 26 (137 mg, 0,38 mmol) wurde in Ethanol (30 ml) gelöst, mit dem Katalysator (Pd/Kohle 5%, 100 mg) versetzt und 2 h bei RT und 3 bar Wasserstoffdruck hydriert. Der nach Abtrennung des Katalysators und Entfernung des Lösungsmittels erhaltene feste Rückstand (128 mg) wurde säulenchromatographisch (Laufmittel: unpolares Isomer EtOAc, polares Isomer EtOAc/EtOH 2 : 1) gereinigt. Das unpolarere Diastereoisomer wurde so in einer Ausbeute von 40 mg (29 %) erhalten, das polarere Diastereoisomer fiel in einer Ausbeute von 55 mg (40 %) an.

**1-Benzyl-4-(5-fluor-3-methyl-1H-indof-2-yl)-N,N-dimethylcyclohexanamin, Citrat (2:1), unpolares Diastereomer**

1-Benzyl-4-(5-fluor-3-methyl-1H-indol-2-yl)-N,N-dimethylcyclohexanamin (unpolares Isomer, 72 mg, 0,2 mmol)

wurde in der Siedehitze in Isopropanol (5 ml) gelöst und mit Citronensäure (40 mg, 0,21 mmol), gelöst in heißem Isopropanol (1 ml), versetzt. Die Lösung wurde auf 5 °C abgekühlt (Kühlschrank) und 17 h stehen gelassen. Der entstandene Niederschlag wurde mittels einer Fritte abgetrennt. Beispiel 77 wurde so in einer Ausbeute von 87 mg (94 %, *Schmelzpunkt:* 228-233 °C, ab 140 **°C** Kristallumwandlung) erhalten.

**1-Benzyl-4-(5-fluor-3-methyl-1H-indol-2-yl)-N,N-dimethylcyclohexanamin, Citrat (1:1), polares Diastereomer**

(1s,4s)-1-benzyl-4-(5-fluor-3-methyl-1H-indol-2-yl)-N,N-dimethylcyclohexanamin (polares Isomer, 80 mg, 0,22 mmol) wurde in der Siedehitze in Isopropanol (3 ml) gelöst und mit Citronensäure (60 mg, 0,31 mmol), gelöst in heißem Isopropanol (2 ml), versetzt. Die Lösung wurde auf 5 °C abgekühlt (Kühlschrank) und 17 h stehen gelassen. Der entstandene Niederschlag wurde mittels einer Fritte abgetrennt. Beispiel 78 wurde so in einer Ausbeute von 84 mg (68 %, *Schmelzpunkt:* 183-184 °C) erhalten.

**Beispiel 79: 1-Butyl-4-(5-fluor-3-methyl-1H-indol-2-yl)-N,N-dimethylcyclohexanamin Hydrochlorid**

**1-Butyl-4-(5-fluor-3-methyl-1H-indol-2-yl)-N,N-dimethylcyclohexanamin (1 Diastereomer)**

5-Fluor-3-methylindol (Ind-8) (596 mg, 4 mmol) wurde zusammen mit Keton Ket-4 (788 mg, 4 mmol) in Dichlormethan (30 ml) gelöst und mit Trifluormethansulfonsäure (500 µl, 5,63 mmol) versetzt. Der Ansatz wurde 24 h bei RT gerührt. Anschließend wurde zu dem Ansatz Triethylsilan (2 ml, 12,4 mmol) gegeben. Das Reaktionsgemisch wurde 60 h bei RT gerührt. Zur Aufarbeitung wurde das Reaktionsgemisch mit 2N NaOH (30 ml) versetzt und 20 min bei RT gerührt. Nach Abtrennung der organischen Phase wurde die wässrige Phase mit Dichlormethan (3 x 15 ml) extrahiert. Die vereinigten organischen Extrakte wurden über $Na_2SO_4$ getrocknet und anschließend eingeengt. Das erhaltene Rohprodukt (1,4 g) wurde durch Säulenchromatographie [Kieselgel 60 (50 g); Ethylacetat (500 ml)] gereinigt. 1-Butyl-4-(5-fluor-3-methyl-1H-indol-2-yl)-N,N-dimethylcyclohexanamin wurde in einer Ausbeute von 266 mg (22 %) als weißer Feststoff erhalten. Es wurde nur eins von zwei möglichen Diastereoisomeren erhalten.

**1-Butyl-4-(5-fluor-3-methyl-1H-indol-2-yl)-N,N-dimethylcyclohexanamin Hydrochlorid**

1-Butyl-4-(5-fluor-3-methyl-1H-indol-2-yl)-N,N-dimethylcyclohexanamin (266 mg, 0,8 mmol) wurde in Ethylmethylketon (30 ml) gelöst. Bei RT wurde dann $Me_3SiCl$ (205 µl, 1,6 mmol) zugetropft und 1 h gerührt. Es fiel ein weißer Niederschlag aus. Der Niederschlag wurde abgesaugt, mit Ethylmethylketon (2 × 5 ml) gewaschen und anschließend getrocknet.

**Beispiel 79** (193 mg, Fp. 255-265 °C, 61 %) war ein weißer Feststoff

**Beispiel 80: 4-(5-Fluor-3-methyl-1H-indol-2-yl)-N,N-dimethyl-1-phenylcyclohexanamin, Citrat (2:1), unpolares Diastereomer**

**Beispiel 81: 4-(5-Fluor-3-methyl-1H-indol-2-yl)-N,N-dimethyl-1-phenylcyclohexanamin, Citrat (1:1), polares Diastereomer**

**4-(5-Fluor-3-methyl-1H-indol-2-yl)-N,N-dimethyl-1-phenylcyclohexanamin (unpolares und polares Diastereomer)**

(±)-2-(4-(Dimethylamino)-4-phenylcyclohex-1-enyl)-3-methyl-5-fluor-1H-indol (freie Base aus **Beispiel 28**) (420 mg, 1,2 mmol) wurde in HBr/Eisessig (33 % HBr, 25 ml) gelöst. Anschließend wurde zu dem Ansatz bei RT Sn-Pulver (1,4 g, 12 mmol) innerhalb von 40 min portionsweise gegeben. Nach Beendigung der Zugabe wurde das Reaktionsgemisch weitere 60 min gerührt. - Zur Aufarbeitung wurde das Gemisch am Rotationsverdampfer bis zur Trockene eingeengt. Der verbliebene Rückstand wurde durch Zugabe von 5N NaOH (40 ml) basisch gestellt. Die erhalte Lösung wurde mit Dichlormethan versetzt und extrahiert (4 x 20 ml). Die vereinigten organischen Phasen wurden mit $MgSO_4$ getrocknet und anschließend eingeengt. Der erhaltene Rückstand (360 mg) wurde in Ethanol (30 ml) aufgekocht, wobei die Substanz nur teilweise in Lösung ging. Die Mischung wurde 1 h bei 5 °C belassen, anschließend wurde der Feststoff abgetrennt. 4-(5-Fluor-3-methyl-1H-indol-2-yl)-N,N-dimethyl-1-phenylcyclohexanamin (unpolareres Isomer) wurde so in einer Ausbeute von 256 mg (60 %, *Schmelzpunkt:* 199-205 °C) erhalten.

Die ethanolische Mutterlauge wurde eingeengt. Der verbliebene Rückstand (162 mg) wurde säulenchromatographisch (Laufmittel: EtOAc/EtOH 2 : 1)) gereinigt. 4-(5-Fluor-3-methyl-1H-indol-2-yl)-N,N-dimethyl-1-phenylcyclohexanamin (polareres Diastereoisomer) wurde so in einer Ausbeute von 150 mg (35 %) erhalten.

**4-(5-Fluor-3-methyl-1H-indol-2-yl)-N,N-dimethyl-1-phenylcyclohexanamin, Citrat (2:1), unpolares Diastereomer**

4-(5-Fluor-3-methyl-1H-indol-2-yl)-N,N-dimethyl-1-phenylcyclohexanamin (unpolareres Isomer) (250 mg, 0,71 mmol) wurde in der Siedehitze in Isopropanol (300 ml) gelöst und mit Citronensäure (138 mg, 0,72 mmol), gelöst in heißem Isopropanol (5 ml), versetzt. Die Lösung wurde auf ca. 130 ml eingeengt, auf 5 °C abgekühlt (Kühlschrank) und 17 h stehen gelassen. Der entstandene Niederschlag wurde mittels einer Fritte abgetrennt. Beispiel 80 wurde so in einer Ausbeute von 218 mg (68 %, *Schmelzpunkt:* 224-229 °C, ab 205 Kristallumwandlung) erhalten.

**4-(5-Fluor-3-methyl-1H-indol-2-yl)-N,N-dimethyl-1-phenylcyclohexanamin, Citrat (1:1), polares Diastereomer**

4-(5-Fluor-3-methyl-1H-indol-2-yl)-N,N-dimethyl-1-phenylcyclohexanamin (polareres Diastereoisomer, 150 mg, 0,43 mmol) wurde in der Siedehitze in Methanol (5 ml) gelöst und mit Citronensäure (84 mg, 0,44 mmol), gelöst in heißem Methanol (2 ml), versetzt. Die Lösung wurde eingeengt. Der erhaltene Rückstand wurde mit Isopropanol (10 ml) erhitzt, wobei die Substanz weitgehend ungelöst blieb. Das Gemisch wurde 1 h bei 5 °C belassen. Der Feststoff wurde anschließend mittels einer Fritte abgetrennt. Beispiel 81 wurde so in einer Ausbeute von 131 mg (56 %, *Schmelzpunkt:* 190-194 °C) erhalten.

**Beispiel 82: (±)-2-(4-(Dimethylamino)-4-butylcyclohex-1-enyl)-3-(2-(1H-benzo[d]imidazol-1-yl)ethyl)-5-fluor-1H-indol**

1-[2-(5-Fluor-1*H* indol-3-yl)-ethyl]-1*H*-benzimidazol (Ind-19, 977 mg, 3.5 mmol) und Keton (Ket-4, 690 mg, 3.5 mmol) wurden in abs. Dichlormethan (30 ml) gelöst und schnell mit Trifluormethansulfonsäure (0.93 ml, 10.5 mmol) versetzt. Der Ansatz wurde 48 h bei RT gerührt, dabei setzte sich ein dunkelbraunes Öl ab. Nach Zugabe von 1 N NaOH (20 ml) und $CH_2Cl_2$(20ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit EE/EtOH (1:4) gereinigt.
*Ausbeute:* 522 mg (33 %), poröser Feststoff
$^1$*H-NMR (DMSO-$d_6$):* 0.88 (3 H, t); 1.23 (10 H, m); 2.11 (3 H, bs); 2.22 (6 H, bs); 3.22 (2 H, t); 4.43 (2 H, t); 5.62 (1 H, s): 6.86 (1 H, m); 7.20 (4 H, m); 7.41 (1 H, d); 7.61 (1 H, d); 7.86 (1 H, s); 10.83 (1 H, s).

**4-(3-(2-(1H-Benzo[d]imidazol-1-yl)ethyl)-5-fluor-1H-indol-2-yl)-1-butyl-N,N-dimethylcyclohexanamin, Citrat (1: 1) : Polareres Diastereomer**

Zu einer Lösung von des soeben hergestellten Olefins (450 mg, 0.98 mmol) in HBr/Eisessig (35 ml) wurde innerhalb von 20 min Zinn (1.25 g) gegeben und 4 h bei RT gerührt. Der Ansatz wurde mit Ethanol versetzt, das Reaktionsgemisch bis zur Trockene eingeengt und der Rückstand in 5N NaOH (50 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit EE/EtOH (1:4→EtOH) getrennt.

*Ausbeute:* 304 mg (67 %), unpolares Diastereomer
119 mg (26 %), polares Diastereomer

Die polare Verbindung (107 mg, 0.232 mmol) wurde in heißem Ethanol (4 ml) gelöst und mit einer Lösung von Citronensäure (45 mg, 0.232 mmol) in heißem Ethanol (3 ml) versetzt. Nach Versetzen mit Ether und 2-stündigem Stehen im Kühlschrank wurde der entstandene Feststoff abgesaugt und i. Vak. getrocknet.
*Ausbeute:* 115 mg (Beispiel 82; 76 %), poröser Feststoff
$^1$*H-NMR (DMSO-$d_6$):* 0.92 (3 H, t); 1.07 (2 H, m); 1.23 (4 H, m); 1.34 (2 H, m); 1.48 (2 H, m); 1.62 (2 H, m); 1.80 (2 H, m); 2.08 (1 H, m); 2.57 (10 H, m); 3.17 (2 H, t); (4.46 (2 H, t); 6.85 (1 H, m); 7.24 (4 H, m); 7.52 (1 H, d); 7.63 (1 H, d); 7.79 (1 H, s); 10.77 (1 H, s), Citrat.

**Beispiel 83: 4-(3-(2-(1H-Benzo[d]imidazol-1-yl)ethyl)-5-fluor-1H-indol-2-yl)-N,N-dimethyl-1-phenylcyclohexanamin, Citrat (1:1)**

**: Unpolareres Diastereomer**

Olefin **Beispiel 54** (836 mg, 1.74 mmol) in HBr/Eisessig (70 ml) wurde innerhalb von 20 min Zinn (2.20 g)

gegeben und 4 h bei RT gerührt. Der Ansatz wurde mit Ethanol versetzt, das Reaktionsgemisch bis zur Trockene eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit EE/EtOH (9:1→1:4) getrennt.

*Ausbeute:* 244 mg (29 %), unpolares Diastereomer
367 mg (44 %), polares Diastereomer

Die soeben erhaltene unpolarere Verbindung (225 mg, 0.468 mmol) wurde in heißem Ethanol (8 ml) gelöst und mit einer Lösung von Citronensäure (90 mg, 0.468 mmol) in heißem Ethanol (5 ml) versetzt. Nach 2-stündigem Stehen im Kühlschrank wurde der entstandene Feststoff abgesaugt und i. Vak. getrocknet.
*Ausbeute:* 206 mg (83,65 %)
*Schmelzpunkt:* 115-125 °C
$^1$*H-NMR (DMSO-*$d_6$*):* 1.17 (2 H, m); 1.45 (2 H, m); 2.10 (6 H, s); 2.28 (1 H, t); 2.64 (6 H, m); 3.19 (2 H, t); 4.44 (2 H, t); 6.84 (1 H, m); 7.11 (2 H, m); 7.26 (8 H, m); 7.48 (1 H, m); 7.85 (1 H, s); 10.67 (1 H, s), Citrat.

**Beispiel 84: 4-(3-(2-(1H-Benzo[d]imidazol-1-yl)ethyl)-5-fluor-1H-indol-2-yl)-N,N-dimethyl-1-phenylcyclohexanamin, Citrat (1:1) : Polareres Diastereomer**

Die unter **Beispiel 83** erhaltene polarere Verbindung (346 mg, 0.72 mmol) wurde in heißem Ethanol (10 ml) gelöst und mit einer Lösung von Citronensäure (138 mg, 0.72 mmol) in heißem Ethanol (6 ml) versetzt. Nach 2-stündigem Stehen im Kühlschrank wurde der entstandene Feststoff abgesaugt und i. Vak. getrocknet.
*Ausbeute:* 348 mg (72 %), poröser Feststoff
$^1$*H-NMR (DMSO-*$d_6$*):* 1.21 (2 H, m); 1.45 (2 H, m); 2.27 (6 H, s); 2.70 (4 H, m); 3.13 (2 H, t); 4.43 (2 H, t); 6.78 (1 H, m); 7.08 (1 H, m); 7.24 (3 H, m); 7.46 (5 H, d); 7.66 (1 H, d); 7.82 (1 H, s); 10.41 (1 H, s), Citrat.

**Beispiel 85: (±)-2-(4-(Dimethylamino)-4-benzylcyclohex-1-enyl)-3-(2-(pyrrolidin-1-yl)ethyl)-5-fluor-1H-indol**

Indol (Ind-36, 700 mg, 3.0 mmol) und Keton (Ket-3, 697 mg, 3.0 mmol) wurden bei RT in abs. $CH_2Cl_2$ (30 ml) gelöst und zügig mit Trifluormethansulfonsäure (1.36 g, 0.80 ml, 9.0 mmol) versetzt. Anschließend fiel ein schwarzes Öl aus. Die Mischung wurde 2 d bei RT gerührt. 1N NaOH (30 ml) wurde zugegeben und das Gemisch 20 min nachgerührt. Die organische Phase wurde abgetrennt und die wässrige Phase mit $CH_2Cl_2$ (2 x 30 ml) extrahiert. Die vereinigten organischen Phasen wurden mit Wasser (15 ml) gewaschen, über $Na_2SO_4$, getrocknet, filtriert und i. Vak. eingeengt. Durch Flash-Chromatographie mit 50 g Kieselgel und Acetonitril/Methanol/wässrige 1 N Ammoniumchlorid-Lösung (9: 1:1) wurde als erste Fraktion das Indol und als zweite Fraktion das gereinigte Produkt erhalten. Die zweite Fraktion wurde i. Vak. eingeengt, mit 2N NaOH basisch gestellt, zweimal mit $CH_2Cl_2$ extrahiert, über $Na_2SO_4$ getrocknet und i. Vak. eingeengt.
*Ausbeute:* 672 mg (50 %), farbloser Feststoff
$^1$*H-NMR (DMSO-*$d_6$*):* 1.40 (2 H; m); 1.67 (4 H, m); 2.01 (4 H, m); 2.31 (6 H, m); 2.45 (5 H, m); 2.75 (4 H, m); 5.89 (1 H, bs); 6.83 (1 H, m); 7.20 (7 H, m); 10.76 (1 H, s).

**1-Benzyl-4-(5-fluor-3-(2-(pyrrolidin-1-yl)ethyl)-1H-indol-2-yl)-N,N-dimethylcyclohexanamin, Citrat (1:1) : Unpolareres Diastereomer**

Das soeben erhaltene Olefin (650 mg, 1.46 mmol) wurde in HBr/Eisessig (33%-ig, 30 ml) gelöst und bei RT innerhalb von 30 min portionsweise mit Zinn (1.90 g) versetzt. Das Gemisch wurde 4 h bei RT gerührt. Ethanol wurde zugegeben, das Lösungsmittelgemisch i. Vak. entfernt und der Rückstand mit 5N NaOH (60 ml) und $CH_2Cl_2$ (80 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet, filtriert und i. Vak. eingeengt. Der verbliebene Rückstand wurde durch Flash-Chromatographie mit Chloroform/Methanol (20:1→4:1 jeweils + 1 % Triethylamin) gereinigt.

*Ausbeute:* 351 mg (54 %), unpolare Verbindung, enthielt Triethylamin
108 mg (17 %), polare Verbindung, enthielt Triethylamin

Die soeben hergestellte unpolarere Verbindung (274 mg, 0.61 mmol) wurde in Ethanol (4 ml) heiß gelöst und

mit einer Lösung von Citronensäure (118 mg, 0.61 mmol) in Ethanol (2 ml) versetzt. Nach 2 Stunden bei RT wurde der entstandene Niederschlag abgesaugt und mit Ether nachgewaschen.
*Ausbeute:* 339 mg ( 85, 87 %)
*Schmelzpunkt:* 200-202 °C
$^1$*H-NMR (DMSO-$d_6$):* 1.17 (2 H, m); 1.43 (2 H, m); 1.67 (2 H, m); 1.96 (8 H; m); 2.38 (6 H, s); 2.59 (4 H, m); 2.86 (2 H, m); 3.11 (2 H, t); 6.81 (1 H, m); 7.24 (7 H, m); 10.79 (1 H, bs);11.1 (1 H, bs), Citrat.

**Beispiel 86: 1-Benzyl-4-(5-fluor-3-(2-(pyrrolidin-1-yl)ethyl)-1H-indol-2-yl)-N,N-dimethylcyclohexanamin, Citrat (1:1) : Polareres Diastereomer**

Die unter Beispiel 85 erhaltene polarere Verbindung (94 mg, 0.21 mmol) wurde in Ethanol (2 ml) heiß gelöst und mit einer Lösung von Citronensäure (40 mg, 0.21 mmol) in Ethanol (1 ml) versetzt. Nach 2 Stunden bei RT wurde der entstandene Niederschlag abgesaugt und mit Ether nachgewaschen.
*Ausbeute:* 76 mg (86,57 %); *Schmelzpunkt:* amorpher Feststoff
$^1$*H-NMR (DMSO-$d_6$):* 1.73 (4 H, m); 1.91 (3 H, m); 2.02 (2 H, m); 2.36 (6 H, s); 2.57 (4 H, m); 2.85-3.19 (7 H, m); 6.85 (1 H, m); 7.29 (7 H, m); 11.0 (1 H, s); 11.2 (2 H, bs), Citrat.

**Beispiel 87: (±)-2-(4-(Dimethylamino)-4-butylcyclohex-1-enyl)-3-(2-(pyrrolidin-1-yl)ethyl)-5-fluor-1H-indol**

Indol (Ind-36, 1.00 g, 4.3 mmol) und Keton (Ket-4, 849 mg, 4.3 mmol) wurden bei RT in abs. $CH_2Cl_2$ (50 ml) gelöst und zügig mit Trifluormethansulfonsäure (1.93 g, 1.15 ml, 12.9 mmol) versetzt. Anschließend fiel ein schwarzes Öl aus. Die Mischung wurde 3 d bei RT gerührt. 1N NaOH (50 ml) wurde zugegeben und das Gemisch 20 min nachgerührt. Die organische Phase wurde abgetrennt und die wässrige Phase mit $CH_2Cl_2$ (2 x 50 ml) extrahiert. Die vereinigten organischen Phasen wurden mit Wasser (20 ml) gewaschen, über $Na_2SO_4$ getrocknet, filtriert und i. Vak. eingeengt. Durch Flash-Chromatographie mit 50 g Kieselgel und Acetonitril/Methanol/wässrige 1 N Ammoniumchlorid-Lösung (9: 1: 1) wurde als erste Fraktion das Indol und als zweite Fraktion das gereinigte Produkt erhalten. Die zweite Fraktion wurde i. Vak. eingeengt, mit 2N NaOH basisch gestellt, zweimal mit $CH_2Cl_2$ extrahiert, über $Na_2SO_4$ getrocknet und i. Vak. eingeengt.
*Ausbeute:* 668 mg (37 %), farbloser Feststoff
$^1$*H-NMR (DMSO-$d_6$):* 0.90 (3 H; t); 1.24-1.60 (6 H, m); 1.91 (4 H, m); 2.11 (2 H, m); 2.38 (6 H, s); 2.57 (4 H, m); 5.87 (1 H, bs); 6.83 (1 H, m); 7.22 (2 H, m); 10.82 (1 H, s).

**1-Butyl-4-(5-fluor-3-(2-(pyrrolidin-1-yl)ethyl)-1H-indol-2-yl)-N,N-dimethylcyclohexanamin, Citrat (1:1) ( 87; : Unpolareres Diastereomer)**

Das soeben hergestellte Olefin (640 mg, 1.55 mmol) wurde in HBr/Eisessig (33%-ig. 35 ml) gelöst und bei RT innerhalb von 30 min portionsweise mit Zinn (2.02 g) versetzt. Das Gemisch wurde 4 h bei RT gerührt. Ethanol wurde zugegeben, das Lösungsmittelgemisch i. Vak. entfernt und der Rückstand mit 5N NaOH (60 ml) und $CH_2Cl_2$ (80 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet, filtriert und i. Vak. eingeengt. Der verbliebene Rückstand wurde durch Flash-Chromatographie mit Chloroform/Methanol (20: 1→4:1 jeweils + 1 % Triethylamin) gereinigt.

*Ausbeute:* 285 mg (44 %), unpolare Verbindung, enthielt Triethylamin
174 mg (27 %), polare Verbindung, enthielt Triethylamin

Die soeben erhaltene unpolarere Verbindung (261 mg, 0.63 mmol) wurde in Ethanol (ml) heiß gelöst und mit einer Lösung von Citronensäure (121 mg, 0.63 mmol) in Ethanol (ml) versetzt. Nach 2 Stunden bei RT wurde der entstandene Niederschlag abgesaugt und mit Ether nachgewaschen.
*Ausbeute:* 242 mg (63 %); *Schmelzpunkt:* 168-186 °C
$^1$*H-NMR (DMSO-$d_6$):* 0.94 (3 H, t); 1.25-1.55 (10 H, m); 1.98 (6 H, m); 2.38 (6 H, s); 2.57 (4 H, m); 2.97 (1 H, m); 3.09 (2 H, t); 3.13 (2 H, t); 6.82 (1 H, m); 7.30 (2 H, m); 10.89 (1 H, s);11.1 (1 H, bs), Citrat.

**Beispiel 88: 1-Butyl-4-(5-fluor-3-(2-pyrrolidin-1-yl)ethyl)-1H-indol-2-yl)-N,N-dimethylcyclohexanamin, Citrat (1: 1) ( 88; : Polareres Diastereomer)**

Die unter Beispiel 87 erhaltene polarere Verbindung (176 mg, 0.43 mmol) wurde in Ethanol ( ml) heiß gelöst und mit einer Lösung von Citronensäure (82 mg, 0.43 mmol) in Ethanol (ml) versetzt. Nach 2 Stunden bei RT wurde

der entstandene Niederschlag abgesaugt und mit Ether nachgewaschen.
*Ausbeute:* 138 mg ( 88, 54 %); amorpher Feststoff
*$^1$H-NMR (DMSO-$d_6$):* 1.04 (3 H, t); 1.40 (4 H, m); 1.76 (12 H, m); 2.02 (2 H, m); 2.47 (6 H, s); 2.59 (4 H, m); 2.88-3.17 (5 H, m); 6.85 (1 H, m); 7.28 (2 H, m); 10.9 (1 H, s); 11.1 (2 H, bs), Citrat.

**Beispiel 89: (±)-2-(4-(Dimethylamino)-4-phenylcyclohex-1-enyl)-3-(2-(pyrrolidin-1-yl)ethyl)-5-fluor-1H-indol**

Indol (Ind-36, 600 mg, 2.60 mmol) und Keton (Ket-10, 561 mg, 2.60 mmol) wurden bei RT in abs. $CH_2Cl_2$ (30 ml) gelöst und zügig mit Trifluormethansulfonsäure (1.16 g, 0.69 ml, 7.7 mmol) versetzt. Anschließend fiel ein schwarzes Öl aus der Lösung aus. Die Mischung wurde 24 h bei RT gerührt. 1 N NaOH (30 ml) wurde zugegeben und das Gemisch 20 min nachgerührt. Die organische Phase wurde abgetrennt und die wässrige Phase mit $CH_2Cl_2$ (2 × 30 ml) extrahiert. Die vereinigten organischen Phasen wurden mit Wasser (15 ml) gewaschen, über $Na_2SO_4$ getrocknet, filtriert und i. Vak. eingeengt. Flash-Chromatographie des Rückstandes mit 50 g Kieselgel und Chloroform/Methanol (9:1 + 1 % Triethylamin) ergab ein Gemisch aus Indol und Produkt. Durch erneute Flash-Chromatographie der i. Vak. eingeengten Mischfraktionen mit 50 g Kieselgel und Acetonitril/Methanol/wässrige 1 N Ammoniumchlorid-Lösung (9:1:1) wurde als erste Fraktion Indol und als zweite Fraktion das gereinigte Produkt erhalten. Die zweite Fraktion wurde i. Vak. eingeengt, mit 2N NaOH basisch gestellt, zweimal mit $CH_2Cl_2$ extrahiert, über $Na_2SO_4$ getrocknet und i. Vak. eingeengt.
*Ausbeute:* 621 mg (55 %), farbloser Feststoff
*$^1$H-NMR (DMSO-$d_6$):* 1.66 (6 H, m); 2.09 (8 H, m); 2.40 (6 H, m); 2.61 (4 H, m); 6.20 (1 H, bs); 6.82 (1 H, m); 7.21 (5 H, m); 7.45 (2 H, m); 10.73 (1 H, s).

**4-(5-Fluor-3-(2-(pyrrolidin-1-yl)ethyl)-1H-indol-2-yl)-N,N-dimethyl-1-phenylcyclohexanamin, Citrat (1: 1) : Unpolareres Diastereomer**

Das soeben hergestellte Olefin (600 mg, 1.40 mmol) wurde in HBr/Eisessig (33%-ig, 30 ml) bei RT innerhalb von 30 min portionsweise mit Zinn (1.82 g) versetzt. Das Gemisch wurde 4 h bei RT gerührt. Ethanol wurde zugegeben, das Lösungsmittelgemisch i. Vak. entfernt und der Rückstand mit 5N NaOH (60 ml) und $CH_2Cl_2$ (80 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet, filtriert und i. Vak. eingeengt. Der verbliebene . Rückstand wurde durch Flash-Chromatographie mit Chloroform/Methanol (20:1→4:1 + 1 % Triethylamin) gereinigt.

| | |
|---|---|
| *Ausbeute:* | 352 mg (58 %), unpolare Verbindung, enthielt Triethylamin |
| | 213 mg (35 %), polare Verbindung, enthielt Triethylamin |

Die soeben erhaltene unpolarere Verbindung (295 mg, 0.68 mmol) wurde in Ethanol (7 ml) heiß gelöst und mit einer Lösung von Citronensäure (131 mg, 0.68 mmol) in Ethanol (2 ml) versetzt. Nach 2 Stunden bei RT wurde der entstandene Niederschlag abgesaugt und mit Ether nachgewaschen.
*Ausbeute:* 193 mg (45 %); *Schmelzpunkt:* 219-222 °C
*$^1$H-NMR (DMSO-$d_6$):* 1.55 (2 H, m); 1.67 (2 H, m); 1.93 (4 H; bs); 2.06 (6 H, s); 2.14 (2 H, m); 2.57 (2 H, m); 2.86 (2 H, d); 3.03 (4 H, m); 3.20 (2 H, t); 6.84 (1 H, m); 7.32 (7 H, m); 10.94 (1 H, bs);11.1 (1 H, bs) Citrat.

**Beispiel 90: 4-(5-Fluor-3-(2-(pyrrolidin-1-yl)ethyl)-1H-indol-2-yl)-N,N-dimethyl-1-phenylcyclohexanamin, Citrat (1:1) : Polareres Diastereomer**

Die unter Beispiel 89 erhaltene polare Verbindung (166 mg, 0.38 mmol) wurde in Ethanol (2 ml) heiß gelöst und mit einer Lösung von Citronensäure (74 mg, 0.38 mmol) in Ethanol (1 ml) versetzt. Nach 2 Stunden bei RT wurde der entstandene Niederschlag abgesaugt und mit Ether nachgewaschen.
*Ausbeute:* 109 mg ( 90, 46 %); *Schmelzpunkt:* 196-198 °C
*$^1$H-NMR (DMSO-$d_6$):* 1.41 (2 H, m); 1.82 (2 H, m); 1.96 (4 H, m); 2.10 (2 H; s); 2.35 (6 H, s); 2.61 (4 H, m); 3.08 (6 H, t); 3.18 (2 H, m); 6.77 (1 H, m); 7.13 (1 H, m); 7.32 (1 H, m); 7.55 (3 H, m); 7.67 (2 H, m); 10.6 (1 H, s), Citrat.

**Beispiel 91: 1-Benzyl-4-(5-fluor-3-(2-(piperidin-1-yl)ethyl)-1H-indol-2-yl)-N,N-dimethylcyclohexanamin, Citrat (1: 1) : Unpolareres Diastereomer**

Olefin Beispiel 55 (691 mg, 1.5 mmol) wurde in HBr/Eisessig (33%-ig, 30 ml) gelöst und bei RT innerhalb von 30 min portionsweise mit Zinn (1.96 g) versetzt. Das Gemisch wurde 4 h bei RT gerührt. Ethanol wurde zugegeben, das Lösungsmittelgemisch i. Vak. entfernt und der Rückstand mit 5N NaOH (60 ml) und $CH_2Cl_2$ (80 ml) gelöst. Die Phasen

wurden getrennt, die wässrige Phase zweimal mit $CH_2Cl_2$ extrahiert, die vereinigten organischen Phasen mit Wasser gewaschen, über $Na_2SO_4$ getrocknet, filtriert und i. Vak. eingeengt. Der verbliebene Rückstand wurde durch Flash-Chromatographie mit 50 g Kieselgel und Chloroform/Methanol (50:1→Methanol) gereinigt.
*Ausbeute:* 512 mg (74 %), unpolare Verbindung; 130 mg (19 %), polare Verbindung

Die soeben hergestellte unpolarere Verbindung (420 mg, 0.91 mmol) wurde in Ethanol (4 ml) heiß gelöst und mit einer Lösung von Citronensäure (175 mg, 0.91 mmol) in Ethanol (2 ml) versetzt. Nach 2 Stunden bei RT wurde der entstandene Niederschlag abgesaugt und mit Ethanol nachgewaschen.
*Ausbeute:* 426 mg (72 %); *Schmelzpunkt:* 191-194 **°C**
$^1$*H-NMR (DMSO-$d_6$):* 1.54-1.75 (8 H, m); 2.10 (6 H, s); 2.56-2.65 (4 H, m); 2.90 (4 H, m); 3.06 (4 H, bs); 6.85 (1 H, m); 7.29 (3 H, m); 7.40 (4 H, m); 11.02 (1 H, s), Citrat.

**Beispiel 92: 1-Benzyl-4-(5-fluor-3-(2-(piperidin-1-yl)ethyl)-1H-indol-2-yl)-N,N-dimethylcyclohexanamin, Citrat (2: 3) : Polareres Diastereomer**

Die unter Beispiel 91 hergestellte polarere Verbindung (111 mg, 0.24 mmol) wurde in Ethanol (2 ml) heiß gelöst und mit einer Lösung von Citronensäure (46 mg, 0.24 mmol) in Ethanol (1 ml) versetzt. Ether wurde zugegeben, daraufhin fiel ein Niederschlag aus. Dieser wurde abgesaugt und mit Ether gewaschen.
*Ausbeute:* 80 mg (51 %); *Schmelzpunkt:* amorpher Feststoff
$^1$*H-NMR (DMSO-$d_6$):* 1.50 (4 H, m); 1.72 (8 H, m); 2.13 (4 H, m); 2.46 (2 H, s); 2.90 (6 H, m); 3.02 (3 H, m); 6.77 (1 H, m); 7.14 (2 H, m); 7.23 (5 H, m); 10.61 (H, bs), Citrat.

**Beispiel 93: 1-Butyl-4-(5-fluor-3-(2-(piperidin-1-yl)ethyl)-1H-indol-2-yl)-N,N-dimethylcyclohexanamin, Citrat (4: 3) : Unpolareres Diastereomer**

Olefin **Beispiel 56** (972 mg, 2.30 mmol) wurde in HBr/Eisessig (33%-ig, 50 ml) gelöst und bei RT innerhalb von 30 min portionsweise mit Zinn (2.98 g) versetzt. Das Gemisch wurde 4 h bei RT gerührt. Ethanol wurde zugegeben, das Lösungsmittelgemisch i. Vak. entfernt und der Rückstand mit 5N NaOH (120 ml) und $CH_2Cl_2$ (160 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet, filtriert und i. Vak. eingeengt. Der verbliebene Rückstand wurde durch Flash-Chromatographie mit 100 g Kieselgel und Chloroform/Methanol (50:1→Methanol) gereinigt. Das unpolare Diastereomer war rein, aber das polare Diastereomer war mit Triethylaminhydrochlorid, das vermutlich im verwendeten Kieselgel enthalten war, stark verunreinigt. Das polare Diastereomer wurde in $CHCl_3$ aufgenommen und mit 1N NaOH gewaschen. Die Phasen wurden getrennt und die wässrige Phase wurde mit $CHCl_3$ extrahiert. Die vereinigten organischen Phasen wurden über $Na_2SO_4$ getrocknet, filtriert und i. Vak. eingeengt.

*Ausbeute:* 472 mg (48 %), unpolare Verbindung
45 mg (5 %), polare Verbindung

Die soeben hergestellte unpolare Verbindung (444 mg, 1.04 mmol) wurde in Ethanol (5 ml) heiß gelöst und mit einer Lösung von Citronensäure (200 mg, 1.04 mmol) in Ethanol (2 ml) versetzt. Ether wurde zugegeben, daraufhin fiel ein Niederschlag aus. Dieser wurde abgesaugt und mit Ether gewaschen.
*Ausbeute:* 390 mg (93, 61 %); amorpher Feststoff
$^1$*H-NMR (DMSO-$d_6$):* 0.96 (3 H, t); 1.33 (6 H, m); 1.62 (6 H, m); 1.88 (6 H, m); 2.01 (2 H, m); 2.29 (2 H, m); 2.73 (6 H, s); 3.08 (6 H, m); 6.84 (1 H, m); 7.21 (1 H, m); 7.39 (1 H, m); 11.66 (1 H, s), Citrat.

**Beispiel 94: 1-Butyl-4-(5-fluor-3-(2-(piperidin-1-yl)ethyl)-1H-indol-2-yl)-N,N-dimethylcyclohexanamin, Citrat (1: 1) : Polareres Diastereomer**

Die unter Beispiel 93 hergestellte polarere Verbindung (43 mg, 0.10 mmol) wurde in Ethanol (1 ml) heiß gelöst und mit einer Lösung von Citronensäure (19 mg, 0.10 mmol) in Ethanol (1 ml) versetzt. Es fiel auch nach Zugabe von Ether kaum Niederschlag aus, daraufhin wurde das Lösungsmittelgemisch i. Vak. entfernt.
*Ausbeute:* 45 mg ( 94, 72 %); amorpher Feststoff
$^1$*H-NMR (DMSO-$d_6$):* 0.96 (3 H, t); 1.42 (6 H, m); 1.75-1.93 (14 H, m); 2.53-2.70 (10 H, m); 3.16 oder 3.34 (4 H, m); 6.84 (1 H, m); 7.28 (1 H, m); 7.40 (1 H, m); 11.22 (1 H, s), Citrat (Probe enthielt noch TEA x HCl).

**Beispiel 95: 4-(5-Fluor-3-(2-(piperidin-1-yl)ethyl)-1H-indol-2-yl)-N,N-dimethyl-1-phenylcyclohexanamin, Citrat (1: 1) : Unpolareres Diastereomer**

Olefin **Beispiel 57** (354 mg, 0.8 mmol) wurde in HBr/Eisessig (33%-ig, 15 ml) gelöst und bei RT innerhalb von 30 min portionsweise mit Zinn (1.03 g) versetzt. Das Gemisch wurde 4 h bei RT gerührt. Ethanol wurde zugegeben, das Lösungsmittelgemisch i. Vak. entfernt und der Rückstand mit 5N NaOH (30 ml) und $CH_2Cl_2$ (40 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet, filtriert und i. Vak. eingeengt. Durch Flash-Chromatographie des Rückstandes mit 50 g Kieselgel Chtoroform/Methanol (20:1→Methanol) wurde das unpolare Diastereomer nur stark verunreinigt erhalten, es wurde durch erneute Flash-Chromatographie mit 25 g Kieselgel und Chloroform/Methanol (50: 1) gereinigt. Das polare Diastereomer war rein.

*Ausbeute:* 161 mg (45 %), unpolare Verbindung
109 mg (30 %), polare Verbindung

Die soeben hergestellte unpolarere Verbindung (140 mg, 0.31 mmol) wurde in Ethanol (3 ml) heiß gelöst und mit einer Lösung von Citronensäure (60 mg, 0.31 mmol) in Ethanol (1 ml) versetzt. Nach 2 Stunden bei RT wurde der entstandene Niederschlag abgesaugt und mit Ethanol nachgewaschen.
*Ausbeute:* 127 mg (63 %); *Schmelzpunkt:* 220-223 °C
*$^1$H-NMR (DMSO-$d_6$):* 1.54-1.75 (8 H, m); 2.10 (6 H, s); 2.56-2.65 (4 H, m); 2.90 (4 H, m); 3.06 (4 H, bs); 6.85 (1 H, m); 7.29 (3 H, m); 7.40 (4 H, m); 11.02 (1 H, s), Citrat.

**Beispiel 96: 4-(5-Fluor-3-(2-(piperidin-1-yl)ethyl)-1H-indol-2-yl)-N,N-dimethyl-1-phenylcyclohexanamin, Citrat (1:1): Polareres Diastereomer**

Die unter **Beispiel 95** hergestellte polare Verbindung (93 mg, 0.21 mmol) wurde in Ethanol (2 ml) heiß gelöst und mit einer Lösung von Citronensäure (40 mg, 0.21 mmol) in Ethanol (1 ml) versetzt. Nach 2 Stunden bei RT wurde der entstandene Niederschlag abgesaugt und mit Ethanol nachgewaschen.
*Ausbeute:* 73 mg (55 %); *Schmelzpunkt:* 153-167 °C
*$^1$H-NMR (DMSO-$d_6$):* 1.50 (4 H, m); 1.72 (8 H, m); 2.13 (4 H, m); 2.46 (2 H, s); 2.90 (6 H, m); 3.02 (3 H, m); 6.77 (1 H, m); 7.14 (2 H, m); 7.23 (5 H, m); 10.61 (H, bs), Citrat.

**Beispiel 97: (±)-2-(4-(Dimethylamino)-4-benzylcyclohex-1-enyl)-3-(2-(1H-pyrazol-1-yl)ethyl)-5-fluor-1H-indol**

Indol (Ind-38, 600 mg, 2.62 mmol) und Keton (Ket-3, 605 mg, 2.62 mmol) wurden in abs. Dichlormethan (20 ml) gelöst und schnell mit Trifluormethansulfonsäure (0.70 ml, 7.9 mmol) versetzt. Der Ansatz wurde 48 h bei RT gerührt., dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (20:1) gereinigt.
*Ausbeute:* 259 mg (22 %), zähes Öl
*$^1$H-NMR (DMSO-$d_6$):* 1.96 (2 H, m); 2.32 (6 H, bs); 2.77 (6 H, m); 3.17 (2 H, t); 4.28 (2 H, t); 5.78 (1 H, bs); 6.12 (1 H, s); 6.86 (2 H, m); 7.17 (6 H, d); 7.54 (1 H, s); 10.85 (1 H, s).

**4-(3-(2-(1H-Pyrazol-1-yl)ethyl)-5-fluor-1H-indol-2-yl)-1-benzyl-N,N-dimethylcyclohexanamin, Citrat (2:3) : Unpolareres Diastereomer**

Zu einer Lösung des soeben hergestellten Olefins (250 mg, 0.565 mmol) in HBr/Eisessig (25 ml) wurde innerhalb von 20 min Zinn (0.70 g) gegeben und 4 h bei RT gerührt. Der Ansatz wurde mit Ethanol versetzt, das Reaktionsgemisch bis zur Trockene eingeengt und der Rückstand in 5N NaOH (30 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (9:1→MeOH) getrennt. Die unpolare Verbindung wurde als Salz (159 mg) erhalten, dieses konnte anschließend durch Rühren mit 1 N NaOH und $CH_2Cl_2$ freigesetzt werden. Die Phasen wurden getrennt, die wässrige Phase wurde mit $CH_2Cl_2$ zweimal extrahiert und die vereinigten organischen Phasen über $Na_2SO_4$ getrocknet, filtriert und i. Vak. eingeengt. Wahrscheinlich war das verwendete Chloroform mit HCl verunreinigt.

*Ausbeute:* 85 mg (34 %), unpolares Diastereomer
37 mg (15 %), polares Diastereomer

Die soeben isolierte unpolarere Verbindung (63 mg, 0.14 mmol) wurde in heißem Ethanol (2 ml) gelöst und mit einer Lösung von Citronensäure (27 mg, 0.14 mmol) in heißem Ethanol (2 ml) versetzt. Da kein Feststoff ausfiel wurde das Substanzgemisch i. Vak. eingeengt und getrocknet.
*Ausbeute:* 90 mg **(Bsp 97**, 100 %), poröser Feststoff
*$^1$H-NMR (DMSO-$d_6$):* 1.18 (2 H, m); 1.31 (2 H, m); 1.80 (2 H; m); 2.03 (1 H, m); 2.59 (6 H, s); 2.79 (2 H, m); 3.02 (2 H, t); 4.19 (2 H, t); 6.04 (1 H, s); 6.78 (1 H, m); 7.09 (1 H, m); 7.29 (8 H, m); 11.50 (1 H, s), freie Base.

**Beispiel 98: 4-(3-(2-(1H-Pyrazol-1-yl)ethyl)-5-fluor-1H-indol-2-yl)-1-benzyl-N,N-dimethylcyclohexanamin, Citrat (1:1) : Polareres Diastereomer**

Die unter **Beispiel 97** hergestellte polarere Verbindung (37 mg, 0.08 mmol) wurde in heißem Ethanol (2 ml) gelöst und mit einer Lösung von Citronensäure (16 mg, 0.08 mmol) in heißem Ethanol (2 ml) versetzt. Da kein Feststoff ausfiel wurde das Substanzgemisch i. Vak. eingeengt und getrocknet.
*Ausbeute:* 53 mg (98, 100 %), poröser Feststoff
*$^1$H-NMR (DMSO-$d_6$):* 1.24 (4 H, m); 1.66 (4 H, m); 1.95 (4 H, m); 2.57 (6 H; s); 3.16 (2 H, t); 4.26 (2 H, t); 6.14 (1 H, s); 6.62 (1 H, m); 6.85 (2 H, m); 7.25 (7 H, m); 10.91 (1 H, s), Citrat.

**Beispiel 99: (±)-2-(4-(Dimethylamino)-4-butylcyclohex-1-enyl)-3-(2-(1H-pyrazol-1-yl)ethyl)-5-fluor-1H-indol**

Indol (Ind-38, 730 mg, 3.18 mmol) und Keton (Ket-4, 628 mg, 3.18 mmol) wurden in abs. Dichlormethan (25 ml) gelöst und schnell mit Trifluormethansulfonsäure (0.85 ml, 9.6 mmol) versetzt. Der Ansatz wurde 72 h bei RT gerührt, dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/Me0H (20:1→9:1) gereinigt.
*Ausbeute:* 402 mg (31 %), poröser Feststoff
*$^1$H-NMR (DMSO-$d_6$):* 0.89 (3 H, t); 1.12-1.68 (6 H, m); 2.00 (4 H, m); 2.69 (6 H, bs); 3.18 (2 H, t); 4.33 (2 H, t); 5.80 (1 H, s); 6.16 (1 H, s); 6.88 (1 H, m); 7.18 (1 H, m); 7.28 (1 H, s); 7.44 (1 H, d); 7.56 (1 H, s); 11.07 (1 H, s).

**4-(3-(2-(1H-Pyrazol-1-yl)ethyl)-5-fluor-1H-indol-2-yl)-1-butyl-N,N-dimethylcyclohexanamin, Citrat (1:1) : Unpolareres Diastereomer**

Zu einer Lösung von des soeben hergestellten Olefins (385 mg, 0.94 mmol) in HBr/Eisessig (30 ml) wurde innerhalb von 20 min Zinn (1.20 g) gegeben und 4 h bei RT gerührt. Der Ansatz wurde mit Ethanol versetzt, das Reaktionsgemisch bis zur Trockene eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/meOH (9:1→MeOH) getrennt. Die unpolare Verbindung wurde als Salz (173 mg) erhalten, dieses konnte anschließend durch Rühren mit 1 N NaOH und $CH_2Cl_2$ freigesetzt werden. Die Phasen wurden getrennt, die wässrige Phase wurde mit $CH_2Cl_2$ zweimal extrahiert und die vereinigten organischen Phasen über $Na_2SO_4$ getrocknet, filtriert und i. Vak. eingeengt. Wahrscheinlich war das verwendete Chloroform mit HCl verunreinigt.

*Ausbeute:* 122 mg (32%), unpolares Diastereomer
46 mg (12 %), polares Diastereomer

Die soeben isolierte unpolarere Verbindung (104 mg, 0.25 mmol) wurde in heißem Ethanol (3 ml) gelöst und mit einer Lösung von Citronensäure (49 mg, 0.25 mmol) in heißem Ethanol (2 ml) versetzt. Da kein Feststoff ausfiel wurde das Substanzgemisch i. Vak. eingeengt und getrocknet.
*Ausbeute:* 153 mg (99, 100 %). poröser Feststoff
*$^1$H-NMR (DMSO-$d_6$):* 0.95 (3 H, t); 1.23-1.96 (14 H, m); 2.64 (6 H, s); 3.32 (2 H, t); 4.27 (2 H, t); 6.14 (1 H, t); 6.82 (1 H, m); 7.18 (1 H, m); 7.28 (1 H, m); 7.44 (1 H, d); 7.54 (1 H, d); 10.6 (1 H, s), Citrat.

**Beispiel 100: 4-(3-(2-(1H-Pyrazol-1-yl)ethyl)-5-fluor-1H-indol-2-yl)-1-butyl-N,N-dimethylcyclohexanamin, Citrat (1:1) : Polareres Diastereomer**

Die unter Beispiel 99 hergestellte polarere Verbindung (46 mg, 0.11 mmol) wurde in heißem Ethanol (2 ml) gelöst und mit einer Lösung von Citronensäure (21 mg, 0.11 mmol) in heißem Ethanol (2 ml) versetzt. Nach Versetzen mit Ether und 2-stündigem Stehen im Kühlschrank wurde der entstandene Feststoff abgesaugt und i. Vak. getrocknet.
*Ausbeute:* 48 mg ( **100,** 73 %), poröser Feststoff
*$^1$H-NMR (DMSO-$d_6$):* 0.96 (3 H, t); 1.29 (2 H, m); 1.37-1.83 (11 H, m); 2.57 (7 H, m); 3.12 (2 H, t); 4.24 (2 H, t); 6.14 (1 H, t); 6.83 (1 H, m); 7.20 (2 H, m); 7.45 (1 H, d); 7.52 (1 H, d); 10.82 (1 H, s), Citrat.

**Beispiel 101: (±)-2-(4-(Dimethylamino)-4-phenylcyclohex-1-enyl)-3-(2-(1H-pyrazol-1-yl)ethyl)-5-fluor-1H-indol**

Indol (**Ind-38,** 500 mg, 2.18 mmol) und Keton (**Ket-10,** 473 mg, 2.18 mmol) wurden in abs. Dichlormethan (20 ml) gelöst und schnell mit Trifluormethansulfonsäure (0.58 ml, 6.54 mmol) versetzt. Der Ansatz wurde über Nacht bei RT gerührt, dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 454 mg (49 %), poröser Feststoff
*$^1$H-NMR (DMSO-$d_6$):* 1.71 (2 H, m); 2.14 (8 H, m); 2.67 (H, d); 3.09 (2 H, t); 4.16 (2 H, t); 6.03 (1 H, s); 6.18 (1 H, s); 6.80 (1 H, m); 7.07-7.32 (6 H, m); 7.52 (3 H, m); 10.8 (1 H, s).

**4-(3-(2-(1H-Pyrazol-1-yl)ethyl)-5-fluor-1H-indol-2-yl)-N,N-dimethyl-1-phenylcyclohexanamin, Citrat (2:1) : Unpolareres Diastereomer**

Zu einer Lösung von des Olefins (450 mg, 1.05 mmol) in HBr/Eisessig (30 ml) wurde innerhalb von 20 min Zinn (1.31 g) gegeben und 4 h bei RT gerührt. Der Ansatz wurde mit Ethanol versetzt, das Reaktionsgemisch bis zur Trockene eingeengt und der Rückstand in 5N NaOH (40 ml) und Dichlormethan (20 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/ MeOH (9:1) getrennt und gereinigt.

*Ausbeute:* 178 mg (39 %), unpolares Diastereomer
209 mg (46 %), polares Diastereomer

Die soeben erhaltene unpolarere Verbindung (167 mg, 0.387 mmol) wurde in heißem Ethanol (5 ml) gelöst und mit einer Lösung von Citronensäure (74 mg, 0.387 mmol) in heißem Ethanol (4 ml) versetzt. Nach 2-stündigem Stehen im Kühlschrank wurde der entstandene Feststoff abgesaugt und i. Vak. getrocknet.
*Ausbeute:* 155 mg (76 %); *Schmelzpunkt:* 218-220 °C
$^1$*H-NMR (DMSO-$d_6$)*: 1.56 (2 H, m); 1.71 (2 H, m); 2.19 (2 H; m); 2.34 (6 H, s); 2.65-2.71 (5 H, m); 2.92 (2 H, d); 3.15 (2 H, t); 4.26 (2 H, t); 6.13 (1 H, s); 6.82 (1 H, m); 7.19 (2 H, m); 7.46 (7 H, m); 11.0 (1 H, bs), Hemicitrat.

**Beispiel 102: 4-(3-(2-(1H-Pyrazol-1-yl)ethyl)-5-fluor-1H-indol-2-yl)-N,N-dimethyl-1-phenylcyclohexanamin, Citrat (1:1) : Polareres Diastereomer**

Die unter **Beispiel 101** erhaltene polarere Verbindung (190 mg, 0.44 mmol) wurde in heißem Ethanol (5 ml) gelöst und mit einer Lösung von Citronensäure (85 mg, 0.44 mmol) in heißem Ethanol (5 ml) versetzt. Nach 2- stündigem Stehen im Kühlschrank wurde der entstandene Feststoff abgesaugt und i. Vak. getrocknet.
*Ausbeute:* 90 mg (33 %); *Schmelzpunkt:* 115-116 °C
*$^1$H-NMR (DMSO-$d_6$):* 1.34 (2 H, m); 1.58 (2 H, m); 1.91 (2 H, m); 2.40 (6 H; s); 2.61 (5 H, m); 2.99 (2 H, m); 3.09 (2 H, t); 4.23 (2 H, t); 6.15 (1 H, s); 6.76 (1 H, m); 7.10 (2 H, m); 7.54 (7 H, m); 10.5 (1 H, s), Citrat.

**Beispiel 103: (±)-2-(4-(Dimethylamino)-4-benzylcyclohex-1-enyl)-3-(2-(1H-pyrazol-1-yl)ethyl)-5-fluor-1H-indol**

Indol (**Ind-39**, 600 mg, 2.62 mmol) und Keton (**Ket-3**, 605 mg, 2.62 mmol) wurden in abs. Dichlormethan (20 ml) gelöst und schnell mit Trifluormethansulfonsäure (0.70 ml, 7.9 mmol) versetzt. Der Ansatz wurde 48 h bei RT gerührt, dabei setzte sich ein dunkelbraunes Öl ab. Nach Zugabe von 1 N NaOH (20 ml) und $CH_2Cl_2$ (20 ml) wurde noch 20 min

nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 558 mg (48 %), poröser Feststoff
*$^1$H-NMR (DMSO-$d_6$):* 1.42 (1 H, m); 1.95 (1 H, m); 1.99 (1 H, m); 2.27-2.40 (9 H, m); 2.80 (2 H, q); 3.13 (2 H, t); 4.13 (2 H, t); 5.71 (1 H, s); 6.85 (2 H, m); 7.09 (1 H, s); 7.25-7.29 (7 H, m); 7.47 (1 H, s); 10.87 (1 H, s).

**4-(3-(2-(1H-Imidazol-1-yl)ethyl)-5-fluor-1H-indol-2-yl)-1-benzyl-N,N-dimethylcyclohexanamin, Citrat (1:1) : Unpolareres Diastereomer)**

Zu einer Lösung des soeben hergestellten Olefins (530 mg, 1.2 mmol) in HBr/Eisessig (30 ml) wurde innerhalb von 20 min Zinn (1.5 g) gegeben und 4 h bei RT gerührt. Der Ansatz wurde mit Ethanol versetzt, das Reaktionsgemisch bis zur Trockene eingeengt und der Rückstand in 5N NaOH (40 ml) und Dichlormethan (40 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (9:1→MeOH) getrennt.

*Ausbeute:* 248 mg (46 %), unpolares Diastereomer
62 mg (12 %), polares Diastereomer

Das soeben erhaltene unpolarere Diastereomer (230 mg, 0.517 mmol) wurde in heißem Ethanol (5 ml) gelöst und mit einer Lösung von Citronensäure (99 mg, 0.517 mmol) in heißem Ethanol (4 ml) versetzt. Nach 2-stündigem Stehen im Kühlschrank wurde der entstandene Feststoff abgesaugt und i. Vak. getrocknet.
*Ausbeute:* 180 mg (55 %); *Schmelzpunkt:* 190-192 °C
*$^1$H-NMR (DMSO-$d_6$):* 1.30 (4 H, m); 2.01 (4 H; m); 2.37-2.80 (9 H, m); 2.98 (2 H, t); 4. 0 (2 H, t); 6.80 (2 H, m); 7.09 (1 H, s); 7.15-7.41 (8 H, m); 11.0 (1 H, bs), freie Base.

**Beispiel 104: 4-(3-(2-(1H-Imidazol-1-yl)ethyl)-5-fluor-1H-indol-2-yl)-1-benzyl-N,N-dimethylcyclohexanamin, Citrat (1:1) : Polareres Diastereomer**

Das unter **Beispiel 103** hergestellte, polarere Diastereomer (62 mg, 0.139 mmol) wurde in heißem Ethanol (2 ml) gelöst und mit einer Lösung von Citronensäure (27 mg, 0.139 mmol) in heißem Ethanol (2 ml) versetzt. Nach Versetzen mit Ether und 2-stündigem Stehen im Kühlschrank wurde der entstandene Feststoff abgesaugt und i. Vak. getrocknet.
*Ausbeute:* 53 mg (60 %), poröser Feststoff
*$^1$H-NMR (DMSO-$d_6$):* 1.56 (4 H, m); 1.90 (2 H, m); 2.26 (8 H; m); 2.62 (1 H, m); 3.06 (2 H, t); 3.41 (3 H, m); 4.11 (2 H, t); 6.82 (2 H, m); 7.13-7.41 (9 H, m); 11.01 (1 H, s), (freie Base).

**Beispiel 105: (±)-2-(4-(Dimethylamino)-4-butylcyclohex-1-enyl)-3-(2-(1H-imidazol-1-yl)ethyl)-5-fluor-1H-indol**

Indol (**Ind-39**, 800 mg, 3.49 mmol) und Keton (**Ket-4**, 690 mg, 3.49 mmol) wurden in abs. Dichlormethan (25 ml) gelöst und schnell mit Trifluormethansulfonsäure (0.93 ml, 10.5 mmol) versetzt. Der Ansatz wurde 72 h bei RT gerührt, dabei setzte sich ein dunkelbraunes Öl ab. Nach Zugabe von 1N NaOH (20 ml) und $CH_2C1_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 548 mg (39 %), poröser Feststoff
*$^1$H-NMR (DMSO-$d_6$):* 0.91 (3 H, t); 1.27 (6 H, m); 1.56 (2 H, m); 1.83 (2 H, m); 2.05 (2 H, m); 2.31 (6 H, s); 3.11 (2 H, t); 4.15 (2 H, t); 5.77 (1 H, s); 6.87 (2 H, m); 7.09 (1 H, s); 7.26 (2 H, m); 7.45 (1 H, s); 10.94 (1 H, s).

**4-(3-(2-(1H-Imidazol-1-yl)ethyl)-5-fluor-1H-indol-2-yl)-1-butyl-N,N-dimethylcyclohexanamin, Citrat (2:3) ( 105; : Unpolareres Diastereomer)**

Zu einer Lösung des soeben hergestellten Olefins (528 mg, 1.29 mmol) in HBr/Eisessig (35 ml) wurde innerhalb von 20 min Zinn (1.60 g) gegeben und 4 h bei RT gerührt. Der Ansatz wurde mit Ethanol versetzt, das Reaktionsgemisch bis zur Trockene eingeengt und der Rückstand in 5N NaOH (50 ml) und Dichlormethan (30 ml) gelöst. Die Phasen wurden getrennt, die wässrige Phase zweimal mit $CH_2Cl_2$ extrahiert, die vereinigten organischen Phasen mit Wasser

gewaschen, über $Na_2SO_4$ getrocknet und i. Vak. eingeengt. Der verbliebene Rückstand wurde durch Flash-Chromatographie mit $CHCl_3$/MeOH (9:1→1:1+1 % TEA) getrennt. Die unpolare Verbindung wurde als Salz (350 mg) erhalten, dieses konnte anschließend durch Rühren mit 1 N NaOH und $CH_2Cl_2$ freigesetzt werden. Die Phasen wurden getrennt, die wässrige Phase wurde mit $CH_2Cl_2$ zweimal extrahiert und die vereinigten organischen Phasen über $Na_2SO_4$ getrocknet, filtriert und i. Vak. eingeengt. Wahrscheinlich war das verwendete Chloroform mit HCl verunreinigt.

*Ausbeute:* 285 mg (54 %) unpolares Diastereomer
123 mg (23 %), polares Diastereomer

Das erhaltene unpolarere Diastereomer (285 mg, 0.694 mmol) wurde in heißem Ethanol (5 ml) gelöst und mit einer Lösung von Citronensäure (133 mg, 0.694 mmol) in heißem Ethanol (5 ml) versetzt. Nach 2-stündigem Stehen im Kühlschrank wurde der entstandene Feststoff abgesaugt und i. Vak. getrocknet.
*Ausbeute:* 220 mg (53 %), poröser Feststoff
*$^1$H-NMR (DMSO-$d_6$):* 0.96 (3 H, t); 1.37 (6 H, m); 1.73 (4 H; m); 1.94 (5 H, m); 2.71 (6 H, m); 3.08 (2 H, t); 4.12 (2 H, t); 6.85 (2 H, m); 7.24 (3 H, s); 7.54 (1 H, s); 11.4 (1 H, bs), freie Base

**Beispiel 106: 4-(3-(2-(1H-Imidazol-1-yl)ethyl)-5-fluor-1H-indol-2-yl)-1-butyl-N,N-dimethylcyclohexanamin, Citrat (1:1) : Polareres Diastereomer**

Das unter **Beispiel 105** hergestellte polarere Diastereomer (123 mg, 0.30 mmol) wurde in heißem Ethanol (3 ml) gelöst und mit einer Lösung von Citronensäure (58 mg, 0.3 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 129 mg ( **106,** 71 %), poröser Feststoff
*$^1$H-NMR (DMSO-$d_6$):* 0.95 (3 H, t); 1.23 (8 H, m); 1.63 (7 H, m); 2.19 (6 H; m); 3.05 (2 H, t); 4.09 (2 H, t); 6.81 (2 H, m); 7.21 (3 H, m); 7.41 (1 H, s); 10.8 (1 H, s), freie Base.

**Beispiel 107: (±)-2-(4-(Dimethylamino)-4-phenylcyclohex-1-enyl)-3-(2-(1H-imidazol-1-yl)ethyl)-5-fluor-1H-indol**

Indol (**Ind-39**, 667 mg, 2.91 mmol) und Keton (**Ket-4**, 631 mg, 2.91 mmol) wurden in abs. Dichlormethan (20 ml) gelöst und schnell mit Trifluormethansulfonsäure (0.78 ml, 8.7 mmol) versetzt. Der Ansatz wurde über Nacht bei RT gerührt, dabei setzte sich ein dunkelbraunes Öl ab. Nach Zugabe von 1 N NaOH (20 ml) und $CH_2Cl_2$ (20 ml) wurde noch 20 min nachgerührt, die Phasen getrennt, die wässrige Phase zweimal mit $CH_2Cl_2$ex-trahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1→4:1) gereinigt.
*Ausbeute:* 941 mg (75 %), poröser Feststoff
*$^1$H-NMR (DMSO-$d_6$):* 1.71 (1 H, m); 2.18 (8 H, m); 2.39 (1 H, d); 2.58 (1 H, s); 2.83 (1 H, d); 2.98 /2 H, m); 3.95 (2 H, m); 6.02 (1 H, s); 6.83 (2 H, m); 7.04 (1 H, s); 7.18-7.50 (8 H, m); 10.82 (1 H, s).

**4-(3-(2-(1H-Imidazol-1-yl)ethyl)-5-fluor-1H-indol-2-yl)-N,N-dimethyl-1-phenylcyclohexanamin, Citrat (1:1) : Unpolareres Diastereomer**

Zu einer Lösung des soeben hergestellten Olefins (900 mg, 2.1 mmol) in HBr/Eisessig (50 ml) wurde innerhalb von 20 min Zinn (2.54 g) gegeben und 4 h bei RT gerührt. Der Ansatz wurde mit Ethanol versetzt, das Reaktionsgemisch bis zur Trockene eingeengt und der Rückstand in 5N NaOH (80 ml) und Dichlormethan (50 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (9:1→1:4) getrennt.

*Ausbeute:* 198 mg (22 %), unpolares Diastereomer
157 mg (17 %), polares Diastereomer

Das soeben erhaltene unpolarere Diastereomer (172 mg, 0.4 mmol) wurde in heißem Dioxan (5 ml) gelöst und mit einer Lösung von Citronensäure (77 mg, 0.4 mmol) in heißem Dioxan (4 ml) versetzt. Nach 2-stündigem Stehen im Kühlschrank wurde der entstandene Feststoff abgesaugt und i. Vak. getrocknet.
*Ausbeute:* 185 mg (74 %); *Schmelzpunkt:* 236-238 °C
*$^1$H-NMR (DMSO-$d_6$):* 1.48 (4 H, m); 2.10 (7 H; bs); 2.74 (3 H, m); 3.05 (2 H, t); 4.10 (2 H, t); 6.83 (2 H, m); 7.20-7.52 (9

H, m); 10.95 (1 H, s), freie Base.

**Beispiel 108: 4-(3-(2-(1H-Imidazol-1-yl)ethyl)-5-fluor-1H-indol-2-yl)-N,N-dimethyl-1-phenylcyclohexanamin, Citrat (2:3) : Polareres Diastereomer**

Das unter **Beispiel 107** (144 mg, 0.33 mmol) wurde in heißem Ethanol (5 ml) gelöst und mit einer Lösung von Citronensäure (64 mg, 0.33 mmol) in heißem Ethanol (4 ml) versetzt. Nach 2-stündigem Stehen im Kühlschrank wurde der entstandene Feststoff abgesaugt und i. Vak. getrocknet.
*Ausbeute:* 120 mg (57 %), poröser Feststoff
*$^1$H-NMR (DMSO-$d_6$):* 1.40 (2 H, m); 1.62 (2 H, m); 1.90 (6 H; s); 2.71 (3 H, m); 3.01 (2 H, t); 4.07 (2 H, t); 6.73 (1 H, m); 6.85 (1 H, s); 7.11-7.43 (9 H, m); 10.47 (1 H, s), freie Base.

**Beispiel 109: (±)-2-(4-(Dimethylamino)-4-benzylcyclohex-1-enyl)-3-(2-(1H-benzo[d]imidazol-1-yl)ethyl)-5-fluor-1H-indol**

Indol (**Ind-40**, 668 mg, 2.39 mmol) und Keton (**Ket-3**, 553 mg, 2.39 mmol) wurden in abs. Dichlormethan (30 ml) gelöst und schnell mit Trifluormethansulfonsäure (0.64 ml, 7.2 mmol) versetzt. Der Ansatz wurde 72 h bei RT gerührt, dabei setzte sich ein dunkelbraunes Öl ab. Nach Zugabe von 1N NaOH (20 ml) und $CH_2Cl_2$ (20 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit EE/EtOH (9:1→1:2) gereinigt.
*Ausbeute:* 395 mg (33 %), poröser Feststoff
*$^1$H-NMR (DMSO-$d_6$):* 1.23 (2 H, m); 2.06 (2 H, m); 2.41 (6 H, bs); 3.22 (2 H, t); 3.33 (2 H, s); 4.43 (2 H, t); 5.58 (1 H, s); 6.84 (1 H, m); 7.08 (3 H, m); 7.29 (6 H, m); 7.40 (1 H, d); 7.56 (1 H, d); 7.86 (1 H, s); 10.92 (1 H, s).

**4-(3-(2-(1H-Benzo[d]imidazol-1-yl)ethyl)-5-fluor-1H-indol-2-yl)-1-benzyl-N,N-dimethylcyclohexanamin , Citrat (4:3) : Unpolareres Diastereomer**

Zu einer Lösung des soeben hergestellten Olefins (395 mg, 0.8 mmol) in HBr/Eisessig (30 ml) wurde innerhalb von 20 min Zinn (1.00 g) gegeben und 4 h bei RT gerührt. Der Ansatz wurde mit Ethanol versetzt, das Reaktionsgemisch bis zur Trockene eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit EE/EtOH (9:1→1:4) getrennt.
*Ausbeute:* 173 mg (44 %), unpolares Diastereomer; 57 mg (14 %), polares Diastereomer
Das erhaltene unpolarere Diastereomer (155 mg, 0.313 mmol) wurde in heißem Ethanol (4 ml) gelöst und mit einer Lösung von Citronensäure (60 mg, 0.313 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 107 mg (50 %); *Schmelzpunkt:* 215-217 °C
*$^1$H-NMR (DMSO-$d_6$):* 1.60 (2 H, m); 1.75 (2 H, m); 2.43 (6 H, s); 2.63 (6 H, m); 3.05 (2 H, t); 3.20 (1 H, m); 4.36 (2 H, t); 6.79 (1 H, m); 6.91 (1 H, m); 7.08-7.44 (8 H, m); 7.55 (1 H, d); 7.61 (1 H, s); 10.46 (1 H, s), Citrat.

**Beispiel 110: 4-(3-(2-(1H-Benzo[d]imidazol-1-yl)ethyl)-5-fluor-1H-indol-2-yl)-1-benzyl-N,N-dimethylcyclohexanamin, , Citrat (2:3) : Polareres Diastereomer**

Das unter **Beispiel 109** hergestellte polare Diastereomer (56 mg, 0.11 mmol) wurde in heißem Ethanol (2 ml) gelöst und mit einer Lösung von Citronensäure (22 mg, 0.11 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 54 mg ( **110,** 69 %), poröser Feststoff
*$^1$H-NMR (DMSO-$d_6$):* 1.11 (2 H, m); 1.58 (2 H, m); 1.73 (2 H, m); 2.11 (2 H, m); 2.39 (6 H, s); 2.58 (4 H, m); 3.10 (2 H, bs); 3.21 (2 H, t); 3.45 (1 H, m); (4.45 (2 H, t); 6.85 (1 H, m); 7.19 (9 H, m); 7.60 (2 H, m); 7.76 (1 H, s); 10.83 (1 H, s), Citrat.

**Beispiel 111: 4-(3-(2-(1H-Benzo[d]imidazol-1-yl)ethyl)-5-fluor-1H-indol-2-yl)-1-butyl-N,N-dimethylcyclohexanamin, Citrat (2:3) : Unpolareres Diastereomer**

Zu einer Lösung von **Beispiel 82** (450 mg, 0.98 mmol) in HBr/Eisessig (35 ml) wurde innerhalb von 20 min Zinn (1.25 g) gegeben und 4 h bei RT gerührt. Der Ansatz wurde mit Ethanol versetzt, das Reaktionsgemisch bis zur Trockene eingeengt und der Rückstand in 5N NaOH (50 ml) und Dichlormethan (30 ml) gelöst. Die Phasen wurden

getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit EE/EtOH (1:4→EtOH) getrennt.

*Ausbeute:* 304 mg (67 %), unpolares Diastereomer
119 mg (26 %), polares Diastereomer

Das soeben erhaltene unpolarere Diastereomer (275 mg, 0.597 mmol) wurde in heißem Ethanol (10 ml) gelöst und mit einer Lösung von Citronensäure (115 mg, 0.597 mmol) in heißem Ethanol (5 ml) versetzt. Nach Versetzen mit Ether und 2-stündigem Stehen im Kühlschrank wurde der entstandene Feststoff abgesaugt und i. Vak. getrocknet.
*Ausbeute:* 165 mg (42 %), poröser Feststoff
*$^1$H-NMR (DMSO-$d_6$):* 0.97 (5 H, m); 1.06 (4 H, m); 1.42 (4 H, m); 1.82 (4 H, m); 2.07 (1 H, m); 2.59 (10 H, m); 3.19 (2 H, t); 4.45 (2 H, t); 6.83 (1 H, m); 7.21 (4 H, m); 7.62 (2 H, d); 7.77 (1 H, s); 10.99 (1 H, s), Citrat.

**Beispiel 112: 4-(3-(2-(1H-1,2,3-Triazol-1-yl)ethyl)-5-fluor-1H-indol-2-yl)-1-benzyl-N,N-dimethylcyclohexanamin, Citrat (4:1) : Unpolareres Diastereomer**

Zu einer Lösung von **Beispiel 59** (356 mg, 0.8 mmol) in HBr/Eisessig (30 ml) wurde innerhalb von 20 min Zinn (1.00 g) gegeben und 4 h bei RT gerührt. Der Ansatz wurde mit Ethanol versetzt, das Reaktionsgemisch bis zur Trockene eingeengt und der Rückstand in 5N NaOH (40 ml) und Dichlormethan (40 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit EE/EtOH (1:4→1:2 + 1 % TEA) getrennt.

*Ausbeute:* 237 mg (66 %), unpolares Diastereomer
73 mg (20 %), polares Diastereomer

Das soeben erhaltene unpolarere Diastereomer (234 mg, 0.525 mmol) wurde in heißem Ethanol (10 ml) gelöst und mit einer Lösung von Citronensäure (101 mg, 0.525 mmol) in heißem Ethanol (5 ml) versetzt. Nach Versetzen mit Ether und 2-stündigem Stehen im Kühlschrank wurde der entstandene Feststoff abgesaugt und i. Vak. getrocknet.
*Ausbeute:* 173 mg ( **112,** 61 %); *Schmelzpunkt:* 262-167 °C
*$^1$H-NMR (DMSO-$d_6$):* 1.34 (4 H, m); 2.11 (2 H, m); 2.46 (1 H, m); 2.56 (2 H, m); 2.75 (6 H, s); 2.92 (2 H, m); 3.14 (2 H, t); 4.44 (2 H, t); 6.79 (1 H, m); 7.13 (2 H, m); 7.28 (5 H, m); 7.55 (1 H, s); 7.90 (1 H, s); 11.41 (1 H, s), Hemicitrat.

**Beispiel 113: 4-(3-(2-(1H-1,2,3-Triazol-1-yl)ethyl)-5-fluor-1H-indol-2-yl)-1-benzyl-N,N-dimethylcyclohexanamin, Citrat (1:1) : Polareres Diastereomer**

Das unter **Beispiel 112** hergestellte polare Diastereomer (73 mg, 0.163 mmol) wurde in heißem Ethanol (3 ml) gelöst und mit einer Lösung von Citronensäure (31 mg, 0.163 mmol) in heißem Ethanol (2 ml) versetzt. Nach Versetzen mit Ether und 2-stündigem Stehen im Kühlschrank wurde der entstandene Feststoff abgesaugt und i. Vak. getrocknet.
*Ausbeute:* 79 mg ( **113,** 76 %), poröser Feststoff
*$^1$H-NMR (DMSO-$d_6$):* 1.64 (4 H, m); 1.89 (4 H, m); 2.47 (11 H, m); 3.22 (2 H, t); 4.55 (2 H, t); 6.85 (1 H, m); 7.27 (7 H, m); 7.65 (1 H, s); 8.01 (1 H, s); 10.98 (1 H, s), Citrat.

**Beispiel 114: 4-(3-(2-(1H-1,2,3-Triazol-1-yl)ethyl)-5-fluor-1H-indol-2-yl)-1-butyl-N,N-dimethylcyclohexanamin , Citrat (2:1) : Unpolareres Diastereomer**

Zu einer Lösung von **Beispiel 60** (440 mg, 1.07 mmol) in HBr/Eisessig (30 ml) wurde innerhalb von 20 min Zinn (1.40 g) gegeben und 4 h bei RT gerührt. Der Ansatz wurde mit Ethanol versetzt, das Reaktionsgemisch bis zur Trockene eingeengt und der Rückstand in 5N NaOH (40 ml) und Dichlormethan (40 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit EE/EtOH (1:4→1:2 + 1 % TEA) getrennt.

*Ausbeute:* 265 mg (60 %), unpolares Diastereomer
134 mg (30 %), polares Diastereomer

Das soeben erhaltene unpolarere Diastereomer (245 mg, 0.595 mmol) wurde in heißem Ethanol (10 ml) gelöst und mit einer Lösung von Citronensäure (114 mg, 0.595 mmol) in heißem Ethanol (5 ml) versetzt. Nach Versetzen mit Ether und 2-stündigem Stehen im Kühlschrank wurde der entstandene Feststoff abgesaugt und i. Vak. getrocknet.
*Ausbeute:* 121 mg (40 %); *Schmelzpunkt:* 221-223 °C
*$^1$H-NMR (DMSO-$d_6$):* 1.03 (3 H, t); 1.33 (6 H, m); 1.57 (2 H, m); 1.70 (2 H, m); 1.99 (2 H, m); 2.16 (2 H, m); 2.66 (11 H, m); 3.21 (2 H, t); 4.53 (2 H, t); 6.85 (1 H, m); 7.21 (2 H, m); 7.64 (1 H, s); 8.02 (1 H, s); 11.43 (1 H, s), Hemicitrat.

**Beispiel 115: 4-(3-(2-(1H-1,2,3-Triazol-1-yl)ethyl)-5-fluor-1H-indol-2-yl)-1-butyl-N,N-dimethylcyclohexanamin, Citrat (1: 1) : Polareres Diastereomer**

Das unter **Beispiel 114** hergestellte polare Diastereomer (130 mg, 0.315 mmol) wurde in heißem Ethanol (5 ml) gelöst und mit einer Lösung von Citronensäure (61 mg, 0.315 mmol) in heißem Ethanol (2 ml) versetzt. Nach Versetzen mit Ether und 2-stündigem Stehen im Kühlschrank wurde der entstandene Feststoff abgesaugt und i. Vak. getrocknet.
*Ausbeute:* 110 mg (57 %), poröser Feststoff
*$^1$H-NMR (DMSO-$d_6$):* 1.03 (3 H, t); 1.35 (6 H, m); 1.86 (8 H, m); 2.65 (11 H, m); 3.22 (2 H, t); 4.54 (2 H, t); 6.85 (1 H, m); 7.26 (2 H, m); 7.65 (1 H, s); 8.05 (1 H, s); 10.90 (1 H, s), Citrat.

**Beispiel 116: 4-(3-(2-(1H-1,2,3-Triazol-1-yl)ethyl)-5-fluor-1H-indol-2-yl)-N,N-dimethyl-1-phenylcyclohexanamin, Citrat (2:1): Unpolareres Diastereomer**

Zu einer Lösung von Beispiel 61 (730 mg, 1.7 mmol) in HBr/Eisessig (70 ml) wurde innerhalb von 20 min Zinn (2.10 g) gegeben und 4 h bei RT gerührt. Der Ansatz wurde mit Ethanol versetzt, das Reaktionsgemisch bis zur Trockene eingeengt und der Rückstand in 5N NaOH (70 ml) und Dichlormethan (70 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit EE/EtOH (9:1→EtOH) getrennt.

*Ausbeute:* 181 mg (25 %), unpolares Diastereomer
265 mg (36 %), polares Diastereomer

Das soeben erhaltene unpolarere Diastereomer (168 mg, 0.389 mmol) wurde in heißem Ethanol (5 ml) gelöst und mit einer Lösung von Citronensäure (75 mg, 0.389 mmol) in heißem Ethanol (3 ml) versetzt. Nach 2-stündigem Stehen im Kühlschrank wurde der entstandene Feststoff abgesaugt und i. Vak. getrocknet.
*Ausbeute:* 89 mg (37 %); *Schmelzpunkt:* 228-229 °C
*$^1$H-NMR (DMSO-$d_6$):* 1.47 (4 H, m); 2.04 (2 H, m); 2.12 (6 H, s); 2.67 (7 H, m); 3.21 (2 H, t); 4.54 (2 H, t); 6.83 (1 H, m); 7.30 (7 H, m); 7.64 (1 H, s); 8.01 (1 H, s); 10.8 (1 H, s), Citrat.

**Beispiel 117: 4-(3-(2-(1H-1,2,3-Triazol-1-yl)ethyl)-5-fluor-1H-indol-2-yl)-N,N-dimethyl-1-phenylcyclohexanamin, Citrat (2:1): Polareres Diastereomer**

Das unter **Beispiel 116** hergestellte polarere Diastereomer (248 mg, 0.574 mmol) wurde in heißem Ethanol (8 ml) gelöst und mit einer Lösung von Citronensäure (110 mg, 0.574 mmol) in heißem Ethanol (6 ml) versetzt. Nach 2-stündigem Stehen im Kühlschrank wurde der entstandene Feststoff abgesaugt und i. Vak. getrocknet.
*Ausbeute:* 262 mg (73 %), poröser Feststoff
*$^1$H-NMR (DMSO-$d_6$):* 1.33 (2 H, m); 1.53 (2 H, m); 1.80 (2 H, m); 2.29 (6 H, s); 2.57 (5 H, m); 2.89 (2 H, m); 3.19 (2 H, t); 4.50 (2 H, t); 6.77 (1 H, m); 7.13 (2 H, m); 7.45 (5 H, s); 7.67 (1 H, s); 7.98 (1 H, s); 10.5 (1 H, s), Citrat.

**Beispiel 118: 2-(4-(Dimethylamino)-4-phenylcyclohexyl)-3-methyl-1H-indol-5-ol, unpolares Diastereomer**

Zu einer Lösung der freien Base aus **Bsp 127** (unpolareres Diastereoisomer, 205 mg, 0,57 mmol) in trockenem Dichlormethan (30 ml) wurde bei RT unter Rühren und Feuchtigkeitsausschluß eine ca. 1 M Lösung von $BBr_3$ in Dichlormethan (3 ml, ca. 3 mmol) gegeben. Nach 10 min fiel ein Niederschlag aus. Der Ansatz wurde 18 h bei RT gerührt. - Zur Aufarbeitung wurde das Gemisch mit ges. $NaHCO_3$-Lösung (20 ml) versetzt und 30 min gerührt. Der an der Phasengrenze befindliche Feststoff (156 mg) wurde abgetrennt. und in einer Mischung aus ges. $NaHCO_3$-Lösung (20 ml) und Methanol (2 ml) 3 d gerührt. Aus dem Gemisch wurde am Rotationsverdampfer das Methanol entfernt und der wässrige Rückstand mit Dichlormethan (5 x 10 ml) extrahiert. Die vereinigten organischen Phasen wurden über $MgSO_4$

getrocknet und anschließend eingeengt. Der erhaltene Rückstand wurde aus Methanol (1 ml) umkristallisiert. **Beispiel 118** wurde so in einer Ausbeute von 62 mg (31%, *Schmelzpunkt:* 227-235 °C) als grün/beiger Feststoff erhalten.
$^1$*H NMR (400 MHz, DMSO-d$_6$) δ ppm:* 1.20-1.55 (m, 2H), 1.57-1.69 (m, 2H), 2.02 (s,6H), 2.05-2.15 - 2.1 (m, 5H), 2.73-2.90 (m, 3H), 6.48 (dd, J = 8.49, 2.27 Hz, 1H), 6.66 (d, J = 2.07 Hz, 1H), 7.06 (d, J = 8.47 Hz, 1H), 7.37 (d, J = 4.20 Hz, 4H), 8.40 (s, 1H), 10.22 (s, 1H). $^{13}$C NMR (101 MHz, DMSO) ❐ ppm 8.5, 27.1, 33.2, 35.5, 37.7, 58.0, 101.5, 102.7, 109.6, 110.9, 126.2, 126.4, 127.2, 129.5, 129.6, 139.7, 140.9, 149.9

**Beispiel 119:** 2-(4-(Dimethylamino)-4-phenylcyclohexyl)-3-methyl-1H-indol-5-ol, polares Diastereomer

Lösung der freien Base aus **Bsp 128** (polareres Diastereoisomer, 340 mg, 0,94 mmolin trockenem Dichlormethan (40 ml) wurde bei RT unter Rühren und Feuchtigkeitsausschluß eine ca. 1 M Lösung von $BBr_3$ in Dichlormethan (5 ml, ca. 5 mmol) gegeben. Der Ansatz wurde 18 h bei RT gerührt. - Zur Aufarbeitung wurde das Gemisch mit Wasser (10 ml) versetzt und das entstandene Gemisch auf ges. $NaHCO_3$-Lösung (30 ml) gegeben. Die Mischung wurde 2 h bei RT gerührt, dann wurde der mittels einer Fritte abgetrennt. Die Phasen des Filtrats wurden getrennt. Die wässrige Phase wurde mit Dichlormethan (2 x 20 ml) extrahiert. Die vereinigten organischen Phasen wurden über $MgSO_4$ getrocknet und anschließend am Rotationsverdampfer eingeengt. Durch säulenchromatographische Reinigung [Kieselgel 60 G (10 g); EtOAc/EtOH 1 : 1 (100 ml)] konnte weiteres Produkt (90 mg) isoliert werden. Nach Umkristallisation der vereinigten Produktfraktionen (Methanol, 3 ml) wurde **Beispiel 119** in einer Ausbeute von 92 mg (28 %, *Schmelzpunkt:* 164-169 °C) als ockerfarbener Feststoff erhalten.
$^1$*H NMR (400 MHz, DMSO-d$_6$) δ ppm:* 1.35-1.52 (m, 2H), 1.75-1.87 (m, 2H), 1.94-2.05 (s, 6H), 2.28-2,45 (m, 5H), 2.81-2,87 (m, 1H), 2,95-3.06 (m, 2H), 6.37-6.41 (m, 2H), 6.62 (s, 1H), 6.92 (d, *J* = 8.49 Hz, 1H), 7.46-7,71 (m, 5H), 8.40 (s, 1H), 9.99 (s, 1H)
$^{13}$*C NMR (101 MHz, DMSO-d$_6$) δ ppm:* 8.4, 28.1, 30,9, 35,0, 37.4, 101.5, 103.2, 109.9, 110.7, 128.8, 129.2, 129,3, 129.4, 138,5, 149.96

**Beispiel 120: 1-Benzyl-N,N-dimethyl-4-(3-methyl-5-(trifluormethoxy)-1H-indol-2-yl)cyclohexanamin, Citrat (1:1), polares Diastereomer**

**(±)-*N*-[1-Benzyl-4-(3-methyl-5-trifluormethoxy-1*H*-indol-2-yl)cyclohex-3-enyl]-*N,N*-dimethylamin**

Das Keton **Ket-3** (462 mg, 2 mmol) wurde zusammen mit Indol **Ind-43** (430 mg, 2 mmol) in Dichlormethan (25 ml) gelöst. Anschließend erfolgte die Zugabe von Trifluormethansulfonsäure (0,2 ml, 338 mg, 2,25 mmol), wobei sich der Ansatz dunkel färbte. Die Lösung wurde 3 d bei RT gerührt. Der Verlauf der Reaktion wurde mittels DC verfolgt. Zur Abtrennung des unumgesetzten Ketons wurde das Reaktionsgemisch mit Wasser (3 × 15 ml) jeweils 10 min gerührt. Nach Phasentrennung wurde die organische Phase mit 1 N NaOH (10 ml) versetzt und 10 min gerührt. Nach Phasentrennung wurde die wässrige Phase mit Dichlormethan (2 × 10 ml) extrahiert. Die vereinigten organischen Phasen wurden mit Natriumsulfat getrocknet, filtriert und im Vakuum eingeengt.,Es wurden 620 mg eines braunen Öls erhalten, das chromatographisch getrennt wurde [Kieselgel 60 (60 g); Ethylacetat/Methanol (10 : 1; 500 ml)]. (±)-*N*-[1-Benzyl-4-(3-methyl-5-trifluormethoxy-1*H*-indol-2-yl)cyclohex-3-enyl]-*N,N*-dimethylamin wurde in einer Ausbeute von 27 % (233 mg) als farbloser Feststoff mit einem Schmelzpunkt von 74-75 °C gewonnen.

**1-Benzyl-N,N-dimethyl-4-(3-methyl-5-(trifluormethoxy)-1H-indol-2-yl)cyclohexanamin (polares Diastereomer)**

(±)-*N*-[1-Benzyl-4-(3-methy)-5-thifluormethoxy-1*H*-indol-2-yl)cyclohex-3-enyl]-*N,N*-dimethylamin (230 mg, 0,53 mmol) wurde unter Erwärmen in Methanol (40 ml) und Ethanol (10 ml) gelöst und unter Argon mit Pd/C (5 %, 100 mg) versetzt. Bei 3 bar wurde 4 h bei 40 °C hydriert. Anschließend wurde der Katalysator über Celite abgesaugt und das Filtrat eingeengt. Der feste farblose Rückstand (227 mg) wurde chromatographisch getrennt [Kieselgel 60 (30 g); Ethylacetat/Methanol (20 : 1; 400 ml); (4 : 1; 500 ml) (2 : 1; 300 ml)]. 1-Benzyl-N,N-dimethyl-4-(3-methyl-5-(trifluormethoxy)-1H-indol-2-yl)cyclohexanamin (polareres Diastereoisomer) wurde in einer Ausbeute von 175 mg (77 %) als farbloser Feststoff erhalten.

**1-Benzyl-N,N-dimethyl-4-(3-methyl-5-(trifluormethoxy)-1H-indol-2-yl)cyclohexanamin, Citrat (1:1), polares Diastereome**r

1-Benzyl-N,N-dimethyl-4-(3-methyl-5-(trifluormethoxy)-1H-indol-2-yl)cyclohexanamin (polareres Diastereoisomer) (159 mg, 0,37 mmol) wurde unter Erwärmen in Ethanol (8 ml) gelöst und mit Citronensäure (78 mg, 0,4 mmol), gelöst in heißem Ethanol (5 ml), versetzt. Nach ca. 30 min begann ein Niederschlag auszufallen. Der Ansatz wurde 20 h gerührt und der Niederschlag abgesaugt. Das Filtrat wurde auf die Hälfte eingeengt und mit Diethylether (10 ml)

versetzt. Nach weiteren 2 h Rühren bei RT konnte der ausgefallene Niederschlag abgesaugt werden. Beide Fraktionen wurden vereinigt. **Beispiel 120** wurde in einer Ausbeute von 66 % (153 mg) gewonnen.

**Beispiel 121: 1-Butyl-N,N-dimethyl-4-(3-methyl-5-(trifluormethoxy)-1H-indol-2-yl)cyclohexanamin, Citrat (1:1), unpolares Diastereomer**

**Beispiel 122: 1-Butyl-N,N-dimethyl-4-(3-methyl-5-(trifluormethoxy)-1H-indol-2-yl)cyclohexanamin, Citrat (1:1), polares Diastereomer**

**(±)-*N*-[1-Butyl-4-(3-methyl-5-trifluormethoxy-1*H*-indol-2-yl)cyclohex-3-enyl]-*N,N*-dimethylamin**

Das Keton **Ket-4** (513 mg, 2,6 mmol) wurde zusammen mit Indol **Ind-43** (560 mg, 2,6 mmol) in Dichlormethan (30 ml) gelöst. Anschließend erfolgte die Zugabe von Trifluormethansulfonsäure (0,26 ml, 439 mg, 2,86 mmol). Die Lösung wurde 3 d bei RT gerührt. Der Verlauf der Reaktion wurde mittels DC verfolgt. Zur Abtrennung des Ketons wurde Wasser (15 ml) zugegeben und die Mischung 10 min kräftig gerührt. Nach Phasentrennung wurde die organische Phase emeut mit Wasser (15 ml) gerührt. Insgesamt wurde die Prozedur dreimal durchgeführt. Anschließend wurde die organische Phase mit 1 N NaOH (10 ml) versetzt und 10 min gerührt. Nach Phasentrennung wurde die wässrige Phase mit Dichlormethan (2 × 10 ml) extrahiert. Die vereinigten organischen Phasen wurden mit Natriumsulfat getrocknet, filtriert und im Vakuum eingeengt. Es wurden 840 mg eines braunen Öls erhalten, das chromatographisch aufgetrennt wurde [Kieselgel 60 (60 g); Ethylacetat/Methanol (10 : 1; 500 ml); (1 : 1; 500 ml)]. (±)-*N*-[1-Butyl-4-(3-methyl-5-trifluormethoxy-1*H*-indol-2-yl)cyclohex-3-enyl)-*N,N*-dimethylamin wurde in einer Ausbeute von 31 % (321 mg) als hellbraunes Öl gewonnen.

**1-Butyl-N,N-dimethyl-4-(3-methyl-5-(trifluormethoxy)-1H-indol-2-yl)cyclohexanamin (unpolares und polares Diastereomer)**

(±)-*N*-[1-Butyl-4-(3-methyl-5-trifluormethoxy-1*H*-indol-2-yl)cyclohex-3-enyl]-*N,N*-dimethylamin (320 mg, 0,81 mmol) wurde in Ethanol (50 ml) gelöst und unter Argon mit Pd/C (5 %, 140 mg) versetzt. Bei 3 bar wurde 1,5 h hydriert. Anschließend wurde der Katalysator über Celite abgesaugt und das Filtrat eingeengt. Der feste hellbraune Rückstand (341 mg) wurde chromatographisch getrennt [Kieselgel 60 (30 g); Ethylacetat/Methanol (1 : 1; 500 ml); (1 : 2; 300 ml)]. 1-Butyl-N,N-dimethyl-4-(3-methyl-5-(trifluormethoxy)-1H-indol-2-yl)cyclohexanamin (unpolareres Diastereoisomer) wurde in einer Ausbeute von 28 mg (9 %) und das polarere Diastereoisomer in einer Ausbeute von 237 mg (74 %) jeweils als farbloser Feststoff erhalten.

**1-Butyl-N,N-dimethyl-4-(3-methyl-5-(trifluormethoxy)-1H-indol-2-yl)cyclohexanamin, Citrat (1:1), unpolares Diastereomer**

1-Butyl-N,N-dimethyl-4-(3-methyl-5-(trifluormethoxy)-1H-indol-2-yl)cyclohexanamin (unpolareres Diastereoisomer) (28 mg, 0,07 mmol) wurde in der Wärme in Ethanol (5 ml) gelöst und mit Citronensäure (15 mg, 0,08 mmol), gelöst in heißem Ethanol (1 ml), versetzt. Die klare farblose Lösung wurde 24 h gerührt und anschließend bis auf ca. 0,5 ml eingeengt. Nach Zugabe von Diethylether (5 ml) wurde 1 h bei RT gerührt und dann der Niederschlag abgesaugt. **Beispiel 121** wurde in einer Ausbeute von 75 % (31 mg) mit einem Schmelzpunkt von 246-253 °C gewonnen.

**1-Butyl-N,N-dimethyl-4-(3-methyl-5-(trifluormethoxy)-1H-indol-2-yl)cyclohexanamin, Citrat (1:1), polares Diastereomer**

1-Butyl-N,N-dimethyl-4-(3-methyl-5-(trifluormethoxy)-1H-indol-2-yl)cyclohexanamin (polareres Diastereoisomer) (230 mg, 0,58 mmol) wurde in der Wärme in Ethanol (15 ml) gelöst und mit Citronensäure (122 mg, 0,64 mmol), gelöst in heißem Ethanol (2 ml), versetzt. Die klare farblose Lösung wurde 24 h gerührt und anschließend bis auf ca. 0,5 ml eingeengt. Nach Zugabe von Diethylether (5 ml) wurde 1 h bei RT gerührt und dann der Niederschlag abgesaugt. **Beispiel 122** wurde in einer Ausbeute von 74 % (252 mg) mit einem Schmelzpunkt von 166-168 °C gewonnen.

**Beispiel 123: N,N-Dimethyl-4-(3-methyl-5-(trifluormethoxy)-1H-indol-2-yl)-1-phenylcyclohexanamin, Citrat (2:1), unpolares Diastereomer**

**Beispiel 124: N,N-dimethyl-4-(3-methyl-5-(trifluormethoxy)-1H-indol-2-yl)-1-phenylcyclohexanamin, Citrat (1:1), polares Diastereomer**

**(±)-*N,N*-Dimethyl-*N*-[4-(3-methyl-5-trifluormethoxy-1*H*-indol-2-yl)-1-phenylcyclohex-3-enyl]amin**

Das Keton **Ket-10** (313 mg, 1,44 mmol) wurde zusammen mit **Ind-43** (310 mg, 1,44 mmol) in Dichlormethan (15 ml) gelöst. Anschließend erfolgte die Zugabe von Trifluormethansulfonsäure (0,144 ml, 1,6 mmol). Es wurde 3 d bei RT gerührt. Zur Aufarbeitung wurde das Reaktionsgemisch mit 1N NaOH (10 ml) versetzt und 10 min gerührt. Nach Phasentrennung wurde die wässrige Phase mit Dichlormethan (2 × 10 ml) extrahiert. Die vereinigten organischen Phasen wurden mit Natriumsulfat getrocknet, filtriert und im Vakuum eingeengt. Es wurden 676 mg eines hellbraunen Feststoffes erhalten, der chromatographisch getrennt wurde (Kieselgel 60 (30 g); Ethylacetat/Methanol (10 : 1; 500 ml)]. Es wurden 491 mg (82 %) (±)-*N,N*-Dimethyl-*N*-[4-(3-methyl-5-trifluormethoxy-1*H*-indol-2-yl)-1-phenylcyclohex-3-enyl] amin gewonnen.

**N,N-dimethyl-4-(3-methyl-5-(trifluormethoxy)-1H-indol-2-yl)-1-phenylcyclohexanamin (unpolares und polares Diastereomer)**

(±)-*N,N*-Dimethyl-*N*-[4-(3-methyl-5-trifluormethoxy-1*H*-indol-2-yl)-1-phenylcydohex-3-enyl]amin (350 mg, 0,84 mmol) wurde mit HBr/Eisessig (33 % HBr, 18 ml) so lange gerührt, bis es vollständig gelöst war. Anschließend wurde zu dem Ansatz bei RT Zinn-Pulver (0,98 g, 8,4 mmol) innerhalb von 30 min portionsweise gegeben. Nach Beendigung der Zugabe wurde das Reaktionsgemisch weitere 1,5 h gerührt und zur Aufarbeitung anschließend am Rotationsverdampfer bis zur Trockne eingeengt. Der verbliebene Rückstand wurde durch Zugabe von 5N NaOH (40 ml) basisch gemacht. Die erhaltene Lösung wurde mit Dichlormethan extrahiert (4 x 30 ml). Die vereinigten organischen Phasen wurden mit $Na_2SO_4$ getrocknet und anschließend eingeengt. Der Rückstand (410 mg) wurde säulenchromatographisch [Kieselgel 60 (30 g); Ethylacetat/Cyclohexan (1 : 1; 500 ml); Ethylacetat/Methanol (2 : 1; 450 ml)] getrennt. N,N-dimethyl-4-(3-methyl-5-(trifluormethoxy)-1H-indol-2-yl)-1-phenylcyclohexanamin (unpolareres Diastereoisomer) wurde in einer Ausbeute von 132 mg (38 %) als farbloser Feststoff mit einem Schmelzpunkt von 78-84 °C erhalten. Das polarere Diastereoisomer wurde in einer Ausbeute von 151 mg (43 %) als farbloser Feststoff mit einem Schmelzpunkt von 219-221 °C erhalten.

**N,N-Dimethyl-4-(3-methyl-5-(trifluormethoxy)-1H-indol-2-yl)-1-phenylcyclohexanamin, Citrat (2:1), unpolares Diastereomer**

N,N-dimethyl-4-(3-methyl-5-(trifluormethoxy)-1H-indol-2-yl)-1-phenylcyclohexanamin (unpolareres Diastereoisomer) (132 mg, 0,317 mmol) wurde in Ethanol (10 ml) gelöst und mit Citronensäure (67 mg, 0,349 mmol), gelöst in heißem Ethanol (2 ml), versetzt. Nach 30 min Rühren bei RT begann ein farbloser Feststoff auszufallen. Nach 20 h Rühren wurde abgesaugt. Beispiel **123** wurde in einer Ausbeute von 52 % (84 mg) mit einem Schmelzpunkt von 209-211 °C gewonnen.

**N,N-dimethyl-4-(3-methyl-5-(trifluormethoxy)-1H-indol-2-yl)-1-phenylcyclohexanamin, Citrat (1:1), polares Diastereomer**

N,N-dimethyl-4-(3-methyl-5-(trifluormethoxy)-1H-indol-2-yl)-1 -phenylcyclohexanamin (polareres Diastereoisomer) (151 mg, 0,36 mmol) wurde in Ethanol (10 ml) gelöst und mit Citronensäure (77 mg, 0,4 mmol), gelöst in heißem Ethanol (2 ml), versetzt. Nach 20 h Rühren bei RT wurde der ausgefallene farblose Feststoff abgesaugt. Beispiel **124** wurde in einer Ausbeute von 50 % (109 mg) mit einem Schmelzpunkt von 198-199 °C gewonnen.

**Beispiel 125: 1-Butyl-4-(5-methoxy-3-methyl-1H-indol-2-yl)-N,N-dimethylcyclohexanamin, Citrat (1:1), unpolares Diastereomer**

**Beispiel 126: 1-Butyl-4-(5-methoxy-3-methyl-1H-indol-2-yl)-N,N-dimethylcyclohexanamin, Citrat (1:1), polares Diastereomer**

**(±)-*N*-[1-Butyl-4-(5-methoxy-3-methyl-1*H*-indol-2-yl)cyclohex-3-enyl]-*N,N*-dimethylamin**

5-Methoxyskatol **(Ind-9,** 806 mg, 5 mmol) wurde zusammen mit **Ket-4** (985 mg, 5 mmol) in Dichlormethan (40 ml) gelöst und mit Trifluormethansulfonsäure (0,65 ml, 7,5 mmol) versetzt. Der Ansatz wurde 24 h bei RT gerührt. - Zur Aufarbeitung wurde das Reaktionsgemisch mit 2N NaOH (20 ml) versetzt und 20 min bei RT gerührt. Nach Abtrennung der organischen Phase wurde die wässrige Phase mit Dichlormethan (3 × 20 ml) extrahiert. Die vereinigten organischen Extrakte wurden über $Na_2SO_4$ getrocknet und anschließend eingeengt. Das Rohprodukt wurde in einer Ausbeute von 1,69 g (99 %) als gelbes Öl erhalten und wurde ohne weitere Reinigung in der nächsten Reaktion eingesetzt.

**1-Butyl-4-(5-methoxy-3-methyl-1H-indol-2-yl)-N,N-dimethylcyclohexanamin (unpolares und polares Diastereomer)**

(±)-*N*-[1-Butyl-4-(5-methoxy-3-methyl-1*H*-indol-2-yl)cyclohex-3-enyl]-*N*,*N*-dimethylamin (1,5 g, 4,4 mmol) wurde in HBr/Eisessig (33 % HBr, 20 ml) suspendiert. Anschließend wurde zu dem Ansatz bei RT Sn-Pulver (2,6 g, 22 mol) innerhalb von 30 min portionsweise gegeben. Nach Beendigung der Zugabe wurde das Reaktionsgemisch weitere 24 h bei RT gerührt. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (100 ml) basisch gestellt. Das erhaltene wässrige Gemisch wurde mit Dichlormethan (4 × 20 ml) extrahiert. Die vereinigten organischen Phasen wurden mit Wasser (50 ml) gewaschen, über $Na_2SO_4$ getrocknet und anschließend eingeengt. Der erhaltene Rückstand (1,5 g) wurde durch Säulenchromatographie [Kieselgel 60 (70 g); EtOAc (400 ml), Methanol (400 ml)] gereinigt. 1-Butyl-4-(5-methoxy-3-methyl-1H-indol-2-yl)-N.N-dimethylcyclohexanamin (unpolareres Diastereomer) wurde in einer Ausbeute von 141 mg (10 %,) als braunes Öl erhalten. Das polarere Produkt mußte durch eine zweite Säulenchromatographie [Kieselgel 60 (50 g); Methanol (200 ml), MeCN/Methanol/1M $NH_4Cl$-Lsg. 9 : 1 : 1 (250 ml)] gereinigt werden. Es wurde in einer Ausbeute von 125 mg (8 %,) als gelbes Öl erhalten.

**1-Butyl-4-(5-methoxy-3-methyl-1H-indol-2-yl)-N,N-dimethylcyclohexanamin, Citrat (1:1), unpolares Diastereomer**

1-Butyl-4-(5-methoxy-3-methyl-1H-indol-2-yl)-N,N-dimethytcyclohexanamin (unpolareres Diastereomer) (50 mg, 0,14 mmol) wurde in heißem Isopropanol (20 ml) gelöst und mit isopropanolischer Citronensäure-Lösung (28 mg, 0,14 mmol in 2 ml) versetzt. Die Mischung wurde 2 h bei Raumtemperatur gerührt. Der weiße Feststoff wurde abgesaugt. Beispiel 125 wurde in einer Ausbeute von 56 mg (71 %) mit einem Schmelzpunkt von 115-121 °C erhalten .

**1-Butyl-4-(5-methoxy-3-methyl-1H-indol-2-yl)-N,N-dimethylcyclohexanamin, Citrat (1:1), polares Diastereomer**

1-Butyl-4-(5-methoxy-3-methyl-1H-indol)-2-yl)-N,N-dimethylcyclohexanamin (polareres Diastereomer) (125 mg, 0,36 mmol) wurde in heißem Isopropanol (30 ml) gelöst und mit isopropanolischer Citronensäure-Lösung (70 mg, 0,36 mmol in 2 ml) versetzt. Die Mischung wurde 2 h bei Raumtemperatur gerührt. Das ausgefallene Feststoff wurde abgesaugt. **Beispiel 126** wurde in einer Ausbeute von 130 mg (65 %) erhalten.

**Beispiel 127: 4-(5-Methoxy-3-methyl-1H-indol-2-yl)-N,N-dimethyl-1-phenylcyclohexanamin, Citrat (1:0.85), unpolares Diastereomer**

**Beispiel 128: 4-(5-Methoxy-3-methyl-1H-indol-2-yl)-N,N-dimethyl-1-phenylcyclohexanamin, Citrat (4:3), polares Diastereomer**

**4-(5-Methoxy-3-methyl-1H-indol-2-yl)-N,N-dimethyl-1-phenylcyclohexanamin (unpolares und polares Diastereomer)**

Die freie Base aus Beispiel **29** (385 mg, 1,07 mmol) wurde in HBr/Eisessig (33 % HBr, 15 ml) suspendiert. Anschließend wurde zu dem Ansatz bei RT Sn-Pulver (1,8 g, 15 mmol) innerhalb von 30 min portionsweise gegeben. Nach Beendigung der Zugabe wurde das Reaktionsgemisch weitere 30 min bei RT gerührt. Dabei entstand eine klare

Lösung. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (60 ml) basisch gemacht. Das erhaltene wässrige Gemisch wurde mit Dichlormethan (4 x 20 ml) extrahiert. Die vereinigten organischen Phasen wurden mit Wasser (50 ml) gewaschen, über $MgSO_4$ getrocknet und anschließend eingeengt. Der erhaltene Rückstand (360 mg) wurde in siedendem Methanol (30 ml) aufgekocht, wobei nicht alle Substanz in Lösung ging. Das Gemisch wurde auf RT gebracht und zur Vervollständigung der Kristallisation 17 h in den Kühlschrank gestellt. 4-(5-Methoxy-3-methyl-1H-indol-2-yl)-N,N-dimethyl-1-phenylcyclohexanamin ((163 mg (42 %)), unpolares Diastereoisomer) wurde durch Filtration wurde als weißer Feststoff mit einem Schmelzpunkt 156-163 °C (aus Isopropanol) abgetrennt. Die methanolische Mutterlauge wurde eingeengt und durch Säulenchromatographie [Kieselgel 60 G (10 g); EtOAc/ EtOH 1 : 1 (150 ml)] gereinigt. Das polarere Diastereomer wurde so in einer Ausbeute von 82 mg (21 %,) als weißer Feststoff mit einem Schmelzpunkt von 227-236 °C erhalten.

**4-(5-Methoxy-3-methyl-1H-indol-2-yl)-N,N-dimethyl-I-phenylcyclohexanamin, Citrat (1:0.85), unpolares Diastereomer**

4-(5-Methoxy-3-methyl-1H-indol-2-yl)-N,N-dimethyl-1-phenylcyclohexanamin (unpolares Diastereomer) (156 mg, 0,43 mmol) wurde in der Siedehitze in Isopropanol (10 ml) gelöst und mit Citronensäure (82 mg, 0,43 mmol), gelöst in heißem Isopropanol (1 ml), versetzt. Beim Abkühlen fiel ein Niederschlag aus. Zur Vervollständigung der Fällung wurde der Ansatz auf 5°C abgekühlt (Kühlschrank) und 17 h bei dieser Temperatur belassen. Der Niederschlag wurde mittels einer Fritte abgetrennt und anschließend getrocknet. Beispiel **127** wurde so in einer Ausbeute von 175 mg (75 %) mit einem Schmelzpunkt von 221-225 °C erhalten.

**4-(5-Methoxy-3-methyl-1H-indol-2-yl)-N,N-dimethyl-1-phenylcyclohexanamin, Citrat (4:3), polares Diastereomer**

4-(5-Methoxy-3-methyl-1H-indol-2-yl)-N,N-dimethyl-1-phenylcyclohexanamin (polares Diastereomer, 82 mg, 0,22 mmol) wurde in der Siedehitze in Isopropanol (10 ml) gelöst und mit Citronensäure (41 mg, 0,22 mmol), gelöst in heißem Isopropanol (1 ml), versetzt. Beim Abkühlen fiel ein Niederschlag aus. Zur Vervollständigung der Fällung wurde der Ansatz auf 5 °C abgekühlt (Kühlschrank) und 17 h bei dieser Temperatur belassen. Der Niederschlag wurde mittels einer Fritte abgetrennt und anschließend getrocknet. Beispiel **128** wurde so in einer Ausbeute von 85 mg (85 %) mit einem Schmelzpunkt von 146-151 °C erhalten.

**Beispiel 129: 1-Benzyl-N,N-dimethyl-4-(3-methyl-1H-indol-2-yl)cyclohexanamin, Citrat (1:1), polares Diastereomer**

**Beispiel 130: 1-Benzyl-N,N-dimethyl-4-(3-methyl-1H-indol-2-yl)cyclohexanamin, Citrat (2:1), unpolares Diastereomer**

**1-Benzyl-N,N-dimethyl-4-(3-methyl-1H-indol-2-yl)cyclohexanamin (unpolares und polares Diastereomer)**

Die freie Base aus Beispiel **30** (280 mg, 0,81 mmol) wurde in Ethanol (30 ml) gelöst, mit dem Katalysator (Pd/ Kohle 5%, 200 mg) versetzt und 3 h (Probe nach 1 h: kaum Umsetzung) bei RT und 3 bar Wasserstoffdruck hydriert. Der nach Abtrennung des Katalysators und Entfernung des Lösungsmittels erhaltene feste Rückstand (270 mg) wurde säulenchromatographisch (Laufmittel: EtOAc) aufgetrennt. 1-Benzyl-N,N-dimethyl-4-(3-methyl-1H-indol-2-yl)cyclohexanamin (unpolares Diastereoisomer) wurde so in einer Ausbeute von 71 mg (25 %) erhalten, das polarere Diastereoisomer fiel in einer Ausbeute von 145 mg (51 %) an.

**1-Benzyl-N,N-dimethyl-4-(3-methyl-1H-indol-2-yl)cyclohexanamin, Citrat (1:1), polares Diastereomer**

1-Benzyl-N,N-dimethyl-4-(3-methyl-1H-indol-2-yl)cyclohexanamin (polares Isomer, 130 mg, 0,38 mmol) wurde in der Siedehitze in Isopropanol (4 ml) gelöst und mit Citronensäure (80 mg, 0,41 mmol), gelöst in heißem Isopropanol (2 ml), versetzt. Die Lösung wurde auf 5 °C abgekühlt (Kühlschrank) und 17 h stehen gelassen. Der entstandene Niederschlag wurde mittels einer Fritte abgetrennt. Beispiel **129** wurde so in einer Ausbeute von 126 mg (61 %, *Schmelzpunkt:* 167-169 °C) erhalten.

**1-Benzyl-N,N-dimethyl-4-(3-methyl-1H-indol-2-yl)cyclohexanamin, Citrat (2:1), unpolares Diastereomer** 1-Benzyl-N,N-dimethyl-4-(3-methyl-1H-indol-2-yl)cyclohexanamin (unpolares Isomer, 68 mg, 0,20 mmol) wurde in der Siedehitze in Methanol (30 ml) gelöst und mit Citronensäure (60 mg, 0,31 mmol), gelöst in heißem Methanol (5 ml), versetzt. Die Lösung wurde auf 5 °C abgekühlt (Kühlschrank) und 17 h stehen gelassen. Der entstandene Niederschlag wurde mittels einer Fritte abgetrennt. Beispiel **130** wurde so in einer Ausbeute von 72 mg (63 %, *Schmelzpunkt:* 193-196

°C) erhalten.

**Beispiel 131: 1-Butyl-N,N-dimethyl-4-(3-methyl-1H-indol-2-yl)cyclohexanamin, Citrat (2:1), unpolares Diastereomer**

**Beispiel 132: 1-Butyl-N,N-dimethyl-4-(3-methyl-1H-indol-2-yl)cyclohexanamin, Citrat (1:1), polares Diastereomer**

**1-Butyl-N,N-dimethyl-4-(3-methyl-1H-indol-2-yl)cyclohexanamin (unpolares und polares Diastereomer)**

3-Methylindol (**Ind-10**, 524 mg, 4 mmol) wurde zusammen mit Keton **Ket-4** (788 mg, 4 mmol) in Dichlormethan (30 ml) gelöst und mit Trifluormethansulfonsäure (0,4 ml, 4,6 mmol) versetzt. Der Ansatz wurde 24 h bei RT gerührt. Anschließend wurde zu dem Ansatz Triethylsilan (2 ml, 12,6 mmol) gegeben. Das Reaktionsgemisch wurde 60 h bei RT gerührt. - Zur Aufarbeitung wurde das Reaktionsgemisch mit 2N NaOH (10 ml) versetzt. Das Gemisch wurde weitere 20 min gerührt. Nach Trennung der Phasen wurde die wässrige Phase mit Dichlormethan (3 x 20 ml) extrahiert. Die vereinigten organischen Extrakte wurden über $MgSO_4$ getrocknet und anschließend eingeengt. Das erhaltene Rohprodukt (1,8 g), das noch Triethylsilyl-Verbindungen enthielt, wurde säulenchromatisch (Laufmittel: Ethylacetat) gereinigt. 1-Butyl-N,N-dimethyl-4-(3-methyl-1H-indol-2-yl)cyclohexanamin (unpolares Isomer) wurde in einer Ausbeute von 122 mg (9 %) erhalten. Das polare Isomer fiel in einer Ausbeute von 230 mg (18 %) an.

**1-Butyl-N,N-dimethyl-4-(3-methyl-1H-indol-2-yl)cyclohexanamin (polares Diastereomer)**

Bsp. **31** (freie Base, 210 mg, 0,68 mg) wurde zusammen mit dem Katalysator (5-proz. Pd/C, 100 mg) in Methanol (50 ml) vorgelegt und bei RT und 3 bar Wasserstoffdruck hydriert. Nach 1 h Reaktionszeit war kein bzw. kaum Umsatz zu beobachten. Nach 6 h Hydrierung bei RT war kein Ausgangsprodukt mehr nachweisbar. - Zur Aufarbeitung wurde der Katalysator mittels einer Fritte abgetrennt und mit Methanol (2 x 20 ml) gewaschen. Nach Entfernung des Lösungsmittels wurde ein Rückstand von 198 mg erhalten. NMR Untersuchungen zeigten, daß es sich bei dem Rohprodukt um 1-Butyl-N,N-dimethyl-4-(3-methyl-1H-indol-2-yl)cyclohexanamin (polares Isomer) (94 % Ausbeute) in beinahe reiner Form handelte.

**1-Butyl-N,N-dimethyl-4-(3-methyl-1H-indol-2-yl)cyclohexanamin, Citrat (2:1), unpolares Diastereomer**

1-Butyl-N,N-dimethyl-4-(3-methyl-1H-indol-2-yl)cyclohexanamin (unpolares Diastereomer, 102 mg, 0,33 mmol) wurde in der Siedehitze in Isopropanol (4 ml) gelöst und mit Citronensäure (62 mg, 0,32 mmol), gelöst in heißem Isopropanol (1 ml), versetzt. Die Lösung wurde im Kühlschrank auf 5 °C abgekühlt und 3 h stehen gelassen. Der entstandene Niederschlag wurde mittels einer Fritte abgetrennt. Das Hemicitrat des unpolaren Isomers Bsp **131** wurde so in einer Ausbeute von 108 mg (80 %, *Schmelzpunkt:* 208-211 °C) erhalten.

**1-Butyl-N,N-dimethyl-4-(3-methyl-1H-indol-2-yl)cyclohexanamin, Citrat (1:1), polares Diastereomer**

1-Butyl-N,N-dimethyl-4-(3-methyl-1H-indol-2-yl)cyclohexanamin (polares Diastereomer, 200 mg, 0,63 mmol) wurde in der Siedehitze in Isopropanol (2 ml) gelöst und mit Citronensäure (120 mg, 0,63 mmol), gelöst in heißem Isopropanol (1 ml), versetzt. Nach Abkühlung der Lösung fiel ein klebriger Niederschlag aus, der bei der Trocknung in Vakuum zu einem glasigen Feststoff erstarrte. Die Substanz wurde in $H_2O$ (6 ml) aufgenommen und angerieben. Der entstandene Feststoff wurde mittels einer Fritte abgetrennt. Das Citrat des polaren Isomers Bsp **132** wurde so in einer Ausbeute von 152 mg (59 %, *Schmelzpunkt:* 124-129 °C) erhalten.

**Beispiel 133: 1-Benzyl-4-(3-cyclopropyl-1H-indol-2-yl)-N,N-dimethylcyclohexanamin, Citrat (1:1), unpolares Diastereomer**

**Beispiel 134: 1-Benzyl-4-(3-cyclopropyl-1H-indol-2-yl)-N,N-dimethylcyclohexanamin, Citrat (1:1), polares Diastereomer**

**1-Benzyl-4-(3-cyclopropyl-1H-indol-2-yl)-N,N-dimethylcyclohexanamin** (unpolares und polares Diastereoemer)

Die frei Base aus Beispiel 33 (210 mg, 0,567 mmol) wurde in Methanol (35 ml) gelöst und mit Palladium auf Kohle (5-proz.; 90 mg) versetzt. Die Reaktionsmischung wurde 5 h bei 3 bar hydriert. Der Katalysator wurde über Celite abgetrennt und das Filtrat eingeengt. Der feste Rückstand (185 mg) wurde chromatographisch aufgetrennt [Kieselgel 60 (40 g); Ethylacetat/Methanol 10 : 1 (250 ml), Ethylacetat/Methanol 4 : 1 (250 ml)]. 1-Benzyl-4-(3-cyclopropyl-1H-indol-2-yl)-N,N-dimethylcyclohexanamin (polareres Diastereoisomer) wurde **in** einer Ausbeute von 50 % (105 mg) mit einem Schmelzpunkt von 65-70 °C gewonnen. Das unpolarere Diastereoisomer wurde in einer Ausbeute von 26 % (54 mg) zusammen mit Nebenprodukten isoliert. Diese Fraktion wurde nochmals chromatographisch [Kieselgel 60 (20 g); Trichlormethan/Methanol 40 : 1 (250 ml)] gereinigt. Das unpolarere Diastereoisomer wurde dabei in einer Ausbeute von 43 mg (20 %) mit Spuren einer Verunreinigung erhalten und so für die Citratherstellung eingesetzt.

**1-Benzyl-4-(3-cyclopropyl-1H-indol-2-yl)-N,N-dimethylcyclohexanamin, Citrat (1:1), unpolares Diastereomer**

1-Benzyl-4-(3-cyclopropyl-1H-indol-2-yl)-N,N-dimethylcyclohexanamin (unpolareres Isomer) (41 mg, 0,11 mmol, verunreinigt) wurde bei 60 °C in Ethanol (7 ml) gelöst und mit einer ethanolischen Lösung (3 ml) von Citronensäure (24 mg, 0,12 mmol) versetzt. Nach einer Reaktionszeit von 2 h bei Raumtemperatur wurde der farblose Feststoff durch Filtration abgetrennt und mit Ethanol (2 ml) und Diethylether (2 ml) gewaschen. Beispiel 133 wurde in einer Ausbeute von 48 % (30 mg) mit einem Schmelzpunkt von 237-242 °C erhalten.

**1-Benzyl-4-(3-cyclopropyl-1H-indol-2-yl)-N,N-dimethylcyclohexanamin, Citrat (1:1), polares Diastereomer**

1-Benzyl-4-(3-cyclopropyl-1H-indol-2-yl)-N,N-dimethylcyclohexanamin (polareres Isomer) (105 mg, 0,29 mmol) wurde bei 40 °C in Ethanol (5 ml) gelöst und mit einer ethanolischen Lösung (3 ml) von Citronensäure (62 mg, 0,32 mmol) versetzt. Nach einer Reaktionszeit von 16 h bei Raumtemperatur war kein Salz ausgefallen. Die Reaktionsmischung wurde eingeengt. Der Rückstand wurde in Ethanol (1,5 ml) gelöst. Dann wurde Diethylether (20 ml) hinzugefügt. Nach 30 min wurde der farblose Feststoff durch Filtration abgetrennt und mit Diethylether (2 × 3 ml) gewaschen. Das Citrat (Bsp 134) wurde in einer Ausbeute von 56 % (91 mg) isoliert

**Beispiel 135: 1-Butyl-4-(3-cyclopropyl-1H-indol-2-yl)-N,N-dimethylcyclohexanamin Hydrochlorid, polares Diastereomer**

Bsp. 34 (337 mg, 1,0 mmol) wurde in Methanol (35 ml) gelöst und mit Palladium auf Kohle (5-proz.; 144 mg) versetzt. Die Reaktionsmischung wurde 4 h bei 3 bar hydriert. Da keine Umsetzung sichtbar war, wurde nochmals Katalysator (Pd/C, 5-proz., 144 mg) hinzugefügt und 2 h bei 3 bar hydriert. Nach 2 h war kein Edukt mehr nachweisbar. Der Katalysator wurde über Celite abgetrennt und das Filtrat eingeengt. Der feste Rückstand (297 mg) wurde chromatographisch aufgetrennt [Kieselgel 60 (50 g); Methanol (600 ml)]. Es wurde 1-Butyl-4-(3-cyclopropyl-1 H-indol-2-yl)-N, N-dimethylcyclohexanamin (polares Diastereomer)in einer Ausbeute von 62 % (209 mg) mit einem Schmelzpunkt von 257-263 °C gewonnen. Ein weiteres Diastereoisomer wurde nicht erhalten.
1-Butyl-4-(3-cyclopropyl-1H-indol-2-yl)-N,N-dimethylcyclohexanamin (polares Diastereomer) (135 mg, 0,398 mmol) wurde in Trichlormethan (15 ml) und Methanol (10 ml) gelöst und mit einer 5N Salzsäure (0,16 ml, 0,8 mmol) in Propan-2-ol versetzt. Die lilafarbene Lösung wurde eingeengt und mit Diethylether (40 ml) versetzt. Nach 30 min wurde das Hydrochlorid durch Filtration und Waschen mit Diethylether (2 × 2 ml) als lilafarbener Feststoff abgetrennt. Beispiel 135 wurde in einer Ausbeute von 56 % (84 mg) mit einem Schmelzpunkt von 274-276 °C gewonnen.

**Beispiel 136: 4-(3-Cyclopropyl-1H-indol-2-yl)-N,N-dimethyl-1-phenylcyclohexanamin, Citrat (2:1**), **polares Diastereomer**

Bsp. 35 (329 mg, 0,92 mmol) wurde in Methanol (100 ml) gelöst und mit Palladium auf Kohle (5-proz.; 124 mg) versetzt. Die Reaktionsmischung wurde 5 h bei 3 bar hydriert. Da die Umsetzung nicht vollständig war, wurde nochmals Katalysator (40 mg) hinzugefügt und weitere 16 h hydriert. Der Katalysator wurde über Celite abgetrennt und das Filtrat eingeengt. Der feste Rückstand (306 mg) wurde chromatographisch aufgetrennt [Kieselgel 60 (45 g); Ethylacetat/Methanol 4 : 1 (500 ml), Methanol (200 ml)]. 4-(3-Cyclopropyl-1H-indol-2-yl)-N,N-dimethyl-1-phenylcyclohexanamin (polares Diastereoisomer) wurde in einer Ausbeute von 62 % (205 mg) gewonnen.4-(3-Cyclopropyl-1H-indol-2-yt)-N.N-dimethyl-1-phenylcyclohexanamin (polares Diastereoisomer) (246 mg, 0,686 mmol) wurde bei 60 °C in Ethanol (36 ml) gelöst und mit einer ethanolischen Lösung (3 ml) von Citronensäure (146 mg, 0,76 mmol) versetzt. Nach einer Reaktionszeit von 16 h bei Raumtemperatur wurde der farbloses Feststoff durch Filtration abgetrennt und mit Ethanol (2 ml) gewaschen (150 mg). Das Filtrat wurde auf 5 ml eingeengt und mit Diethylether (50 ml) versetzt. Nach 30 min wurde weiterer Feststoff abgetrennt und mit Diethylether (2 × 5 ml) gewaschen (91 mg). Beide Fraktionen wurden vereinigt. Beispiel 136 wurde so in einer Ausbeute von 64 % mit einem Schmelzpunkt von 238-245 °C erhalten.

**Beispiel 137: Methyl 2-(2-(4-(dimethylamino)-4-phenylcyclohexyl)-1H-indol-3-yl)acetat, Citrat (2:1), unpolares Diastereomer**

**Beispiel 138: Methyl 2-(2-(4-(dimethylamino)-4-phenylcyclohexyl)-1H-indol-3-yl)acetat, Citrat (2:1), polares Diastereomer**

**(±)-Methyl 2-(2-(4-(dimethylamino)-4-phenylcyclohex-1-enyl)-1H-indol-3-yl)acetat**

Methyl 2-(1H-indol-3-yl)acetat (Ind-47, 757 mg, 4 mmol) wurde zusammen mit Keton **Ket-10** (868 mg, 4 mmol) in Dichlormethan (80 ml) gelöst und mit Trifluormethansulfonsäure (540 μl, 6 mmol) versetzt. Der Ansatz wurde 16 h bei RT gerührt. Zur Aufarbeitung wurde das Reaktionsgemisch mit 2N NaOH (50 ml) versetzt und 20 min bei RT gerührt. Nach Abtrennung der organischen Phase wurde die wässrige Phase mit Dichlormethan (3 × 20 ml) extrahiert. Die vereinigten organischen Extrakte wurden über $Na_2SO_4$ getrocknet und anschließend eingeengt. Das erhaltene Rohprodukt (1,52 g) konnte durch Säulenchromatographie [Kieselgel 60 (100 g); MeOH (500 ml)] gereinigt werden. (±)-Methyl 2-(2-(4-(dimethylamino)-4-phenylcyclohex-1-enyl)-1H-indol-3-yl)acetat wurde in einer Ausbeute von 936 mg (60 %) als gelber Feststoff erhalten.

**Methyl 2-(2-(4-(dimethylamino)-4-phenylcyclohexyl)-1H-indol-3-yl)acetat (unpolares und polares Diastereomer)**

(±)-Methyl 2-(2-(4-(dimethylamino)-4-phenylcyclohex-1-enyl)-1H-indol-3-yl)acetat (936 mg, 2,4 mmol) wurde in HBr/Eisessig (33 % HBr, 30 ml) gelöst. Anschließend wurde zu dem Ansatz bei RT Sn-Pulver (2,88 g, 24 mmol) innerhalb von 1 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von 2N NaOH (100 ml) basisch gemacht. Das erhaltene wässrige Gemisch wurde mit Dichlormethan extrahiert (4 × 40 ml). Die vereinigten organischen Phasen wurden über $Na_2SO_4$ getrocknet und anschließend eingeengt. Das erhaltene Rohprodukt (900 mg) wurde durch Säulenchromatographie [Kieselgel 60 (100 g); MeOH (1000 ml)] gereinigt. Methyl 2-(2-(4-(dimethylamino)-4-phenylcyclohexyl)-1H-indol-3-yl)acetat (unpolareres Diastereomer) wurde in einer Ausbeute von 290 mg (31 %), das polarere Diastereomer in einer Ausbeute von 200 mg (21 %) erhalten. Beide Produkte waren farblose Öle.

**Methyl 2-(2-(4-(dimethylamino)-4-phenylcyclohexyl)-1H-indol-3-yl)acetat, Citrat(2:1), unpolares Diastereomer**

Methyl 2-(2-(4-(dimethylamino)-4-phenylcyclohexyl)-1H-indol-3-yl)acetat (unpolareres Diastereomer) (290 mg, 0,74 mmol) wurde in heißem Dichlormethan (150 ml) gelöst und mit Citronensäure (143 mg, 0,74 mmol) versetzt. Die klare Lösung wurde 16 h bei 4 °C stehen gelassen. Der ausgefallene farblose Feststoff wurde abgesaugt und getrocknet. Beispiel 137 wurde in einer Ausbeute von 250 mg (67 %) mit einem Schmelzpunkt von 226-229 °C erhalten.
$^1$*H NMR (400 MHz, DMSO-$d_6$) δ ppm:* 1.52-1.73 (m, 4H), 2.12 (s, 2H), 2.43-2.75 (m, 4H), 2.76-2.88 (m, 2H), 2.90-3.02 (m, 2H), 3.17 (s, 1H), 3.57 (s, 3H), 3.71 (s, 1H), 6.87-7.07 (m, 2H), 7.23-7.51 (m, 7H), 10.76 (s, 1H)
$^{13}$*C NMR (101 MHz, DMSO-$d_6$) δ ppm:* 27.0, 29.5, 32.7. 34.7, 37.8, 43.2, 51.4, 56.0, 72,0, 102.0, 110.9, 117.5, 118.3, 120.1, 126.8, 127.5(s,1C), 128.0, 135.2, 141.4, 171.2, 172,1, 175,3

**Methyl 2-(2-(4-(dimethylamino)-4-phenylcyclohexyl)-1H-indol-3-yl)acetat, Citrat (2:1), polares Diastereomer**

[Methyl 2-(2-(4-(dimethylamino)-4-phenylcyclohexyl)-1H-indol-3-yl)acetat (polareres Diastereomer) (200 mg, 0,51 mmol) wurde in heißem Dichlormethan (100 ml) gelöst und mit Citronensäure (99 mg, 0,96 mmol) versetzt. Die klare Lösung wurde 16 h bei 4 °C stehen gelassen. Der ausgefallene farblose Feststoff wurde abgesaugt und getrocknet. Beispiel 138 wurde in einer Ausbeute von 170 mg (57 %) mit einem Schmelzpunkt von 190-193 °C erhalten.
$^1$*H NMR (400 MHz, DMSO-$d_6$)* öppm: 1.38-1.56 (m, 2H), 1.73-1.90 (m, 4H), 2.22 (s, 6H), 2.46-2,62 (m, 4H), 2.85-3.02 (m, 3H), 3.56 (s, 3H), 3.66 (s, 2H), 6.83-7.01 (m, 1H), 7.17 (d, J = 7.78 Hz, 1H), 7.39-7.46 (m, 1 ), 7.48-7.63 (m, 4H), 10.54 (s, 1H)
$^{13}$C *NMR (101 MHz, DMSO-$d_6$) δ ppm:* 28.5, 29.4, 31.9, 35.3, 37.7, 44.2, 51.4, 64.4, 71.2, 102.2, 110.7, 117.4, 118.3, 120.1, 127.8, 127.9, 128.3. 128.6, 133.8, 135.0, 140.4, 171.2. 172.0, 176,8

**Beispiel 139: 1-Benzyl-4-(3-(cyclohexylmethyl)-1H-indol-2-yl)-N,N-dimethylcyclohexanamin, Citrat (2:1), unpolares Diastereomer**

**Beispiel 140: 1-Benzyl-4-(3-(cyclohexylmethyl)-1H-indol-2-yl)-N,N-dimethylcyclohexanamin, Citrat (1:1), polares Diastereomer**

**1-Benzyl-4-(3-(cyclohexylmethyl)-1H-indol-2-yl)-N,N-dimethylcyclohexanamin (unpolares und polares Diastereomer)**

Bsp. 36 (705 mg, 1,522 mmol) wurde in Bromwasserstoff/Eisessig (33 % HBr, 35 ml) gelöst. Anschließend wurde zu dem Ansatz bei Raumtemperatur Zinn-Pulver (1,808 g, 15,23 mmol) innerhalb von 40 min portionsweise gegeben und der Ansatz noch 20 min gerührt. Anschließend wurde das Gemisch mit Wasser (300 ml) verdünnt. Der Ansatz wurde 1 h bei 5 °C gerührt. Das Produkt fiel als Hydrobromid aus. Dieses wurde abfiltriert und mit Wasser (2 × 5 ml) gewaschen. Dem rosafarbenen, nassen Feststoff (5,0 g) wurde 1 N NaOH (50 ml) zugesetzt. Das Gemisch wurde mit Dichlormethan (3 × 30 ml) extrahiert. Die vereinigten organischen Phasen wurden mit $Na_2SO_4$ getrocknet und filtriert. Die flüchtigen Bestandteile wurden im Vakuum vollständig entfernt. Das gelbe Öl (630 mg) wurde mit Methanol (10 ml) 1 h bei 5 °C gerührt. 1-Benzyl-4-(3-(cyclohexylmethyl)-1H-indol-2-yl)-N,N-dimethylcyclohexanamin (unpolareres Diasteroisomer) fiel als weißer Feststoff aus. Dieser wurde abgesaugt und mit Methanol (2 × 1 ml) gewaschen (326 mg, 48 %. Schmp.: 120 - 126 °C. Das Filtrat wurde eingeengt (165 mg Öl). Nach chromatographischer Trennung dieses Gemisches [Kieselgel 60 (20 g); Chloroform/Methanol 40 : 1 (410 ml)] wurde das polarere Diastereoisomer (95 mg, 15 %) als gelbes Öl gewonnen.

**1-Benzyl-4-(3-(cyclohexylmethyl)-1H-indol-2-yl)-N,N-dimethylcyclohexanamin, Citrat (2:1), unpolares Diastereomer**

1-Benzyl-4-(3-(cyclohexylmethyl)-1H-indol-2-yl)-N,N-dimethylcyclohexanamin (unpolareres Diasteroisomer, 285 mg, 0,641 mmol) wurde in heißem Ethanol (12 ml) gelöst und mit einer ebenfalls heißen, ethanolischen Citronensäure-Lösung (135 mg, 0,703 mmol in 2 ml) versetzt. Anschließend wurde die Mischung über Nacht bei Raumtemperatur gerührt, dann filtriert und mit etwas Ethanol gewaschen. Beispiel 139 wurde als weisser Feststoff in einer Ausbeute von 99 % (334 mg) mit einem Schmelzpunkt von 208-213 °C erhalten.

**1-Benzyl-4-(3-(cyclohexylmethyl)-1H-indol-2-yl)-N,N-dimethylcyclohexanamin, Citrat (1:1**), **polares Diastereomer**

1-Benzyl-4-(3-(cyclohexylmethyl)-1H-indol-2-yl)-N,N-dimethylcyclohexanamin (polareres Diastereomer) (92 mg, 0,215 mmol) und Citronensäure (44 mg, 0,229 mmol) wurden in heißem Ethanol (0,5 ml) gelöst. Zur klaren Lösung wurde bei Raumtemperatur langsam Diethylether (10 ml) zugetropft. Das Gemisch wurde bei Raumtemperatur über Nacht gerührt. Es fiel ein weißer Feststoff aus, der filtriert und mit Diethylether gewaschen wurde. Bsp. **140** wurde in einer Ausbeute von 66 % (88 mg) mit einem Schmelzpunkt von 110-115 °C erhalten.

**Beispiel 141: 1-Benzyl-4-(3-benzyl-1H-indol-2-yl)-N,N-dimethylcyclohexanamin, Citrat (1:1), unpolares Diastereomer**

**Beispiel 142: 1-Benryl-4-(3-benzyl-1H-indol-2-yl)-N,N-dimethylcyclohexanamin, Citrat (1:1), polares Diastereomer**

**1-Benzyl-4-(3-benzyl-1H-indol-2-yl)-N,N-dimethylcyclohexanamin (unpolares und polares Diastereomer)**

Bsp. 37 (750 mg, 1,64 mmol) wurde in Bromwasserstoff/Eisessig (33 % HBr, 40 ml) gelöst. Anschließend wurde zu dem Ansatz bei Raumtemperatur Zinn-Pulver (1,950 g, 16,43 mmol) innerhalb von 40 min portionsweise gegeben. Der Ansatz wurde noch 20 min gerührt. Anschließend wurde das Gemisch mit Wasser (300 ml) verdünnt. Der Ansatz wurde 1 h bei 5 °C gerührt. Das Produkt fiel als Hydrobromid aus. Dieses wurde abfiltriert und mit Wasser (2 × 5 ml) gewaschen. Dem rosafarbenen, feuchten Feststoff (5,0 g) wurde 1 N NaOH (50 ml) zugesetzt. Das Gemisch wurde mit Dichlormethan (3 × 30 ml) extrahiert. Die vereinigten organischen Phasen wurden mit $Na_2SO_4$ getrocknet und dann filtriert. Die flüchtigen Bestandteile des Filtrates wurden im Vakuum vollständig entfernt. Der Feststoff (615 mg) wurde mit Methanol (6 ml) über Nacht bei Raumtemperatur gerührt. 1-Benzyl-4-(3-benzyl-1H-indol-2-yl)-N,N-dimethylcyclohexanamin (unpolareres Diasteroisomer) fiel als beigefarbiger Feststoff aus. Es wurde abfiltriert und mit Methanol (2 × 1 ml) und Diethylether (2 × 1 ml) gewaschen (277 mg). Das Filtrat wurde eingeengt (330 mg Öl). Nach chromatographischer

Trennung dieses Oles [Kieselgel 60 (40 g); Ethylacetat (1500 ml)] wurden weitere Mengen des unpolareren Diastereoisomers (61 mg, insgesamt 49 %. Schmp.: 140-143 °C) und das polarere Diastereoisomer (207 mg, 30 %. Öl) gewonnen.

**1-Benzyl-4-(3-benzyl-1H-indol-2-yl)-N,N-dimethylcyclohexanamin, Citrat (1:1), unpolares Diastereomer**

**1-Benzyl-4-(3-benzyl-1H-indol-2-yl)-N,N-dimethylcyclohexanamin** (unpolareres Diasteroisomer) (304 mg, 0,719 mmol) wurde in einer Mischung von Dichlormethan (4 ml) und Ethanol (2 ml) gelöst und mit ethanolischer Citronensäure-Lösung (145 mg, 0,755 mmol in 4 ml) versetzt. Die Lösung wurde auf ca. 2 ml eingeengt. Der Feststoff wurde abgesaugt und mit Ethanol (2 × 0,5 ml) gewaschen. Beispiel **141** wurde so in einer Ausbeute von 72 % (320 mg) als weißer Feststoff mit einem Schmelzpunkt von 214-217 °C erhalten.

**1-Benzyl-4-(3-benzyl-1H-indol-2-yl)-N,N-dimethylcyclohexanamin, Citrat (1:1), polares Diastereomer)**

1-Benzyl-4-(3-benzyl-1H-indol-2-yl)-N,N-dimethylcyclohexanamin (polareres Diasteroisomer) (85 mg, 0,201 mmol) und Citronensäure (43 mg, 0.224 mmoi) wurden in Ethanol (2 ml) gelöst. Zur klaren Lösung wurde bei Raumtemperatur langsam Diethylether (20 ml) zugetropft. Das Gemisch wurde bei Raumtemperatur 1 h gerührt. Ein beigefarbener Feststoff fiel aus, der abfiltriert und mit Diethylether (2 × 1 ml) gewaschen wurde. Beispiel 142 wurde so in einer Ausbeute von 69 % (76 mg) mit einem Schmelzpunkt von 110-115 °C erhalten.

**Beispiel 143: 1-Butyl-4-(3-(cyclohexylmethyl)-1H-indol-2-yl)-N,N-dimethylcyclohexanamin, Citrat (2:1), unpolares Diastereomer**

**Beispiel 144: 1-Butyl-4-(3-(cyclohexylmethyl)-1H-indol-2-yl)-N,N-dimethylcyclohexanamin Hydrochlorid (1:1), polares Diastereomer**

**1-Butyl-4-(3-(cyclohexylmethyl)-1H-indol-2-yl)-N,N-dimethylcyclohexanamin (unpolares und polares Diastereomer)**

Bsp. 38 (777 mg, 1,811 mmol) wurde in Bromwasserstoff/Eisessig (33 % HBr, 40 ml) gelöst. Anschließend wurde zu dem Ansatz bei Raumtemperatur Zinn-Pulver (2,150 g, 18,1 mmol) innerhalb von 40 min portionsweise gegeben und der Ansatz noch 20 min gerührt. Anschließend wurde das Gemisch mit Wasser (250 ml) verdünnt. Der Ansatz wurde 1 h bei 5 °C gerührt. Das Produkt fiel als Hydrobromid aus. Dieses wurde abfiltriert und mit Wasser (2 × 5 ml) gewaschen. Zum beigefarbigen nassen Feststoff (5,0 g) wurde 1 N NaOH (50 ml) zugesetzt. Das Gemisch wurde mit Dichlormethan (3 × 30 ml) extrahiert. Die vereinigten organischen Phasen wurden mit $Na_2SO_4$ getrocknet, filtriert und die flüchtigen Bestandteile vollständig im Vakuum entfernt. Es blieb ein gelbes Öl (650 mg) zurück. Nach chromatographischer Trennung dieses Gemisches [Kieselgel 60 (80 g); Ethylacetat/Methanol 10 : 1 (1100 ml), Ethylacetat/Methanol 2 : 1 (540 ml)] wurden 1-Butyl-4-(3-(cyclohexylmethyl)-1H-indol-2-yl)-N,N-dimethylcyclohexanamin (unpolareres Diastereoisomer, 306 mg, 43 %, Schmp.: 155-165 °C) und das polarere Diastereoisomer (163 mg, 23 %, Schmp.: 258-264 °C) als weiße Feststoffe gewonnen.

**1-Butyl-4-(3-(cyclohexylmethyl)-1H-indol-2-yl)-N,N-dimethylcyclohexanamin, Citrat (2:1), unpolares Diastereomer**

1-Butyl-4-(3-(cyclohexylmethyl)-1H-indol-2-yl)-N,N-dimethylcyclohexanamin (unpolareres Diastereoisomer) (277 mg, 0,702 mmol) und Citronensäure (142 mg, 0,739 mmol) wurden in heißem Ethanol (20 ml) gelöst. Es fiel bei Raumtemperatur ein Feststoff aus. Das Gemisch wurde über Nacht bei 5 °C stehen gelassen, dann filtriert und mit etwas Ethanol gewaschen. Beispiel 143 wurde in einer Ausbeute von 72 % (298 mg) mit einem Schmelzpunkt von 228-232 °C erhalten..

**1-Butyl-4-(3-(cyclohexylmethyl)-1H-indol-2-yl)-N,N-dimethylcyclohexanamin Hydrochlorid (1:1), polares Diastereomer**

1-Butyl-4-(3-(cyclohexylmethyl)-1H-indol-2-yl)-N.N-dimethylcyclohexanamin (polares Diastereomer) (135 mg, 0,342 mmol) und Citronensäure (71 mg, 0,370 mmol) wurden in heißem Ethanol (4 ml) gelöst. Zur klaren Lösung wurde bei Raumtemperatur langsam Diethylether (26 ml) zugetropft. Das Gemisch wurde bei Raumtemperatur über Nacht gerührt. Der weiße Feststoff wurde abfiltriert und mit Diethylether gewaschen. wurde in einer Ausbeute von 50 % (101 mg) mit einem Schmelzpunkt von 268-272 °C erhalten. *Dieses Produkt wurde als Hydrochlorid identifiziert.*

**Beispiel 145: 4-(3-Benzyl-1H-indol-2-yl)-1-butyl-N,N-dimethylcyclohexanamin, Citrat (1:1), unpolares Diastereomer**

**Beispiel 146: 4-(3-Benzyl-1H-indol-2-yl)-1-butyl-N,N-dimethylcyclohexanamin, Citrat (1:1), polares Diastereomer**

**4-(3-Benzyl-1H-indol-2-yl)-1-butyl-N,N-dimethylcyclohexanamin (unpolares und polares Diastereomer)**

Bsp. 39 (438 mg, 1,133 mmol) wurde in Bromwasserstoff/Eisessig (33 % HBr, 25 ml) gelöst. Anschließend wurde zu dem Ansatz bei Raumtemperatur Zinn-Pulver (1,345 g, 11,330 mmol) innerhalb von 40 min portionsweise gegeben und der Ansatz noch 20 min gerührt. Anschließend wurde das Gemisch mit Wasser (100 ml) verdünnt. Der Ansatz wurde 1 h bei 5 °C gerührt. Das Produkt fiel als Hydrobromid aus. Dieses wurde abfiltriert und mit Wasser (2 × 5 ml) gewaschen. Dem feuchten gelben Feststoff (4,0 g) wurde 1 N NaOH (50 ml) zugesetzt. Das Gemisch wurde mit Dichlormethan (3 × 30 ml) extrahiert. Die vereinigten organischen Phasen wurden mit $Na_2SO_4$ getrocknet und filtriert. Die flüchtigen Bestandteile wurden im Vakuum vollständig entfernt. Es blieb ein Öl (425 mg) zurück. Nach chromatographischer Trennung dieses Gemisches [Kieselgel 60 (40 g); Ethylacetat (500 ml), Ethylacetat/Methanol 4 : 1 (750 ml)] wurde 4-(3-Benzyl-1H-indol-2-yl)-1-butyl-N,N-dimethylcyclohexanamin (unpolarere Diastereoisomer, 210 mg, unrein) und das polarere Diastereoisomer (178 mg, 40 %) als gelbes Öl gewonnen.
Das unpolarere Diastereoisomer wurde weiter gereinigt: Es wurde mit heißem Methanol (6 ml) versetzt und über Nacht bei -10 °C stehen gelassen. Der ausgeschiedenen farblose Feststoff (144 mg, 33 %, Schmelzpunkt von 72-78 °C und 97-99 "C) wurde abgesaugt und mit kaltem Methanol (2 × 0,5 ml) gewaschen.

**4-(3-Benzyl-1H-indol-2-yl)-1-butyl-N,N-dimethylcyclohexanamin, Citrat(1:1), unpolares Diastereomer**

4-(3-Benzyl)-1H-indol-2-yl)-1-butyl-N,N-dimethylcyclohexanamin (unpolareres Diastereoisomer, 121 mg, 0,311 mmol) in heißem Ethanol (2 ml) gelöst und mit ethanolischer Citronensäure-Lösung (63 mg, 0,328 mmol in 1 ml) versetzt. Die Mischung wurde 2 h bei Raumtemperatur gerührt. Der ausgefallene Feststoff wurde abgesaugt und mit Diethylether (2 × 1 ml) gewaschen. Beispiel 145 wurde in einer Ausbeute von 61 % (111 mg) mit einem Schmelzpunkt von 167-171 °C erhalten.

**4-(3-Benzyl-1H-indol-2-yl)-1-butyl-N,N-dimethylcyclohexanamin, Citrat (1:1), polares Diastereomer**

4-(3-Benzyl-1H-indol-2-yl)-1-butyl-N,N-dimethylcyclohexanamin (polareres Diastereoisomer, 157 mg, 0,404 mmol) wurde in Diethylether (10 ml) gelöst und mit ethanolischer Citronensäure-Lösung (83 mg, 0,432 mmol in 0,5 ml) versetzt. Die Mischung wurde 2 h bei Raumtemperatur gerührt. Der ausgefallene Feststoff wurde abgesaugt und mit Diethylether (2 × 1 ml) gewaschen. Beispiel 146 (beigefarbener Feststoff ) wurde in einer Ausbeute von 55 % (130 mg) mit einem Schmelzpunkt von 90-95 °C erhalten (HPLC-Reinheit ca. 88%)

**Beispiel 147: 4-(3-(Cyclohexylmethyl)-1H-indol-2-yl)-N,N-dimethyl-1-phenylcyclohexanamin, Citrat (2:1), unpolares Diastereomer**

**Beispiel 148: 4-(3-(Cyclohexylmethyl)-1H-indol-2-yl)-N,N-dimethyl-1-phenylcyclohexanamin, Citrat (1:1), polares Diastereomer**

**4-(3-(Cyclohexylmethyl)-1H-indol-2-yl)-N,N-dimethyl-1-phenylcyclohexanamin(unpolares und polares Diastereomer)**

Bsp. 40 (913 mg, 2,033 mmol) wurde in Bromwasserstoff/Eisessig (33 % HBr, 46 ml) gelöst. Anschließend wurde zu dem Ansatz bei Raumtemperatur Zinn-Pulver (2,415 g, 20,344 mmol) innerhalb von 40 min portionsweise gegeben und der Ansatz noch 20 min gerührt. Anschließend wurde das Gemisch mit Wasser (300 ml) verdünnt. Der Ansatz wurde 1 h bei 5 °C gerührt. Das Produkt fiel als Hydrobromid aus. Dieses wurde abfiltriert und mit Wasser (2 × 5 ml) gewaschen. Zum rosafarbigen nassen Feststoff (5,7 g) wurde 1 N NaOH (50 ml) zugesetzt. Das Gemisch wurde mit Dichlormethan (3 × 30 ml) extrahiert. Die vereinigten organischen Phasen wurden mit $Na_2SO_4$ getrocknet, filtriert und die flüchtigen Bestandteile vollständig im Vakuum entfernt. Es blieb ein gelber Feststoff (776 mg) zurück. Nach chromatographischer Trennung dieses Gemisches [Kieselgel 60 (80 g); Ethylacetat (500 ml), Ethylacetat/Methanol 20 : 1 (525 ml), Ethylacetat/Methanol 5 : 1 (600 ml), Ethylacetat/Methanol 2 : 1 (600 ml)] wurden 4-(3-(Cyclohexylmethyl)-1H-indol-2-yl)-N,N-dimethyl-1-phenylcyclohexanamin unpolareres Diastereoisomer) (472 mg, verunreinigt) und das polarere Diastereoisomer (269 mg, 32 %, Schmp.: 195-198 °C) als weiße Feststoffe gewonnen.
Das unpolarere Diastereomer wurde weiter gereinigt: Es wurde mit Methanol (15 ml) versetzt und über Nacht gerührt,

dann filtriert. Der Rückstand wurde mit Methanol (3 × 1 ml) gewaschen. Der weiße Feststoff wurde in einer Ausbeute von 40 % (336 mg) mit einem Schmelzpunkt von 206-209 °C erhalten ).

**4-(3-(Cyclohexylmethyl)-1H-indol-2-yl)-N,N-dimethyl-1-phenylcyclohexanamin, Citrat (2:1), unpolares Diastereomer**

4-(3-(Cyclohexylmethyl)-1H-indol-2-yl)-N,N-dimethyl-1-phenylcyclohexanamin (unpolares Diastereomer) (294 mg, 0,706 mmol) und Citronensäure (141 mg, 0,734 mmol) wurden in heißem Ethanol (7 ml) gelöst. Es fiel bei Raumtemperatur ein Festaoff aus. Das Gemisch wurde über Nacht bei Raumtemperatur gerührt, dann filtriert und mit etwas Ethanol gewaschen. Beispiel **147** wurde so in einer Ausbeute von 99 % (355 mg) mit einem Schmelzpunkt von 224-228 °C als weisser Feststoff erhalten.

**4-(3-(Cyclohexylmethyl)-1H-indol-2-yl)-N,N-dimethyl-1-phenylcyclohexanamin, Citrat (1:1), polares Diastereomer**

4-(3-(Cyclohexylmethyl)-1H-indol-2-yt)-N,N-dimethyl-1-phenylcyclohexanamin (polares Diastereomer) (245 mg, 0,588 mmol) und Citronensäure (122 mg, 0,635 mmol) wurden in heißem Ethanol (2 ml) gelöst. Zur klaren Lösung wurde bei Raumtemperatur langsam Diethylether (12 ml) zugetropft. Das Gemisch wurde bei Raumtemperatur über Nacht gerührt. Der weisse Feststoff wurde filtriert und mit Diethylether gewaschen. Beispiel 148 wurde so in einer Ausbeute von 81 % (290 mg) mit einem Schmelzpunkt von 190-192 °C erhalten.

**Beispiel 149: 4-(3-Benzyl-1H-indol-2-yl)-N,N-dimethyl-1-phenylcyclohexanamin, Citrat (2:1**), **unpolares Diastereomer**

**Beispiel 150: 4-(3-Benzyl-1H-indol-2-yl)-N,N-dimethyl-1-phenylcyclohexanamin, Citrat (1:1**), **polares Diastereomer**

**4-(3-Benzyl-1H-indol-2-yl)-N,N-dimethyl-1-phenylcyclohexanamin (unpolares und polares Diastereomer)**

Bsp. 41 (768 mg, 1,733 mmol) wurde in Bromwasserstoff/Eisessig (33 % HBr, 40 ml) gelöst. Anschließend wurde zu dem Ansatz bei Raumtemperatur Zinn-Pulver (2,060 g, 17,353 mmol) innerhalb von 40 min portionsweise gegeben und der Ansatz noch 20 min gerührt. Anschließend wurde das Gemisch mit Wasser (100 ml) verdünnt. Der Ansatz wurde 1 h bei 5 °C gerührt. Das Produkt fiel als Hydrobromid aus. Dieses wurde abfiltriert und mit Wasser (2 × 5 ml) gewaschen. Dem beigefarbigen feuchten Feststoff (4,3 g) wurde 1 N NaOH (50 ml) zugesetzt. Das Gemisch wurde mit Dichlormethan (3 × 30 ml) extrahiert. Die vereinigten organischen Phasen wurden mit $Na_2SO_4$ getrocknet und anschließend filtriert. Die flüchtigen Bestandteile wurden im Vakuum vollständig entfernt. Es blieb ein Öl (619 mg) zurück. Nach chromatographischer Trennung dieses Gemisches [Kieselgel 60 (80 g); Ethylacetat (500 ml), Ethylacetat/Methanol 20 : 1 (525 ml), Ethylacetat/Methanol 5 : 1 (600 ml), Ethylacetat/Methanol 2 : 1 (600 ml)] wurden 4-(3-Benzyl-1H-indol-2-yl)-N,N-dimethyl-1-phenylcyclohexanamin (unpolareres Diastereoisomer) (186 mg, 26 %, Schmp.: 194-199 °C) und das polarere Diastereoisomer (241 mg, 34 %, Schmp.: 205-209 °C) als beigefarbene Feststoffe gewonnen.

**4-(3-Benzyl-1H-indol-2-yl)-N,N-dimethyl-1-phenylcyclohexanamin, Citrat (2:1), unpolares Diastereomer ( 149)**

4-(3-Benzyl-1H-indol-2-yl)-N.N-dimethyl-1-phenylcyclohexanamin (unpolareres Diastereoisomer) (146 mg, 0,357 mmol) wurde in einer Mischung von Dichlormethan (4 ml) und Ethanol (0,5 ml) gelöst und mit ethanolischer Citronensäure-Lösung (73 mg, 0,380 mmol in 3,5 ml) versetzt. Die Lösung wurde auf ca. 2 ml eingeengt. Der ausgefallene Feststoff wurde abgesaugt und mit Ethanol (2 × 0,5 ml) gewaschen. Bsp. **149** wurde in einer Ausbeute von 96 % (174 mg) als weisser Feststoff mit einem Schmelzpunkt von 220-223 °C erhalten

**4-(3-Benzyl-1H-indol-2-yl)-N,N-dimethyl-1-phenylcyclohexanamin, Citrat (1:1), polares Diastereomer ( 150)**

4-(3-Benzyl-1H-indol-2-yl)-N,N-dimethyl-1-phenylcyclohexanamin (polareres Diastereoisomer) (150 mg, 0,367 mmol) wurde in Dichlormethan (10 ml) gelöst und mit ethanolischer Citronensäure-Lösung (77 mg, 0,401 mmol in 10 ml) versetzt. Die Lösung wurde auf ca. 2 ml eingeengt. Der ausgefallene Feststoff wurde abgesaugt und mit Ethanol (2 × 0,5 ml) und mit Diethylether (2 × 0,5 ml) gewaschen.Bsp. **150** wurde in einer Ausbeute von 74 % (163 mg) mit einem Schmelzpunkt von 140-143 °C erhalten.

**Beispiel 151: N,N-Dimethyl-1-phenyl-4-(3-(pyridin-2-ylmethyl)-1H-indol-2-yl)cyclohexanamin, unpolares Diastereomer**

**Beispiel 152: N,N-Dimethyl-1-phenyl-4-(3-(pyridin-2-ylmethyl)-1H-indol-2-yl)cyclohexanamin, polares Diastereomer**

**(±)-Dimethyl-(1-phenyl-4-(3-pyridin-2-ylmethyl-1*H*-indol-2-yl)cyclohex-3-enyl]amin** Zu einer Lösung von **Ind-49** (2.17 g, 10.4 mmol) und **Ket-10** (2.70 g, 12.5 mmol) in wasserfreiem Dichlormethan (80 ml) wurde unter Eiskühlung Trifluormethansulfonsäure (3.95 g, 2.30 ml, 26 mmol) gegeben. Das Reaktionsgemisch wurde 2 d bei Raumtemperatur gerührt und anschließend mit 0.5 *M* Natronlauge (50 ml) versetzt und 2 h bei Raumtemperatur gerührt. Die Phasen wurden getrennt, die wässrige Phase mit Dichlormethan (3 × 30 ml) extrahiert, die vereinigten organischen Phasen mit Natriumsulfat getrocknet und i. Vak. eingeengt. Das Rohprodukt (6.45 g) wurde durch Flashchromatographie (400 g, 20 × 7.6 cm) mit Chloroform / Methanol (9:1) gereinigt.
*Ausbeute* ((±)-Dimethyl-[1-phenyl-4-(3-pyridin-2-ylmethyl-1*H*-indol-2-yl)cyclohex-3-enyl]amin): 949 mg (22 %), beigefarbenes Öl
$^1$*H-NMR* (DMSO-$d_6$):1.67-1.85 (m 2H); 1.99 (s, 2H); 2.09 (s, 6H); 2.57-2.77 (m, 2H); 4.17 (s, 2H); 6.23 (s, 1H); 6.85-6.90 (m, 2H); 6.99 (dt, 1H, J = 7.0 und 1.0 Hz); 7.14 (ddd, 1H, J = 7.4. 4.8 und 0.8 Hz); 7.18-7.40 (m. 5H); 7.45 (d, 2H, J = 7.7 Hz); 7.55 (dt. 1H, J = 7.6 und 1.8 Hz); 8.45 (d, 1H, J = 3.9 Hz); 10.76 (1H, s).

**N,N-Dimethyl-1-phenyl-4-(3-(pyridin-2-ylmethyl)-1H-indol-2-yl)cyclohexanamin, unpolares Diastereomer (Bsp 151**) **und**

**N,N-Dimethyl-1-phenyl-4-(3-(pyridin-2-ylmethyl)-1H-indol-2-yl)cyclohexanamin, polares Diastereomer (Bsp 152)**

(±)-Dimethyl-[1-phenyl-4-(3-pyridin-2-ylmethyl-1H-indol-2-yl)cyclohex-3-enyl]amin (200 mg, 0.49 mmol) wurde durch einstündiges Rühren bei Raumtemperatur in einer 33 % Lösung von Bromwasserstoff in Eisessig gelöst. Die Lösung wurde anschließend innerhalb von 30 min portionsweise mit Zinnpulver (622 mg) versetzt. Das Gemisch wurde danach über Nacht bei Raumtemperatur gerührt. Das Gemisch wurde mit Ethanol (10 ml) versetzt und i. Vak. eingeengt. Der sehr schlecht Jösliche Rückstand wurde mit 5 N Natronlauge (25 ml) und Dichlormethan (30 ml) versetzt, die Phasen wurden getrennt und die wässrige Phase mit Dichlormethan (5 × 30 ml) extrahiert. Die vereinigten organischen Phasen wurden mit Natriumsulfat getrocknet und i. Vak. eingeengt. Das Rohprodukt (135 mg) wurde mittels Flashchromatographie (10 g, 20 × 2.1 cm) mit Chloroform / Methanol (4:1) gereinigt. Das unpolare und das polare Diastereomer konnten separiert werden und fielen aufgrund des verwendeten Chloroforms als Hydrochlorid an. Um die freie Base zu erhalten, wurde jeweils Natriumhydrogencarbonat-Lösung zugesetzt und mit Dichlormethan (3 × 10 ml) extrahiert.
**Die vereinigten organischen Phasen wurden mit Natriumsulfat getrocknet und i. Vak. eingeengt.**
*Ausbeute:* 25 mg (12 %), gelblicher, amorpher Feststoff
$^1$*H-NMR (DMSO-$d_6$):* 1.46 (t, 2H, J = 13.0 Hz); 1.58 (d, 2H, J = 11.0 Hz); 2.01 (s, 6H); 2.11 (q, 2H, J = 12.5 Hz); 2.79 (d, 2H, J = 12.7 Hz); 3.04 (m, 1H); 4.16 (s, 2H); 6.86 (t, 1H, J = 7.4 Hz); 6.96 (t, 1H, J = 7.5 Hz); 7.06-7.16 (m, 2H); 7.22-7.43 (m, 7H); 7.59 (dt, 1H, J = 7.7 und 1.8 Hz); 8.44 (d, 1H, J = 3.9 Hz); 10.77 (s, 1H).
**Ausbeute (Bsp 152):** 43 mg (21 %), weißer Feststoff: *Schmelzpunkt:* 205-210 °C
$^1$*H-NMR (DMSO-$d_6$):* 1.48 (t, 2H, J = 12.3 Hz); 1.65 (t, 4H, J = 12.1 Hz); 1.92 (s, 6H); 2.77 (d, 2H, J = 11.3 Hz); 3.03 (t, 1H, J = 11.8 Hz); 4.11 (s, 2H); 6.83 (t, 1H, J = 7.3 Hz); 6.91 (t, 1H, J = 7.0 Hz), 7.03 (d, 1H, J = 7.8 Hz), 7.13 (dd, 1H, J = 7.9 und 4.9 Hz); 7.17 (d, 1H, J = 7.9 Hz), 7.30 (d, 2H, J = 7.6 Hz); 7.36-7.46 (m, 4H); 7.59 (dt, 1H, J = 7.6 und 1.8 Hz); 8.45 (d, 1H, J = 3.8 Hz); 10.45 (s, 1H).

**Beispiel 153: 3-(2-(4-(dimethylamino)-4-phenylcyclohexyl)-1H-indol-3-yl)propionsäure Hydrochlorid, polares Diastereomer**

**3-[2-(4-Dimethylamino-4-phenylcyclohexyl)-1*H*-indol-3-yl]propionsäure** (polareres Diastereomer)

Keton **Ket-10** (1,9 g, 8,75 mmol) und Indol **Ind-50** (1,66 g, 8,75 mmol) wurden in HBr/Eisessig (33 % HBr, 50 ml) gelöst und 16 h bei RT gerührt. Anschließend wurde zu dem Ansatz bei RT Sn-Pulver (10,5 g, 87,5 mmol) innerhalb von 2 h portionsweise gegeben. Nach Beendigung der Zugabe wurde das Reaktionsgemisch weitere 48 h bei RT gerührt. Zur Aufarbeitung wurde das Gemisch mit EtOH (20 ml) verdünnt und am Rotationsverdampfer bis zur Trockene eingeengt. Das erhaltene Rohprodukt konnte durch Säulenchromatographie [Kieselgel 60 (300 g); MeOH (3000 ml)] gereinigt werden. 3-[2-(4-Dimethylamino-4-phenylcyclohexyl)-1*H*-indol-3-yl]propionsäure (polareres Diastereomer) wurde in einer Ausbeute von 419 mg (12 %) als weißer Feststoff erhalten.

**3-(2-(4-(dimethylamino)-4-phenylcyclohexyl)-1H-indol-3-yl)propionsäure Hydrochlorid, polares Diastereomer ( 153)**

3-[2-(4-Dimethylamino-4-phenylcyclohexyl)-1H-indol-3-yl]propionsäure (polareres Diastereomer) (419 mg, 1,08 mmol) wurde in Ethylacetat (50 ml) gelöst. Bei RT wurde $Me_3SiCl$ (276 µl, 1,16 mmol) zugetropft und 16 h gerührt. Das Lösungsmittel wurde am Rotationsverdampfer abdestilliert und Beispiel 153 (458 mg, Fp. 242-245 °C. 100 %) als weißer Feststoff erhalten.

**Beispiel 156: 1-Benzyl-N,N-dimethyl-4-(3-propyl-1H-indol-2-yl)cyclohexanamin, Citrat (1:1), 1 Diastereomer**

**1-Benzyl-N,N-dimethyl-4-(3-propyl-1H-indol-2-yl)cyclohexanamin (1 Diastereomer)**

**Bsp. 42** (956 mg, 2,57 mmol) wurde in Methanol (60 ml) gelöst und mit dem Pd/C (5-proz., 300 mg) versetzt und bei 3 bar 3 h bei 40 °C unter Rühren hydriert. Zur Aufarbeitung wurde der Katalysator über Celite abfiltriert und das Methanol im Vakuum entfernt. Nach chromatographischer Trennung des verbliebenen Rückstands. (834 mg gelbes Öl) [Kieselgel 60 (70 g); Cyclohexan/Ethylacetat 2 : 1 (450 ml), Cyclohexan/Ethylacetat 1 : 1 (1000 ml), Ethylacetat (500 ml), Ethylacetat/Methanol 1 : 1 (300 ml)] wurde ein beigefarbiger Feststoff (432 mg) gewonnen. Dieser wurde in Ethylacetat (3 ml) suspendiert, dann filtriert und mit Ethylacetat (3 × 0,5 ml) gewaschen. Es wurde eins von den zwei möglichen Diastereoisomeren als beigefarbiger Feststoff erhalten (208 mg, 22 %, *Schmelzpunkt:* 232-250 °C)**.**
1-Benzyl-N.N-dimethyl-4-(3-propyl-1H-indol-2-yl)cyclohexanamin (200 mg, 0,534 mmol) und Citronensäure (105 mg, 0,547 mmol) wurden in heißem Methanol (6 ml) gelöst. Die klare Lösung wurde im Vakuum eingeengt. Der Rückstand wurde mit Ethylacetat (5 ml) über Nacht gerührt, dann filtriert und mit Diethylether (2 × 2 ml) gewaschen. Beispiel 156 wurde so in einer Ausbeute von 61 % (185 mg) mit einem Schmelzpunkt von 250-265 °C (über 230 °C sublimiert) erhalten..

**Beispiel 157: 1-Butyl-N,N-dimethyl-4-(3-propyl-1H-indol-2-yl)cyclohexanamin Hydrochlorid, 1 Diastereomer**

**Beispiel 158: 1-Butyl-N,N-dimethyl-4-(3-propyl-1H-indol-2-yl)cyclohexanamin, Citrat (1:1), 1 Diastereomer**

**1-Butyl-N,N-dimethyl-4-(3-propyl-1H-indol-2-yl)cyclohexanamin (1 Diastereomer) und 1-Butyl-N,N-dimethyl-4-(3-propyl-1H-indol-2-yl)cyclohexanamin Hydrochlorid, 1 Diastereomer ( 157)**

Eine Lösung des Beispiels **43** (736 mg, 2,17 mmol) in Methanol (50 ml) wurde mit Pd/C (230 mg, 5-proz.) versetzt und bei 3 bar 3 h bei 40 °C unter Rühren hydriert. Zur Aufarbeitung wurde der Katalysator abfiltriert und das Methanol im Vakuum entfernt. Nach chromatographischer Trennung des verbliebenen Rückstands (769 mg gelbes Öl) [Kieselgel 60 (70 g); Ethylacetat/Methanol 100 : 1 (500 ml), Ethylacetat/Methanol 20 : 1 (525 ml), Ethylacetat/Methanol 10 : 1 (1100 ml), Ethylacetat/Methanol 5 : 1 (350 ml), Ethylacetat/Methanol 1 : 1 (300 ml)] wurde ein gelbes Gemisch aus Öl und Feststoff (550 mg) gewonnen. Dieses wurde in Diethylether (10 ml) suspendiert und dann filtriert. Der abfiltrierte Feststoff wurde mit Diethylether (2 × 2 ml) gewaschen und aus Isopropylalkohol (6 ml) umkristallisiert. Es wurde Beispiel **157** als beigefarbiger Feststoff gewonnen (244 mg, 16 %, Schmp.: 258-272 °C). Das etherische Filtrat wurde eingeengt und der Rückstand aus Cyclohexan (3 ml) umkristallisiert. Es wurde ein weißer Feststoff erhalten (1-Butyl-N,N-dimethyl-4-(3-propyl-1H-indol-2-yl)cyclohexanamin, 1 Diastereomer, 78 mg, 8 %, Schmp.: 127-130 °C).
1-Butyl-N,N-dimethyl-4-(3-propyl-1H-indol-2-yl)cyclohexanamin **(1 Diastereomer)** (73 mg, 0,214 mmol) und Citronensäure (43 mg, 0,224 mmol) wurden in heißem Methanol (5 ml) gelöst. Die klare Lösung wurde im Vakuum eingeengt. Der Rückstand wurde mit Diethylether (15 ml) über Nacht gerührt, dann filtriert und mit Diethylether (2 × 1 ml) gewaschen. **Beispiel 158** wurde als weißer Feststoff in einer Ausbeute von 71 % (81 mg) mit einem Schmelzpunkt von 83-90 °C erhalten.

**Beispiel 159: N,N-Dimethyl-1-phenyl-1-(3-propyl-1H-indol-2-yl)cyclohexanamin, Citrat (2:1), unpolares Diastereomer**

**Beispiel 160: N,N-Dimethyl-1-phenyl-4-(3-propyl-1H-indol-2-yl)cyclohexanamin, Citrat (2:1), polares Diastereomer**

**N,N-Dimethyl-1-phenyl-4-(3-propyl-1H-indol-2-yl)cyclohexanamin (unpolares und polares Diastereomer)**

**Bsp. 44** (1057 mg, 2,948 mmol) wurde in Bromwasserstoff/Eisessig (33 % HBr, 70 ml) gelöst. Anschließend wurde zu dem Ansatz bei Raumtemperatur Zinn-Pulver (5,250 g, 44,2 mmol) innerhalb von 1 h portionsweise gegeben und der Ansatz noch 1 h gerührt. Anschließend wurde das Gemisch mit Dichlormethan (250 ml) verdünnt. Unter Kühlung

wurde 3N Natriumhydroxid-Lösung (750 ml) zugegeben. Der Ansatz wurde 20 min bei Raumtemperatur gerührt. Nach Trennung der Phasen wurde die wässrige Phase mit Dichlormethan (3 × 20 ml) extrahiert. Die vereinigten organischen Extrakte wurden über $Na_2SO_4$ getrocknet und anschließend eingeengt (976 mg beigefarbiger Feststoff). Nach chromatographischer Trennung dieses Gemisches [Kieselgel 60 (100 g); Ethylacetat/Methanol 10 : 1 (550 ml), Ethylacetat/Methanol 20 : 3 (575 ml), Ethylacetat/Methanol 5 : 1 (1200 ml), Ethylacetat/Methanol 3 : 1 (400 ml)] wurden N,N-Dimethyl-1-phenyl-4-(3-propyl-1H-indol-2-yl)cyclohexanamin (unpolareres Diastereoisomer) (318 mg, 30 %, Schmp.: 177-181 °C) und das polarere Diastereoisomer (267 mg, 25 %, Schmp.: 211-215 °C) als weiße bzw. beigefarbige Feststoffe gewonnen.

**N,N-Dimethyl-1-phenyl-4-(3-propyl-1H-indol-2-yl)cyclohexanamin, Citrat (2:1), unpolares Diastereomer ( 159)**

N,N-Dimethyl-1-phenyl-4-(3-propyl-1H-indol-2-yl)cyclohexanamin (unpolareres Diastereoisomer) (155 mg, 0,430 mmol) wurde in heißem Methanol (80 ml) gelöst und mit einer methanolischen Citronensäure-Lösung (85 mg, 0,442 mmol in 5 ml) versetzt. Anschließend wurde die Mischung 2 h bei 0 bis 5 °C gerührt und dann filtriert. Beispiel 159 wurde als weißer Feststoff in einer Ausbeute von 77 % (182 mg) mit einem Schmelzpunkt von 243-249 °C erhalten.

**N,N-Dimethyl-1-phenyl-4-(3-propyl-1H-indol-2-yl)cyclohexanamin, Citrat (2:1), polares Diastereomer**

N,N-Dimethyl-1-phenyl-4-(3-propyl-1H-indol-2-yl)cyclohexanamin (polareres Diastereoisomer) (248 mg, 0,688 mmol) und Citronensäure (136 mg, 0,707 mmol) wurden in Methanol (5 ml) gelöst. Die klare Lösung wurde im Vakuum eingeengt. Der Rückstand wurde mit Ethylacetat (5 ml) 1 h gerührt, dann filtriert und mit Diethylether gewaschen. Beispiel 160 wurde als beigefarbener Feststoff in einer Ausbeute von 60 % (187 mg) mit einem Schmelzpunkt von 188-192 °C erhalten.

**Beispiel 161: 1-Benzyl-N,N-dimethyl-4-(3-(2-(pyridin-4-yl)ethyl)-1H-indol-2-yl)cyclohexanamin, Citrat (1:1) : Unpolareres Diastereomer**

Eine Lösung des Olefins Beispiel **45** (335 mg, 0,769 mmol) in Methanol (40 ml) wurde mit dem Katalysator (110 mg, 5 % Pd/C) versetzt und bei 3 bar 4 h bei 40 °C unter Rühren hydriert. Zur Aufarbeitung wurde der Katalysator abfiltriert und das Methanol im Vakuum entfernt. Nach chromatographischer Trennung des verbliebenen Rückstands [Kieselgel 60 (40 g): Ethylacetat/Methanol (10 : 1, 550 ml), Ethylacetat/Methanol (1 : 1, 450 ml), Ethylacetat/Methanol (1 : 2, 480 ml), Methanol (750 ml)] wurden das unpolarere Diastereoisomer (93 mg, 27 %, Schmp.: 233-237 °C) und das polarere Diastereoisomer (150 mg, 44 %, Schmp.: 198-203 °C) als weißer Feststoff gewonnen.

<u>Unpolareres Diastereomer:</u>

$^1$H NMR (400 MHz, $CDCl_3$) *δ ppm*; 1.10-1.35 (m, 4H), 1.72-1.95 (m, 4H), 2.22-2.48 (m, 7H), 2.67 (s, 2H), 2.81-2.92 (m, 2H), 2.92-3.03 (m, 2H), 6.94-7.02 (m, 2H), 7.02-7.22 (m, 4H), 7.22-7.40 (m, 4H), 7.44-7.52 (m, 4H), 7.48 (d, J = 7.81 Hz, 1H), 8.33 (s, broad, 1H), 8.39 (dd, J = 4.48, 1.48 Hz, 1H)
$^{13}$C *NMR (101 MHz, $CDCl_3$) δ ppm:* 25.1, 27.0, 32.3, 34.8, 36.4, 36.8, 37.0, 57.7 (broad), 108.4, 110.6, 117.8, 118.9. 120.9, 124.1, 125.9, 127.9, 128.0, 130.9. 135.3, 138.9 (broad), 140.2, 149.4, 151.2

<u>Polareres Diastereomer</u>

$^1$H NMR (400 MHz, $CDCl_3$) *δ ppm*: 1.16-1.45 (m, 1H), 1.45-1.69 (m, 5H),1.69-1.87 (m, 2H), 2.22-2.50 (m, 6H), 2.50-2.72 (m, 1H), 2.78-2.96 (m, 4H), ), 2.96-3.10 (m, 2H), 7.00 (d, J = 4.76 Hz, 1H), 7.03-7.18 (m, 2H), 7.18-7.37 (m, 5H), 7.50 (d, J = 7.51 Hz, 1H), 7.79 (s, 1H), 8.43 (d, J = 4.79 Hz, 1H)
$^{13}$*C NMR (101 MHz, $CDCl_3$) δ ppm:* 25.5, 28.6, 31.8, 34.0, 36.27, 36.30, 37.8, 57.7, 108.9, 110.5, 117.9, 119.2, 121.1, 124.2, 126.0, 127.9, 128.0, 130.7, 135.2, 139.2, 139.9, 149.4, 151.2

Das soeben hergestellte unpolarere Diastereomer (70 mg, 0,160 mmol) wurde in einem Gemisch aus Methanol (2 ml) und Chloroform (2 ml) gelöst und mit Citronensäure (33 mg, 0,172 mmol), gelöst in Methanol (2 ml), versetzt. Die klare Lösung wurde im Vakuum eingeengt. Der Rückstand wurde in heißem Ethanol (4 mi) gelöst. Zur Lösung wurde bei RT langsam Ethylacetat (4 ml) und Diethylether (4 ml) zugetropft. Das gewünschte Citrat fiel als weißer Feststoff aus. Das Gemisch wurde weitere 2 h bei RT gerührt, dann filtriert und mit Diethylether gewaschen. Der weiße Feststoff wurde in einer Ausbeute von 81 % (82 mg) mit einem Schmelzpunkt von 150-155 °C erhalten.
$^1$*H NMR (400 MHz, DMSO-$d_6$)* □ ppm: 1.10-1.32 (m, 4H), 1.71-1.90 (m, 2H), 1.91-2.08 (m, 2H), 2.40-2.53 (m, 7H), 2.63 (dd, J = 35.78, 15.32 Hz, 4H), 2.72-2.83 (m, 4H), 2.84-2.94 (m, 2H), 6.86-6.94 (m, 1H), 6.94-7.01 (m, 1H), 7.06-7.13 (m, 2H), 7.19-7.46 (m, 7H), 8.28-8.36 (m, 2H), 10.3.(s, 1H)

**Beispiel 162: 1-Benzyl-N,N-dimethyl-4-(3-(2-(pyridin-4-yl)ethyl)-1H-indol-2-yl)cyclohexanamin, Citrat (1:1) : Polareres Diastereomer**

Das unter Beispiel 161 erhaltene polarere Diastereomer (135 mg, 0,308 mmol) und Citronensäure (60 mg, 0,312 mmol) wurden in Methanol (4 ml) gelöst. Die klare Lösung wurde im Vakuum eingeengt, und der Rückstand in heißem Isopropanol (10 ml) gelöst. Das Gemisch wurde bei 5 °C 2 h gerührt. Das gewünschte Citrat fiel als weißer Feststoff aus, der filtriert und mit Diethylether gewaschen wurde. Das Produkt wurde in einer Ausbeute von 61 % (119 mg) mit einem Schmelzpunkt von 122-150 °C erhalten.

*$^1$H NMR (400 MHz, DMSO-$d_6$) δ ppm:* 1.04 (d, J = 6.10 Hz, 3H), 1.34-1.49 (m, 2H), 1.53-1.72 (m, 2H), 1.73-1.89 (m, 2H), 1.89-2.06 (m, 2H), 2.50 (m, under DMSO), 2.55 (dd, J = 32.28, 17.12 Hz, 4H), 2.82-2.92 (m, 2H), 2.95-3.06 (m, 2H), 3.23 (s,), 6.91-6.99 (m, 1H), 6.99-7.06 (m, 1H), ), 7.11-7.19 (m, 2H), 7.23-7.44 (m, 6H), 7.48 (d, J = 7.62 Hz, 1H), 8.40 (d, J = 5.38 Hz, 2H), 10.74 (s, 1H)

**Beispiel 163: 1-Butyl-N,N-dimethyl-4-(3-(2-(pyridin-4-yl)ethyl)-1H-indol-2-yl)cyclohexanamin, Citrat (1:1) : Unpolareres Diastereomer**

Eine Lösung des Olefins Beispiel 46 (390 mg, 0,971 mmol) in Methanol (40 ml) wurde mit dem Katalysator (140 mg, 5 % Pd/C) versetzt und bei 3 bar 2 h bei 40 °C unter Rühren hydriert. Zur Aufarbeitung wurde der Katalysator abfiltriert und das Methanol im Vakuum entfernt. Nach chromatographischer Trennung des verbliebenen Rückstands [Kieselgel 60 (50 g); Ethylacetat/Methanol (5 : 1, 250 ml), Ethylacetat/methanol (1 : 1, 450 ml), Methanol (1800 ml)] wurden das unpolarere Diastereoisomer (89 mg, 23 %, Schmp.: 210-250 °C, sublimiert über 200 °C) und das polarere Diastereoisomer (221 mg, 56 %, Schmp.: 206-212 °C) als weißer Feststoff gewonnen.

Polareres Diastereomer:

*$^1$H NMR (300 MHz, $CDCl_3$) δ ppm:* 0.75-0.92 (m, 3H), 0.97 (t, J = 6.88 Hz, 2H), 1.11-1.65 (m, 15H), 1.75-1.90 (m, 1 H), 2.08-2.36 (m, 1H), 2.36-2.51 (m, 1H), ), 2.51-2.79 (m, 3H), ), 2.84-2.96 (m, 1H), ), 2.96-3.10 (m, 1H), 6.90-7.00 (m, 2H), 7.00-7.16 (m, 2H), 7.39-7.57 (m, 2H), 8.39 (d, J = 5.08 Hz, 2H), 10.59 (s, broad, 1H)

*$^{13}$C NMR (101 MHz, $CDCl_3$) δ* ppm 13.9, 23.4, 25.6, 26.1, 26.6, 29.7, 31.1, 34.2, 36.4, 37.2, 107.6, 111.5, 117.5, 118.4, 120.8, 124.4, 127.3, 135.9, 138.8 (broad), 149.3, 151.5

Zur Herstellung des Citrates wurde das soeben erhaltene unpolarere Diastereomer (63 mg, 0,156 mmol) und Citronensäure (31 mg, 0,161 mmol) in heißem Methanol (20 ml) gelöst. Die klare Lösung wurde im Vakuum eingeengt, und der Rückstand in heißem Ethanol (2 ml) gelöst. Zur Lösung wurde bei RT langsam Ethylacetat (8 ml) und Diethylether (30 ml) zugetropft. Das gewünschte Citrat fiel als weißer Feststoff aus. Das Gemisch wurde über Nacht bei RT gerührt, dann filtriert und mit Diethylether gewaschen. Das gelbe Pulver wurde in einer Ausbeute von 61 % (57 mg) mit einem Schmelzpunkt von 184-187 °C.

*$^1$H NMR (400 MHz,* DMSO-$d_6$) ppm: 0.87-1.04 (m, 3H), 1.28-1.47 (m, 4H), 1.54-1.68 (m, 2H), 1.68-1.83 (m, 3H), 1.93-2.08 (m, 3H), 2.14-2.34 (m, 3H), 2.42-2.58 (m), 2.61-2.80 (m), 2.82-2.93 (m), 6.88-6.97 (m, 1H), 6.97-7.06 (m, 1H), 7.12-7.21 (m, 2H), 7.21-7.28 (m, 1H), 7.41-7.51 (m, 1H), 8.36-8.46 (m, 2H), 11.14 (s, 1H)

**Beispiel 164: 1-Butyl-N,N-dimethyl-4-(3-(2-(pyridin-4-yl)ethyl)-1H-indol-2-yl)cyclohexanamin, Citrat (1:1): Polareres Diastereomer**

Das unter Beispiel **163** erhaltene polarere Diastereomer (234 mg, 0,580 mmol) und Citronensäure (113 mg, 0,588 mmol) wurden in heißem Methanol (20 ml) gelöst. Die klare Lösung wurde im Vakuum eingeengt, und der Rückstand in heißem Ethanol (4 ml) gelöst. Zur Lösung wurde bei RT langsam Ethylacetat (6 ml) und Diethylether (20 ml) zugetropft. Das Gemisch wurde bei RT über Nacht gerührt. Das gewünschte Citrat fiel als gelbes Pulver aus, das filtriert und mit Diethylether gewaschen wurde. Das Produkt wurde in einer Ausbeute von 76 % (261 mg) mit einem Schmelzpunkt von 97-103 °C erhalten.

**Beispiel 165: N,N-Dimethyl-1-phenyl-4-(3-(2-(pyridin-4-yl)ethyl)-1H-indol-2-yl)cyclohexanamin, Citrat (1:4) : Unpolareres Diastereomer**

Beispiel **47** (400 mg, 0,95 mmol) wurde in HBr/Eisessig (33 % HBr, 25 ml) gelöst. Anschließend wurde zu dem Ansatz bei RT Sn-Pulver (1,126 g, 9,49 mmol) innerhalb von 40 min portionsweise gegeben. Da die Umsetzung laut DC nicht vollständig war, wurde weiteres Sn-Pulver (0,57 g, 4,8 mmol) zugegeben und der Ansatz noch 1 h gerührt. Anschließend wurde das Gemisch mit Dichlormethan (250 ml) verdünnt. Dann wurde 3N NaOH-Lösung (280 ml) langsam unter Kühlung zugegeben, so daß die Temperatur 25 °C nicht überschritt. Das Gemisch wurde 20 min gerührt. Die

Phasen wurden getrennt. Die wäßrige Phase wurde mit Dichlormethan (3 x 20 ml) extrahiert. Die vereinigten organischen Phasen wurden mit $Na_2SO_4$ getrocknet, filtriert und die flüchtigen Bestandteile vollständig im Vakuum entfernt. Es blieb ein gelber Feststoff (369 mg) zurück. Nach chromatographischer Trennung dieses Gemisches [Kieselgel 60 (50 g); Chloroform/Methanol (50 : 1, 250 ml), Chloroform/Methanol (25 : 1, 250 ml), Ethylacetat/Methanol (10 : 1, 250 ml), Ethylacetat/Methanol (5: 1, 280 ml), Ethylacetat/Methanol (10 : 3, 300 ml), Ethytacetat/Methanol (5 : 2, 320 ml)] wurden das unpolarere Diastereoisomer (148 mg, 37 %, Schmp.: 267-270 °C) und das polarere Diastereoisomer (81 mg, 20 %, Schmp.: 228-232 °C) als weiße Feststoffe gewonnen.

Unpolareres Diastereomer:

$^1$*H NMR (400 MHz, DMSO-*$d_6$ + 0.02 ml 20 % DCl in $D_2O$) *δ ppm:* 1.51 (d, J = 12.14 Hz, 2H), 1.75-2.05 (m, 2H), 2.25-2.69 (m, 9H), 2.86-3.33 (m, 6H), 6.83-7.13 (m, 2H), 7.24 (d, J = 7.81 Hz, 1H), 7.38-7.63 (m, 4H), 7.63-7.81 (m, 2H), 7.90 (d, J = 5.13 Hz, 1H), 8.75 (d, J = 5.18 Hz, 1H)
$^{13}$*C NMR (101 MHz, DMSO-*$d_6$ + 0.02 ml 20 % DCl in $D_2O$) *δ ppm:* 24.4, 26.7, 30.9, 32.1, 36.3, 37.1, 38.1, 66.5, 107.0, 110.6, 117.9, 118.3, 120.6, 127.1, 127.6, 128.6, 128.7, 129.7, 134.1, 135.2, 140.4, 140.5, 163.4

Polareres Diastereomer:

$^1$*H NMR (400 MHz, DMSO-*$d_6$ + 0.02 ml 20 % DCl in $D_2O$) □ *ppm:* 1.29-1.51 (m, 2H), 1.51-1.71 (m, 2H), 2.09-2.31 (m, 2H).*2.31-2.59 (m, 7H), 2.66-2.86 (m, 1H), 2.86-3.23 (m, 5H), 6.78-7.05 (m, 2H), 7.05-7.26 (m, 1H), 7.28-7.47 (m, 1H), 7.47-7.65 (m, 3H), 7.65-7.81 (m, 2H), 7.81-7.99 (m, 2H), 8.82 (d, J = 4.83 Hz, 1H)
$^{13}$*C NMR (101 MHz, DMSO-*$d_6$ + 0.02 ml 20 % DCl in $D_2O$) □ *ppm:* 24.4, 28.6, 30.4, 34.8, 36.8, 37.2, 38.9, 68.5, 107.2, 110.8, 117.6, 118.3, 120.4, 127.3, 127.6, 129.2, 129.6, 130.4, 135.1, 138.7, 140.5, 163.2

Zur Herstellung des gewünschten Citrates wurden das soeben erhaltene unpolarere Diastereomer (122 mg, 0,288 mmol) und Citronensäure (57 mg, 0,297 mmol) in Methanol (200 ml) unter Erwärmen gelöst. Die Lösung wurde auf 10 ml eingeengt und über Nacht bei 5 °C stehen gelassen. Das ausgefallene Citrat wurde abgesaugt und mit Methanol (2 x 0,5 ml) gewaschen. Der weiße Feststoff wurde in einer Ausbeute von 86 % (153 mg) mit einem Schmelzpunkt von 198-203 °C, und 273-278 °C erhalten.

$^1$*H NMR (300 MHz, DMSO-*$d_6$*) δ ppm:* 1.26-1.45 (m, 2H), 1.45-1.67 (m, 2H), 1.87-2.26 (m, 9H), 2.65 (dd, J = 30.24, 15.31 Hz, 4H), 2.77-2.92 (m, 4H), 2.92-3.04 (m, 2H), 6.88-7.05 (m, 2H), 7.12-7.21 (m, 2H), 7.26-7.36 (m, 2H), 7.36-7.54 (m, 5H), 8.39 (d, J = 5.76 Hz, 2H), 10.51 (s, 1H)
$^{13}$*C NMR (101 MHz, DMSO-*$d_6$*) δ ppm:* 25.1, 27.2, 32.6, 34.7, 36.0, 37.8, 43.1, 72.0, 107.6, 110.8, 117.4, 118.0, 119.9, 124.1, 126.9, 127.5, 127.8, 135.4, 140.2, 149.1, 150.8, 166.8, 171.2,175,2

**Beispiel 166: N,N-Dimethyl-1-phenyl-4-(3-(2-(pyridin-4-yl)ethyl)-1H-indol-2-yl)cyclohexanamin, Citrat (2:1) : Polareres Diastereomer**

Das unter Beispiel **165** hergestellte polarere Diastereomer (58 mg, 0,137 mmol) und Citronensäure (28 mg, 0,146 mmol) wurden in Methanol (80 ml) unter Erwärmen gelöst. Die Lösung wurde auf 10 ml eingeengt und über Nacht bei 5 °C stehen gelassen. Das ausgefallene Citrat wurde abgesaugt und mit Methanol (2 x 0,5 ml) gewaschen. Der weiße Feststoff wurde in einer Ausbeute von 65 % (55 mg) mit einem Schmelzpunkt von 223-225 °C erhalten.

$^1$*H NMR (300 MHz, DMSO-*$d_6$) □*ppm:* 1.28-1.58 (m, 3H), 1.60-1.82 (m, 1H), 2.05-2.29 (m, 3H), 2.42-2.62 (m), 2.62-2.74 (m, 1H), 2.74-2.84 (m, 2H), 2.84-2.94 (m, 2H), 6.84-6.99 (m, 2H), 7.06-7.20 (m, 3H), 7.33-7.60 (m, 6H), 8.42 (d, J = 5.50 Hz, 2H), 10.33 (s, 1H)

**Beispiel 167: (±)-2-4(Dimethylamino)-4-phenylcyclohex-1-enyl)-3(2-(1-methyl-1H-benzo[d]imidazol-2-yl)ethyl)-1H-indol**

Indol **(Ind-54,** 0.850 g, 3.08 mmol) und Keton **(Ket-10,** 668 mg, 3.08 mmol) wurden in abs. Dichlormethan (30 ml) gelöst und schnell mit Trifluormethansulfonsäure (0.82 ml, 9.24 mmol) versetzt. Der Ansatz wurde über Nacht bei RT gerührt, dabei setzte sich ein dunkelbraunes Öl ab. Nach Zugabe von 1 N NaOH (15 ml) und $CH_2Cl_2$ (20 ml) wurde noch 60 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 777 mg (53 %), poröser Feststoff
$^1$*H-NMR (DMSO-*$d_6$*):* 1.74 (1 H, m); 2.12 (8 H, m); 2.37 (2 H, m); 2.56 (1 H, m); 2.98 (2 H, t); 3.18 (2 H, t); 3.49 (3 H, s); 6.08 (1 H, s); 6.88 (1 H, t); 7.01 (1 H, m); 7.22 (6 H, m): 7.44 (4 H, m); 7.61 (1 H, d); 10.66 (1 H, s).

**N,N-Dimethyl-4-(3-(2-(1-methyl-1H-benzo[d]imidazol-2-yl)ethyl)-1H-indol-2-yl)-1-phenylcyclohexanamin, Citrat (1:1)** : **Unpolareres Diastereomer**

Zu einer Lösung des soeben hergestellten Olefins (770 mg, 1.62 mmol) in HBr/Eisessig (40 ml) wurde innerhalb von 30 min Zinn (1.92 g) gegeben und 5 h bei RT gerührt. Der Ansatz wurde mit Ethanol versetzt, das Reaktionsgemisch bis zur Trockne eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (20:1 → 1:1) getrennt.

*Ausbeute:* 223 mg (29 %), unpolares Diastereomer
176 mg (23 %), polares Diastereomer

Das erhaltene unpolarere Diastereomer (200 mg, 0.42 mmol) wurde in heißem Ethanol (5 ml) gelöst und mit einer Lösung von Citronensäure (80 mg, 0.42 mmol) in heißem Ethanol (5 ml) versetzt. Nach 2- stündigem Stehen im Kühlschrank wurde der entstandene Feststoff abgesaugt und i. Vak. getrocknet.
*Ausbeute:* 152 mg (54%); *Schmelzpunkt:* 171-172 °C
*$^1$H-NMR (DMSO-$d_6$):* 1.48 (4 H, m); 2.18 (8 H, m); 2.72 (7 H, m); 3.16 (4 H, m); 3.52 (3 H, s); 6.98 (2 H, m); 7.01 (2 H, m); 7.16 (2 H, m); 7.44 (9 H, m); 10.67 (1 H, s).
*Citrat:* (Ethanol)

**Beispiel 168: N,N-Dimethyl-4-(3-(2-(1-methyl-1H-benzo[d]imidazol-2-yl)ethyl)-1H-indol-2-yl)-1-phenylcyclohexanamin, Citrat (1:1) : Polareres Diastereomer**

Das unter Beispiel **167** gewonnene polarere Diastereomer (168 mg, 0.35 mmol) wurde in heißem Ethanol (5 ml) gelöst und mit einer Lösung von Citronensäure (68 mg, 0.35 mmol) in heißem Ethanol (3 ml) versetzt. Nach Versetzen mit Diethylether und 2- stündigem Stehen im Kühlschrank wurde der entstandene Feststoff abgesaugt und i. Vak. getrocknet.
*Ausbeute:* 87 mg (37%); *Schmelzpunkt:* >270 °C (Zers.)
*$^1$H-NMR (DMSO-$d_6$):* 1.35 (2 H, m); 1.54 (2 H, m); 2.00 (2 H, m); 2,37 (6 H; s); 2.61 (2 H, m); 2.83 (4 H, m); 3.14 (4 H, m); 3.53 (3 H, s); 6.88 (3 H, m); 7.19 (3 H, m); 7.49 (7 H, m); 10.36 (1 H, s).
*Citrat:* (Ethanol)

**Beispiel 169: (±)-2-(4-(Dimethylamino)-4-phenylcyclohex-1-enyl)-3-(2-(pyridin-2-yl)ethyl)-1 H-indol**

3-(2-Pyridin-2-yl-ethyl)-1*H*-indol (Ind-55, 444 mg, 2.0 mmol) und Keton (Ket-10, 434 mg, 2.0 mmol) wurden in abs. Dichlormethan (20 ml) gelöst und schnell mit Trifluormethansulfonsäure (0.54 ml, 6.0 mmol) versetzt. Der Ansatz wurde über Nacht bei RT gerührt, dabei setzte sich ein dunkelbraunes Öl ab. Nach Zugabe von 1 N NaOH (10 ml) und $CH_2Cl_2$ (10 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 320 mg (38 %), poröser Feststoff
*$^1$H-NMR (DMSO-$d_6$):* 1.75 (2 H, m); 2.12 (8 H, m); 2.40-2.69 (3 H, m); 2.90 (2 H, m); 3.06 (2 H, m); 6.15 (1 H, bs); 6.89 (1 H, m); 6.98 (1 H, m); 7.09 (1 H, m); 7.22 (5 H, m); 7.39 (1 H, m); 7.49 (2 H, m); 7.64 (1 H, m); 8.52 (1 H, m); 10.58 (1 H, s).

**N,N-Dimethyl-1-phenyl-4-(3-(2-(pyridin-2-yl)ethyl)-1H-indol-2-yl)cyclohexanamin, Citrat (1:1) : Unpolareres Diastereomer**

Zu einer Lösung des soeben hergestellten Olefins (300 mg, 0.71 mmol) in HBr/Eisessig (15 ml) wurde innerhalb von 20 min Zinn (0.9 g) gegeben und 3 h bei RT gerührt. Der Ansatz wurde mit Ethanol versetzt, das Reaktionsgemisch bis zur Trockne eingeengt und der Rückstand in 5N NaOH (20 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (9:1 → 4:1) getrennt.

*Ausbeute:* 141 mg (47 %), unpolares Diastereomer
58 mg (19 %), polares Diastereomer

Das soeben erhaltene unpolarere Diastereomer (116 mg, 0.27 mmol) wurde in heißem Ethanol (3 ml) gelöst und mit einer Lösung von Citronensäure (53 mg, 0.27 mmol) in heißem Ethanol (3 ml) versetzt. Nach 2- stündigem Stehen im Kühlschrank wurde der entstandene Feststoff abgesaugt und i. Vak. getrocknet.
*Ausbeute:* 120 mg (71%); *Schmelzpunkt:*139-140 °C

*$^1$H-NMR (DMSO-$d_6$):* 1.42 (2 H, m); 1.56 (2 H, m); 2.01 (2 H, m); 2.15 (6 H, s); 2.64-2.77 (6 H, m); 3.04 (2 H, t); 3.45 (2 H, t); 6.93 (2 H, m); 7.14 (2 H, m); 7.42 (6 H, s); 7.62 (1 H, m); 8.49 (1 H, m); 10.49 (1 H, s).
Citrat: (Ethanol)

**Beispiel 170: N,N-Dimethyl-1-phenyl-4-(3-(2-(pyridin-2-yl)ethyl)-1H-indol-2-yl)cyclohexanamin, Citrat (2:3) : Polareres Diastereomer**

Das unter Beispiel 169 gewonnene polarere Diastereomer (58 mg, 0.14 mmol) wurde in heißem Ethanol (3 ml) gelöst und mit einer Lösung von Citronensäure (27 mg, 0.14 mmol) in heißem Ethanol (2 ml) versetzt. Nach 2- stündigem Stehen im Kühlschrank wurde der entstandene Feststoff abgesaugt und i. Vak. getrocknet.
*Ausbeute:* 40 mg (47%), poröser Feststoff
*$^1$H-NMR (DMSO-$d_6$):* 1.40 (2 H, m); 1.58 (2 H, m); 1.88 (2 H, m); 2.41 (6 H, m); 2.54-2.66 (4 H, m); 2.76 (1 H, m); 2.99 (6 H, m); 6.87 (2 H, m); 7.04-7.23 (3 H, m); 7.41 (1 H, m); 7.61 (6 H, m); 10.30 (1 H, s).
Citrat: (Ethanol), poröser Feststoff

**Beispiel 171: 4-(2-(4-Benzyl-4-(dimethylamino)cyclohexyl)-1H-indol-3-yl)butansäure Hydrochlorid, 1 Diastereomer**

4-[2-(4-Benzyl-4-dimethylaminocyclohex-1-enyl)-1*H*-indol-3-yl]butansäure 4-(1H-Indol-3-yl)butansäure (Ind-56, 813 mg, 4 mmol) wurde zusammen mit dem Keton Ket-3 (926 mg, 4 mmol) in Dichlormethan (80 ml) gelöst und mit Trifluormethansulfonsäure (540 μl, 6 mmol) versetzt. Der Ansatz wurde 48 h bei RT gerührt. Zur Aufarbeitung wurde das Reaktionsgemisch mit $H_2O$ (20 ml) versetzt und 20 min bei RT gerührt. Nach Abtrennung der organischen Phase wurde die wässrige Phase mit Dichlormethan (3 x 20 ml) extrahiert. Die vereinigten organischen Extrakte wurden über $Na_2SO_4$ getrocknet und anschließend eingeengt. Das erhaltene Rohprodukt (1,39 g) konnte durch Säulenchromatographie [Kieselgel 60 (100 g); MeOH (1000 ml)] gereinigt werden. 4-[2-(4-benzyl-4-dimethylaminocyclohex-1-enyl)-1H-indol-3-yl]butansäure wurde in einer Ausbeute von 1,35 g (81 %) als gelber Schaum erhalten.

**4-(2-(4-Benzyl-4-(dimethylamino)cyclohexyl)-1H-indol-3-yl)butansäure (1 Diastereomer)** 4-[2-(4-Benzyl-4-dimethylaminocyclohex-1-enyl)-1H-indol-3-yl]butansäure (300 mg,

0,7 mmol) wurde in abs. Methanol (30 ml) mit Palladium als Katalysator (Pd/C, 5 %, 120 mg) versetzt und 6 h bei RT hydriert (Wasserstoffdruck: 3 bar). Der Katalysator wurde mit Hilfe einer Fritte, versehen mit einer 1 cm hohen Schicht Celite, entfernt. Es wurde mit Methanol (500 ml) gründlich nachgewaschen. Das Lösungsmittel wurde im Vakuum abdestilliert. 4-(2-(4-Benzyl-4-(dimethylamino)cyclohexyl)-1H-indol-3-yl)butansäure wurde in einer Ausbeute von 260 mg (86 %) als gelber Feststoff erhalten (eins der zwei möglichen Diastereoisomeren).

**4-(2-(4-Benzyl-4-(dimethylamino)cyclohexyl)-1H-indol-3-yl)butansäure Hydrochlorid, 1 Diastereomer (171)**

4-(2-(4-Benzyl-4-(dimethylamino)cyclohexyl)-1H-indol-3-yl)butansäure (260 mg, 0,6 mmol) wurde in Ethylacetat (50 ml) gelöst. Bei RT wurde dann $Me_3SiCl$ (153 μl, 1,2 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 171 (197 mg, Fp. 90-93 °C, 70 %) wurde als weißer Feststoff erhalten (Reinheit <95 %).

**Beispiel 172: 4-(2-(4-(Dimethylamino)-4-phenylcyclohexyl)-1H-indol-3-yl)butansäure Hydrochlorid, 1 Diastereomer**

**(±)-4-[2-(4-Dimethylamino-4-phenyl-cyclohex-1-enyl)-1*H*-indol-3-yl]butansäure**

4-(Indol-3-yl)buttersäure (Ind-56, 1,626 g, 8 mmol) wurde zusammen mit Keton Ket-10 (1,736 g, 8 mmol) in Dichlormethan (160 ml) gelöst und mit Trifluormethansulfonsäure (1,08 ml, 12 mmol) versetzt. Der Ansatz wurde 16 h bei RT gerührt. Zur Aufarbeitung wurde das Reaktionsgemisch mit $H_2O$ (40 ml) versetzt und 20 min bei RT gerührt. Nach Abtrennung der organischen Phase wurde die wässrige Phase mit Dichlormethan (3 x 30 ml) extrahiert. Die vereinigten organischen Extrakte wurden über $Na_2SO_4$ getrocknet und anschließend eingeengt. Das erhaltene Rohprodukt (2 g) konnte durch Säulenchromatographie [Kieselgel 60 (100 g); MeOH (1000 ml)] gereinigt werden. (±)-4-[2-(4-Dimethylamino-4-phenyl-cyclohex-1-enyl)-1*H*-indol-3-yl]butansäure wurde in einer Ausbeute von 500 mg (15 %) als gelber Feststoff erhalten.

**4-[2-(4-Dimethylamino-4-phenylcyclohexyl)-1*H*-indol-3-yl]butansäure (1 Diastereomer)**

(±)-4-[2-(4-Dimethylamino-4-phenyl-cyclohex-1-enyl)-1H-indol-3-yl]butansäure (500 mg,1,2 mmol) wurde in abs. Methanol (50 ml) mit Palladium als Katalysator (Pd/C, 5 %, 200 mg) versetzt und 6 h bei RT hydriert (Wasserstoffdruck: 3 bar). Der Katalysator wurde mit Hilfe einer Fritte, versehen mit einer 1 cm hohen Schicht Celite, entfernt. Es wurde mit Methanol (500 ml) gründlich nachgewaschen. Das Lösungsmittel wurde im Vakuum abdestilliert. 4-[2-(4-Dimethylamino-4-phenylcyclohexyl)-1*H*-indol-3-yl]butansäure wurde in einer Ausbeute von 340 mg (68 %) als weißer Feststoff erhalten (eins der zwei möglichen Diastereoisomeren).

**4-(2-(4-(Dimethylamino)-4-phenylcyclohexyl)-1H-indol-3-yl)butansäure Hydrochlorid, 1 Diastereomer**

4-[2-(4-Dimethylamino-4-phenylcyclohexyl)-1*H*-indol-3-yl]butansäure (340 mg, 0,81 mmol) wurde in Ethylacetat (50 ml) gelöst. Bei RT wurde dann $Me_3SiCl$ (207 µl, 1,62 mmol) zugetropft und 16 h gerührt. Das Lösungsmittel am Rotationsverdampfer abdestilliert. Beispiel **172** (370 mg, Fp. 227-230 °C, 100 %) war ein beigefarbener Feststoff

**Beispiel 173: 4-(2-(4-(Dimethylamino)-4-phenylcyclohexyl)-1H-indol-3-yl)butan-1-ol Hydrochlorid, unpolares Diastereomer**

**(±)-4-[2-(4-Dimethylamino-4-phenylcyclohex-1-enyl)-1*H*-indol-3-yl]butan-1-ol**

4-(1H-indol-3-yl)butan-1-ol (Ind-57) (797 mg, 4,21 mmol wurde zusammen mit dem Keton Ket-10 (914 mg, 4,21 mmol) in Dichlormethan (80 ml) gelöst und mit Trifluormethansulfonsäure (540 µl, 6 mmol) versetzt. Der Ansatz wurde 24 h bei RT gerührt. Zur Aufarbeitung wurde das Reaktionsgemisch mit 5N NaOH (30 ml) versetzt und 20 min bei RT gerührt. Nach Abtrennung der organischen Phase wurde die wässrige Phase mit Dichlormethan (3 x 30 ml) extrahiert. Die vereinigten organischen Extrakte wurden über $Na_2SO_4$ getrocknet und anschließend eingeengt. Das erhaltene Rohprodukt (2 g) konnte durch Säulenchromatographie [Kieselgel 60 (100 g); MeOH (1000 ml)] gereinigt werden. (±)-4-[2-(4-Dimethylamino-4-phenylcyclohex-1-enyl)-1*H*-indol-3-yl]butan-1-ol wurde in einer Ausbeute von 600 mg (37 %) als weißer Schaum erhalten.

**4-[2-(4-Dimethylamino-4-phenylcyclohexyl)-1*H*-indol-3-yl]butan-1-ol (unpolares Diastereomer)**

(±)-4-[2-(4-Dimethylamino-4-phenylcyclohex-1-enyl)-1*H*-indol-3-yl]butan-1-ol (600 mg, 1,54 mmol) wurde in HBr/Eisessig (33 % HBr, 10 ml) gelöst. Anschließend wurde zu dem Ansatz bei RT Sn-Pulver (1,81 g, 15,4 mmol) innerhalb von 2 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von 5N NaOH (40 ml) basisch gemacht. Das erhaltene wässrige Gemisch wurde mit Dichlormethan extrahiert (3 x 30 ml). Die vereinigten organischen Phasen wurden über $Na_2SO_4$ getrocknet und anschließend eingeengt. Das erhaltene Rohprodukt konnte durch Säulenchromatographie [Kieselgel 60 (50 g); MeOH (500 ml)] gereinigt werden. 4-[2-(4-Dimethylamino-4-phenylcyclohexyl)-1*H*-indol-3-yl]butan-1-ol (unpolareres Diastereomer) wurde in einer Ausbeute von 173 mg (29 %) als weißer Feststoff erhalten.

**4-(2-(4-(Dimethylamino)-4-phenylcyclohexyl)-1H-indol-3-yl)butan-1-ol Hydrochlorid, unpolares Diastereomer**

4-[2-(4-Dimethylamino-4-phenylcyclohexyl)-1*H*-indol-3-yl]butan-1-ol (unpolareres Diastereomer) (170 mg, 0,43 mmol) wurde in Ethylacetat (50 ml) gelöst. Bei RT wurde dann $Me_3SiCl$ (108 µl, 0,86 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 173 (170 mg, Fp. 223-226 °C, 91 %) wurde als ein weißer Feststoff erhalten.

**Beispiel 174: 4-(2-(4-Benzyl-4-(dimethylamino)cyclohexyl)-1H-indol-3-yl)butyl acetat Hydrochlorid, unpolares Diastereomer**

**Beispiel 175: 4-(2-(4-Benzyl.4-(dimethylamino)cyclohexyl)-1H-indol-3-yl)butyl acetat Hydrochlorid, polares Diastereomer**

**4-(2-(4-Benzyl-4-(dimethylamino)cyclohexyl)-1H-indol-3-yl)butyl acetat (unpolares und polares Diastereomer)**

Das Keton **Ket-3** (463 mg, 2 mmol) und **Ind-57** (379 mg, 2 mmol) wurden in HBr/Eisessig (33 % HBr, 2 ml) gelöst und 24 h bei RT gerührt. Anschließend wurde zu dem Ansatz bei RT Sn-Pulver (2,4 g, 20 mmol) innerhalb von 1 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von festem $NaHCO_3$ neutralisiert und mit $H_2O$ (40 ml) versetzt. Das erhaltene wässrige Gemisch wurde mit Dichlormethan extrahiert (4 x 20 ml). Die vereinigten organischen Phasen wurden über $Na_2SO_4$ getrocknet und anschließend eingeengt. Das erhaltene Rohprodukt (800 mg) konnte durch Säulenchromatographie [Kieselgel 60 (50 g); MeOH (500 ml)] gereinigt werden. 4-(2-(4-Benzyl-4-(dimethylamino)cyclohexyl)-1H-indol-3-yl)butyl acetat (unpolareres Produkt) wurde in einer Ausbeute von 120 mg (13 %) als gelber Feststoff erhalten. Das polarere Diastereomer wurde in einer Ausbeute von 87 mg (10 %) als weißer Feststoff erhalten.

**4-(2-(4-Benzyl-4-(dimethylamino)cyclohexyl)-1H-indol-3-yl)butyl acetat Hydrochlorid, unpolares Diastereomer**

4-(2-(4-Benzyl-4-(dimethylamino)cyclohexyl)-1H-indol-3-yl)butyl acetat (unpolareres Diastereomer) (120 mg, 0,27 mmol) wurde in Ethylacetat (10 ml) gelöst und bei RT $Me_3SiCl$ (68 μl, 0,54 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel **174** (117 mg, Fp. 187-191 °C, 90 %) fiel als weißer Feststoff an

**4-(2-(4-Benzyl-4-(dimethylamino)cyclohexyl)-1H-indol-3-yl)butyl acetat Hydrochlorid, polares Diastereomer (175)**

4-(2-(4-Benzyl-4-(dimethylamino)cyclohexyl)-1H-indol-3-yl)butyl acetat (polareres Diastereomer) (87 mg, 0,19 mmol) wurde in Ethylacetat (7 ml) gelöst. Bei RT wurde dann $Me_3SiCl$ (49 μl, 0,39 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel **175** (85 mg, Fp. 194-197 °C, 90 %) wurde als weißer Feststoff erhalten..

**Beispiel 176: 4-(2-(4-Butyl-4-(dimethylamino)cyclohexyl)-1H-indol-3-yl)butyl acetat Hydrochlorid, polares Diastereomer**

**Beispiel 177: 4-(2-(4-Butyl-4-(dimethylamino)cyclohexyl)-1H-indol-3-yl)butyl acetat Hydrochlorid, unpolares Diastereomer**

**4-(2-(4-Butyl-4-(dimethylamino)cyclohexyl)-1H-indol-3-yl)butyl acetat (unpolares und polares Diastereomer)**

Das Keton **Ket-4** (395 mg, 2 mmol) und **Ind-57** (379 mg, 2 mmol) wurden in HBr/Eisessig (33 % HBr, 2 ml) gelöst und 24 h bei RT gerührt. Anschließend wurde zu dem Ansatz bei RT Sn-Pulver (2,4 g, 20 mmol) innerhalb von 1 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von festem $NaHCO_3$ neutralisiert und mit 40 ml $H_2O$ versetzt. Das erhaltene wässrige Gemisch wurde mit Dichlormethan extrahiert (3 x 30 ml). Die vereinigten organischen Phasen wurden über $Na_2SO_4$ getrocknet und anschließend eingeengt. Das erhaltene Rohprodukt konnte durch Säulenchromatographie [Kieselgel 60 (50 g); MeOH (500 ml)] gereinigt werden. 4-(2-(4-Butyl-4-(dimethylamino)cyclohexyl)-1H-indol-3-yl)butyl acetat (unpolares Diastereomer) wurde in einer Ausbeute von 206 mg (25 %) als weißer Feststoff erhalten. Das polarere Diastereomer wurde in einer Ausbeute von 124 mg (15 %) als weißer Feststoff erhalten.

**4-(2-(4-Butyl-4-(dimethylamino)cyclohexyl)-1H-indol-3-yl)butyl acetat Hydrochlorid, polares Diastereomer ( 176)**

Essigsäure-4-[2-(4-butyl-4-dimethylaminocyclohexyl)-1*H*-indol-3-yl]butylester (polares Diastereomer) (124 mg, 0,3 mmol) wurde in Ethylacetat (50 ml) gelöst. Bei RT wurde dann $Me_3SiCl$ (76 μl, 0,39 mmol) zugetropft und 1 h gerührt. Es fiel ein weißer Niederschlag aus. Der Niederschlag wurde abgesaugt, mit Ethylacetat (2 x 5 ml) gewaschen

und anschließend getrocknet. Beispiel **176** (100 mg, Fp. 173-176 °C, 74 %) fiel als weißer Feststoff an

**4-(2-(4-Butyl-4-(dimethylamino)cyclohexyl)-1H-indol-3-yl)butyl acetat Hydrochlorid, unpolares Diastereomer ( 177)**

Essigsäure-4-[2-(4-butyl-4-dimethylaminocyclohexyl)-1*H*-indol-3-yl]butylester (unpolares Diastereomer) (206 mg, 0,5 mmol) wurde in Ethylacetat (50 ml) gelöst. Bei RT wurde dann $Me_3SiCl$ (126 µl, 1 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 177 (150 mg, Fp. 128-131 °C, 67 % wurde als weißer Feststoff erhalten

**Beispiel 178: 4-(2-(4-(Dimethylamino)-4-phenylcyclohexyl)-1H-indol-3-yl)butyl acetat Hydrochlorid, unpolares Diastereomer**

**Beispiel 179: 4-(2-(4-(Dimethylamino)-4-phenylcyclohexyl)-1H-indol-3-yl)butyl acetat Hydrochlorid, polares Diastereomer**

**4-(2-(4-(Dimethylamino)-4-phenylcyclohexyl)-1H-indol-3-yl)butyl acetat (unpolares und polares Diastereomer)**

Das Keton **Ket-10** (434 mg, 2 mmol) und **Ind-57** (379 mg, 2 mmol; wurden in HBr/Eisessig (33 % HBr, 1,75 ml) gelöst und 24 h bei RT gerührt. Anschließend wurde zu dem Ansatz bei RT Sn-Pulver (2,4 g, 20 mmol) innerhalb von 1 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von festem $NaHCO_3$ neutralisiert und mit 40 ml $H_2O$ versetzt. Das erhaltene wässrige Gemisch wurde mit Dichlormethan extrahiert (4 x 20 ml). Die vereinigten organischen Phasen wurden über $Na_2SO_4$ getrocknet und anschließend eingeengt. Das erhaltene Rohprodukt (865 mg) konnte durch Säulenchromatographie [Kieselgel 60 (50 g); MeOH (800 ml)] gereinigt werden. 4-(2-(4-(Dimethylamino)-4-phenylcyclohexyl)-1H-indol-3-yl)butyl acetat (unpolares Diastereomer) wurde in einer Ausbeute von 234 mg (27 %) als weißer Feststoff erhalten. Das polarere Diastereomer wurde in einer Ausbeute von 112 mg (13 %) als weißer Feststoff erhalten.

**4-(2-(4-(Dimethylamino)-4-phenylcyclohexyl)-1H-indol-3-yl)butyl acetat Hydrochlorid, unpolares Diastereomer**

4-(2-(4-(Dimethylamino)-4-phenylcyclohexyl)-1H-indol-3-yl)butyl acetat (unpolares Diastereomer) (234 mg, 0,54 mmol) wurde in Ethylacetat (50 ml) gelöst. Bei RT wurde dann $Me_3SiCl$ (136 µl, 1,08 mmol) zugetropft. Das Gemisch wurde 1 h gerührt. Es fiel ein weißer Niederschlag aus. Der Niederschlag wurde abgesaugt, mit Ethylacetat (2 x 5 ml) gewaschen und anschließend getrocknet. Beispiel 178 (155 mg, Fp. 238-241 °C, 61 %, NMR-Reinheit <95%) wurde als ein weißer Feststoff erhalten.

**4-(2-(4-(Dimethylamino)-4-phenylcyclohexyl)-1H-indol-3-yl)butyl acetat Hydrochlorid, polares Diastereomer**

4-(2-(4-(Dimethylamino)-4-phenylcyclohexyl)-1H-indol-3-yl)butyl acetat (polares Diastereomer) (112 mg, 0,26 mmol) wurde in Ethylacetat (20 ml) gelöst. Bei RT wurde dann $Me_3SiCl$ (65 µl, 0,52 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 179 (97 mg, Fp. 165-169 °C, 80 %) war ein weißer Feststoff.

**Beispiel 180: 3-(2-(4-Benzyl-4-(dimethylamino)cyclohexyl)-1H-indol-3-yl)propan-1-ol, Citrat (1:1), unpolares Diastereomer**

Die freie Base aus Beispiel **48** (300 mg, 0,77 mmol) wurde in HBr/Eisessig (33 % HBr, 10 ml) gelöst. Anschließend wurde zu dem Ansatz bei RT Sn-Pulver (906 mg, 7,7 mmol) innerhalb von 1 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von 2N NaOH (40 ml) basisch gestellt. Das erhaltene wässrige Gemisch wurde mit Dichlormethan extrahiert (4 x 20 ml). Die vereinigten organischen Phasen wurden über $Na_2SO_4$ getrocknet und anschließend eingeengt. Das erhaltene Rohprodukt (250 mg) konnte durch Säulenchromatographie [Kieselgel 60 (20 g); MeOH (500 ml)] gereinigt werden. Erhalten wurde 3-(2-(4-Benzyl-4-(dimethylamino)cyclohexyl)-1H-indol-3-yl)propan-1-ol (unpolareres Diastereomer) in einer Ausbeute von 129 mg (43 %) als farbloses Öl.

Das unpolarere Diastereomer (129 mg, 0,33 mmol) wurde in der Siedehitze in Isopropanol (4 ml) gelöst und mit Citronensäure (64 mg, 0,33 mmol), gelöst in heißem Isopropanol (2 ml), versetzt. Die Lösung wurde im Kühlschrank

auf 5 °C abgekühlt und 16 h stehen gelassen. Der entstandene weiße Niederschlag wurde mittels einer Fritte abgetrennt. Beispiel 180 wurde in einer Ausbeute von 100 mg (52 %, *Schmelzpunkt:* 82-85 °C) erhalten.

**Beispiel 181: 3-(2-(4-Butyl-4-(dimethylamino)cyclohexyl)-1H-indol-3-yl)propan-1-ol Hydrochlorid, polares Diastereomer**

**Beispiel 182: 3-(2-(4-Butyl-4-(dimethylamino)cyclohexyl)-1H-indol-3-yl)propan-1-ol Hydrochlorid, unpolares Diastereomer**

Die freie Base aus Beispiel **49** (300 mg, 0,85 mmol) wurde in HBr/Eisessig (33 % HBr, 20 ml) gelöst. Anschließend wurde zu dem Ansatz bei RT Sn-Pulver (1 g, 8,5 mmol) innerhalb von 1 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von 2N NaOH (80 ml) basisch gestellt. Das erhaltene wässrige Gemisch wurde mit Dichlormethan extrahiert (4 x 20 ml). Die vereinigten organischen Phasen wurden über $Na_2SO_4$ getrocknet und anschließend eingeengt. Das erhaltene Rohprodukt (300 mg) wurde durch Säulenchromatographie [Kieselgel 60 (20 g); MeOH (500 ml)] gereinigt. 3-(2-(4-Butyl-4-(dimethylamino)cyclohexyl)-1H-indol-3-yl)propan-1-ol (unpolareres Diastereomer) wurde in einer Ausbeute von 111 mg (37 %) als farbloses Öl erhalten. Das polarere Produkt wurde in einer Ausbeute von 54 mg (18 %) als farbloses Öl erhalten.

**3-(2-(4-Butyl-4-(dimethylamino)cyclohexyl)-1H-indol-3-yl)propan-1-ol Hydrochlorid, polares Diastereomer**

3-(2-(4-Butyl-4-(dimethylamino)cyclohexyl)-1H-indol-3-yl)propan-1-ol (polareres Diastereomer) (54 mg, 0,15 mmol) wurde in Ethylacetat (5 ml) gelöst. Bei RT wurde dann $Me_3SiCl$ (38 µl, 0,3 mmol) zugetropft und 1 h gerührt. Es fiel ein weißer Niederschlag aus. Der Niederschlag wurde abgesaugt, mit Ethylacetat (2 x 3 ml) gewaschen und anschließend getrocknet. Beispiel 181 (57 mg, Fp. 128-132 °C, 95 %) wurde als weißer Feststoff erhalten.

**3-(2-(4-Butyl-4-(dimethylamino)cyclohexyl)-1H-indol-3-yl)propan-1-ol Hydrochlorid, unpolares Diastereomer**

3-(2-(4-Butyl-4-(dimethylamino)cyclohexyl)-1H-indol-3-yl)propan-1-ol (unpolareres Diastereomer) (110 mg, 0,25 mmol) wurde in Ethylacetat (10 ml) gelöst. Bei RT wurde dann $Me_3SiCl$ (63 µl, 0,5 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 182 (104 mg, Fp. 218-222 °C, 85 %) wurde als weißer Feststoff erhalten.

**Beispiel 183: 3-(2-(4-(Dimethylamino)-4-phenylcyclohexyl)-1H-indol-3-yl)propan-1-ol Hydrochlorid, polares Diastereomer**

**Beispiel 184: 3-(2-(4-(Dimethylamino)-4-phenylcyclohexyl)-1H-indol-3-yl)propan-1-ol Hydrochlorid, unpolares Diastereomer**

**(±)-3-[2-(4-Dimethylamino-4-phenylcyclohex-1-enyl)-1*H*-indol-3-yl]propan-1-ol**

Indol **Ind-16** (701 mg, 4 mmol) wurde zusammen mit Keton **Ket-10** (868 mg, 4 mmol) in Dichlormethan (80 ml) gelöst und mit Trifluormethansulfonsäure (540 µl, 6 mmol) versetzt. Der Ansatz wurde 24 h bei RT gerührt. Zur Aufarbeitung wurde das Reaktionsgemisch mit 5N NaOH (30 ml) versetzt und 20 min bei RT gerührt. Nach Abtrennung der organischen Phase wurde die wässrige Phase mit Dichlormethan (3 x 30 ml) extrahiert. Die vereinigten organischen Extrakte wurden über $Na_2SO_4$ getrocknet und anschließend eingeengt. Das erhaltene Rohprodukt (1,55 g) konnte durch Säulenchromatographie [Kieselgel 60 (100 g); MeOH (500 ml)] gereinigt werden. (±)-3-[2-(4-Dimethylamino-4-phenylcyclohex-1-enyl)-1H-indol-3-yl]propan-1-ol wurde in einer Ausbeute von 985 mg (66 %) als weißer Feststoff erhalten.

**3-(2-(4-(Dimethylamino)-4-phenylcyclohexyl)-1H-indol-3-yl)propan-1-ol (unpolares und polares Diastereomer)**

(±)-3-[2-(4-Dimethylamino-4-phenylcyclohex-1-enyl)-1*H*-indol-3-yl]propan-1-ol (500 mg, 1,335 mmol) wurde in HBr/Eisessig (33 % HBr, 10 ml) gelöst. Anschließend wurde zu dem Ansatz bei RT Sn-Pulver (1,57 g, 13,35 mmol) innerhalb von 2 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von 5N NaOH (40 ml) basisch gemacht. Das erhaltene wässrige Gemisch wurde mit Dichlormethan extrahiert (4 x 20 ml). Die vereinigten organischen Phasen wurden

über $Na_2SO_4$ getrocknet und anschließend eingeengt. Das erhaltene Rohprodukt konnte durch Säulenchromatographie [Kieselgel 60 (50 g); MeOH (600 ml)] gereinigt werden. 3-(2-(4-(Dimethylamino)-4-phenylcyclohexyl)-1H-indol-3-yl)propan-1-ol (unpolares Diastereomer) wurde in einer Ausbeute von 160 mg (32 %) als weißer Feststoff erhalten. Das polarere Diastereomer wurde in einer Ausbeute von 130 mg (26 %) als weißer Feststoff erhalten.

**3-(2-(4-(Dimethylamino)-4-phenylcyclohexyl)-1H-indol-3-yl)propan-1-ol Hydrochlorid, polares Diastereomer (Bsp. 183)**

3-(2-(4-(Dimethylamino)-4-phenylcyclohexyl)-1H-indol-3-yl)propan-1-ol (polares Diastereomer) (130 mg, 0,35 mmol) wurde in Ethylacetat (15 ml) gelöst. Bei RT wurde dann $Me_3SiCl$ (88 μl, 0,7 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 183 (125 mg, Fp. 210-214 °C, 87 %, NMR-Reinheit <95%) war ein weißer Feststoff.

**3-(2-(4-(Dimethylamino)-4-phenylcyclohexyl)-1H-indol-3-yl)propan-1-ol Hydrochlorid, unpolares Diastereomer (Bsp 184)**

3-(2-(4-(Dimethylamino)-4-phenylcyclohexyl)-1H-indol-3-yl)propan-1-ol (unpolares Diastereomer) (160 mg, 0,43 mmol) wurde in Ethylacetat (20 ml) gelöst. Bei RT wurde dann $Me_3SiCl$ (108 μl, 0,86 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 184 (159 mg, Fp. 248-250 °C, 90 %, NMR-Reinheit <95%) fiel als weißer Feststoff an.

**Beispiel 185: 3-(2-(4-(Dimethylamino)-4-phenylcyclohexyl)-1H-indol-3-yl)propyl acetat Hydrochlorid, polares Diastereomer**

**Beispiel 186: 3-(2-(4-(Dimethylamino)-4-phenylcyclohexyl)-1H-indol-3-yl)propyl acetat Hydrochlorid, unpolares Diastereomer**

**3-(2-(4-(Dimethylamino)-4-phenylcyclohexyl)-1H-indol-3-yl)propyl acetat (unpolares und polares Diastereomer)**

Das Keton Ket-10 (434 mg, 2 mmol) und Ind-16 (350 mg, 2 mmol) wurden in HBr/Eisessig (33 % HBr, 2 ml) gelöst und 24 h bei RT gerührt. Anschließend wurde zu dem Ansatz bei RT Sn-Pulver (2,4 g, 20 mmol) innerhalb von 1 h portionsweise gegeben. Nach Beendigung der Zugabe wurde das Reaktionsgemisch weitere 16 h bei RT gerührt. Zur Aufarbeitung wurde das Gemisch mit EtOH (10 ml) verdünnt und am Rotationsverdampfer bis zur Trockene eingeengt. Der verbliebene Rückstand wurde durch Zugabe von festem $NaHCO_3$ neutralisiert und mit 40 ml $H_2O$ versetzt. Das erhaltene wässrige Gemisch wurde mit Dichlormethan extrahiert (4 x 30 ml). Die vereinigten organischen Phasen wurden über $Na_2SO_4$ getrocknet und anschließend eingeengt. Das erhaltene Rohprodukt (640 mg) konnte durch Säulenchromatographie [Kieselgel G (50 g); MeOH (600 ml)] gereinigt werden. 3-(2-(4-(Dimethylamino)-4-phenylcyclohexyl)-1H-indol-3-yl)propyl acetat (unpolareres Diastereomer) wurde in einer Ausbeute von 190 mg (25 %) als weißer Feststoff erhalten. Das polarere Diastereomer wurde in einer Ausbeute von 60 mg (8 %) als weißer Feststoff erhalten.

**3-(2-(4-(Dimethylamino)-4-phenylcyclohexyl)-1H-indol-3-yl)propyl acetat Hydrochlorid, polares Diastereomer (Bsp 185)**

3-(2-(4-(Dimethylamino)-4-phenylcyclohexyl)-1H-indol-3-yl)propyl acetat (polareres Diastereomer) (60 mg, 0,16 mmol) wurde in Ethylacetat (20 ml) gelöst. Bei RT wurde dann $Me_3SiCl$ (40 μl, 0,32 mmol) zugetropft und 1 h gerührt. Es fiel ein weißer Niederschlag aus. Der Niederschlag wurde abgesaugt, mit Ethylacetat (1 x 5 ml) gewaschen und anschließend getrocknet. Beispiel 185 (55 mg, Fp. 229-232 °C, 84 %) war ein weißer Feststoff.

$^1$*H NMR (400 MHz, DMSO-$d_6$) δppm:* 1.40-1.59 (m, 2H), 1.71-187 (m, 3H), 1.87-2.02 (m, 1H), 2.07 (s, 3H), 2.19-2.33 (m, 2H), 2.44 (d, *J* = 4.73, 6H), 2.58-2.70 (m, 2H), 2.82-2.96 (m, 1H), 3.09 (d, *J* = 12.32 Hz, 2H), 3.91 (t, *J* = 6.36, 6.36 Hz, 2H), 6.81-6.98 (m, 2H), 7.15 (d, *J* = 7.77 Hz, 1H), 7.35 (d, *J* = 7.67 Hz, 1H), 7.52-7.65 (3H), 7.70-7.81 (m, 2H), 10.40 (s, 1H), 11.20-11.31 (m, 1H),

$^{13}$*C NMR (101 MHz, DMSO-$d_6$)* δ ppm 19.7, 20.9, 28.6, 29.3, 30.4, 34.8, 37.0, 63.1, 68.3, 108.1, 110.7, 117.4, 118.0, 120.0, 127.7, 129.0, 129.4, 129.6, 130.4, 135.3, 138.4, 170.4

**3-(2-(4-(Dimethylamino)-4-phenylcyclohexyl)-1H-indol-3-yl)propyl acetat Hydrochlorid, unpolares Diastereomer**

3-(2-(4-(Dimethylamino)-4-phenylcyclohexyl)-1H-indol-3-yl)propyl acetat (unpolareres Diastereomer) (190 mg,

0,51 mmol) wurde in Ethylacetat (50 ml) gelöst. Bei RT wurde dann $Me_3SiCl$ (128 μl, 1,02 mmol) zugetropft. Das Gemisch wurde 1 h gerührt. Es fiel ein weißer Niederschlag aus. Der Niederschlag wurde abgesaugt, mit Ethylacetat (2 x 5 ml) gewaschen und anschließend getrocknet. Beispiel **186** (120 mg, Fp. 217-220 °C, 58 %) war ein weißer Feststoff *$^1H$ NMR (300 MHz, DMSO-$d_6$) δ ppm:* 1.68-2.03- (m, 10H), 2.37-2.47 (m, 1 H), 2.54- 2.62 (m, 6H), 2.68-2.78 (m, 2H), 2.98-3.11 (m, 1H), 3.12-3.22 (m, 2H), 3.93 (t, *J* = 6.50, 6.50 Hz, 2H), 6.87-7.06 (m, 2H), 7.25 (d, *J* = 7.80 Hz, 1H), 7.42 (d, *J* = 7.80, 1H) 7.48-7.60 (m, 3H), 7.67-7.82- (m, 2H), 10,00 (s, 1H), 11.34 (s, 1H)

**Beispiel 187: 1-(2-(2-(4-(Dimethylamino)-4-phenylcyclohex-1-enyl)-1H-indol-3-yl)ethyl)pyrrolidin-2,5-dion**

Das Indol (Ind-61, 3.00 g, 12.4 mmol) und das Aminoketon (Ket-10, 2.70 g, 12.4 mmol) wurden in Dichlormethan (160 m) gelöst, Trifluormethansäure (3.0 ml) addiert und über Nacht bei RT gerührt. Der Ansatz wurde mit 3.5N NaOH (50 ml) versetzt, die wässrige Phase abgetrennt, mit $CH_2Cl_2$ (zweimal 140 ml) extrahiert, die organischen Phasen vereinigt, getrocknet ($Na_2SO_4$) und i. Vak. eingeengt. Der Rückstand wurde durch Flash-Chromatographie mit MeOH/ Essigester (1:1) gereinigt.
*Ausbeute:* 3.30 g (57 %)
*$^1H$-NMR (DMSO-$d_6$):* 1.68 (2 H, m); 2.18 (8 H, m); 2.62 (6 H, m); 2.85 (2 H, t); 3.47 (2 H, t); 6.30 (1 H, s); 6.96 (2 H, m): 7.21 (4 H, m); 7.46 (2 H, m); 10.69 (1 H, bs).

**1-(2-(2-(4-(Dimethylamino)-4-phenylcyclohexyl)-1H-indol-3-yl)ethyl)pyrrolidin-2,5-dion : Unpolareres Diastereomer**

Das soeben hergestellten Olefin (1.50 g, 3.00 mmol) wurde in 33 %-iger HBr in Eisessig (50 ml) gelöst. Unter Eiskühlung wurde Zinnpulver (3.56 g, 30 mmol) portionsweise innerhalb einer Stunde addiert. Nach 1 h wurde der Ansatz i. Vak. eingeengt. Nach Zugabe von 5N NaOH (100 ml) und $CH_2Cl_2$ (50 ml) wurde die wässrige Phase abgetrennt, mit $CH_2Cl_2$ (zweimal 50 ml) extrahiert, die organischen Phasen vereinigt, getrocknet ($Na_2SO_4$) und i. Vak. eingeengt. Der Rückstand wurde durch Flash-Chromatographie mit Methanol/Essigester (1:1) gereinigt. Sowohl das polarere als auch das unpolarere Diastereomer wurde erhalten.

*Ausbeute:* 350 mg (27 %) Unpolareres Diastereomer
484 mg (37 %) Polareres Diastereomer

Das soeben erhaltene unpolarere Diastereomer wird als Beispiel 187 geführt. *$^1H$-NMR (DMSO-$d_6$):* 1.47 (2 H, m); 1.68 (2 H, m); 2.13 (8 H, m); 2.46 (2 H, m); 2.87 (4 H, m); 3.51 (2 H, t); 6.98 (2 H, m); 7.38 (7 H, m); 10.76 (1 H, bs).

**Beispiel 188: 1-(2-(2-(4-(Dimethylamino)-4-phenylcyclohexyl)-1H-indol-3-yl)ethyl)pyrrolidin-2,5-dion : polareres Diastereomer**

Das unter Beispiel **187** gewonnene polarere Diastereomer wir als Beispiel 188 geführt. *Ausbeute:* 484 mg (37 %) *$^1H$-NMR (DMSO-$d_6$):* 1.48 (2 H, m); 1.66 (4 H, m); 1.99 (6 H, s); 2.59 (4 H, m); 2.81 (4 H, m); 3.50 (2 H, m); 6.91 (2 H, m); 7.17 (1 H, m); 7.38 (6 H, m); 10.42 (1 H, bs).

**Beispiel 189: (±)-2-(4-(Dimethylamino)-4-phenylcyclohex-1-enyl)-3-(2-(3,4-dihydroquinolin-1(2H)-yl)ethyl)-1H-indol**

Das Amino-Keton (**Ket-10,** 2.58 g, 11.9 mmol) wurde mit dem Indol (**Ind-62,** 3.28 g, 11.9 mmol) in Di-chlormethan (200 ml) gelöst. Anschließend erfolgte bei Raumtemperatur eine schnelle Zugabe der Trifluormethansulfonsäure (3.62 ml, 41.7 mmol). Der Ansatz rührte bei Raumtemperatur 24 h. Zur Aufarbeitung wurde der Ansatz mit 2N NaOH (200 ml) versetzt und bei Raumtemperatur 20 min gerührt. Die organische Phase wurde abgetrennt und die wässrige Phase mit Dichlormethan (2 x 25 ml) extrahiert. Die vereinigten organischen Phasen wurden über $Na_2SO_4$ getrocknet und i. Vak. zur Trockene eingeengt. Das Rohprodukt wurde mittels Flash-Chromatographie mit Cyclohexan/EE (2:1→1:1→EE) und Methanol gereinigt, wobei das Produkt erst mit Methanol vollständig erhalten wurde.
*Ausbeute:* 2.22 g (40 %)
*$^1H$-NMR ($CDCl_3$):* 1.80 (4 H, m); 2.09 (8 H, m); 2.64 (4 H, m); 2.87 (2 H, m); 3.09 (2 H; m); 6.22 (1 H, s); 6.48 (1 H, t); 6.61 (1 H, d); 7.02 (4 H, m); 7.26 (4 H, m); 7.45 (3 H, m); 10.69 (1 H, s).

**4-(3-(2-(3,4-Dihydroquinolin-1(2H)-yl)ethyl)-1H-indol-2-yl)-N,N-dimethyl-1-phenylcyclohexanamin, Citrat (4:3) : Unpolareres Diastereomer**

Zu einer Lösung des soeben hergestellten Olefins (2.00 g, 4.2 mmol) in HBr/Eisessig (85 ml) wurde innerhalb von 20 min Zinn (5.00 g) zugegeben. Das Reaktionsgemisch rührte anschließend bei Raumtemperatur 2 h nach. Das Gemisch wurde mit Ethanol versetzt und i. Vak. bis zur Trockene eingeengt. Der Rückstand wurde mit 5N NaOH (120 ml) versetzt und mit Dichlormethan (3 x 50 ml) extrahiert. Die vereinigten organischen Phasen wurden über $Na_2SO_4$ getrocknet. Das Rohprodukt wurde mittels Flash-Chromatographie und Cyclohexan/EE (3:1→1:1 und Methanol) gereinigt, wobei das unpolare Produkt mit Cyclohexan/EE (1:1) und das polare Produkt mit Methanol erhalten wurde.

*Ausbeute:* 0.140 g (7 %), unpolares Diastereomer
0.650 g (33 %), polares Diastereomer

Das soeben erhaltene unpolarere Diastereomer (0.140 g, 0.293 mmol) wurde in heißem Ethanol (4 ml) gelöst und bei Raumtemperatur mit Citronensäure (0.056 g, 0.29 mmol), gelöst in heißem Ethanol (2 ml), versetzt. Der Ansatz rührte bei Raumtemperatur 2 h. Da keine Niederschlagsbildung beobachtet werden konnte, wurde die Lösung i. Vak. eingeengt und der Rückstand mit Ether durchgerührt. Es verblieb ein Feststoff, welcher abgesaugt und i. Vak. getrocknet wurde.
*Ausbeute:* 74 mg (Bsp 189, 8 %), poröser Feststoff
*$^1$H-NMR (DMSO-$d_6$):* 1.66 (6 H, m); ); 2.11 (6 H, m); 2.58 (4 H, m); 2.63 (4 H; m); 2.88 (4 H, m); 6.41 (2 H, m); 6.63 (1 H, m); 6.95 (3 H, m); 7.34 (6 H, m); 10.64 (1 H, s), Citrat.

**Beispie 190: 4-(3-(2-(3,4-Dihydroquinolin-1(2H)-yl)ethyl)-1H-indol-2-yl)-N,N-dimethyl-1-phenylcyclohexanamin, Citrat (1:1) : Polareres Diastereomer**

Das unter Beispiel 189 hergestellte polarere Diastereomer (0.650 g, 1.36 mmol) wurde in heißem Ethanol (20 ml) gelöst und bei Raumtemperatur mit Citronensäure (0.260 g, 1.36 mmol), gelöst in heißem Ethanol (10 ml), versetzt. Der Ansatz rührte bei Raumtemperatur 2 h. Da keine Niederschlagsbildung beobachtet werden konnte, wurde die Lösung i. Vak. eingeengt und der Rückstand mit Ether durchgerührt. Es verblieb ein Feststoff, welcher abgesaugt und i. Vak. getrocknet wurde.
*Ausbeute:* 272 mg (Bsp **190,** 30 %), poröser Feststoff
*$^1$H-NMR (DMSO-$d_6$): ):* 1.52 (2 H, m); ); 1.80 (6 H, m); 2.36 (6 H; s); 2.64 (4 H, m); 2.86 (4 H, m); 2.97 (4 H, m); 6.50 (1 H, t); 6.62 (1 H, d); 6.91 (2 H, m); 7.03 (1 H, m); 7.17 (1 H, m); 7.54 (6 H, m); 10.46 (1 H, s), Citrat.

**Beispiel 191: Methyl 1-(2-(2-(4-(dimethylamino)-4-phenylcyclohex-1-enyl)-1H-indol-3-yl)ethyl)-1H-1,2,3-triazol-4-carboxylat**

3-[2-(1*H*-Indol-3-yl)-ethyl)-3*H*-(1,2,3)triazol-4-carbonsäure-methylester (320 mg, 1.18 mmol) und 4-Dimethylamino-4-phenyl-cyclohexanon (256 mg, 1.18 mmol) wurden in abs. Dichlormethan (15 ml) gelöst und schnell mit Trifluormethansulfonsäure (0.21 ml, 2.4 mmol) versetzt. Der Ansatz wurde über Nacht bei RT gerührt, dabei setzte sich ein dunkles Öl ab. Nach Zugabe von 1 N NaOH (10 ml) und $CH_2Cl_2$ (10 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9: 1) gereinigt.
*Ausbeute:* 245 mg (44 %)
*$^1$H-NMR (DMSO-$d_6$):* 1.71-2.60 (12 H, m); 3.28 (2 H; m); 3.80 (3 H, s); 4.49 (2 H, m); 6.06 (1 H, bs); 6.92 (2 H, m); 7.24 (4 H, m); 7.44 (3 H, m); 8.62 (1 H, s); 10.72 (1 H, s).
*$^{13}$C-NMR (DMSO-$d_6$):* 25.61; 26.53; 27.00; 32.43; 38.14; 50.30; 51.63; 60.11; 105.42; 110.82; 117.74; 118.54; 121.08; 126.13; 126.36; 126.97; 127.43; 127.99; 128.99; 129.29; 135.07; 136.77; 138.31; 142.57; 160.74.

**Methyl 1-(2-(2-(4-(dimethylamino)-4-phenylcyclohexyl)-1H-indol-3-yl)ethyl)-1H-1,2,3-triazol-4-carboxylat, Citrat (1:1) Unpolareres Diastereomer**

Zu einer Lösung des soeben hergestellten Olefins (218 mg, 0.46 mmol) in HBr/Eisessig (10 ml) wurde innerhalb von 20 min Zinn (0.60 g) gegeben und 3 h bei RT gerührt. Der Ansatz wurde mit Ethanol versetzt, das Lösungsmittelgemisch bis zur Trockne eingeengt und der Rückstand in 5N NaOH (20 ml) und Dichlormethan (30 ml) gelöst. Die Phasen wurden getrennt, die wässrige Phase zweimal mit $CH_2Cl_2$ ex-trahiert, die vereinigten organischen Phasen mit

Wasser gewaschen und über $Na_2SO_4$ getrocknet. Die Dichlormethanphase wurde über Celite abgesaugt (war trüb) und die Lösung i. Vak. eingeengt. Der verbliebene Rückstand wurde durch Flash-Chromatographie mit $CHCl_3$/MeOH (20: 1→9:1) gereinigt.

*Ausbeute:* 70 mg (32 %) unpolares Diastereomer
39 mg (18 %) polares Diastereomer

Das erhaltene unpolarere Diastereomer (66 mg, 0.14 mmol) wurde in heißem Ethanol (3 ml) gelöst und mit einer Lösung von Citronensäure (27 mg, 0.14 mmol) in heißem Ethanol (2 ml) versetzt. Das Citrat fiel nicht aus, deshalb wurde das Lösungsmittel entfernt und der Rückstand i. Vak. getrocknet.
*Ausbeute:* 93 mg (100 %)
*$^1$H-NMR (DMSO-$d_6$):* 1.40 (4 H, m); 2.04 (8 H; m); 2.58 (1 H, m) 2.72 (2 H, m); 3.26 (2 H, t); 3.79 (3 H, s); 4.60 (2 H, t); 6.94 (2 H, t); 7.38 (6 H, m); 7.47 (1 H, m); 8.73 (1 H, s); 10.73 (1 H, bs), freie Base.

**Beispiel 192: Methyl 1-(2-(2-(4-(dimethylamino)-4-phenylcyclohexyl)-1H-indol-3-yl)elhyl)-1H-1,2,3-triazol-4-carboxylat, Citrat (1:1) : Polareres Diastereomer)**

Das unter Beispiel 191 gewonnene polarere Diastereomer (39 mg, 0.082 mmol) wurde in heißem Ethanol (2 ml) gelöst und mit einer Lösung von Citronensäure (16 mg, 0.082 mmol) in heißem Ethanol (2 ml) versetzt. Das Citrat fiel nicht aus, deshalb wurde das Lösungsmittel entfernt und der Rückstand i. Vak. getrocknet.
*Ausbeute:* 55 mg (100 %)
*$^1$H-NMR (DMSO-$d_6$):* 1.39 (4 H, m); 1.64 (2 H m); 1.98 (6 H; m); 2.50 (1 H, m); 2.71 (2 H, m); 3.22 (2 H, t); 3.85 (3 H, s); 4.57 (2 H, t); 6.92 (2 H, t); 7.17 (1 H, m); 7.42 (6 H, m); 8.72 (1 H, s); 10.40 (1 H, bs), freie Base.

**Beispiel 193: (±)-2-(4-(Dimethylamino)-4-phenylcyclohex-1-enyl)-3-(2-(isoindolin-2-yl)ethyl)-1H-indol**

Indol (**Ind-64,** 1.05 g, 4.0 mmol) und Keton (**Ket-10;** 868 mg, 4.0 mmol) wurden in abs. Dichlormethan (30 ml) gelöst und schnell mit Trifluormethansulfonsäure (1.06 ml, 12.0 mmol) versetzt. Der Ansatz wurde über Nacht bei RT gerührt, dabei setzte sich ein dunkelbraunes Öl ab. Nach Zugabe von 1 N NaOH (20 ml) und $CH_2Cl_2$ (30 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (20:1) gereinigt.
*Ausbeute:* 605 mg (33 %), poröser Feststoff
*$^1$H-NMR (DMSO-$d_6$):* 1.83 (1 H, m); 2.16 (7 H, m); 2.57 (1 H, m); 2.72 (4 H, m); 2.88 (2 H, m); 3.80 (4 H, s); 6.22 (1 H, s); 6.98 (2 H, s); 7.26 (8 H, m); 7.47 (3 H, m); 10.66 (1 H, s).

**4-(3-(2-(Isoindolin-2-yl)ethyl)-1H-indol-2-yl)-N,N-dimethyl-1-phenylcyclohexanamin, Citrat (1:1) : Unpolareres Diastereomer**

Zu einer Lösung de soeben hergestellten Olefins (588 mg, 1.27 mmol) in HBr/Eisessig (30 ml) wurde innerhalb von 30 min Zinn (1.50 g) gegeben und 4 h bei RT gerührt. Der Ansatz wurde mit Ethanol versetzt, das Reaktionsgemisch bis zur Trockne eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (20:1 → 9:1) getrennt.

*Ausbeute:* 304 mg (52 %), unpolares Diastereomer
108 mg (18 %), polares Diastereomer

Das soeben erhaltene unpolarere Diastereomer (264 mg, 0.57 mmol) wurde in heißem Ethanol (5 ml) gelöst und mit einer Lösung von Citronensäure (109 mg, 0.57 mmol) in heißem Ethanol (5 ml) versetzt. Nach 2- stündigem Stehen im Kühlschrank wurde der entstandene Feststoff abgesaugt und i. Vak. getrocknet.
*Ausbeute:* 289 mg (77%); *Schmelzpunkt:* 225 °C
*$^1$H-NMR (DMSO-$d_6$):* 1.66 (4 H, m); 2.08 (6 H, m); 2.18 (2 H, m); 2.57-2.66 (4 H, m); 2.85 (2 H, d); 3.04 (3 H, m); 3.20 (2 H, t); 4.36 (4 H, s); 6.98 (2 H, s); 7.32 (10 H, m); 7.49 (1 H, m); 10.75 (1 H, s).
*Citrat:* (Ethanol)

**Beispiel 194: 4-(3-(2-(Isoindolin-2-yl)ethyl)-1H-indol-2-yl)-N,N-dimethyl-1-phenylcyclohexanamin, Citrat (1:1): Polareres Diastereomer**

Das unter Beispiel **193** gewonnene polarere Diastereomer (93 mg, 0.20 mmol) wurde in heißem Ethanol (3 ml) gelöst und mit einer Lösung von Citronensäure (38 mg, 0.20 mmol) in heißem Ethanol (2 ml) versetzt. Nach 2- stündigem Stehen im Kühlschrank wurde der entstandene Feststoff abgesaugt und i. Vak. getrocknet.
*Ausbeute:* 103 mg (78 %), poröser Feststoff
*$^1$H-NMR (DMSO-$d_6$):* 1.50 (2 H, m); 1.80 (2 H, m); 1.98 (2 H, m); 2.32 (6 H, s); 2.58 (4 H, m); 2.96 (6 H, m); 4.15 (4 H, s); 6.91 (2 H, s); 7.15-7.34 (5 H, m); 7.43-7.65 (5 H, m); 10.48 (1 H, s).
*Citrat:* (Ethanol), poröser Feststoff

**Beispiel 195: (±)-2-(4-(Dimethylamino)-4-phenylcyclohex-1-enyl)-3-(2-(3,4-dihydroisoquinolin-2(1H)-yl)ethyl)-1H-indol**

Indol (Ind-65, 1.10 g, 4.0 mmol) und Keton (Ket-10, 868 mg, 4.0 mmol) wurden in abs. Dichlormethan (30 ml) gelöst und schnell mit Trifluormethansulfonsäure (1.06 ml, 12.0 mmol) versetzt. Der Ansatz wurde über Nacht bei RT gerührt, dabei setzte sich ein dunkelbraunes Öl ab. Nach Zugabe von 1N NaOH (20 ml) und $CH_2Cl_2$ (30 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (20:1) gereinigt.
*Ausbeute:* 637 mg (33 %), poröser Feststoff
*$^1$H-NMR (DMSO-$d_6$):* 1.75 (2 H, m); 1.94 (2 H, m); 2.11 (6 H, s); 2.55-2.89 (8 H, m); 3.82 (1 H, m); 6.22 (1 H, s); 6.91 (13 H, m); 10.62 (1 H, s).

**4-(3-(2-(3,4-Dihydroisoquinolin-2(1H)-yl)ethyl)-1H-indol-2-yl)-N,N-dimethyl-1-phenylcyclohexanamin, Citrat (1: 1) : Unpolareres Diastereomer**

Zu einer Lösung von de soeben hergestellten Olefins (600 mg, 1.26 mmol) in HBr/Eisessig (30 ml) wurde innerhalb von 30 min Zinn (1.50 g) gegeben und 4 h bei RT gerührt. Der Ansatz wurde mit Ethanol versetzt, das Reaktionsgemisch bis zur Trockne eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$, getrocknet und i. Vak. eingeengt. Der verbliebene Rückstand wurde durch Flash-Chromatographie mit $CHCl_3$/MeOH (20:1 → 9:1) getrennt.

*Ausbeute:* 207 mg (34 %), unpolares Diastereomer
128 mg (21 %), polares Diastereomer

Das erhaltene unpolarere Diastereomer (195 mg, 0.41 mmol) wurde in heißem Ethanol (10 ml) gelöst und mit einer Lösung von Citronensäure (78 mg, 0.41 mmol) in heißem Ethanol (3 ml) versetzt. Nach 2- stündigem Stehen im Kühlschrank wurde der entstandene Feststoff abgesaugt und i. Vak. getrocknet.
*Ausbeute:* 197 mg (72 %); *Schmelzpunkt:* 153-154 °C
*$^1$H-NMR (DMSO-$d_6$):* 1.60 (4 H, m); 2.08 (6 H, m); 2.17 (2 H, m); 2.57-2.61 (6 H, m); 3.02 (8 H, m); 4.14 (2 H, s); 6.98 (2 H, m); 7.16 (3 H, m); 7.34 (6 H, m); 7.49 (1 H, m); 10.77 (1 H, s).
*Citrat:* (Ethanol)

**Beispiel 196: 4-(3-(2-(3,4-Dihydroisoquinolin-2(1H)-yl)ethyl)-1 H-indol-2-yl)-N,N-dimethyl-1-phenylcyclohexanamin, Citrat (2:3) : Polareres Diastereomer**

Das unter Beispiel **195** gewonnene polarere Diastereomer (102 mg, 0.21 mmol) wurde in heißem Ethanol (3 ml) gelöst und mit einer Lösung von Citronensäure (41 mg, 0.21 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 75 mg (52 %), poröser Feststoff
*$^1$H-NMR (DMSO-$d_6$):* 1.53 (2 H, m); 1.81 (2 H, m); 2.01 (2 H, m); 2,37 (6 H, s); 2.60 (4 H, m); 2.90 (10 H, m); 4.00 (2 H, t); 6.93 (2 H, m); 7.18 (5 H, m); 7.17-7.68 (6 H, m); 10.48 (1 H, s). *Citrat:* (Ethanol), poröser Feststoff

**Beispiel 197: (±)-2-(4-(Dimethylamino)-4-phenylcyclohex-1-enyl)-3-(2-(pyrrolidin-1-yl)ethyl)-1H-indol**

Indol (**Ind-66,** 1.00 g, 4.7 mmol) und Keton (**Ket-10,** 1.01 g, 4.7 mmol) wurden bei RT in abs. $CH_2Cl_2$ (50 ml) gelöst und zügig mit Trifluormethansulfonsäure versetzt. Anschließend fiel ein schwarzes Öl aus. Die Mischung wurde 24 h bei RT gerührt, 1 N NaOH (50 ml) zugegeben und das Gemisch 20 min gerührt. Die organische Phase wurde abgetrennt und die wässrige Phase mit $CH_2Cl_2$ (2 x 50 ml) extrahiert. Die vereinigten organische Phasen wurden mit Wasser (20 ml) gewaschen, über $Na_2SO_4$ getrocknet, filtriert und i. Vak. eingeengt. Der Rückstand wurde durch Flash-Chromatographie mit Chloroform/Methanol (9/1 + 1 % Triethylamin) gereinigt.
*Ausbeute:* 789 mg (41 %), gelber Feststoff
*$^1$H-NMR (DMSO-$d_6$):* 1.75 (4 H, m); 2.09 (8 H; m); 2.62 (6 H, m); 2.82 (4 H, m); 6.19 (1 H, s); 6.97 (2 H, m); 7.21-7.48 (7 H, m); 10.62 (1 H, s).

**N,N-Dimethyl-1-phenyl-4-(3-(2-(pyrrolidin-1-yl)ethyl)-1H-indol-2-yl)cyclohexanamin, Citrat (1:1) : Unpolareres Diastereomer**

Das soeben hergestellte Olefin (666 mg, 1.6 mmol) wurde in 33%-iger HBr/Eisessig (35 ml) gelöst und bei RT innerhalb von 30 min portionsweise mit Zinn (2.10 g) versetzt. Das Gemisch wurde 4 h bei RT gerührt. Ethanol wurde zugegeben, das Lösungsmittelgemisch i. Vak. entfernt und der Rückstand mit 5N NaOH (70 ml) und $CH_2Cl_2$ (100 ml) gelöst. Die Phasen wurden getrennt, die wässrige Phase mit $CH_2Cl_2$ (2x) extrahiert, die vereinigten organischen Phasen mit Wasser gewaschen, über $Na_2SO_4$ getrocknet, filtriert und i. Vak. eingeengt. Der verbliebene Rückstand wurde durch Flash-Chromatographie mit Chloroform/Methanol (20:1 → 9:1) gereinigt.
*Ausbeute:* 544 mg (82 %), unpolare Verbindung, enthielt Triethylamin und Verunreinigung
259 mg (39 %), polare Verbindung, enthielt Triethylamin und Verunreinigung Bei der Umsetzung des soeben erhaltenen unpolareren Diastereomers mit einer molaren Menge an Citronensäure in Ethanol fiel das Citrat als gelber Feststoff aus.
*Ausbeute:* 308 mg, unpolares Diastereomer; *Schmelzpunkt:* 209-210 °C
*$^1$H-NMR (DMSO-$d_6$):* 1.57 (4 H, m); 1.94 (4 H, m); 2.08 (8 H, m); 2.58 (4 H, m); 2.83-3.41 (10 H, m); 6.99 (2 H, m); 7.41 (6 H, m); 7.51 (1 H, m); 10.88 (1 H, s).

**Beispiel 198: N,N-Dimethyl-1-phenyl-4-(3-(2-(pyrrolidin-1-yl)ethyl)-1H-indol-2-yl)cyclohexanamin, Citrat (1:1) Polareres Diastereomer**

Bei der Umsetzung des unter Beispiel **197** erhaltenen polareren Diastereomers mit einer molaren Menge an Citronensäure in Ethanol fiel nur wenig Niederschlag, nach Zugabe von Ether fiel das Citrat als gelber Feststoff aus.
*Ausbeute:* 204 mg, polares Diastereomer; *Schmelzpunkt:* amorpher Feststoff
*$^1$H-NMR (DMSO-$d_6$):* 1.15 (2 H, m); 1.44 (2 H, m); 1.75 (2 H, m); 1.94-2.06 (5 H, m); 2.30 (4 H, m); 2.58 (4 H, m); 3.09 (6 H, m); 3.43 (5 H, m); 6.94 (2 H, m); 7.18 (1 H, m); 7.53 (6 H, m); 10.55 (1 H, s).

**Beispiel 199: N,N-Dimethyl-1-phenyl-4-(3-(2-(piperidin-1-yl)ethyl)-1H-indol-2-yl)cyclohexanamin, Citrat (1:1) : Eines von 2 möglichen Diastereomeren**

Zu einer Lösung von Beispiel 58 (0.209 g, 0.488 mmol) in HBr/Eisessig (10 ml) wurde innerhalb von 20 min Sn-Pulver (0.57g) gegeben. Das Reaktionsgemisch rührte anschließend bei Raumtemperatur 2.5 h. Das Gemisch wurde mit Ethanol versetzt und i. Vak. bis zur Trockene eingeengt. Der Rückstand wurde mit 5N NaOH (20 ml) versetzt und mit Dichlormethan (3 x 20 ml) extrahiert, die organische Phase mit $Na_2SO_4$ getrocknet, i. Vak. eingeengt und der Rückstand mittels Flash-Chromatographie mit Chloroform/Methanol (15:1) gereinigt.
*Ausbeute:* 0.05 g (24 %)
Das soeben erhaltene Reduktionsprodukt (0.050 g, 0.116 mmol) wurde in heißem Ethanol (1.4 ml) gelöst und mit Citronensäure (0.022 g, 0.116 mmol), gelöst in heißem Ethanol (0.865 ml), versetzt. Nach 2 h entstand ein Niederschlag, welcher abfiltriert, mit Ethanol gewaschen und i. Vak. getrocknet wurde.
*Ausbeute:* 0.055 g; *Schmelzpunkt:* 214-215 °C
*$^1$H-NMR (DMSO-$d_6$):* 1.51-1.72 (10 H, m); 2.05 (6 H, s); 2.19 (6 H, m); 2.56 (4 H, m); 2.92 (4 H, m); 3.04 (2 H, s); 6.94 (3 H, m); 7.29 (2 H, m); 7.40 (3 H, m); 7.48 (1 H, m); 10.82 (1 H, s).

**Beispiel 200: (±)-2-(4-(Dimethylamino)-4-phenylcyclohex-1-enyl)-3-(2-(4-methylpiperazin-1-yl)ethyl)-1 H-indol**

Indol (**Ind-68,** 1.23 g, 5.05 mmol) und Keton (**Ket-10,** 1.09 g, 5.05 mmol) wurden unter Argon in abs. $CH_2Cl_2$ (60 ml) vorgelegt und mit Trifluormethansulfonsäure (1.77 ml, 20.2 mmol) versetzt. Der Ansatz wurde über Nacht bei RT gerührt. Zur Aufarbeitung wurde die Lösung mit 1 N NaOH versetzt und 30 min gerührt. Die Phasen wurden getrennt

und die wässrige Phase mit $CH_2Cl_2$ (3 x 20 ml) extrahiert. Die organische Phase wurde über $Na_2SO_4$ getrocknet und i. Vak. eingeengt. Der Rückstand wurde durch Flash-Chromatographie mit $CHCl_3$/MeOH (1:1) gereinigt.
*Ausbeute:* 382 mg (17 %)
*$^1$H-NMR (DMSO-$d_6$):* 1.77 (2 H, m); 2.10 (6 H, m); 2.16 (3 H, s); 2.06 -2.44 (12 H, m); 2.77 (2 H, m); 3.27 (2 H, m); 6.18 (1 H, m); 6.96 (2 H, m); 7.22 (6 H, m); 7.46 (2 H, m); 10 61 (1 H, s).
*$^{13}$C-NMR (DMSO-$d_6$):* 22.01; 26.50; 27.03; 32.69; 38.18; 45.78; 52.52; 54.76; 59.11; 60.17; 108.51: 110.71; 117.83; 118.28; 120.86; 125.25; 126.36; 127.00; 127.46; 128.49; 129.83; 135.17; 135.63; 142.72.

**N,N-dimethyl-4-(3-(2-(4-methylpiperazin-1-yl)ethyl)-1H-indol-2-yl)-1-phenylcyclohexanamin, Citrat (1:1) : Eines von 2 möglichen Diastereomeren**

Das soeben erhaltene Olefin (375 mg, 0.847 mmol) wurde in HBr/Eisessig (18 ml) vorgelegt, innerhalb von 30 min mit Zinn (988 mg) versetzt und 3 h bei RT nachgerührt. Der Ansatz wurde mit Ethanol verdünnt, 20 min bei RT gerührt und i. Vak. eingeengt. Der Rückstand wurde mit 5N NaOH versetzt und mit Dichlormethan (3 x 20 ml) extrahiert. Die organische Phase wurde über $Na_2SO_4$ getrocknet und i. Vak. eingeengt. Der Rückstand wurde durch Flash-Chromatographie mit $CHCl_3$/MeOH (9:1) gereinigt.
*Ausbeute:* 160 mg (43 %)
Bei der Umsetzung mit einer molaren Menge an Citronensäure in Ethanol fiel das Ci-trat als weißer Feststoff aus.
*Ausbeute:* 151 mg (70 %); *Schmelzpunkt:* 210-213 °C
*$^1$H-NMR (DMSO-$d_6$):* 1.53 (4 H, m); 2.06 (6 H, m); 2.54-2.69 (10 H, m); 2.81 (10 H, m); 6.96 (2 H, s); 7.29 (2 H, m); 7.40 (5 H, m); 10.72 (1 H, s).

**Beispiel 201: (±)-2-(4-(Dimethylamino)-4-phenylcyclohex-1-enyl)-3-(2-morpholinoethyl)-1H-indol**

Indol (**Ind-69,** 1.05 g, 4.56 mmol) und Keton (**Ket-10,** 0.99 g, 4.56 mmol) wurden bei RT in abs. $CH_2Cl_2$ (50 ml) gelöst und zügig mit Trifluormethansulfonsäure (2.05 g, 2.2 ml, 13.7 mmol) versetzt, anschließend fiel ein schwarzes Öl aus. Die Mischung wurde 3 d bei RT gerührt. Nach Zugabe von 1 N NaOH (50 ml) wurde die Mischung 20 min gerührt, dann die organische Phase abgetrennt und die wässrige Phase mit $CH_2Cl_2$ (2 x 50 ml) ex-trahiert. Die vereinigten organische Phasen wurden mit Wasser (20 ml) gewaschen, über $Na_2SO_4$ getrocknet, filtriert und i. Vak. eingeengt. Der erhaltene Rückstand wurde durch Flash-Chromatographie mit 100 g Kieselgel und Chloroform/Methanol (9:1) gereinigt.
*Ausbeute:* 644 mg (33 %)
*$^1$H-NMR (DMSO-$d_6$):* 1.78 (2 H, m); 2.11 (6 H, s); 2.37 (8 H, m); 2.80 (4 H, d); 3.59 (4 H, d); 6.19 (1 H, s); 6.94 (2 H, m); 7.21-7.50 (7 H, m); 10.64 (1 H, s).

**N,N-dimethyl-4-(3-(2-morpholinoethyl)-1H-indol-2-yl)-1-phenylcyclohexanamin, Citrat (1:1) : Unpolares Diastereomer**

Das soeben erhaltene Olefin (617 mg, 1.4 mmol) wurde in 33%-iger HBr/Eisessig (30 ml) gelöst und anschließend bei RT innerhalb von 30 min portionsweise mit Zinn (1.87 g) versetzt. Das Gemisch wurde 4 h bei RT gerührt. Ethanol wurde zugegeben, das Lösungsmittelgemisch i. Vak. entfernt und der Rückstand mit 5N NaOH (60 ml) und $CH_2Cl_2$ (90 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet, filtriert und die Lösung i. Vak. eingeengt. Der verbliebene Rückstand wurde durch Flash-Chromatographie mit Chloroform/Methanol (9:1 → 4:1) gereinigt.

*Ausbeute:* 334 mg (54 %), unpolare Verbindung
119 mg (19 %), polare Verbindung

Bei der Umsetzung des soeben erhaltenen unpolareren Diastereomers mit einer molaren Menge an Citronensäure in Ethanol fiel das Ci-trat als farbloser Feststoff aus.
*Ausbeute:* 346 mg, unpolare Verbindung; *Schmelzpunkt:* 234-242 °C
*$^1$H-NMR (DMSO-$d_6$):* 1.63 (4 H, m); 2.11 (8 H; m); 2.55-2.91 (14 H, m); 3.68 (4 H, m); 6.96 (2 H, m); 7.37 (7 H, m); 10.71 (1 H, s).

**Beispiel 202: N,N-dimethyl-4-(3-(2-morpholinoethyl)-1H-indol-2-yl)-1-phenylcyclohexanamin, Citrat (1:1) : Polares Diastereomer**

Bei der Umsetzung des unter Beispiel **201** erhaltenen polareren Diastereomers mit einer molaren Menge an Citronensäure in Ethanol das Citrat als farbloser Feststoff aus. *Ausbeute:* 75 mg, polare Verbindung; *Schmelzpunkt:* 224-228 °C
*$^1$H-NMR (DMSO-$d_6$):* 1.51 (2 H, m); 1.83 (2 H, m); 2.00 (2 H, m); 2.36 (6 H; s); 2.59 (8 H, m); 2.78 (2 H, t); 3.02 (3 H, m); 3.65 (4 H, m); 6.90 (2 H, m); 7.16 (1 H, d); 7.35 (1 H, d); 7.56 (5 H, m); 10.44 (1 H, s).

**Beispiel 203: (±)-2-(4-(Dimethylamino)-4-phenylcyclohex-1-enyl)-3-(2-(1H-benzo[d]imidazol-1-yl)ethyl)-1H-indol**

Indol (**Ind-70,** 650 mg, 2.5 mmol) und Keton (**Ket-10,** 542 mg, 2.5 mmol) wurden in abs. Dichlormethan (20 ml) gelöst und schnell mit Trifluormethansulfonsäure (0.66 ml, 7.5 mmol) versetzt. Der Ansatz wurde über Nacht bei RT gerührt, dabei setzte sich ein dunkelbraunes Öl ab. Nach Zugabe von 1 N NaOH (10 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chroma-tographie mit $CHCl_3$/MeOH (9:1) gereinigt.
*Ausbeute:* 454 mg (39 %), poröser Feststoff
*$^1$H-NMR (DMSO-$d_6$):* 2.09 (10 H, m); 2.41 (2 H, m); 3.19 (2 H, m); 4.29 (2 H, m); 5.83 (1 H, s); 6.89-7.28 (7 H, m); 7.41 (6 H, m); 7.65 (1 H, s); 10.64 (1 H, bs).
*$^{13}$C-NMR (DMSO-$d_6$):* 30.41; 30.91; 42.48; 47.93; 63.48; 71.61; 108.19; 125.46; 126.90; 127.96; 144.23.

**4-(3-(2-(1H-benzo[d]imidazol-1-yl)ethyl)-1H-indol-2-yl)-N,N-dimethyl-1-phenylcyclohexanamin, Citrat (1:11): Unpolareres Diastereomer**

Zu einer Lösung von des soeben hergestellten Olefins (429 mg, 0.93 mmol) in HBr/Eisessig (20 ml) wurde innerhalb von 20 min Zinn (1.20 g) gegeben und 3 h bei RT gerührt. Der Ansatz wurde mit Ethanol versetzt, das Reaktionsgemisch bis zur Trockne eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (20:1 → 9:1) getrennt.

*Ausbeute:* 165 mg (38 %), unpolares Diastereomer
144 mg (33 %), polares Diastereomer

*Das soeben erhaltene unpolarere Diastereomer* (143 mg, 0.31 mmol) wurde in heißem Ethanol (5 ml) gelöst und mit einer Lösung von Citronensäure (59 mg, 0.31 mmol) in heißem Ethanol (3 ml) versetzt. Nach 2- stündigem Stehen im Kühlschrank wurde der entstandene Feststoff abgesaugt und i. Vak. getrocknet.
*Ausbeute:* 101 mg (50 *%); Schmelzpunkt:* 227-228 °C
*$^1$H-NMR (DMSO-$d_6$):* 1.19 (4 H, m); 1.89 (2 H, m); 2.09 (6 H, s); 2.33 (2 H, t); 2.67 (4 H, m); 3.21 (2 H, t); 4.45 (2 H, t); 7.00 (2 H, m); 7.14 (2 H, m); 7.32-7.61 (9 H, m); 7.84 (1 H, s); 10.55 (1 H, s).
*Citrat:* (Ethanol)

**Beispiel 204: 4-(3-(2-(1H-benzo[d]imidazol-1-yl)ethyl)-1H-indol-2-yl)-N,N-dimethyl-1-phenylcyclohexanamin, Citrat (1:1) : Polareres Diastereomer**

Das unter Beispiel 203 gewonnene polarere Diastereomer (135 mg, 0.27 mmol) wurde in heißem Ethanol (5 ml) gelöst und mit einer Lösung von Citronensäure (52 mg, 0.27 mmol) in heißem Ethanol (3 ml) versetzt. Nach 2-stündigem Stehen im Kühlschrank wurde der entstandene Feststoff abgesaugt und i. Vak. getrocknet.
*Ausbeute:* 68 mg (36 %), poröser Feststoff
*$^1$H-NMR (DMSO-$d_6$):* 1.61 (2 H, m); 2.33 (2 H, m); 2.41 (6 H, s); 2.63-2.83 (6 H, m); 3.18 (2 H, t); 4.43 (2 H, t); 6.93 (3 H, m); 7.20 (3 H, m); 7.42-7.67 (7 H, m); 7.83 (1 H, s); 10.33 (1 H, s).
*Citrat:* (Ethanol), poröser Feststoff

**Beispiel 205: (±)-2-(4-(Dimethylamino)-4-phenylcyclohex-1-enyl)-3-(2-(1H-imidazol-1-yl)ethyl)-1H-indol**

Indol **(Ind-71**, 490 mg, 2.32 mmol) und Keton **(Ket-10**, 503 mg, 2.32 mmol) wurden in abs. Dichlormethan (20 ml) gelöst und schnell mit Trifluormethansulfonsäure (0.62 ml, 7 mmol) versetzt. Der Ansatz wurde über Nacht bei RT gerührt, dabei setzte sich ein dunkelbraunes Öl ab. Nach Zugabe von 1N NaOH (10 ml) und $CH_2Cl_2$ (20 ml) wurde noch 90 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (4:1) gereinigt.
*Ausbeute:* 311 mg (33 %), zähes Öl
*$^1$H-NMR (DMSO-$d_6$):* 1.68 (2 H, m); 1.88 (1 H, m); 2.08 (6 H, s); 2.38 (1 H, d); 2.60 (2 H, d); 3.06 (2 H, m); 4.02 (2 H, m); 6.00 (1 H, s); 6.78-7.03 (4 H, m); 7.19-7.48 (8 H, m); 10 70 (1 H, s).
*$^{13}$C-NMR (DMSO-$d_6$):* 26.59; 26.77; 27.23; 32.42; 38.15; 46.61; 60.21; 106.31; 110.73: 117.92; 118.45; 119.15; 120.99; 126.00; 126.40; 127.07; 127.43; 128.11; 129.41; 135.09; 136.64; 136.88; 142.29.

**4-(3-(2-(1H-imidazol-1-yl)ethyl)-1H-indol-2-yl)-N,N-dimethyl-1-phenylcyclohexanamin, Citrat (1:1): Unpolareres Diastereomer**

Zu einer Lösung von des soeben hergestellten Olefins (300 mg, 0.708 mmol) in HBr/Eisessig (15 ml) wurde innerhalb von 20 min Zinn (0.90 g) gegeben und 3 h bei RT gerührt. Der Ansatz wurde mit Ethanol versetzt, das Reaktionsgemisch bis zur Trockne eingeengt und der Rückstand in 5N NaOH (20 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (20:1→1:1) getrennt.

*Ausbeute:* 111 mg (38 %), unpolares Diastereomer
33 mg (11 %), polares Diastereomer

Das soeben erhaltene unpolarere Diastereomer (87 mg, 0.21 mmol) wurde in heißem Ethanol (3 ml) gelöst und mit einer Lösung von Citronensäure (40 mg, 0.21 mmol) in heißem Ethanol (2 ml) versetzt. Nach 2- stündigem Stehen im Kühlschrank wurde der entstandene Feststoff abgesaugt und i. Vak. getrocknet.
*Ausbeute:* 91 mg (72 %); *Schmelzpunkt:* 203 °C
*$^1$H-NMR (DMSO-$d_6$):* 1.46 (4 H, m); 2.03 (6 H; s); 2.10 (1 H, m); 2.70 (3 H, m); 3.08 (2 H, t); 4.13 (2 H, t); 6.85 (1 H, s); 6.99 (2 H, m); 7.15 (1 H, s); 7.25 bis 7.49 (8 H, m); 10.74 (1 H, s), (freie Base).
*Citrat:* (Ethanol)

**Beispiel 206: 4-(3-(2-(1H-imidazol-1-yl)ethyl)-1H-indol-2-yl)-N,N-dimethyl-1-phenylcyclohexanamin, Citrat (1:1): Polareres Diastereomer**

Das unter Beispiel 205 gewonnene polarere Diastereomer (33 mg, 0.08 mmol) wurde in heißem Ethanol (2 ml) gelöst und mit einer Lösung von Citronensäure (15 mg, 0.08 mmol) in heißem Ethanol (2 ml) versetzt. Das Citrat fiel nicht aus, deshalb wurde das Lösungsmittel entfernt und der Rückstand i. Vak. getrocknet.
*Ausbeute:* 48 mg (100 %), poröser Feststoff
*$^1$H-NMR (DMSO-$d_6$):* 1.39 (2 H, m); 1.51 (2 H, m); 1.60 (2 H, m); 1.99 (6 H; s); 2.69 (3 H, m); 3.04 (2 H, t); 4.08 (2 H, t); 6.91 (3 H, m); 7.18 (2 H, m); 7.42 (7 H, m); 10.40 (1 H, s), (freie Base).
*Citrat:* (Ethanol) poröser Feststoff

**Beispiel 207: (±)-2-(4-(Dimethylamino)-4-phenylcyclohex-1-enyl)-3-(2-(1H-1,2,4-triazol-1-yl)ethyl)-1H-indol**

Indol (**Ind-72**, 800 mg, 3.77 mmol) und Keton (**Ket-10**, 819 mg, 3.77 mmol) wurden in abs. Dichlormethan (20 ml) gelöst und schnell mit Trifluormethansulfonsäure (1.0 ml, 11.3 mmol) versetzt. Der Ansatz wurde über Nacht bei RT gerührt, dabei setzte sich ein dunkelbraunes Öl ab. Nach Zugabe von 1 N NaOH (20 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9: 1) gereinigt.
*Ausbeute:* 465 mg (30 %), farbloser Feststoff
*$^1$H-NMR (DMSO-$d_6$):* 1.70 (2 H, m); 2.11 (8 H, m); 2.63 (2 H, m); 3.17 (2 H, t); 4.25 (2 H, t); 6.08 (1 H, s); 6.94 (2 H, m); 7.32 (5 H, m); 7.48 (2 H, m); 7.99 (1 H, s); 8.28 (1 H, s); 10.74 (1 H, s).

**4-(3-(2-(1H-1,2,4-triazol-1-yl)ethyl)-1H-indol-2-yl)-N,N-dimethyl-1-phenylcyclohexanamin, Citrat (1:1) : Unpolareres Diastereomer**

Zu einer Lösung von des soeben hergestellten Olefins (450 mg, 1.09 mmol) in HBr/Eisessig (25 ml) wurde innerhalb von 30 min Zinn (1.3 g) gegeben und 6 h bei RT gerührt. Der Ansatz wurde mit Ethanol versetzt, das Reaktionsgemisch bis zur Trockne eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (20:1 → 9:1) getrennt.

*Ausbeute:* 191 mg (42 %), unpolares Diastereomer
131 mg (29 %), polares Diastereomer

Das soeben erhaltene unpolarere Diastereomer (174 mg, 0.42 mmol) wurde in heißem Ethanol/Dioxan (1:1, 10 ml) gelöst und mit einer Lösung von Citronensäure (81 mg, 0.42 mmol) in heißem Ethanol (3 ml) versetzt. Nach 2-stündigem Stehen im Kühlschrank wurde der entstandene Feststoff abgesaugt und i. Vak. getrocknet.
*Ausbeute:* 107 mg (42 %); *Schmelzpunkt:* 234-236 °C
*$^1$H-NMR (DMSO-$d_6$):* 1.47 (4 H, m); 2.01 (2 H, m); 2.36 (6 H, s); 2.58-2.64 (6 H, m); 3.12 (2 H, t); 4.33 (2 H, t); 6.92 (2 H, m); 7.15 (1 H, m); 7.39-7.64 (6 H, m); 8.00 (1 H, s); 8.21 (1 H, s); 10.43 (1 H, s).
*Citrat:* (Ethanol/Dioxan)

**Beispiel 208: 4-(3-(2-(1H-1,2,4-triazol-1-yl)ethyl)-1H-indol-2-yl)-N,N-dimethyl-1-phenylcyclohexanamin, Citrat (1: 1) : Polareres Diastereomer**

Das unter Beispiel 207 gewonnene polarere Diastereomer (120 mg, 0.29 mmol) wurde in heißem Ethanol (5 ml) gelöst und mit einer Lösung von Citronensäure (56 mg, 0.29 mmol) in heißem Ethanol (3 ml) versetzt. Nach 2-stündigem Stehen im Kühlschrank wurde der entstandene Feststoff abgesaugt und i. Vak. getrocknet.
*Ausbeute:* 80 mg (45 %); *Schmelzpunkt:* 183-184 °C
*$^1$H-NMR (DMSO-$d_6$):* 1.52 (4 H, m); 2.06 (2 H, m); 2.12 (6 H, s); 2.51-2.83 (6 H, m); 3.17 (2 H, t); 4.34 (2 H, t); 6.99 (2 H, m); 7.41 (7 H, m); 7.94 (1 H, s); 8.25 (1 H, s); 10.68 (1 H, s).
Citrat: (Ethanol)

**Beispiel 209: (±)-2-(4-(Dimethylamino)-4-phenylcyclohex-1-enyl)-3-(2-(thiazolidin-3-yl)ethyl)-1H-indol**

Indol (Ind-73, 542 mg, 2.33 mmol) und Keton (Ket-10, 503 mg, 2.33 mmol) wurden unter Argon in abs. $CH_2Cl_2$ (50 ml) vorgelegt und mit Trifluormethansulfonsäure (612 µL, 6.99 mmol) versetzt. Der Ansatz wurde bei RT über Nacht gerührt. Zur Aufarbeitung wurde zu der Lösung 1 N NaOH addiert und 30 min gerührt. Die Phasen wurden getrennt, die wässrige Phase mit $CH_2Cl_2$ (3 x 20 ml) extrahiert, die organische Phase über $Na_2SO_4$ getrocknet und i. Vak. eingeengt. Der Rückstand wurde durch Flash-Chromatographie mit $CHCl_3$/MeOH (1:1) gereinigt.
*Ausbeute:* 417 mg (42 %)
*$^1$H-NMR (DMSO-$d_6$):* 1.77 (2 H, m); 2.08 (8 H, m); 2.10-2.68 (8 H, m); 2.92 (2 H, m); 3.17 (2 H, s); 6.18 (1 H, bs); 6.94 (2 H, m); 7.23 (2 H, m); 7.32 (2 H, m); 7.48 (3 H, m); 10.67 (1 H, s).

**N,N-dimethyl-1-phenyl-4-(3-(2-(thiazolidin-3-yl)ethyl)-1H-indol-2-yl)cyclohexanamin, Citrat (1:1) ( 209; : Unpolareres Diastereomer**

Das soeben erhaltene Olefin (417 mg, 0.96 mmol) wurde in HBr/Eisessig (30 ml) vorgelegt, innerhalb von 30 min mit Zinn (1.13 g) versetzt und 3 h bei RT gerührt. Zur Aufarbeitung wurde der Ansatz mit Ethanol verdünnt, 20 min bei RT gerührt und anschließend i. Vak. eingeengt. Der Rückstand wurde mit 5N NaOH versetzt, mit $CH_2Cl_2$ (3 x 20 ml) extrahiert, die organische Phase über $Na_2SO_4$ getrocknet und i. Vak. eingeengt. Der Rückstand wurde durch Flash-Chromatographie mit $CHCl_3$/MeOH (4:1 → 1:1) gereinigt.
*Ausbeute:* 86 mg (20 %) unpolares Diastereomer
*$^1$H-NMR (DMSO-$d_6$):* 1.51 (2 H, m); ); 1.67 (2 H, m); 2.03 (6 H; s); 2.16 (2 H, m); 2.85 (10 H, m); 6.95 (2 H, m); 7.32 (5 H, m); 7.60 (2 H, m); 10.74 (1 H, s).

*Ausbeute:* 119 mg (28 %) polares Diastereomer

*$^1H$-NMR (DMSO-$d_6$):* ): 1.50 (2 H, m); ); 1.75 (6 H, m); 1.94 (6 H; s); 2.54-2.72 (10 H, m); 6.89 (2 H, m); 7.17 (1 H, m); 7.34 (6 H, m); 10.38 (1 H, s).
Das soeben erhaltene unpolarere Diastereomer (86 mg, 0.198 mmol) wurde in heißem Ethanol (5 ml) gelöst. Citronensäure (37 mg, 0.198 mmol) wurde in heißem Ethanol (1 ml) gelöst und zugegeben. Der Ansatz wurde abgekühlt auf RT, dabei fiel ein Niederschlag aus. Der Niederschlag wurde i. Vak. getrocknet.
*Ausbeute:* 69 mg (55 %) unpolar; *Schmelzpunkt:* 185-188 °C

**Beispiel 210: N,N-dimethyl-1-phenyl-4-(3-(2-(thiazolidin-3-yl)ethyl)-1H-indol-2-yl)cyclohexanamin, Citrat (1:1): Polareres Diastereomer**

Das unter Beispiel 209 erhaltene polarere Diastereomer (119 mg, 0.274 mmol) wurde in heißem Ethanol (5 ml) gelöst. Citronensäure (51 mg, 0.274 mmol) wurde in heißem Ethanol (1 ml) gelöst und zugegeben. Der Ansatz wurde abgekühlt auf RT, dabei fiel ein Niederschlag aus. Der Niederschlag wurde i. Vak. getrocknet.
*Ausbeute:* 58 mg (34 %) polar; *Schmelzpunkt:* 138-141 °C

**Beispiel 211: (±)-2-(4-(Dimethylamino)-4-phenylcyclohex-1-enyl)-3-(2-(5-methyl-2H-tetrazol-2-yl)ethyl)-1H-indol**

Indol (Ind-74, 620 mg, 2.72 mmol) und Keton (Ket-10, 592 mg, 2.72 mmol) wurden in abs. Dichlormethan (15 ml) gelöst und schnell mit Trifluormethansulfonsäure (0.73 ml, 8.2 mmol) versetzt. Der Ansatz wurde über Nacht bei RT gerührt, dabei setzte sich ein dunkelbraunes Öl ab. Nach Zugabe von 1N NaOH (10 ml) und $CH_2Cl_2$ (20 ml) wurde noch 60 min nachgerührt, die Phasen getrennt, die wässrige Phase zweimal mit $CH_2Cl_2$ ex-trahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 443 mg (Ind-74, 38%)
*$^1H$-NMR (DMSO-$d_6$):* 1.74 (2 H, m); 2.13 (8 H, m); 2.42 (3 H, s); 2.61 (2 H, m); 3.31 (2 H, m); 4.63 (2 H, t); 6.07 (1 H, s); 6.90 (1 H, m); 7.01 (1 H, m); 7.24 (5 H, m); 7.50 (2 H, m); 10.76 (1 H, s).

**N,N-dimethyl-4-(3-(2-(5-methyl-2H-tetrazol-2-yl)ethyl)-1H-indol-2-yl)-1-phenylcyclohexanamin, Citrat (2:1): Unpolareres Diastereomer**

Zu einer Lösung des soeben hergestellten Olefins (418 mg, 0.98 mmol) in HBr/Eisessig (30 ml) wurde innerhalb von 20 min Zinn (1.20 g) gegeben und 3 h bei RT gerührt. Der Ansatz wurde mit Ethanol versetzt, das Reaktionsgemisch bis zur Trockene eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (20:1$\rightarrow$9:1) getrennt.

*Ausbeute:* 91 mg (22 %), unpolares Diastereomer
139 mg (33 %), polares Diastereomer

Das soeben erhaltene unpolarere Diatereomer (91 mg, 0.212 mmol) wurde in heißem Ethanol (2 ml) gelöst und mit einer Lösung von Citronensäure (41 mg, 0.212 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 72 mg (211, 54 %); *Schmelzpunkt:* 208-209 °C
*$^1H$-NMR (DMSO-$d_6$):* 1.46 (2 H, m); ); 1.65 (2 H, m); 2.17 (4 H, m); 2.28 (8 H; s); 2.41 (3 H, s); 2.93 (2 H, m); 4.77 (2 H, t); 6.98 (2 H, m); 7.27 (1 H, d); 7.43 (4 H, m); 7.55 (2 H, m); 10.92 (1 H, s), Citrat.

**Beispiel 212: N,N-dimethyl-4-(3-(2-(5-methyl-2H-tetrazol-2-yl)ethyl)-1H-indol-2-yl)-1-phenylcyclohexanamin, Citrat (1:1): Polareres Diastereomer**

Das die unter Beispiel **211** hergestellte polarere Diastereomer (133 mg, 0.31 mmol) wurde in heißem Ethanol (3 ml) gelöst und mit einer Lösung von Citronensäure (60 mg, 0.31 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 103 mg ( **212,** 53 %), poröser Feststoff
*$^1H$-NMR (DMSO-$d_6$): ):* 1.40 (4 H, m); ); 1.88 (2 H, m); 2.41 (9 H; m); 2.57 (4 H, m); 3.01 (2 H, m); 3.34 (2 H, m); 4.73

(2 H, t); 6.91 (2 H, m); 7.13 (1 H, m); 7.39 (1 H, m); 7.53 (5 H, m); 10.45 (1 H, s), Citrat.

**Beispiel 213: (±)-2-(4-(Dimethylamino)-4-phenylcylohex-1-enyl)-3-((1H-pyrazol-1-yl)ethyl)-1H-indol**

Indol (Ind-75, 615 mg, 2.9 mmol) und Keton (**Ket-10**, 632 mg, 2.9 mmol) wurden in abs. Dichlormethan (20 ml) gelöst und schnell mit Trifluormethansulfonsäure (0.77 ml, 8.7 mmol) versetzt. Der Ansatz wurde über Nacht bei RT gerührt, dabei setzte sich ein dunkelbraunes Öl ab. Nach Zugabe von 1N NaOH (20 ml) und $CH_2Cl_2$ (20 ml) wurde noch 60 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 233 mg (20 %), poröser Feststoff
*$^1$H-NMR (DMSO-$d_6$):* 1.74 (2 H, m); 2.11 (9 H, m); 3.13 (2 H, t); 3.85 (1 H, m); 4.18 (2 H, t); 6.01 (1 H, s); 6.19 (1 H, s); 6.90 (1 H, m); 7.01 (1 H, m); 7.22 bis 7.49 (9 H, m); 10.68 (1 H, s).

**4-(3-(2-(1H-pyrazol-1-yl)ethyl)-1H-indol-2-yl)-N,N-dimethyl-1-phenylcyclohexanamin, Citrat (1:1): Unpolareres Diastereomer**

Zu einer Lösung des soeben hergestellten Olefins (230 mg, 0.56 mmol) in HBr/Eisessig (10 ml) wurde innerhalb von 20 min Zinn (0.8 g) gegeben und 3 h bei RT gerührt. Der Ansatz wurde mit Ethanol versetzt, das Reaktionsgemisch bis zur Trockne eingeengt und der Rückstand in 5N NaOH (20 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (9:1) getrennt.

*Ausbeute:* 48 mg (21 %) unpolares Diastereomer
47 mg (20 %) polares Diastereomer

Das erhaltene unpolarere Diastereomer (48 mg, 0.116 mmol) wurde in heißem Ethanol (3 ml) gelöst und mit einer Lösung von Citronensäure (22 mg, 0.116 mmol) in heißem Ethanol (2 ml) versetzt. Nach 2- stündigem Stehen im Kühlschrank wurde der entstandene Feststoff abgesaugt und i. Vak. getrocknet.
*Ausbeute:* 34 mg (47 %); *Schmelzpunkt:* 130 °C
*$^1$H-NMR (DMSO-$d_6$):* 1.51 (4 H, m); 2.04 (2 H, m); 2.17 (6 H, s); 2.60-2.83 (7 H, m); 3.18 (2 H, t); 4.28 (2 H, t); 6.15 (1 H, d); 6.99 (2 H, m); 7.31-7.55 (9 H, m); 10.61 (1 H, s).
Citrat: (Ethanol)

**Beispiel 214: 4-(3-(2-(1H-pyrazol-1-yl)ethyl)-1H-indol-2-yl)-N,N-dimethyl-1-phenylcyclohexanamin, Citrat (1 :2) : Polareres Diastereomer**

Das unter Beispiel 213 gewonnene polarere Diastereomer (47 mg, 0.114 mmol) wurde in heißem Ethanol (3 ml) gelöst und mit einer Lösung von Citronensäure (22 mg, 0.114 mmol) in heißem Ethanol (2 ml) versetzt. Das Citrat fiel nicht aus, deshalb wurde das Lösungsmittel entfernt und der Rückstand i. Vak. getrocknet.
*Ausbeute:* 74 mg (100 %), zähes Öl
*$^1$H-NMR (DMSO-$d_6$):* 1.41 (2 H, m); 1.61 (2 H, m); 1.95 (2 H, m); 2.14 (2 H, m); 2.61 (6 H, s); 2.66-2.70 (5 H, m); 3.03 (2 H, t); 4.23 (2 H, t); 6.17 (1 H, d); 6.93 (2 H, m); 7.14 (1 H, m); 7.41-7.60 (6 H, m); 7.68 (2 H, m); 10.39 (1 H, s).
*Citrat:* (Ethanol), zähes Öl

**Beispiel 215: (±)-2-(4-(Dimethylamino)-4-phenylcyclohex-1-enyl)-3-(2-(1H-1,2,3-triazol-1-yl)ethyl)-1H-indol**

Indol (Ind-76, 500 mg, 2.35 mmol) und Keton (Ket-10, 511 mg, 2.35 mmol) wurden in abs. Dichlormethan (20 ml) gelöst und schnell mit Trifluormethansulfonsäure (0.62 ml, 7.0 mmol) versetzt. Der Ansatz wurde über Nacht bei RT gerührt, dabei setzte sich ein dunkelbraunes Öl ab. Nach Zugabe von 1 N NaOH (20 ml) und $CH_2Cl_2$ (20 ml) wurde noch 60 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 327 mg (34 %), farbloses Öl
*$^1$H-NMR (DMSO-$d_6$):* 1.70 (2 H, m); 2.14 (6 H, m); 2.37 (2 H, m); 2.65 (2 H, m); 3.23 (2 H, t); 4.45 (2 H, t); 6.04 (1 H, s); 6.95 (2 H, m); 7.21-7.51 (7 H, m); 7.67 (1 H, s); 7.94 (1 H, s); 10.74 (1 H, s).

**4-3-2-(1H-1,2,3-triazol-1-yl)ethyl)-1H-indol-2-yl)-N,N-dimethyl-1-phenylcyclohexanamin, Citrat (2:1): Unpolareres Diastereomer**

Zu einer Lösung des soeben hergestellten Olefins (300 mg, 0.73 mmol) in HBr/Eisessig (20 ml) wurde innerhalb von 20 min Zinn (0.90 g) gegeben und 5 h bei RT gerührt. Der Ansatz wurde mit Ethanol versetzt, das Reaktionsgemisch bis zur Trockne eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (20:1 → 9:1) getrennt.

*Ausbeute:* 96 mg (32 %), unpolares Diastereomer
77 mg (26 %), polares Diastereomer

Das soeben erhaltene unpolarere Diastereomer (84 mg, 0.20 mmol) wurde in heißem Ethanol (3 ml) gelöst und mit einer Lösung von Citronensäure (39 mg, 0.20 mmol) in heißem Ethanol (2 ml) versetzt. Nach 2- stündigem Stehen im Kühlschrank wurde der entstandene Feststoff abgesaugt und i. Vak. getrocknet.
*Ausbeute:* 86 mg (70 %); *Schmelzpunkt:* 230-232 °C
*$^1$H-NMR (DMSO-$d_6$):* 1.47 (4 H, m); 2.02 (2 H, m); 2.12 (6 H, s); 2.57-2.83 (6 H, m); 3.21 (2 H, t); 4.55 (2 H, t); 6.97 (2 H, m); 7.39 (7 H, m); 7.65 (1 H, s); 8.02 (1 H, s); 10.70 (1 H, s).
*Citrat:* (Ethanol)

**Beispiel 216: -(3-(2-(1H-1,2,3-triazol-1-yl)ethyl)-1H-indol-2-yl)-N,N-dimethyl-1-phenylcyclohexanamin, Citrat (1: 1) : Polareres Diastereomer**

Das unter Beispiel **215** gewonnene polarere Diastereomer (73 mg, 0.17 mmol) wurde in heißem Ethanol (3 ml) gelöst und mit einer Lösung von Citronensäure (34 mg, 0.17 mmol) in heißem Ethanol (2 ml) versetzt. Nach 2- stündigem Stehen im Kühlschrank wurde der entstandene Feststoff abgesaugt und i. Vak. getrocknet.
*Ausbeute:* 62 mg (58 %), poröser Feststoff
*$^1$H-NMR (DMSO-$d_6$):* 1.40 (2 H, m); 1.58 (2 H, m); 1.88 (2 H, m); 2.40 (6 H, m); 2.57 (8 H, m); 2.99 (1 H, m); 3.20 (2 H, t); 4.54 (2 H, t); 6.91 (1 H, m); 7.14 (1 H, m); 7.42-7.67 (8 H, m); 7.95 (1 H, s); 10.45 (1 H, s).
*Citrat:* (Ethanol), poröser Feststoff

**Beispiel 217: (3)-2-(4-(Dimethylamino)-4-phenylcyclohex-1-enyl)-3-(2-(5-methyl-1H-tetrazol-1-yl)ethyl)-1H-indol**

Indol (Ind-77, 900 mg, 3.96 mmol) und Keton (Ket-10, 860 mg, 3.96 mmol) wurden in abs. Dichlormethan (40 ml) gelöst und schnell mit Trifluormethansulfonsäure (1.06 ml, 12 mmol) versetzt. Der Ansatz wurde über Nacht bei RT gerührt, dabei setzte sich ein dunkelbraunes Öl ab. Nach Zugabe von 1N NaOH (20 ml) und $CH_2Cl_2$ (40 ml) wurde noch 60 min nachgerührt, die Phasen getrennt, die wässrige Phase zweimal mit $CH_2Cl_2$ ex-trahiert, die vereinigten organischen Phasen mit Wasser gewaschen, getrocknet ($Na_2SO_4$) und die Lösung i. Vak. eingeengt. Der verbliebene Rückstand wurde durch Flash-Chromatographie mit $CHCl_3$/MeOH (9:1) gereinigt.
*Ausbeute:* 700 mg (41%), poröser Feststoff
*$^1$H-NMR (DMSO-$d_6$):* 1.69 (2 H, m); 2.02-2.32 (11 H, m); 2.61 (2 H, m); 3.19 (2 H, m); 4.36 (2 H, m); 6.02 (1 H, s); 6.90 (1 H, m); 7.01 (1 H, m); 7.22 (5 H, m); 7.48 (2 H, m); 10.78 (1 H, s).

**N,N-dimethyl-4-(3-(2-(5-methyl-1H-tetrazol-1-yl)ethyl)-1H-indol-2-yl)-1-phenylcyclohexanamin, Citrat (2:1) : Unpolareres Diastereomer**

Zu einer Lösung von des soeben hergestellten Olefins (670 mg, 1.57 mmol) in HBr/Eisessig (40 ml) wurde innerhalb von 20 min Zinn (1.90 g) gegeben und 3 h bei RT gerührt. Der Ansatz wurde mit Ethanol versetzt, das Reaktionsgemisch bis zur Trockene eingeengt und der Rückstand in 5N NaOH (40 ml) und Dichlormethan (30 ml) gelöst. Die Phasen wurden getrennt, die wässrige Phase zweimal mit $CH_2Cl_2$ extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit $CHCl_3$/MeOH (9:1→1:1) getrennt.

*Ausbeute:* 107 mg (16 %), unpolares Diastereomer
145 mg (22 %), polares Diastereomer

Das soeben erhaltene unpolarere Diastereomer (103 mg, 0.24 mmol) wurde in heißem Ethanol (2 ml) gelöst und mit einer Lösung von Citronensäure (46 mg, 0.24 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 86 mg ( 217, 58 %); *Schmelzpunkt:* 198-199 °C
*$^1$H-NMR (DMSO-$d_6$):* 1.40 (2 H, m); ); 1.59 (2 H, m); 1.97 (3 H, s); 2.10 (2 H; m); 2.25 (6 H, s); 2.57-2.74 (4 H, m); 3.31 (2 H, t); 4.52 (2 H, t); 6.96 (2 H, m); 7.39 (7 H, m); 10.86 (1 H, s), Citrat.

**Beispiel 218: N,N-dimethyl-4-(3-(2-(5-methyl-1H-tetrazol-1-yl)ethyl)-1H-indol-2-yl)-1-phenylcyclohexanamin, Citrat (2:1) : Polareres Diastereomer**

Das unrter Beispiel **217** erhaltene polarere Diastereomer (130 mg, 0.30 mmol) wurde in heißem Ethanol (3 ml) gelöst und mit einer Lösung von Citronensäure (58 mg, 0.30 mmol) in heißem Ethanol (2 ml) versetzt. Nach 2-stündigem Stehen im Kühlschrank wurde der entstandene Feststoff abgesaugt und i. Vak. getrocknet.
*Ausbeute:* 106 mg ( 218, 56 %); *Schmelzpunkt:* 252-253 °C
*$^1$H-NMR (DMSO-$d_6$):* 1.41 (2 H, m); 1.50 (2 H, m); ); 1.96 (3 H, s); 2.05 (2 H; m); 2.37 (6 H, s); 2.61 (2 H, m); 3.02 (2 H, m); 3.17 (2 H, t); 4.50 (2 H, t); 6.93 (2 H, m); 7.16 (1 H, m); 7.29 (1 H, m); 7.51 (3 H, m); 7.68 (2 H, m); 10.48 (1 H, s), Citrat.

**Beispiel 219: 2-[2-(4-Dimethylamino-4-phenyl-cyclohexyl)-1H-indol-3-yl]-ethanol, 1 Diastereomer ( 219a)**

**Bsp. 220** (1.70 g, 4.2 mmol) wurde in Methanol (35 ml) suspendiert, mit einer Lösung von KOH (800 mg, 14.4 mmol) in Wasser (7 ml) versetzt und unter Rückfluss gekocht. Nach 16 h wurde weiteres KOH (2.66 g, 47 mmol) in fester Form zugegeben und 72 h bei RT gerührt. Der Niederschlag wurde abgesaugt. Der Niederschlag wurde mit Wasser gewaschen und löste sich dabei auf. Die wässrige Lösung wurde sechsmal mit $CHCl_3$ extrahiert, die organische Phase über $Na_2SO_4$ getrocknet, filtriert und eingeengt.
*Ausbeute **(219):*** 645 mg (42 %, 1 Diastereomer); *Schmelzpunkt:* 125-127 °C
*$^1$H-NMR (DMSO-$d_6$):* 1.47 (2 H, m); 1.70 (4 H, m); 1.93 (6 H, s); 2.78 (4 H, m); 2.92 (1 H, t); 3.51 (2 H, m); 4.57 (1 H; s); 6.91 (2 H, m); 7.15 (1 H, m); 7.38 (6 H, m); 10.31 (1 H, bs). *$^{13}$C-NMR (DMSO-$d_6$):* 28.04; 29.23; 33.11; 35.86; 37.97; 60.79; 61.91; 105.92; 110.64; 117.36; 117.85; 119.61; 126.16; 127.68; 127.85; 128.20; 135.20; 136.82; 140.14.

**Beispiel 220: 2-(2-(4-(Dimethylamino)-4-phenylcyclohexyl)-1H-indol-3-yl)ethyl acetat, 1 Diastereomer**

**2-(2-(4-(Dimethylamino)-4-phenylcyclohex-1-enyl)-1H-indol-3-yl)ethyl acetat 2-(1*H*-indol-**3-yl)-ethanol (**Ind-5**, 6.44 g, 40 mmol) und **Ket-10** (8.68 g, 40 mmol) wurden unter Argon und Eiskühlung in 33%-iger HBr/Eisessig (35 ml) gelöst und 3 d bei RT gerührt.
Unter Eiskühlung wurde die zum Neutralisieren von HBr nötige Menge $NaHCO_3$ (93.7 g, 1.12 mol) in fester Form portionsweise zugegeben und das Gemisch 3 h bei 40 °C gerührt, bis sich kein Gas mehr bildete. Die Essigsäure wurde i. Vak. entfernt, der Rückstand mit Essigester versetzt und mit gesättigter $NaHCO_3$-Lösung 1 h bei 40 °C gerührt, bis sich kein Gas mehr bildete. Die wässrige Phase wurde abgetrennt und dreimal mit Essigester extrahiert. Die vereinigten organischen Phasen wurden mit gesättigter $NaHCO_3$-Lösung und Wasser gewaschen und über $Na_2SO_4$ getrocknet. Die organische Phase wurde filtriert, i. Vak. eingeengt und durch Flash-Chromatographie mit 500 g Kieselgel und Chloroform/Methanol (20:1) gereinigt.
*Ausbeute* (2-(2-(4-(Dimethylamino)-4-phenylcyclohex-1-enyl)-1H-indol-3-yl)ethyl acetat): 15.7 g (98 %), gelber Feststoff; *Schmelzpunkt:* 146-148 °C
*$^1$H-NMR (DMSO-$d_6$):* 1.72 (2 H, m); 1.98 (3 H, s); 2.14 (7 H, m); 2.58 (1 H, m); 2.77 (1 H, m); 3.01 (2 H, m); 4.08 (2 H; t); 6.22 (1 H, bs); 6.94 (2 H, m); 7.31 (4 H, m); 7.45 (3 H, m); 10.7 (1 H, bs).
*$^{13}$C-NMR (DMSO-$d_6$):* 20.70; 24.04; 26.61; 27.00; 32.61; 37.93; 60.19; 63.93; 105.68; 110.77; 117.84; 118.45; 120.96; 125.81; 126.32; 127.42; 128.42; 129.48; 135.14; 136.51; 142.61; 170.20.

**2-(2-(4-(Dimethylamino)-4-phenylcyclohexyl)-1H-indol-3-yl)ethyl acetat, 1 Diastereomer (220)**

2-(2-(4-(Dimethylamino)-4-phenylcyclohex-1-enyl)-1H-indol-3-yl)ethyl acetat (6.80 g, 16.9 mmol) wurde in Essigsäure (300 ml) gelöst, mit Palladium/Aktivkohle 10%-ig (2.10 g) versetzt und 16 h bei 50 °C und 3 bar mit Wasserstoff hydriert, dann 16 h bei RT weiter hydriert, anschließend wurde der Katalysator über Celite abfiltriert.
Die Essigsäure wurde i. Vak. entfernt. Das erhaltene Diastereomerengemisch (6.24 g, 90 %) war leicht verunreinigt und wurde deshalb mit Ether gewaschen. Dabei wurde ein Diastereomer isoliert.
*Ausbeute* ( 220): 1.70 g (25 %); *Schmelzpunkt:* 164-172 °C
*$^1$H-NMR (DMSO-$d_6$):* 1.51 (2 H, m); 1.70 (3 H, m); 1.90-1.99 (10 H, m); 2.79 (2 H, m); 2.89 (3 H, m); 4.08 (2 H; t); 6.88 (2 H, m); 6.95 (1 H, m); 7.31 (6 H, m); 10.44 (1 H, bs).

**Beispiel 221: N,N-Dimethyl-4-(3-methyl-1H-pyrrolo[3,2-c]pyridin-2-yl)-1-phenylcyclohexanamin, Citrat (1:2), unpolares Diastereomer**

**Beispiel 222: N,N-Dimethyl-4-(3-methyl-1H-pyrrolo[3,2-c]pyridin-2-yl)-1-phenylcyclohexanamin, Citrat (1:2), polares Diastereomer**

**4-(Dimethylamino)-4-phenyl-1-(prop-1-ynyl)cyclohexanol**

Das Keton **Ket-10** (3000 mg 13,81 mmol) wurde in Tetrahydrofuran (absolut, 50 ml) bei -78 °C vorgelegt. Unter Argon wurde Prop-1-inyl Magnesiumbromid (31,8 ml, 15,88 mmol; 0,5 M in Tetrahydrofuran) hinzugetropft. Anschließend wurde die Reaktionsmischung bei - 78 °C 15 min gerührt. Danach wurde auf Raumtemperatur erwärmt und 1 h bei dieser Temperatur gerührt. Anschließend wurde Ammoniumchlorid-Lösung (50 ml; 1,0 M) hinzugegeben. Die Phasen wurden getrennt. Die wässrige Phase wurde mit Tetrahydrofuran (3 × 50 ml) extrahiert. Die vereinigten organischen Phasen wurden mit Natriumsulfat getrocknet und anschließend die flüchtigen Bestandteile vollständig im Vakuum entfernt. Es verblieb ein hellbraunes Öl, welches mit Diethylether (20 ml) versetzt wurde. Es fiel ein weißer Feststoff aus (990 mg, 4-Dimethylamino-4-phenyl-1-(prop-1-inyl)cyclohexanol, unpolares Diastereomer).Die Waschlösung wurde im Vakuum bis auf 5 ml eingeengt. Es fiel abermals ein weißer Feststoff aus (1350 mg; Diastereomerengemisch). Die Waschlösung wurde langsam auf 3 ml eingedampft. Es fiel erneut eine Produktfraktion aus (410 mg; beide Diastereoisomere).
Ausbeute (4-(Dimethylamino)-4-phenyl-1-(prop-1-ynyl)cyclohexanol): 2750 mg (10,68 mmol; 77 %, Diastereomerengemisch)

**4-Dimethylamino-1-(3-methyl-1H-pyrrolo[3,2-c]pyridin-2-yl)-4-phenylcyclohexanol**

4-Amino-3-iodpyridin (2331 mg, 10,60 mmol), 4-(Dimethylamino)-4-phenyl-1-(prop-1-ynyl)cyclohexanol (3000 mg, 11,66 mmol, Diastereomerengemisch), Lithiumchlorid (472 mg, 11,13 mmol) und Natriumcarbonat (3369 mg, 31,79 mmol) wurden in Dimethylformamid (absolut, 45 ml) in einer Argonatmosphäre vereinigt. Anschließend wurde der Katalysator ([Pd(dppf)$Cl_2$ × $CH_2Cl_2$], 865 mg, 1,06 mmol) hinzugegeben. Die rote Lösung wurde für 4 h auf 100 °C (Ölbadtemperatur) erwärmt. Zu der auf Raumtemperatur abgekühlten schwarzen Reaktionsmischung wurden nacheinander Wasser (50 ml; 10 min rühren), Dichlormethan (50 ml) und gesättigte Natriumchlorid-Lösung (100 ml zur besseren Phasentrennung) gegeben. Die Phasen wurden getrennt. Das Gemisch wurde vorher über Kieselgur filtriert. Die wässrige Phase wurde mit Dichlormethan (3 × 30 ml) extrahiert. Die vereinigten organischen Phasen wurden mit gesättigter Natriumchlorid-Lösung (3 × 20 ml) gewaschen, mit Natriumsulfat getrocknet, filtriert und anschließend die flüchtigen Bestandteile vollständig im Vakuum entfernt. Der Rückstand wurde auf Kieselgel aufgezogen und chromatographisch getrennt (Kieselgel [200 g]; Chloroform/Ethanol [9 : 1, 1000 ml, 5 : 1, 500 ml, 3 : 1, 500 ml, 1 : 1, 500ml und 1 : 3, 500ml]). Es wurden 800 mg 4-Dimethylamino-1-(3-methyl-1*H*-pyrrolo[3,2-c]pyridin-2-yl)-4-phenylcyclohexanol (unpolares Diastereoisomer) als farbloser Feststoff und 765 mg des polaren Diastereoisomers als farbloser Feststoff isoliert.

**(±)-*N,N*-Dimethyl-*N*-[4-(3-methyl-1*H*-pyrrolo[3,2-c]pyridin-2-yl)-1-phenylcyclohex-3-enyl]amin**

Zu einer Lösung 4-Dimethylamino-1-(3-methyl-1*H*-pyrrolo[3,2-c]pyridin-2-yl)-4-phenylcyclohexanol (600 mg, 1,72 mmol) in Methansulfonsäure (20 ml) wurde $P_4O_{10}$ (ca. 1 g, zwei Spatelspitzen) hinzugegeben. Die leicht hellbraun gefärbte Lösung wurde 3 h bei 77 °C (Ölbadtemperatur) gerührt. Der Reaktionsmischung wurde Wasser (10 ml) zugefügt und diese mit Natriumhydroxid-Lösung (5 M) basisch gestellt. Anschließend wurde Dichlormethan (30 ml) hinzugegeben und 10 min gerührt. Die Phasen wurden getrennt. Die wässrige Phase wurde mit Dichlormethan (3 × 35 ml) extrahiert. Die vereinigten organischen Phasen wurden über Natriumsulfat getrocknet. Anschließend wurden die flüchtigen Bestandteile vollständig im Vakuum entfernt. Es verblieben 425 mg (±)-*N,N*-Dimethyl-*N*-[4-(3-methyl-1H-pyrrolo[3,2-c] pyridin-2-yl)-1-phenylcyclohex-3-enyl]amin (dunkelbrauner Feststoff, leicht verunreinigt).

**N,N-Dimethyl-4-(3-methyl-1H-pyrrolo[3,2-c]pyridin-2-yl)-1-phenylcyclohexanamin (unpolares und polares Diastereomer)**

*N,N*-Dimethyl-N-[4-(3-methyl-1*H*-pyrrolo[3,2-c]pyridin-2-yl)-1-phenylcyclohex-3-enyl]amin (525 mg, 1,58 mmol) in HBr/Eisessig (33-proz.; 30 ml) wurde innerhalb von 150 min ZinnPulver (3,00 g, 45 mmol) gegeben (heftige Gasentwicklung). Die Reaktionsmischung färbte sich dunkelbraun. Es wurde über Nacht bei Raumtemperatur gerührt. Anschließend wurde die Reaktionsmischung im Vakuum zur Trockne eingedampft und der Rückstand mit 5N Natriumhydroxid-Lösung (20 ml) versetzt. Die helle Suspension wurde mit Dichlormethan (20 ml) versetzt und 10 min gerührt. Anschließend wurde unlösliches, graues Pulver abfiltriert. Danach wurden die Phasen des Filtrats getrennt. Die wässrige

Phase wurde mit Dichlormethan (2 × 20 ml) und Trichormetan/Ethanol (2 × 20 ml; 1 : 1) extrahiert. Die vereinigten organischen Phasen wurden mit Natriumsulfat getrocknet und anschließend die flüchtigen Bestandteile vollständig im Vakuum entfernt. Der Rückstand (250 mg hellgelbes Pulver) wurde chromatographisch getrennt [Kieselgel 60 (100 g), Ethylacetat/Methanol 1 : 1 (500 ml); Methanol (1000 ml)]. Es wurden 20 mg (0,06 mmol; 4 %) N,N-Dimethyl-4-(3-methyl-1H-pyrrolo[3,2-c]pyridin-2-yl)-1-phenylcyclohexanamin (unpolareres Diastereoisomer) sowie 78 mg (0,41 mmol; 15 %) des polareren Diastereoisomers isoliert.

**N,N-Dimethyl-4-(3-methyl-1H-pyrrolo[3,2-c]pyridin-2-yl)-1-phenylcyclohexanamin, Citrat (1:2), unpolares Diastereomer**

Dimethyl-4-(3-methyl-1H-pyrrolo[3,2-c]pyridin-2-yl)-1-phenylcyclohexanamin (unpolareres Diastereoisomer) (20 mg, 0,06 mmol) wurde in Ethanol (5 ml) suspendiert und mit Citronensäure (13 mg, 0,07 mmol) versetzt. Die Reaktionsmischung wurde 1 h in der Siedehitze stark gerührt, anschließend auf Raumtemperatur abgekühlt und im Vakuum auf ca. 3 ml eingeengt. Die Lösung wurde über Nacht bei Raumtemperatur stehen gelassen. Es fiel ein farbloser Feststoff aus. Die Fällung wurde durch Versetzen mit Diethylether (5 ml) vervollständigt. Die Mischung wurde für 3 h bei Raumtemperatur stehen gelassen. Die überstehende Lösung wurde abgehebert. Der Feststoff wurde mit Diethylether (10 ml) gewaschen und im Vakuum getrocknet. Beispiel **221** (Schmp. 103-105 °C) wurde als hellbrauner Feststoff (28 mg, 0,04 mmol; 89 %) erhalten. $^1$*H NMR (400 MHz, RT, Pyridin-D*$_5$*)* $\delta$ *ppm:* 1.70 (psq, 2H), 1.94 (psd, 2H), 2.60 (pst, 2H) 2.19 (s, 6H), 2.29 (s, 3H), 2.85 (psd, 2H), 3.17 (pst, 1 H), 3.67 (dd, 8H), 7.28 (t, 2H), 7.35 (psd, 2H), 7.39-7.46 (m, 1H), 7.48-7.54 (m, 1 H), 8.44 (psq, 1H), 9.15 (s, 1 H), 9.20-10.4 (s, br, 12 [incl. $H_2O$]), 12.38 (s, 1 H). $^{13}$*C NMR (101 MHz, Pyridin-D*$_5$*)* $\delta$ *ppm:* 8.3, 29.3, 33.0, 36.2, 38.0, 45.0, 63.3, 74.3, 106.3, 106.9, 126.6, 127.6, 128.0, 128.5, 128.7, 137.0, 138.9, 140.7, 142.9, 173.7, 178.1.

**N,N-Dimethyl-4-(3-methyl-1H-pyrrolo[3,2-c]pyridin-2-yl)-1-phenylcyclohexanamin, Citrat (1:2), polares Diastereomer**

N,N-Dimethyl-4-(3-methyl-1H-pyrrolo[3,2-c]pyridin-2-yl)-1-phenylcyclohexanamin (polareres Diastereoisomer) (78 mg, 0,23 mmol) wurde in Ethanol (5 ml) suspendiert und mit Citronensäure (49 mg, 0,26 mmol) versetzt. Es wurde 1 h in der Siedehitze stark gerührt. Die Reaktionsmischung wurde auf Raumtemperatur abgekühlt und über Nacht bei Raumtemperatur stehen gelassen. Es fiel ein farbloser Feststoff aus. Die Fällung wurde durch Versetzen mit Diethylether (5 ml) vervollständigt. Die Mischung wurde für 3 h bei Raumtemperatur stehen gelassen. Die überstehende Lösung wurde abgehebert. Der Feststoff mit Diethylether (10 ml) gewaschen und im Vakuum getrocknet. Beispiel 222 Schmp. 116-119 °C) wurde als ockerfarbener Feststoff (114 mg, 0,16 mmol; 93 %) erhalten. $^1$H NMR (400 MHz, RT, Pyridin-D$_5$) $\delta$ *ppm:* 1.69 (pst, 2H), 1.86 (psd, 2H), 2.32 (s, br. 9H), 2.64 (psd, 2 H), 2.96 (psd, 2H), 3.11 (pst, 1H), 3.67 (dd, 8H), 7.42-7.59 (m, 6H), 8.48 (s, 1H), 9.16 (s, 1H, liegt über folgendem Signal drüber), 8.80-10.0 (s, br, 10 [incl. $H_2O$]), 13.37 (s, 1 H).
$^{13}$C NMR (101 MHz, Pyridin-*D*$_5$) $\delta$ *ppm:* 8.3, 27.3, 33.1, 34.7, 37.8, 44.8, 61.0 (br), 74.3, 106.5, 107.4, 126.4, 127.6, 127.9, 128.3, 137.7, 141.2, 144.4, 173.7, 178.1, 2 Signale im aromatischen Bereich (n.b.).

**Beispiel 223: N,N-Dimethyl-4-(3-methyl-1H-pyrrolo[2,3-b]pyridin-2-yl)-1-phenylcyclohexanamin, Citrat (1:1), unpolares Diastereomer**

**Beispiel 224: N,N-dimethyl-4-(3-methyl-1H-pyrrolo[2,3-b]pyridin-2-yl)-1-phenylcyclohexanamin, Citrat (1:1), polares Diastereomer**

**N,N-dimethyl-4-(3-methyl-1H-pyrrolo[2,3-b]pyridin-2-yl)-1-phenylcyclohexanamin (unpolares und polares Diastereomer)**

Zu einer Suspension der freien Base aus Beispiel 32 (325 mg, 0,98 mmol) in HBr/Eisessig (35 %; 20 ml) wurde innerhalb von 90 min Zinn-Pulver (1500 mg) gegeben. Die Reaktionsmischung färbte sich dunkelbraun. Es wurde 3 h gerührt. Anschließend wurde die Reaktionsmischung im Vakuum zur Trockne eingedampft und der Rückstand mit 5 N Natriumhydroxid-Lösung (20 ml) versetzt. Die helle Suspension wurde mit Dichlormethan extrahiert (3 × 20 ml). Unlösliche Bestandteile in Form eines grauen Pulvers wurden abgesaugt. Die vereinigten organischen Phasen wurden mit Natriumsulfat getrocknet. Die flüchtigen Bestandteile wurden anschließend im Vakuum vollständig entfernt. Der Rückstand (hellgelbes Pulver) wurde chromatographisch getrennt (Kieselgel [100 g]; Chloroform/Ethanol [19 : 1, 500 ml]; [9 : 1, 500 ml]; [5 : 1; 1000 ml]). Es konnten 85 mg (0,26 mmol, 26 %) N,N-dimethyl-4-(3-methyl-1H-pyrrolo[2,3-b]pyridin-2-yl)-1-phenylcyclohexanamin (unpolares . Diastereomer) und 137 mg (0,41 mmol; 42 %) des polaren Diastereoisomers als farbloses Pulver isoliert werden.

**N,N-Dimethyl-4-(3-methyl-1H-pyrrolo[2,3-b]pyridin-2-yl)-1-phenylcyclohexanamin, Citrat (1:1), unpolares Diastereomer ( 223)**

N,N-dimethyl-4-(3-methyl-1H-pyrrolo[2,3-b]pyridin-2-yl)-1-phenylcyclohexanamin (unpolares Diastereomer) (70 mg, 0,18 mmol) wurde in Ethanol (10 ml) suspendiert und mit Citronensäure (44 mg, 0,23 mmol) versetzt. Es wurde 1 h in der Siedehitze stark gerührt. Die Reaktionsmischung wurde auf Raumtemperatur abgekühlt und über Nacht stehen gelassen. Die überstehende Lösung wurde von der farblosen Fällung abgehebert und verworfen. Beispiel 223 (25 mg; 23 %) wurde als farbloser Feststoff *(Schmelzpunkt:* 176-179 °C) isoliert.

**N,N-dimethyl-4-(3-methyl-1H-pyrrolo[2,3-b]pyridin-2-yl)-1-phenylcyclohexanamin, Citrat (1:1), polares Diastereomer ( 224)**

N,N-dimethyl-4-(3-methyl-1H-pyrrolo[2,3-b]pyridin-2-yl)-1-phenylcyclohexanamin (polares Diastereomer) (100 mg, 0,30 mmol) wurde in Ethanol (10 ml) suspendiert und mit Citronensäure (63 mg, 0,32 mmol) versetzt. Es wurde 1 h in der Siedehitze stark gerührt. Die Reaktionsmischung wurde auf Raumtemperatur abgekühlt und über Nacht gelagert. Es fiel ein farbloser Feststoff aus. Die überstehende Lösung wurde von der farblosen Fällung abgehebert und verworfen. Es wurden 114 mg (0,22 mmol; 72 %) Beispiel 224 ( *Schmelzpunkt:* 194-197 °C) isoliert.

**Beispiel 225: N,N-dimethyl-4-(3-methylbenzofuran-2-yl)-1-phenylcyclohexanamin, Citrat (1:1) : Polareres Diastereomer**

Das unter Beispiel **240** hergestellte Olefin (500 mg, 1,9 mmol) wurde in HBr/Eisessig (33 % HBr, 20 ml) gelöst und innerhalb von 30 min mit Sn-Pulver (1,4 g, 12 mmol) portionsweise bei RT versetzt. Nach Beendigung der Zugabe wurde das Reaktionsgemisch weitere 14 h gerührt. - Zur Aufarbeitung wurde das Gemisch am Rotationsverdampfer bis zur Trockene eingeengt. Der verbliebene Rückstand wurde durch Zugabe von 5N NaOH (40 ml) basisch gemacht. Die erhaltene Lösung wurde mit Dichlormethan versetzt und extrahiert (4 × 20 ml). Die vereinigten organischen Phasen wurden mit $MgSO_4$ getrocknet und anschließend eingeengt. Der erhaltene Rückstand (442 mg) wurde säulenchromatographisch (Laufmittel: 1. EtOAc/EtOH 10 : 1; 2. EtOAc/EtOH 2 : 1) gereinigt. Auf diese Weise wurde das unpolare Diastereoisomer (156 mg, 31 %, Schmp.: ab 128 °C, Hauptmenge 136-141 °C) und das polare Diastereoisomer (250 mg, 49 %, Schmp.: 136-138 °C) erhalten.

Das soeben isolierte polarere Diastereoisomer des Amins (157 mg, 0,47 mmol) wurde in der Siedehitze in Isopropanol (5 ml) gelöst und mit Citronensäure (96 mg, 0,5 mmol), gelöst in heißem Isopropanol (5 ml), versetzt. Das Lösungsmittelvolumen wurde auf etwa 5 ml reduziert. Die verbliebene Lösung wurde auf 5 °C abgekühlt (Kühlschrank) und 17 h stehen gelassen. Der entstandene Niederschlag wurde mittels einer Fritte abgetrennt. Das Citrat des polaren Amins, wurde so in einer Ausbeute von 135 mg (54 %, *Schmelzpunkt:* 169-171 °C) erhalten.

**Beispiel 226: N,N-dimethyl-4-(3-methylbenzofuran-2-yl)-1-phenylcyclohexanamin, Citrat (1:1) : Unpolareres Diastereomer**

Das unter Beispiel **225** hergestellte unpolarere Diastereoisomer des Amins (146 mg, 0,44 mmol) wurde in der Siedehitze in Isopropanol (10 ml) gelöst und mit Citronensäure (96 mg, 0,5 mmol), gelöst in heißem Isopropanol (1 ml), versetzt. Die Lösung wurde auf 5 °C abgekühlt (Kühlschrank) und 17 h stehen gelassen. Der entstandene Niederschlag wurde mittels einer Fritte abgetrennt. Das Hemicitrat des unpolaren Amins, Verbindung, wurde so in einer Ausbeute von 167 mg (88 %, *Schmelzpunkt:* 184-185 °C) erhalten.

**Beispiel 227: 4-(1H-indol-3-yl)-N,N-dimethyl-1-phenylcyclohexanamin, Citrat (1:1) : Eines von 2 möglichen Diastereomeren**

1H-indol (351 mg, 3 mmol) wurde zusammen mit dem Keton (**Ket-10,** 651 mg, 3 mmol) in Dichlormethan (20 ml) gelöst und mit Trifluormethansulfonsäure (0,3 ml, 3,4 mmol) versetzt. Nach ca. 30 min fiel ein Niederschlag aus. Der Ansatz wurde 17 h bei RT gerührt. Anschließend wurde der Ansatz mit Triethylsilan (1 ml, 6,2 mmol) versetzt und weitere 2 d bei RT gerührt. Da während der ganzen Zeit ein Niederschlag existierte, wurde der Ansatz mit Ethanol (10 ml) versetzt, bis eine klare Lösung entstand. Das erhaltene Reaktionsgemisch wurde mit Triethylsilan (1 ml, 6,2 mmol) versetzt und 20 h gerührt (wahrscheinlich ist diese Behandlung überflüssig, da bei einem Versuch, bei dem bereits vor der ersten Zugabe des Silans eine homogene Lösung mittels Ethanol erzeugt wurde, daß gewünschte Produkt nicht isoliert werden konnte). - Zur Aufarbeitung wurde das Reaktionsgemisch mit 2N NaOH (10 ml) versetzt. Das Gemisch wurde weitere 10 min gerührt und zur Erleichterung einer Phasentrennung mit gesättigter $NH_4Cl$-Lösung (30 ml) versetzt. Nach Abtrennung der organischen Phase wurde die wäßrige Phase mit Dichlormethan (3 × 20 ml) extrahiert. Die

vereinigten organischen Extrakte wurden über $MgSO_4$ getrocknet und anschließend eingeengt. Das erhaltene Rohprodukt (1,12 g) wurde mit Ethylacetat (ca. 20 ml) versetzt, die unlöslichen Anteile wurden mittels einer Fritte abgetrennt. Die erhaltene Lösung wurde eingeengt, der Rückstand wurde säulenchromatographisch (Laufmittel: EtOAc) gereinigt. Eins der beiden möglichen Isomeren wurde so in einer Ausbeute von 140 mg (11 %, Schmelzpunkt 210-214 °C, nach Umkristallisation aus 2-Propanol) erhalten.
Das soeben erhaltene Amin (120 mg, 0,38 mmol) wurde in der Siedehitze in Isopropanol (10 ml) gelöst und mit Citronensäure (80 mg, 0,4 mmol), gelöst in heißem Isopropanol (4 ml), versetzt. Unmittelbar nach der Zugabe der Säure fiel ein Niederschlag aus. Zur Vervollständigung der Fällung wurde der Ansatz auf 5 °C abgekühlt (Kühlschrank) und 12 h stehen gelassen. Der Niederschlag wurde mittels einer Fritte abgetrennt und anschließend getrocknet. Das gewünschte Produkt wurde so in einer Ausbeute von 111 mg (71 %, *Schmelzpunkt:* ab 197-201 °C) als Hemicitrat erhalten.

**Beispiel 228: (±)-3-(4-(Dimethylamino)-4-benrylcyclohex-1-enyl)-1H-pyrrolo[2,3-b]pyridin**

7-Azaindol (**Ind-86**) (637 mg, 5,39 mmol) und Keton **Ket-3** (1,247 g, 5,39 mmol) wurden in 2N KOH/MeOH (50 ml) gelöst und 16 h am Rückfluß gekocht. Zur Aufarbeitung wurde MeOH abdestilliert und das Reaktionsgemisch mit $H_2O$ (60 ml) versetzt. Die wässrige Phase wurde mit Dichlormethan (3 × 30 ml) extrahiert. Die vereinigten organischen Extrakte wurden über $Na_2SO_4$ getrocknet und anschließend eingeengt. Der Rückstand wurde aus MeOH (20 ml) umkristallisiert. Beispiel **228** wurde in einer Ausbeute von 929 mg als weißer Feststoff erhalten (52 %, *Schmelzpunkt:* 222-225 °C).
$^1$*H NMR (300 MHz, DMSO-d$_6$)* δ ppm: 1.25-1.52 (m, 1H), 1.84-1.99 (m, 1H), 1.99-2.13 (m, 1H), 2.30 (s, 6H), 2.32-2.39 (m, 2H), 2.39-2.46 (m, 1H), ), 2.75 (dd, J = 13.42, 6.72 Hz, 2H), 5.97-6.12 (m, 1H), 7.05 (dd, J = 7.92, 4.71 Hz, 1H), 7.10-7.22 (m, 1H), 7.22-7.31 (m, 4H), 7.42 (s, 1H), 8.12-8.22 (m, 2H), 11.54 (s, 1H)

**Beispiel 229: (±)-3-(4-(Dimethylamino)-4-butylcyclohex-1-enyl)-1H-pyrrolo[2,3-b]pyridin**

7-Azaindol (**Ind-86**) (591 mg, 5 mmol) und **Ket-4** (987 mg, 5 mmol) wurden in 2N KOH/MeOH (50 ml) gelöst und 16 h am Rückfluß gekocht. Zur Aufarbeitung wurde MeOH abdestilliert und das Reaktionsgemisch mit $H_2O$ (100 ml) versetzt. Die wässrige Phase wurde mit Dichlormethan (4 × 30 ml) extrahiert. Die vereinigten organischen Extrakte wurden über $Na_2SO_4$ getrocknet und anschließend eingeengt. Der Rückstand wurde aus MeOH (20 ml) umkristallisiert. Beispiel **229** wurde in einer Ausbeute von 553 mg als weißer Feststoff erhalten (37 %, *Schmelzpunkt:* 142-145 °C).
$^1$*H NMR (300 MHz, DMSO-d$_6$) δ ppm:* 0.87 (t, J = 6.91 Hz, 3H), 1.10-1.55 (m, 6H), 1.55-1.68 (m, 1H), 1.68-1. 48 (m, 1H), 1.85-2.02 (m, 1H), 2.03-2.60 (m, 9H), 6.02-6.23 (m, 1H), 7.06 (dd, J = 7.91, 4.70 Hz, 1H), 7.45 (s, 1H), 8.13-8.23 (m, 2H), 11.54 (s, 1H)
$^{13}$*C NMR (101 MHz, DMSO-d$_6$) δ ppm:* 14.1, 23.0, 25.1, 25.8, 28.2, 29.8, 31.8, 37.6, 55.2, 115.3, 115.5, 116.9, 119.2, 122.4, 128.3, 130.1, 142.4, 149.0

**Beispiel 230: (±)-3-(4-(Dimethylamino)-4-phenylcyclohex-1-enyl)-1H-pyrrolo[2,3-b]pyridin, Citrat (1:2)**

**(±)-3-(4-(Dimethylamino)-4-phenylcyclohex-1-enyl)-1H-pyrrolo[2,3-b]pyridin**

7-Azaindol (Ind-86) (294 mg, 2,49 mmol) und Ket-10 (540 mg, 2,58 mmol) wurden in 2N KOH/MeOH (20 ml) gelöst und 10 h am Rückfluß gekocht. Zur Aufarbeitung wurde MeOH abdestilliert und das Reaktionsgemisch mit $H_2O$ (40 ml) versetzt. Die wässrige Phase wurde mit Dichlormethan (3 x 20 ml) extrahiert. Die vereinigten organischen Extrakte wurden über $Na_2SO_4$ getrocknet und anschließend eingeengt. (±)-3-(4-(Dimethylamino)-4-phenylcyclohex-1-enyl)-1H-pyrrolo[2,3-b]pyridin wurde in einer Ausbeute von 740 mg (94 %) als beiger Feststoff erhalten.

**(±)-*N,N*-Dimethyl-*N*-[1-phenyl-4-(1*H*-pyrrolo[2,3-b]pyridin-3-yl)cyclohex-3-enyl]ammonium-citrat**

(±)-3-(4-(Dimethylamino)-4-phenylcyclohex-1-enyl)-1H-pyrrolo[2,3-b]pyridin (640 mg, 2 mmol) wurde in der Siedehitze in Isopropanol (20 ml) gelöst und mit Citronensäure (385 mg, 2 mmol), gelöst in heißem Isopropanol (5 ml), versetzt. Der Ansatz wurde 16 h bei 5 °C stehen gelassen. Der entstandene weiße Niederschlag wurde mittels einer Fritte abgetrennt. Beispiel 230 wurde in einer Ausbeute von 670 mg (65 %, *Schmelzpunkt:* 107-110 °C) erhalten.
$^1$*H NMR (300 MHz, DMSO-d$_6$) δ ppm:* 1.78-1.90 (m, 1H), 2.16-2.32 (m, 1H), 2.42 (s, 6H) 2.53-2.70 (m, 6H), 2.72-2.90 (m, 1H) 3.05-3.19 (m, 1H), 6.25 (m, 1H), 7.08 (dd, *J* = 7.90, 4.62 Hz, 1H), 7.28-7.50 (m, 4H), 7.63 (d, *J* = 7.49 Hz, 2H), 8.12-8.23 (m, 2H), 11.58 (s, 1H) $^{13}$*C NMR (101 MHz, DMSO-d6) δ ppm:* 25.9, 27.9, 30.1, 37.8, 38.9, 43.6, 62.0, 71.7, 114.3, 115.6, 116.7, 117.1, 123.0, 127.9, 128.2, 130.6, 136.5, 142.6, 148.9, 171.2, 176.0

**Beispiel 231: (±)-3-(4-(Dimethylamino)-4-phenylcyclohex-1-enyl)-1H-indol**

1H-indol (468 mg, 4 mmol) wurde zusammen mit dem Keton (**Ket-10**, 868 mg, 4 mmol) in Dichlormethan (40 ml) gelöst und mit Trifluormethansulfonsäure (0,4 ml, 4,6 mmol) versetzt. Der Ansatz wurde 20 h bei RT gerührt. - Zur Aufarbeitung wurde das Reaktionsgemisch mit 2N NaOH (20 ml) versetzt. Das Gemisch wurde weitere 20 min gerührt. Nach Trennung der Phasen wurde die wäßrige Phase mit Dichlormethan (2 x 20 ml) extrahiert. Die vereinigten organischen Extrakte wurden über $Na_2SO_4$ getrocknet und anschließend eingeengt. Das erhaltene Rohprodukt (1,2 g) wurde mit Ethylacetat (ca. 10 ml) versetzt, die unlöslichen Anteile wurden mittels einer Fritte abgetrennt. Die erhaltene klare Lösung wurde säulenchromatographisch (Laufmittel: EtOAc) gereinigt. Es wurde ein Gemisch (230 mg) erhalten, welches neben dem gewünschten Produkt geringe Mengen eines Isomeren (wahrscheinlich Substitution in 2-Stellung des Indols) enthielt. Durch Anreiben mit Ethanol (ca. 5 ml) wurde das gewünschte Produkt in einer Ausbeute von 173 mg (13 %, Schmp.: 164-172 °C) in kristalliner Form erhalten.
Das erhaltene Olefin (158 mg, 0,5 mmol) wurde in der Siedehitze in Isopropanol (20 ml) gelöst und mit Citronensäure (100 mg, 0,52 mmol), gelöst in heißem Isopropanol (4 ml), versetzt. Unmittelbar nach der Zugabe der Säure fiel ein Niederschlag aus. Zur Vervollständigung der Fällung wurde der Ansatz auf 5 °C abgekühlt (Kühlschrank) und 12 h stehen gelassen. Der Niederschlag wurde mittels einer Fritte abgetrennt und anschließend getrocknet. Das gewünschte Produkt wurde so in einer Ausbeute von 109 mg (52 %, *Schmelzpunkt:* ab 98-102 °C) als Hemicitrat erhalten.

**Beispiel 232: (±)-3-(4-(Dimethylamino)-4-phenylcyclohex-1-enyl)-1H-indol**

KOH (56 mg, 1 mmol) wird in MeOH (10 ml) gelöst. 1H-Indol (234 mg, 2 mmol) und Keton (Ket-10, 434 mg, 2 mmol) werden zugesetzt und das Gemisch 12 h am Rückfluß gekocht. Der Verlauf der Reaktion wird durch DC verfolgt. Der entstandene weiße Feststoff wird abgesaugt und mit $H_2O$ (3 × 5 ml) nachgespült. Nach Trocknen des Feststoffes erhält man das gewünschte Olefin in einer Ausbeute von 250 mg (39 %). Zur Herstellung des Hydrochlorids wird das Olefin (250 mg, 0,785 mmol) in Ethylmethylketon gelöst und mit 1,85N 1,85N EtOH/HCl (0,65 ml) versetzt. Der dabei entstandene weiße Feststoff wird getrocknet. Man erhält so das gewünschte Produkt 265 mg (37 %) mit einem Schmelzpunkt von 193-195 °C.

**Beispiel 233: 2-(3-(4-(dimethylamino)-4-phenylcyclohex-1-enyl)benzo[b]thiophen-2-yl)ethanol Hydrochlorid**

Unter Argon wurde das Keton (Ket-10, 217 mg, 1 mmol) zusammen mit 2-Benzo[b]thiophen-2-ylethanol (Ind-89, 178 mg, 1 mmol) in absolutem Dichlormethan (5 ml) gelöst. Anschließend erfolgte die Zugabe von Methansulfonsäure (3 ml). Der Ansatz wurde 24 h bei RT gerührt. Zur Aufarbeitung wurde das Reaktionsgemisch mit Eis (30 g) versetzt. Dabei fiel ein farbloser Feststoff aus, der in 1 N Natronlauge (10 ml) und Trichlormethan (30 ml) suspendiert und 30 min gerührt wurde. Die organische Phase wurde abgetrennt und die wäßrige Phase mit Trichlormethan (30 ml) extrahiert. Die organischen Extrakte wurden nach dem Trocknen eingeengt und der Rückstand (295 mg) chromatographisch an Kieselgel (40 g) mit Ethylacetat/Methanol (4 : 1) getrennt. Das gewünschte Olefin wurde in einer Ausbeute von 14 % (45 mg) als farbloser Feststoff mit einem Schmelzpunkt von 151-153 °C erhalten. Das isolierte Olefin (47 mg, 0,124 mmol) wurde in Ethanol (5 ml) gelöst und mit 5N isopropanolischer Salzsäure (0,04 ml, 0,2 mmol) versetzt. Nach 1 h wurde die klare Lösung auf 3 ml eingeengt, mit Diethylether (40 ml) versetzt und 1 h gerührt. Das gewünschte Hydrochlorid wurde als farbloser Feststoff in einer Ausbeute von 75 % (38 mg) mit einem Schmelzpunkt von 219-221 °C gewonnen.

**Beispiel 234: 2-(3-(4-(dimethylamino)-4-(pyridin-2-yl)cyclohex-1-enyl)benzo[b]thiophen-2-yl)ethanol Hydrochlorid**

Unter Argon wurde das Keton (Ket-11, 218 mg, 1 mmol) zusammen mit 2-Benzo[b]thiophen-2-ylethanol (Ind-89, 178 mg, 1 mmol) in absolutem Dichlormethan (5 ml) gelöst. Anschließend erfolgte die Zugabe von Methansulfonsäure (3 ml). Der Ansatz wurde 3 d bei RT gerührt. Zur Aufarbeitung wurde das Reaktionsgemisch mit Eis (5 g) und Wasser (30 ml) versetzt. Nach der Neutralisation mit $NaHCO_3$ (4,4 g, 52 mmol) und der Zugabe von 5N NaOH (1 ml) wurde Dichlormethan (10 ml) hinzugefügt. Die organische Phase wurde abgetrennt und die wäßrige Phase mit Dichlormethan (2 × 30 ml) extrahiert. Die organischen Extrakte wurden nach dem Trocknen eingeengt und der Rückstand (375 mg) chromatographisch an Kieselgel (45 g) mit Ethylacetat/Methanol (10 : 1), (4 : 1) und Methanol getrennt. Das gewünschte Olefin wurde in einer Ausbeute von 26 % (100 mg) das als farblose ölige Verbindung erhalten.
Das soeben isolierte Olefin (100 mg, 0,264 mmol) wurde in Ethanol (5 ml) gelöst und mit 5N isopropanolischer Salzsäure (0,104 ml, 0,52 mmol) versetzt. Nach 2,5 h wurde die klare Lösung auf 3 ml eingeengt, mit Diethylether (30 ml) versetzt und 1,5 h gerührt. Das Hydrochlorid wurde als farbloser Feststoff in einer Ausbeute von 67 % (73 mg) gewonnen. Ein Schmelzpunkt konnte nicht bestimmt werden.

**Beispiel 235: (±)-2-(4-(Dimethylamino)-4-phenylcyclohex-1-enyl)-1H-indol**

1-Phenylsulfonyl-1*H*-indol (Ind-90, 1,2 g, 4,66 mmol) wurde in trockenem THF gelöst und bei -5 °C (Eis/Kochsalz-Mischung) innerhalb von 30 min mit n-Butyllithium (2,2 ml, 2,5N Lösung in THF, 5.5 mmol) versetzt. Das Reaktionsgemisch wurde 2 h bei 0 °C gerührt, bevor das Keton (Ket-10, 986 mg, 4,66 mmol), gelöst in trockenem THF (10 ml), unter Beibehaltung der Temperatur innerhalb von 15 min zugegeben wurde. Anschließend wurde das Reaktionsgemisch eine weitere Stunde bei 0 °C und nach Entfernen der Kühlung 5 d bei RT gerührt. - Zur Aufarbeitung wurde der Ansatz mit gesättigter $NH_4Cl$-Lösung (30 ml) versetzt und 2 h gerührt. Das Reaktionsgemisch wurde dann mit Wasser (20 ml) und Ethylacetat (50 ml) versetzt. Die organische Phase wurde abgetrennt, die wäßrige Phase wurde mit Ethylacetat (3 × 20 ml) extrahiert. Die vereinigten organischen Extrakte wurden über $MgSO_4$ getrocknet und anschließend eingeengt. Da das gewünschte Produkt aus dem erhaltenen Rohprodukt (1,8 g) durch Kristallisation aus 2-Propanol (10 ml) nicht in ausreichender Reinheit isoliert werden konnte, erfolgte eine säulenchromatographische Reinigung [Kieselgel 60 (100 g); EtOAc (1000 ml)]. Das gewünschte Olefin wurde so in einer Ausbeute von 490 mg (33 %. Schmelzpunkt 141-146 °C) erhalten.

4-(1H-indol-2-yl)-N,N-dimethyl-1-phenylcyclohexanamin, Citrat (1:1) : Polareres Diastereomer Das soeben isolierte Olefin (450 mg, 1,42 mmol) wurde in HBr/Eisessig (33 % HBr, 20 ml) gelöst. Anschließend wurde zu dem Ansatz bei RT Sn-Pulver (2 g, 16,95 mmol) innerhalb von 2 h portionsweise gegeben. Nach Beendigung der Zugabe wurde das Reaktionsgemisch weitere 60 min bei RT gerührt. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (60 ml) basisch gemacht. Das erhaltene wäßrige Gemisch wurde mit Dichlormethan extrahiert (4 × 20 ml). Die vereinigten organischen Phasen wurden mit Wasser (50 ml) gewaschen, über $MgSO_4$ getrocknet und anschließend eingeengt. Nachdem es sich herausgestellt hatte, das eine Reinigung durch Umkristallisation nur bedingt möglich war, wurde das erhaltene Rohprodukt (400 mg) durch Säulenchromatographie [Kieselgel 60 G (10 g); Etylacetat, Ethylacetat/ Ethanol 2 : 1, Methanol (insgesamt 250 ml)] gereinigt. Das unpolarere Produkt wurde in einer Ausbeute von 106 mg (23 %, *Schmelzpunkt:* 159-164 °C), das polarere Produkt in einer Ausbeute von 135 mg [30 %. *Schmelzpunkt:* 205-211 °C (aus 2-Propanol)] erhalten.

Das soeben isolierte polarere Diastereomer (68 mg, 0,21 mmol) wurde in der Siedehitze in Isopropanol (5 ml) gelöst und mit Citronensäure (59 mg, 0,3 mmol), gelöst in heißem Isopropanol (1 ml), versetzt. Unmittelbar nach der Zugabe der Säure fiel ein Niederschlag aus. Zur Vervollständigung der Fällung wurde der Ansatz auf 5 °C abgekühlt (Kühlschrank) und 17 h stehen gelassen. Der Niederschlag wurde mittels einer Fritte abgetrennt und anschließend getrocknet. Das gewünschte Produkt wurde so in einer Ausbeute von 83 mg (76 %, *Schmelzpunkt:* ab 194-197 °C) als Citrat erhalten.

**Beispiel 236: 4-(1H-indol-2-yl)-N,N-dimethyl-1-phenylcyclohexanamin, Citrat (2:1) : Unpolareres Diastereomer**

Das unter Beispiel 235 isolierte unpolarere Diastereomer (100 mg, 0,31 mmol) wurde in der Siedehitze in Isopropanol (5 ml) gelöst und mit Citronensäure (60 mg, 0,31 mmol), gelöst in heißem Isopropanol (2 ml), versetzt. Unmittelbar nach der Zugabe der Säure fiel ein Niederschlag aus. Zur Vervollständigung der Fällung wurde der Ansatz auf 5 °C abgekühlt (Kühlschrank) und 17 h stehen gelassen. Der Niederschlag wurde mittels einer Fritte abgetrennt und anschließend getrocknet. Das gewünschte Produkt wurde so in einer Ausbeute von 103 mg (79 %, *Schmelzpunkt:* ab 231-233 °C) als Hemicitrat erhalten.

**Beispiel 237: 1-Benzyl-N,N-dimethyl-4-(1H-pyrrolo[2,3-b]pyridin-3-yl)cyclohexanamin Hydrochlorid, Diastereomerengemisch**

Bsp. 228 (540 mg, 1,63 mmol) wurde in abs. Methanol (100 ml) mit Palladiumkatalysator (Pd/C, 5 %, 216 mg) versetzt und 36 h bei RT hydriert (Wasserstoffdruck: 3 bar). Der Katalysator wurde mit Hilfe einer Fritte, versehen mit einer 1 cm hohen Schicht Celite, entfernt. Die Fritte wurde mit Methanol (1000 ml) gründlich gewaschen. Das Lösungsmittel wurde im Vakuum abdestilliert. 1-Benzyl-N,N-dimethyl-4-(1H-pyrrolo[2,3-b]pyridin-3-yl)cyclohexanamin wurde in einer Ausbeute von 350 mg (65 %) als weißer Feststoff erhalten. Es handelte sich hierbei um ein Gemisch aus den beiden Diastereoisomeren.

Das Diastereoisomerengemisch (350 mg, 1,05 mmol) wurde in Ethylacetat (100 ml) gelöst. Bei RT wurde $Me_3SiCl$ (265 μl, 2,1 mmol) zugetropft und 1 h gerührt. Es fiel ein weißer Niederschlag aus. Der Niederschlag wurde abgesaugt, mit Ethylacetat (2 × 5 ml) gewaschen und anschließend getrocknet.

Bsp. 237 (350 mg, Fp. 215-219 °C, 90 %) wurde als weißer Feststoff erhalten.

*$^1H$ NMR (400 MHz, DMSO-$d_6$) δppm:* 1.39-1.94 (m, 4H), 2.01-2.316 (m,4H), 2.57, 2.58 (2 s, 6H), 2.80-3.00 (m, 3H), 7.23-7.48 (m, 6H), 7.53-7.91 (m, Hz, 1H), 8.30-8.44 (m, 1H), 8.53-8.80 (m, 1H), 10.03, 10.89 (2 s, 1H), 12.65 (s, 1H)

$^{13}C$ *NMR (101 MHz, DMSO-$d_6$) δppm:* 26.5, 28.8, 30.0, 31.4, 32.4, 32.6, 34.5, 37.3, 37.4, 66.02, 66.1, 114.6, 114.8,

119.8. 120.5., 123.1, 123.8, 124-8, 126.2, 127.0, 127.2, 128.2, 128.6, 130.7, 131.2, 133.9, 134.2, 134.9, 135.3, 136.0, 138.9, 140.2

**Beispiel 238:1-Butyl-N,N-dimethyl-4-(1H-pyrrolo[2,3-b]pyridin-3-yl)cyclohexanamin Hydrochlorid, 1 Diastereomer ( 238)**

Bsp. 229 (500 mg, 1,68 mmol) wurde in abs. Methanol (100 ml) mit Palladiumkatalysator (Pd/C, 5 %, 200 mg) versetzt und 36 h bei RT hydriert (Wasserstoffdruck: 3 bar). Der Katalysator wurde mit Hilfe einer Fritte, versehen mit einer 1 cm hohen Schicht Celite, entfernt. Die Fritte wurde mit Methanol (1000 ml) gründlich gewaschen. Das Lösungsmittel wurde im Vakuum abdestilliert. 1-Butyl-N,N-dimethyl-4-(1H-pyrrolo[2,3-b]pyridin-3-yl)cyclohexanamin wurde in einer Ausbeute von 428 mg (85 %) als weißer Feststoff erhalten. Es wurde nur eins von zwei möglichen Diastereoisomeren erhalten.

1-Butyl-N,N-dimethyl-4-(1H-pyrrolo[2,3-b]pyridin-3-yl)cyclohexanamin (425 mg, 1,42 mmol) wurde in Ethylacetat (100 ml) gelöst. Bei RT wurde dann $Me_3SiCl$ (358 µl, 2,84 mmol) zugetropft und 1 h gerührt. Es fiel ein weißer Niederschlag aus. Der Niederschlag wurde abgesaugt, mit Ethylacetat (2 × 10 ml) gewaschen und anschließend getrocknet. Beispiel 238 (435 mg, 91 %) war ein farbloser Feststoff

*$^1H$ NMR (300 MHz, DMSO-d6) δppm:* 0.79-0.97(m, 4H), 1.21-1.48 (m, 5H), 1.53-1.71 (m, 3H), 1.74-1.88 (m, 3H), 1.97 (s, 6 H) 2.69-2.96 (m, 3H), 7.44-7.52 (m, 1H), 7.57-7.60 (m, 1H), 8.40-846 (m, 1H), 8.68 (d, J = 7.51 Hz, 1H), 10.62-10.43 (m, 1H), 12.88 (s, 1H)

*$^{13}C$ NMR (101 MHz, DMSO-$d_6$) δ ppm:* 13.8, 22.6, 24.6, 26.7, 28.7, 29.5, 32.6, 37.1, 37.3, 66.00 (s,1C), 114.8, 120.2, 123.7, 125.0, 134.1, 134.9, 139.3

**Beispiel 239: 4-(Benzofuran-2-yl)-1-benzyl-N,N-dimethylcyclohex-3-enamin Hydrochlorid**

Eine Lösung von Benzo[b]furan (Ind-92, 612 mg, 5,12 mmol) in trockenem THF (40 ml) wurde unter einem Argonstrom auf -8 °C gekühlt. Danach wurde vorsichtig tert-Butyllithium (6,22 mmol, 4,14 ml einer 1,5 M Pentanlösung) so zugetropft, daß dabei eine Reaktionstemperatur von -5 °C nicht überschritten wurde. Nach beendeter Zugabe wurde die Reaktionsmischung 2 h bei -5 °C gerührt. Im Anschluß wurde eine Lösung von Keton (Ket-3, 1,198 g, 5,18 mmol) in trockenem THF (10 ml) bei 0 °C zugetropft. Die Mischung wurde 1 h bei 0 °C und anschließend 4 d bei Raumtemperatur gerührt. Das Reaktionsgemisch wurde mit gesättigter Ammoniumchloridlösung (20 ml) gequencht, die organische Phase abgetrennt und die wäßrige Phase mit Dichlormethan (4 × 30 ml) extrahiert. Die vereinigten organischen Phasen wurden über Natriumsulfat getrocknet. Danach wurde das Lösungsmittel im Vakuum entfernt. Die Reinigung erfolgte mittels Flash-Chromatographie [Kieselgel, Cyclohexan/EtOAc (8 : 2)]. Es wurden 380 mg (21 %) des Cyclohexanols mit einem Schmelzpunkt von 121-124 °C erhalten

Ein Lösung soeben isolierten Cyclohexanols (250 mg, 0,72 mmol) in Bromwasserstoffsäure (5 ml, 48 %) wurde 15 min am Rückfluß erhitzt. Die abgekühlte Reaktionsmischung wurde mit 5N NaOH-Lösung auf einen pH-Wert von 9 eingestellt. Danach wurde die Mischung mit Dichlormethan (4 x 10 ml) extrahiert. Die vereinigten organischen Phasen wurden über Natriumsulfat getrocknet. Danach wurde das Lösungsmittel im Vakuum entfernt. Die Reinigung erfolgte mittels Flash-Chromatographie [Kieselgel, Cyclohexan/EtOAc (1 : 1)]. Es wurden 170 mg (71 %) des gewünschten Olefins erhalten.

Zur Herstellung des Hydrochlorids wurde das soeben isolierte Olefin (170 mg, 0,512 mmol) in Ethylmethylketon (5 ml) gelöst, mit Chlortrimethylsilan (105 mg, 0,769 mmol) versetzt und 45 min bei Raumtemperatur im offenen Reaktionsgefäß gerührt. Der dabei entstehende Feststoff wurde abgesaugt. Das Hydrochlorid wurde so in einer Ausbeute von 160 mg (61 %) als weißer Feststoff mit einem Schmelzpunkt von 115-119 °C gewonnen.

**Beispiel 240: N,N-Dimethyl-4-(3-methylbenzofuran-2-yl)-1-phenylcyclohex-3-enamin, Citrat (1:1)**

3-Methylbenzofuran (354 mg, 3 mmol) wurde zusammen mit dem Keton (Ket-10, 651 mg, 3 mmol) in Dichlormethan (25 ml) gelöst und mit Trifluormethansulfonsäure (0,3 ml, 3,4 mmol) versetzt. Der Ansatz wurde 20 h bei RT gerührt. - Zur Aufarbeitung wurde das Reaktionsgemisch mit 2N NaOH (10 ml) versetzt. Das Gemisch wurde weitere 20 min gerührt. Nach Trennung der Phasen wurde die wäßrige Phase mit Dichlormethan (3 x 20 ml) extrahiert. Die vereinigten organischen Extrakte wurden über $MgSO_4$ getrocknet und anschließend eingeengt. Das erhaltene Rohprodukt (950 mg) wurde säulenchromatographisch (Laufmittel: EtOAc) gereinigt. Das gewünschte Olefin wurde in 348 mg Ausbeute (35 %) als klebriges Öl erhalten.

Das soeben isolierte Olefin (331 mg, 1 mmol) wurde in der Siedehitze in Isopropanol (10 ml) gelöst und mit Citronensäure (192 mg, 1 mmol), gelöst in heißem Isopropanol (5 ml), versetzt. Die Lösung wurde 15 h bei 5 °C aufbewahrt. Die entstandenen Kristalle wurden mittels einer Fritte abgetrennt. Die Kristalle zerflossen an der Luft. weshalb sie schnell in ein Proberöhrchen gefüllt und anschließend im Vakuum getrocknet wurden. Das gewünschte Citrat wurde so in einer Ausbeute von 185 mg (35 %) als glasiger Feststoff erhalten.

**Beispiel 241: 2-(2-(4-(Dimethylamino)-4-phenylcyclohex-1-enyl)benzofuran-3-yl)ethanethiol, Citrat (1:1)**

Unter Argon wurde das Keton (Ket-10, 2,06 g, 9,5 mmol) zusammen mit 2-(Benzofuran-3-yl)ethanthiol (Ind-94, 1,70 g) in absolutem Dichlormethan (25 ml) vorgelegt. Anschließend erfolgte die Zugabe von Methansulfonsäure (680 µl, 10,45 mmol). Der Ansatz wurde 4 d bei RT gerührt. Zur Aufarbeitung wurde der Ansatz mit $H_2O$ (15 ml) versetzt. Die wäßrige Phase wurde abgetrennt und mit Dichlormethan (3 × 20 ml) extrahiert. Die vereinigten organischen Phasen wurden mit 2N $H_2SO_4$ gewaschen. Die Dichlormethanphase wurde am Rotationsverdampfer eingeengt. Der klebrige, gelbe Rückstand wurde mit Diethylether (3 × 10 ml) gewaschen. Die etherische Waschlösung wurde verworfen. Der verbleibende Rückstand wurde anschließend mit 2N NaOH (20 ml) versetzt. Das erhaltene Gemisch wurde mit Diethylether (3 × 15 ml) extrahiert. Die etherische Phase wurde über Natriumsulfat getrocknet und am Rotationsverdampfer eingeengt. Aus dem so erhaltenen Rückstand wurde mit Hilfe der Säulenchromatographie [Kieselgel 60 (100 g); Ethylacetat, Ethanol (9 : 1)] das gewünschte Olefin i als zähflüssiges Öl in einer Ausbeute von 381 mg (10 %, bezogen auf eingesetztes Keton) erhalten.
Das soeben isolierte Olefin (350 mg, 0,928 mmol) wurde in siedendem Ethanol (8 ml) gelöst, mit einer Lösung von Citronensäure (178 mg, 0,928 mmol) in heißem Ethanol (2 ml) versetzt, 10 min gerührt und auf RT kommen gelassen. Anschließend wurde der Ansatz im Kühlschrank auf 5 °C gebracht. Dabei fiel ein weißer Niederschlag aus, dessen Konsistenz bei Raumtemperatur nicht stabil war. Deshalb wurde das Ethanol bei ca. 5 °C abgegossen und der verbleibende feste Rückstand im Vakuum getrocknet. Das Citrat des Olefins wurde als glasiger Feststoff in einer Ausbeute von 252 mg (47 %) mit einem Schmelzpunkt von 55-57 °C erhalten.

**Beispiel 242: N,N-Dimethyl-4-(3-methylbenzo[b]thiophen-2-yl)-1-phenylcyclohex-3-enamin, Citrat (1:1)**

3-Methylbenzo[b]thiophen (Ind-95, 0,27 ml, 2 mmol) wurde zusammen mit dem Keton (Ket-10, 434 mg, 2 mmol) in Dichlormethan (20 ml) gelöst und mit Trifluormethansulfonsäure (0,2 ml, 2,3 mmol) versetzt. Der Ansatz wurde 2 Tage bei RT gerührt, wobei ein braunes Öl ausfiel. - Zur Aufarbeitung wurde das Reaktionsgemisch mit 2N NaOH (10 ml) versetzt. Das Gemisch wurde weitere 20 min gerührt. Nach Trennung der Phasen wurde die wäßrige Phase mit Dichlormethan (2 x 20 ml) extrahiert. Die vereinigten organischen Extrakte wurden über $MgSO_4$ getrocknet und anschließend eingeengt. Das erhaltene Rohprodukt (856 mg) wurde säulenchromatographisch (Laufmittel: EtOAc) gereinigt. Das gewünschte Olefin wurde so in 380 mg Ausbeute (54 %) als klebriges Öl erhalten. Durch Verreiben mit Methanol (3 ml) wurde eine Kristallbildung erreicht. Der erhaltene Feststoff (295 mg, Schmp.: 49-52 °C) wurden beim Stehen wieder klebrig.
Das soeben isolierte Olefin (295 mg, 0,85 mmol) wurde in der Siedehitze in Methanol (30 ml) gelöst und mit Citronensäure (163 mg, 0,85 mmol), gelöst in heißem Methanol (2 ml), versetzt. Da kein Niederschlag ausfiel, wurde das Lösungsmittel abgezogen. Und das gewünschte Citrat (458 mg, 100 %) erhalten.

Beispiel 243: N,N-dimethyl-4-(3-methylbenzo[b]thiophen-2-yl)-1-phenylcyclohexanamin, Citrat (1:1) Eines von 2 möglichen Diastereomeren 3-Methylbenzo[b]thiophen (Ind-95, 0,27 ml, 2 mmol) wurde zusammen mit dem Keton (Ket-10, 434 mg, 2 mmol) in HBr/Eisessig (33 % HBr, 20 ml) gelöst und 50 h bei RT gerührt. Anschließend wurde zu dem Ansatz Sn-Pulver (1 g, 8,5 mmol) innerhalb von 30 min portionsweise bei RT gegeben. Nach Beendigung der Zugabe wurde das Reaktionsgemisch weitere 20 h gerührt. - Zur Aufarbeitung wurde das Gemisch am Rotationsverdampfer bis zur Trockene eingeengt. Der verbliebene Rückstand wurde durch Zugabe von 5N NaOH (40 ml) basisch gemacht. Die erhalte Lösung wurde mit Dichlormethan versetzt und extrahiert (4 x 20 ml). Die vereinigten organischen Phasen wurden mit $MgSO_4$ getrocknet und anschließend eingeengt. Der erhaltene Rückstand (552 mg) wurde säulenchromatographisch (Laufmittel: 1. EtOAc; 2. EtOAc/EtOH 2 : 1) gereinigt. Neben Olefin (120 mg) wurde so eins der zwei möglichen Diastereoisomeren Reduktionsprodukte in einer Ausbeute von 200 mg (28 %) erhalten.
Das isolierte Reduktionsprodukt (224 mg, 0,64 mmol) wurde in der Siedehitze in Methanol (5 ml) gelöst und mit Citronensäure (124 mg, 0,64 mmol), gelöst in heißem Methanol (2 ml), versetzt. Da kein Niederschlag ausfiel, wurde das Lösungsmittel abgezogen. Der erhaltene feste Rückstand wurde mit Dichlormethan verrieben. Das gewünschte Produkt (271 mg, 78 %) wurde so als Citrat mit einem Schmelzpunkt von 178-179 °C erhalten.

**Beispiel 244: N,N-Dimethyl-1-phenyl-4-(1H-pyrrolo[2,3-b]pyridin-3-yl)cyclohexanamin, Citrat (1:1), 1 Diastereomer**

Die freie Base aus Beispiel 230 (300 mg, 0,95 mmol) wurde in abs. Methanol (40 ml) mit Palladiumkatalysator (Pd/C, 5 %, 120 mg) versetzt und 36 h bei RT hydriert (Wasserstoffdruck: 3 bar). Der Ablauf der Reaktion wurde durch DC verfolgt. Der Katalysator wurde mit Hilfe einer Fritte, versehen mit einer 1 cm hohen Schicht Celite, entfernt. Die Fritte wurde mit Methanol (1000 ml) gründlich gewaschen. Das Lösungsmittel wurde im Vakuum abdestilliert. Das erhaltene Rohprodukt (310 mg) konnte durch Säulenchromatographie [Kieselgel 60 (20 g); MeOH (500 ml)] gereinigt werden. N,N-Dimethyl-1-phenyl-4-(1H-pyrrolo[2,3-b]pyridin-3-yl)cyclohexanamin (1 Diastereomer) wurde in einer Ausbeute von 180 mg (60 %) als weißer Feststoff erhalten. Der Feststoff (180 mg, 0,56 mmol) wurde in der Siedehitze in Isopropanol (10 ml) gelöst und mit Citronensäure (108 mg, 0,56 mmol), gelöst in heißem Isopropanol (3 ml), versetzt. Der Ansatz wurde 16 h bei 5 °C stehen gelassen. Der entstandene weiße Niederschlag wurde mittels einer Fritte abgetrennt. Beispiel 244 wurde in einer Ausbeute von 173 mg (60 %, *Schmelzpunkt:* 130-133 °C) erhalten.
*$^1$H NMR (300 MHz, DMSO-$d_6$) $\delta$ ppm:* 1.18-1,45 (m, 2H), 1.84-2.15 (m, 4H), 2.40 (s, 6H), 2,52-2.67 (m, 4H), 2.78-3.02 (m, 3H), 6.82-6.96 (m, 1H), 7,00 (S, 1H), 7.39-7.59 (m, 3H), 7.59-7.77 (m, 3H), 8.03-8.18 (m, 1H), 11.24 (s, 1H)
*$^{13}$C NMR (101 MHz, DMSO-$d_6$) $\delta$ppm:* 29.2, 31.1, 34.1, 37.5, 44.0, 66.6, 71.4, 114.5, 117.6, 118.1, 120.9, 126.6, 128.6, 128.7, 129.2, 132.2, 142.2, 148.7, 171.21, 176.5

**Beispiel 246: 1-(Dimethylamino)-3-(2-(4-(dimethylamino)-4-phenylcyclohexyl)-3-methyl-1H-indol-1-yl)propan-2-ol, unpolares Diastereomer**

Zu einer Suspension von Beispiel 247 (freie Base) (115 mg, 0,40 mmol) in Ethanol (15 ml) wurde bei Raumtemperatur Dimethylamin (324 mg, 2,36 mmol; 33-proz. in Ethanol, 0,76 g/ml) gegeben. Die Reaktionsmischung wurde 9 h bei 59 °C (Ölbadtemperatur) gerührt. Anschließend wurde die Reaktionsmischung im Vakuum vollständig von flüchtigen Bestandteilen befreit. Der Rückstand wurde mit Diethylether (3 ml) versetzt. Die Mischung wurde 3 Tage bei 0 °C gelagert. Es fiel ein Niederschlag aus. Die überstehende Lösung wurde abdekantiert. Beispiel 244 (Smp. 54-57 °C) wurde als farbloser Feststoff in einer Ausbeute von 50 % (64 mg, 0,15 mmol) erhalten.

**Beispiel 247: (±)-N,N-Dimethyl-4-(3-methyl-1-(oxiran-2-ylmethyl)-1H-indol-2.yl)-1-phenylcyclohexanamin, Citrat (1:1), unpolares Diastereomer**

Die freie Base aus Beispiel 17 (unpolares Diastereomer, 279 mg, 0,84 mmol) wurde in Dimethylformamid/Tetrahydrofuran (20 ml, 1 : 1) gelöst und die klare hellgelbe Lösung mit Natriumhydrid (60-proz. Suspension in Mineralöl, 70 mg, 1,75 mmol) versetzt. Anschließend fiel ein heller Feststoff aus der Reaktionsmischung aus. Es wurde 1 h bei 57 °C (Ölbadtemperatur) gerührt. Dann wurde bei dieser Temperatur das Epichlorhydrin (163 mg, 1,76 mmol; 1,183 g/ml) hinzugegeben. Es wurde 1 h bei 57 °C (Ölbadtemperatur) gerührt. Anschließend wurde die Reaktionsmischung mit Wasser (30 ml) und Diethylether (20 ml) versetzt. Die Mischung wurde 10 min gerührt. Anschließend wurden die Phasen getrennt. Die wässrige Phase wurde mit Ethylacetat (2 x 20 ml) extrahiert. Die vereinigten organischen Phasen (Ethylacetat und Diethylether) wurden mit gesättigter Natriumchlorid-Lösung (3 × 10 ml) gewaschen, mit Natriumsulfat getrocknet und filtriert. Anschließend wurden die flüchtigen Bestandteile im Vakuum vollständig entfernt. Es verblieben 406 mg (±)-N.N-Dimethyl-4-(3-methyl-1-(oxiran-2-ylmethyl)-1H-indol-2-yl)-1-phenylcyclohexanamin. Das Rohprodukt wurde ohne weitere Reinigung weiter umgesetzt.
(±)-N,N-Dimethyl-4-(3-methyl-1-(oxiran-2-ylmethyl)-1H-indol-2-yl)-1-phenylcyclohexanamin (62 mg. 0,16 mmol) wurde in siedendem Ethanol (5 ml) in Lösung gebracht. Citronensäure (34 mg, 0,18 mmol) wurde dann hinzugegeben. Die klare Lösung wurde in der Siedehitze 3 h gerührt. Anschließend wurde die Reaktionsmischung auf Raumtemperatur abgekühlt und 24 h bei dieser Temperatur stehen gelassen. Es fiel ein farbloser, mikrokristalliner Niederschlag aus. Dieser wurde abfiltriert und mit Ethanol (2 × 5 ml) gewaschen. Es wurden 69 mg (0,12 mmol; 75 %) Beispiel 247, (Schmp.: 175-178 °C) erhalten.

**Beispiel 248: 4-(1,3-Dimethyl-1H-indol-2-yl)-N,N-dimethyl-1-phenylcyclohexanamin, unpolares Diastereomer**

Die freie Base aus Beispiel 17 (unpolares Diastereomer, 176 mg, 0,53 mmol) wurde in einer Argonatmosphäre in trockenem Dimethylformamid (15 ml) vorgelegt und mit Natriumhydrid (60-proz. Suspension in Mineralöl, 21 mg, 0,53 mmol) versetzt. Die Reaktionsmischung wurde 1 h bei Raumtemperatur gerührt und anschließend im Eisbad auf 0 °C gekühlt. Zu der gelblichen Lösung wurde Methyliodid (150 mg, 0,06 ml, 1,06 mmol, 2,28 g/ml) gegeben, worauf eine sofortige Entfärbung einsetzte. Die Reaktionsmischung wurde innerhalb von 2 h auf Raumtemperatur erwärmt und über Nacht bei Raumtemperatur gerührt. Zur Aufarbeitung wurde die Reaktionsmischung mit 2N Natriumhydroxid-Lösung (10 ml) und Wasser (10 ml) versetzt und 10 min gerührt. Die trübe Mischung wurde über Nacht stehen gelassen und am nächsten Tag filtriert. Der erhaltene farblose Feststoff wurde im Vakuum getrocknet. Es wurden 135 mg (0,39 mmol; 73 %) Beispiel 248 (Schmp.: 209-212 °C) isoliert.

**Beispiel 249: 2-(4-Butyl-4-(dimethylamino)cyclohexyl)-3-methyl-1H-indol-5-ol, Citrat (1:2), unpolares Diastereomer**

Zu einer Lösung der freien Base aus Beispiel 125 (unpolareres Diastereoisomer, 115 mg, 0,33 mmol) in trockenem Dichlormethan (20 ml) wurde bei RT unter Rühren und Feuchtigkeitsausschluß eine 1 M Lösung von $BBr_3$ in Dichlormethan (1 ml, 1 mmol) gegeben. Nach 10 min fiel ein Niederschlag aus. Der Ansatz wurde 24 h bei RT gerührt.

- Zur Aufarbeitung wurde das Gemisch mit ges. $NaHCO_3$-Lösung (15 ml) versetzt und 24 h gerührt. Die organische Phase wurde abgetrennt und die wässrige Phase mit Dichlormethan (3 × 10 ml) extrahiert. Die vereinigten organischen Phasen wurden über $Na_2SO_4$ getrocknet und anschließend eingeengt. 2-(4-Butyl-4-(dimethylamino)cyclohexyl)-3-methyl-1H-indol-5-ol wurde so in einer Ausbeute von 107 mg (99 %) erhalten.
2-(4-Butyl-4-(dimethylamino)cyclohexyl)-3-methyl-1H-indol-5-ol (unpolares Diastereomer, 107 mg, 0,32 mmol) wurde in heißem Isopropanol (50 ml) gelöst und mit isopropanolischer Citronensäure-Lösung (62 mg, 0,32 mmol in 2 ml) versetzt. Die Mischung wurde 2 h bei Raumtemperatur gerührt. Das ausgefallene weiße Feststoff wurde abgesaugt und Beispiel 249 in einer Ausbeute von 47 mg (27 %) mit einem Schmelzpunkt von 197-208 °C erhalten

**Beispiel 250: 2-(4-Butyl-4-(dimethylamino)cyclohexyl)-3-methyl-1H-indol-5-ol, Citrat (1:1), polares Diastereomer**

Zu einer Lösung Zu einer Lösung der freien Base des Beispiel 126 (polareres Diastereoisomer, 263 mg, 0,77 mmol) in trockenem Dichlormethan (40 ml) wurde bei RT unter Rühren und Feuchtigkeitsausschluß eine 1 M Lösung von $BBr_3$ in Dichlormethan (0,66 ml, 0,66 mmol) gegeben. Nach 10 min fiel ein Niederschlag aus. Der Ansatz wurde 24 h bei RT gerührt. -Zur Aufarbeitung wurde das Gemisch mit ges. $NaHCO_3$-Lösung (15 ml) versetzt und 24 h gerührt. Der an der Phasengrenze befindliche Feststoff (100 mg) wurde abgetrennt. wurde in einer Mischung aus ges. $NaHCO_3$-Lösung (20 ml) und Ethylacetat (10 ml) 1 h gerührt. Die organische Phase wurde abgetrennt. Der wässrige Rückstand wurde mit Ethylacetat (5 × 10 ml) extrahiert. Die vereinigten organischen Phasen wurden über $Na_2SO_4$ getrocknet und anschließend eingeengt. 2-(4-Butyl-4-(dimethylamino)cyclohexyl)-3-methyl-1H-indol-5-ol wurde so in einer Ausbeute von 35 mg (13 %) erhalten. Das Filtrat wurde erneut mit Dichlormethan (4 × 10 ml) extrahiert. Die vereinigten organischen Phasen wurden über $Na_2SO_4$ getrocknet und anschließend eingeengt. 2-(4-Butyl-4-(dimethylamino)cyclohexyl)-3-methyl-1H-indol-5-ol (polareres Diastereoisomer) wurde insgesamt in einer Ausbeute von 135 mg (53 %) erhalten.

2-(4-Butyl-4-(dimethylamino)cyclohexyl)-3-methyl-1H-indol-5-ol (polareres Diastereoisomer) (135 mg, 0,41 mmol) wurde in heißem Isopropanol (30 ml) gelöst und mit isopropanolischer Citronensäure-Lösung (79 mg, 0,41 mmol in 3 ml) versetzt. Die Mischung wurde 2 h bei Raumtemperatur gerührt. Der beige Feststoff wurde abgesaugt und Beispiel 250 in einer Ausbeute von 160 mg (74 %) mit einem Schmelzpunkt von 145-159 °C erhalten.

**Beispiel 251: (±)-2-(4-(Dimethylamino)-4-(thiophen-2-yl)-cyclohex-1-enyl)-3-(2-(pyridin-4-yl)ethyl)-1H-indol**

Ind-14 (667 mg, 3 mmol) wurde zusammen mit Keton Ket-12 (671 mg, 3 mmol) in abs. Dichlormethan (45 ml) gelöst und mit Trifluormethansulfonsäure (0,660 ml, 7,43 mmol) versetzt. Der Ansatz wurde 64 h bei RT gerührt, wobei ein braunes Öl ausfiel. Zur Aufarbeitung wurde die Reaktionslösung mit 1N NaOH (30 ml) und Methanol (10 ml) versetzt. Das Gemisch wurde weitere 60 min gerührt. Nach Trennung der Phasen wurde die wässrige Phase mit Dichlormethan (3 × 10 ml) extrahiert. Die vereinigten organischen Extrakte wurden über $Na_2SO_4$ getrocknet und anschließend eingeengt. Das erhaltene Rohprodukt (1,33 g) wurde säulenchromatographisch gereinigt [Kieselgel 60 (100 g); Ethylacetat/Methanol 10 : 1 (1650 ml), Ethylacetat/Methanol 5 : 1 (600 ml)]. Bsp. 251 wurde als gelber Feststoff (373 mg, 29 %. Fp.: 189-193 °C) erhalten.

**Beispiel 252: ±)-2-(4-(Dimethylamino)-4-(3-fluorphenyl)-cyclohex-1-enyl)-3-(2-(pyridin-4-yl)ethyl)-1 H-indol**

Ind-14 (667 mg, 3 mmol) wurde zusammen mit Ket-13 (706 mg, 3 mmol) in abs. Dichlormethan (45 ml) gelöst und mit Trifluormethansulfonsäure (0,660 ml, 7,43 mmol) versetzt. Der Ansatz wurde 64 h bei RT gerührt, wobei ein braunes Öl ausfiel. Zur Aufarbeitung wurde die Reaktionslösung mit 1 N NaOH (30 ml) und Methanol (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über $Na_2SO_4$ getrocknet und anschließend eingeengt. Das erhaltene Rohprodukt (1,33 g) wurde säulenchromatographisch gereinigt [Kieselgel 60 (100 g); Ethylacetat/Methanol 10 : 1 (1100 ml), Ethylacetat/Methanol 5 : 1 (900 ml)]. Das erhaltene Produkt (425 mg) wurde aus Methylethylketon (2,5 ml) umkristallisiert (bei -5 °C) und Beispiel 252 als hellgelber Feststoff (309 mg, 23 %, Schmp.: 194-198 °C) erhalten.

**Beispiel 253: ±)-2-(4-Butyl-4-(pyrrolidin-1-yl)cyclohex-1-enyl)-3-(2-(pyridin-4-yl)ethyl)-1 H-indol**

Ind-14 (667 mg, 3 mmol) wurde zusammen mit Ket-14 (671 mg, 3 mmol) in abs. Dichlormethan (45 ml) gelöst und mit Trifluormethansulfonsäure (0,613 ml, 6,9 mmol) versetzt. Der Ansatz wurde 64 h bei RT gerührt, wobei ein braunes Öl ausfiel. Zur Aufarbeitung wurde die Reaktionslösung mit 1N NaOH (30 ml) und Methanol (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über $Na_2SO_4$ getrocknet und anschließend eingeengt. Das erhaltene Rohprodukt (1,35 g) wurde säulenchromatographisch gereinigt [Kieselgel 60 (90 g); Ethylacetat/Methanol

1 : 1 (2400 ml)]. Bsp. 253 wurde als beigefarbener Feststoff (292 mg, 23 %, Schmp.: 187-195 °C) erhalten

**Beispiel 254: ±)-2-(4-(Methylamino)-4-phenylcyclohex-1-enyl)-3-(2-(pyridin-4-yl)ethyl)-1H-indol (254)**

Ind-14 (667 mg, 3 mmol) wurde zusammen mit Ket-15 (610 mg, 3 mmol) in abs. Dichlormethan (45 ml) gelöst und mit Trifluormethansulfonsäure (0,613 ml, 6,9 mmol) versetzt. Der Ansatz wurde 64 h bei RT gerührt, wobei ein braunes Öl ausfiel. Zur Aufarbeitung wurde die Reaktionslösung mit 1N NaOH (30 ml) und Methanol (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über $Na_2SO_4$ getrocknet und anschließend eingeengt. Das erhaltene Rohprodukt (1,47 g) wurde säulenchromatographisch gereinigt [Kieselgel 60 (90 g); Ethylacetat/Methanol 5 : 1 (1200 ml), Ethylacetat/Methanol 1 : 1 (1200 ml)]. Bsp. 254 wurde als hellgelber Feststoff (578 mg, 47 %, Schmp.: 168-172 °C) erhalten.

**Beispiel 255: (±)-2-(4-(Dimethylamino)-4-(thiophen-2-yl)-cyclohex-1-enyl)-3-methyl-1H-indol, Citrat (1:1)**

Ind-10 (686 mg, 5,24 mmol) wurde zusammen mit Ket-12 (1,17 g, 5,24 mmol) in Dichlormethan (40 ml) gelöst und mit Trifluormethansulfonsäure (0,5 ml, 5,6 mmol) versetzt. Der Ansatz wurde 20 h bei RT gerührt. - Zur Aufarbeitung wurde das Reaktionsgemisch mit $H_2O$ (20 ml) versetzt wobei ein klebriger Niederschlag ausfiel. Die Mischung wurde 10 min gerührt, anschließend wurde die wässrige Phase, die geringe Mengen unumgesetztes Keton enthielt (< 100 mg), abgetrennt. Die organische Phase und der klebrige Feststoff wurden mit ges. $NaHCO_3$-Lösung (20 ml) versetzt. Das Lösungsmittelgemisch wurde bei RT gerührt (60 min), bis alle Subtanz gelöst war. Nach Abtrennung der organischen Phase wurde die wässrige Phase mit Dichlormethan (3 x 20 ml) extrahiert. Die vereinigten organischen Extrakte wurden mit Wasser gewaschen, über $MgSO_4$ getrocknet und anschließend eingeengt. Das erhaltene Rohprodukt (2 g) konnte durch Säulenchromatographie [Kieselgel 60 G (10 g); EtOAc (100 ml)] gereinigt werden. (±)-2-(4-(Dimethylamino)-4-(thiophen-2-yl)-cyclohex-1-enyl)-3-methyl-1H-indol wurde in einer Ausbeute von 882 mg (50 %) als Feststoff *(Schmelzpunkt:* 183-192 °C) erhalten.
(±)-2-(4-(Dimethylamino)-4-(thiophen-2-yl)-cyclohex-1-enyl)-3-methyl-1H-indol (168 mg, 0,5 mmol) wurde in der Siedehitze in Isopropanol (15 ml) gelöst und mit Citronensäure (102 mg, 0,53 mmol), gelöst in heißem Isopropanol (2 ml), versetzt. Nach der Zugabe der Säure fiel ein Niederschlag aus. Zur Vervollständigung der Fällung wurde der Ansatz auf 5 °C abgekühlt (Kühlschrank) und 17 h bei dieser Temperatur belassen. Der Niederschlag wurde mittels einer Fritte abgetrennt und anschließend getrocknet. Beispiel 255 wurde so in einer Ausbeute von 163 mg (61 % als Feststoff erhalten.

**Beispiel 256: N,N-Dimethyl-4-(3-methyl-1H-indol·2-yl)-1-(thiophen-2-yl)cyclohexanamin, Citrat (2:1), unpolares Diastereomer**

**Beispiel 257: N,N-Dimethyl-4-(3-methyl-1H-indol-2-yl)-1-(thiophen-2-yl)cyclohexanamin, Citrat (2:1), polares Diastereomer**

**N,N-Dimethyl-4-(3-methyl-1H-indol-2-yl)-1-(thiophen-2-yl)cyclohexanamin (unpolares und polares Diastereomer)**

Die freie Base aus Beispiel 255 (436 mg, 1,3 mmol) wurde in HBr/Eisessig (33 % HBr, 30 ml) aufgenommen. Anschließend wurde zu dem Ansatz bei RT innerhalb von 4 h Sn-Pulver (4 g, 33 mmol) portionsweise gegeben. Nach Beendigung der Zugabe wurde das Reaktionsgemisch weitere 18 h bei RT gerührt. - Zur Aufarbeitung wurde das Gemisch mit EtOH (20 ml) verdünnt und das Lösungsmittelvolumen am Rotationsverdampfer auf ca. 10 ml eingeengt. Der verbliebene Rückstand wurde auf 200 ml Wasser gegeben. Der ausgefallene Feststoff wurde mittels Filtration abgetrennt und anschließend mit 2N NaOH (50 ml) versetzt. Das erhaltene Gemisch wurde mit Dichlormethan extrahiert (4 x 20 ml). Die vereinigten organischen Phasen wurden mit Wasser (50 ml) gewaschen, über $MgSO_4$ getrocknet und anschließend eingeengt. Der erhaltene Rückstand (380 mg) wurde durch Säulenchromatographie [Kieselgel 60 G (10 g); EtOAc (150 ml)] gereinigt. N,N-Dimethyl-4-(3-methyl-1H-indol-2-yl)-1-(thiophen-2-yl)cyclohexanamin (unpolareres Diastereomer) wurde in einer Ausbeute von 108 mg (24 %) als weißer Feststoff mit einem Schmelzpunkt von 191-197 °C (aus Methanol) erhalten. Das polarere Diastereomer wurde in einer Ausbeute von 70 mg (16 % Ausbeute) als weißer Feststoff mit einem Schmelzpunkt von 218-225 °C erhalten.

**N,N-Dimethyl-4-(3-methyl-1 H-indol-2-yl)-1-(thiophen-2-yl)cyclohexanamin, Citrat (2:1), unpolares Diastereomer**

N,N-Dimethyl-4-(3-methyl-1H-indol-2-yl)-1-(thiophen-2-yl)cyclohexanamin (unpolareres Diastereomer) (91 mg, 0,27 mmol) wurde in der Siedehitze in Isopropanol (4 ml) gelöst und mit Citronensäure (60 mg, 0,31 mmol), gelöst

in heißem Isopropanol (1 ml), versetzt. Sofort fiel ein Niederschlag aus. Zur Vervollständigung der Fällung wurde der Ansatz auf 5 °C abgekühlt (Kühlschrank) und 2 h bei dieser Temperatur belassen. Der Niederschlag wurde mittels einer Fritte abgetrennt und anschließend getrocknet. Beispiel 256 wurde so in einer Ausbeute von 81 mg (68 %, *Schmelzpunkt:* 196-198 °C) erhalten.

**N,N-Dimethyl-4-(3-methyl-1H-indol-2-yl)-1-(thiophen-2-yl)cyclohexanamin, Citrat (2:1), polares Diastereomer**

N,N-Dimethyl-4-(3-methyl-1H-indol-2-yl)-1-(thiophen-2-yl)cyclohexanamin (polareres Diastereomer) (64 mg, 0,19 mmol) wurde in der Siedehitze in Isopropanol (4 ml) gelöst und mit Citronensäure (52 mg, 0,27 mmol), gelöst in heißem Isopropanol (1 ml), versetzt. Das Lösungsmittel wurde entfernt und der Rückstand in Methanol (10 ml) aufgenommen. Die Lösung wurde dann mit Wasser versetzt (3 ml) und das Methanol am Rotationsverdampfer abgezogen. Der so erhaltene Niederschlag wurde mittels einer Fritte abgetrennt und anschließend getrocknet. Beispiel 257 wurde so in einer Ausbeute von 62 mg (75 %, Schmelzpunkt ab 168 °C)erhalten.

**Beispiel 258: (±)-2-(4-Butyl-4-(pyrrolidin-1-yl)cyclohex-1-enyl)-3-methyl-1H-indol**

Das Keton Ket-14 (800 mg, 3,58 mmol) und Ind-10 (470 mg, 3,58 mmol) wurden in abs. Dichlormethan (50 ml) gelöst, mit Trifluormethansulfonsäure (0,953 ml, 1,61 g, 10,74 mmol) versetzt und 18 h bei Raumtemperatur gerührt. Der Ansatz wurde mit Wasser (20 ml) und 1 N Natronlauge (15 ml) versetzt und 1 h gerührt. Die Phasen wurden getrennt. Die wässrige Phase wurde mit Dichlormethan (2 × 20 ml) extrahiert. Die organischen Phasen wurden vereinigt, mit Wasser (20 ml) gewaschen, mit Natriumsulfat getrocknet und eingeengt. Der Rückstand war ein braunes Öl (1,24 g ), das chromatographisch [Kieselgel 60 (100 g); Ethylacetat/Methanol 4 : 1 (500 ml), Methanol (600 ml)] aufgetrennt wurde. Beispiel 258 wurde als beigefarbener Feststoff in einer Ausbeute von 36 % (426 mg) gewonnen.

**Beispiel 259: N-Methyl-4-(3-methyl-1H-indol-2-yl)-1-phenylcyclohexanaminhydrobromide, unpolares Diastereomer**

**Beispiel 260: N-Methyl-4-(3-methyl-1H-indol-2-yl)-1-phenylcyclohexanamin, Citrat (1:1), polares Diastereomer**

**(±)-*N*-Methyl-*N*-[4-(3-methyl-1*H*-indol-2-yl)-1-phenylcyclohex-3-enyl]amin**

3-Methylindol (Ind-10, 393 mg, 3 mmol) wurde zusammen mit Ket-15 (609 mg, 3 mmol) in Dichlormethan (25 ml) gelöst und mit Trifluormethansulfonsäure (0,4 ml, 4,5 mmol) versetzt. Der Ansatz wurde 24 h bei RT gerührt. - Zur Aufarbeitung wurde das Reaktionsgemisch mit 2N NaOH (20 ml) versetzt und 20 min bei RT gerührt. Nach Abtrennung der organischen Phase wurde die wässrige Phase mit Dichlormethan (2 x 20 ml) extrahiert. Die vereinigten organischen Extrakte wurden über $Na_2SO_4$ getrocknet und anschließend eingeengt. Das Rohprodukt ((±)-*N*-Methyl-*N*-[4-(3-methyl-1*H*-indol-2-yl)-1-phenylcyclohex-3-enyl]amin) wurde in einer Ausbeute von 945 mg (99 %) als gelbes Öl erhalten und wurde ohne weitere Reinigung in der nächsten Reaktion eingesetzt.

**N-Methyl-4-(3-methyl-1H-indol-2-yl)-1-phenylcyclohexanaminhydrobromide, unpolares Diastereomer (259) und N-Methyl-4-(3-methyl-1H-indol-2-yl)-1-phenylcyclohexanamin (polares Diastereomer)**

(±)-*N*-Methyl-*N*-[4-(3-methyl-1*H*-indol-2-yl)-1-phenylcyclohex-3-enyl]amin (900 mg, 2,8 mmol) wurde in HBr/Eisessig (33 % HBr, 30 ml) gelöst. Anschließend wurde zu dem Ansatz bei RT Sn-Pulver (1,66 g, 14 mmol) innerhalb von 20 min portionsweise gegeben. Nach Beendigung der Zugabe wurde das Reaktionsgemisch 9 d (wahrscheinlich wesentlich kürzere Reaktionszeit ausreichend) bei RT gerührt. Dabei entstand eine klare Lösung. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (50 ml) basisch gemacht. Das erhaltene wässrige Gemisch wurde mit Ethylacetat (4 x 20 ml) extrahiert. Die vereinigten organischen Phasen wurden mit Wasser (50 ml) gewaschen, über $Na_2SO_4$ getrocknet und anschließend eingeengt. Das Rohprodukt (1 g) wurde als Gemisch aus Base und Hydrobromid erhalten und konnte durch Säulenchromatographie [Kieselgel 60 (50 g); Ethylacetat (250 ml), Methanol (250 ml)] getrennt werden. Das unpolarere Produkt ( 259) wurde als Hydrobromid in einer Ausbeute von 67 mg (6 %) mit einem Schmelzpunkt von 288-298 °C erhalten. Das polarere Produkt (N-Methyl-4-(3-methyl-1H-indol-2-yl)-1-phenylcyclohexanamin) wurde als Base in einer Ausbeute von 390 mg (39 %) isoliert.

$^1$H NMR (300 MHz, RT, DMSO-$D_6$) δ ppm: 1.80 (psd, 2H), 2.03 (pst, 2H), 2.19 (s, 3H), 2.21 (s, 3H), 2.36-2.70 (m, 4H, [über der DMSO-D5-Resonanz]), 3.05 (pst, 1H), 4.43 (d, 1H), 6.94 (t, 1H), 7.02 (t, 1H), 7.32-7.58 (m, 4H), 7.68 (psd, 1H), 9.37 (s, vbr, 2H), 11.10 (s, 1H).

$^{13}$C NMR (101 MHz, RT, DMSO-$D_6$) δ ppm: 8.3, 26.3, 26.8, 32.1, 32.4, 61.2, 103.8, 110.3, 117.6, 117.9, 120.2, 126.2,

128.4, 128.6, 128.9, 135.1, 139.1. 139.2 [br].

**N-Methyl-4-(3-methyl-1H-indol-2-yl)-1-phenylcyclohexanamin, Citrat (1:1), polares Diastereomer**

N-Methyl-4-(3-methyl-1H-indol-2-yl)-1-phenylcyclohexanamin (polareres Diastereomer) (390 mg, 1,22 mmol) wurde in heißem Isopropanol (250 ml) gelöst und mit ethanolischer Citronensäure-Lösung (234 mg, 1,22 mmol in 5 ml) versetzt. Die Mischung wurde 2 h bei Raumtemperatur gerührt. Der ausgefallene Feststoff wurde abgesaugt. Beispiel 260 wurde in einer Ausbeute von 270 mg (43 %, weißer Feststoff) mit einem Schmelzpunkt ab 189 °C erhalten.
$^{1}$*H NMR (300 MHz, RT, DMSO-D$_6$)* δ *ppm:* 1.54 (psq, 2H), 1.83 (psd, 2H), 2.05-2.20 (m, 8H), 2.76-3.03 (m, 3H), 3.38 (s, br, 3H [Wasser drunter]), 6.91 (dqu, 2H), 7.15 *(dd, 1H), 7.32 (psd, 1H), 7.40-7.65 (m, 4H), 7.71 (psd, 1H), 10.41 (s, 1H).*
$^{13}$*C NMR (101 MHz, RT, DMSO-D$_6$)* δ *ppm:* 8.3, 25.8, 27.8, 32.2, 35.2, 61.7, 104.0, 110.5, 117.3, 117.9, 119.9, 128.3, 128.6, 128.8, 129.0,134.4,135.0, 138.1.

**Beispiel 261: 1-(3-Fluorphenyl)-N,N-dimethyl-4-(3-methyl-1H-indol-2-yl)cyclohexanamin, Citrat (2:1), unpolares Diastereomer**

**Beispiel 262: 1-(3-Fluorphenyl)-N,N-dimethyl-4-(3-methyl-1H-indol-2-yl)cyclohexanamin, Citrat (2:1), polares Diastereomer**

**1-(3-Fluorphenyl)-N,N-dimethyl-4-(3-methyl-1H-indol-2-yl)cyclohexanamin (unpolares und polares Diastereomer)**

Bsp. 263 (450 mg, 1,3 mmol) wurde in HBr/Eisessig (33 % HBr, 15 ml) suspendiert. Anschließend wurde zu dem Ansatz bei RT Sn-Pulver (771 mg, 6,5 mmol) innerhalb von 10 min portionsweise gegeben. Nach Beendigung der Zugabe wurde das Reaktionsgemisch 48 h bei RT gerührt. Dabei entstand eine klare Lösung. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (30 ml) basisch gemacht. Das erhaltene wässrige Gemisch wurde mit Dichlormethan (4 x 20 ml) extrahiert. Die vereinigten organischen Phasen wurden mit Wasser (50 ml) gewaschen, über $Na_2SO_4$ getrocknet und anschließend eingeengt. Das erhaltene Rohprodukt (300 mg) konnte durch Säulenchromatographie [Kieselgel 60 (30 g); 1. Ethylacetat (150 ml), 2. Methanol (150 ml)] gereinigt werden. 1-(3-Fluorphenyl)-N,N-dimethyl-4-(3-methyl-1H-indol-2-yl)cyclohexanamin (unpolareres Diastereoisomer) wurde in einer Ausbeute von 130 mg (29 %) erhalten. Das polarere Diastereoisomer wurde in einer Ausbeute von 130 mg (29 %) isoliert.

**1-(3-Fluorphenyl)-N,N-dimethyl-4-(3-methyl-1H-indol-2-yl)cyclohexanamin, Citrat (2:1), unpolares Diastereomer (261)**

1-(3-Fluorphenyl)-N,N-dimethyl-4-(3-methyl-1H-indol-2-yl)cyclohexanamin (unpolareres Diastereoisomer) (120 mg, 0,34 mmol) wurde in heißem Isopropanol (110 ml) gelöst und mit ethanolischer Citronensäure-Lösung (66 mg, 0,34 mmol in 3 ml) versetzt. Die Mischung wurde 2 h bei Raumtemperatur gerührt. Der ausgefallene Feststoff wurde abgesaugt. Beispiel 261 wurde in einer Ausbeute von 90 mg (59 %, weißer Feststoff) mit einem Schmelzpunkt von 228-237 °C erhalten.
$^{1}$H NMR (300 MHz, RT, DMSO-D$_6$) δ *ppm:* 1.4-1.72 (m, 4H), 2.02-2.24 (m, 9H), 2.66 (dd, 2H), 2.78 (psd, 2H), 2.93 (pst, 1H), 6.90 (dqu, 2H), 7.14 (psd, 1H), 7.27-7.43 (m, 2H), 7.46-7.69 (m, 3H), 10.38 (s, 1H).
$^{13}$C NMR (101 MHz, RT, DMSO-D$_6$) *δppm:* 8.4, 29.9, 32.8; 34.9, 37.7, 59.1 (br), 72.09, 103.7, 110.6, 113.4 (d), 113.7 (d), 117.2, 117.8, 119.7, 122.7 (d), 128.8, 129.1 (d), 135.1, 139.5, 142.1 (br), 162.0 (d), 172.2, 175.1.

**1-(3-Fluorphenyl)-N,N-dimethyl-4-(3-methyl-1H-indol-2-yl)cyclohexanamin, Citrat (2:1), polares Diastereomer ( 262)**

1-(3-Fluorphenyl)-N,N-dimethyl-4-(3-methyl-1H-indol-2-yl)cyclohexanamin (polareres Diastereoisomer) (120 mg, 0,34 mmol) wurde in heißem Isopropanol (50 ml) gelöst und mit ethanolischer Citronensäure-Lösung (66 mg, 0,34 mmol in 3 ml) versetzt. Die Mischung wurde 2 h bei Raumtemperatur gerührt. Der ausgefallene Feststoff wurde abgesaugt und Beispiel 262 in einer Ausbeute von 64 mg (42 %) mit einem Schmelzpunkt von 276-285 °C erhalten.
$^{1}$H NMR (300 MHz, RT, DMSO-D$_6$) δ ppm: 1.45 (psq, 2H), 1.81 (psd, 2H), 2.06-2.25 (m, 5H), 2.35 (s, br, 6H), 2.91 (pst, 1H), 2.99 (psd, 2H), 6.90 (dqu, 2H), 7.14 (psd, 1H), 7.27-7.43 (m, 2H), 7.46-7.69 (m, 3H), 10.38 (s, 1H).
$^{13}$C NMR (101 MHz, RT, OMSO-D$_6$) δ *ppm:* 8.3, 28.1, 30.9, 34.8, 37.3, 66.4 (br), 104.0, 110.5, 115.7 (d), 116.5 (d), 117.3, 117.9, 119.9, 125.5, 128.6, 130.6 (d), 135.0, 138.1, 162.6 (d), ein $C_{ipso}$ (n.b.).

**Beispiel 263: (±)-2-(4-(Dimethylamino)-4-(3-fluorphenyl)-cyclohex-1-enyl)-3-methyl-1H-indol**

3-Methylindol (Ind-10, 393 mg, 3 mmol) wurde zusammen mit Ket-13 (705 mg, 3 mmol) in Dichlormethan (25 ml) gelöst und mit Trifluormethansulfonsäure (0,4 ml, 4,5 mmol) versetzt. Der Ansatz wurde 24 h bei RT gerührt. - Zur Aufarbeitung wurde das Reaktionsgemisch mit 2N NaOH (20 ml) versetzt und 20 min bei RT gerührt. Nach Abtrennung der organischen Phase wurde die wässrige Phase mit Dichlormethan (2 x 20 ml) extrahiert. Die vereinigten organischen Extrakte wurden über $Na_2SO_4$ getrocknet und anschließend eingeengt. Das Rohprodukt wurde in einer Ausbeute von 1 g (100 %) als gelbes Öl erhalten. Das Öl wurde in heißen Methanol (10 ml) gelöst. Das Gemisch wurde auf RT gebracht und zur Vervollständigung der Kristallisation 17 h in den Kühlschrank gestellt. Durch Filtration wurde Beispiel 263 in einer Ausbeute von 480 mg (48 %) als hellgelber Feststoff mit einem Schmelzpunkt von 157-163 °C abgetrennt.
$^1$H NMR (300 MHz, RT, DMSO-D6) δ *ppm:* 1.74 (pst, 1H), 2.05-2.15 s über m, 8H), 2.23 (s, 3 H), 2.46 (m [unter DMSO-$D_5$], br, 1H), 2.67 (psq, 2 H), 6.17 (s, 1H), 6.88-7.10 (m, 3H), 7.18-7.42 (m, 5H), 10.56 (s, 1H).

**Beispiel 264: 2-(4-Benzyl-4-(dimethylamino)cyclohexyl)-3-methyl-1H-indol-5-ol, Citrat (1:1), polares Diastereomer**

**Beispiel 265: 2-(4-Benzyl-4-(dimethylamino)cyclohexyl)-3-methyl-1H-indol-5-ol, Citrat (1:1), unpolares Diastereomer**

(±)-*N*-[1-Benzyl-4-(5-methoxy-3-methyl-1*H*-indol-2-yl)cyclohex-3-enyl]-*N,N*-dimethylamin 5-Methoxyskatol (Ind-9, 806 mg, 5 mmol) wurde zusammen mit Ket-3 (1,15 g, 5 mmol) in Dichlormethan (40 ml) gelöst und mit Trifluormethansulfonsäure (0,65 ml, 7,5 mmol) versetzt. Der Ansatz wurde 24 h bei RT gerührt. - Zur Aufarbeitung wurde das Reaktionsgemisch mit 2N NaOH (20 ml) versetzt und 20 min bei RT gerührt. Nach Abtrennung der organischen Phase wurde die wässrige Phase mit Dichlormethan (3 x 20 ml) extrahiert. Die vereinigten organischen Extrakte wurden über $Na_2SO_4$ getrocknet und anschließend eingeengt. Das Rohprodukt wurde in einer Ausbeute von 2 g (98 %) als gelbes Öl erhalten und wurde ohne weitere Reinigung in der nächsten Reaktion eingesetzt.

**1-Benzyl-4-(5-methoxy-3-methyl-1H-indol-2-yl)-N,N-dimethylcyclohexanamin (unpolares und polares.Diastereomer)**

(±)-N-[1-Benzyl-4-(5-methoxy-3-methyl-1*H*-indol-2-yl)cyclohex-3-enyl]-*N*,*N*-dimethylamin (1,97 g, 5,26 mmol) wurde in HBr/Eisessig (33 % HBr, 30 ml) suspendiert. Anschließend wurde zu dem Ansatz bei RT Sn-Pulver (3,12 g, 26,3 mmol) innerhalb von 30 min portionsweise gegeben. Nach Beendigung der Zugabe wurde das Reaktionsgemisch weitere 48 h bei RT gerührt. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (100 ml) basisch gestellt. Das erhaltene wässrige Gemisch wurde mit Dichlormethan (4 × 20 ml) extrahiert. Die vereinigten organischen Phasen wurden mit Wasser (50 ml) gewaschen, über $Na_2SO_4$ getrocknet und anschließend eingeengt. Der erhaltene Rückstand (1,7 g) wurde in Ethylacetat (10 ml) gelöst. Das Gemisch wurde zur Kristallisation 2 h in den Kühlschrank gestellt. Durch Filtration wurde 1-Benzyl-4-(5-methoxy-3-methyl-1H-indol-2-yl)-N,N-dimethylcyclohexanamin (unpolareres Diastereoisomer) in einer Ausbeute von 500 mg (25 %) als beiger Feststoff abgetrennt. Die Mutterlauge wurde eingeengt und durch Säulenchromatographie (Kieselgel 60 G (150 g); EtOAc (1000 ml)] gereinigt. Das polarere Produktwurde in einer Ausbeute von 134 mg (7 %) als braunes Öl erhalten.

**2-(4-Benzyl-4-(dimethylamino)cyclohexyl)-3-methyl-1H-indol-5-ol, Citrat (1:1), polares Diastereomer (264)**

Zu einer Lösung von 1-Benzyl-4-(5-methoxy-3-methyl-1H-indol-2-yl)-N,N-dimethylcyclohexanamin (polareres Diastereoisomer, 130 mg, 0,345 mmol) in trockenem Dichlormethan (20 ml) wurde bei RT unter Rühren und Feuchtigkeitsausschluß eine 1M Lösung von $BBr_3$ in Dichlormethan (1,03 ml, 1,03 mmol) gegeben. Nach 10 min fiel ein Niederschlag aus. Der Ansatz wurde 24 h bei RT gerührt. - Zur Aufarbeitung wurde das Gemisch mit ges. $NaHCO_3$-Lösung (15 ml) versetzt und 48 h gerührt. Die organische Phase wurde abgetrennt und die wässrige Phase mit Dichlormethan (3 × 10 ml) extrahiert. Die vereinigten organischen Phasen wurden über $Na_2SO_4$ getrocknet und anschließend eingeengt. 2-(4-Benzyl-4-(dimethylamino)cyclohexyl)-3-methyl-1H-indol-5-ol (polares Diastereomer) wurde so in einer Ausbeute von 63 mg (50 %) erhalten.
2-(4-Benzyl-4-(dimethylamino)cyclohexyl)-3-methyl-1H-indol-5-ol (polares Diastereomer) (63 mg, 0,173 mmol) wurde in heißem Isopropanol (10 ml) gelöst und mit isopropanolischer Citronensäure-Lösung (34 mg, 0,173 mmol in 2 ml) versetzt. Die Mischung wurde 2 h bei Raumtemperatur gerührt. Der weiße Feststoff wurde abgesaugt. Beispiel 264 wurde in einer Ausbeute von 55 mg (57 %) mit einem Schmelzpunkt von 165-173 °C erhalten.
$^1$H NMR (300 MHz, RT, DMSO-$D_6$) δ *ppm:* 1.64-2.08 (m, 10H), 2.10 (s, 3H), 2.30-2.40 (m, 2 H), 2.54 (dd, 8H), 2.62-2.90

(m, 3 H), 3.24 (s, br, 2 H), 4.33 (s, br, 1H), 6.52 (dd, 1H). 6.68 (d, 1H), 7.05 (d, 1H), 7.30-7.45 (m, 5H), 8.47 (s, br, 1H), 10.40 (s, 1H), 10.50-12.00 (s, vbr, 2H).

**2-(4-Benzyl-4-(dimethylamino)cyclohexyl)-3-methyl-1H-indol-5-ol, Citrat (1:1), unpolares Diastereomer (265)**

Zu einer Lösung von 1-Benzyl-4-(5-methoxy-3-methyl-1H-indol-2-yl)-N,N-dimethylcyclohexanamin (unpolareres Diastereoisomer, 437 mg, 1,16 mmol) in trockenem Dichlormethan (50 ml) wurde bei RT unter Rühren und Feuchtigkeitsausschluß eine 1M Lösung von $BBr_3$ in Dichlormethan (3,48 ml, 3,48 mmol) gegeben. Nach 10 min fiel ein Niederschlag aus. Der Ansatz wurde 24 h bei RT gerührt. - Zur Aufarbeitung wurde das Gemisch mit ges. $NaHCO_3$-Lösung (20 ml) versetzt und 48 h gerührt. Die organische Phase wurde abgetrennt und die wässrige Phase mit Dichlormethan (3 x 10 ml) extrahiert. Die vereinigten organischen Phasen wurden über $Na_2SO_4$ getrocknet und anschließend eingeengt. 2-(4-Benzyl-4-(dimethylamino)cyclohexyl)-3-methyl-1H-indol-5-ol (unpolareres Diastereoisomer) wurde so in einer Ausbeute von 291 mg (69 %) erhalten. N-[1-Benzyl-4-(5-hydroxy-3-methyl-1H-indol-2-yl)cyclohexyl)-N,N-dimethylamin (unpolareres Diastereoisomer) (291 mg, 0,8 mmol) wurde in heißem Isopropanol (30 ml) gelöst und mit isopropanolischer Citronensäure-Lösung (154 mg, 0,8 mmol in 2 ml) versetzt. Die Mischung wurde 2 h bei Raumtemperatur gerührt. Der weiße Feststoff wurde abgesaugt. Beispiel 265 wurde in einer Ausbeute von 300 mg (67 %) mit einem Schmelzpunkt von 224-239 °C erhalten.
$^1$H NMR (300 MHz, RT, DMSO-$D_6$)$\delta$ *ppm:* 1.25 (t, 2H), 1.47 (d, 2H), 1.75-2.20 (m, br, 7H), 2.53 (s, br, 6H), 2.64 (dd, 4H), 2.71-2.89 (m, 3 H), 6.47 (dd, 1H), 6.63 (d, 1H), 7.05 (d, 1H), 7.19-7.38 (m, 5H), 8.82 (s, br, 1H), 10.05 (s, 1H), 10.20-11.80 (s, vbr, 4 H).
$^{13}$C NMR (101 MHz, RT, DMSO-$D_6$) $\delta$ *ppm:* 8.3. 25.9, 31.4. 34.7, 36.4, 36.9, 43.8, 59.7 (br), 71.7, 101.6, 102.9, 109.8, 110.8, 126.0, 127.9, 129.4, 129.5, 130.8, 137.7, 139.9, 150.0, 171.4, 176.3.

**Beispiel 266: 2-(4-Butyl-4-(pyrrolidin-1-yl)cyclohexyl)-3-(2-(pyridin-4-yl)ethyl)-1H-indol, Citrat (1:1), unpolares Diastereomer**

**Beispiel 267: 2-(4-Butyl-4-(pyrrolidin-1-yl)cyclohexyl)-3-(2-(pyridin-4-yl)ethyl)-1H-indol, Citrat (1:1), polares Diastereomer**

**2-[4-Butyl-4-(pyrrolidin-1-yl)cyclohexyl]-3-(2-pyridin-4-ylethyl)-1*H*-indol (unpolares und polares Diastereomer)**

Bsp. 253 (228 mg, 0,533 mmol) wurde in HBr/Eisessig (33 % HBr, 10 ml) gelöst. Anschließend wurde zu dem Ansatz bei RT Sn-Pulver (0,633 g, 5,33 mmol) innerhalb von 40 min portionsweise gegeben. Der Ansatz wurde noch 20 min gerührt. Anschließend wurde das Gemisch mit Dichlormethan (100 ml) verdünnt. Dann wurde 5 N NaOH-Lösung (60 ml) langsam unter Kühlung zugegeben, so daß die Temperatur 25 °C nicht überschritt. Das Gemisch wurde 30 min gerührt. Die Phasen wurden getrennt. Die wässrige Phase wurde mit Dichlormethan (3 x 20 ml) extrahiert. Die vereinigten organischen Phasen wurden mit $Na_2SO_4$ getrocknet, filtriert und die flüchtigen Bestandteile vollständig im Vakuum entfernt. Es blieb ein Öl (230 mg) zurück. Nach chromatographischer Trennung dieses Gemisches [Kieselgel 60 (20 g); Ethylacetat/Methanol 10 : 1 (550 ml), Ethylacetat/Methanol 1 : 1 (500 ml), Methanol (800 ml)] wurden 2-(4-Butyl-4-(pyrrolidin-1-yl)cyclohexyl)-3-(2-(pyridin-4-yl)ethyl)-1H-indol (unpolareres Diastereoisomer, 106 mg, weißer Feststoff, 46 %, Schmp.: 176-179 °C) und das polarere Diastereoisomer (46 mg. 20 %. Schmp.: 165-175 °C) als beigefarbener Feststoff gewonnen.

**2-(4-Butyl-4-(pyrrolidin-1-yl)cyclohexyl)-3-(2-(pyridin-4-yl)ethyl)-1H-indol, Citrat (1:1), unpolares Diastereomer (266)**

2-(4-Butyl-4-(pyrrolidin-1-yl)cyclohexyl)-3-(2-(pyridin-4-yl)ethyl)-1H-indol (unpolareres Diastereoisomer, 80 mg, 0,186 mmol) wurde in Dichlormethan (1 ml) gelöst und mit Citronensäure (40 mg, 0,208 mmol), gelöst in Ethylacetat (6 ml), versetzt. Während der Zugabe der Säure fiel ein Niederschlag aus. Anschließend wurde die Mischung 2 h bei 23 °C gerührt und dann filtriert. Der Niederschlag wurde mit Ethylacetat (2 x 0,5 ml) gewaschen. Beispiel 266 wurde als weißer Feststoff in einer Ausbeute von 94 % (109 mg) mit einem Schmelzpunkt von 132-139 °C erhalten.
$^1$H NMR (400 MHz, $CD_3OD$) $\delta$ ppm: 1.01 (t, J= 6.94, 3H), 1.14-1.49 (m, 6H), 1.49-1.64 (m, 2H), 1.62-1.85 (m, 4H), 1.85-2.23 (m, 6H), 2.42-2.63 (m, 1H), 2.71-2.93 (m, 4H), 2.93-3.01 (m, 2H), 3.01-3.13 (m, 2H), 3.16-3.62 (m, 4H), 6.91-7.07 (m, 2H), 7.12 (d, J = 5.05 Hz, 2H), 7.37 (s, 1H), 7.45 (d, J = 7.59 Hz, 1H), 8.30 (d, J = 4.83 Hz, 2H)
$^{13}$C NMR (101 MHz, $CD_3OD$) $\delta$ *ppm:* 14.3, 24.3, 25.6, 26.3, 27.2, 27.5, 32.4, 32.9, 35.0, 37.4, 45.0, 49.3, 67.3, 74.5, 109.1. 112.2, 118.7, 119.6, 121.9, 126.3, 129.2, 137.3, 140.2, 149.2, 154.8, 175.2, 179.4

**2-(4-Butyl-4-(pyrrolidin-1-yl)cyclohexyl)-3-(2-(pyridin-4-yl)ethyl)-1H-indol, Citrat (1:1), polares Diastereomer (267)**

2-(4-Butyl-4-(pyrrolidin-1-yl)cyclohexyl)-3-(2-(pyridin-4-yl)ethyl)-1H-indol (polareres Diastereoisomer, 44 mg, 0,102 mmol) wurde in Dichlormethan (0,5 ml) gelöst und mit Citronensäure (22 mg, 0,114 mmol), gelöst in Ethylacetat (4 ml), versetzt. Während der Zugabe der Säure fiel ein Niederschlag aus. Anschließend wurde die Mischung 2 h bei 23 °C gerührt, dann filtriert und der Niederschlag mit Ethylacetat (2 $\times$ 0,5 ml) gewaschen. Beispiel 267 wurde als beigefarbener Feststoff in einer Ausbeute von 93 % (59 mg) mit einem Schmelzpunkt von 105-112 °C erhalten .
$^1$H NMR (400 MHz, $CD_3OD$) $\delta$ *ppm:* 1.04 (t, J = 7.05 Hz, 3H), 1.28-1.56 (m, 6H), 1.62-1.81 (m, 4H), 1.81-1.93 (m, 2H), 1.93-2.02 (m, 2H), 2.02-2.15 (m, 4H), 2.46-2.66 (m, 1H), 2.80 (dd, J = 35.74, 15.44 Hz, 4H), 2.93-3.04 (m, 2H), 3.04-3.15 (m, 2H), 3.34-3.50 (m, 4H), 6.92-7.01 (m, 1H), 7.01-7.08 (m, 1H), 7.13 (d, J = 5.15 Hz, 2H), 7.26 (d, J = 7.94 Hz, 1H), 7.46 (d, J = 7.81 Hz, 1H), 8.26-8.42 (m, 2H)
$^{13}$C NMR (101 MHz, $CD_3OD$) $\delta$ *ppm:* 14.3, 24.1, 24.7, 25.6, 26.3, 29.3, 31.2, 32.2, 35.7, 37.3, 44.7, 48.4, 49.1, 67.6, 74.1, 109.2, 111.7, 118.8, 119.6, 121.8, 126.3, 129.2, 137.5, 139.8, 149.2, 154.8, 174.7, 179.0

**Beispiel 268: ($\pm$)-2-(4-(azetidin-1-yl)-4-phenylcyclohex-1-enyl)-3-methyl-1H-indol**

Indol (Ind-10, 525 mg, 4 mmol) wurde zusammen mit dem Keton (Ket-16, 917 mg, 4 mmol) in Dichlormethan (80 ml) gelöst und mit Trifluormethansulfonsäure (0,54 ml, 6 mmol) versetzt. Der Ansatz wurde 24 h bei RT gerührt. Zur Aufarbeitung wurde das Reaktionsgemisch mit 5N NaOH (40 ml) versetzt und 20 min bei RT gerührt. Nach Abtrennung der organischen Phase wurde die wäßrige Phase mit Dichlormethan (2 x 30 ml) extrahiert. Die vereinigten organischen Extrakte wurden über $Na_2SO_4$ getrocknet und anschließend eingeengt. Das Rohprodukt wurde durch Säulenchromatographie [Kieselgel 60 (50 g); Methanol (500 ml)] gereinigt. Es enthielt aber dennoch das Eduktketon (ca. 40 %). Es wurden 730 mg (53 %) Olefin-Keton-Gemisch erhalten. Dieses wurde ohne weitere Reinigung für die nächste Reaktion eingesetzt.

**2-(4-(Azetidin-1-yl)-4-phenylcyclohexyl)-3-methyl-1H-indol, Citrat (1:1) : Polareres Diastereomer**

Das soeben hergestellte Olefin (730 mg, 2,11 mmol) wurde in HBr/Eisessig (33 % HBr, 20 ml) gelöst. Anschließend wurde zu dem Ansatz bei RT Sn-Pulver (2,5 g, 21 mmol) innerhalb von 40 min portionsweise gegeben. Nach Beendigung der Zugabe wurde das Reaktionsgemisch 16 h bei RT gerührt. Dabei entstand eine klare Lösung. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH basisch gemacht. Das erhaltene wäßrige Gemisch wurde mit Dichlormethan (4 x 30 ml) extrahiert. Die vereinigten organischen Phasen wurden mit Wasser (50 ml) gewaschen, über $Na_2SO_4$ getrocknet und anschließend eingeengt. Das Rohprodukt (0,6 g) konnte durch Säulenchromatographie [Kieselgel 60 (50 g); Methanol (500 ml)] gereinigt werden. Das unpolarere Diastereomer wurde in einer Ausbeute von 220 mg (30 %) erhalten. Das polarere Diastereomer wurde in einer Ausbeute von 165 mg (22 %) erhalten.

Zur Herstellung des Citrates wurde das soeben hergestellte polarere Diastereomer (165 mg, 0,48 mmol) in heißem Methanol (100 ml) gelöst und mit Citronensäure (185 mg, 0,96 mmol) versetzt. Die klare Lösung wurde 16 h bei 4 °C stehen gelassen. Das ausgefallene weiße Citrat wurde abgesaugt und getrocknet. Das Citrat (Beispiel 268) wurde in einer Ausbeute von 180 mg (67 %) mit einem Schmelzpunkt von 150-155 °C erhalten.
$^1$*H NMR (300 MHz, DMSO-$d_6$)* $\delta$ *ppm:* 1.34-1.59 (m, 2H), 1.65-2.02 (m, 6H), 2.11 (s, 3H), 2.55 (dd, J = 25.02, 15.10 Hz, 2H), 2.59-2.74 (m, 2H), 2.86-3.06 (m, 1H), 3.40-3.62 (m, 4H), 6.80-7.00 (m, 2H), 7.10-7.21 (m, 1H), 7.08-7.19 (m, 1H), 7.41-7.51 (m. 1H), 7.51-7.59 (m, 2H), 7.59-7.71 (m, 2H), 10.34 (s, 1H)
$^{13}$C *NMR (101 MHz, DMSO-$d_6$)* $\delta$ *ppm:* 8.3, 15.4, 27.7, 29.5, 35.2, 44.3, 47.1, 62.4, 71.3, 103.8, 110.5, 117.2, 117.9, 119.8, 128.3, 128.6, 128.8, 134.1, 135.0, 138.5, 171.3, 176.8

**Beispiel 269: 2-(4-(Azetidin-1-yl)-4-phenylcyclohexyl)-3-methyl-1H-indol, Citrat (1:1): Unpolareres Diastereomer**

Zur Herstellung des Citrates wurde dasunter Beispiel 268 hergestellte unpolarere Diastereomer (220 mg, 0,63 mmol) in heißem Methanol (150 ml) gelöst und mit Citronensäure (243 mg, 1,26 mmol) versetzt. Die klare Lösung wurde 16 h bei 4 °C stehen gelassen. Das ausgefallene weiße Citrat wurde abgesaugt und getrocknet. Das Citrat (Beispiel 269) wurde in einer Ausbeute von 183 mg (54 %) mit einem Schmelzpunkt von 165-167 °C erhalten.
$^1$*H NMR (300 MHz, DMSO-$d_6$)* $\delta$ *ppm:* 1.51-1.74 (m, 4H), 1.74-1.92 (m, 2H), 2.05-2.34 (m, 6H), 2.34-2.49 (m, 1H), 2.65 (dd, J = 29.23, 15.25 Hz, 2H), 2.78-2.99 (m, 1H), 3.02-3.58 (m, 4H), 6.82-7.09 (m, 2H), 7.22-7.34 (m, 1H), 7.34-7.44 (m, 2H), 7.44-7.62 (m, 4H), 10.68 (s, 1H)
$^{13}$*C NMR (101 MHz, DMSO-$d_6$)* $\delta$ ppm: 8.4, 15.3, 27.1, 30.8, 30.8, 34.1, 43.3, 46.9, 58.5, 71.9, 103.7, 110.6, 117.3, 117.8, 119.8. 125.9, 127.2, 127,4 127.9, 128.7, 135.1, 139.5, 171.2, 175.5

**Beispiel 270: (±)-2-(4-(azetidin-1-yl)-4-phenylcyclohex-1-enyl)-3-(2-(pyridin-4-yl)ethyl)-1H-indol**

Iindol (Ind-14, 667 mg, 3 mmol) wurde zusammen mit dem Keton (Ket-16, 688 mg, 3 mmol) in abs. Dichlormethan (45 ml) gelöst und mit Trifluormethansulfonsäure (0,613 ml, 6,9 mmol) versetzt. Der Ansatz wurde 64 h bei RT gerührt, wobei ein braunes Öl ausfiel. Zur Aufarbeitung wurde die Reaktionslösung mit 1 N NaOH (30 ml) und Methanol (10 ml) versetzt. Das Gemisch wurde weitere 60 min gerührt. Nach Trennung der Phasen wurde die wäßrige Phase mit Dichlormethan (3 x 10 ml) extrahiert. Die vereinigten organischen Extrakte wurden über $Na_2SO_4$ getrocknet und anschließend eingeengt. Das erhaltene Rohprodukt (1,42 g) wurde säulenchromatographisch gereinigt [Kieselgel 60 (90 g); Ethylacetat/Methanol 5 : 1 (1800 ml), Ethylacetat/Methanol 2 : 1 (600 ml)]. Das Olefin wurde als beigefarbener Feststoff (315 mg, 24 %, Fp. 213-219 °C) erhalten.

**2-(4-(Azetidin-1-yl)-4-phenylcyclohexyl)-3-(2-(pyridin-4-yl)ethyl)-1H-indol Hydrochlorid: Polareres Diastereomer**

Das hergestellte Olefin (288 mg, 0,66 mmol) wurde in HBr/Eisessig (33 % HBr, 10 ml) suspendiert. Anschließend wurde zu dem Ansatz bei RT Sn-Pulver (797 mg, 6,6 mmol) innerhalb von 30 min portionsweise gegeben. Nach Beendigung der Zugabe wurde das Reaktionsgemisch 24 h bei RT gerührt. Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (75 ml) basisch gemacht und mit Dichlormethan (70 ml) versetzt. Dieses Gemisch wurde 2 h bei Raumtemperatur gerührt. Die organische Phase wurde abgetrennt und die wäßrige Phase mit Dichlormethan (3 x 30 ml) extrahiert. Die vereinigten organischen Phasen wurden über $Na_2SO_4$ getrocknet und anschließend eingeengt. Das erhaltene Rohprodukt (196 mg) konnte durch Säulenchromatographie [Kieselgel 60 (20 g); Methanol (150 ml)] gereinigt werden. Das unpolarere Diastereoisomer wurde in einer Ausbeute von 100 mg (35 %) erhalten. Das polarere Diastereoisomer wurde in einer Ausbeute von 36 mg (12 %) isoliert.
Das hergestellte polarere Diastereomer (36 mg, 0,08 mmol) wurde in Dichlormethan (50 mi) gelöst. Bei RT wurde dann $Me_3SiCl$ (20 µl, 0,16 mmol) zugetropft und 1 h gerührt. Da der ausgefallene Feststoff hygroskopisch war, wurde das Lösungsmittel am Rotationsverdampfer entfernt. Das polarere Hydrochlorid (Beispiel 270) (39 mg, Fp. 250-254 °C, Ausbeute 100 %) war ein weißer Feststoff.
$^1$*H NMR (400 MHz, DMSO-d$_6$) ℶ ppm:* 1.33-1.51 (m, 2H), 1.52-1.67 (m, 2H), 1.72-1.89 (m, 1H), 1.94-2.12 (m, 2H), 2.14-2.32 (m, 1H), 2.68-2.88 (m, 3H), 2.94-3.03 (m, 2H), 3.03-3.12 (m, 2H), 3.58-3.73 (m, 2H), 3.81-4.00 (m, 2H), 6.83-6.99 (m, 2H), 7.11-7.19 (d, 1H), ), 7.37-7.46 (d, 1H), 7.50-7.65 (m, 3H), 7.01-7.81 (m, 4H), 8.76 (d, J = 6.42 Hz, 2H), 10.44 (s, 1H), 11.53-11.70 (m, 1H)

**Beispiel 271: -(4-(Azetidin-1-yl)-4-phenylcyclohexyl)-3-(2-(pyridin-4-yl)ethyl)-1H-indol Hydrochlorid : Unpolareres Diastereomer**

Das unter Beispiel 270 hergestellte unpolarere Diastereomer (100 mg, 0,23 mmol) wurde in Dichlormethan (150 ml) gelöst. Bei RT wurde dann $Me_3SiCl$ (50 µl, 0,4 mmol) zugetropft und 1 h gerührt. Da der ausgefallene Feststoff hygroskopisch war, wurde das Lösungsmittel am Rotationsverdampfer entfernt. Das unpolarere Hydrochlorid (Beispiel 271) (108 mg, Fp. 242-245 °C, Ausbeute 100 %) war ein weißer Feststoff.
$^1$*H NMR (400 MHz, DMSO-d$_6$) ℶ ppm:* 6.89-6.99 (m, 1H), 6.99-7.08 (m, 1H), 7.21-7.29 (d, 1H), 7.44-7.53 (d, 2H), 7.53-7.66 (m, 2H), 7.71-7.88 (m, 4H), 8.65-8.74 (d, 2H), 10.64-10.86 (m, 1H), 11.29 (s, 1H)

**Beispiel 272: (±)-2-(4-butyl-4-(pyrrolidin-1-yl)cyclohexyl)-3-methyl-1H-indol**

Das Keton (Ket-17, 800 mg, 3,58 mmol) und Skatol (Ind-10, 470 mg, 3,58 mmol) wurden in abs. Dichlormethan (50 ml) gelöst, mit Trifluormethansulfonsäure (0,953 ml, 1,61 g, 10,74 mmol) versetzt und 18 h bei Raumtemperatur gerührt. Der Ansatz wurde mit Wasser (20 ml) und 1N Natronlauge (15 ml) versetzt und 1 h gerührt. Die Phasen wurden getrennt. Die wäßrige Phase wurde mit Dichlormethan (2 x 20 ml) extrahiert. Die organischen Phasen wurden vereinigt, mit Wasser (20 ml) gewaschen, mit Natriumsulfat getrocknet und eingeengt. Der Rückstand war ein braunes Öl (1.24 g ), das chromatographisch [Kieselgel 60 (100 g); Ethylacetat/Methanol 4 : 1 (500 ml), Methanol (600 ml)] aufgetrennt wurde. Das Olefin wurde als beigefarbener Feststoff in einer Ausbeute von 36 % (426 mg) gewonnen. Ein Schmelzpunkt konnte nicht bestimmt werden.

**2-(4-Butyl-4-(pyrrolidin-1-yl)cyclohexyl)-3-methyl-1H-indol, Citrat (1:1) : Polareres Diastereomer**

**Variante 1:** Das soeben hergestellte Olefin (211 mg, 0,627 mmol) wurde in Methanol (30 ml) gelöst und mit Palladium

auf Kohle (5-proz., 50 mg) versetzt. Die Reaktionsmischung wurde 3,5 h bei 3 bar hydriert. Der Katalysator wurde über Celite abgetrennt und das Filtrat eingeengt. Der Rückstand (200 mg, hellbraunes **Öl)** wurde chromatographisch aufgetrennt [Kieselgel 60 (20 g); Ethylacetat/Methanol 10 : 1 (200 ml), Ethylacetat/Methanol 4 : 1 (200 ml), Methanol (200 ml)]. Das unpolarere Diastereoisomer wurde in einer Ausbeute von 10 % (20 mg) das polarere Diastereoisomer in einer Ausbeute von 67 % (143 mg) gewonnen. Beide Diastereoisomere lagen nach der Chromatographie als farblose Salze vor.

**Variante 2:** Das soeben hergestellte Olefin (180 mg, 0,535 mmol) wurde mit HBr/Eisessig (33 % HBr, 10 ml) innerhalb von 1 h bei Raumtemperatur gelöst. Anschließend wurde zu dem Ansatz Zinn-Pulver (64 mg, 0,535 mmol) innerhalb von 10 min portionsweise gegeben. Nach Beendigung der Zugabe wurde das Reaktionsgemisch weitere 30 min gerührt. Unter Eiskühlung wurde der Ansatz mit Wasser (20 ml) versetzt und 15 min bei Raumtemperatur gerührt. Der ausgefallene beigefarbene Feststoff wurde abgesaugt, mit Wasser (4 × 5 ml) und mit Dichlormethan (2 x 5 ml) gewaschen. Das Hydrobromid des Diastereoisomerengemisches wurde in einer Ausbeute von 69 % (155 mg) erhalten. Das Salz wurde in einem Gemisch aus Dichlormethan (30 ml), Wasser (20 ml) und 1N Natronlauge (2 ml) aufgenommen und 30 min bei Raumtemperatur gerührt. Die Phasen wurden getrennt. Die wäßrige Phase wurde mit Dichlormethan (20 ml) extrahiert. Die organischen Phasen wurden vereinigt, mit Natriumsulfat getrocknet und eingeengt. Der Rückstand (107 mg, beigefarbenes **Öl)** wurde chromatographisch [Kieselgel 60 (20 g); Ethylacetat/Methanol 10 : 1 (200 ml), Ethylacetat/Methanol 4 : 1 (200 ml), Methanol (200 ml)] aufgetrennt. Das unpolarere Diastereoisomer wurde in einer Ausbeute von 31 % (56 mg) und das polarere Diastereoisomer in einer Ausbeute von 17 % (31 mg) erhalten. Beide Diastereoisomere lagen nach der Chromatographie als farblose Salze vor.

Das soeben hergestellte polarere Diastereomer (105 mg, 0,29 mmol) wurde in Ethanol (5 ml) gelöst und mit einer ethanolischen Lösung (2 ml) von Citronensäure (63 mg, 0,326 mmol) versetzt. Nach einer Reaktionszeit von 16 h bei Raumtemperatur wurde das Citrat (Beispiel 272) als farbloser Feststoff durch Filtration abgetrennt und mit Diethylether ( 2 × 2 ml) gewaschen. Das Salz wurde in einer Ausbeute von 51 % (80 mg) mit einem Schmelzpunkt von 239-240 °C gewonnen.

$^1$*H NMR (400 MHz, DMSO-$d_6$) δppm:* 0.98 (t, 3H), 1.25-1.48 (m, 4H), 1.60-1.90 (m, 14H), 2.17 (s, 3H), 2.50 (dd, 4H), 2.85 (t, 1H), 2.90-3.08 (m, 4H), 6.88-6.95 (m, 1H), 6.95-7.02 (m, 1H), 7.26 (d, 1H), 7.36 (d, 1H), 10.61 (s, 1H)

**Beispiel 273: 4-(dimethylamino)-4-phenyl-1-(5-(tetrahydro-2H-pyran-2-yloxy)pent-1-ynyl)cyclohexanol**

Unter Argon-Atmosphäre wurde 2-(4-Pentinyloxy)tetrahydro-2*H*-pyran (2394 mg, 13,81 mmol; 97 %) in absolutem Tetrahydrofuran (50 ml) gelöst und die Lösung auf -78 **°C** gekühlt. Anschließend wurde langsam Lithiumdiisopropylamid-Lösung (17,95 mmol, 9,9 ml, 1,8M) zugetropft. Nach beendeter Zugabe wurde die nun rötliche Lösung zwischenzeitlich auf Raumtemperatur erwärmt (15 min), wobei die Lösung heller wurde. Das Keton (Ket-10, 3000 mg, 13,81 mmol) wurde in Tetrahydrofuran (15 ml) gelöst und bei -78°C langsam zur *in situ* hergestellten Organolithiumverbindung getropft. Nach 10 min wurde die Kühlung entfernt und die hellbraune Lösung 17 h gerührt. Zur Aufarbeitung des Reaktionsgemisches wurde unter Eiskühlung gesättigte Ammoniumchlorid-Lösung (50 ml) zugegeben. Dabei entstand ein weißer Feststoff, der mit etwas Wasser (10 ml) in Lösung gebracht wurde. Die Phasen wurden getrennt. Die wäßrige Phase wurde mit Diethylether (3 x 50 ml) extrahiert. Die vereinigten organischen Phasen wurden über Natriumsulfat getrocknet und anschließend die flüchtigen Bestandteile im Vakuum vollständig entfernt. Es blieb ein rotbraunes Öl zurück. Dieses wurde chromatographisch gereinigt [Kieselgel 60 (200 g), Cyclohexan/Ethylacetat 5 : 1 (100 ml), Chloroform/Ethanol 19 : 1 (1000ml)]. Der gewünschte Alkohol konnte in einer Ausbeute von 50 % (2643 mg, 6,86 mmol) als rotbraune Paste erhalten werden.

**4-(dimethylamino)-4-phenyl-1-(3-(3-(tetrahydro-2H-pyran-2-yloxy)propyl)-1H-pyrrolo[2,3-b]pyridin-2-yl)cyclohexanol**

4-(Dimethylamino)-4-phenyl-1-(5-(tetrahydro-2H-pyran-2-yloxy)pent-1-ynyl)cyclohexanol (700 mg, 1,82 mmol), 2-Amino-3-iodpyridin (363 mg, 1,65 mmol), Lithiumchlorid (74 mg, 1,73 mmol) und Natriumcarbonat (525 mg, 4,95 mmol) wurde unter Argon-Atmosphäre in Dimethylformamid (20 ml) gelöst. Anschließend wurde die Lösung 10 min lang mit Argon durchströmt und dann der Katalysator ([Pd(dppf)$Cl_2$ × $CH_2Cl_2$). 135 mg, 0,17 mmol) zugegeben. Das Reaktionsgemisch wurde 3 h bei 105°C (Ölbadtemperatur) erhitzt. Ein DC zeigte, daß die Ausgangstoffe vollständig verbraucht waren. Zu der auf Raumtemperatur abgekühlten schwarzen Reaktionsmischung wurden nacheinander Wasser (50 ml, 10 min rühren), Dichlormethan (50 ml) und gesättigte Natriumchlorid-Lösung (zur Phasentrennung; 80 ml) gegeben. Die Phasen wurden getrennt und die wäßrige Phase mit Dichlormethan (3 x 20 ml) extrahiert. Die vereinigten organischen Phasen wurden mit Wasser (20 ml) und mit gesättigter Natriumchlorid-Lösung (3 × 20 ml) gewaschen, über Natriumsulfat getrocknet, filtriert und anschließend die flüchtigen Bestandteile vollständig im Vakuum entfernt. Der

Rückstand wurde chromatographisch getrennt [Kieselgel 60 (200 g): Chloroform/Ethanol 19 : 1 (500 ml), 9 : 1 (500 ml), 4 : 1 (500 ml), Methanol (1000 ml)]. Ein Diastereoisomer des Alkohols (4-(Dimethylamino-4-phenyl-1-(3-(3-(tetrahydro-2H-pyran-2-yloxy)propyl)-1H-pyrrolo[2,3-b]pyridin-2-yl)cyclohexanol) (Smp.: 238 °C) konnte in einer Ausbeute von 28 % (221 mg, 0,46 mmol) als weißes Pulver erhalten werden.

**3-(2-(4-(Dimethylamino)-4-phenylcyclohex-1-enyl)-1H-pyrrolo[2,3-b]pyridin-3-yl)propan-1-ol**

Der soeben hergestellte Alkohol (662 mg, 1,39 mmol) wurde in Methansulfonsäure (45 ml) gelöst und das $P_4O_{10}$ (ca. 2 g) hinzugegeben. Die rotgefärbte Reaktionsmischung wurde 3 h bei 73°C (Ölbadtemperatur) gerührt. Die Farbe der Reaktionsmischung hatte sich nicht geändert. Zu der Reaktionslösung wurde unter Eiskühlung 5N Natriumhydroxid-Lösung (130 ml), Wasser (70 ml) und Dichlormethan (40 ml) gegeben und 10 min gerührt. Anschließend wurden die Phasen getrennt. Die wässrige Phase wurde mit Dichlormethan (2 x 30 ml) extrahiert. Die vereinigten organischen Phasen wurden über Natriumsulfat getrocknet, filtriert und anschließend die flüchtigen Bestandteile vollständig im Vakuum entfernt. Es verblieb ein oranger Schaum (478 mg). Der Rückstand wurde mit Diethylether (10 ml) versetzt, für 5 min ins Ultraschallbad gestellt, die etherische Phase abgehebert und der verbliebene Rückstand im Vakuum getrocknet. Das gewünschte Olefin konnte in einer Ausbeute von 74 % (386 mg, 1,03 mmol) als gelbes Pulver erhalten werden.

**3-(2-(4-(dimethylamino)-4-phenylcyclohexyl)-1H-pyrrolo[2,3-b]pyridin-3-yl)propan-1-ol, Citrat (1:1): Eines von zwei möglichen Diastereoisomern**

Zu einer Suspension des soeben erhaltenen Olefins (341 mg, 0,91 mmol) in HBr/Eisessig (20 ml, 35 %) wurde innerhalb von 60 min das Zinn (1430 mg, 12,05 mmol) gegeben (Gasentwicklung). Es wurde 20 h bei Raumtemperatur gerührt. Die Reaktionsmischung färbte sich hellbraun. Die Reaktionsmischung wurde im Vakuum zur Trockne eingedampft und der hellbraune Rückstand mit 5N Natriumhydroxid-Lösung (20 ml) und Dichlormethan (20 ml) versetzt. Die Mischung wurde filtriert, der erhaltene Feststoff in Methanol (5 ml) gelöst und mit der organischen Phase vereinigt. Die wäßrige Phase wurde abgetrennt und mit Dichlormethan (2 × 20 ml) extrahiert. Die organischen Phasen wurden vereinigt, über Natriumsulfat getrocknet und anschließend auf ca. 5 ml im Vakuum eingeengt. Aus der Lösung fiel über Nacht ein kristalliner, gelber Feststoff aus. Der Feststoff wurde abgefrittet. Das gelbe Filtrat wurde vollständig eingedampft (706 mg) und chromatographisch getrennt [Kieselgel 60 (200g); Chloroform/Ethanol 19: 1 (500 ml), 9 : 1 (1000 ml), 4 : 1 (1000 ml), Methanol (500 ml)]. Ein Diastereoisomer des gewünschten Alkohols konnte in einer Ausbeute von 24 % (83 mg, 0,22 mmol) als gelbes Pulver erhalten werden.
Der soeben hergestellte Alkohol (76 mg, 0,20 mmol) wurde in Ethanol (5 ml) vorgelegt und die trübe, schwach gelbe Lösung zur Siedehitze erwärmt. Die Citronensäure (39 mg, 0,20 mmol) wurde hinzugefügt und die Reaktionslösung in der Siedehitze 30 min gerührt. Die Lösung wurde im Kühlschrank auf 5°C abgekühlt und 16 h stehen gelassen. Es fiel ein farbloser Niederschlag aus. Die überstehende Lösung wurde abgehebert und der Rückstand im Vakuum getrocknet. Es konnten 67 mg (0,12 mmol, 59 %) der Zielverbindung (Smp.: 220-223 °C) isoliert werden.
$^1$H NMR (400 MHz, RT, DMSO-$D_6$) *δppm:* 1.64 (pst, br, 2H), 1.80 (psd, br, 2H), 1.86-2.20 (m, 4H), 2.46 (s, br, 6H), 2.63 (dd, 4H), 2.67-2.78 (m, br, 2H), 2.88-3.13 (m, br, 2H), 2.46 (t, 2H), 4.16 (t, 1H), 6.93 (dd, 1H), 7.51-7.63 (m, 3H), 7.71 (psd, 2H), 7.79 (dd, 1H), 8.04 (dd, 1H), 11.22 (s, 1H), zusätzlich von 1.5-4.5 (sehr br).
$^{13}$C NMR (101 MHz, RT, DMSO-$D_6$) *δppm:* 20.4, 28.2, 30.8, 33.3, 35.0, 37.4, 43.4 44.9, 68.1, 71.9, 106.6, 114.7, 120.0, 125.3, 128.9, 129.3, 129.5, 139.6, 141.4, 148.1, 171.4, 175.7.

**Beispiel 274: 1-(2-(4-(dimethylamino)-4-phenylcyclohexyl)-3-methyl-1H-indol-1-yl)-3-(methylamino)propan-2-ol, Citrat (1:1) : Polareres Diastereomer**

Zu einer Suspension des polareren Epoxids (220 mg, 0,57 mmol) in Ethanol (15ml) wurde Methylamin (49 mg, 1,59 mmol; 2M in Tetrahydrofuran, 0,79 ml) bei Raumtemperatur gegeben. Da bei Raumtemperatur keine Reaktion einsetzte, wurde die Reaktionsmischung 5 h bei 59 °C (Ölbadtemperatur) gerührt. Anschließend wurde die Reaktionsmischung im Vakuum af ca. 5 ml eingeengt. Es fiel ein farbloser Feststoff aus. Es wurde Diethylether (3 ml) hinzugegeben. Die Mischung wurde 3 d bei 0 °C gelagert. Die überstehende Lösung wurde abdekantiert. Das polare Produkt konnte quantitativ (234 mg, 0,57 mmol) gewonnen werden.

Der soeben hergestellte polare Aminoalkohol (298 mg, 0,71 mmol) wurde in siedendem Ethanol (15 ml) vorgelegt. Die Citronensäure (150 mg, 0,78 mmol) wurde dann hinzugegeben und die klare Lösung in der Siedehitze 3 h gerührt. Anschließend wurde die Reaktionsmischung auf Raumtemperatur gekühlt und 24 h bei dieser Temperatur stehen gelassen. Es fiel ein farbloser mikrokristalliner Niederschlag an. Der Niederschlag wurde abfiltriert und mit Ethanol gewaschen. Das gewünschte Produkt (Beispiel 274) konnte als farbloser Feststoff (Smp.: 173-180 °C) in einer Ausbeute von 60 % (260 mg, 0,43 mmol) erhalten werden.
$^1$H NMR (400 MHz, RT, DMSO-$D_6$) δ ppm: 1.50-2.00 (m, 9H), 2.25 (s, 6H), 2.46 (s, br, 3H) 2.53 (dd, 4H), 2.80-3.10 (m,

4H), 3.90-4.25 (m, 3H), 6.89 (t, 1H), 6.99 (t, 1H), 7.23 (d, 1H), 7.31 (d, 1H), 7.41 (pst, 1H), 7.50 (pst, 2H), 7.55 (pst, 2H), 8.00-11.00 (s, br, 6H).
$^{13}C$ NMR (101 MHz, RT, DMSO-$D_6$) δ *ppm:* 8.7, 26.8, 27.1, 31.4, 31.5, 32.9, 33.7, 37.6, 44.4, 46.4, 51.3, 64.9, 66.1, 71.5, 104.5, 109.5, 117.1, 118.4, 120.3, 127.9, 128.4, 128.8, 129.1, 133.4, 135-3, 139.5, 171.6, 177.1.

**Beispiel 275: ±)-1-Benzyl-3-(2-(2-(4-(dimethylamino)-4-phenylcyclohex-1-enyl)-1H-indol-3-yl)ethyl-harnstoff**

Indol (Ind-100, 820 mg, 2.8 mmol) und Keton (Ket-10, 608 mg, 2.8 mmol) wurden in abs. Dichlormethan (30 ml) gelöst und schnell mit Trifluormethansulfonsäure (1.00 ml, 11.2 mmol) versetzt. Der Ansatz wurde 3 d bei RT gerührt, dabei setzte sich ein dunkelbraunes Öl ab. Nach Zugabe von 1 N NaOH (50 ml) und $CH_2Cl_2$ (20 ml) wurde 1 h nachgerührt bis das Öl gelöst war. Die Phasen wurden getrennt, die wässrige Phase wurde dreimal mit $CH_2Cl_2$ extrahiert, die vereinigten organischen Phasen wurden mit Wasser gewaschen, getrocknet ($Na_2SO_4$) und die Lösung i. Vak. eingeengt. Der verbliebene Rückstand wurde durch Flash-Chromatographie mit 50 g Kieselgel und $CHCl_3$/MeOH (9:1→1:1) gereinigt. *Ausbeute:* 647 mg (47 %)

**1-Benzyl-3-(2-(2-(4-dimethylamino)-4-phenylcyclohexyl)-1H-indol-3-yl)ethyl)-harnstoff, Citrat (1:1): Unpolareres Diastereomer**

Zu einer Lösung des soeben hergestellten Olefins (871 mg, 1.77 mmol) in HBr/Eisessig (35 ml) wurde innerhalb von 20 min Zinn (2.27 g) gegeben und 4 h bei RT gerührt. Der Ansatz wurde mit Ethanol versetzt, das Reaktionsgemisch bis zur Trockene eingeengt und der Rückstand in 5N NaOH (100 ml) und Dichlormethan (150 ml) gelöst. Die Phasen wurden getrennt, die wässrige Phase dreimal mit $CH_2Cl_2$extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit 50 g Kieselgel und $CHCl_3$/MeOH (20:1→9:1→4:1→1:1) getrennt. Beide Diastereomere lagen laut NMR als Salz vor und wurden daher jeweils mit 1 N NaOH (10 ml) versetzt und 7 x mit $CH_2Cl_2$ extrahiert, die organischen Phasen wurden jeweils getrocknet ($Na_2SO_4$) und i. Vak. eingeengt.

*Ausbeute:* 126 mg (14 %), unpolares Diastereomer
189 mg (22 %), polares Diastereomer

Citrat Unpolar:

Das soeben hergestellte unpolarere Diastereomer (108 mg, 0.22 mmol) wurde in Ethanol (10 ml) heiß gelöst und mit einer Lösung von Citronensäure (42 mg, 0.22 mmol) in Ethanol (0.5 ml) versetzt. Da kaum Niederschlag ausfiel wurde die Lösung i. Vak. auf die Hälfte eingeengt und über Nacht im Kühlschrank stehen gelassen. Der ausgefallene Niederschlag wurde abgesaugt und mit Ether nachgewaschen. *Ausbeute:* 85 mg (57 %)
*$^1H$-NMR (DMSO-$d_6$):* 1.64-1.71 (4H, m); 2.20 (8H, s); 2.59-2.75 (4H, m); 2.74-2.86 (6H, m); 2.91-2.99 (1H, m); 3.17-3.23 (2H, m); 4.19 (2H, d); 5.94 (1H, t); 6.32 (1H, t); 6.90-7.01 (2H, m); 7.17-7.49 (12H, m); 10.62 (1H, s); 11.10 (4H, bs).

**Beispiel 276: 1-Benzyl-3-(2-(2-(4-(dimethylamino)-4-phenylcyclohexyl)-1H-indol-3-yl)ethyl)-harnstoff, Citrat (1: 1) : Polareres Diastereomer**

Citrat Polar:

Das unter Beispiel 275 hergestellte polarere Dieastereomer (175 mg, 0.35 mmol) wurde in Ethanol (5 ml) heiß gelöst und mit einer Lösung von Citronensäure (68 mg, 0.35 mmol) in Ethanol (0.5 ml) versetzt. Da kaum Niederschlag ausfiel, wurde die Lösung i. Vak. auf die Hälfte eingeengt und über Nacht im Kühlschrank stehen gelassen. Der Niederschlag wurde abgesaugt und mit Ether nachgewaschen. *Ausbeute:* 108 mg (44 %)
*$^1H$-NMR (DMSO-$d_6$):* 1.44-1.54 (2H, m); 1.79-1.92 (4H, m); 2.30 (6H, s); 2.49-2.61 (4H, m); 2.76 (2H, s); 2.93-2.96 (3H, m); 3.17-3.18 (2H, m); 4.24 (2H, d); 5.93 (1H, t); 6.34 (1H, t); 6.87-6.94 (2H, m); 7.14-7.34 (6H, m); 7.41-7.61 (6H, m); 10.37 (1H, s); 11.10 (4H, bs).

**Beispiel 277: (±)-1-(2-(2-(4-(dimethylamino)-4-phenylcyclohex-1-enyl)-1H-indol-3-yl)ethyl)-3-phenyl-harnstoff**

Indol (Ind-101, 600 mg, 2.15 mmol) und Keton (Ket-10, 467 mg, 2.15 mmol) wurden in abs. Dichlormethan (30 ml) gelöst und schnell mit Trifluormethansulfonsäure (0.76 ml, 8.6 mmol) versetzt. Der Ansatz wurde 3 d bei RT gerührt, dabei setzte sich ein dunkelbraunes Öl ab. Nach Zugabe von 1 N NaOH (40 ml) und $CH_2Cl_2$ (30 ml) wurde noch 2 h

nachgerührt bis das **Öl** gelöst war. Die Phasen wurden getrennt, die wässrige Phase wurde mit $CH_2Cl_2$ (3x 40 ml) extrahiert, die vereinigten organischen Phasen wurden mit Wasser (10 ml) gewaschen, getrocknet ($Na_2SO_4$) und die Lösung i. Vak. eingeengt. Der verbliebene Rückstand wurde durch Flash-Chromatographie mit 100 g Kieselgel und $CHCl_3$/MeOH (9:1) gereinigt. *Ausbeute:* 927 mg (90 %)

**1-(2-(2-(4-(Dimethylamino)-4-phenylcyclohexyl)-1H-indol-3-yl)ethyl)-3-phenyl-harnstoff, Citrat (1:1) : Unpolareres Diastereomer**

Zu einer Lösung des soeben hergestellten Olefins (638 mg, 1.33 mmol) in HBr/Eisessig (25 ml) wurde innerhalb von 20 min Zinn (1.50 g) gegeben und 4 h bei RT gerührt. Der Ansatz wurde mit Ethanol versetzt, das Reaktionsgemisch bis zur Trockene eingeengt und der Rückstand in 5N NaOH (75 ml) und Chloroform (175 ml) gelöst. Nach 2 h wurden die Phasen getrennt, die wässrige Phase mit $CHCl_3$ (3x 100 ml) extrahiert, die vereinigten organischen Phasen mit Wasser (30 ml) gewaschen, über $Na_2SO_4$ getrocknet und i. Vak. eingeengt. Der verbliebene Rückstand wurde durch Flash-Chromatographie mit 50 g Kieselgel und $CHCl_3$/MeOH (20:1→9:1→4:1→1:1) getrennt. Beide Diastereomere lagen laut NMR als Salz vor. Das unpolare Diastereomer wurde mit 1 N NaOH (10 ml) versetzt und 7x mit $CH_2Cl_2$ extrahiert. Die organische Phase wurde getrocknet ($Na_2SO_4$,) und i. Vak. eingeengt. Es wurden 28 mg eines nichtkristallinen Feststoffes erhalten, der nicht weiter untersucht wurde. Die wässrige Phase wurde mit Essigester (40 ml) versetzt, aber die Phasen trennten sich anschließend nicht wieder. Beim Einengen i. Vak. fiel das unpolare Diastereomer als weißer Feststoff aus, dieser wurde abgesaugt, mit Wasser nachgewaschen und i. Vak. getrocknet. Das polare Diastereomer wurde mit 1 N NaOH (10 ml) versetzt und mit $CHCl_3$ (4x 20 ml) extrahiert. Die organische Phase wurde über $Na_2SO_4$ getrocknet und i. Vak. eingeengt.

*Ausbeute:* 62 mg (10 %), unpolares Diastereomer
261 mg (41 %), polares Diastereomer

Citrat Unpolar:

Das soeben erhaltene unpolarere Diastereomer (61 mg, 0.13 mmol) wurde in einer Mischung aus Ethanol (10 ml) und Methanol (2 ml) heiß gelöst, mit Citronensäure (24 mg, 0.13 mmol) versetzt und nochmals erhitzt. Die Löslichkeit der Substanz wurde durch Zugabe der Citronensäure sichtbar verbessert, deshalb wurde i. Vak. Lösungsmittel entfernt, bis die Lösung sich trübte. Der nach anschließendem Stehen im Kühlschrank ausgefallene Niederschlag wurde abgesaugt und mit Ether nachgewaschen. *Ausbeute:* 51 mg (60 %) $^1$*H-NMR (DMSO-$d_6$):* 1.44-1.53 (2H, m); 1.64-1.68 (2H, m); 2.09 (8H, s); 2.56-2.71 (4H, m); 2.71-2.91 (5H, m); 3.26-3.32 (2H, m); 6.08 (1H, t); 6.84-7.01 (3H, m); 7.18 (2H, t); 7.28-7.37 (8H, m); 7.49 (1H, d); 8.37 (1H, s); 10.64 (1H, s); 11.10 (4H, bs).

**Beispiel 278: 1-(2-(2-(4-(Dimethylamino)-4-phenylcyclohexyl)-1H-indol-3-yl)ethyl)-3-phenyl-harnstoff, Citrat (1: 1) : Polareres Diastereomer**

Citrat Polar:

Das unter Beispiel **277** hergestellte polarere Diastereomer (236 mg, 0.49 mmol) wurde in Ethanol (5 ml) heiß gelöst und mit Citronensäure (94 mg, 0.49 mmol) versetzt. Die Lösung wurde nochmals kurz erhitzt. Da kaum Niederschlag ausfiel, wurde die Lösung i. Vak. auf die Hälfte eingeengt und über Nacht in den Kühlschrank gestellt. Der ausgefallene Niederschlag wurde abgesaugt und mit Ether nachgewaschen. *Ausbeute:* 71 mg (21 %)
$^1$*H-NMR (DMSO-$d_6$):* 1.44-1.53 (2H, m); 1.79-1.87 (4H, m); 2.25 (6H, s); 2.49-2.62 (4H, m); 2.78-2.96 (5H, m); 3.23-3.28 (2H, m); 6.11 (1H, t); 6.87-6.95 (3H, m); 7.15-7.25 (3H, m); 7.41-7.59 (8H, m); 8.42 (1H, s); 10.39 (1H, s); 11.10 (4H, bs).

**Beispiel 279: (±)-1-Cyclopentyl-3-(2-(2-(4-(dimethylamino)-4-phenylcyclohex-1-enyl)-1H-indol-3-yl)ethyl)-harnstoff**

Indol (Ind-102, 1.21 g, 4.5 mmol) und Keton (Ket-10, 968 mg, 4.5 mmol) wurden in abs. Dichlormethan (50 ml) gelöst und schnell mit Trifluormethansulfonsäure (1.58 ml, 17.8 mmol) versetzt. Der Ansatz wurde 3 d bei RT gerührt, dabei setzte sich ein dunkelbraunes Öl ab. Nach Zugabe von 1N NaOH (80 ml) und $CH_2Cl_2$(120 ml) wurde 3 h nachgerührt bis das Öl gelöst war. Die Phasen wurden getrennt, die wässrige Phase wurde mit $CH_2Cl_2$ (3x 80 ml) extrahiert, die vereinigten organischen Phasen wurden mit Wasser gewaschen, getrocknet ($Na_2SO_4$) und die Lösung i. Vak. eingeengt. Der verbliebene Rückstand wurde durch Flash-Chromatographie mit 100 g Kieselgel und $CHCl_3$/MeOH (9:1→1:1) gereinigt.
*Ausbeute:* 1.39 mg (66 %)

**1-Cyclopentyl-3-(2-(2-(4-(dimethylamino)-4-phenylcyclohexyl)-1H-indol-3-yl)ethyl)-harnstoff, Citrat (1:1) : Unpolareres Diastereomer**

Zu einer Lösung des soeben hergestellten Olefins (1.37 g, 2.91 mmol) in HBr/Eisessig (50 ml) wurde innerhalb von 20 min Zinn (3.28 g) gegeben und 4 h bei RT gerührt. Der Ansatz wurde mit Ethanol versetzt, das Reaktionsgemisch bis zur Trockene eingeengt und der Rückstand in 5N NaOH (80 ml) und Dichlormethan (170 ml) gelöst. Die Phasen wurden getrennt, die wässrige Phase mit $CH_2Cl_2$ (3× 80 ml) extrahiert, die vereinigten organischen Phasen mit Wasser (20 ml) gewaschen, über $Na_2SO_4$ getrocknet und i. Vak. eingeengt. Der verbliebene Rückstand wurde durch Flash-Chromatographie mit 100 g Kieselgel und $CHCl_3$/MeOH (20:1→9:1→4:1→1:1) getrennt. Beide Diastereomere lagen laut NMR als Salz vor und wurden daher jeweils mit 1 N NaOH (10 ml) versetzt und 7 x mit $CHCl_3$ extrahiert, die organischen Phasen wurden jeweils getrocknet ($Na_2SO_4$) und i. Vak. eingeengt.
*Ausbeute:* 125 mg (9 %), unpolares Diastereomer 293 mg (21 %), polares Diastereomer

Citrat Unpolar:

Das soeben erhaltene unpolarere Diastereomer (109 mg, 0.23 mmol) wurde in Ethanol (10 ml) und Methanol (5 ml) heiß gelöst, mit Citronensäure (44 mg, 0.23 mmol) versetzt und kurz zum Sieden erhitzt. Da kaum Niederschlag ausfiel, wurde die Lösung i. Vak. auf die Hälfte eingeengt und im Kühlschrank stehen gelassen. Der ausgefallene Niederschlag wurde abgesaugt und mit Ether nachgewaschen. *Ausbeute:* 95 mg (62 %)
*$^1$H-NMR (DMSO-$d_6$):* 1.20-1.28 (2H, m); 1.43-1.57 (4H, m); 2.14-2.17 (2H, m); 2.23 (6H, s); 2.60-2.74 (4H, m); 2.74-2.95 (5H, m); 3.13-3.18 (2H, m); 3.82 (1H, qu); 5.66 (1H, t); 5.77 (1H, d); 6.90-7.00 (2H, m); 7.31-7.50 (7H, m); 10.63 (1H, s); 11.10 (4H, bs).

**Beispiel 280: 1-Cyclopentyl-3-(2-(2-(4-(dimethylamino)-4-phenylcyclohexyl)-1H-indol-3-yl)ethyl)-hamstoff, Citrat (1 :1) : Polareres Diastereomer**

Citrat Polar:

Das unter Beispiel 279 erhaltene polarere Diastereomer (274 mg, 0.58 mmol) wurde in Ethanol (5 ml) und Methanol (2 ml) heiß gelöst, mit Citronensäure (111 mg, 0.58 mmol) versetzt und das Gemisch kurz zum Sieden erhitzt. Da kaum Niederschlag ausfiel, wurde die Lösung i. Vak. auf die Hälfte eingeengt und dann im Kühlschrank stehen gelassen. Der ausgefallene Niederschlag wurde abgesaugt und mit Ether nachgewaschen. *Ausbeute:* 230 mg (60 %)
*$^1$H-NMR* (DMSO-$d_6$): 1.25-1.32 (2H, m); 1.44-1.61 (6H, m); 1.77-1.83 (4H, m); 1.91-1.97 (2H, m); 2.36 (6H, s); 2.52-2.64 (4H, m); 2.72 (2H, t); 2.90-3.00 (3H, m); 3.11-3.16 (2H, m); 3.87-3.92 (1H, m); 5.67 (1H, t); 5.80 (1H, d); 6.86-6.94 (2H, m); 7.15 (1H, d); 7.41 (1H, d); 7.54-7.66 (5H, m); 10.36 (1H, s); 11.10 (4H, bs).

**Beispiel 281: N-(2-(2-(4-(dimethylamino)-4-phenylcyclohex-1-enyl)-1H-indol-3-yl)ethyl)cyclopentanesulfonamid**

Indol (Ind-103, 736 mg, 2.5 mmol) und Keton **(Ket-10,** 547 mg, 2.5 mmol) wurden in abs. Dichlormethan (30 ml) gelöst und schnell mit Trifluormethansulfonsäure (0.89 ml, 10.1 mmol) versetzt. Der Ansatz wurde 3 d bei RT gerührt, dabei setzte sich ein dunkelbraunes Öl ab. Nach Zugabe von 1 N NaOH (40 ml) und $CH_2Cl_2$ (30 ml) wurde 2 h gerührt bis das Öl gelöst war. Die Phasen wurden getrennt, die wässrige Phase wurde mit $CH_2Cl_2$ (3x 40 ml) extrahiert, die vereinigten organischen Phasen wurden mit Wasser (10 ml) gewaschen, getrocknet ($Na_2SO_4$) und die Lösung i. Vak. eingeengt. Der verbliebene Rückstand wurde durch Flash-Chromatographie mit 100 g Kieselgel und $CHCl_3$/MeOH (9: 1) gereinigt.
*Ausbeute:* 1.04 g (84 %)

**N-(2-(2-(4-(dimethylamino)-4-phenylcyclohexyl)-1H-indol-3-yl)ethyl)cyclopentanesulfonamide, Citrat (1:1) : Unpolareres Diastereomer**

Zu einer Lösung des soeben isolierten Olefins (464 mg, 0.94 mmol) in HBr/Eisessig (20 ml) wurde innerhalb von 20 min Zinn (1.07 g) gegeben und 4 h bei RT gerührt. Der Ansatz wurde mit Ethanol versetzt, das Reaktionsgemisch bis zur Trockene eingeengt und der Rückstand in 5N NaOH (50 ml) und Dichlormethan (150 ml) gelöst. Die Phasen wurden getrennt, die wässrige Phase mit $CH_2Cl_2$ (3x 50 ml) extrahiert, die vereinigten organischen Phasen mit Wasser gewaschen, über $Na_2SO_4$ getrocknet und i. Vak. eingeengt. Der verbliebene Rückstand wurde durch Flash-Chromatographie mit 25 g Kieselgel und $CHCl_3$/MeOH (100:1→11:1) getrennt. Beide Diastereomere lagen laut NMR als Salz vor und wurden daher jeweils mit 1 N NaOH (10 ml) versetzt und 7 x mit $CHCl_3$ extrahiert, die organischen Phasen wurden jeweils getrocknet ($Na_2SO_4$) und i. Vak. eingeengt.

*Ausbeute:* 177 mg (38 %), unpolares Diastereomer
203 mg (44 %), polares Diastereomer

Citrat Unpolar:

Das soeben isolierte unpolarere Diastereomer (155 mg, 0.31 mmol) wurde in einer Mischung aus Ethanol (5 ml) und Methanol (15 ml) heiß gelöst, mit Citronensäure (60 mg, 0.31 mmol) versetzt und nochmals erhitzt. Der nach anschließendem Stehen im Kühlschrank ausgefallene Niederschlag wurde abgesaugt und mit Ether nachgewaschen.
*Ausbeute:* 126 mg (72 %)
$^1$*H-NMR (DMSO-d$_6$):* 1.47-1.71 (8H, m); 1.78-1.82 (4H, m); 2.15 (6H, s); 2.58-2.73 (4H, m); 2.83-3.00 (5H, m); 3.06-3.13 (2H, m); 3.38-3.49 (1H, m); 6.91-7.02 (2H, m); 7.08 (1H, t); 7.31-7.47 (7H, m); 10.69 (1H, s); 11.10 (4H, bs).

**Beispiel 282: N-(2-(2-(4-(dimethylamino)-4-phenylcyclohexyl)-1H-indol-3-yl)ethyl)cyclopentanesulfonamide, Citrat (1:1) : Polareres Diastereomer**

Citrat Polar:

Das unter Beispie**l 281** isolierte polarere Diastereomer (176 mg, 0.36 mmol) wurde in Ethanol (5 ml) und Methanol (15 ml) heiß gelöst, mit Citronensäure (68 mg, 0.36 mmol) versetzt, kurz zum Sieden erhitzt und in den Kühlschrank gestellt. Da kaum Niederschlag ausfiel, wurde die Lösung i. Vak. auf die Hälfte eingeengt und über Nacht im Kühlschrank stehen gelassen. Der ausgefallene Niederschlag wurde abgesaugt und mit Ether nachgewaschen.
*Ausbeute:* 63 mg (26 %)
$^1$*H-NMR (DMSO-d$_6$):* 1.42-1.68 (6H, m); 1.80-1.92 (8H, m); 2.29 (6H, s); 2.48-2.61 (4H, m); 2.82-3.08 (7H, m); 3.40-3.47 (1H, m); 6.87-6.95 (2H, m); 7.07-7.16 (2H, m); 7.35-7.62 (6H, m); 10.42 (1H, s); 11.10 (4H, bs).

**Beispiel 283: (±)-N-(2-(2-(4-(dimethylamino)-4-phenylcyclohex-1-enyl)-1H-indol-3-yl)ethyl)benzenesulfonamid**

Keton (**Ket-10**, 1.33 g, 6.15 mmol) und Indol (Ind-104, 1.85 g, 6.15 mmol) wurden in abs. Dichlormethan (40 ml) unter Argon gelöst. Anschließend wurde Trifluormethansulfonsäure (1.07 ml, 12.3 mmol) schnell zugegeben und 16 h bei RT gerührt. Zur Aufarbeitung wurde der Ansatz mit 1 N NaOH basisch gemacht und 15 min bei RT nachgerührt. Die Phasen wurden abgetrennt. Die wässrige Phase wurde mit Dichlormethan (3 x 20 ml) extrahiert. Die organische Phase wurde über $Na_2SO_4$ getrocknet und i. Vak. eingeengt. Der Rückstand wurde mit $CHCl_3$/MeOH (9:1, 10 ml) versetzt, dabei fiel ein unlöslicher Feststoff aus. Dieser Feststoff wurde abgesaugt und i. Vak. getrocknet. *Ausbeute:* 1.14 g (37 %)

**N-(2-(2-(4-(dimethylamino)-4-phenylcyclohexyl)-1H-indol-3-yl)ethyl)benzenesulfonamide, Citrat (1:1) : Polareres Diastereomer**

Das erhaltene Olefin (1.14 g/ 2.28 mmol) wurde in HBr/Eisessig (55 ml) gelöst. Innerhalb von 30 min wurde Zinn (2.64 g/ 2.28 mmol) zugegeben und 4 h bei RT gerührt. Der Ansatz wurde mit Ethanol versetzt und über Nacht bei RT gerührt. (reichen aber eigentlich 20 min). Anschließend wurde der Ansatz i. Vak. zur Trockene eingeengt, mit 5N NaOH versetzt und mit Dichlormethan (3 x 20 ml) extrahiert. Die organische Phase wurde über $Na_2SO_4$ getrocknet und i. Vak. eingeengt. Der Rückstand wurde durch Flash-Chromatographie EE/EtOH (2:1) gereinigt.
*Ausbeute:* 45 mg (4 %) unpolar
285 mg (25 %) polar

Citrat Polar:

Das soeben isolierte polarere Diastereomer (280 mg/ 0.558 mmol) wurde in heißem Ethanol (5 ml) gelöst. Citronensäure (106 mg/ 0.558 mmol) wurde in heißem Ethanol (1 ml) gelöst und zugegeben. Der Ansatz wurde auf RT abgekühlt , dabei ist ein Niederschlag ausgefallen. Der Niederschlag wurde abgesaugt und i. Vak. getrocknet.
*Ausbeute:* 154 mg (40 %); *Schmelzpunkt:* 145-149 °C
$^1$*H-NMR (DMSO-d$_6$):* 1.40-1.49 (2H, m); 1.72 (2H, d); 1.85 (2H, t); 2.22 (6H, s); 2.40-2.59 (4H, m); 2.70-2.74 (2H, m); 2.82-2.93 (5H, m); 6.83-6.92 (2H. m); 7.12-7.23 (2H, dd); 7.42 (1H, t); 7.49-7.64 (7H, m); 7.80 (2H, d); 10.38 (1H, s); 11.42 (4H, bs).

**Beispiel 284: (±)-N-(2-(2-(4-(dimethylamino)-4-phenylcyclohex-1-enyl)-1H-indol-3-yl)ethyl)thiophen-2-sulfonamid**

Keton (Ket-10, 975 mg, 4.5 mmol) und Indol (Ind-105, 1.38 g, 4.5 mmol) wurden in abs. Dichlormethan (40 ml) unter Argon gelöst. Anschließend wurde Trifluormethansulfonsäure (781 µL, 9.0 mmol) schnell zugegeben und 16 h bei RT gerührt. Zur Aufarbeitung wurde der Ansatz mit 1 N NaOH basisch gemacht und 15 min bei RT nachgerührt. Die Phasen wurden getrennt. Die wässrige Phase wurde mit Dichlormethan (3 x 20 ml) extrahiert. Die organische Phase wurde über $Na_2SO_4$ getrocknet und i. Vak. eingeengt. Der Rückstand wurde durch Flash-Chromatographie $CHCl_3$/MeOH (15:1, 1:1, MeOH+1 % TEA) gereinigt.
*Ausbeute:* 1.26 g (55 %)

**N-(2-(2-(4-(dimethytamino)-4-phenylcyclohexyl)-1H-indol-3-yl)ethyl)thiophen-2-sulfonamide, Citrat(1:1): Unpolareres Diastereomer**

Das soeben isolierte Olefin (1.26 g, 2.49 mmol) wurde in HBr/Eisessig (50 ml) gelöst. Innerhalb von 30 min wurde Zinn (2.88 g, 2.49 mmol) zugegeben und 3 h bei RT gerührt. Der Ansatz wurde mit Ethanol versetzt und 10 min bei RT gerührt. Anschließend wurde der Ansatz i. Vak. zur Trockene eingeengt, mit 5N NaOH versetzt und mit Dichlormethan (3 x 20 ml) extrahiert. Die organische Phase wurde über $Na_2SO_4$ getrocknet und i. Vak. eingeengt. Der Rückstand wurde durch Flash-Chromatographie $CHCl_3$/MeOH (20:1, 4:1, MeOH+ 1 % TEA) getrennt.

*Ausbeute:* 268 mg (22 %) unpolareres Diastereomer
262 mg (20 %) polareres Diastereomer

Citrat Unpolar:

Das soeben erhaltene unpolarere Diastereomer (268 mg/ 0.528 mmol) wurde in heißem Ethanol (15 ml) und Methanol (5 ml) gelöst. Citronensäure (101 mg/ 0.528 mmol) wurde in heißem Ethanol (1 ml) gelöst und zugegeben. Der Ansatz wurde auf RT abgekühlt, dabei ist ein Niederschlag ausgefallen. Der Niederschlag wurde abgesaugt und i. Vak. getrocknet. *Ausbeute:* 232 mg (62 %); *Schmelzpunkt:* 233-235 °C
*$^1$H-NMR (DMSO-$d_6$):* 1.51-1.64 (4H, m); 2.11 (8H, s); 2.57-2.72 (4H, m); 2.80-2.97 (7H, m); 6.88-7.00 (2H. m); 7.13-7.15 (1H, m); 7.29-7.45 (7H, m); 7.55-7-56 (1H, m); 7.87-7.94 (2H, m) 10.65 (1H, s); 11.1 (4H, bs).

**Beispiel 285: N-(2-(2-(4-(dimethylamino)-4-phenylcyclohexyl)-1H-indol-3-yl)ethyl)thiophen-2-sulfonamide, Citrat (1:1) : Polareres Diastereomer**

Citrat Polar:

Das unter Beispiel 284 erhaltene polarere Diastereomer (262 mg/ 0.516 mmol) wurde in heißem Ethanol (15 ml) und Methanol (5 ml) gelöst. Citronensäure (98 mg/ 0.516 mmol) wurde in heißem Ethanol (1 ml) gelöst und zugegeben. Der Ansatz wurde auf RT abgekühlt , dabei ist ein Niederschlag ausgefallen. Der Niederschlag wurde abgesaugt und i. Vak. getrocknet.
*Ausbeute:* 229 mg (63 %); *Schmelzpunkt:* 143-145 °C
*$^1$H-NMR (DMSO-$d_6$):* 1.40-1.52 (2H, m); 1.71-1.86 (4H, m); 2.21 (6H, s); 2.47-2.60 (4H, m); 2.74-2.94 (7H, m); 6.84-6.94 (2H, m); 7.13-7.28 (3H, m); 7.42-7.59 (6H, m); 7.92-7.94 (2H, m) 10.41 (1H, s); 11.1 (4H, bs).

**Beispiel 286: (±)-N-(2-(2-(4-(dimethylamino)-4-phenylcyclohex-1-enyl)-1H-indol-3-yl)ethyl)nicotinamid**

Keton (Ket-10, 392 mg, 1.81 mmol) und Indol (Ind-106, 482 mg, 1.81 mmol) wurden in abs. Dichlormethan (40 ml) unter Argon gelöst. Anschliessend wurde Trifluormethansulfonsäure (471 µL, 5.43 mmol) schnell zugegeben und 16 h bei RT gerührt. Zur Aufarbeitung wurde der Ansatz mit 1 N NaOH basisch gemacht und 15 min bei RT nachgerührt. Die Phasen wurden abgetrennt. Die wässrige Phase wurde mit Dichlormethan (3 x 20 ml) extrahiert. Die organische Phase wurde über $Na_2SO_4$ getrocknet und i. Vak. eingeengt. Der Rückstand wurde durch Flash-Chromatographie $CHCl_3$/MeOH (20:1, 1:1, MeOH) gereinigt.
*Ausbeute:* 277 mg (33 %)

**N-(2-(2-(4-(dimethylamino)-4-phenylcyclohexyl)-1H-indol-3-yl)ethyl)nicotinamide, Citrat (1:1) : Unpolareres Diastereomer**

Das soeben isolierte Olefin (333 mg/ 0.716 mmol) wurde in HBr/Eisessig (25 ml) gelöst. Innerhalb von 30 min wurde Zinn (828 mg/ 0.716 mmol) zugegeben und 3 h bei RT gerührt. Der Ansatz wurde mit Ethanol versetzt und 10 min bei RT gerührt. Anschliessend wurde der Ansatz i. Vak. zur Trockene eingeengt, mit 5N NaOH versetzt und mit Dichlormethan (3 x 20 ml) extrahiert. Die organische Phase wurde über $Na_2SO_4$ getrocknet und i. Vak. eingeengt. Der Rückstand wurde durch Flash-Chromatographie $CHCl_3$/MeOH (50:1) gereinigt.

*Ausbeute:* 91 mg (27 %) unpolareres Diastereomer
248 mg (74 %) polareres Diastereomer, leicht verunreinigt

Citrat Unpolar:

Das soeben erhaltene unpolarere Diastereomer (86 mg/ 0.184 mmol) wurde in heißem Ethanol (8 ml) gelöst. Citronensäure (35 mg/ 0.184 mmol) wurde in heißem Ethanol (1 ml) gelöst und zugegeben. Der Ansatz wurde i. Vak. zur Trockene eingeengt und mit Ether versetzt. Der ausgefallene Niederschlag wurde abgesaugt und i. Vak. getrocknet.
*Ausbeute:* 59 mg (48 %); *Schmelzpunkt:* 263-265 °C
$^1$*H-NMR (DMSO-$d_6$):* 1.67-1.71 (4H, m); 2.33 (8H, s); 2.92-2.97 (5H, m); 3.44 (2H, qu); 6.91-7.03 (2H, m); 7.28 (1H, d); 7.44-7.54 (7H, m); 8.14-8.18 (1H, m); 8.67-8.69 (1H,m); 8.82 (1H, t); 8.98 (1H, d); 11.1 (1H, s).

**Beispiel 287: N-(2-(2-(4-(dimethylamino)-4-phenylcyclohexyl)-1H-indol-3-yl)ethyl)nicotinamide, Citrat (1:1) : Polareres Diastereomer**

Citrat Polar:

Das unter Beispiel 286 erhaltene polarere Diastereomer (248 mg/ 0.531 mmol) wurde in heißem Ethanol (10 ml) und Methanol (5 ml) gelöst. Citronensäure (101 mg/ 0.531 mmol) wurde in heißem Ethanol (1 ml) gelöst und zugegeben. Der Ansatz wurde auf RT abgekühlt , dabei ist ein Niederschlag ausgefallen. Der Niederschlag wurde abgesaugt und i. Vak. getrocknet.
*Ausbeute:* 49 mg (14 %); *Schmelzpunkt:* 205-210 °C
$^1$*H-NMR (DMSO-$d_6$):* 1.41-1.54 (2H, m); 1.78-1.83 (2H, m); 2.08-2.20 (2H, m); 2.38 (6H, s); 2.49-2.62 (4H, m); 2.86-3.16 (5H, m); 3.40-3.45 (2H, m) 6.88-6.93 (2H, m); 7.14-16 (1H, m); 7.48-7.59 (5H, m); 7.72 (2H, d); 8.24-8.27 (1H,m); 8.69-8.71 (1H, m); 8.99-9.03 (2H, m); 10.42 (1H, s); 11.45 (4H, bs).

**Beispiel 288: 4-(3-(2-Bromoethyl)-1H-indol-2-yl)-N,N-dimethyl-1-phenylcyclohexanamin**

2-(2-(4-Dimethylamino-4-pheny-cyclohexyl)-1*H*-indo)-3-yl]-ethanol (Beispiel 219, 3.20 g, 8.8 mmol) wurde bei RT in abs. $CH_2Cl_2$ (50 ml) gelöst und mit Tetrabrommethan (4.39 g, 13.2 mmol) versetzt. Anschließend wurde bei RT Triphenylphosphin (3.61 g, 12.6 mmol) zugegeben. Die Lösung wurde 2.5 h bei RT gerührt und anschließend i. Vak. eingeengt. Flash-Chromatographie des Rückstandes mit 300 g Kieselgel und Essigester/Ethanol (1:2) ergab 1.98 g verunreinigtes Produkt, das mit 1N NaOH versetzt und dreimal mit $CHCl_3$ extrahiert wurde. Die organische Phase wurde über $Na_2SO_4$ getrocknet und i. Vak. eingeengt. *Ausbeute:* 1.57 g (42 %)

**4-(3-(2-aminoethyl)-1H-indol-2-yl)-N,N-dimethyl-1-phenylcyclohexanamin**

Das soeben hergestellte Bromid (868 mg, 2.0 mmol) wurde in 2N $NH_3$-Lösung in Ethanol (15 ml) gelöst und in einem Druckgefäß 7 h bei 100°C gerührt. Die Lösung wurde i. Vak. eingeengt. Der erhaltene Rückstand wurde mit Ethanol gewaschen und dann i. Vak. getrocknet.
*Ausbeute:* 924 mg (>100 %), Diastereomerengemisch
$^1$*H-NMR (DMSO-$d_6$):* 1.43-1.52 (2 H, m); 1.76-1.79 (2 H, m); 2.17 (6 H, s); 2.89-3-06 (7 H, m); 6.91-6.95 (2 H, m); 7.17 (1 H, d); 7.39-7.62 (6 H, m); 8.08 (2 H, bs); 10.48/10.49 (1 H, 2s).

**N-(2-(2-4-(dimethylamino)-4-phenytcyclohexyl)-1H-indol-3yl)ethyl)benzamide, Citrat (1:1) : Eienes von zwei möglichen Diastereomeren**

Das soeben hergestellte 4-(3-(2-aminoethyl)-1H-indol-2-yl)-N,N-dimethyl-1-phenylcyclohexanamin (200 mg,

0.55 mmol) wurde in abs. $CH_2Cl_2$ (5 ml) suspendiert. Anschließend wurden bei RT Triethylamin (78 μL, 0.63 mmol) und Benzoylchlorid (73 μL, 0.63 mmol) zugegeben und der Ansatz 1 d bei RT gerührt. Der Ansatz wurde mit Wasser versetzt und dreimal mit $CH_2Cl_2$ extrahiert. Die organische Phase wurde über $Na_2SO_4$ getrocknet und i. Vak. eingeengt. Der erhaltene Rückstand wurde durch Flash-Chromatographie mit 50 g Kieselgel und Essigester/Ethanol (1:1→1:2→1:4) gereinigt.
*Ausbeute:* 129 mg (50 %), Diastereomerengemisch

Citrat:

Da das Spektrum der soeben hergestellten Verbindung auf ein Salz hindeutete wurde sie (119 mg, 0.26 mmol) mit 1 N NaOH versetzt und mehrmals mit $CH_2Cl_2$ extrahiert, die organische Phase wurde anschließend über $Na_2SO_4$ getrocknet und i. Vak. eingeengt. Die erhaltene reine Verbindung (52 mg, 0.11 mmol) wurde in Ethanol (1 ml) heiß gelöst und mit Citronensäure (22 mg, 0.11 mmol) gelöst in Ethanol (0.5 ml) versetzt. Nach 2 Stunden wurde der ausgefallene Niederschlag abgesaugt und mit Ether nachgewaschen.
*Ausbeute:* 15 mg (20 %)
*$^1H$-NMR (DMSO-$d_6$):* 1.40-1.55 (2H, m); 1.74-1.84 (4H, m); 2.31 (6H, s); 2.49-2.63 (4H, m); 2.89-2.93 (5H, m); 3.36-3.42 (2H, m); 6.87-6.95 (2H, m); 7.14-7.16 (1H, m); 7.45-7.61 (9H, m); 7.86-7.89 (2H, m); 8,61 (1H, t); 10.40 (1H, s); 11.10 (4H, bs).

**Beispiel 289: 1-(3-Fluorphenyl)-N,N-dimethyl-4-(3-(2-(pyridin-4-yl)ethyl)-1H-indol-2-yl)cyclohexanamin, unpolares Diastereomer**

**Beispiel 290: 1-(3-Fluorphenyl)-N,N-dimethyl-4-(3-(2-(pyridin-4-yl)ethyl)-1H-indol-2-yl)cyclohexanamin, Citrat (1:1), polares Diastereomer**

**1-(3-Fluorphenyl)-N,N-dimethyl-4-(3-(2-(pyridin-4-yl)ethyl)-1H-indol-2-yl)cyclohexanamin (polares Diastereomer) und**

**1-(3-Fluorphenyl)-N,N-dimethyl-4-(3-(2-(pyridin-4-yl)ethyl)-1H-indol2-yl)cyclohexanamin, unpolares Diastereomer ( 289)**

Bsp. 252 (257 mg, 0,58 mmol) wurde in HBr/Eisessig (33 % HBr, 15 ml) gelöst. Anschließend wurde zu dem Ansatz bei RT Sn-Pulver (2 g, 16,8 mmol) innerhalb von 60 min portionsweise gegeben. Der Ansatz wurde über Nacht (18 h) bei RT gerührt. Der ausgefallene gelbe Feststoff wurde mittels einer Fritte abgetrennt. Der Filterkuchen wurde mit Eisessig (10 ml) gewaschen, in Ethanol (10 ml) zum Sieden erhitzt und anschließen durch Filtration abgetrennt. Der Feststoff wurde mit 2N NaOH (10 ml) und Ethylacetat (20 ml) versetzt und 20 min gerührt. Der an der Phasengrenze befindliche.Feststoff (1-(3-Fluorphenyl)-N,N-dimethyl-4-(3-(2-(pyridin-4-yl)ethyl)-1H-indol-2-yl)cyclohexanamin, polares Diastereomer) wurde mittels einer Fritte abgetrennt und getrocknet (158 mg, *Schmelzpunkt:* 301-314 °C, 61 % Ausbeute) und wurde zur Citratbildung (s. sp. 290) eingesetzt. - Die Eisessig/HBr Lösung, die Ethylacetatphase und die ethanolische Lösung wurden am Rotationsverdampfer bis zur Trockene eingeengt und die jeweiligen Rückstände vereinigt. Das so erhaltene Gemisch wurde mit 5N NaOH (10 ml) basisch gestellt und mit Ethylacetat (5 x 20 ml) extrahiert. Die vereinigten organischen Phasen wurden mit Wasser (50 ml) gewaschen, über $MgSO_4$ getrocknet und anschließend eingeengt. Der Rückstand wurde durch Säulenchromatographie [Kieselgel 60 G (10 g); EtOAc/ EtOH 1 : 1 (150 ml)] gereinigt. Beispiel 289 (unpolareres Diastereomer) wurde so in einer Ausbeute von 30 mg (11 %) als weißer Feststoff mit einem Schmelzpunkt von 211-218 °C erhalten. *$^1H$ NMR (300 MHz, $CDCl_3$) δ ppm:* 1.18-1.36 (m, 2H), 1.39-1.52 (m, 2H), 1.58-1.74 (m, 2H), 2.08 (s, 6H), 2.46-2.50 (m, 1H), 2.63-2.75 (m, 1H), 2.86-3.08 (m, 4H), 6.90- 7.15- (m, 6H), 7.18-7.32 (m, 1H), 7.34-7.45 (m, 1H), 7.48-7.54 (m, 2H), 8.43 (dd, *J* = 4.44, 1.57 Hz, 2H)
*$^{13}C$ NMR (101 MHz, $CDCl_3$) δ ppm:* 25.5, 29.4, 33.5, 35.7, 36.2, 38.1, 61.5, 108.9,110.5, 113.7, 113.9, 115.0, 115.2, 117.9, 119.2, 121.2, 123.8, 124.2, 128.1, 129.4, 129.5, 135.1, 139.0, 149.37, 151.2, 161.8, 164.3

**1-(3-Fluorphenyl)-N,N-dimethyl-4-(3-(2-(pyridin-4-yl)ethyl)-1H-indol-2-yl)cyclohexanamin, Citrat (1:1), polares Diastereomer ( 290)**

1-(3-Fluorphenyl)-N,N-dimethyl-4-(3-(2-(pyridin-4-yl)ethyl)-1H-indol-2-yl)cyclohexanamin (polareres Diastereomer, 140 mg, 0,36 mmol) wurde in Isopropanol (100 ml) zum Sieden erhitzt. Noch ungelöste Partikel wurden mittels einer Fritte abgetrennt. Die erhaltene Lösung wurde mit Citronensäure (160 mg, 0.83 mmol), gelöst in heißem Isopropanol (3 ml), versetzt. Das Lösungsmittelvolumen wurde am Rotationsverdampfer auf ca. 20 ml reduziert, anschließend wurde die Mischung auf 5 °C abgekühlt (Kühlschrank) und 2 h bei dieser Temperatur belassen. Der entstandene Niederschlag

wurde mittels einer Fritte abgetrennt und anschließend im Hochvakuum getrocknet. Beispiel 290 wurde so in einer Ausbeute von 84 mg (41 %) mit einem Schmelzpunkt von 143-151 °C erhalten.

$^1H$ *NMR (300 MHz, DMSO-$d_6$) ∃ppm:* 1.29-1.49 (m, 4H), 2.02 (s, 6H), 2.52-2.67 (m, 6H), 2.67-2.78 (m, 3H), 2.79-3.03 (m, 4H), 6.86-7.03 (m, 2H), 7.05-7.26 (m, 5H), 7.26-7.33 (m 1H), 7.36-7.50 (m, 2H), 8.39 (dd, J = 4.53, 1.36 Hz, 2H), 10.60 (s, 1H)

**Beispiel 291: -Methyl-1-phenyl-4-(3-(2-(pyridin-4-yl)ethyl)-1H-indol-2-yl)cyclohexanamin, Citrat (2:1), unpolares Diastereomer**

**Beispiel 292: -Methyl-1-phenyl-4-(3-(2-(pyridin-4-yl)ethyl)-1H-indol-2-yl)cyclohexanamin, Citrat (1:1), polares Diastereomer**

**N-Methyl-1-phenyl-4-(3-(2-(pyridin-4-yl)ethyl)-1H-indol-2-yl)cyclohexanamin (unpolares und polares Diastereomer)**

Bsp. 254 (350 mg, 0,86 mmol) wurde in Bromwasserstoff/Eisessig (33 % HBr, 18 ml) gelöst. Anschließend wurde zu dem Ansatz bei Raumtemperatur Zinn-Pulver (1,02 g, 8,6 mmol) innerhalb von 40 min portionsweise gegeben. Es entstand eine gelbe Suspension. Der Ansatz wurde noch 20 h gerührt. Anschließend wurde das Gemisch mit Dichlormethan (50 ml) verdünnt und unter Eiskühlung mit 5N Natriumhydroxidlösung (50 ml) alkalisch gemacht. Die Phasen wurden getrennt. Die wässrige Phase wurde mit Dichlormethan (3 x 30 ml) extrahiert. Die vereinigten organischen Phasen wurden mit $Na_2SO_4$ getrocknet und dann filtriert. Die flüchtigen Bestandteile des Filtrates wurden im Vakuum vollständig entfernt. Es blieb ein hellgelber Feststoff (534 mg) zurück. Dieser wurde chromatographisch getrennt [Kieselgel 60 (50 g); Ethylacetat/Methanol 4 : 1 (500 ml); Methanol/Ethylacetat 3 : 2 (500 ml); 4 : 1 (500 ml)]. N-Methyl-1-phenyl-4-(3-(2-(pyridin-4-yl)ethyl)-1H-indol-2-yl)cyclohexanamin (unpolareres Diastereoisomer) (178 mg, 50 %, Schmp.: 251-256 °C) sowie das polarere Diastereoisomer (97 mg, 28 %, Schmp.: 224-227 °C) konnten so gewonnen werden.

**N-Methyl-1-phenyl-4-(3-(2-(pyridin-4-yl)ethyl)-1H-indol-2-yl)cyclohexanamin, Citrat (2:1), unpolares Diastereomer**

N-Methyl-1-phenyl-4-(3-(2-(pyridin-4-yl)ethyl)-1H-indol-2-yl)cyclohexanamin (unpolareres Diastereoisomer) (178 mg, 0,43 mmol) wurde in Ethanol (20 ml) unter Erwärmen gelöst und mit Citronensäure (100 mg, 0,52 mmol), gelöst in Ethanol (4 ml) versetzt. Die Lösung wurde 17 h gerührt, dann auf ca. 3 ml eingeengt und mit Diethylether (5 ml) bis zur Kristallisation versetzt. Der ausgefallene farblose Niederschlag wurde abgesaugt und mit Diethylether (5 ml) gewaschen. Beispiel 291 wurde so in einer Ausbeute von 53% (115 mg) mit einem Schmelzpunkt von 267-277 °C gewonnen.

$^1H$ *NMR (400 MHz, DMSO-$d_6$) ∃ppm:* 1.30-1.48 (m, 2H), 1.80-1.99 (m, 2H), 2.18 (s, 3H), 2.21-2.38 (m, 2H), 2.38-2.49 (m, 2H), 2.60 (dd, J = 33.53, 15.20 Hz, 2H), 2.72-2.93 (m, 3H), 2.93-3.08 (m, 2H), 6.90-6.99 (m, 1H), 6.99-7.08 (m, 1H), 7.11-7.20 (m, 2H), 7.23-7.31 (m, 1H), 7.37-7.45 (m, 1H), 7.45-7.55 (m, 3H), 7.59-7.69 (m, 2H), 8.39 (d, J = 5.09 Hz, 2H), 10.98 (s, 1H)

$^{13}C$ *NMR (101 MHz, DMSO-$d_6$)* C: 24.8, 26.4, 26.9, 32.3, 32.5, 36.0, 43.7, 60.8, 71.6, 107.6, 110.5, 117.7, 118.1, 120.2, 124.2, 126.1, 127.4, 128.4, 128.8, 135.2, 139.8, 149.1, 150.7, 171.2, 176.1

**N-Methyl-1-phenyl-4-(3-(2-(pyridin-4-yl)ethyl)-1H-indol-2-yl)cyclohexanamin, Citrat (1:1), polares Diastereomer (292)**

N-Methyl-1-phenyl-4-(3-(2-(pyridin-4-yl)ethyl)-1H-indol-2-yl)cyclohexanamin (polareres Diastereoisomer) ( (97 mg, 0,284 mmol) wurde unter Erwärmen in Ethanol (10 ml) gelöst und mit Citronensäure (55 mg, 0,284 mmol), gelöst in Ethanol (2 ml) versetzt. Nach 1 h Rühren bei Raumtemperatur begann ein farbloser Feststoff auszufallen. Dieser wurde abfiltriert und mit Diethylether (5 ml) gewaschen. Beispiel 292 wurde in einer Ausbeute von 52 % (74 mg) mit einem Schmelzpunkt von 243-245 °C erhalten.

$^1H$ *NMR (300 MHz, DMSO$d_6$)* δ ppm: 1.33-1.58 (m, 4H), 1.62-1.87 (m, 2H), 2.01 (s, 3H), 2.51 (dd, J = 24.19, 15.02 Hz, +DMSO), 2.60-2.75 (m, 3H), 2.75-2.86 (m, 2H), 2.86-2.99 (m, 2H), 6.84-7.01 (m, 2H), 7.06-7.13 (m, 2H), 7.13-7.22 (m, 1H), 7.33-7.54 (m, 4H), 7.54-7.66 (m, 2H), 8.37-8.48 (m, 2H), 10.41 (s, 1H)

$^{13}C$ *NMR (101 MHz, DMSO-$d_6$)* δ: ppm: 25.1, 26.9, 28.5, 33.8, 35.7, 36.0, 44.6, 59.9, 71.1, 107.7, 110.7, 117.4, 118.0, 120.0, 124.0, 127.7, 127.8, 128.7, 135.2, 137.8, 139.2, 149.2, 150.7, 171.5, 177.2

**Beispiel 293: -(4-Butyl-4-(pyrrolidin-1-yl)cyclohexyl)-3-methyl-1H-indol, Citrat (2:1), unpolares Diastereomer**

**2-(4-Butyl-4-(pyrrolidin-1-yl)cyclohexyl)-3-methyl-1H-indol (unpolares und polares Diastereomer)**

**Variante 1: Beispiel 258 (211 mg, 0,627 mmol) wurde in Methanol (30 ml) gelöst und mit** Palladium auf Kohle (5-proz., 50 mg) versetzt. Die Reaktionsmischung wurde 3,5 h bei 3 bar hydriert. Der Katalysator wurde über Celite abgetrennt und das Filtrat eingeengt. Der Rückstand (200 mg, hellbraunes Öl) wurde chromatographisch aufgetrennt (Kieselgel 60 (20 g); Ethylacetat/Methanol 10 : 1 (200 ml). Ethylacetat/Methanol 4 : 1 (200 ml), Methanol (200 ml)]. 2-(4-Butyl-4-(pyrrolidin-1-yl)cyclohexyl)-3-methyl-1H-indol (unpolareres Diastereoisomer) wurde in einer Ausbeute von 10 % (20 mg) das polarere Diastereoisomerin einer Ausbeute von 67 % (143 mg) gewonnen. Beide Diastereoisomere lagen nach der Chromatographie als farblose Salze vor.

**Variante 2**: Beispiel 258 (180 mg, 0,535 mmol) wurde mit HBr/Eisessig (33 % HBr, 10 ml) innerhalb von 1 h bei Raumtemperatur gelöst. Anschließend wurde zu dem Ansatz Zinn-Pulver (64 mg, 0,535 mmol) innerhalb von 10 min portionsweise gegeben. Nach Beendigung der Zugabe wurde das Reaktionsgemisch weitere 30 min gerührt. Unter Eiskühlung wurde der Ansatz mit Wasser (20 ml) versetzt und 15 min bei Raumtemperatur gerührt. Der ausgefallene beigefarbene Feststoff wurde abgesaugt, mit Wasser (4 × 5 ml) und mit Dichlormethan ( 2 × 5 ml) gewaschen. Das Hydrobromid des Diastereoisomerengemisches wurde in einer Ausbeute von 69 % (155 mg) erhalten. Das Salz wurde in einem Gemisch aus Dichlormethan (30 ml), Wasser (20 ml) und 1N Natronlauge (2 ml) aufgenommen und 30 min bei Raumtemperatur gerührt. Die Phasen wurden getrennt. Die wässrige Phase wurde mit Dichlormethan (20 ml) extrahiert. Die organischen Phasen wurden vereinigt, mit Natriumsulfat getrocknet und eingeengt. Der Rückstand (107 mg, beigefarbenes Öl) wurde chromatographisch [Kieselgel 60 (20 g); Ethylacetat/Methanol 10 : 1 (200 ml), Ethylacetat/Methanol 4 : 1 (200 ml), Methanol (200 ml)] aufgetrennt. Das unpolarere Diastereoisomer wurde in einer Ausbeute von 31 % (56 mg) und das polarere Diastereoisomer in einer Ausbeute von 17 % (31 mg) erhalten. Beide Diastereoisomere lagen nach der Chromatographie als farblose Salze vor.

**2-(4-Butyl-4-(pyrrolidin-1-yl)cyclohexyl)-3-methyl-1H-indol, Citrat (2:1), unpolares Diastereomer ( 293)**

Das Salz des unpolareren Diastereomers (76 mg) wurde in einem Gemisch aus Dichlormethan (30 ml), Wasser (20 ml) und 1 N Natronlauge (1 ml) aufgenommen und 1 h bei Raumtemperatur gerührt. Die Phasen wurden getrennt. Die wässrige Phase wurde mit Dichlormethan (20 ml) extrahiert. Die vereinigten organischen Phasen wurden mit Natriumsulfat getrocknet und eingeengt. Der Rückstand (beigefarbenes Öl, 75 mg, 0,221 mmol) wurde in Ethanol (5 ml) gelöst und mit einer ethanolischen Lösung (1,5 ml) von Citronensäure (46 mg, 0,24 mmol) versetzt. Eine Fällung war sofort sichtbar. Nach 30 min wurde Diethylether (15 ml) hinzugefügt. Nach einer Reaktionszeit von 1 h bei Raumtemperatur wurde der Feststoff durch Filtration abgetrennt und mit Diethylether (2 x 2 ml) gewaschen. Beispiel 293 wurde in einer Ausbeute von 70 % (67 mg) mit einem Schmelzpunkt von 214-216 °C erhalten.
1*H NMR (300 MHz, DMSO-$d_6$,)* δ pm: 0.94 (t, 3H), 1.12,-1.37 (m, 5H), 1.37-1.70 (m, 7H), 1.70-2.03 (m, 8H), 2.18 (s, 3H), 2.54 (dd, 2H), 2.63-3.11 (m,3H), 6.88-7.02 (m, 2H), 7.30 (d, 1H), 7.36 (d, 1H), 10.32 (s, 1H)

**Beispiel 294: (±)-3-(4-(Dimethylamino)-4-phenylcyclohex-1-enyl)-(1-(phenylsulfonyl)-1H-indol)**

1-Benzolsulfonyl-1*H*-indol (504 mg, 2 mmol) wurde zusammen mit dem Keton (Ket-10. 434 mg, 2 mmol) in Dichlormethan (20 ml) gelöst und mit Trifluormethansulfonsäure (0,2 ml, 2,3 mmol) versetzt. Der Ansatz wurde 15 h bei RT gerührt. Da die Umsetzung laut DC nicht vollständig war, wurde das Gemisch weitere 3 d gerührt. - Zur Aufarbeitung wurde die Reaktionslösung mit 2N NaOH (10 ml) versetzt. Das Gemisch wurde weitere 20 min gerührt. Nach Trennung der Phasen wurde die wäßrige Phase mit Dichlormethan (3 x 20 ml) extrahiert. Die vereinigten organischen Extrakte wurden über $Na_2O_4$ getrocknet und anschließend eingeengt. Das erhaltene Rohprodukt (917 mg) wurde säulenchromatographisch (Laufmittel: EtOAc) gereinigt. Neben einer Bisindolverbindung (178 mg, Schmp.: 237-242 °C) wurde das gewünschte Olefin in einer Ausbeute von 440 mg (48 %, Schmp.: 165-167 °C) in kristalliner Form erhalten.

**N,N-dimethyl-1-phenyl-4-(1-(phenytsutfonyl)-1H-indol-2-yl)cyclohexanamin : Unpolareres Diastereoisomer**

Das soeben erhaltene Olefin (300 mg, 0,66 mmol) wurde in HBr/Eisessig (33 % HBr, 10 ml) gelöst (nicht alle Subtanz war gelöst, größere Menge HBr erscheint deshalb als angebracht). Anschließend wurde zu dem Ansatz bei RT Sn-Pulver (0,8 g, 7 mmol) innerhalb von 40 min portionsweise gegeben. Nach Beendigung der Zugabe wurde das Reaktionsgemisch weitere 3 h gerührt. - Zur Aufarbeitung wurde das Gemisch am Rotationsverdampfer bis zur Trockene

eingeengt. Der verbliebene Rückstand wurde durch Zugabe von 5N NaOH (20 ml) basisch gemacht. Die erhalte Lösung wurde mit Dichlormethan versetzt und extrahiert (4 x 20 ml). Die vereinigten organischen Phasen wurden mit $MgSO_4$ getrocknet und anschließend eingeengt. Der erhaltene Rückstand (290 mg) wurde säulenchromatographisch (Laufmittel: 1) EtOAc; 2) EtOAc/EtOH 2 : 1; 3) EtOH) gereinigt. Das unpolarere Diastereoisomer, welches im Dünnschichtchromatogramm bei Verwendung von EtOAc in der Nähe der Lösungsmittelfront lief, wurde so in einer Ausbeute von 29 mg (9 %) in Form eines gelblichen Öls erhalten. Das polarerere Diastereoisomer, welches im Dünnschichtchromatogramm bei Verwendung von EtOAc in Nähe des Startflecks blieb, wurde in einer Ausbeute von 132 mg (43 %) als weißer Feststoff (Schmp.: 139-142 °C) erhalten. Das Ausgangsprodukt wurde zu 40 % zurückgewonnen.

$^1$*H NMR (300 MHz, $CDCl_3$)* δ ppm: 1.54-1.78 (m, 2H), 1.82-1.96 (m, 2H), 1.99-2.17 (m, 8H), 2.65-2.90 (m, 3H), 7.17-7.64 (m, 12H), 7.82-8.03 (m, 3H)

$^{13}$*C NMR (101 MHz, $CDCl_3$)* δ ppm: 27.7, 33.3, 34.7, 37.8, 58.8, 113.8, 119.9, 121.7, 122.9, 124.5, 126.8, 127.5, 129.0, 129.2, 130.6, 133.5, 135.4, 138.5, 139.8

**Beispiel 295: ,N-dimethyl-1-phenyl-4-(1-(phenylsulfonyl)-1H-indol-2-yl)cydohexanamin : Polareres Diastereoisomer**

Das unter Beispiel 294 erhaltene polarere Diastereomer wird im weiteren als Beispiel 295 geführt.

$^1$*H NMR (300 MHz, $CDCl_3$)* δ ppm: 1.30-1.51 (m, 2H), 1.87-2.07 (m, 4H), 2.19 (s, 6H), 2.75-2.94 (m, 3H), 7.09 (s, 1H), 7.12-7.20 (m, 1H), 7.21-7.29 (m, 1H), 7.31-7.52 (m, 9H), 7.33-7.81 (m, 2H), 7.91 (d, J = 8.21 Hz, 1H)

$^{13}$*C NMR (101 MHz, $CDCl_3$)* δppm: 29.1, 33.0, 34.7, 38.1, 63.1, 113.7, 119.7, 121.2, 122.9, 124.5, 126.6, 127.3, 127.8, 128.3, 129.1, 130.2, 133.5, 134.8, 135.4, 138.2

**Beispiel 296: (±)-2-(4-(Dimethylamino)-4-phenyicyclohex-1-enyl)-3-(methyl)-(1-(oxiran-2-ylmethyl)-1H-indol) : Diastereomerengemisch**Die Freie Base des Beispiels 16 (350 mg, 1,06 mmol) wurde in Dimethylformamid/Tetrahydrofuran (20 ml, 1 : 1) vorgelegt. Die klare hellgelbe Lösung wurde bei Raumtemperatur mit Natriumhydrid (60-proz. Suspension in Mineralöl, 110 mg, 2,75 mmol) versetzt. Die Reaktionsmischung gaste erst. Anschließend fiel ein heller Feststoff aus der Reaktionsmischung aus. Es wurde 1 h bei 57°C (Ölbadtemperatur) gerührt. Dann wurde bei dieser Temperatur das Epichlorhydrin (255 mg, 0,22 ml, 2,75 mmol; 1,183 g/ml) hinzugegeben. Die Reaktionsmischung begann zu sieden. Es wurde 1 h bei 57°C (Ölbadtemperatur) gerührt. Anschließend wurde die Reaktionsmischung mit Wasser (30 ml) und Diethylether (20 ml) versetzt. Die Mischung wurde 10 min gerührt. Anschließend wurden die Phasen getrennt. Die wäßrige Phase wurde mit Ethylacetat (2 × 20 ml) extrahiert. Die vereinigten organischen Phasen wurden mit gesättigter wäßriger NaCl-Lösung (3 × 10 ml) gewaschen, mit Natriumsulfat getrocknet und filtriert. Anschließend wurden die flüchtigen Bestandteile vollständig im Vakuum entfernt. Es verblieb ein hellgelbes Öl, welches noch Dimethylformamid enthielt. Aus dem Öl kristallisierte über Nacht ein farbloser Feststoff aus. Dieser löste sich nicht in Methanol. Der Feststoff wurde abfiltriert und im Vakuum getrocknet (100 mg, 0,26 mmol; 24 % beide Diastereoisomere). Das Filtrat wurde auf grobes Kieselgel aufgezogen und chromatographisch getrennt [Kieselgel 60 (150 g); Ethylacetat (500 ml); Ethylacetat/Methanol 5 : 1 (500 ml), 2 : 1 (500 ml)]. Es wurde weiteres Epoxid (200 mg, 0,52 mmol; 49 %; beide Diastereoisomere, Schmp. 147-150 °C) isoliert.

$^1$H NMR (400 MHz, RT, DMSO-$D_6$) δ *ppm:* 1.56 (s, br, 1H), 1.88-2.25 (m, br, 12H), 2.36 (psd, 1H), 2.53-3.03 (m, 4H), 3.77-3.83 (m, 1H), 3.90-4.14 (m, 1H), 5.96 (s, br, 1H), 6.97 (pst, 1H), 7.06 (pst, 1H), 7.27 (pst, 1H), 7.36 u. 7.51 (pst u. psd, 6H).

$^{13}$C NMR (101 MHz, RT, DMSO-$D_6$) δ *ppm:* 8.66, 8.68, 26.93, 26.99, 28.96, 29.01, 32.71, 32.75, 44.66, 44.84, 44.94, 45.19, 50.71, 50.75, 60.18, 60.20, 106.12, 106.16, 110.08, 110.09, 117.94, 117.95, 118.66, 118.67, 120.84, 120.86, 126.45, 127.17, 127.19, 127.47, 127.49, 127.95, 127.96, 129.23, 129.25, 131.00, 135.99, 136.02, 138.42, 138.49, 142.26, 142.27.

**Beispiel 297: N,N-dimethyl-4-(3-methyl-1-(oxiran-2-ylmethyl)-1H-indol-2-yl)-1-phenylcyclohexanamin, Citrat (1: 1) : Polareres'Diastereomer**

Die freie Base des polareren Skatol-Derivats Beispiel 18 (500 mg, 1,50 mmol) wurde in Dimethylformamid/Tetrahydrofuran (20 ml, 1 : 1) vorgelegt und die klare hellgelbe Lösung mit Natriumhydrid (60-proz. Suspension in Mineralöl, 210 mg, 3,13 mmol) versetzt. Die Reaktionsmischung gaste. Anschließend fiel ein heller Feststoff aus der Reaktionsmischung aus. Es wurde 1 h bei 57 °C (Ölbadtemperatur) gerührt. Dann wurde bei dieser Temperatur das Epichlorhydrin (290 mg, 0,122 ml, 3,13 mmol; 1,183 g/ml) hinzugegeben. Die Reaktionsmischung begann zu sieden. Es wurde 1 h bei 57 °C (Ölbadtemperatur) gerührt. Anschließend wurde die Reaktionsmischung mit Wasser (30 ml) und Diethylether (20 ml) versetzt. Die Mischung wurde 10 min gerührt. Anschließend wurden die Phasen getrennt. Die wäßrige Phase wurde mit Ethylacetat (2 × 20 ml) extrahiert. Es wurde versucht, die wäßrige Phase mit Dichlormethan zu extrahieren. Dies war aber erfolglos. Es konnte kein Epoxid extrahiert werden. Die vereinigten organischen Phasen (Ethylacetat und Diethylether) wurden mit gesättigter wäßriger NaCl-Lösung (3 × 10 ml) gewaschen, mit Natriumsulfat

getrocknet und filtriert. Anschließend wurden die flüchtigen Bestandteile im Vakuum vollständig entfernt. Es wurde versucht, den Rückstand chromatographisch zu trennen [Kieselgel 60 (150 g), Ethylacetat/Methanol 1 : 1 (1500 ml)]. Es konnten 215 mg (0,55 mmol; 37 %) der polaren Zielverbindung als farbloses Pulver isoliert werden.

Citrat Polar:

Das soeben erhaltene polarere Skatol-Derivat (50 mg, 0,129 mmol) wurde in siedendem Ethanol (3 ml) in Lösung gebracht. Citronensäure (27 mg, 0,14 mmol) wurde dann hinzugegeben. Die klare Lösung wurde in der Siedehitze 30 min gerührt. Anschließend wurde die Reaktionsmischung auf Raumtemperatur abgekühlt und 24 h bei dieser Temperatur stehen gelassen. Es fiel ein farbloser, mikrokristalliner Niederschlag aus. Dieser wurde abfiltriert und mit Ethanol (2 × 5 ml) gewaschen. Es wurden 52 mg (0,090 mmol; 69 %) des polareren Citrates (Schmp. 182-184 °C) erhalten.

**Beispiel 298: 1-(Dimethylamino)-3-(2-(4-(dimethylamino)-4-phenylcyclohex-1-enyl)-3-methyl-1H-indol-1-yl)propan-2-ol, Citrat (1:1) : Racemat**

Beispiel 296 (150 mg, 0,39 mmol) wurde in Ethanol (15 ml) suspendiert. Die Suspension wurde bei Raumtemperatur mit Dimethylamin (35 mg, 0,78 mmol; 33-proz. in Ethanol, 0,14 ml; 0,76 g/ml) versetzt. Da bei Raumtemperatur keine Reaktion einsetzte, wurde die Reaktionsmischung 10 h bei 59 °C (Ölbadtemperatur) gerührt. Anschließend wurde die Reaktionsmischung im Vakuum zur Trockne eingeengt. Der Aminoalkohol wurde leicht verunreinigt als hellgelbes Öl (120 mg, 0,26 mmol; 67 %) isoliert und so zur Citratbildung eingesetzt.
Zu einer Lösung des soeben hergestellten Aminoalkohols (110 mg, 0,26 mmol) in siedendem Ethanol (3 ml) wurde Citronensäure (54 mg, 0,28 mmol) hinzugegeben. Die klare Lösung wurde in der Siedehitze 30 min gerührt. Anschließend wurde die Reaktionsmischung auf Raumtemperatur abgekühlt. Es fiel ein hellgelber Feststoff aus. Dieser wurde abfiltriert und mit Ethanol (3 × 1 ml) gewaschen. Es wurden 48 mg (0,08 mmol; 30 %) des Citrats (Beispiel 298) (Smp. 170-173 °C) isoliert.

**Beispiel 299: 1-(Dimethylamino)-3-(2-(4-(dimethylamino)-4-phenylcyclohexyl)-3-methyl-1H-indol-1-yl)propan-2-ol, Citrat (1 :1) : Polareres Diastereomer**

Zu der freien Base des polareren Epoxids Beispiel 297 (160 mg, 0,41 mmol) in Ethanol (15 ml) wurde Dimethylamin (37 mg, 0,82 mmol; 33-proz. in Ethanol, 0,15 ml; 0,76 g/ml) bei Raumtemperatur gegeben. Da bei Raumtemperatur keine Reaktion einsetzte, wurde die Reaktionsmischung 4 h bei 59 °C (Ölbadtemperatur) und über Nacht bei Raumtemperatur gerührt. Anschließend wurde die Reaktionsmischung im Vakuum auf ca. 5 ml eingeengt. Es fiel ein farbloser Feststoff aus. Dieser wurde abfiltriert. Es wurden 132 mg (0,30 mmol; 74 %) des polareren Aminoalkohols isoliert.

Citrat Polar:

Zu einer Lösung des soeben isolierten polareren Aminoalkohols (112 mg, 0,26 mmol) in siedendem Ethanol (3 ml) wurde Citronensäure (55 mg, 0,284 mmol) hinzugegeben. Die klare Lösung wurde in der Siedehitze 30 min gerührt. Anschließend wurde die Reaktionsmischung auf Raumtemperatur abgekühlt und 24 h bei dieser Temperatur stehen gelassen. Es fiel schon aus dem warmen Ethanol ein farbloser mikrokristalliner Niederschlag aus. Dieser Niederschlag wurde abfiltriert und im Vakuum getrocknet. Es wurden 128 mg (0,21 mmol; 79 %) des polareren Citrates (Beispiel 299) isoliert.

**Beispiel 300: (±)-2-(3-(4-(Dimethylamino)-4-phenylcyclohex-1-enyl)-1H-indol-1-yl)ethanol Hydrochlorid**

Unter Schutzgas wurde das Keton **(Ket-10,** 217 mg, 1 mmol) zusammen mit 2-Indol-1-yl-ethanol **(Ind-107, 161** mg, 1 mmol) in absolutem Dichlormethan (10 ml) vorgelegt. Anschließend erfolgte die Zugabe von Trifluormethansulfonsäure-trimethylsilylester (0,2 ml, 1,03 mmol). Der Ansatz wurde 24 h bei RT gerührt. Zur Aufarbeitung wurde die klare lilafarbene Lösung mit 1N Natronlauge (10 ml) versetzt, worauf Entfärbung eintrat. Es wurde 30 min gerührt. Die Phasen wurden getrennt. Die wäßrige Phase wurde mit Dichlormethan (2 × 10 ml) extrahiert. Die vereinigten organischen Phasen wurden mit Wasser gewaschen, über $Na_2SO_4$ getrocknet und eingeengt. Das Produkt wurde durch Chromatographie an Kieselgel G [40 g; Methanol/$NH_3$ (500 : 1)] gereinigt. Es wurde das gewünschte Olefin als ein hellbraunes Öl in einer Ausbeute von 30 % (104 mg) erhalten.

Citrat:

Zu einer Lösung des soeben isolierten Olefins (104 mg, 0,3 mmol) in Ethylmethylketon (5 ml) wurde Chlortrimethylsilan (0,06 ml, 0,45 mmol) gegeben und 2 h bei Raumtemperatur gerührt. Das Reaktionsgemisch färbte sich rötlich. Das Hydrochlorid (Beispiel 300) wurde als lachsfarbener Feststoff in einer Ausbeute von 68 % (81 mg) mit einem Schmelzpunkt von 217-219 °C gewonnen.

$^{1}$*H NMR (400 MHz, DMSO-$d_6$)* δ ppm: 1.84-2.04 (m, 1H), 2.34-2.27 (m), 2.80-2.94 (m, 1H), 2.94-3.08 (m, 1H), 3.36-3.49 (m, 1H). 3.64 (t, J = 5.53 Hz, 2H), 4.01-4.20 (m, 2H), 4.81 (s, 1H), 6.10-6.21 (m, 1H), 6.98-7.07 (m, 1H), 7.07-7.17 (m, 1H), 7.26 (s, 1H), 7.35-7.56 (m, 4H), 7.69-7.75 (m, 1H), 7.75-7.85 (m, 2H), 10.64-10.88 (m, 1H)

**Beispiel 301: (±)-3-(4-(Dimethylamino)-4-phenylcyclohex-1-enyl)-(1-(phenylsulfonyl)-1H-indol) Hydrochlorid, Citrat (1:1)**

Das unter Beispiel **294** hergestellte Olefin N,N-dimethyl-1-phenyl-4-(1-(phenylsulfonyl)-1H-indol-2-yl)cyclohex-3-enamin (150 mg, 0,33 mmol) wurde in der Siedehitze in Ethanol (20 ml) gelöst und mit Citronensäure (65 mg, 0,34 mmol), gelöst in heißem Ethanol (3 ml), versetzt. Da auch nach Abkühlen des Ansatzes kein Niederschlag ausfiel, wurde das Lösungsmittel am Rotationsverdampfer eingeengt. Der Rückstand wurde in Isopropanol (12 ml) in der Siedehitze in Lösung gebracht. Beim Abkühlen fiel ein klebriger Niederschlag aus, der nach Anreiben und längerem Stehen kristallin wurde. Der Feststoff wurde mit Hilfe einer Fritte abgetrennt und anschließend getrocknet. Das gewünschte Produkt wurde so in einer Ausbeute von 171 mg (63 %, *Schmelzpunkt:* 198-200 °C) als Citrat (Beispiel **301)** erhalten.

$^{1}$*H NMR (400 MHz, DMSO-$d_6$)* δ ppm: 1.68-1.88 (m, 1H), 2.13-2.41 (m, 9H), 2.45- 2.76 (m, 4H), 2.67-2.76 (m, 1H), 2.93-3.06 (m, 1H), 6.37 (s, 1H), 7.21-7.45 (m, 5H), 7.49-7.61 (m, 4H), 7.61-7.69 (m, 2H), 7.72-7.80 (m, 1H), 7.89-8.01 (m, 3H)

$^{13}$C NMR (101 MHz, DMSO) δ ppm: 26.4,27.8,30.8,38.0,62.7,71.6 (s,1C)**,** 113.3, 121.2, 122.6, 123.0, 123.3, 123.7, 124.9, 126.6, 127.5, 127.9, 128.1, 128.9, 129.8, 134.6, 134.7, 136.8, 138.5, 171.2, 176.2

**Beispiel 302: (±)-2-(4-(Dimethylamino)-4-benzylcyclohex-1-enyl)-1H-indol Hydrochlorid**

**4-Benzyl-4-dimethylamino-1-(1-methyl-1*H*-indol-2-yl)cyclohexanol**

Eine Lösung von 2-Methylindol (500 mg, 3,81 mmol) in trockenem THF (20 ml) wird unter einem Argonstrom auf -5 °C gekühlt. Danach wird vorsichtig *tert*-Butyllithium (4,19 mmol, 2,47 ml einer 1,7M Pentanlösung) so zugetropft, daß dabei eine Reaktionstemperatur von 0 °C nicht überschritten wird. Nach beendeter Zugabe wird die Reaktionsmischung 2 h bei 0 °C gerührt. Im Anschluß wird eine Lösung von 4-Benzyl-4-dimethylaminocyclohexanon (**Ket-3,** 880 mg, 3,81 mmol) in trockenem THF (7 ml) bei 0 °C zugetropft. Die Mischung wird 15 min bei 0 °C und anschließend 4 h bei Raumtemperatur gerührt. Das Reaktionsgemisch wird mit gesättigter Ammoniumchloridlösung (20 ml) gequencht, die organische Phase abgetrennt und die wäßrige Phase viermal mit Methylenchlorid (20 ml) extrahiert. Die vereinigten organischen Phasen werden über Natriumsulfat getrocknet und danach das Lösungsmittel im Vakuum entfernt. Die Reinigung erfolgt mittels Flash-Chromatographie (Kieselgel, Cyclohexan/EtOAc 8 : 2). Es werden 456 mg (33 %) des gewünschten Cyclohexanols mit einem Schmelzpunkt von Fp = 105-107 °C erhalten. Es entsteht nur 1 von 2 möglichen Diastereoisomeren.

**[1-Benzyl-4-(1-methyl-1*H*-indol-2-yl)cyclohex-3-enyl]dimethylamin**

Eine Lösung des soeben hergestellten Cyclohexanols (500 mg, 1,53 mmol) in Bromwasserstoffsäure (5 ml, 48 %) wurde 15 min am Rückfluß erhitzt. Die abgekühlte Reaktionsmischung wurde mit 5N NaOH-Lösung auf einen pH-Wert von 9 eingestellt. Danach wurde die Mischung mit Dichlormethan (4 → 10 ml) extrahiert. Die vereinigten organischen Phasen wurden über Natriumsulfat getrocknet und danach das Lösungsmittel im Vakuum entfernt. Die Reinigung erfolgte mittels Flashchromatographie [Kieselgel, Cyclohexan/EtOAc (1: 1)]. Es wurden 230 mg (44 %) des gewünschten Olefins erhalten.

**[1-Benzyl-4-(1-methyl-1H-indol-2-yl)cyclohex-3-enyl]dimethylamin-hydro-chlorid (302)**

Zur Herstellung des Hydrochlorids wurde das soeben erhaltene Olefin (220 mg, 0,638 mmol) in Ethylmethylketon (5 ml) gelöst, mit Chlortrimethylsilan (105 mg, 0,96 mmol) versetzt und 1 h bei Raumtemperatur im offenen Reaktionsgefäß gerührt. Der dabei entstandene Feststoff wurde abgesaugt. Das Hydrochlorid **(302)** wurde so in einer Ausbeute von 160 mg (66 %) als weißer Feststoff mit einem Schmelzpunkt von 244-246 °C erhalten.

**Beispiel 303: N,N-Dimethyl-4-(3-(2-(pyridin-4-yl)ethyl)-1H-indol-2-yl)-1-(thiophen-2-yl)cyclohexanamin Hydrochlorid, unpolares Diastereomer**

**Beispiel 304: N,N-dimethyl-4-(3-(2-(pyridin-4-yl)ethyl)-1H-indol-2-yl)-1-(thiophen-2-yl)cyclohexanamin Hydrochlorid, polares Diastereomer**

**N,N-dimethyl-4-(3-(2-(pyridin-4-yl)ethyl)-1H-indol-2-yl)-1-(thiophen-2-yl)cyclohexanamin (unpolares und polares Diastereomer)**

Bsp. 251 (316 mg, 0.74 mmol) wurde in HBr/Eisessig (33 % HBr, 10 ml) suspendiert. Anschließend wurde zu dem Ansatz bei RT Sn-Pulver (439 mg, 3,7 mmol) innerhalb von 10 min portionsweise gegeben. Nach Beendigung der Zugabe wurde das Reaktionsgemisch 24 h bei RT gerührt. Dabei entstand eine klare Lösung. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (75 ml) basisch gestellt und mit Ethylacetat (70 ml) versetzt. Dieses Gemisch wurde 24 h bei Raumtemperatur gerührt. Die organische Phase wurde abgetrennt und die wäßrige Phase mit Ethylacetat (4 x 20 ml) extrahiert. Die vereinigten organischen Phasen wurden mit Wasser (50 ml) gewaschen, über $Na_2SO_4$ getrocknet und anschließend eingeengt. Das erhaltene Rohprodukt (220 mg) konnte durch Säulenchromatographie [Kieselgel 60 (30 g); Methanol (300 ml)] gereinigt werden. N,N-dimethyl-4-(3-(2-(pyridin-4-yl)ethyl)-1H-indol-2-yl)-1-(thiophen-2-yl)cyclohexanamin (unpolareres Diastereoisomer) wurde in einer Ausbeute von 60 mg (19 %) erhalten. Das polarere Diastereoisomer wurde in einer Ausbeute von 43 mg (13 %) isoliert.

**N,N-dimethyl-4-(3-(2-(pyridin-4-yl)ethyl)-1H-indol-2-yl)-1-(thiophen-2-yl)cyclohexanamin Hydrochlorid, unpolares Diastereomer**

N,N-dimethyl-4-(3-(2-(pyridin-4-yl)ethyl)-1H-indol-2-yl)-1-(thiophen-2-yl)cyclohexanamin (unpolareres Diastereoisomer) (60 mg, 0,13 mmol) wurde in Ethylacetat (20 ml) gelöst. Bei RT wurde dann $Me_3SiCl$ (25 μl, 0,2 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 303 (59 mg, Fp. 199-204 °C, Ausbeute 90 %) war ein weißer Feststoff.
$^1H$ NMR (400 MHz, RT, DMSO-$D_6$) δ ppm: 1.49 (psd, 2H), 2.08 (pst, 1H), 2.38 (psq, 2 H), 2.65 (psd, 6H), 2.90 (psd, 2H), 2.99 (psqt, 1H), 3.08 (pst, 4H), 6.94 (pst, 1H), 7.02 (pst, 1H), 7.22-7.27 (m, 2H), 7.48 (psd, 1H), 7.51 (psd, 1H), 7.76 (d, 2H), 7.82 (psd, 1H), 8.69 (d, 2H), 10.19 (s, br, 1H), 11.36 (s, 1H).

**N,N-dimethyl-4-(3-(2-(pyridin-4-yl)ethyl)-1H-indol-2-yl)-1-(thiophen-2-yl)cyclohexanamin Hydrochlorid, polares Diastereomer (304)**

N,N-dimethyl-4-(3-(2-(pyridin-4-yl)ethyl)-1H-indol-2-yl)-1-(thiophen-2-yl)cyclohexanamin (polareres Diastereoisomer) (43 mg, 0,1 mmol) wurde in Ethylacetat (20 ml) gelöst. Bei RT wurde dann $Me_3SiCl$ (19 μl, 0,15 mmol) zugetropft und 1 h gerührt. Es fiel ein weißer Niederschlag aus. Der Niederschlag wurde abgesaugt, mit Ethylacetat (2 x 5 ml) gewaschen und anschließend getrocknet. Beispiel 304 (40 mg, Fp. 188-191 °C, Ausbeute 87 %) war ein weißer Feststoff.
$^1$*H NMR (300 MHz, DMSO-$d_6$)* δ ppm: 1.47-1.67- (m, 4H), 2.17-2.37-(m, 2H), 2.51-2.57 (m, 6H), 2.57-2.65 (m, 1H), 2.70-2.83 (m, 2H), 2.95-3.14 (m, 4), 6.83-7.04 (m, 2H), 7.12-7.21 (m, 1H), 7.25-7.33 (m, 1H), 7.38-7.48- (m, 1H), 7.49-7.55 (m, 1H), 7.71-7.81 (m, 2H), 7.86-7.93 (m, 1H), 8.78 (d, J = 6.43 Hz, 2H), 10.57 (s, 1H), 11.33 (s, 1H)

**Beispiel 305: N-Methyl-4-(3-methyl-1H-indol-2-yl)-1-(4-methylthiazol-2-yl)cyclohexanamin, polares Diastereomer**

**Beispiel 306: N-methyl-4-(3-methyl-1H-indol-2-yl)-1-(4-methylthiazol-2-yl)cyclohexanamin, unpolares Diastereomer**

**(±)-2-(4-(Methylamino)-4-(4-methylthiazol-2-yl)cyclohex-1-enyl)-3-methyl-1H-indol**

3-Methylindol **(Ind-10,** 367 mg, 2,8 mmol) wurde zusammen mit dem Keton Ket-18 (750 mg, 3,3 mmol) in Dichlormethan (25 ml) gelöst und mit Trifluormethansulfonsäure (0,4 ml, 4,5 mmol) versetzt. Der Ansatz wurde 24 h bei RT gerührt. - Zur Aufarbeitung wurde das Reaktionsgemisch mit 2N NaOH (20 ml) versetzt und 20 min bei RT gerührt. Nach Abtrennung der organischen Phase wurde die wäßrige Phase mit Ethylacetat (2 x 20 ml) extrahiert. Die vereinigten organischen Extrakte wurden über $Na_2SO_4$ getrocknet und anschließend eingeengt. Das Rohprodukt ((±)-2-(4-(Methylamino)-4-(4-methylthiazol-2-yl)cyclohex-1-enyl)-3-methyl-1H-indol) wurde in einer Ausbeute von 940 mg (99 %) als gelbes Öl erhalten und wurde ohne weitere Reinigung in der nächsten Reaktion eingesetzt.

**N-Methyl-4-(3-methyl-1H-indol-2-yl)-1-(4-methylthiazol-2-yl)cyclohexanamin,** polares **Diastereomer (305) und N-methyl-4-(3-methyl-1H-indol-2-yl)-1-(4-methylthiazol-2-yl)cyclohexanamin, unpolares Diastereomer( 306)**

(±)-2-(4-(Methylamino)-4-(4-methylthiazol-2-yl)cyclohex-1-enyl)-3-methyl-1H-indol (935 mg. 2,7 mmol) wurde in HBr/Eisessig (33 % HBr, 40 ml) suspendiert. Anschließend wurde zu dem Ansatz bei RT Sn-Pulver (1,6 g, 13,8 mmol) innerhalb von 30 min portionsweise gegeben. Nach Beendigung der Zugabe wurde das Reaktionsgemisch weitere 24 h bei RT gerührt. - Zur Aufarbeitung wurde das Gemisch mit EtOH (20 ml) verdünnt und am Rotationsverdampfer bis zur Trockene eingeengt. Der verbliebene Rückstand wurde durch Zugabe von 5N NaOH (100 ml) basisch gemacht und 18 h mit Ethylacetat (30 ml) bei Raumtemperatur gerührt. Die Phasen wurden getrennt. Die wässrige Phase wurde mit Ethylacetat (4 × 20 ml) extrahiert. Die vereinigten organischen Phasen wurden mit Wasser (30 ml) gewaschen, über $Na_2SO_4$ getrocknet und anschließend eingeengt. Der erhaltene Rückstand (1 g) wurde durch Säulenchromatographie [Kieselgel 60 (70 g); EtOAc (400 ml), Methanol (400 ml)] gereinigt. Beispiel 305 wurde in einer Ausbeute von 416 mg (30 %,) als gelbes Öl erhalten. Beispiel 306 wurde in einer Ausbeute von 249 mg (18 %,) als gelbes Öl erhalten.
$^1$*H NMR (300 MHz, DMSO-d$_6$)* δ ppm: 1.66-1.87 (m, 3H), 1.89-2.01 (m, 3H), 2.15 (s, 3H), 2.17 (s, 3H), 2.31-2.39 (m, 1H), 2.42-2.47 (m, 3H), 2.56-2.72 (m, 2H), 2.82-2.98- (m, 1H), 6.83-7.01 (m, 2H), 7.08-7.24 (m, 1H), 7.34 (d, *J* = 7.22 Hz, 1H), 7.47 (s, 1H), 10.45 (s, 1H)

$^1$*H NMR (400 MHz, DMSO-d$_6$)* δ ppm: 1.60-1.67 (m, 2H), 1.91-2.05 (m, 6H), 2.19 (S, 3H), 2.20 (s, 3H), 2.34 (s, 3H), 2.52-2.62 (m, 1H), 2.85-297 (m, 1H), 6.89-6.95 (m, 1H), 6.96-7.01 (m, 1H), 7.08-7.12 (m, 1H), 7.24-7.31 (m, 1H), 7.35-7.38 (m, 1H) 10.55 (s, 1H)
$^{13}$*C NMR (101 MHz, DMSO) δ ppm:* 8.3, 17.0, 26.9, 28.9, 34.1, 35.1, 58.6, 103.6, 110.4, 113.8, 117.4, 117.8, 119.9, 128.6, 135.1, 139.8, 151.2, 180.2

**Beispiel 307: 2-(2-(4-(Dimethylamino)-4-phenylcyclohexyl)-1H-indol-3-yl)ethanol, Diastereomerengemisch**

**2-(2-(4-(Dimethylamino)-4-phenylcyclohexyl)-1H-indol-3-yl)ethanol, Diastereomerengemisch (219b)**

**Ket-10** (8.8 g, 40.48 mmol) und **Ind-5** (6.52 g, 40.48 mmol) wurden in abs. Dichlormethan (250 ml) bei 0 °C vorgelegt. Anschließend wurde Trifluormethansulfonsäuresilylester (8.76 ml, 44.52 mmol) in abs. Dichlormethan (10 ml) rasch zugegeben und 20 min unter Eiskühlung gerührt. Der Ansatz wurde über Nacht bei RT gerührt. Zur Aufarbeitung wurde das Reaktionsgemisch mit 1N NaOH (120 ml) versetzt und 10 min bei RT gerührt, wobei der Ansatz sich gelb färbte und ein Niederschlag ausfiel. Unter Eiskühlung wurde 20 min weiter gerührt, der ausgefallene Niederschlag abgesaugt, noch mal in Ethanol aufgeschlemmt, abgesaugt und i. Vak. getrocknet.
Dieses Gemisch wurde zur weiteren Synthese eingesetzt.
*Ausbeute:* 8.63 g (59 %, 1:1 Gemisch aus 2-(2-(4-(dimethylamino)-4-phenylcyclohex-1-enyl)-1H-indol-3-yl)ethanol und N,N-dimethyl-4-phenyl-4',9'-dihydro-3'H-spiro[cyclohexan-1,1'-pyrano[3,4-b]indol]-4-amin) Das Gemisch (8.63 g. 23.88 mmol) wurde in konz. HCl (730 ml) vorgelegt und über Nacht bei RT gerührt. Anschließend wurde Zinn (35.1 g, 303.33 mmol) innerhalb von 2 h portionsweise zugegeben (dabei fiel immer ein Niederschlag aus, der sich nach längerem Rühren wieder löste, und zum Ende der Zugabe änderte sich die Farbe von Rot/Orange nach Grau) und 2.5 h bei RT nachgerührt. Zur Aufarbeitung wurde der Ansatz unter Eiskühlung mit 5N NaOH (ca. 1500 ml) versetzt und 10 min bei RT gerührt, wobei ein weißer Niederschlag ausfiel. Der Niederschlag wurde über Celite abgesaugt und mit $CH_2Cl_2$ (2 × 50 ml) nachgewaschen. Die Phasen wurden getrennt. Die wässrige Phase wurde mit $CH_2Cl_2$ (3 x 100 ml) extrahiert. Die organische Phase wurde über $Na_2SO_4$ getrocknet und i. Vak. eingeengt (nur 412 mg Produkt isoliert). Die Celite wurde mit $CH_2Cl_2$ (2 x 100 ml) und mit Ethanol ausgekocht (3 x 100 ml) und anschließend abfiltriert. Die Filtrat wurden i. Vak. eingeengt. Der feste Rückstand wurde aus Toluol umkristallisiert.
Ausbeute **(307)**: 4.29 g (49 %, Diastereomerengemisch); *Schmelzpunkt:* 205-210 °C

Diastereomer 1:

$^1$H-NMR (DMSO-d$_6$): 1.49 (2 H, m); 1.62 (2 H, m); 2.03 (6 H, s); 2.15 (2 H, m); 2.81 (4 H; m); 3.49 (2 H, m); 4.57 (1 H, t, OH); 6.90 (2 H, m); 7.28 (2 H, m); 7.38 (5 H, m); 10.64 (1 H, s). Diastereomer 2:$^1$H-NMR (DMSO-d$_6$): 1.48 (2 H, m); 1.80 (2 H, m); 1.96 (2 H, m); 2.38 (6 H, s); 2.56-2.64 (4 H, m); 2.81 (2 H; m); 2.96 (2 H, m); 3.49 (2 H, m); 6.89 (2 H, m); 7.14 (1 H, m); 7.35 (1 H, m); 7.51 (5 H, m); 10.35 (1 H, s), Citrat.

Die folgenden Beispiele wurden über HPLC-MS-Analytik identifiziert:

| Beispiel | MS-Peak |
|---|---|
| **1** | 363.2 |
| **3** | 403.2 |
| **6** | 375.2 |
| **8** | 403.2 |
| **9** | 428.1 |
| **10** | 379.2 |
| **16** | 286.3 |
| **17** | 333.3 |
| **18** | 288.3 |
| **22** | 370.2 |
| **23** | 311.2 |
| **24** | 379.2 |
| **25** | 399.2 |
| **26** | 363.2 |
| **27** | 329.3 |
| **28** | 304.2 |
| **29** | 316.2 |
| **30** | 345.3 |
| **31** | 311.3 |
| **33** | 371.2 |
| **34** | 337.3 |
| **35** | 312.3 |
| **36** | 427.2 |
| **37** | 421.2 |
| **38** | 393.3 |
| **39** | 387.3 |
| **40** | 368.2 |
| **41** | 407.2 |
| **42** | 373.2 |
| **43** | 339.3 |
| **44** | 314.3 |
| **45** | 298.2 |
| **48** | 389.2 |
| **49** | 355.3 |
| **51** | 490.2 |
| **62** | 440.3 |
| **63** | 402.2 |
| **64** | 438.3 |

(fortgesetzt)

| Beispiel | MS-Peak |
|---|---|
| **65** | 409.2 |
| **66** | 448.3 |
| **67** | 405.2 |
| **68** | 372.2 |
| **69** | 372.2 |
| **70** | 338.3 |
| **71** | 338.3 |
| **72** | 358.2 |
| **73** | 415.2 |
| **74** | 415.2 |
| **75** | 381.2 |
| **76** | 401.2 |
| **77** | 365.2 |
| **78** | 365.2 |
| **79** | 331.3 |
| **80** | 351.2 |
| **81** | 351.3 |
| **120** | 431.2 |
| **121** | 397.2 |
| **122** | 397.2 |
| **123** | 417.2 |
| **124** | 417.2 |
| **125** | 343.3 |
| **126** | 343.3 |
| **127** | 363.2 |
| **128** | 318.2 |
| **129** | 347.3 |
| **130** | 347.3 |
| **131** | 313.3 |
| **132** | 313.3 |
| **133** | 373.2 |
| **134** | 373.2 |
| **135** | 339.3 |
| **136** | 314.3 |
| **139** | 429.3 |
| **140** | 429.2 |
| **141** | 423.2 |
| **142** | 423.2 |

(fortgesetzt)

| Beispiel | MS-Peak |
|---|---|
| **143** | 395.3 |
| **144** | 395.3 |
| **145** | 389.3 |
| **146** | 389.3 |
| **147** | 415.3 |
| **148** | 415.3 |
| **149** | 409.2 |
| **150** | 409 |
| **153** | 391.2 |
| **156** | 375.2 |
| **157** | 341.3 |
| **158** | 341.3 |
| **159** | 361.3 |
| **160** | 316.3 |
| **164** | 359.3 |
| **171** | 419.2 |
| **172** | 405.2 |
| **173** | 433.2 |
| **174** | 447.2 |
| **175** | 447.2 |
| **176** | 413.3 |
| **177** | 41.3 |
| **178** | 433.3 |
| **179** | 433.2 |
| **180** | 391.2 |
| **181** | 357.3 |
| **182** | 357.3 |
| **183** | 377.3 |
| **184** | 377.3 |
| **219** | 363.2 |
| **220** | 405.2 |
| **223** | 289.3 |
| **224** | 289.3 |
| **225** | 289.3 |
| **226** | 334.3 |
| **227** | 274.3 |
| **231** | 272.3 |
| **232** | 272.3 |

(fortgesetzt)

| Beispiel | MS-Peak |
|---|---|
| **233** | 378.2 |
| **234** | 379.1 |
| **235** | 274.3 |
| **236** | 274.3 |
| **239** | 332.3 |
| **240** | 287.3 |
| **241** | 333.2 |
| **242** | 303.2 |
| **243** | 350.2 |

**Pharmakologische Untersuchungen:**

**Messung der ORL1-Bindung**

Die erfindungsgemäßen Verbindngen wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 $\mu$g Membranprotein je 200 $\mu$l Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer $K_i$-Wert in oder % Inhibition bei c=1 $\mu$M angegeben.

**Messung der $\mu$-Bindung**

Die Rezeptoraffinität zum humanen $\mu$-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 $\mu$g Protein pro 250 $\mu$l Inkubationsansatz) von CHO-K1-Zellen, welche den humanen $\mu$-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 $\mu$l für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 $\mu$mol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen $\mu$-Opiatrezeptor bei einer Konzentration der Prüfsubstanzen von 1 $\mu$mol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

**Analgesieprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc. Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet

und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

**Analgesieprüfung im Tail-Flick-Test an der Ratte**

Die analgetische Wirksamkeit der Testverbindungen wurde im Brennstrahl (Tail-flick)-Test an der Ratte nach der Methode von D'Amour and Smith (J. Pharm. Exp. Ther. 72, 74 79 (1941) untersucht. Dazu wurden weibliche Sprague Dawley mit einem Gewicht zwischen 134 und 189 g verwendet. Die Tiere wurden einzeln in spezielle Testkäfige gesetzt und die Schwanzbasis einem focussierten Wärmestrahl einer Lampe (Tail-flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/(T_2 - T_0)] \times 100$$

Dabei ist die $T_0$ die Latenzzeit vor und $T_1$ die Latenzzeit nach Substanzapplikation, $T_2$ ist die maximale Expositionszeit (12 sec).

Zur Bestimmung der Dosisabhängigkeit wurde die jeweilige Testverbindung in 3 - 5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum, 20 Minuten nach intravenöser Substanzgabe.

Beispielhaft wurden folgende Werte bestimmt:

| **Beispiel** | **%Hemmung (ORL1) bei 1 μM oder Ki [μM]** | **%Hemmung (μ) bei 1 μM oder Ki [μM]** | **Tail-flick, i.v.** |
|---|---|---|---|
| **1** | 0.0012 | 0.0044 | 99% MPE bei 100μg/kg (Maus) |
| **3** | 58% | 0.1100 | |
| **4** | 0.0200 | 0.0092 | |
| **6** | 66% | 0.0240 | |
| **7** | 2 | 40% | |
| **8** | - | - | |
| **9** | 39% | 0.0370 | |
| **10** | 37% | 0.0330 | |
| **11** | 49% | 0.3200 | |
| **13** | 10% | 23% | |
| **14** | 3 | 0.0360 | |
| **15** | - | 64% | |
| **16** | 16% | 51% | |
| **17** | 0.0009 | 0.0004 | 79% MPE bei 100 μg/kg (Maus) |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **18** | 0.0140 | 0.0130 | |
| **19** | 70% | 89% | |
| **20** | 100% | 102% | |
| **21** | 53% | 60% | |
| **22** | - | 62% | |
| **23** | 87% | 96% | |
| **24** | 73% | 0.1400 | |
| **25** | 27% | 53% | |
| **26** | - | 61% | |
| **27** | 58% | 93% | |
| **28** | 12% | 39% | |
| **29** | 35% | 58% | |
| **30** | 75% | 89% | |
| **31** | 96% | 100% | |
| **32** | 35% | 60% | |
| **33** | - | 30% | |
| **34** | 26% | 50% | |
| **35** | 17% | 41% | |
| **36** | - | 20% | |
| **37** | - | 13% | |
| **38** | 11% | - | |
| **39** | 55% | 78% | |
| **40** | - | - | |
| **41** | 29% | 40% | |
| **42** | 50% | 73% | |
| **43** | 48% | 0.5300 | |
| **44** | 17% | 37% | |
| **45** | 16% | 97% | |
| **46** | 78% | 101% | |
| **47** | 62% | 92% | |
| **48** | 64% | 95% | |
| **49** | 74% | 98% | |
| **51** | 36% | 61% | |
| **52** | 35% | 100% | |
| **53** | 88% | 100% | |
| **54** | 89% | 95% | |
| **55** | 31% | 90% | |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **56** | 80% | 98% | |
| **57** | 66% | 83% | |
| **58** | 90% | 92% | |
| **59** | 84% | 96% | |
| **60** | 52% | 97% | |
| **61** | 52% | 86% | |
| **62** | 78% | 96% | |
| **63** | 79% | 98% | |
| **64** | 63% | 99% | |
| **65** | 65% | 98% | |
| **66** | - | 21% | |
| **67** | 46% | 98% | |
| **68** | 13% | 83% | |
| **69** | 46% | 98% | |
| **70** | 68% | 97% | |
| **71** | 10% | 50% | |
| **72** | 38% | 54% | |
| **73** | 18% | 60% | |
| **74** | 57% | 67% | |
| **75** | - | 21% | |
| **76** | 18% | 24% | |
| **77** | - | 75% | |
| **78** | 55% | 90% | |
| **79** | 92% | 102% | |
| **80** | 94% | 0.0012 | |
| **81** | 24% | 0.5600 | |
| **82** | 38% | 74% | |
| **83** | 102% | 97% | |
| **84** | - | - | |
| **85** | 81% | 100% | |
| **86** | 32% | 68% | |
| **87** | 99% | 100% | |
| **88** | 19% | 41% | |
| **89** | 98% | 100% | |
| **90** | 27% | 36% | |
| **91** | 89% | 100% | |
| **92** | 26% | 64% | |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **93** | 99% | 99% | |
| **94** | 91% | 98% | |
| **95** | 100% | 101% | |
| **96** | 87% | 91% | |
| **97** | 57% | 98% | |
| **98** | 19% | 48% | |
| **99** | 98% | 100% | |
| **100** | 10% | 34% | |
| **101** | 99% | 99% | |
| **102** | 50% | 57% | |
| **103** | 80% | 99% | |
| **104** | - | 42% | |
| **105** | 99% | 100% | |
| **106** | 14% | 38% | |
| **107** | 99% | 98% | |
| **108** | 37% | 43% | |
| **109** | 62% | 99% | |
| **110** | 23% | 53% | |
| **111** | 101% | 100% | |
| **112** | 72% | 98% | |
| **113** | 55% | 83% | |
| **114** | 99% | 100% | |
| **115** | 38% | 62% | |
| **116** | 99% | 100% | |
| **117** | 42% | 55% | |
| **118** | 87% | 94% | |
| **119** | 19% | 56% | |
| **120** | - | - | |
| **121** | 30% | 94% | |
| **122** | - | 16% | |
| **123** | 29% | 77% | |
| **124** | 67% | 65% | |
| **125** | 69% | 97% | |
| **126** | - | 61% | |
| **127** | 70% | 94% | |
| **128** | 27% | 38% | |
| **129** | 15% | 51% | |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **130** | 68% | 93% | |
| **131** | 97% | 99% | |
| **132** | 62% | 85% | |
| **133** | 63% | 83% | |
| **134** | 18% | 35% | |
| **135** | 18% | 27% | |
| **136** | 32% | 28% | |
| **137** | 98% | 99% | |
| **138** | 52% | 72% | |
| **139** | - | 10% | |
| **140** | 11% | 21% | |
| **141** | 35% | 37% | |
| **142** | 28% | 47% | |
| **143** | 61% | 79% | |
| **144** | - | 12% | |
| **145** | 95% | 95% | |
| **146** | 62% | 87% | |
| **147** | 73% | 87% | |
| **148** | 18% | 29% | |
| **149** | 93% | 94% | |
| **150** | 27% | 26% | |
| **151** | 98% | 100% | |
| **152** | 76% | 92% | |
| **153** | 84% | 99% | |
| **156** | 11% | 41% | |
| **157** | - | 76% | |
| **158** | 17% | 54% | |
| **159** | 95% | 98% | |
| **160** | - | 29% | |
| **161** | 77% | 98% | |
| **162** | 87% | 100% | |
| **163** | 100% | 102% | |
| **164** | 84% | 99% | |
| **165** | 99% | 101% | |
| **166** | 98% | 100% | |
| **167** | 99% | 99% | |
| **168** | 45% | 84% | |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **169** | 99% | 101% | 100% MPE bei 100 μg/kg (Ratte) |
| **170** | 88% | 95% | |
| **171** | 36% | 88% | |
| **172** | 83% | 99% | |
| **173** | 99% | 100% | |
| **174** | 55% | 90% | |
| **175** | 67% | 96% | |
| **176** | 66% | 81% | |
| **177** | 99% | 100% | |
| **178** | 99% | 95% | |
| **179** | 38% | 66% | |
| **180** | 86% | 98% | |
| **181** | 64% | 91% | |
| **182** | 99% | 101% | 100% MPE bei 100μg/kg (Ratte) |
| **183** | 79% | 82% | |
| **184** | 99% | 101% | |
| **185** | 69% | 65% | |
| **186** | 98% | 101% | |
| **187** | 100% | 100% | |
| **188** | 41% | 68% | |
| **189** | 100% | 98% | |
| **190** | 76% | 87% | |
| **191** | 99% | 100% | |
| **192** | 71% | 86% | |
| **193** | 98% | 101% | |
| **194** | 96% | 96% | |
| **195** | 100% | 101% | |
| **196** | 95% | 99% | |
| **197** | 100% | 100% | |
| **198** | 78% | 80% | |
| **199** | 99% | 95% | |
| **200** | 99% | 0.0003 | |
| **201** | 100% | 0.0003 | |
| **202** | 72% | 0.1100 | |
| **203** | 99% | 101% | 96% MPE bei 100 μg/kg (Ratte) |
| **204** | 87% | 98% | |
| **205** | 99% | 101% | 100% MPE bei 100 μg/kg (Ratte) |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **206** | 87% | 94% | |
| **207** | 99% | 0.0003 | 100% MPE bei 10μg/kg (Ratte) |
| **208** | 56% | 0.4500 | |
| **209** | 100% | 99% | |
| **210** | 63% | 65% | |
| **211** | 100% | 102% | |
| **212** | 73% | 65% | |
| **213** | 99% | 100% | |
| **214** | 82% | 89% | |
| **215** | 100% | 102% | |
| **216** | 72% | 89% | |
| **217** | 99% | 100% | |
| **218** | 43% | 43% | |
| **219** | 54% | 60% | |
| **220** | 23% | 62% | |
| **221** | 81% | 97% | |
| **222** | 35% | 88% | |
| **223** | 80% | 95% | |
| **224** | 31% | 59% | |
| **225** | 15% | 26% | |
| **226** | 40% | 89% | |
| **227** | 50% | 67% | |
| **228** | 32% | 80% | |
| **229** | 12% | 49% | |
| **230** | 37% | 72% | |
| **231** | 16% | 13% | |
| **232** | 27% | n. d. | |
| **233** | 65% | 0.2100 | |
| **234** | - | 31% | |
| **235** | 47% | 65% | |
| **236** | 96% | 101% | |
| **237** | 22% | 36% | |
| **238** | 74% | 64% | |
| **239** | - | n. d. | |
| **240** | - | 20% | |
| **241** | 56% | 0.0320 | |
| **242** | - | 42% | |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **243** | - | 14% | |
| **244** | 39% | 62% | |
| **246** | 95% | 97% | |
| **247** | 95% | 97% | |
| **248** | 67% | 91% | |
| **249** | 63% | 97% | |
| **250** | 15% | 54% | |
| **251** | n. d. | 92% | |
| **252** | n. d. | 67% | |
| **253** | n. d. | 35% | |
| **254** | n. d. | 85% | |
| **255** | n. d. | 64% | |
| **256** | n. d. | 99% | |
| **257** | n. d. | 29% | |
| **258** | n. d. | 20% | |
| **259** | 91% | 101% | |
| **260** | 68% | 73% | |
| **261** | 94% | 99% | |
| **262** | 23% | 11% | |
| **263** | 40% | 38% | |
| **264** | - | 35% | |
| **265** | - | 58% | |
| **266** | 73% | 97% | |
| **267** | 58% | 78% | |
| **268** | 77% | 79% | |
| **269** | 94% | 97% | |
| **270** | 20% | 49% | |
| **271** | 90% | 97% | |
| **272** | - | 15% | |
| **273** | 67% | 88% | |
| **274** | 57% | 57% | |
| **294** | 13% | 67% | |
| **295** | 21% | 25% | |
| **296** | 29% | 64% | |
| **297** | 14% | 42% | |
| **298** | 23% | 43% | |
| **299** | 24% | 52% | |

(fortgesetzt)

| Beispiel | %Hemmung (ORL1) bei 1 μM oder Ki [μM] | %Hemmung (μ) bei 1 μM oder Ki [μM] | Tail-flick, i.v. |
|---|---|---|---|
| **300** | 20% | 0.8100 | |
| **301** | 32% | 18% | |
| **302** | 0.1300 | 0.1033 | |
| **307** | 99% | 99% | |

**Parenterale Lösung eines erfindungsgemäßen spirocyclischen-Derivats**

3 g eines der erfindungsgemäßen substituierten Indol-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte Heteroaryl-Derivate der allgemeinen Formel I,

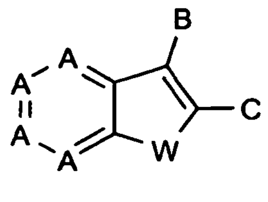

I

worin

A für N oder $CR^{7-10}$ steht, wobei A höchstens zweimal für N steht,
W für O, S oder $NR^4$ steht,
mit der Maßgabe, dass wenn W für O oder S steht, A $CR^{7-10}$ bedeutet;
einer der Reste B oder C für H; $C_{1-8}$-Alkyl jeweils gesättigt oder ungesättigt, verzweigt oder unverzweigt, einfach oder mehrfach substituiert oder unsubstituiert, $COR^{12}$; $SO_2R^{12}$; über $C_{1-3}$-Alkyl gebundenes Aryl, $C_{3-8}$-Cycloalkyl oder Heteroaryl, jeweils einfach oder mehrfach substituiert oder unsubstituiert, Aryl-, oder Heteroaryl, jeweils einfach oder mehrfach substituiert oder unsubstituiert; $C_{3-8}$-Cycloalkyl, jeweils einfach oder mehrfach substituiert oder unsubstituiert; steht, und der jeweils andere Rest B oder C für

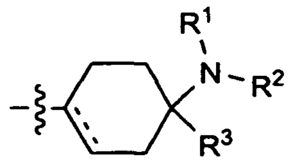

steht, wobei

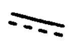

für eine Einfachbindung oder eine Doppelbindung steht

$R^1$ und $R^2$, unabhängig voneinander für H; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils einfach oder mehrfach

substituiert oder unsubstituiert; Aryl oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{11}CH_2CH_2$ oder $(CH_2)_{3-6}$ stehen,

wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; C(O)Phenyl, C(O)Heteroaryl, C(O)$C_{1-5}$-Alkyl, jeweils substituiert oder unsubstituiert, bedeutet;

und

$R^3$ für $C_{1-8}$-Alkyl, jeweils gesättigt oder ungesättigt, verzweigt oder unverzweigt, einfach oder mehrfach substituiert oder unsubstituiert; $C_{3-8}$-Cycloalkyl, jeweils einfach oder mehrfach substituiert oder unsubstituiert; Aryl oder Heteroaryl, jeweils unsubstituiert oder einfach oder mehrfach substituiert; über $C_{1-3}$-Alkyl-Gruppe gebundenes Aryl, Heteroaryl oder $C_{3-8}$-Cycloalkyl, jeweils unsubstituiert oder einfach oder mehrfach substituiert, steht

$R^4$ für H; $C_{1-5}$-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$-Alkyl-Gruppe gebundenes Aryl, Heteroaryl oder Cycloalkyl, jeweils einfach oder mehrfach substituiert oder unsubstituiert; $COR^{12}$ ; $SO_2R^{12}$ steht,

wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $OR^{13}$; $NR^{14}R^{15}$ bedeutet;

$R^7$, $R^8$, $R^9$ und $R^{10}$ unabhängig voneinander für

H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$ NHC(O)$NHR^{13}$, NHC(O)$R^{13}$, NH($CNR^{13}$)$NHR^{13}$, $SO_2NHR^{13}$; $C_{1-5}$-Alkyl, $C_{3-8}$-Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, stehen;

wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeutet;

oder $R^7$, $R^8$ und $R^9$ die oben angegebene Bedeutung haben und $R^{10}$ mit B zusammen für - $CH_2CH_2CH_2$- steht und $R^{10}$ und B somit einen sechsgliedrigen Ring bilden,

$R^{14}$ und $R^{15}$ unabhängig voneinander H; $C_{1-5}$-Alkyl, jeweils gesättigt oder ungesättigt, verzweigt oder unverzweigt, unsubstituiert oder einfach oder mehrfach substituiert; oder $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeuten;

oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$-Alkyl gesättigt oder ungesättigt, verzweigt oder unverzweigt, unsubstituiert oder einfach oder mehrfach substituiert, bedeutet;

worin der Ausdruck "Cycloalkyl" oder "$C_{3-8}$-Cycloalkyl" für cyclische Kohlenwasserstoffe mit 3, 4, 5, 6, 7 oder 8 Kohlenstoffatomen steht, wobei die Kohlenwasserstoffe gesättigt oder ungesättigt (aber nicht aromatisch), unsubstituiert oder ein- oder mehrfach substituiert sein können, und wobei auch gesättigte oder ungesättigte (aber nicht aromatische) Cycloalkyle, in denen ein oder zwei Kohlenstoffatome durch ein Heteroatom S, N oder O ersetzt sind, umfasst sind, und wobei ein Cycloalkyl-Ring auch mit einem weiteren Ring kondensiert sein kann, der gesättigt oder ungesättigt (auch aromatisch) sein kann,

worin der Ausdruck "Aryl" für aromatische Kohlenwasserstoffe steht, diese Aryl-Reste auch mit weiteren gesättigten, (partiell) ungesättigten oder aromatischen Ringsystemen kondensiert sein können, und jeder dieser Aryl-Reste unsubstituiert oder einfach oder mehrfach substituiert sein kann, wobei die Aryl-Substituenten gleich oder verschieden und in jeder beliebigen und möglichen Position des Aryls sein können,

worin der Ausdruck "Heteroaryl" für einen 5-, 6- oder 7-gliedrigen cyclischen aromatischen Rest steht, der mindestens 1, ggf. auch 2, 3, 4 oder 5 Heteroatome ausgewählt aus der Gruppe bestehend aus Stickstoff, Sauerstoff und Schwefel enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls

sein; und wobei der Heterocyclus auch Teil eines bi- oder polycyclischen Systems sein kann, worin die vorstehend genannten $C_{1-8}$-Alkyle. $C_{1-5}$-Alkyle, $C_{1-3}$-Alkyle bzw. $C_{1-3}$-Alkylene bzw. $C_{3-8}$-Cycloalkylreste jeweils einfach oder mehrfach mit F, Cl, Br, I, -CN, $NH_2$, NH-$C_{1-6}$-Alkyl, NH-$C_{1-6}$-Alkyl-OH, N($C_{1-6}$-Alkyl)$_2$, N($C_{1-6}$-Alkyl-OH)$_2$, $NO_2$, SH, S-$C_{1-6}$-Alkyl, S-Benzyl, $OCF_3$, O-$C_{1-6}$-Alkyl, OH, O-$C_{1-6}$-Alkyl-OH, =O, $C_{1-6}$-Alkyl, Benzyl, O-Benzyl, O-Phenyl, C(=O)$C_{1-6}$-Alkyl, $CO_2H$, NHC(=O)$C_{1-6}$-Alkyl, OC(=O)$C_{1-6}$-Alkyl, $CO_2$-$C_{1-6}$-Alkyl, substituiert sein können,
worin die vorstehend genannten Aryl- oder Heteroaryl-Reste jeweils einfach oder mehrfach mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, $CO_2H$, $CO_2$-$C_{1-6}$-Alkyl, $CF_3$, $OCF_3$, $C_{1-6}$-Alkyl oder Phenoxy substituiert sein können,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

**2.** Heteroaryl-Derivate gemäß Anspruch 1, worin

$R^1$ und $R^2$ unabhängig voneinander für H; $C_{1-5}$-Alkyl, gesättigt oder ungesättigt, verzweigt oder unverzweigt, einfach oder mehrfach substituiert oder unsubstituiert, stehen;
oder die Reste $R^1$ und $R^2$ zusammen einen Ring bilden und $CH_2CH_2OCH_2CH_2$, $CH_2CH_2NR^{11}CH_2CH_2$ oder $(CH_2)_{3-6}$ bedeuten,

wobei $R^{11}$ H; $C_{1-5}$-Alkyl, gesättigt oder ungesättigt, verzweigt oder unverzweigt, einfach oder mehrfach substituiert oder unsubstituiert, bedeutet.

**3.** Heteroaryl-Derivate gemäß Anspruch 1 oder 2, worin

$R^1$ und $R^2$ unabhängig voneinander für $CH_3$ oder H stehen, wobei $R^1$ und $R^2$ nicht gleichzeitig H bedeuten.

**4.** Substituierte Heteroaryl-Derivate gemäß Anspruch 1, worin

$R^3$ Butyl, Phenyl, Thiophenyl, Thiazolyl, Cyclopentyl, Cyclohexyl, Naphthyl, Benzyl, Benzofuranyl, 1,2,4-Triazolyl, Benzimidazolyl, Benzodioxanyl, Benzodioxolanyl, Pyridyl oder Benzothiophenyl, jeweils unsubstituiert oder einfach oder mehrfach substituiert; über eine gesättigte, unverzweigte $C_{1-3}$-Alkyl-Gruppe gebundenes Phenyl, Furyl oder Thiophenyl, jeweils unsubstituiert oder einfach oder mehrfach substituiert, bedeutet.

**5.** Substituierte Heteroaryl-Derivate gemäß Anspruch 4, worin

$R^3$ Phenyl, 4-Fluorphenyl, Benzyl, Butyl oder Benzothiophenyl bedeutet.

**6.** Substituierte Heteroaryl-Derivate gemäß Anspruch 1, worin

B oder C für $(CH_2)_{1-4}$-$R^{21}$ steht, wobei $R^{21}$ für H, OH, SH, COO$C_{1-6}$-Alkyl, COOH, OC(=O)$C_{1-6}$-Alkyl, $NH_2$, NHC (=O)$C_{1-6}$-Alkyl; oder $C_{3-8}$-Cycloalkyl, Aryl oder Heteroaryl, jeweils unsubstituiert oder einfach oder mehrfach substituiert, steht.

**7.** Substituierte Heteroaryl-Derivate gemäß Anspruch 6, worin

$R^{21}$ für OH, SH, $COOCH_3$, COOH, OC(=O)$CH_3$, $NH_2$, NHC(=O)$CH_3$, NHC(=O)$CH_2C(CH_3)_3$; oder Benzimidazol, Pyridyl, Triazolyl, Phenyl, Pyrazolyl, Tetrazolyl oder Imidazolyl, jeweils unsubstituiert oder substituiert mit $COOCH_3$, $CH_3$; oder Cyclopropyl, Cyclohexyl, Pyrrolidinyl, Tetrahydrochinolinyl, Pyrrolidinyl, Piperidyl, Tetrahydroisochinolinyl, Isoindolinyl, Piperazinyl, Morpholinyl oder Thiazolinyl jeweils unsubstituiert oder substituiert mit =O oder $CH_3$, steht.

**8.** Substituierte Heteroaryl-Derivate gemäß Anspruch 1, worin

$R^7$, $R^8$, $R^9$ und $R^{10}$ unabhängig voneinander für H; Methyl; Ethyl; Propyl; Butyl; Pyridyl, O-Benzyl, F, Cl, Br, I, CN, $CF_3$, $OCF_3$, OH, $OCH_3$, $NH_2$, COOH, $COOCH_3$, $NHCH_3$ oder $N(CH_3)_2$ oder $NO_2$ stehen.

**9.** Substituierte Heteroaryl-Derivate gemäß Anspruch 8, worin

$R^7$, $R^8$, $R^9$ und $R^{10}$ unabhängig voneinander für H, F, Cl, $NO_2$, CN, $CF_3$, $OCH_3$, $OCF_3$ oder OH stehen.

**10.** Substituierte Heteroaryl-Derivate gemäß Anspruch 1 aus der Gruppe

(1) 2-(2-(4-(Dimethylamino)-4-phenylcyclohexyl)-1H-indol-3-yl)ethanol, Citrat
(3) (±) 2-(2-(4-(Dimethylamino)-4-phenylcyclohex-1-enyl)-1H-indol-3-yl)ethyl acetat Hydrochlorid
(4) (±) 2-(4-(Dimethylamino)-4-phenylcyclohex-1-enyl)-3-(3-aminopropyl)-1H-indol, Citrat
(6) (±) 3-(2-(4-(Dimethylamino)-4-phenylcyclohex-1-enyl)-1H-indol-3-yl)propan-1-ol Hydrochlorid
(7) (±) 2-(5,6-Dichlor-2-(4-(dimethylamino)-4-phenylcyclohex-1-enyl)-1H-indol-3-yl)ethanol, Citrat
(8) (±) 2-(2-(4-Morpholino-4-phenylcyclohex-1-enyl)-1H-indo)-3-yl)ethanol, Citrat
(9) (±) 2-(4,6-Dichlor-2-(4-(dimethylamino)-4-phenylcyclohex-1-enyl)-1H-indol-3-yl)ethanol, Citrat
(10) (±) 2-(2-(4-(Dimethylamino)-4-phenylcyclohex-1-enyl)-5-fluor-1H-indol-3-yl)ethanol, Citrat
(11) (±) 2-(2-(4-(Dimethylamino)-4-phenylcyclohex-1-enyl)-5-(pyridin-3-yl)-1H-indol-3-yl)ethanol, Citrat
(13) (±) 2-(2-(4-(Dimethylamino)-4-phenylcyclohex-1-enyl)-5-nitro-1H-indol-3-yl)ethanol, Citrat
(14) (±) 2-(2-(4-(Benzo[b]thiophen-2-yl)-4-(dimethylamino)cyclohex-1-enyl)-1H-indol-3-yl)ethanol, Citrat
(15) (±) 2-(2-(4-(Benzo[b]thiophen-2-yl)-4-(dimethylamino)cyclohex-1-enyl)-5-fluor-1H-indol-3-yl)ethanol, Citrat
(16) (±) 2-(4-(Dimethylamino)-4-phenylcyclohex-1-enyl)-3-methyl-1H-indol, Citrat
(17) N,N-Dimethyl-4-(3-methyl-1H-indol-2-yl)-1-phenylcyclohexanamin, Citrat
(18) N,N-Dimethyl-4-(3-methyl-1H-indol-2-yl)-1-phenylcyclohexanamin, Citrat
(19) 2-(2-(2-(4-(Dimethylamino)-4-phenylcyclohexyl)-1H-indol-3-yl)ethyl)isoindolin-1,3-dione, Citrat
(20) N-(2-(2-(4-(Dimethylamino)-4-phenylcyclohexyl)-1H-indol-3-yl)ethyl)acetamid, Citrat
(21) N-(2-(2-(4-(Dimethylamino)-4-phenylcyclohexyl)-1H-indol-3-yl)ethyl)acetamid, Citrat
(22) (±)-2-(4-Benzyl-4-(dimethylamino)cyclohex-1-enyl)-3-methyl-1H-indol-5-carbonitril
(23) (±)-2-(4-(Dimethylamino)-4-phenylcyclohex-1-enyl)-3-methyl-1H-indol-5-carbonitril
(24) (±)-2-(4-(Dimethylamino)-4-butylcyclohex-1-enyl)-3-methyl-5-trifluormethyl-1H-indol
(25) (±)-2-(4-(Dimethylamino)-4-phenylcyclohex-1-enyl)-3-methyl-5-trifluormethyl-1H-indol, Citrat
(26) (±)-2-(4-(Dimethylamino)-4-benzylcyclohex-1-enyl)-3-methyl-5-fluor-1H-indol, Citrat
(27) (±)-2-(4-(Dimethylamino)-4-butylcyclohex-1-enyl)-3-methyl-5-fluor-1H-indol, Citrat
(28) (±)-2-(4-(Dimethylamino)-4-phenytcyclohex-1-enyl)-3-methyl-5-fluor-1H-indol, Citrat
(29) (±2)-(4-(Dimethylamino)-4-phenylcyclohex-1-enyl)-3-methyl-5-methoxy-1H-indol, Citrat
(30) (±)-2-(4-(Dimethylamino)-4-benzylcyclohex-1 -enyl)-3-methyl-1H-indol, Citrat
(31) (±)-2-(4-(Dimethylamino)-4-butylcyclohex-1-enyl)-3-methyl-1H-indol, Citrat
(32) (±)-2-(4-(Dimethylamino)-4-phenylcyclohex-1-enyl)-3-methyl-1H-pyrrolo[2,3-b]pyridin, Citrat
(33) (±)-2-(4-(Dimethylamino)-4-benzylcyclohex-1-enyl)-3-cyclopropyl-1H-indol Hydrochlorid
(34) (±)-2-(4-(Dimethylamino)-4-butylcyclohex-1-enyl)-3-cyclopropyl-1H-indol
(35) (±)-2-(4-(Dimethylamino)-4-phenylcyclohex-1-enyl)-3-cyclopropyl-1H-indol
(36) (±)-2-(4-(Dimethylamino)-4-benzylcyclohex-1-enyl)-3-(cyclohexylmethyl)-1H-indol
(37) (±)-2-(4-(Dimethylamino)-4-benzylcyclohex-1-enyl)-3-benzyl-1H-indol Hydrochlorid
(38) (±)-2-(4-(Dimethylamino)-4-butylcyclohex-1-enyl)-3-(cyclohexylmethyl)-1H-indol Hydrochlorid
(39) (±)-2-(4-(Dimethylamino)-4-butylcyclohex-1-enyl)-3-benzyl-1H-indol
(40) (±)-2-(4-(Dimethylamino)-4-phenylcyclohex-1-enyl)-3-(cyclohexylmethyl)-1H-indol Hydrochlorid
(41) (±)-2-(4-(Dimethylamino)-4-phenylcyclohex-1-enyl)-3-benzyl-1H-indol Hydrochlorid
(42) (±)-2-(4-(Dimethylamino)-4-benzylcyclohex-1-enyl)-3-propyl-1H-indol
(43) (±)-2-(4-(Dimethylamino)-4-butylcyclohex-1-enyl)-3-propyl-1H-indol
(44) (±)-2-(4-(Dimethylamino)-4-phenylcyclohex-1-enyl)-3-propyl-1H-indol
(45) (±) 2-(4-(dimethylamino)-4-benzylcyclohex-1-enyl)-3-(2-(pyridin-4-yl)ethyl)-1H-indol
(46) (±) 2-(4-(dimethylamino)-4-butylcyclohex-1-enyl)-3-(2-(pyridin-4-yl)ethyl)-1H-indol
(47) (±) 2-(4-(dimethylamino)-4-phenylcyclohex-1-enyl)-3-(2-(pyridin-4-yl)ethyl)-1H-indol
(48) (±)-3-(2-(4-Benzyl-4-(dimethylamino)cyclohex-1-enyl)-1H-indol-3-yl)propan-1-ol, Citrat
(49) (±)-3-(2-(4-Butyl-4-(dimethylamino)cyclohex-1-enyl)-1H-indol-3-yl)propan-1-ol, Citrat
(51) (±) 2-(2-(2-(4-(Dimethylamino)-4-phenylcyclohex-1-enyl)-1H-indol-3-yl)ethyl)isoindolin-1,3-dione
(52) (±) 2-(4-(Dimethylamino)-4-benzylcyclohex-1-enyl)-3-(2-(1H-benzo[d]imidazol-1-yl)ethyl)-5-fluor-1H-Indol
(53) (±) 2-(4-(Dimethylamino)-4-butylcyclohex-1-enyl)-3-(2-(1H-benzo[d]imidazol-1-yl)ethyl)-5-fluor-1H-indol
(54) (±) 2-(4-(Dimethylamino)-4-phenylcyclohex-1-enyl)-3-(2-(1H-benzo[d]imidazol-1-yl)ethyl)-5-fluor-1H-indol
(55) (±) 2-(4-(Dimethylamino)-4-benzylcyclohex-1-enyl)-3-(2-(piperidin-1-yl)ethyl)-5-fluor-1H-indol

(56) (±) 2-(4-(Dimethylamino)-4-butylcyclohex-1-enyl)-3-(2-(piperidin-1-yl)ethyl)-5-fluor-1H-indol
(57) (±) 2-(4-(Dimethylamino)-4-phenylcyclohex-1-enyl)-3-(2-(piperidin-1-yl)ethyl)-5-fluor-1H-indol
(58) (±) 2-(4-(Dimethylamino)-4-phenylcyclohex-1-enyl)-3-(2-(piperidin-1-yl)ethyl)-1H-indol, Citrat
(59) (±) 2-(4-(Dimethylamino)-4-benzylcyclohex-1-enyl)-3-(2-(1H-1,2,3-triazol-1-yl)ethyl)-5-fluor-1H-indol
(60) (±) 2-(4-(Dimethylamino)-4-butylcyclohex-1-enyl)-3-(2-(1H-1,2,3-triazol-1-yl)ethyl)-5-fluor-1H-indol
(61) (±) 2-(4-(Dimethylamino)-4-phenylcyclohex-1-enyl)-3-(2-(1H-1,2,3-triazol-1-yl)ethyl)-5-fluor-1H-indol
(62) N-(2-(2-(4-Butyl-4-(dimethylamino)cyclohexyl)-1H-indol-3-yl)ethyl)-3,3-dimethylbutanamid, Citrat
(63) (±) N-(2-(2-(4-(Dimethylamino)-4-phenylcyclohex-1-enyl)-1H-indol-3-yl)ethyl)acetamid
(64) (±) N-(2-(2-(4-Butyl-4-(dimethylamino)cyclohex-1-enyl)-1H-indol-3-yl)ethyl)-3,3-dimethylbutanamid
(65) (±)-2-(2-(4-(Dimethylamino)-4-phenylcyclohex-1-enyl)-5-fluor-6-methoxy-1H-indol-3-yl)ethanol, Citrat
(66) (±)-2-(2-(4-Benzyl-4-(4-methylpiperazin-1-yl)cyclohex-1-enyl)-5-fluor-1H-indol-3-yl)ethanol
(67) (±)-2-(5-fluor-2-(4-phenyl-4-(pyrrolidin-1-yl)cyclohex-1-enyl)-1H-indol-3-yl)ethanol
(68) 2-(4-Benzyl-4-(dimethylamino)cyclohexyl)-3-methyl-1H-indol-5-carbonitril, Citrat
(69) 2-(4-Benzyl-4-(dimethylamino)cyclohexyl)-3-methyl-1H-indol-5-carbonitril, Citrat
(70) 2-(4-Butyl-4-(dimethylamino)cyclohexyl)-3-methyl-1H-indol-5-carbonitril, Citrat
(71) 2-(4-Butyl-4-(dimethylamino)cyclohexyl)-3-methyl-1H-indol-5-carbonitril, Citrat
(72) 2-(4-(Dimethylamino)-4-phenylcyclohexyl)-3-methyl-1H-indol-5-carbonitril, Citrat
(73) 1-Benzyl-N,N-dimethyl-4-(3-methyl-5-(trifluormethyl)-1H-indol-2-yl)cyclohexanamin, Citrat
(74) 1-Benzyl-N,N-dimethyl-4-(3-methyl-5-(trifluormethyl)-1H-indol-2-yl)cyclohexanamin, Citrat
(75) 1-Butyl-N,N-dimethyl-4-(3-methyl-5-(trifluormethyl)-1H-indol-2-yl)cyclohexanamin, Citrat
(76) N,N-Dimethyl-4-(3-methyl-5-(trifluormethyl)-1H-indol-2-yl)-1-phenylcyclohexanamin. Citrat
(77) 1-Benzyl-4-(5-fluor-3-methyl-1H-indol-2-yl)-N,N-dimethylcyclohexanamin, Citrat
(78) 1-Benzyl-4-(5-fluor-3-methyl-1H-indol-2-yl)-N,N-dimethylcyclohexanamin, Citrat
(79) 1-Butyl-4-(5-fluor-3-methyl-1H-indol-2-yl)-N,N-dimethylcyclohexanamin Hydrochlorid
(80) 4-(5-Fluor-3-methyl-1H-indol-2-yl)-N,N-dimethyl-1-phenylcyclohexanamin, Citrat
(81) 4-(5-Fluor-3-methyl-1H-indol-2-yl)-N,N-dimethyl-1-phenylcyclohexanamin, Citrat
(82) 4-(3-(2-(1H-Benzo[d]imidazol-1-yl)ethyl)-5-fluor-1H-indol-2-yl)-1-butyl-N,N-dimethylcyclohexanamin, Citrat
(83) 4-(3-(2-(1H-Benzo[d]imidazol-1-yl)ethyl)-5-fluor-1H-indol-2-yl)-N,N-dimethyl-1-phenylcyclohexanamin, Citrat
(84) 4-(3-(2-(1H-Benzo[d]imidazol-1-yl)ethyl)-5-fluor-1H-indol-2-yl)-N,N-dimethyl-1-phenylcyclohexanamin, Citrat
(85) 1-Benzyl-4-(5-fluor-3-(2-(pyrrolidin-1-yl)ethyl)-1H-indol-2-yl)-N,N-dimethylcyclohexanamin, Citrat
(86) 1-Benzyl-4-(5-fluor-3-(2-(pyrrolidin-1-yl)ethyl)-1H-indol-2-yl)-N,N-dimethylcyclohexanamin, Citrat
(87) 1-Butyl-4-(5-fluor-3-(2-(pyrrolidin-1-yl)ethyl)-1H-indol-2-yl)-N,N-dimethylcyclohexanamin, Citrat
(88) 1-Butyl-4-(5-fluor-3-(2-(pyrrolidin-1-yl)ethyl)-1H-indol-2-yl)-N,N-dimethylcyclohexanamin, Citrat
(89) 4-(5-Fluor-3-(2-(pyrrolidin-1-yl)ethyl)-1H-indol-2-yl)-N,N-dimethyl-1-phenylcyclohexanamin, Citrat
(90) 4-(5-Fluor-3-(2-(pyrrolidin-1-yl)ethyl)-1H-indol-2-yl)-N,N-dimethyl-1-phenylcyclohexanamin, Citrat
(91) 1-Benzyl-4-(5-fluor-3-(2-(piperidin-1-yl)ethyl)-1H-indol-2-yl)-N,N-dimethylcyclohexanamin, Citrat
(92) 1-Benzyl-4-(5-fluor-3-(2-(piperidin-1-yl)ethyl)-1H-indol-2-yl)-N,N-dimethylcyclohexanamin, Citrat (2:3)
(93) 1-Butyl-4-(5-fluor-3-(2-(piperidin-1-yf)ethyl)-1H-indol-2-yl)-N,N-dimethylcyclohexanamin, Citrat (4:3)
(94) 1-Butyl-4-(5-fluor-3-(2-(piperidin-1-yl)ethyl)-1H-indol-2-yl)-N,N-dimethylcyclohexanamin, Citrat
(95) 4-(5-Fluor-3-(2-(piperidin-1-yl)ethyl)-1H-indol-2-yl)-N,N-dimethyl-1-phenylcyclohexanamin, Citrat
(96) 4-(5-Fluor-3-(2-(piperidin-1-yl)ethyl)-1H-indol-2-yl)-N,N-dimethyl-1-phenylcyclohexanamin, Citrat
(97) 4-(3-(2-(1H-pyrazol-1-yl)ethyl)-5-fluor-1H-indol-2-yl)-1-benzyl-N,N-dimethylcyclohexanamin, Citrat (2:3)
(98) 4-(3-(2-(1H-Pyrazol-1-yl)ethyl)-5-fluor-1H-indol-2-yl)-1-benzyl-N,N-dimethylcyclohexanamin, Citrat
(99) 4-(3-(2-(1H-Pyrazol-1-yl)ethyl)-5-fluor-1H-indol-2-yl)-1-butyl-N,N-dimethylcyclohexanamin, Citrat
(100) 4-(3-(2-(1H-Pyrazol-1-yl)ethyl)-5-fluor-1H-indol-2-yl)-1-butyl-N,N-dimethylcyctohexanamin, Citrat
(101) 4-(3-(2-(1H-Pyrazol-1-yl)ethyl)-5-fluor-1H-indol-2-yl)-N,N-dimethyl-1-phenylcyclohexanamin, Citrat
(102) 4-(3-(2-(1H-Pyrazol-1-yl)ethyl)-5-fluor-1H-indol-2-yl)-N,N-dimethyl-1-phenylcyclohexanamin, Citrat
(103) 4-(3-(2-(1H-Imidazol-1-yl)ethyl)-5-fluor-1H-indol-2-yl)-1-benzyl-N,N-dimethylcyclohexanamin, Citrat
(104) 4-(3-(2-(1H-Imidazol-1-yl)ethyl)-5-fluor-1H-indol-2-yl)-1-benzyl-N,N-dimethylcyclohexanamin, Citrat
(105) 4-(3-(2-(1H-Imidazol-1-yl)ethyl)-5-fluor-1H-indol-2-yl)-1-butyl-N,N-dimethylcyclohexanamin, Citrat (2:3)
(106) 4-(3-(2-(1H-Imidazol-1-yl)ethyl)-5-fluor-1H-indol-2-yl)-1-butyl-N,N-dimethylcyclohexanamin, Citrat
(107) 4-(3-(2-(1H-Imidazol-1-yl)ethyl)-5-fluor-1H-indol-2-yl)-N,N-dimethyl-1-phenylcyclohexanamin, Citrat
(108) 4-(3-(2-(1H-Imidazol-1-yl)ethyl)-5-fluor-1H-indol-2-yl)-N,N-dimethyl-1-phenylcyclohexanamin, Citrat (2:3)
(109) 4-(3-(2-(1H-Benzo[d]imidazol-1-yl)ethyl)-5-fluor-1H-indol-2-yl)-1-benzyl-N,N-dimethylcyclohexanamin, Citrat (4:3)

(110) 4-(3-(2-(1H-Benzo[d]imidazol-1-yl)ethyl)-5-fluor-1H-indol-2-yl)-1-benzyl-N,N-dimethylcyclohexanamin, Citrat (2:3)
(111) 4-(3-(2-(1H-Benzo[d]imidazol-1-yl)ethyl)-5-fluor-1H-indol-2-yl)-1-butyl-N,N-dimethylcyclohexanamin, Citrat (2:3)
(112) 4-(3-(2-(1H-1,2,3-Triazol-1-yl)ethyl)-5-fluor-1H-indol-2-yl)-1-benzyl-N,N-dimethylcyclohexanamin, Citrat (4:1)
(113) 4-(3-(2-(1H-1,2,3-Triazol-1-yl)ethyl)-5-fluor-1H-indol-2-yl)-1-benzyl-N,N-dimethylcyclohexanamin, Citrat
(114) 4-(3-(2-(1H-1,2,3-Triazol-1-yl)ethyl)-5-fluor-1H-indol-2-yl)-1-butyl-N,N-dimethylcyclohexanamin, Citrat
(115) 4-(3-(2-(1H-1,2,3-Triazol-1-yl)ethyl)-5-fluor-1H-indol-2-yl)-1-butyl-N,N-dimethylcyclohexanamin, Citrat
(116) 4-(3-(2-(1H-1,2,3-Triazol-1-yl)ethyl)-5-fluor-1H-indol-2-yl)-N,N-dimethyl-1-phenylcyclohexanamin, Citrat
(117) 4-(3-(2-(1H-1,2,3-Triazol-1-yl)ethyl)-5-fluor-1H-indol-2-yl)-N,N-dimethyl-1-phenytcydohexanamin, Citrat
(118) 2-(4-(Dimethylamino)-4-phenylcyclohexyl)-3-methyl-1H-indol-5-ol
(119) 2-(4-(Dimethylamino)-4-phenylcyctohexyl)-3-methyl-1H-indol-5-ol
120) 1-Benzyl-N,N-dimethyl-4-(3-methyl-5-(trifluormethoxy)-1H-indol-2-yl)cyclohexanamin, Citrat
(121) 1-Butyl-N,N-dimethyl-4-(3-methyl-5-(trifluormethoxy)-1H-indol-2-yl)cyclohexanamin, Citrat
(122) 1-Butyl-N,N-dimethyl-4-(3-methyl-5-(trifluormethoxy)-1H-indol-2-yl)cyclohexanamin, Citrat
(123) N,N-Dimethyl-4-(3-methyl-5-(trifluormethoxy)-1H-indol-2-yl)-1-phenylcyclohexanamin, Citrat
(124) N,N-Dimethyl-4-(3-methyl-5-(trifluormethoxy)-1H-indol-2-yl)-1-phenylcyclohexanamin, Citrat
(125) 1-Butyl-4-(5-methoxy-3-methyl-1H-indol-2-yl)-N,N-dimethylcyclohexanamin, Citrat
(126) 1-Butyl-4-(5-methoxy-3-methyl-1H-indol-2-yl)-N,N-dimethylcyclohexanamin, Citrat
(127) 4-(5-Methoxy-3-methyl-1H-indol-2-yl)-N,N-dimethyl-1-phenylcyclohexanamin, Citrat
(128) 4-(5-Methoxy-3-methyl-1H-indol-2-yl)-N,N-dimethyl-1-phenylcyclohexanamin, Citrat (4:3)
(129) 1-Benzyl-N,N-dimethyl-4-(3-methyl-1H-indol-2-yl)cyclohexanamin, Citrat
(130) 1-Benzyl-N,N-dimethyl-4-(3-methyl-1H-indol-2-yl)cyclohexanamin, Citrat
(131) 1-Butyl-N,N-dimethyl-4-(3-methyl-1H-indol-2-yl)cyclohexanamin, Citrat
(132) 1-Butyl-N,N-dimethyl-4-(3-methyl-1H-indol-2-yl)cyclohexanamin, Citrat
(133) 1-Benzyl-4-(3-cyclopropyl-1H-indol-2-yl)-N,N-dimethylcyclohexanamin, Citrat
(134) 1-Benzyl-4-(3-cyclopropyl-1H-indol-2-yl)-N,N-dimethylcyclohexanamin, Citrat
(135) 1-Butyl-4-(3-cyclopropyl-1H-indol-2-yl)-N,N-dimethylcyclohexanamin Hydrochlorid
(136) 4-(3-Cyclopropyl-1H-indol-2-yl)-N,N-dimethyl-1-phenylcyclohexanamin, Citrat
(137) Methyl 2-(2-(4-(dimethylamino)-4-phenylcyclohexyl)-1H-indol-3-yl)acetate, Citrat
(138) Methyl 2-(2-(4-(dimethylamino)-4-phenylcyclohexyl)-1H-indol-3-yl)acetate, Citrat
(139) 1-Benzyl-4-(3-(cyclohexylmethyl)-1H-indol-2-yl)-N,N-dimethylcyclohexanamin, Citrat
(140) 1-Benzyl-4-(3-(cyclohexylmethyl)-1H-indol-2-yl)-N,N-dimethylcyclohexanamin, Citrat
(141) 1-Benzyl-4-(3-Benzyl-1H-indol-2-yl)-N,N-dimethylcyclohexanamin, Citrat
(142) 1-Benzyl-4-(3-Benzyl-1H-indol-2-yl)-N,N-dimethylcyclohexanamin, Citrat
(143) 1-Butyl-4-(3-(cyclohexylmethyl)-1H-indol-2-yl)-N,N-dimethylcyclohexanamin, Citrat
(144) 1-Butyl-4-(3-(cyclohexylmethyl)-1H-indol-2-yl)-N,N-dimethylcyclohexanamin Hydrochlorid
(145) 4-(3-Benzyl-1H-indol-2-yl)-1-butyl-N,N-dimethylcyclohexanamin, Citrat
(146) 4-(3-Benzyl-1H-indol-2-yl)-1-butyl-N,N-dimethylcydohexanamin, Citrat
(147) 4-(3-(Cyclohexylmethyl)-1H-indol-2-yl)-N,N-dimethyl-1-phenylcyclohexanamin, Citrat
(148) 4-(3-(Cyclohexylmethyl)-1H-indol-2-yl)-N,N-dimethyl-1-phenylcyclohexanamin, Citrat
(149) 4-(3-Benzyl-1H-indol-2-yl)-N,N-dimethyl-1-phenylcyclohexanamin, Citrat
(150) 4-(3-Benzyl-1H-indol-2-yl)-N,N-dimethyl-1-phenylcyclohexanamin, Citrat
(151) N,N-Dimethyl-1-phenyl-4-(3-(pyridin-2-ylmethyl)-1H-indol-2-yl)cyclohexanamin
(152) N,N-Dimethyl-1-phenyl-4-(3-(pyridin-2-ylmethyl)-1H-indol-2-yl)cyclohexanamin
(153) 3-(2-(4-(Dimethylamino)-4-phenylcyclohexyl)-1H-indol-3-yl)propanoic acid Hydrochlorid
(156) 1-Benzyl-N,N-dimethyl-4-(3-propyl-1H-indol-2-yl)cyclohexanamin, Citrat
(157) 1-Butyl-N,N-dimethyl-4-(3-propyl-1H-indol-2-yl)cyclohexanamin Hydrochlorid
(158) 1-Butyl-N,N-dimethyl-4-(3-propyl-1H-indol-2-yl)cyclohexanamin, Citrat
(159) N,N-Dimethyl-1-phenyl-4-(3-propyl-1H-indol-2-yl)cyclohexanamin, Citrat
(160) N,N-Dimethyl-1-phenyl-4-(3-propyl-1H-indol-2-yl)cyclohexanamin, Citrat
(161) 1-Benzyl-N,N-dimethyl-4-(3-(2-(pyridin-4-yl)ethyl)-1H-indol-2-yl)cyclohexanamin, Citrat
(162) 1-Benzyl-N,N-dimethyl-4-(3-(2-(pyridin-4-yl)ethyl)-1H-indol-2-yl)cyclohexanamin, Citrat
(163) 1-Butyl-N,N-dimethyl-4-(3-(2-(pyridin-4-yl)ethyl)-1H-indol-2-yl)cyclohexanamin, Citrat
(164) 1-Butyl-N,N-dimethyl-4-(3-(2-(pyridin-4-yl)ethyl)-1H-indol-2-yl)cyclohexanamin, Citrat
(165) N,N-Dimethyl-1-phenyl-4-(3-(2-(pyridin-4-yl)ethyl)-1H-indol-2-yl)cyclohexanamin, Citrat (1:4)
(166) N,N-Dimethyl-1-phenyl-4-(3-(2-(pyridin-4-yl)ethyl)-1H-indol-2-yl)cyclohexanamin, Citrat

(167) N,N-Dimethyl-4-(3-(2-(1-methyl-1H-benzo[d]imidazol-2-yl)ethyl)-1H-indol-2-yl)-1-phenylcyclohexanamin, Citrat
(168) N,N-Dimethyl-4-(3-(2-(1-methyl-1H-benzo[d]imidazol-2-yl)ethyl)-1H-indol-2-yl)-1-phenylcyclohexanamin, Citrat
(169) N,N-Dimethyl-1-phenyl-4-(3-(2-(pyridin-2-yl)ethyl)-1H-indol-2-yl)cyclohexanamin, Citrat
(170) N,N-Dimethyl-1-phenyl-4-(3-(2-(pyridin-2-yl)ethyl)-1H-indol-2-yl)cyclohexanamin, Citrat (2:3)
(171) 4-(2-(4-Benzyl-4-(dimethylamino)cyclohexyl)-1H-indol-3-yl)butanoic acid Hydrochlorid
(172) 4-(2-(4-(Dimethylamino)-4-phenylcyclohexyl)-1H-indol-3-yl)butanoic acid Hydrochlorid
(173) 4-(2-(4-(Dimethylamino)-4-phenylcyclohexyl)-1H-indol-3-yl)butan-1-ol Hydrochlorid
(174) 4-(2-(4-Benzyl-4-(dimethylamino)cyclohexyl)-1H-indol-3-yl)butyl acetate Hydrochlorid
(175) 4-(2-(4-Benzyl-4-(dimethylamino)cyclohexyl)-1H-indol-3-yl)butyl acetate Hydrochlorid
(176) 4-(2-(4-Butyl-4-(dimethylamino)cyclohexyl)-1H-indol-3-yl)butyl acetate Hydrochlorid
(177) 4-(2-(4-Butyl-4-(dimethylamino)cyclohexyl)-1H-indol-3-yl)butyl acetate Hydrochlorid
(178) 4-(2-(4-(Dimethylamino)-4-phenylcyclohexyl)-1H-indol-3-yl)butyl acetate Hydrochlorid
(179) 4-(2-(4-(Dimethylamino)-4-phenylcyclohexyl)-1H-indol-3-yl)butyl acetate Hydrochlorid
(180) 3-(2-(4-Benzyl-4-(dimethylamino)cyclohexyl)-1H-indol-3-yl)propan-1-ol, Citrat
(181) 3-(2-(4-Butyl-4-(dimethylamino)cyclohexyl)-1H-indol-3-yl)propan-1-ol Hydrochlorid
(182) 3-(2-(4-Butyl-4-(dimethylamino)cyclohexyl)-1H-indol-3-yl)propan-1-ol Hydrochlorid
(183) 3-(2-(4-(Dimethylamino)-4-phenylcyclohexyl)-1H-indol-3-yl)propan-1-ol Hydrochlorid
(184) 3-(2-(4-(Dimethylamino)-4-phenylcyclohexyl)-1H-indol-3-yl)propan-1-ol Hydrochlorid
(185) 3-(2-(4-(Dimethylamino)-4-phenylcyclohexyl)-1H-indol-3-yl)propyl acetate Hydrochlorid
(186) 3-(2-(4-(Dimethylamino)-4-phenylcyclohexyl)-1H-indol-3-yl)propyl acetate Hydrochlorid
(187) 1-(2-(2-(4-(Dimethylamino)-4-phenylcyclohexyl)-1H-indol-3-yl)ethyl)pyrrolidine-2,5-dione
(188) 1-(2-(2-(4-(Dimethylamino)-4-phenylcyclohexyl)-1H-indol-3-yl)ethyl)pyrrolidine-2,5-dione
(189) 4-(3-(2-(3,4-Dihydrochinolin-1(2H)-yl)ethyl)-1H-indol-2-yl)-N,N-dimethyl-1-phenylcyclohexanamin, Citrat (4:3)
(190) 4-(3-(2-(3,4-Dihydrochinolin-1(2H)-yl)ethyl)-1H-indol-2-yl)-N,N-dimethyl-1-phenylcyclohexanamin, Citrat
(191) Methyl 1-(2-(2-(4-(dimethylamino)-4-phenylcyclohexyl)-1H-indol-3-yl)ethyl)-1H-1,2,3-triazole-4-carboxylat, Citrat
(192) Methyl 1-(2-(2-(4-(dimethylamino)-4-phenylcyclohexyl)-1H-indol-3-yl)ethyl)-1H-1,2,3-triazole-4-carboxylate, Citrat
(193) 4-(3-(2-(Isoindolin-2-yl)ethyl)-1H-indol-2-yl)-N,N-dimethyl-1-phenylcyclohexanamin, Citrat
(194) 4-(3-(2-(Isoindolin-2-yl)ethyl)-1H-indol-2-yl)-N,N-dimethyl-1-phenylcyclohexanamin, Citrat
(195) 4-(3-(2-(3,4-Dihydroisochinolin-2(1H)-yl)ethyl)-1H-indol-2-yl)-N,N-dimethyl-1-phenylcyclohexanamin, Citrat
(196) 4-(3-(2-(3,4-Dihydroisochinolin-2(1H)-yl)ethyl)-1H-indol-2-yl)-N,N-dimethyl-1-phenylcyclohexanamin, Citrat (2:3)
(197) N,N-Dimethyl-1-phenyl-4-(3-(2-(pyrrolidin-1-yl)ethyl)-1H-indol-2-yl)cyclohexanamin, Citrat
(198) N,N-Dimethyl-1-phenyl-4-(3-(2-(pyrrolidin-1-yl)ethyl)-1H-indol-2-yl)cyclohexanamin, Citrat
(199) N,N-Dimethyl-1-phenyl-4-(3-(2-(piperidin-1-yl)ethyl)-1H-indol-2-yl)cyclohexanamin, Citrat
(200) N,N-Dimethyl-4-(3-(2-(4-methylpiperazin-1-yl)ethyl)-1H-indol-2-yl)-1-phenylcyclohexanamin, Citrat
(201) N,N-Dimethyl-4-(3-(2-morpholinoethyl)-1H-indol-2-yl)-1-phenylcyclohexanamin, Citrat
(202) N,N-Dimethyl-4-(3-(2-morpholinoethyl)-1H-indol-2-yl)-1-phenylcyclohexanamin, Citrat
(203) 4-(3-(2-(1H-Benzo[d]imidazol-1-yl)ethyl)-1H-indol-2-yl)-N,N-dimethyl-1-phenylcyclohexanamin, Citrat
(204) 4-(3-(2-(1H-Benzo[d]imidazol-1-yl)ethyl)-1H-indol-2-yl)-N,N-dimethyl-1-phenylcyclohexanamin, Citrat
(205) 4-(3-(2-(1H-Imidazol-1-yl)ethyl)-1H-indol-2-yl)-N,N-dimethyl-1-phenylcyclohexanamin, Citrat
(206) 4-(3-(2-(1H-Imidazol-1-yl)ethyl)-1H-indol-2-yl)-N,N-dimethyl-1-phenylcyclohexanamin, Citrat
(207) 4-(3-(2-(1H-1,2,4-Triazol-1-yl)ethyl)-1H-indol-2-yl)-N,N-dimethyl-1-phenylcyclohexanamin, Citrat
(208) 4-(3-(2-(1H-1,2,4-Triazol-1-yl)ethyl)-1H-indol-2-yl)-N,N-dimethyl-1-phenylcyclohexanamin, Citrat
(209) N,N-Dimethyl-1-phenyl-4-(3-(2-(thiazolidin-3-yl)ethyl)-1H-indol-2-yl)cyclohexanamin, Citrat
(210) N,N-Dimethyl-1-phenyl-4-(3-(2-(thiazolidin-3-yl)ethyl)-1H-indol-2-yl)cyclohexanamin, Citrat
(211) N,N-Dimethyl-4-(3-(2-(5-methyl-2H-tetrazol-2-yl)ethyl)-1H-indol-2-yl)-1-phenylcyclohexanamin, Citrat
(212) N,N-Dimethyl-4-(3-(2-(5-methyl-2H-tetrazol-2-yl)ethyl)-1H-indol-2-yl)-1-phenylcyclohexanamin, Citrat
(213) 4-(3-(2-(1H-pyrazol-1-yl)ethyl)-1H-indol-2-yl)-N,N-dimethyl-1-phenylcyclohexanamin, Citrat
(214) 4-(3-(2-(1H-Pyrazol-1-yl)ethyl)-1H-indol-2-yl)-N,N-dimethyl-1-phenylcyclohexanamin, Citrat
(215) 4-(3-(2-(1H-1,2,3-Triazol-1-yl)ethyl)-1H-indol-2-yl)-N,N-dimethyl-1-phenylcyclohexanamin, Citrat
(216) 4-(3-(2-(1H-1,2,3-Triazol-1-yl)ethyl)-1H-indol-2-yl)-N,N-dimethyl-1-phenylcyclohexanamin, Citrat
(217) N,N-Dimethyl-4-(3-(2-(5-methyl-1H-tetrazol-1-yl)ethyl)-1H-indol-2-yl)-1-phenylcyclohexanamin, Citrat

(218) N,N-Dimethyl-4-(3-(2-(5-methyl-1H-tetrazol-1-yl)ethyl)-1H-indol-2-yl)-1-phenylcyclohexanamin, Citrat
(219) 2-(2-(4-(Dimethylamino)-4-phenylcyclohexyl)-1H-indol-3-yl)ethanol
(220) 2-(2-(4-(Dimethylamino)-4-phenylcyclohexyl)-1H-indol-3-yl)ethyl acetate
(221) N,N-Dimethyl-4-(3-methyl-1H-pyrrolo[3,2-c]pyridin-2-yl)-1-phenylcyclohexanamin, Citrat
(222) N,N-Dimethyl-4-(3-methyl-1H-pyrrolo[3,2-c]pyridin-2-yl)-1-phenylcyclohexanamin, Citrat
(223) N,N-Dimethyl-4-(3-methyl-1H-pyrrolo[2,3-b]pyridin-2-yl)-1-phenylcyclohexanamin, Citrat
(224) N,N-Dimethyl-4-(3-methyl-1H-pyrrolo[2,3-b]pyridin-2-yl)-1-phenylcyclohexanamin, Citrat
(225) N,N-Dimethyl-4-(3-methylbenzofuran-2-yl)-1-phenylcyclohexanamin, Citrat
(226) N,N-Dimethyl-4-(3-methylbenzofuran-2-yl)-1-phenylcyclohexanamin, Citrat
(227) 4-(1H-Indol-3-yl)-N,N-dimethyl-1-phenylcyclohexanamin, Citrat
(228) (±)-3-(4-(Dimethylamino)-4-benzylcyclohex-1-enyl)-1H-pyrrolo[2,3-b]pyridin
(229) (±)-3-(4-(Dimethylamino)-4-butylcyclohex-1-enyl)-1H-pyrrolo[2,3-b]pyridin
(230) (±)-3-(4-(Dimethylamino)-4-phenylcyclohex-1-enyl)-1H-pyrrolo[2,3-b]pyridin, Citrat
(231) (±)-4-(1H-Indol-3-yl)-N,N-dimethyl-1-phenylcyclohex-3-enamin, Citrat
(232) (±)-4-(1H-Indol-3-yl)-N,N-dimethyl-1-phenylcyclohex-3-enamin
(233) (±)-2-(3-(4-(Dimethylamino)-4-phenylcyclohex-1-enyl)benzo[b]thiophen-2-yl)ethanol Hydrochlorid
(234) (±)-2-(3-(4-(Dimethylamino)-4-(pyridin-2-yl)cyclohex-1-enyl)benzo[b]thiophen-2-yl)ethanol Hydrochlorid
(235) 4-(1H-Indol-2-yl)-N,N-dimethyl-1-phenylcyclohexanamin, Citrat
(236) 4-(1H-Indol-2-yl)-N,N-dimethyl-1-phenylcyclohexanamin, Citrat
(237) 1-Benzyl-N,N-dimethyl-4-(1H-pyrrolo[2,3-b]pyridin-3-yl)cyclohexanamin Hydrochlorid
(238) 1-Butyl-N,N-dimethyl-4-(1H-pyrrolo[2,3-b]pyridin-3-yl)cyclohexanamin Hydrochlorid
(239) (±)-4-(Benzofuran-2-yl)-1-benzyl-N,N-dimethylcyclohex-3-enamin Hydrochlorid
(240) (±)-N,N-Dimethyl-4-(3-methylbenzofuran-2-yl)-1-phenylcyclohex-3-enamin, Citrat
(241) (±)-2-(2-(4-(Dimethylamino)-4-phenylcyclohex-1-enyl)benzofuran-3-yl)ethanethiol, Citrat
(242) (±)-N,N-Dimethyl-4-(3-methylbenzo[b]thiophen-2-yl)-1-phenylcyclohex-3-enamin, Citrat
(243) N,N-Dimethyl-4-(3-methylbenzo[b]thiophen-2-yl)-1-phenylcyclohexanamin, Citrat
(244) N,N-Dimethyl-1-phenyl-4-(1H-pyrrolo[2,3-b]pyridin-3-yl)cyclohexanamin, Citrat
(246) 1-(Dimethylamino)-3-(2-(4-(Dimethylamino)-4-phenylcyclohexyl)-3-methyl-1H-indol-1-yl)propan-2-ol
(247) N,N-Dimethyl-4-(3-methyl-1-(oxiran-2-ylmethyl)-1H-indol-2-yl)-1-phenylcyclohexanamin
(248) 4-(1,3-Dimethyl-1H-indol-2-yl)-N,N-dimethyl-1-phenylcyclohexanamin
(249) 2-(4-Butyl-4-(dimethylamino)cyclohexyl)-3-methyl-1H-indol-5-ol, Citrat
(250) 2-(4-Butyl-4-(dimethylamino)cyclohexyl)-3-methyl-1H-indol-5-ol, Citrat
(251) (±)-2-(4-(Dimethylamino)-4-(thiophen-2-yl)-cyclohex-1-enyl)-3-(2-(pyridin-4-yl)ethyl)-1H-indol
(252) (±)-2-(4-(Dimethylamino)-4-(3-fluorphenyl)-cyclohex-1-enyl)-3-(2-(pyridin-4-yl)ethyl)-1H-indol
(253) (±)-2-(4-Butyl-4-(pyrrolidin-1-yl)cyclohex-1-enyl)-3-(2-(pyridin-4-yl)ethyl)-1H-indol
(254) (±)-2-(4-(Methylamino)-4-phenylcyclohex-1-enyl)-3-(2-(pyridin-4-yl)ethyl)-1H-indol
(255) (±)-2-(4-(Dimethylamino)-4-(thiophen-2-yl)-cyclohex-1-enyl)-3-methyl-1H-indol, Citrat
(256) N,N-Dimethyl-4-(3-methyl-1H-indol-2-yl)-1-(thiophen-2-yl)cyclohexanamin, Citrat
(257) N,N-Dimethyl-4-(3-methyl-1H-indol-2-yl)-1-(thiophen-2-yl)cyclohexanamin, Citrat
(258) 2-(4-Butyl-4-(pyrrolidin-1-yl)cyclohex-1-enyl)-3-methyl-1H-indol
(259) N-Methyl-4-(3-methyl-1H-indol-2-yl)-1-phenylcyclohexanaminhydrobromid
(260) N-Methyl-4-(3-methyl-1H-indol-2-yl)-1-phenylcyclohexanamin, Citrat
(261) 1-(3-Fluorphenyl)-N,N-dimethyl-4-(3-methyl-1H-indol-2-yl)cyclohexanamin, Citrat
(262) 1-(3-Fluorphenyl)-N,N-dimethyl-4-(3-methyl-1H-indol-2-yl)cyclohexanamin, Citrat
(263) (±)-2-(4-(Dimethylamino)-4-(3-fluorphenyl)-cyclohex-1-enyl)-3-methyl-1H-indol
(264) 2-(4-Benzyl-4-(dimethylamino)cyclohexyl)-3-methyl-1H-indol-5-ol, Citrat
(265) 2-(4-Benzyl-4-(dimethylamino)cyclohexyl)-3-methyl-1H-indol-5-ol, Citrat
(266) 2-(4-Butyl-4-(pyrrolidin-1-yl)cyclohexyl)-3-(2-(pyridin-4-yl)ethyl)-1H-indol, Citrat
(267) 2-(4-Butyl-4-(pyrrolidin-1-yl)cyclohexyl)-3-(2-(pyridin-4-yl)ethyl)-1H-indol, Citrat
(268) 2-(4-(Azetidin-1-yl)-4-phenylcyclohexyl)-3-methyl-1H-indol, Citrat
(269) 2-(4-(Azetidin-1-yl)-4-phenylcyclohexyl)-3-methyl-1H-indol, Citrat
(270) 2-(4-(Azetidin-1-yl)-4-phenylcyclohexyl)-3-(2-(pyridin-4-yl)ethyl)-1H-indol Hydrochlorid
(271) 2-(4-(Azetidin-1-yl)-4-phenylcyclohexyl)-3-(2-(pyridin-4-yl)ethyl)-1H-indol Hydrochlorid
(272) 2-(4-Butyl-4-(pyrrolidin-1-yl)cyclohexyl)-3-methyl-1H-indol, Citrat
(273) 3-(2-(4-(Dimethylamino)-4-phenylcyclohexyl)-1H-pyrrolo[2,3-b]pyridin-3-yl)propan-1-ol, Citrat
(274) 1-(2-(4-(Dimethylamino)-4-phenylcyclohexyl)-3-methyl-1H-indol-1-yl)-3-(methylamino)propan-2-ol, Citrat
(275) 1-Benzyl-3-(2-(2-(4-(dimethylamino)-4-phenylcyclohexyl)-1H-indol-3-yl)ethyl)harnstoff, Citrat
(276) 1-Benzyl-3-(2-(2-(4-(dimethylamino)-4-phenylcyclohexyl)-1H-indol-3-yl)ethyl)harnstoff, Citrat

(277) 1-(2-(2-(4-(Dimethylamino)-4-phenylcyclohexyl)-1H-indol-3-yl)ethyl)-3-phenylharnstoff, Citrat
(278) 1-(2-(2-(4-(Dimethylamino)-4-phenylcyclohexyl)-1H-indol-3-yl)ethyl)-3-phenylharnstoff, Citrat
(279) 1-Cyclopentyl-3-(2-(2-(4-(dimethylamino)-4-phenylcyclohexyl)-1H-indol-3-yl)ethyl)harnstoff, Citrat
(280) 1-Cyclopentyl-3-(2-(2-(4-(dimethylamino)-4-phenylcyclohexyl)-1H-indol-3-yl)ethyl)harnstoff, Citrat
(281) N-(2-(2-(4-(Dimethylamino)-4-phenylcyclohexyl)-1H-indol-3-yl)ethyl) cyclopentansulfonamid, Citrat
(282) N-(2-(2-(4-(Dimethylamino)-4-phenylcyclohexyl)-1H-indol-3-yl)ethyl)cyclopentanesulfonamid, Citrat
(283) N-(2-(2-(4-(Dimethylamino)-4-phenylcyclohexyl)-1H-indol-3-yl)ethyl)benzenesulfonamid, Citrat
(284) N-(2-(2-(4-(Dimethylamino)-4-phenylcyclohexyl)-1H-indol-3-yl)ethyl)thiophen-2-sulfonamid, Citrat
(285) N-(2-(2-(4-(Dimethylamino)-4-phenylcyclohexyl)-1H-indol-3-yl)ethyl)thiophen-2-sulfonamid, Citrat
(286) N-(2-(2-(4-(Dimethylamino)-4-phenylcyclohexyl)-1H-indol-3-yl)ethyl)nicotinamid, Citrat
(287) N-(2-(2-(4-(Dimethylamino)-4-phenylcyclohexyl)-1H-indol-3-yl)ethyl)nicotinamid, Citrat
(288) N-(2-(2-(4-(Dimethylamino)-4-phenylcyclohexyl)-1H-indol-3-yl)ethyl)benzamid, Citrat
(289) 1-(3-Fluorphenyl)-N,N-dimethyl-4-(3-(2-(pyridin-4-yl)ethyl)-1H-indol-2-yl)cyclohexanamin
(290) 1-(3-Fluorphenyl)-N,N-dimethyl-4-(3-(2-(pyridin-4-yl)ethyl)-1H-indol-2-yl)cyclohexanamin, Citrat
(291) N-Methyl-1-phenyl-4-(3-(2-(pyridin-4-yl)ethyl)-1H-indol-2-yl)cyclohexanamin, Citrat
(292) N-Methyl-1-phenyl-4-(3-(2-(pyridin-4-yl)ethyl)-1H-indol-2-yl)cyclohexanamin, Citrat
(293) 2-(4-Butyl-4-(pyrrolidin-1-yl)cyclohexyl)-3-methyl-1H-indol, Citrat
(294) N,N-Dimethyl-1-phenyl-4-(1-(phenylsulfonyl)-1H-indol-2-yl)cyclohexanamin
(295) N,N-Dimethyl-1-phenyl-4-(1-(phenylsulfonyl)-1H-indol-2-yl)cyclohexanamin
(296) N,N-Dimethyl-4-(3-methyl-1-(oxiran-2-ylmethyl)-1H-indol-2-yl)-1-phenylcyclohex-3-enamin
(297) N,N-Dimethyl-4-(3-methyl-1-(oxiran-2-ylmethyl)-1H-indol-2-yl)-1-phenylcyclohexanamin, Citrat
(298) 1-(Dimethylamino)-3-(2-(4-(Dimethylamino)-4-phenylcyclohex-1-enyl)-3-methyl-1H-indol-1-yl)propan-2-ol, Citrat
(299) 1-(Dimethylamino)-3-(2-(4-(Dimethylamino)-4-phenylcyclohexyl)-3-methyl-1H-indol-1-yl)propan-2-ol, Citrat
(300) 2-(3-(4-(Dimethylamino)-4-phenylcyclohex-1-enyl)-1H-indol-1-yl)ethanol Hydrochlorid
(301) (±)3-(4-(Dimethylamino)-4-phenylcyclohex-1-enyl)-(1-(phenylsulfonyl)-1H-indol) Hydrochlorid
(302) 1-Benzyl-N,N-dimethyl-4-(1-methyl-1H-indol-2-yl)cyclohex-3-enamin; Hydrochlorid
(303) N,N-dimethyl-4-(3-(2-(pyridin-4-yl)ethyl)-1H-indol-2-yl)-1-(thiophen-2-yl)cyclohexanamin Hydrochlorid
(304) N,N-dimethyl-4-(3-(2-(pyridin-4-yl)ethyl)-1H-indol-2-yl)-1-(thiophen-2-yl)cyclohexanamin Hydrochlorid
(305) N-Methyl-4-(3-methyl-1H-indol-2-yl)-1-(4-methylthiazol-2-yl)cyclohexanamin
(306) N-Methyl-4-(3-methyl-1H-indol-2-yl)-1-(4-methylthiazol-2-yl)cyclohexanamin
(307) 2-(2-(4-(Dimethylamino)-4-phenylcyclohexyl)-1H-indol-3-yl)ethanol

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

**11.** Verfahren zur Herstellung von substituierten Heteroaryl-Derivaten der Formel Ic

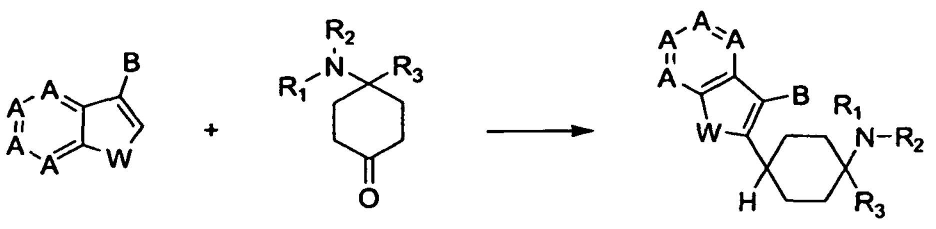

A B Ic

wobei Ketone der allgemeinen Formel B mit Heteroaromaten der allgemeinen Formel A in organischen Lösungsmitteln oder Lösungsmittelgemischen, beispielsweise Ethylacetat, Chloroform, Dichlormethan (DCM), Dichlorethan (DCE), Diethylether ($Et_2O$), Acetonitril (MeCN) oder Nitromethan unter Zusatz einer organischen oder anorganischen Säure, beispielsweise HCl, HBr, Trifluormethansulfonsäure, Methansulfonsäure, Essigsäure, Trifluoressigsäure oder lösungsmittelfrei in einer organischen oder anorganischen Säure oder Säuregemischen bei Temperaturen zwischen 0°C und 150°C ggf. unter Verwendung von Mikrowelleneinstrahlung umgesetzt und anschließend unter Zugabe eines organischen oder anorganischen Reduktionsmittels, z.B. Triethylsilan oder Zinn-Pulver, bei Temperaturen zwischen 0°C und 150°C ggf. unter Verwendung von Mikrowelleneinstrahlung umgesetzt werden.

**12.** Verfahren zur Herstellung von substituierten Heteroaryl-Derivaten der allgemeinen Formel **Id** oder **Ie**,

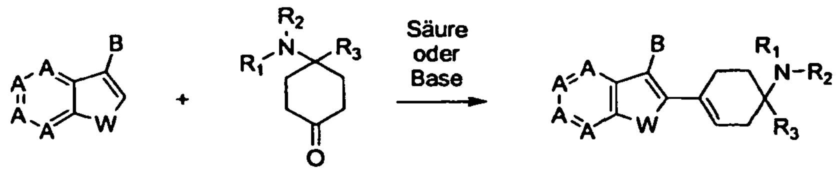


**A** **B** **Id**

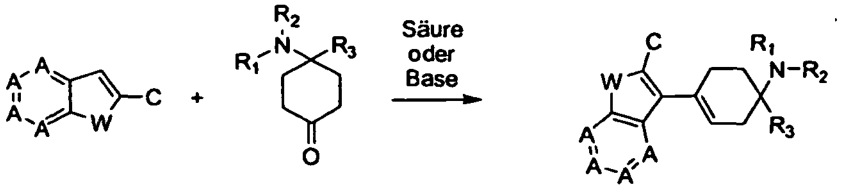


**A'** **B** **Ie**

wobei Heteroaromaten der allgemeinen Formel **A** oder **A'** mit Cyclohexanonen der allgemeinen Formel **B** in organischen Lösungsmitteln oder Lösungsmittelgemischen, beispielsweise Chloroform, Dichlormethan (DCM), Dichlorethan (DCE), Diethylether ($Et_2O$), Acetonitril (MeCN) oder Nitromethan unter Zusatz einer organischen oder anorganischen Säure, beispielsweise HCl, HBr, Trifluormethansulfonsäure, Methansulfonsäure, Essigsäure, Trifluoressigsäure bei Temperaturen zwischen 0°C und 150°C ggf. unter Verwendung von Mikrowelleneinstrahlung umgesetzt werden,

oder Heteroaromaten der allgemeinen Formel **A** oder **A'** mit Cyclohexanonen der allgemeinen Formel **B** unter Zusatz einer Base, beispielsweise KOH oder NaOH, in einem organischen Lösungsmittel, beispielsweise Methanol, bei Temperaturen zwischen 20 und 100°C umgesetzt werden.

**13.** Verfahren zur Herstellung von substituierten Heteroaryl-Derivaten der allgemeinen Formeln **Ic** oder **If**

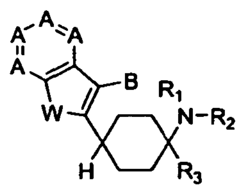

**Ic**

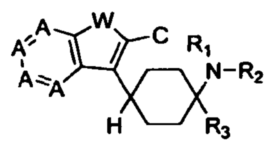

**If**

durch Reduktion der Verbindungen **Id** oder **Ie** mittels Wasserstoff in Form von HBr/Eisessig//Sn oder HCl/Sn (nascierender Wasserstoff) oder $H_2$ in Gegenwart eines Metallkatalysators wie z.B. Palladium auf Kohle, Platin auf Kohle, Platinoxid, Raney-Nickel, Rhodium oder Rutheniumkomplexen in einem geeigneten Lösungsmittel oder Lösungsmittelgemisch, beispielsweise Methanol, Ethanol, Aceton, Essigsäureethylester, HBr oder Essigsäure bei Temperaturen zwischen 0°C und 150°C.

**14.** Arzneimittel enthaltend wenigstens ein substituiertes Heteroaryl-Derivat gemäß einem der Ansprüche 1 bis 10, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz - und/oder Hilfsstoffe und/oder gegebenenfalls weiterer Wirkstoffe.

**15.** Ein substituiertes Heteroaryl-Derivat gemäß einem der Ansprüche 1 bis 10, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch

verträglichen Salze oder Salze physiologisch verträglicher Säuren oder Kationen; oder in Form seiner Solvate, insbesondere der Hydrate; zur Verwendung zur Behandlung von Schmerz, insbesondere von akutem, neuropathischem, chronischem Schmerz oder Entzündungsschmerz.

**16.** Ein substituiertes Heteroaryl-Derivat gemäß einem der Ansprüche 1 bis 10 zur Verwendung zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen - und/oder Medikamentenmißbrauch und/oder -abhängigkeit, sexuellen Dysfunktionen, cardiovaskulären Erkrankungen, Hypotension, Hypertension, Tinnitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

**1.** Substituted heteroaryl derivatives of the general formula I

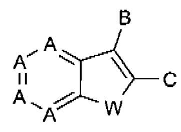

I

wherein

A represents N or $CR^{7-10}$, wherein A represents N at most twice
W represents O, S or $NR^4$
with the proviso that if W represents O or S, A denotes $CR^{7-10}$;

one of the radicals B or C represents H; $C_{1-8}$-alkyl, in each case saturated or unsaturated, branched or unbranched, mono- or polysubstituted or unsubstituted, $COR^{12}$; $SO_2R^{12}$; aryl, $C_{3-8}$-cycloalkyl or heteroaryl bonded via $C_{1-3}$-alkyl and in each case mono- or polysubstituted or unsubstituted, aryl-, or heteroaryl, in each case mono- or polysubstituted or unsubstituted; $C_{3-8}$-cycloalkyl, in each case mono- or polysubstituted or unsubstituted; and the other particular radical B or C represents

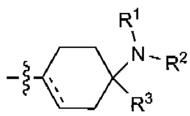

wherein

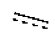

represents a single bond or a double bond,

$R^1$ and $R^2$ independently of one another represent H; $C_{1-5}$-alkyl, in each case saturated or unsaturated, branched or unbranched, mono- or polysubstituted or unsubstituted; $C_{3-8}$-cycloalkyl, in each case mono- or polysubstituted or unsubstituted; aryl or heteroaryl, in each case mono- or polysubstituted or unsubstituted; or aryl, $C_{3-8}$-cycloalkyl or heteroaryl, bonded via $C_{1-3}$-alkyl and in each case mono- or polysubstituted or unsubstituted;
or the radicals $R^1$ and $R^2$ together represent $CH_2CH_2OCH_2CH_2$, $CH_2CH_2NR^{11}CH_2CH_2$ or $(CH_2)_{3-6}$,

wherein $R^{11}$ denotes H; $C_{1-5}$-alkyl, in each case saturated or unsaturated, branched or unbranched, mono- or polysubstituted or unsubstituted: $C_{3-8}$-cycloalkyl, in each case mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkyl and in each case mono- or polysubstituted or unsubstituted; C(O)phenyl, C(O)heteroaryl, C(O)$C_{1-5}$-alkyl, in each case substituted or unsubstituted;
and
$R^3$ represents $C_{1-8}$-alkyl, in each case saturated or unsaturated, branched or unbranched, mono- or polysubstituted or unsubstituted: $C_{3-8}$-cycloalkyl, in each case mono- or polysubstituted or unsubstituted; aryl or heteroaryl, in each case unsubstituted or mono- or polysubstituted; aryl, heteroaryl or $C_{3-8}$-cycloalkyl, bonded via $C_{1-3}$-alkyl group and in each case unsubstituted or mono- or polysubstituted;
$R^4$ represents H; $C_{1-5}$-alkyl, saturated or unsaturated, branched or unbranched, unsubstituted or mono- or polysubstituted; aryl, or heteroaryl, in each case substituted or unsubstituted; aryl, heteroaryl or cycloalkyl, bonded via a $C_{1-3}$-alkyl group and in each case mono- or polysubstituted or unsubstituted; $COR^{12}$; $SO_2R^{12}$,
wherein $R^{12}$ denotes H; $C_{1-5}$-alkyl, in each case saturated or unsaturated, branched or unbranched, mono- or polysubstituted or unsubstituted; $C_{3-8}$-cycloalkyl, in each case saturated or unsaturated, mono- or polysubstituted or unsubstituted; aryl-, or heteroaryl, in each case mono- or polysubstituted or unsubstituted; or aryl, $C_{3-8}$-cycloalkyl or heteroaryl, bonded via $C_{1-3}$-alkyl and in each case mono- or polysubstituted or unsubstituted; $OR^{13}$; $NR^{14}R^{15}$;
$R^7$, $R^8$, $R^9$ and $R^{10}$ independently of one another represent
H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$, $NHC(O)NHR^{13}$, $NHC(O)R^{13}$, $NH(CNR^{13})NHR^{13}$, $SO_2NHR^{13}$; $C_{1-5}$-alkyl, $C_{3-8}$-cycloalkyl, unsubstituted or mono- or polysubstituted; aryl-, or heteroaryl, unsubstituted or mono-or polysubstituted; or aryl, $C_{3-8}$-cycloalkyl or heteroaryl, bonded via $C_{1-3}$-alkyl and unsubstituted or mono- or polysubstituted;
wherein $R^{13}$ denotes H; $C_{1-5}$-alkyl, in each case saturated or unsaturated, branched or unbranched, unsubstituted or mono- or polysubstituted; $C_{3-8}$-cycloalkyl, in each case saturated or unsaturated, unsubstituted or mono- or polysubstituted; aryl-, or heteroaryl, unsubstituted or mono- or polysubstituted; or aryl, $C_{3-8}$-cycloalkyl or heteroaryl, bonded via $C_{1-3}$-alkyl and unsubstituted or mono- or polysubstituted;
or $R^7$, $R^8$ and $R^9$ have the abovementioned meaning and $R^{10}$ together with B represents $-CH_2CH_2CH_2-$ and $R^{10}$ and B therefore form a six-membered ring,
$R^{14}$ and $R^{15}$ independently of one another denote H; $C_{1-5}$-alkyl, in each case saturated or unsaturated, branched or unbranched, unsubstituted or mono- or polysubstituted; or $C_{3-8}$-cycloalkyl, in each case saturated or unsaturated, unsubstituted or mono- or polysubstituted: aryl-, or heteroaryl, unsubstituted or mono- or polysubstituted; or aryl, $C_{3-8}$-cycloalkyl or heteroaryl, bonded via $C_{1-3}$-alkyl and unsubstituted or mono- or polysubstituted:
or $R^{14}$ and $R^{15}$ together form $CH_2CH_2OCH_2CH_2$. $CH_2CH_2NR^{16}CH_2CH_2$ or $(CH_2)_{3-6}$,
wherein $R^{16}$ denotes H; $C_{1-5}$-alkyl, saturated or unsaturated, branched or unbranched, unsubstituted or mono- or polysubstituted;
wherein the expression "cycloalkyl" or "$C_{3-8}$-cycloalkyl" represents cyclic hydrocarbons having 3, 4, 5, 6, 7 or 8 carbon atoms, wherein the hydrocarbons can be saturated or unsaturated (but not aromatic), unsubstituted or mono- or polysubstituted, and wherein saturated or unsaturated (but not aromatic) cycloalkyls in which one or two carbon atoms are replaced by a hetero atom S, N or O are also included, and wherein a cycloalkyl ring can also be condensed with a further ring, which can be saturated, unsaturated (also aromatic),
wherein the expression "aryl" represents aromatic hydrocarbons, these aryl radicals can also be condensed with further saturated, (partially) unsaturated or aromatic ring systems, and each of these aryl radicals can be unsubstituted or mono- or polysubstituted, wherein the substituents on the aryl can be identical or different and can be in any desired and possible position of the aryl.
wherein the expression "heteroaryl" represents a 5-, 6- or 7-membered cyclic aromatic radical which contains at least 1, if appropriate also 2, 3, 4 or 5 hetero atoms chosen from the group consisting of nitrogen, oxygen and sulfur, wherein the hetero atoms are identical or different and the heterocyclic ring can be unsubstituted or mono- or polysubstituted; in the case of substitution on the heterocyclic ring, the substituents can be identical or different and can be in any desired and possible position of the heteroaryl; and wherein the heterocyclic ring can also be part of a bi- or polycyclic system,
wherein the abovementioned $C_{1-8}$-alkyls, $C_{1-5}$-alkyls, $C_{1-3}$-alkyls or $C_{1-3}$-alkylenes or $C_{3-8}$-cycloalkyl radicals can in each case be mono- or polysubstituted by F, Cl, Br, I, -CN, $NH_2$, NH-$C_{1-6}$-alkyl, NH-$C_{1-6}$-alkyl-OH, N($C_{1-6}$-alkyl)$_2$, N($C_{1-6}$-alkyl-OH)$_2$, $NO_2$, SH, S-$C_{1-6}$-alkyl, S-benzyl, $OCF_3$, O-$C_{1-6}$-alkyl, OH, O-$C_{1-6}$-alkyl-OH, =O, $C_{1-6}$-alkyl, benzyl, O-benzyl, O-phenyl, C(=O)$C_{1-6}$-alkyl, $CO_2H$, NHC(=O)$C_{1-6}$-alkyl, OC(=O)$C_{1-6}$-alkyl, $CO_2$-$C_{1-6}$-alkyl,
wherein the abovementioned aryl or heteroaryl radicals can in each case be mono- or polysubstituted by F, Cl, Br, I, CN, $NH_2$, NH-$C_{1-6}$-alkyl, NH-$C_{1-6}$-alkyl-OH, N($C_{1-6}$alkyl)$_2$, N($C_{1-6}$-alkyl-OH)$_2$, $NO_2$, SH, S-$C_{1-6}$-alkyl, OH,

O-$C_{1-6}$-alkyl, O-$C_{1-6}$alkyl-OH, C(=O)$C_{1-6}$-alkyl, $CO_2H$ $CO_2$-$C_{1-6}$-alkyl, $CF_3$, $OCF_3$, $C_{1-6}$-alkyl or phenoxy, in the form of the racemate; of the enantiomers, diastereomers, mixtures of the enantiomers or diastereomers or of an individual enantiomer or diastereomer; of the bases and/or salts of physiologically acceptable acids.

**2.** Heteroaryl derivatives according to claim 1, wherein

$R^1$ and $R^2$ independently of one another represent H; $C_{1-5}$-alkyl, saturated or unsaturated, branched or unbranched, mono- or polysubstituted or unsubstituted;
or the radicals $R^1$ and $R^2$ together form a ring and denote $CH_2CH_2OCH_2CH_2$, $CH_2CH_2NR^{11}CH_2CH_2$ or $(CH_2)_{3-6}$. wherein $R^{11}$ denotes H; $C_{1-5}$-alkyl, saturated or unsaturated, branched or unbranched, mono- or polysubstituted or unsubstituted.

**3.** Heteroaryl derivatives according to claim 1 or 2, wherein

$R^1$ and $R^2$ independently of one another represent $CH_3$ or H, wherein $R^1$ and $R^2$ do not simultaneously denote H.

**4.** Substituted heteroaryl derivatives according to claim 1, wherein

$R^3$ denotes butyl, phenyl, thiophenyl, thiazolyl, cyclopentyl, cyclohexyl, naphthyl, benzyl, benzofuranyl, 1,2,4-triazolyl, benzimidazolyl, benzodioxanyl, benzodioxolanyl, pyridyl or benzothiophenyl, in each case unsubstituted or mono-or polysubstituted: phenyl, furyl or thiophenyl bonded via a saturated, unbranched $C_{1-3}$-alkyl group and in each case unsubstituted or mono- or polysubstituted.

**5.** Substituted heteroaryl derivatives according to claim 4, wherein

$R^3$ denotes phenyl, 4-fluorophenyl, benzyl, butyl or benzothiophenyl.

**6.** Substituted heteroaryl derivatives according to claim 1, wherein

B or C represents $(CH_2)_{1-4}$-$R^{21}$, wherein $R^{21}$ represents H, OH, SH, COO$C_{1-6}$-alkyl, COOH, OC(=O)$C_{1-6}$-alkyl, $NH_2$, NHC(=O)$C_{1-6}$-alkyl; or $C_{3-8}$-cycloalkyl, aryl or heteroaryl, in each case unsubstituted or mono- or polysubstituted.

**7.** Substituted heteroaryl derivatives according to claim 6, wherein

$R^{21}$ represents OH, SH, $COOCH_3$, COOH, OC(=O)$CH_3$, $NH_2$, NHC(=O)$CH_3$, NHC(=O)$CH_2C(CH_3)_2$; or benzimidazole, pyridyl, triazolyl, phenyl, pyrazolyl, tetrazolyl or imidazolyl, in each case unsubstituted or substituted by $COOCH_3$, $CH_3$: or cyclopropyl, cyclohexyl, pyrrolidinyl tetrahydroquinolinyl, pyrrolidinyl, piperidyl, tetrahydroisoquinolinyl, isoindolinyl, piperazinyl, morpholinyl or thiazolinyl, in each case unsubstituted or substituted by =O or $CH_3$.

**8.** Substituted heteroaryl derivatives according to claim 1, wherein

$R^7$, $R^8$, $R^9$ and $R^{10}$ independently of one another represent H; methyl; ethyl; propyl; butyl; pyridyl, O-benzyl, F, Cl, Br, I, CN, $CF_3$, $OCF_3$, OH, $OCH_3$, $NH_2$, COOH, $COOCH_3$, $NHCH_3$ or $N(CH_3)_2$ or $NO_2$.

**9.** Substituted heteroaryl derivatives according to claim 8, wherein

$R^7$, $R^8$, $R^9$ and $R^{10}$ independently of one another represent H, F, Cl, $NO_2$, CN, $CF_3$, $OCH_3$, $OCF_3$ or OH.

**10.** Substituted heteroaryl derivatives according to claim 1 from the group

(1) 2-(2-(4-(dimethylamino)-4-phenylcyclohexyl)-1H-indol-3-yl)ethanol, citrate (3) (±) 2-(2-(4-(dimethylamino)-4-phenylcyclohex-1-enyl)-1H-indol-3-yl)ethyl acetate hydrochloride
(4) (±) 2-(4-(dimethylamino)-4-phenylcyclohex-1-enyl)-3-(3-aminopropyl)-1H-indole, citrate
(6) (±) 3-(2-(4-(dimethylamino)-4-phenylcyclohex-1-enyl)-1H-indol-3-yl)propan-1-ol hydrochloride
(7) (±) 2-(5,6-dichloro-2-(4-(dimethylamino)-4-phenylcyclohex-1-enyl)-1H-indol-3-yl)ethanol, citrate
(8) (±)2-(2-(4-morpholino-4-phenylcyclohex-1-enyl)-1H-indol-3-yl)ethanol, citrate

(9) (±)2-(4,6-dichloro-2-(4-(dimethylamino)-4-phenylcyclohex-1-enyl)-1H-indol-3-yl)ethanol, citrate
(10) (±)2-(2-(4-(dimethylamino)-4-phenylcyclohex-1-enyl)-5-fluoro-1H-indol-3-yl)ethanol, citrate
(11) (±)2-(2-(4-(dimethylamino)-4-phenylcyclohex-1-enyl)-5-(pyridin-3-yl)-1H-indol-3-yl)ethanol, citrate
(13) (±)2-(2-(4-(dimethylamino)-4-phenylcyclohex-1-enyl)-5-nitro-1H-indol-3-yl)ethanol, citrate
(14) (±) 2-(2-(4-(benzo[b]thiophen-2-yl)-4-(dimethylamino)cyclohex-1-enyl)-1H-indol-3-yl)ethanol, citrate
(15) (±)2-(2-(4-(benzo[b]thiophen-2-yl)-4-(dimethylamino)cyclohex-1-enyl)-5-fluoro-1H-indol-3-yl)ethanol, citrate
(16) (±) 2-(4-(dimethylamino)-4-phenylcyclohex-1-enyl)-3-methyl-1H-indole, citrate
(17) N,N-dimethyl-4-(3-methyl-1H-indol-2-yl)-1-phenylcyclohexanamine, citrate
(18) N,N-dimethyl-4-(3-methyl-1H-indol-2-yl)-1-phenylcyclohexanamine, citrate
(19) 2-(2-(2-(4-(dimethylamino)-4-phenylcyclohexyl)-1H-indol-3-yl)ethyl)isoindoline-1,3-dione, citrate
(20) N-(2-(2-(4-(dimethylamino)-4-phenylcyclohexyl)-1H-indol-3-yl)ethyl)acetamide, citrate
(21) N-(2-(2-(4-(dimethylamino)-4-phenylcyclohexyl)-1H-indol-3-yl)ethyl)acetamide, citrate
(22) (±)-2-(4-benzyl-4-(dimethylamino)cyclohex-1-enyl)-3-methyl-1H-indole-5-carbonitrile
(23) (±)-2-(4-(dimethylamino)-4-phenylcyclohex-1-enyl)-3-methyl-1H-indole-5-carbonitrile
(24) (±)-2-(4-(dimethylamino)-4-butylcyclohex-1-enyl)-3-methyl-5-trifluoromethyl-1H-indole
(25) (±)-2-(4-(dimethylamino)-4-phenylcyclohex-1-enyl)-3-methyl-5-trifluoromethyl-1H-indole, citrate
(26) (±)-2-(4-(dimethylamino)-4-benzylcyclohex-1-enyl)-3-methyl-5-fluoro-1H-indole, citrate
(27) (±)-2-(4-(dimethylamino)-4-butylcyclohex-1-enyl)-3-methyl-5-fluoro-1H-indole, citrate
(28) (±)-2-(4-(dimethylamino)-4-phenylcyclohex-1-enyl)-3-methyl-5-fluoro-1H-indole, citrate
(29) (±)-2-(4-(dimethylamino)-4-phenylcyclohex-1-enyl)-3-methyl-5-methoxy-1H-indole, citrate
(30) (±)-2-(4-(dimethylamino)-4-benzylcyclohex-1-enyl)-3-methyl-1H-indole, citrate
(31) (±)-2-(4-(dimethylamino)-4-butylcyclohex-1-enyl)-3-methyl-1H-indole, citrate
(32) (±)-2-(4-(dimethylamino)-4-phenylcyclohex-1-enyl)-3-methyl-1H-pyrrolo[2,3-b]pyridine, citrate
(33) (±)-2-(4-(dimethylamino)-4-benzylcyclohex-1-enyl)-3-cyclopropyl-1H-indole hydrochloride
(34) (±)-2-(4-(dimethylamino)-4-butylcyclohex-1-enyl)-3-cyclopropyl-1H-indole
(35) (±)-2-(4-(dimethylamino)-4-phenylcyclohex-1-enyl)-3-cyclopropyl-1H-indole
(36) (±)-2-(4-(dimethylamino)-4-benzylcyclohex-1-enyl)-3-(cyclohexylmethyl)-1H-indole
(37) (±)-2-(4-(dimethylamino)-4-benzylcyclohex-1-onyl)-3-benzyl-1H-indole hydrochloride
(38) (±)-2-(4-(dimethylamino)-4-butylcyclohex-1-enyl)-3-(cyclohexylmethyl)-1H-indole hydrochloride
(39) (±)-2-(4-(dimethylamino)-4-butylcyclohex-1-enyl)-3-benzyl-1H-indole
(40) (±)-2-(4-(dimethylamino)-4-phenylcyclohex-1-enyl)-3-(cyclohexylmethyl)-1H-indole hydrochloride
(41) (±)-2-(4-(dimethylamino)-4-phenylcyclohex-1-enyl)-3-benzyl-1H-indole hydrochloride
(42) (±)-2-(4-(dimethylamino)-4-benzylcyclohex-1-enyl)-3-propyl-1H-indole
(43) (±)-2-(4-(dimethylamino)-4-butylcyclohex-1-enyl)-3-propyl-1H-indole
(44) (±)-2-(4-(dimethylamino)-4-phonylcyclohex-1-enyl)-3-propyl-1H-indole
(45) (±)2-(4-(dimethylamino)-4-benzylcyclohex-1-enyl)-3-(2-(pyridin-4-yl)ethyl)-1H-indole
(46) (±)2-(4-(dimethylamino)-4-butylcyclohex-1-enyl)-3-(2-(pyridin-4-yl)ethyl)-1H-indole
(47) (±)2-(4-(dimethylamino)-4-phenylcyclohex-1-enyl)-3-(2-(pyridin-4-yl)ethyl)-1H-indole
(48) (±)-3-(2-(4-benzyl-4-(dimethylamino)cyclohex-1-enyl)-1H-indol-3-yl)propan-1-ol, citrate
(49) (±)-3-(2-(4-butyl-4-(dimethylamino)cyclohex-1-enyl)-1H-indol-3-yl)propan-1-ol, citrate
(51) (±)2-(2-(2-(4-(dimethylamino)-4-phenylcyclohex-1-enyl)-1H-indol-3-yl)ethyl)isoindoline-1,3-dione
(52) (±)2-(4-(dimethylamino)-4-benzylcyclohex-1-enyl)-3-(2-(1H-benzo[d]imidazol-1-yl)ethyl)-5-fluoro-1H-indole
(53) (±)2-(4-(dimethylamino)-4-butylcyclohex-1-enyl)-3-(2-(1H-benzo[d]imidazol-1-yl)ethyl)-5-fluoro-1H-indole
(54) (±)2-(4-(dimethylamino)-4-phenylcyclohex-1-enyl)-3-(2-(1H-benzo[d]imidazol-1-yl)ethyl)-5-fluoro-1H-indole
(55) (±)2-(4-(dimethylamino)-4-benzylcyclohex-1-enyl)-3-(2-(piperidin-1-yl)ethyl)-5-fluoro-1H-indole
(56) (±)2-(4-(dimethylamino)-4-butylcyclohex-1-enyl)-3-(2-(piperidin-1-yl)ethyl)-5-fluoro-1H-indole
(57) (±)2-(4-(dimethylamino)-4-phenylcyclohex-1-enyl)-3-(2-(piperidin-1-yl)ethyl)-5-fluoro-1H-indole
(58) (±)2-(4-(dimethylamino)-4-phenylcyclohex-1-enyl)-3-(2-(piperidin-1-yl)ethyl)-1H-indole, citrate
(59) (±)2-(4-(dimethylamino)-4-benzylcyclohex-1-enyl)-3-(2-(1H-1,2,3-triazol-1-yl)ethyl)-5-fluoro-1H-indole
(60) (±) 2-(4-(dimethylamino)-4-butylcyclohex-1-enyl)-3-(2-(1H-1,2,3-triazol-1-yl)ethyl)-5-fluoro-1H-indole
(61) (±)2-(4-(dimethylamino)-4-phenylcyclohex-1-enyl)-3-(2-(1H-1,2,3-triazol-1-yl)ethyl)-5-fluoro-1H-indole
(62) N-(2-(2-(4-butyl-4-(dimethylamino)cyclohexyl)-1H-indol-3-yl)ethyl)-3,3-dimethylbutanamide, citrate
(63) (±)N-(2-(2-(4-(dimethylamino)-4-phenylcyclohex-1-enyl)-1H-indol-3-yl)ethyl)acetamide
(64) (±)N-(2-(2-(4-butyl-4-(dimethylamino)cyclohex-1-enyl)-1H-indol-3-yl)ethyl)-3,3-dimethylbutanamide
(65) (±)-2-(2-(4-(dimethylamino)-4-phenylcyclohex-1-enyl)-5-fluoro-6-methoxy-1H-indol-3-yl)ethanol, citrate

(66) (±)-2-(2-(4-benzyl-4-(4-methylpiperazin-1-yl)cyclohex-1-enyl)-5-fluoro-1H-indol-3-yl)ethanol
(67) (±)-2-(5-fluoro-2-(4-phenyl-4-(pyrrolidin-1-yl)cyclohex-1-enyl)-1H-indol-3-yl)ethanol
(68) 2-(4-benzyl-4-(dimethylamino)cyclohexyl)-3-methyl-1H-indole-5-carbonitrile, citrate
(69) 2-(4-benzyl-4-(dimethylamino)cyclohexyl)-3-methyl-1H-indole-5-carbonitrile, citrate
(70) 2-(4-butyl-4-(dimethylamino)cyclohexyl)-3-methyl-1H-indole-5-carbonitrile, citrate
(71) 2-(4-butyl-4-(dimethylamino)cyclohexyl)-3-methyl-1H-indole-5-carbonitrile, citrate
(72) 2-(4-(dimethylamino)-4-phenylcyclohexyl)-3-methyl-1H-indole-5-carbonitrile, citrate
(73) 1-benzyl-N,N-dimethyl-4-(3-methyl-5-(trifluoromethyl)-1H-indol-2-yl)cyclohexanamine, citrate
(74) 1-benzyl-N,N-dimethyl-4-(3-methyl-5-(trifluoromethyl)-1H-indol-2-yl)cyclohexanamine, citrate
(75) 1-butyl-N,N-dimethyl-4-(3-methyl-5-(trifluoromethyl)-1H-indol-2-yl)cyclohexanamine, citrate
(76) N,N-dimethyl-4-(3-methyl-5-(trifluoromethyl)-1H-indol-2-yl)-1-phenylcyclohexanamine, citrate
(77) 1-benzyl-4-(5-fluoro-3-methyl-1H-indol-2-yl)-N,N-dimethylcyclohexanamine, citrate
(78) 1-benzyl-4-(5-fluoro-3-methyl-1H-indol-2-yl)-N,N-dimethylcyclohexanamine, citrate
(79) 1-butyl-4-(5-fluoro-3-methyl-1H-indol-2-yl)-N,N-dimethylcyclohexanamine hydrochloride
(80) 4-(5-fluoro-3-methyl-1H-indol-2-yl)-N,N-dimethyl-1-phenylcyclohexanamine, citrate
(81) 4-(5-fluoro-3-methyl-1H-indol-2-yl)-N,N-dimethyl-1-phenylcyclohexanamine, citrate
(82) 4-(3-(2-(1H-benzo[d]imidazol-1-yl)ethyl)-5-fluoro-1H-indol-2-yl)-1-butyl-N,N-dimethylcyclohexanamine, citrate
(83) 4-(3-(2-(1H-benzo[d]imidazol-1-yl)ethyl)-5-fluoro-1H-indol-2-yl)-N,N-dimethyl-1-phenylcyclohexanamine, citrate
(84) 4-(3-(2-(1H-benzo[d]imidazol-1-yl)ethyl)-5-fluoro-1H-indol-2-yl)-N,N-dimethyl-1-phenylcyclohexanamine, citrate
(85) 1-benzyl-4-(5-fluoro-3-(2-(pyrrolidin-1-yl)ethyl)-1H-indol-2-yl)-N,N-dimethylcyclohexanamine, citrate
(86) 1-benzyl-4-(5-fluoro-3-(2-(pyrrolidin-1-yl)ethyl)-1H-indol-2-yl)-N,N-dimethylcyclohexanamine, citrate
(87) 1-butyl-4-(5-fluoro-3-(2-(pyrrolidin-1-yl)ethyl)-1H-indol-2-yl)-N,N-dimethylcyclohexanamine, citrate
(88) 1-butyl-4-(5-fluoro-3-(2-(pyrrolidin-1-yl)ethyl)-1H-indol-2-yl)-N,N-dimethylcyclohexanamine, citrate
(89) 4-(5-fluoro-3-(2-(pyrrolidin-1-yl)ethyl)-1H-indol-2-yl)-N,N-dimethyl-1-phenylcyclohexanamine, citrate
(90) 4-(5-fluoro-3-(2-(pyrrolidin-1-yl)ethyl)-1H-indol-2-yl)-N,N-dimethyl-1-phenylcyclohexanamine, citrate
(91) 1-benzyl-4-(5-fluoro-3-(2-(piperidin-1-yl)ethyl)-1H-indol-2-yl)-N,N-dimethylcyclohexanamine, citrate
(92) 1-benzyl-4-(5-fluoro-3-(2-(piperidin-1-yl)ethyl)-1H-indol-2-yl)-N,N-dimethylcyclohexanamine, citrate (2:3)
(93) 1-butyl-4-(5-fluoro-3-(2-(piperidin-1-yl)ethyl)-1H-indol-2-yl)-N,N-dimethylcyclohexanamine, citrate (4:3)
(94) 1-butyl-4-(5-fluoro-3-(2-(piperidin-1-yl)ethyl)-1H-indol-2-yl)-N,N-dimethylcyclohexanamine, citrate
(95) 4-(5-fluoro-3-(2-(piperidin-1-yl)ethyl)-1H-indol-2-yl)-N,N-dimethyl-1-phenylcyclohexanamine, citrate
(96) 4-(5-fluoro-3-(2-(piperidin-1-yl)ethyl)-1H-indol-2-yl)-N,N-dimethyl-1-phenylcyclohexanamine, citrate
(97) 4-(3-(2-(1H-pyrazol-1-yl)ethyl)-5-fluoro-1H-indol-2-yl)-1-benzyl-N,N-dimethylcyclohexanamine, citrate (2:3)
(98) 4-(3-(2-(1H-pyrazol-1-yl)ethyl)-5-fluoro-1H-indol-2-yl)-1-benzyl-N,N-dimethylcyclohexanamine, citrate
(99) 4-(3-(2-(1H-pyrazol-1-yl)ethyl)-5-fluoro-1H-indol-2-yl)-1-butyl-N,N-dimethylcyclohexanamine, citrate
(100) 4-(3-(2-(1H-pyrazol-1-yl)ethyl)-5-fluoro-1H-indol-2-yl)-1-butyl-N,N-dimethylcyclohexanamine, citrate
(101) 4-(3-(2-(1H-pyrazol-1-yl)ethyl)-5-fluoro-1H-indol-2-yl)-N,N-dimethyl-1-phenylcyclohexanamine, citrate
(102) 4-(3-(2-(1H-pyrazol-1-yl)ethyl)-5-fluoro-1H-indol-2-yl)-N,N-dimethyl-1-phenylcyclohexanamine, citrate
(103) 4-(3-(2-(1H-imidazol-1-yl)ethyl)-5-fluoro-1H-indol-2-yl)-1-benzyl-N,N-dimethylcyclohexanamine, citrate
(104) 4-(3-(2-(1H-imidazol-1-yl)ethyl)-5-fluoro-1H-indol-2-yl)-1-benzyl-N,N-dimethylcyclohexanamine, citrate
(105) 4-(3-(2-(1H-imidazol-1-yl)ethyl)-5-fluoro-1H-indol-2-yl)-1-butyl-N,N-dimethylcyclohexanamine, citrate (2:3)
(106) 4-(3-(2-(1H-imidazol-1-yl)ethyl)-5-fluoro-1H-indol-2-yl)-1-butyl-N,N-dimethylcyclohexanamine, citrate
(107) 4-(3-(2-(1H-imidazol-1-yl)ethyl)-5-fluoro-1H-indol-2-yl)-N,N-dimethyl-1-phenylcyclohexanamine, citrate
(108) 4-(3-(2-(1H-imidazol-1-yl)ethyl)-5-fluoro-1H-indol-2-yl)-N,N-dimethyl-1-phenylcyclohexanamine, citrate (2:3)
(109) 4-(3-(2-(1H-benzo[d]imidazol-1-yl)ethyl)-5-fluoro-1H-indol-2-yl)-1-benzyl-N,N-dimethylcyclohexanamine, citrate (4:3)
(110) 4-(3-(2-(1H-benzo[d]imidazol-1-yl)ethyl)-5-fluoro-1H-indol-2-yl)-1-benzyl-N,N-dimethylcyclohexanamine, citrate (2:3)
(111) 4-(3-(2-(1H-benzo[d]imidazol-1-yl)ethyl)-5-fluoro-1H-indol-2-yl)-1-butyl-N,N-dimethylcyclohexanamine, citrate (2:3)
(112) 4-(3-(2-(1H-1,2,3-triazol-1-yl)ethyl)-5-fluoro-1H-indol-2-yl)-1-benzyl-N,N-dimethylcyclohexanamine, citrate (4:1)
(113) 4-(3-(2-(1H-1,2,3-triazol-1-yl)ethyl)-5-fluoro-1H-indol-2-yl)-1-benzyl-N,N-dimethylcyclohexanamine, citrate
(114) 4-(3-(2-(1H-1,2,3-triazol-1-yl)ethyl)-5-fluoro-1H-indol-2-yl)-1-butyl-N,N-dimethylcyclohexanamine, citrate
(115) 4-(3-(2-(1H-1,2,3-triazol-1-yl)ethyl)-5-fluoro-1H-indol-2-yl)-1-butyl-N,N-dimethylcyclohexanamine, citrate
(116) 4-(3-(2-(1H-1,2,3-triazol-1-yl)ethyl)-5-fluoro-1H-indol-2-yl)-N,N-dimethyl-1-phenylcyclohexanamine, citrate
(117) 4-(3-(2-(1H-1,2,3-triazol-1-yl)ethyl)-5-fluoro-1H-indol-2-yl)-N,N-dimethyl-1-phenylcyclohexanamine, citrate
(118) 2-(4-(dimethylamino)-4-phenylcyclohexyl)-3-methyl-1H-indol-5-ol
(119) 2-(4-(dimethylamino)-4-phenylcyclohexyl)-3-methyl-1H-indol-5-ol 120) 1-benzyl-N,N-dimethyl-4-(3-methyl-5-(trifluoromethoxy)-1H-indol-2-yl)cyclohexanamine, citrate
(121) 1-butyl-N,N-dimethyl-4-(3-methyl-5-(trifluoromethoxy)-1H-indol-2-yl)cyclohexanamine, citrate
(122) 1-butyl-N,N-dimethyl-4-(3-methyl-5-(trifluoromethoxy)-1H-indol-2-yl)cyclohexanamine, citrate
(123) N,N-dimethyl-4-(3-methyl-5-(trifluoromethoxy)-1H-indol-2-yl)-1-phenylcyclohexanamine, citrate
(124) N,N-dimethyl-4-(3-methyl-5-(trifluoromethoxy)-1H-indol-2-yl)-1-phenylcyclohexanamine, citrate
(125) 1-butyl-4-(5-methoxy-3-methyl-1H-indol-2-yl)-N,N-dimethylcyclohexanamine, citrate
(126) 1-butyl-4-(5-methoxy-3-methyl-1H-indol-2-yl)-N,N-dimethylcyclohexanamine, citrate
(127) 4-(5-methoxy-3-methyl-1H-indol-2-yl)-N,N-dimethyl-1-phenylcyclohexanamine, citrate
(128) 4-(5-methoxy-3-methyl-1H-indol-2-yl)-N,N-dimethyl-1-phenylcyclohexanamine, citrate (4:3)
(129) 1-benzyl-N,N-dimethyl-4-(3-methyl-1H-indol-2-yl)cyclohexanamine, citrate
(130) 1-benzyl-N,N-dimethyl-4-(3-methyl-1H-indol-2-yl)cyclohexanamine, citrate
(131) 1-butyl-N,N-dimethyl-4-(3-methyl-1H-indol-2-yl)cyclohexanamine, citrate
(132) 1-butyl-N,N-dimethyl-4-(3-methyl-1H-indol-2-yl)cyclohexanamine, citrate
(133) 1-benzyl-4-(3-cyclopropyl-1H-indol-2-yl)-N,N-dimethylcyclohexanamine, citrate
(134) 1-benzyl-4-(3-cyclopropyl-1H-indol-2-yl)-N,N-dimethylcyclohexanamine, citrate
(135) 1-butyl-4-(3-cyclopropyl-1H-indol-2-yl)-N,N-dimethylcyclohexanamine hydrochloride
(136) 4-(3-cyclopropyl-1H-indol-2-yl)-N,N-dimethyl-1-phenylcyclohexanamine, citrate
(137) methyl 2-(2-(4-(dimethylamino)-4-phenylcyclohexyl)-1H-indol-3-yl)acetate, citrate
(138) methyl 2-(2-(4-(dimethylamino)4-phenylcyclohexyl)-1H-indol-3-yl)acetate, citrate
(139) 1-benzyl-4-(3-(cyclohexylmethyl)-1H-indol-2-yl)-N,N-dimethylcyclohexanamine, citrate
(140) 1-benzyl-4-(3-(cyclohexylmethyl)-1H-indol-2-yl)-N,N-dimethylcyclohexanamine, citrate
(141) 1-benzyl-4-(3-benzyl-1H-indol-2-yl)-N,N-dimethylcyclohexanamine, citrate
(142) 1-benzyl-4-(3-benzyl-1H-indol-2-yl)-N,N-dimethylcyclohexanamine, citrate
(143) 1-butyl-4-(3-(cyclohexylmethyl)-1H-indol-2-yl)-N,N-dimethylcyclohexanamine, citrate
(144) 1-butyl-4-(3-(cyclohexylmethyl)-1H-indol-2-yl)-N,N-dimethylcyclohexanamine hydrochloride
(145) 4-(3-benzyl-1H-indol-2-yl)-1-butyl-N,N-dimethylcyclohexanamine, citrate
(146) 4-(3-benzyl-1H-indol-2-yl)-1-butyl-N,N-dimethylcyclohexanamine, citrate
(147) 4-(3-(cyclohexylmethyl)-1H-indol-2-yl)-N,N-dimethyl-1-phenylcyclohexanamine, citrate
(148) 4-(3-(cyclohexylmethyl)-1H-indol-2-yl)-N,N-dimethyl-1-phenylcyclohexanamine, citrate
(149) 4-(3-benzyl-1H-indol-2-yl)-N,N-dimethyl-1-phenylcyclohexanamine, citrate
(150) 4-(3-benzyl-1H-indol-2-yl)-N,N-dimethyl-1-phenylcyclohexanamine, citrate
(151) N,N-dimethyl-1-phenyl-4-(3-(pyridin-2-ylmethyl)-1H-indol-2-yl)cyclohexanamine
(152) N,N-dimethyl-1-phenyl-4-(3-(pyridin-2-ylmethyl)-1H-indol-2-yl)cyclohexanamine
(153) 3-(2-(4-(dimethylamino)-4-phenylcyclohexyl)-1H-indol-3-yl)propanoic acid hydrochloride
(156) 1-benzyl-N,N-dimethyl-4-(3-propyl-1H-indol-2-yl)cyclohexanamine, citrate
(157) 1-butyl-N,N-dimethyl-4-(3-propyl-1H-indol-2-yl)cyclohexanamine hydrochloride
(158) 1-butyl-N,N-dimethyl-4-(3-propyl-1H-indol-2-yl)cyclohexanamine, citrate
(159) N,N-dimethyl-1-phenyl-4-(3-propyl-1H-indol-2-yl)cyclohexanamine, citrate
(160) N,N-dimethyl-1-phenyl-4-(3-propyl-1H-indol-2-yl)cyclohexanamine, citrate
(161) 1-benzyl-N,N-dimethyl-4-(3-(2-(pyridin-4-yl)ethyl)-1H-indol-2-yl)cyclohexanamine, citrate
(162) 1-benzyl-N,N-dimethyl-4-(3-(2-(pyridin-4-yl)ethyl)-1H-indol-2-yl)cyclohexanamine, citrate
(163) 1-butyl-N,N-dimethyl-4-(3-(2-(pyridin-4-yl)ethyl)-1H-indol-2-yl)cyclohexanamine, citrate
(164) 1-butyl-N,N-dimethyl-4-(3-(2-(pyridin-4-yl)ethyl)-1H-indol-2-yl)cyclohexanamine, citrate
(165) N,N-dimethyl-1-phenyl-4-(3-(2-(pyridin-4-yl)ethyl)-1H-indol-2-yl)cyclohexanamine, citrate (1:4)
(166) N,N-dimethyl-1-phenyl-4-(3-(2-(pyridin-4-yl)ethyl)-1H-indol-2-yl)cyclohexanamine, citrate
(167) N,N-dimethyl-4-(3-(2-(1-methyl-1H-benzo[d]imidazol-2-yl)ethyl)-1H-indol-2-yl)-1-phenylcyclohexanamine, citrate
(168) N,N-dimethyl-4-(3-(2-(1-methyl-1H-benzo[d]imidazol-2-yl)ethyl)-1H-indol-2-yl)-1-phenylcyclohexanamine, citrate

(169) N,N-dimethyl-1-phenyl-4-(3-(2-(pyridin-2-yl)ethyl)-1H-indol-2-yl)cyclohexanamine, citrate
(170) N,N-dimethyl-1-phenyl-4-(3-(2-(pyridin-2-yl)ethyl)-1H-indol-2-yl)cyclohexanamine, citrate (2:3)
(171) 4-(2-(4-benzyl-4-(dimethylamino)cyclohexyl)-1H-indol-3-yl)butanoic acid hydrochloride
(172) 4-(2-(4-(dimethylamino)-4-phenylcyclohexyl)-1H-indol-3-yl)butanoic acid hydrochloride
(173) 4-(2-(4-(dimethylamino)-4-phenylcyclohexyl)-1H-indol-3-yl)butan-1-ol hydrochloride
(174) 4-(2-(4-benzyl-4-(dimethylamino)cyclohexyl)-1H-indol-3-yl)butyl acetate hydrochloride
(175) 4-(2-(4-benzyl-4-(dimethylamino)cyclohexyl)-1H-indol-3-yl)butyl acetate hydrochloride
(176) 4-(2-(4-butyl-4-(dimethylamino)cyclohexyl)-1H-indol-3-yl)butyl acetate hydrochloride
(177) 4-(2-(4-butyl-4-(dimethylamino)cyclohexyl)-1H-indol-3-yl)butyl acetate hydrochloride
(178) 4-(2-(4-(dimethylamino)-4-phenylcyclohexyl)-1H-indol-3-yl)butyl acetate hydrochloride
(179) 4-(2-(4-(dimethylamino)-4-phenylcyclohexyl)-1H-indol-3-yl)butyl acetate hydrochloride
(180) 3-(2-(4-benzyl-4-(dimethylamino)cyclohexyl)-1H-indol-3-yl)propan-1-ol, citrate
(181) 3-(2-(4-butyl-4-(dimethylamino)cyclohexyl)-1H-indol-3-yl)propan-1-ol hydrochloride
(182) 3-(2-(4-butyl-4-(dimethylamino)cyclohexyl)-1H-indol-3-yl)propan-1-ol hydrochloride
(183) 3-(2-(4-(dimethylamino)-4-phenylcyclohexyl)-1H-indol-3-yl)propan-1-ol hydrochloride
(184) 3-(2-(4-(dimethylamino)-4-phenylcyclohexyl)-1H-indol-3-yl)propan-1-ol hydrochloride
(185) 3-(2-(4-(dimethylamino)-4-phenylcyclohexyl)-1H-indol-3-yl)propyl acetate hydrochloride
(186) 3-(2-(4-(dimethylamino)-4-phenylcyclohexyl)-1H-indol-3-yl)propyl acetate hydrochloride
(187) 1-(2-(2-(4-(dimethylamino)-4-phenylcyclohexyl)-1H-indol-3-yl)ethyl)pyrrolidine-2,5-dione
(188) 1-(2-(2-(4-(dimethylamino)-4-phenylcyclohexyl)-1H-indol-3-yl)ethyl)pyrrolidine-2,5-dione
(189) 4-(3-(2-(3,4-dihydroquinolin-1(2H)-yl)ethyl)-1H-indol-2-yl)-N,N-dimethyl-1-phenylcyclohexanamine, citrate (4:3)
(190) 4-(3-(2-(3,4-dihydroquinolin-1(2H)-yl)ethyl)-1H-indol-2-yl)-N,N-dimethyl-1-phenylcyclohexanamine, citrate
(191) methyl 1-(2-(2-(4-(dimethylamino)-4-phenylcyclohexyl)-1H-indol-3-yl)ethyl)-1H-1,2,3-triazole-4-carboxylate, citrate
(192) methyl 1-(2-(2-(4-(dimethylamino)-4-phenylcyclohexyl)-1H-indol-3-yl)ethyl)-1H-1,2,3-triazole-4-carboxylate, citrate
(193) 4-(3-(2-(isoindolin-2-yl)ethyl)-1H-indol-2-yl)-N,N-dimethyl-1-phenylcyclohexanamine, citrate
(194) 4-(3-(2-(isoindolin-2-yl)ethyl)-1H-indol-2-yl)-N,N-dimethyl-1-phenylcyclohexanamine, citrate
(195) 4-(3-(2-(3,4-dihydroisoquinolin-2(1H)-yl)ethyl)-1H-indol-2-yl)-N,N-dimethyl-1-phenylcyclohexanamine, citrate
(196) 4-(3-(2-(3,4-dihydroisoquinolin-2(1H)-yl)ethyl)-1H-indol-2-yl)-N,N-dimethyl-1-phenylcyclohexanamine, citrate (2:3)
(197) N,N-dimethyl-1-phenyl-4-(3-(2-(pyrrolidin-1-yl)ethyl)-1H-indol-2-yl)cyclohexanamine, citrate
(198) N,N-dimethyl-1-phenyl-4-(3-(2-(pyrrolidin-1-yl)ethyl)-1H-indol-2-yl)cyclohexanamine, citrate
(199) N,N-dimethyl-1-phenyl-4-(3-(2-(piperidin-1-yl)ethyl)-1H-indol-2-yl)cyclohexanamine, citrate
(200) N,N-dimethyl-4-(3-(2-(4-methylpiperazin-1-yl)ethyl)-1H-indol-2-yl)-1-phenylcyclohexanamine, citrate
(201) N,N-dimethyl-4-(3-(2-morpholinoethyl)-1H-indol-2-yl)-1-phenylcyclohexanamine, citrate
(202) N,N-dimethyl-4-(3-(2-morpholinoethyl)-1H-indol-2-yl)-1-phenylcyclohexanamine, citrate
(203) 4-(3-(2-(1H-benzo[d]imidazol-1-yl)ethyl)-1H-indol-2-yl)-N,N-dimethyl-1-phenylcyclohexanamine, citrate
(204) 4-(3-(2-(1H-benzo[d]imidazol-1-yl)ethyl)-1H-indol-2-yl)-N,N-dimethyl-1-phenylcyclohexanamine, citrate
(205) 4-(3-(2-(1H-imidazol-1-yl)ethyl)-1H-indol-2-yl)-N,N-dimethyl-1-phenylcyclohexanamine, citrate
(206) 4-(3-(2-(1H-imidazol-1-yl)ethyl)-1H-indol-2-yl)-N,N-dimethyl-1-phenylcyclohexanamine, citrate
(207) 4-(3-(2-(1H-1,2,4-triazol-1-yl)ethyl)-1H-indol-2-yl)-N,N-dimethyl-1-phenylcyclohexanamine, citrate
(208) 4-(3-(2-(1H-1,2,4-triazol-1-yl)ethyl)-1H-indol-2-yl)-N,N-dimethyl-1-phenylcyclohexanamine, citrate
(209) N,N-dimethyl-1-phenyl-4-(3-(2-(thiazolidin-3-yl)ethyl)-1H-indol-2-yl)cyclohexanamine, citrate
(210) N,N-dimethyl-1-phenyl-4-(3-(2-(thiazolidin-3-yl)ethyl)-1H-indol-2-yl)cyclohexanamine, citrate
(211) N,N-dimethyl-4-(3-(2-(5-methyl-2H-tetrazol-2-yl)ethyl)-1H-indol-2-yl)-1-phenylcyclohexanamine, citrate
(212) N,N-dimethyl-4-(3-(2-(5-methyl-2H-tetrazol-2-yl)ethyl)-1H-indol-2-yl)-1 phenylcyclohexanamine, citrate
(213) 4-(3-(2-(1H-pyrazol-1-yl)ethyl)-1H-indol-2-yl)-N,N-dimethyl-1-phenylcyclohexanamine, citrate
(214) 4-(3-(2-(1H-pyrazol-1-yl)ethyl)-1H-indol-2-yl)-N,N-dimethyl-1-phenylcyclohexanamine, citrate
(215) 4-(3-(2-(1H-1,2,3-triazol-1-yl)ethyl)-1H-indol-2-yl)-N,N-dimethyl-1-phenylcyclohexanamine, citrate
(216) 4-(3-(2-(1H-1,2,3-triazol-1-yl)ethyl)-1H-indol-2-yl)-N,N-dimethyl-1-phenylcyclohexanamine, citrate
(217) N,N-dimethyl-4-(3-(2-(5-methyl-1H-tetrazol-1-yl)ethyl)-1H-indol-2-yl)-1-phenylcyclohexanamine, citrate
(218) N,N-dimethyl-4-(3-(2-(5-methyl-1H-tetrazol-1-yl)ethyl)-1H-indol-2-yl)-1-phenylcyclohexanamine, citrate
(219) 2-(2-(4-(dimethylamino)-4-phenylcyclohexyl)-1H-indol-3-yl)ethanol
(220) 2-(2-(4-(dimethylamino)-4-phenylcyclohexyl)-1H-indol-3-yl)ethyl acetate

(221) N,N-dimethyl-4-(3-methyl-1H-pyrrolo[3,2-c]pyridin-2-yl)-1-phenylcyclohexanamine, citrate
(222) N,N-dimethyl-4-(3-methyl-1H-pyrrolo[3,2-c]pyridin-2-yl)-1-phenylcyclohexanamine, citrate
(223) N,N-dimethyl-4-(3-methyl-1H-pyrrolo[2,3-b]pyridin-2-yl)-1-phenylcyclohexanamine, citrate
(224) N,N-dimethyl-4-(3-methyl-1H-pyrrolo[2,3-b]pyridin-2-yl)-1-phenylcyclohexanamine, citrate
(225) N,N-dimethyl-4-(3-methylbenzofuran-2-yl)-1-phenylcyclohexanamine, citrate
(226) N,N-dimethyl-4-(3-methylbenzofuran-2-yl)-1-phenylcyclohexanamine, citrate
(227) 4-(1H-indol-3-yl)-N,N-dimethyl-1-phenylcyclohexanamine, citrate
(228) (±)-3-(4-(dimethylamino)-4-benzylcyclohex-1-enyl)-1H-pyrrolo[2,3-b]pyridine
(229) (±)-3-(4-(dimethylamino)-4-butylcyclohex-1-enyl)-1H-pyrrolo[2,3-b]pyridine
(230) (±)-3-(4-(dimethylamino)-4-phenylcyclohex-1-enyl)-1H-pyrrolo[2,3-b]pyridine, citrate
(231) (±)-4-(1H-indol-3-yl)-N,N-dimethyl-1-phenylcyclohex-3-enamine, citrate
(232) (±)-4-(1H-indol-3-yl)-N,N-dimethyl-1-phenylcyclohex-3-enamine
(233) (±)-2-(3-(4-(dimethylamino)-4-phenylcyclohex-1-enyl)benzo[b]thiophen-2-yl)ethanol hydrochloride
(234) (±)-2-(3-(4-(dimethylamino)-4-(pyridin-2-yl)cyclohex-1-enyl)benzo[b]thiophen-2-yl)ethanol hydrochloride
(235) 4-(1H-indol-2-yl)-N,N-dimethyl-1-phenylcyclohexanamine, citrate
(236) 4-(1H-indol-2-yl)-N,N-dimethyl-1-phenylcyclohexanamine, citrate
(237) 1-benzyl-N,N-dimethyl-4-(1H-pyrrolo[2,3-b]pyridin-3-yl)cyclohexanamine hydrochloride
(238) 1-butyl-N,N-dimethyl-4-(1H-pyrrolo[2,3-b]pyridin-3-yl)cyclohexanamine hydrochloride
(239) (±)-4-(benzofuran-2-yl)-1-benzyl-N,N-dimethylcyclohex-3-enamine hydrochloride
(240) (±)-N,N-dimethyl-4-(3-methylbenzofuran-2-yl)-1-phenylcyclohex-3-enamine, citrate
(241) (±)-2-(2-(4-(dimethylamino)-4-phenylcyclohex-1-enyl)benzofuran-3-yl)ethanethiol, citrate
(242) (±)-N,N-dimethyl-4-(3-methylbenzo[b]thiophen-2-yl)-1-phenylcyclohex-3-enamine, citrate
(243) N,N-dimethyl-4-(3-methylbenzo[b]thiophen-2-yl)-1-phenylcyclohexanamine, citrate
(244) N,N-dimethyl-1-phenyl-4-(1H-pyrrolo[2,3-b]pyridin-3-yl)cyclohexanamine, citrate
(246) 1-(dimethylamino)-3-(2-(4-(dimethylamino)-4-phenylcyclohexyl)-3-methyl-1H-indol-1-yl)propan-2-ol
(247) N,N-dimethyl-4-(3-methyl-1-(oxiran-2-ylmethyl)-1H-indol-2-yl)-1-phenylcyclohexanamine
(248) 4-(1,3-dimethyl-1H-indol-2-yl)-N,N-dimethyl-1-phenylcyclohexanamine
(249) 2-(4-butyl-4-(dimethylamino)cyclohexyl)-3-methyl-1H-indol-5-ol, citrate
(250) 2-(4-butyl-4-(dimethylamino)cyclohexyl)-3-methyl-1H-indol-5-ol, citrate
(251) (±)-2-(4-(dimethylamino)-4-(thiophen-2-yl)-cyclohex-1-enyl)-3-(2-(pyridin-4-yl)ethyl)-1H-indole
(252) (±)-2-(4-(dimethylamino)-4-(3-fluorophenyl)-cyclohex-1-enyl)-3-(2-(pyridin-4-yl)ethyl)-1H-indole
(253) (±)-2-(4-butyl-4-(pyrrolidin-1-yl)cyclohex-1-enyl)-3-(2-(pyridin-4-yl)ethyl)-1H-indole
(254) (±)-2-(4-(methylamino)-4-phenylcyclohex-1-enyl)-3-(2-(pyridin-4-yl)ethyl)-1H-indole
(255) (±)-2-(4-(dimethylamino)-4-(thiophen-2-yl)-cyclohex-1-enyl)-3-methyl-1H-indole, citrate
(256) N,N-dimethyl-4-(3-methyl-1H-indol-2-yl)-1-(thiophen-2-yl)cyclohexanamine, citrate
(257) N,N-dimethyl-4-(3-methyl-1H-indol-2-yl)-1-(thiophen-2-yl)cyclohexanamine, citrate
(258) 2-(4-butyl-4-(pyrrolidin-1-yl)cyclohex-1-enyl)-3-methyl-1H-indole
(259) N-methyl-4-(3-methyl-1H-indol-2-yl)-1-phenylcyclohexanamine hydrobromide
(260) N-methyl-4-(3-methyl-1H-indol-2-yl)-1-phenylcyclohexanamine, citrate
(261) 1-(3-fluorophenyl)-N,N-dimethyl-4-(3-methyl-1H-indol-2-yl)cyclohexanamine, citrate
(262) 1-(3-fluorophenyl)-N,N-dimethyl-4-(3-methyl-1H-indol-2-yl)cyclohexanamine, citrate
(263) (±)-2-(4-(dimethylamino)-4-(3-fluorophenyl)-cyclohex-1-enyl)-3-methyl-1H-indole
(264) 2-(4-benzyl-4-(dimethylamino)cyclohexyl)-3-methyl-1H-indol-5-ol, citrate
(265) 2-(4-benzyl-4-(dimethylamino)cyclohexyl)-3-methyl-1H-indol-5-ol, citrate
(266) 2-(4-butyl-4-(pyrrolidin-1-yl)cyclohexyl)-3-(2-(pyridin-4-yl)ethyl)-1H-indole, citrate
(267) 2-(4-butyl-4-(pyrrolidin-1-yl)cyclohexyl)-3-(2-(pyridin-4-yl)ethyl)-1H-indole, citrate
(268) 2-(4-(azetidin-1-yl)-4-phenylcyclohexyl)-3-methyl-1H-indole, citrate
(269) 2-(4-(azetidin-1-yl)-4-phenylcyclohexyl)-3-methyl-1H-indole, citrate
(270) 2-4-(azetidin-1-yl)-4-phenylcyclohexyl)-3-(2-(pyridin-4-yl)ethyl)-1H-indole hydrochloride
(271) 2-(4-(azetidin-1-yl)-4-phenylcyclohexyl)-3-(2-(pyridin-4-yl)ethyl)-1H-indole hydrochloride
(272) 2-(4-butyl-4-(pyrrolidin-1-yl)cyclohexyl)-3-methyl-1H-indole, citrate
(273) 3-(2-(4-(dimethylamino)-4-phenylcyclohexyl)-1H-pyrrolo[2,3-b]pyridin-3-yl)propan-1-ol, citrate
(274) 1-(2-(4-(dimethylamino)-4-phenylcyclohexyl)-3-methyl-1H-indol-1-yl)-3-(methylamino)propan-2-ol, citrate
(275) 1-benzyl-3-(2-(2-(4-(dimethylamino)-4-phenylcyclohexyl)-1H-indol-3-yl)ethyl)urea, citrate
(276) 1-benzyl-3-(2-(2-(4-(dimethylamino)-4-phenylcyclohexyl)-1H-indol-3-yl)ethyl)urea, citrate
(277) 1-(2-(2-(4-(dimethylamino)-4-phenylcyclohexyl)-1H-indol-3-yl)ethyl)-3-phenylurea, citrate
(278) 1-(2-(2-(4-(dimethylamino)-4-phenylcyclohexyl)-1H-indol-3-yl)ethyl)-3-phenylurea, citrate

(279) 1-cyclopentyl-3-(2-(2-(4-(dimethylamino)-4-phenylcyclohexyl)-1H-indol-3-yl)ethyl)urea, citrate
(280) 1-cyclopentyl-3-(2-(2-(4-(dimethylamino)-4-phenylcyclohexyl)-1H-indol-3-yl)ethyl)urea, citrate
(281) N-(2-(2-(4-(dimethylamino)-4-phenylcyclohexyl)-1H-indol-3-yl)ethyl)-cyclopentanesulfonamide, citrate
(282) N-(2-(2-(4-(dimethylamino)-4-phenylcyclohexyl)-1H-indol-3-yl)ethyl)cyclopentanesulfonamide, citrate
(283) N-(2-(2-(4-(dimethylamino)-4-phenylcyclohexyl)-1H-indol-3 yl)ethyl)benzenesulfonamide, citrate
(284) N-(2-(2-(4-(dimethylamino)-4-phenylcyclohexyl)-1H-indol-3-yl)ethyl)thiophene-2-sulfonamide, citrate
(285) N-(2-(2-(4-(dimethylamino)-4-phenylcyclohexyl)-1H-indol-3-yl)ethyl)thiophene-2-sulfonamide, citrate
(286) N-(2-(2-(4-(dimethylamino)-4-phenylcyclohexyl)-1H-indol-3-yl)ethyl)nicotinamide, citrate
(287) N-(2-(2-(4-(dimethylamino)-4-phenylcyclohexyl)-1H-indol-3-yl)ethyl)nicotinamide, citrate
(288) N-(2-(2-(4-(dimethylamino)-4-phenylcyclohexyl)-1H-indol-3-yl)ethyl)benzamide, citrate
(289) 1-(3-fluorophenyl)-N,N-dimethyl-4-(3-(2-(pyridin-4-yl)ethyl)-1H-indol-2-yl)cyclohexanamine
(290) 1-(3-fluorophenyl)-N,N-dimethyl-4-(3-(2-(pyridin-4-yl)ethyl)-1H-indol-2-yl)cyclohexanamine, citrate
(291) N-methyl-1-phenyl-4-(3-(2-(pyridin-4-yl)ethyl)-1H-indol-2-yl)cyclohexanamine, citrate
(292) N-methyl-1-phenyl-4-(3-(2-(pyridin-4-yl)ethyl)-1H-indol-2-yl)cyclohexanamine, citrate
(293) 2-(4-butyl-4-(pyrrolidin-1-yl)cyclohexyl)-3-methyl-1H-indole, citrate
(294) N,N-dimethyl-1-phenyl-4-(1-(phenylsulfonyl)-1H-indol-2-yl)cyclohexanamine
(295) N,N-dimethyl-1-phenyl-4-(1-(phenylsulfonyl)-1H-indol-2-yl)cyclohexanamine
(296) N,N-dimethyl-4-(3-methyl-1-(oxiran-2-ylmethyl)-1H-indol-2-yl)-1-phenylcyclohex-3-enamine
(297) N,N-dimethyl-4-(3-methyl-1-(oxiran-2-ylmethyl)-1H-indol-2-yl)-1-phenylcyclohexanamine, citrate
(298) 1-(dimethylamino)-3-(2-(4-(dimethylamino)-4-phenylcyclohex-1-enyl)-3-methyl-1H-indol-1-yl)propan-2-ol, citrate
(299) 1-(dimethylamino)-3-(2-(4-(dimethylamino)-4-phenylcyclohexyl)-3-methyl-1H-indol-1-yl)propan-2-ol, citrate
(300) 2-(3-(4-(dimethylamino)-4-phenylcyclohex-1-enyl)-1H-indol-1-yl)ethanol hydrochloride
(301) (±) 3-(4-(dimethylamino)-4-phenylcyclohex-1-enyl)-(1-(phenylsulfonyl)-1H-indole) hydrochloride
(302) 1-benzyl-N,N-dimethyl-4-(1-methyl-1H-indol-2-yl)cyclohex-3-enamine;
hydrochloride
(303) N,N-dimethyl-4-(3-(2-(pyridin-4-yl)ethyl)-1H-indol-2-yl)-1-(thiophen-2-yl)cyclohexanamine hydrochloride
(304) N,N-dimethyl-4-(3-(2-(pyridin-4-yl)ethyl)-1H-indol-2-yl)-1-(thiophen-2-yl)cyclohexanamine hydrochloride
(305) N-methyl-4-(3-methyl-1H-indol-2-yl)-1-(4-methylthiazol-2-yl)cyclohexanamine
(306) N-methyl-4-(3-methyl-1H-indol-2-yl)-1-(4-methylthiazol-2-yl)cyclohexanamine
(307) 2-(2-(4-(dimethylamino)-4-phenylcyclohexyl)-1H-indol-3-yl)ethanol
in the form of the racemate; of the enantiomers, diastereomers, mixtures of the enantiomers or diastereomers or of an individual enantiomer or diastereomer; of the bases and/or salts of physiologically acceptable acids.

**11.** Process for the preparation of substituted heteroaryl derivatives of the formula Ic

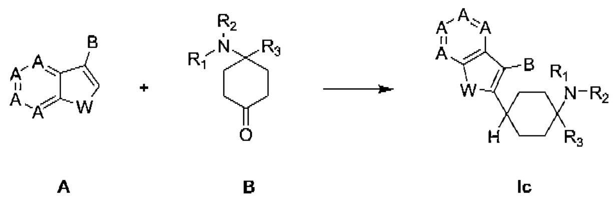

wherein ketones of the general formula B are reacted with heteroaromatics of the general formula A in organic solvents or solvent mixtures, for example ethyl acetate, chloroform, methylene chloride (MC), dichloroethane (DCE), diethyl ether ($Et_2O$), acetonitrile (MeCN) or nitromethane, with the addition of an organic or inorganic acid, for example HCl, HBr, trifluoromethanesulfonic acid, methanesulfonic acid, acetic acid, trifluoroacetic acid, or without a solvent in an organic or inorganic acid or acid mixtures at temperatures of between 0 °C and 150 °C, optionally using microwave irradiation, and then reacted with the addition of an organic or inorganic reducing agent, e.g. triethylsilane or tin powder, at temperatures of between 0 °C and 150 °C, optionally using microwave irradiation.

**12.** Process for the preparation of substituted heteroaryl derivatives of the general formula **Id** or **Ie**

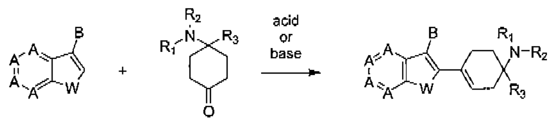


**A** **B** **Id**

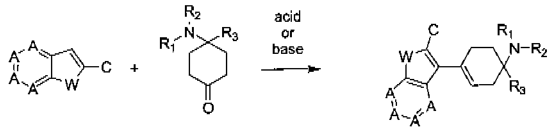


**A'** **B** **Ie**

wherein heteroaromatics of the general formula **A** or **A'** are reacted with cyclohexanones of the general formula **B** in organic solvents or solvent mixtures, for example chloroform, methylene chloride (DCM), dichloroethane (DCE), diethyl ether ($Et_2O$), acetonitrile (MeCN) or nitromethane, with the addition of an organic or inorganic acid, for example HCl, HBr, trifluoromethanesulfonic acid, methanesulfonic acid, acetic acid, trifluoroacetic acid, at temperatures of between 0 °C and 150 °C, optionally using microwave irradiation
or heteroaromatics of the general formula **A** or **A'** are reacted with cyclohexanones of the general formula **B** with the addition of a base, for example, KOH or NaOH, in an organic solvent, for example methanol, at temperatures of between 20 and 100 °C.

**13.** Process for the preparation of substituted heteroaryl derivatives of the general formulae **Ic** or **If**

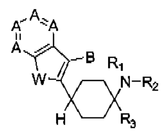

**Ic**

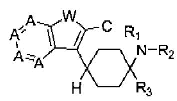

**If**

by reduction of the compounds **Id** or Ie by means of hydrogen in the form of HBr/glacial acetic acid/Sn or HCl/Sn (nascent hydrogen) or $H_2$ in the presence of a metal catalyst, such as e.g. palladium on charcoal, platinum on charcoal, platinum oxide, Raney nickel, rhodium or ruthernium complexes, in a suitable solvent or solvent mixture, for example methanol, ethanol, acetone, ethyl acetate, HBr or acetic acid, at temperatures of between 0 °C and 150 °C.

**14.** Medicament containing at least one substituted heteroaryl derivative according to one of claims 1 to 10, optionally in the form of its racemate, of the pure stereoisomers, in particular enantiomers and diastereomers, in any desired mixture ratio: in the form of its acids or of its bases or in the form of its salts, in particular the physiologically acceptable salts or salts of physiologically acceptable acids or cations; or in the form of its solvates, in particular the hydrates, and optionally containing suitable additives and/or auxiliary substances and/or optionally further active compounds.

**15.** A substituted heteroaryl derivative according to one of claims 1 to 10, optionally in the form of its racemate, of the pure stereoisomers, in particular enantiomers and diastereomers, in any desired mixture ratio; in the form of its acids or of its bases or in the form of its salts, in particular the physiologically acceptable salts or salts of physiologically acceptable acids or cations; or in the form of its solvates, in particular the hydrates; for use for treatment of pain, in particular acute, neuropathic, chronic pain or inflammation pain.

**16.** A substituted heteroaryl derivative according to one of claims 1 to 10 for use for treatment of anxiety states, of stress and syndromes associated with stress, depression, epilepsy, Alzheimer's disease, senile dementia, catalepsy, general cognitive dysfunctions, learning and memory disorders (as a nootropic), withdrawal symptoms, alcohol and/or drug and/or medicament abuse and/or dependency, sexual dysfunctions, cardiovascular diseases, hypotension, hypertension, tinnitus, pruritus, migraine, impaired hearing, lack of intestinal motility, impaired food intake, anorexia, obesity, locomotor disorders, diarrhoea, cachexia, urinary incontinence or as a muscle relaxant, anticonvulsive or anaesthetic or for co-administration in treatment with an opioid analgesic or with an anaesthetic, for diuresis or antinatriuresis, anxiolysis, for modulation of motor activity, for modulation of neurotransmitter secretion and treatment of neurodegenerative diseases associated therewith, for treatment of withdrawal symptoms and/or

for reduction of the addiction potential of opioids.

## Revendications

1. Dérivés hétéroaryliques substitués de formule générale I

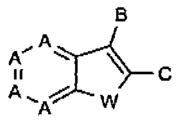

I

dans laquelle

A représente N ou $CR^{7-10}$, A représentant au plus deux fois N,
W représente O, S ou $NR^4$,
à condition qu'A signifie CR $^{7-10}$ lorsque W représente O ou S ;
un des radicaux B ou C représente H ; alkyle en $C_{1-6}$ à chaque fois saturé ou insaturé, ramifié ou non ramifié, substitué une ou plusieurs fois ou non substitué, $COR^{12}$ ; $SO_2R^{12}$ ; aryle, cycloalkyle en $C_3$-$_8$ ou hétéroaryle relié par alkyle en $C_{1-3}$, à chaque fois substitué une ou plusieurs fois ou non substitué ; aryle ou hétéroaryle, à chaque fois substitué une ou plusieurs fois ou non substitué ; cycloalkyle en $C_3$-$_8$, à chaque fois substitué une ou plusieurs fois ou non substitué ; et l'autre radical B ou C représente

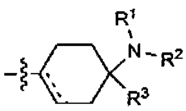

,

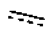

représentant une simple liaison ou une double liaison,

$R^1$ et $R^2$ représentant indépendamment l'un de l'autre H ; alkyle en $C_{1-5}$ à chaque fois saturé ou insaturé, ramifié ou non ramifié, substitué une ou plusieurs fois ou non substitué ; cycloalkyle en $C_{3-8}$, à chaque fois substitué une ou plusieurs fois ou non substitué ; aryle ou hétéroaryle, à chaque fois substitué une ou plusieurs fois ou non substitué ; ou aryle, cycloalkyle en $C_{3-8}$ ou hétéroaryle relié par alkyle en $C_{1-3}$, à chaque fois substitué une ou plusieurs fois ou non substitué ;
ou les radicaux $R^1$ et $R^2$ représentant ensemble $CH_2CH_2OCH_2CH_2$, $CH_2CH_2NR^{11}CH_2CH_2$ ou $(CH_2)_{3-6}$,
$R^{11}$ signifiant H ; alkyle en $C_{1-5}$ à chaque fois saturé ou insaturé, ramifié ou non ramifié, substitué une ou plusieurs fois ou non substitué ; cycloalkyle en $C_{3-8}$, à chaque fois substitué une ou plusieurs fois ou non substitué ; aryle ou hétéroaryle, à chaque fois substitué une ou plusieurs fois ou non substitué ; ou aryle, cycloalkyle en $C_{3-9}$ ou hétéroaryle relié par alkyle en $C_{1-3}$, à chaque fois substitué une ou plusieurs fois ou non substitué ; C(0)phényle, C(O)hétéroaryle, C(O)alkyle en $C_{1-5}$, à chaque fois substitué ou non substitué ;
et
$R^3$ représentant alkyle en $C_{1-8}$ à chaque fois saturé ou insaturé, ramifié ou non ramifié, substitué une ou plusieurs fois ou non substitué ; cycloalkyle en $C_{3-8}$ à chaque fois substitué une ou plusieurs fois ou non substitué ; aryle ou hétéroaryle, à chaque fois non substitué ou substitué une ou plusieurs fois ; aryle, hétéroaryle ou cycloalkyle en $C_{3-8}$ relié par alkyle en $C_{1-3}$, à chaque fois non substitué ou substitué une ou plusieurs fois,
$R^4$ représentant H ; alkyle en $C_{1-5}$ saturé ou insaturé, ramifié ou non ramifié, non substitué ou substitué une ou plusieurs fois ; aryle ou hétéxoaryle, à chaque fois substitué ou non substitué ; aryle, hétéroaryle ou cycloalkyle relié par un groupe alkyle en $C_{1-3}$, à chaque fois substitué une ou plusieurs fois ou non substitué ; $COR^{12}$ ; $SO_2R^{12}$,
$R^{12}$ signifiant H ; alkyle en $C_{1-5}$ à chaque fois saturé ou insaturé, ramifié ou non ramifié, substitué une ou plusieurs fois ou non substitué ; cycloalkyle en $C_{3-8}$, à chaque fois saturé ou insaturé, substitué une ou plusieurs

fois ou non substitué ; aryle ou hétéroaryle, à chaque fois substitué une ou plusieurs fois ou non substitué ; ou aryle, cycloalkyle en $C_{3-8}$ ou hétéroaryle relié par alkyle en $C_{1-3}$, à chaque fois substitué une ou plusieurs fois ou non substitué ; $OR^{13}$ ; $NR^{14}R^{15}$ ;

$R^7$, $R^8$, $R^9$ et $R^{10}$ représentant indépendamment les uns des autres

H, F, Cl, Br, I, $NO_2$, $CF_3$, $OR^{13}$, $SR^{13}$, $SO_2R^{13}$, $SO_2OR^{13}$, CN, $COOR^{13}$, $NR^{14}R^{15}$, $NHC(O)NHR^{13}$, $NHC(O)R^{13}$, $NH(CNR^{13})NHR^{13}$, $SO_2NHR^{13}$ ; alkyle en $C_{1-5}$, cycloalkyle en $C_{3-8}$, non substitué ou substitué une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ;

$R^{13}$ signifiant H ; alkyle en $C_{1-5}$, à chaque fois saturé ou insaturé, ramifié ou non ramifié, non substitué ou substitué une ou plusieurs fois, cycloalkyle en $C_{3-8}$, à chaque fois saturé ou insaturé, non substitué ou substitué une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ;

ou $R^7$, $R^8$ et $R^9$ ayant la signification donnée précédemment et $R^{10}$ représentant avec B $-CH_2CH_2CH_2-$ et $R^{10}$ et B formant ainsi un cycle à six éléments,

$R^{14}$ et $R^{15}$ signifiant indépendamment l'un de l'autre H ; alkyle en $C_{1-5}$, à chaque fois saturé ou insaturé, ramifié ou non ramifié, non substitué ou substitué une ou plusieurs fois ; ou cycloalkyle en $C_{3-8}$, à chaque fois saturé ou insaturé, non substitué ou substitué une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ; ou $R^{14}$ et $R^{15}$ formant ensemble $CH_2CH_2OCH_2CH_2$, $CH_2CH_2NR^{16}CH_2CH_2$ ou $(CH_2)_{3-6}$,

$R^{16}$ signifiant H ; alkyle en $C_{1-5}$, saturé ou insaturé, ramifié ou non ramifié, non substitué ou substitué une ou plusieurs fois ;

le terme « cycloalkyle » ou « cycloalkyle en $C_{3-8}$ » représentant des hydrocarbures cycliques de 3, 4, 5, 6, 7 ou 8 atomes de carbone, les hydrocarbures pouvant être saturés ou insaturés (mais pas aromatiques), non substitués ou substitués une ou plusieurs fois, et les cycloalkyles saturés ou insaturés (mais pas aromatiques) dans lesquels un ou deux atomes de carbone sont remplacés par un hétéroatome S, N ou 0 étant compris, et un cycle cycloalkyle pouvant également être condensé avec un autre cycle, qui peut être saturé ou insaturé (également aromatique),

le terme « aryle » représentant des hydrocarbures aromatiques, ces radicaux aryle pouvant également être condensés avec d'autres systèmes cycliques saturés, (partiellement) insaturés ou aromatiques, et chacun de ces radicaux aryle pouvant être non substitué ou substitué une ou plusieurs fois, les substituants de l'aryle pouvant être identiques ou différents et se trouver à toute position quelconque et possible de l'aryle,

le terme « hétéroaryle » représentant un radical aromatique cyclique de 5, 6 ou 7 éléments, qui contient au moins 1, éventuellement également 2, 3, 4 ou 5 hétéroatomes choisis dans le groupe constitué par l'azote, l'oxygène et le soufre, les hétéroatomes étant identiques ou différents et l'hétérocycle pouvant être non substitué ou substitué une ou plusieurs fois ; les substituants pouvant être identiques ou différents et se trouver à toute position quelconque et possible de l'hétéroaryle en cas de substitution de l'hétérocycle ; et l'hétérocycle pouvant également faire partie d'un système bi- ou polycyclique,

les radicaux alkyle en $C_{1-8}$, alkyle en $C_{1-5}$, alkyle en $C_{1-3}$ ou alkylène en $C_{1-3}$ ou cycloalkyle en $C_{3-8}$ mentionnés précédemment pouvant à chaque fois être substitués une ou plusieurs fois avec F, Cl, Br, I, -CN, $NH_2$, N-alkyle en $C_{1-6}$, NH-alkyle en $C_{1-6}$-OH, N (alkyle en $C_{1-6})_2$, N (alkyle en $C_{1-6}$-OH$)_2$, $NO_2$, SH, S-alkyle en $C_{1-6}$, S-benzyle, $OCF_3$, O-alkyle en $C_{1-6}$, OH, O-alkyle en $C_{1-6}$-OH, =0, alkyle en $C_{1-6}$, benzyle, O-benzyle, 0-phényle, C(=O)-alkyle en $C_{1-6}$, $CO_2H$, NHC(=O)alkyle en $C_{1-6}$, OC(=O)alkyle en $C_{1-6}$, $CO_2$-alkyle en $C_{1-6}$,

les radicaux aryle ou hétéroaryle mentionnés précédemment pouvant à chaque fois être substitués une ou plusieurs fois avec F, Cl, Br, I, CN, $NH_2$, NH-alkyle en $C_{1-6}$, NH-alkyle en $C_{1-6}$-OH, N (alkyle en $C_{1-6})_2$, N (alkyle en $C_{1-6}$-OH$)_2$, $NO_2$, SH, S-alkyle en $C_{1-6}$, OH, O-alkyle en $C_{1-6}$, O-alkyle en $C_{1-6}$-OH, C(=O)-alkyle en $C_{1-6}$, $CO_2H$, $CO_2$-alkyle en $C_{1-6}$, $CF_3$, $OCF_3$, alkyle en $C_{1-6}$ ou phénoxy,

sous la forme du racémat ; des énantiomères, des diastéréomères, de mélanges des énantiomères ou des diastéréomères, ou d'un énantiomère ou diastéréomère individuel ; des bases et/ou des sels d'acides physiologiquement compatibles.

**2.** Dérivés hétéroaryliques selon la revendication 1, dans lesquels

$R^1$ et $R^2$ représentent indépendamment l'un de l'autre H; alkyle en $C_{1-5}$, saturé ou insaturé, ramifié ou non ramifié, substitué une ou plusieurs fois ou non substitué ;

ou les radicaux $R^1$ et $R^2$ forment ensemble un cycle et signifient $CH_2CH_2OCH_2CH_2$, $CH_2CH_2NR^{11}CH_2CH_2$ ou $(CH_2)_{3-6}$,

$R^{11}$ signifiant H ; alkyle en $C_{1-5}$, saturé ou insaturé, ramifié ou non ramifié, substitué une ou plusieurs fois ou non substitué.

**3.** Dérivés hétéroaryliques selon la revendication 1 ou 2, dans lesquels $R^1$ et $R^2$ représentent indépendamment l'un de l'autre $CH_3$ ou H, $R^1$ et $R^2$ ne signifiant pas simultanément H.

**4.** Dérivés hétéroaryliques substitués selon la revendication 1, dans lesquels
$R^3$ signifie butyle, phényle, thiophényle, thiazolyle, cyclopentyle, cyclohexyle, naphtyle, benzyle, benzofuranyle, 1,2,4-triazolyle, benzimidazolyle, benzodioxanyle, benzodioxolanyle, pyridyle ou benzothiophényle, à chaque fois non substitué ou substitué une ou plusieurs fois ; phényle, furyle ou thiophényle relié par un groupe alkyle en $C_{1-3}$ saturé, non ramifié, à chaque fois non substitué ou substitué une ou plusieurs fois.

**5.** Dérivés hétéroaryliques substitués selon la revendication 4, dans lesquels
$R^3$ signifie phényle, 4-fluorophényle, benzyle, butyle ou benzothiophényle.

**6.** Dérivés hétéroaryliques substitués selon la revendication 1, dans lesquels
B ou C représente $(CH_2)_{1-4}$-$R^{21}$, $R^{21}$ représentant H, OH, SH, COO-alkyle en $C_{1-6}$, COOH, OC(=O)-alkyle en $C_{1-6}$, $NH_2$, NHC(=O)-alkyle en $C_{1-6}$ ; ou cycloalkyle en $C_{3-8}$, aryle ou hétéroaryle, à chaque fois non substitué ou substitué une ou plusieurs fois.

**7.** Dérivés hétéroaryliques substitués selon la revendication 6, dans lesquels
$R^{21}$ représente OH, SH, $COOCH_3$, COOH, $OC(=O)CH_3$, $NH_2$, $NHC(=O)CH_3$, $NHC(=O)CH_2C(CH_3)_3$ ; ou benzimidazole, pyridyle, triazolyle, phényle, pyrazolyle, tétrazolyle ou imidazolyle, à chaque fois non substitué ou substitué avec $COOCH_3$, $CH_3$; ou cyclopropyle, cyclohexyle, pyrrolidinyle, tétrahydroquinolinyle, pyrrolidinyle, pipéridyle, tétrahydroisoquinolinyle, isoindolinyle, pipérazinyle, morpholinyle ou thiazolinyle, à chaque fois non substitué ou substitué avec =0 ou $CH_3$.

**8.** Dérivés hétéroaryliques substitués selon la revendication 1, dans lesquels
$R^7$, $R^8$, $R^9$ et $R^{10}$ représentent indépendamment les uns des autres H ; méthyle ; éthyle ; propyle ; butyle ; pyridyle, O-benzyle, F, Cl, Br, I, CN, $CF_3$, $OCF_3$, OH, $OCH_3$, $NH_2$, COOH, $COOCH_3$, $NHCH_3$ ou $N(CH_3)_2$ ou $NO_2$.

**9.** Dérivés hétéroaryliques substitués selon la revendication 8, dans lesquels
$R^7$, $R^8$, $R^9$ et $R^{10}$ représentent indépendamment les uns des autres H, F, Cl, $NO_2$, CN, $CF_3$, $OCH_3$, $OCF_3$ ou OH.

**10.** Dérivés hétéroaryliques substitués selon la revendication 1, du groupe constitué par

(1) 2-(2-(4-(diméthylamino)-4-phénylcyclohexyl)-1H-indol-3-yl)éthanol, citrate
(3) acétate de (±)2-(2-(4-(diméthylamino)-4-phénylcyclohex-1-ényl)-1H-indol-3-yl)éthyle, chlorhydrate,
(4) (±)-2-(4-(diméthylamino)-1-phénylcyclohex-1-ényl)-3-(3-aminopropyl)-1H-indole, citrate
(6) (±)-3-(2-(4-(diméthylamino)-4-phénylcyclohex-1-ényl)-1H-indol-3-yl)propan-1-ol, chlorhydrate
(7) (±)-2-(5,6-dichloro-2-(4-(diméthylamino)-4-phénylcyclohex-1-ényl)-1H-indol-3-yl)éthanol, citrate
(8) (±)-2-(2-(4-morpholino-4-phénylcyclohex-1-ényl)-1H-indol-3-yl)éthanol, citrate
(9) (±)-2-(4,6-dichloro-2-(4-(diméthylamino)-4-phénylcyclohex-1-ényl)-1H-indol-3-yl)éthanol, citrate
(10) (±)-2-(2-(4-(diméthylamino)-4-phénylcyclohex-1-ényl)-5-fluoro-1H-indol-3-yl)éthanol, citrate
(11) (±)-2-(2-(4-(diméthylamino)-4-phénylcyclohex-1-ényl)-5-(pyridin-3-yl)-1H-indol-3-yl)éthanol, citrate
(13) (±)-2-(2-(4-(diméthylamino)-4-phénylcyclohex-1-ényl)-5-nitro-1H-indol-3-yl)éthanol, citrate
(14) (±)-2-(2-(4-(benzo[b]thiophén-2-yl)-4-(diméthylamino)cyclohex-1-ényl)-1H-indol-3-yl)éthanol, citrate
(15) (±)-2-(2-(4-(benzo[b]thiophén-2-yl)-4-(diméthylamino)cyclohex-1-ényl)-5-fluoro-1H-indol-3-yl)éthanol, citrate
(16) (±)-2-(4-(diméthylamino)-4-phénylcyclohex-1-ényl)-3-méthyl-1H-indole, citrate
(17) N,N-diméthyl-4-(3-méthyl-1H-indol-2-yl)-1-phénylcyclohexanamine, citrate
(18) N,N-diméthyl-4-(3-méthyl-1H-indol-2-yl)-1-phénylcyclohexanamine, citrate
(19) 2-(2-(2-(4-(diméthylamino)-4-phénylcyclohexyl)-1H-indol-3-yl)éthyl)isoindoléine-1,3-dione, citrate
(20) N-(2-(2-(4-(diméthylamino)-4-phénylcyclohexyl)-1H-indol-3-yl)éthyl)acétamide, citrate
(21) N-(2-(2-(4-(diméthylamino)-4-phénylcyclohexyl)-1H-indol-3-yl)éthyl)acétamide, citrate
(22) (±)-2-(4-benzyl-4-(diméthylamino)cyclohex-ényl)-3-méthyl-1H-indol-5-carbonitrile
(23) (±)-2-(4-(diméthylamino)-4-phénylcyclohex-1-ényl)-3-méthyl-1H-indol-5-carbonitrile
(24) (±)-2-(4-(diméthylamino)-4-butylcyclohex-1-ényl)-3-méthyl-5-trifluorométhyl-1H-indole
(25) (±)-2-(4-(diméthylamino)-4-phénylcyclohex-1-ényl)-3-méthyl-5-trifluorométhyl-1H-indole, citrate
(26) (±)-2-(4-(diméthylamino)-4-benzylcyclohex-1-ényl)-3-méthyl-5-fluoro-1H-indole, citrate
(27) (±)-2-(4-(diméthylamino)-4-butylcyclohex-1-ényl)-3-méthyl-5-fluoro-1H-indole, citrate

(28) (±)-2-(4-(diméthylamino)-4-phénylcyclohex-1-ényl)-3-méthyl-5-fluoro-1H-indole, citrate
(29) (±)-2-(4-(diméthylamino)-4-phénylcyclohex-1-ényl)-3-méthyl-5-méthoxy-1H-indole, citrate
(30) (±)-2-(4-(diméthylamino)-4-benzylcyclohex-1-ényl)-3-méthyl-1H-indole, citrate
(31) (±)-2-(4-(diméthylamino)-4-butylcyclohex-1-ényl)-3-méthyl-1H-indole, citrate
(32) (±)-2-(4-(diméthylamino)-4-phénylcyclohex-1-ényl)-3-méthyl-1H-pyrrolo[2,3-b]pyridine, citrate
(33) (±)-2-(4-(diméthylamino)-4-benzylcyclohex-1-ényl)-3-cyclopropyl-1H-indole, chlorhydrate
(34) (±)-2-(4-(diméthylamino)-4-butylcyclohex-1-ényl)-3-cyclopropyl-1H-indole
(35) (±)-2-(4-(diméthylamino)-4-phénylcyclohex-1-ényl)-3-cyclopropyl-1H-indole
(36) (±)-2-(4-(diméthylamino)-4-benzylcyclohex-1-ényl)-3-(cyclohexylméthyl)-1H-indole
(37) (±)-2-(4-(diméthylamino)-4-benzylcyclohex-1-ényl)-3-benzyl-1H-indole, chlorhydrate
(38) (±)-2-(4-(diméthylamino)-4-butylcyclohex-1-ényl)-3-(cyclohexylméthyl)-1H-indole, chlorhydrate
(39) (±)-2-(4-(diméthylamino)-4-butylcyclohex-1-ényl)-3-benzyl-1H-indole
(40) (±)-2-(4-(diméthylamino)-4-phénylcyclohex-1-ényl)-3-(cyclohexylméthyl)-1H-indole, chlorhydrate
(41) (±)-2-(4-(diméthylamino)-4-phénylcyclohex-1-ényl)-3-benzyl-1H-indole, chlorhydrate
(42) (±)-2-(4-(diméthylamino)-4-benzylcyclohex-1-ényl)-3-propyl-1H-indole
(43) (±)-2-(4-(diméthylamino)-4-butylcyclohex-1-ényl)-3-propyl-1H-indole
(44) (±)-2-(4-(diméthylamino)-4-phénylcyclohex-1-ényl)-3-propyl-1H-indole
(45) (±)-2-(4-(diméthylamino)-4-benzylcyclohex-1-ényl)-3-(2-(pyridin-4-yl)éthyl)-1H-indole
(46) (±)-2-(4-(diméthylamino)-4-butylcyclohex-1-ényl)-3-(2-(pyridin-4-yl)éthyl)-1H-indole
(47) (±)-2-(4-(diméthylamino)-4-phénylcyclohex-1-ényl)-3-(2-(pyridin-4-yl)éthyl)-1H-indole
(48) (±)-3-(2-(4-benzyl-4-(diméthylamino)cyclohex-1-ényl)-1H-indol-3-yl)propan-1-ol, citrate
(49) (±)-3-(2-(4-butyl-4-(diméthylamino)cyclohex-1-ényl)-1H-indol-3-yl)propan-1-ol, citrate
(51) (±)-2-(2-(2-(4-(diméthylamino)-4-phénylcyclohex-1-ényl)-1H-indol-3-yl)éthyl)isoindoléine-1,3-dione
(52) (±)-2-(4-(diméthylamino)-4-benzylcyclohex-1-ényl)-3-(2-(1H-benzo[d]imidazol-1-yl)éthyl)-5-fluoro-1H-indole
(53) (±)-2-(4-(diméthylamino)-4-butylcyclohex-1-ényl)-3-(2-(1H-benzo[d]imidazol-1-yl)éthyl)-5-fluoro-1H-indole
(54) (±)-2-(4-(diméthylamino)-4-phénylcyclohex-1-ényl)-3-(2-(1H-benzo[d]imidazol-1-yl)éthyl)-5-fluoro-1H-indole
(55) (±)2-(4-(diméthylamino)-4-benzylcyclohex-1-ényl)-3-(2-(pipéridin-1-yl)éthyl)-5-fluoro-1H-indole
(56) (±)-2-(4-(diméthylamino)-4-butylcyclohex-1-ényl)-3-(2-(pipéridin-1-yl)éthyl)-5-fluoro-1H-indole
(57) (±)-2-(4-{diméthylamino)-4-phénylcyclohex-1-ényl)-3-(2-(pipéridin-1-yl)éthyl)-5-fluoro-1H-indole
(58) (±)-2-(4-(diméthylamino)-4-phénylcyclohex-1-ényl)-3-(2-(pipéridin-1-yl)éthyl)-1H-indole, citrate
(59) (±)-2-(4-(diméthylamino)-4-benzylcyclohex-1-ényl)-3-(2-(1H-1,2,3-triazol-1-yl)éthyl)-5-fluoro-1H-indole
(60) (±)-2-(4-(diméthylamino)-4-butylcyclohex-1-ényl)-3-(2-(1H-1,2,3-triazol-1-yl)éthyl)-5-fluoro-1H-indole
(61) (±)-2-(4-(diméthylamino)-4-phénylcyclohex-1-ényl)-3-(2-(1H-1,2,3-triazol-1-yl)éthyl)-5-fluoro-1H-indole
(62) N-(2-(2-(4-butyl-4-(diméthylamino)cyclohexyl)-1H-indol-3-yl)éthyl)-3,3-diméthylbutanamide, citrate
(63) (±)-N-(2-(2-(4-(diméthylamino)-4-phénylcyclohex-1-ényl)-1H-indol-3-yl)éthyl)acétamide
(64) (±)-N-(2-(2-(4-butyl-4-(diméthylamino)cyclohex-1-ényl)-1H-indol-3-yl)éthyl)-3,3-diméthylbutanamide
(65) (±)-2-(2-(4-(diméthylamino)-4-phénylcyclohex-1-ényl)-5-fluoro-6-méthoxy-1H-indol-3-yl)éthanol, citrate
(66) (±)-2-(2-(4-benzyl-4-(4-méthylpipérazin-1-yl)cyclohex-1-ényl)-5-fluoro-1H-indol-3-yl)éthanol
(67) (±)-2-(5-fluoro-2-(4-phényl-4-(pyrrolidin-1-yl)cyclohex-1-ényl)-1H-indol-3-yl)éthanol
(68) 2-(4-benzyl-4-(diméthylamino)cyclohexyl)-3-méthyl-1H-indol-5-carbonitrile, citrate
(69) 2-(4-benzyl-4-(diméthylamino)cyclohexyl)-3-méthyl-1H-indol-5-carbonitrile, citrate
(70) 2-(4-butyl-4-(diméthylamino)cyclohexyl)-3-méthyl-1H-indol-5-carbonitrile, citrate
(71) 2-(4-butyl-4-(diméthylamino)cyclohexyl)-3-méthyl-1H-indol-5-carbonitrile, citrate
(72) 2-(4-(diméthylamino)-4-phénylcyclohexyl)-3-méthyl-1H-indol-5-carbonitrile, citrate
(73) 1-benzyl-N,N-diméthyl-4-(3-méthyl-5-(trifluorométhyl)-1H-indol-2-yl)cyclohexanamine, citrate
(74) 1-benzyl-N,N-diméthyl-4-(3-méthyl-5-(trifluorométhyl)-1H-indol-2-yl)cyclohexanamine, citrate
(75) 1-butyl-N,N-diméthyl-4-(3-méthyl-5-(trifluorométhyl)-1H-indol-2-yl)cyclohexanamine, citrate
(76) N,N-diméthyl-4-(3-méthyl-5-(trifluorométhyl)-1H-indol-2-yl)-1-phénylcyclohexanamine, citrate
(77) 1-benzyl-4-(5-fluoro-3-méthyl-1H-indol-2-yl)-N,N-diméthylcyclohexanamine, citrate
(78) 1-benzyl-4-(5-fluoro-3-méthyl-1H-indol-2-yl)-N,N-diméthylcyclohexanamine, citrate
(79) 1-butyl-4-(5-fluoro-3-méthyl-1H-indol-2-yl)-N,N-diméthylcyclohexanamine, chlorhydrate
(80) 4-(5-fluoro-3-méthyl-1H-indol-2-yl)-N,N-diméthyl-1-phénylcyclohexanamine, citrate
(81) 4-(5-fluoro-3-méthyl-1H-indol-2-yl)-N,N-diméthyl-1-phénylcyclohexanamine, citrate
(82) 4-(3-(2-(1H-benzo[d]imidazol-1-yl)éthyl)-5-fluoro-1H-indol-2-yl)-1-butyl-N,N-diméthylcyclohexanamine, citrate

(83) 4-(3-(2-(1H-benzo[d]imidazol-1-yl)éthyl)-5-fluoro-1H-indol-2-yl)-N,N-diméthyl-1-phénylcyclohexanamine, citrate
(84) 4-(3-(2-(1H-benzo[d]imidazol-1-yl)éthyl)-5-fluoro-1H-indol-2-yl)-N,N-diméthyl-1-phénylcyclohexanamine, citrate
(85) 1-benzyl-4-(5-fluoro-3-(2-(pyrrolidin-1-yl)éthyl)-1H-indol-2-yl)-N,N-diméthylcyclohexanamine, citrate
(86) 1-benzyl-4-(5-fluoro-3-(2-(pyrrolidin-1-yl)éthyl)-1H-indol-2-yl)-N,N-diméthylcyclohexanamine, citrate
(87) 1-butyl-4-(5-fluoro-3-(2-(pyrrolidin-1-yl)éthyl)-1H-indol-2-yl)-N,N-diméthylcyclohexanamine, citrate
(88) 1-butyl-4-(5-fluoro-3-(2-(pyrrolidin-1-yl)éthyl)-1H-indol-2-yl)-N,N-diméthylcyclohexanamine, citrate
(89) 4-(5-fluoro-3-(2-(pyrrolidin-1-yl)éthyl)-1H-indol-2-yl)-N,N-diméthyl-1-phénylcyclohexanamine, citrate
(90) 4-(5-fluoro-3-(2-(pyrrolidin-1-yl)éthyl)-1H-indol-2-yl)-N,N-diméthyl-1-phénylcyclohexanamine, citrate
(91) 1-benzyl-4-(5-fluoro-3-(2-(pipéridin-1-yl)éthyl)-1H-indol-2-yl)-N,N-diméthylcyclohexanamine, citrate
(92) 1-bonzyl-4-(5-fluor-3-(2-(pipéridin-1-yl)éthyl)-1H-indol-2-yl)-N,N-diméthylcyclohexanamine, citrate (2:3)
(93) 1-butyl-4-(5-fluor-3-(2-(pipéridin-1-yl)éthyl)-1H-indol-2-yl)-N,N-diméthylcyclohexanamine, citrate (4:3)
(94) 1-butyl-4-(5-fluoro-3-(2-(pipéridin-1-yl)éthyl)-1H-indol-2-yl)-N,N-diméthylcyclohexanamine, citrate
(95) 4-(5-fluoro-3-(2-(pipéridin-1-yl)éthyl)-1H-indol-2-yl)-N,N-diméthyl-1-phénylcyclohexanamine, citrate
(96) 4-(5-fluoro-3-(2-(pipéridin-1-yl)éthyl)-1H-indol-2-yl)-N,N-diméthyl-1-phénylcyclohexanamine, citrate
(97) 4-(3-(2-(1H-pyrazol-1-yl)éthyl)-5-fluoro-1H-indol-2-yl)-1-benzyl-N,N-diméthylcyclohexanamine, citrate (2:3)
(98) 4-(3-(2-(1H-pyrazol-1-yl)éthyl)-5-fluoro-1H-indol-2-yl)-1-benzyl-N,N-diméthylcyclohexanamine, citrate
(99) 4-(3-(2-(1H-pyrazol-1-yl)éthyl)-5-fluoro-1H-indol-2-yl)-1-butyl-N,N-diméthylcyclohexanamine, citrate
(100) 4-(3-(2-(1H-pyrazol-1-yl)éthyl)-5-fluoro-1H-indol-2-yl)-1-butyl-N,N-diméthylcyclohexanamine, citrate
(101) 4-(3-(2-(1H-pyrazol-1-yl)éthyl)-5-fluoro-1H-indol-2-yl)-N,N-diméthyl-1-phénylcyclohexanamine, citrate
(102) 4-(3-(2-(1H-pyrazol-1-yl)éthyl)-5-fluoro-1H-indol-2-yl)-N,N-diméthyl-1-phénylcyclohexanamine, citrate
(103) 4-(3-(2-(1H-imidazol-1-yl)étnyl)-5-fluoro-1H-indol-2-yl)-1-benzyl-N,N-diméthylcyclohexanamine, citrate
(104) 4-(3-(2-(1H-imidazol-1-yl)éthyl)-5-fluoro-1H-indol-2-yl)-1-benzyl-N,N-diméthylcyclohexanamine, citrate
(105) 4-(3-(2-(1H-imidazol-1-yl)éthyl)-5-fluoro-1H-indol-2-yl)-1-butyl-N,N-diméthylcyclohexanamine, citrate (2:3)
(106) 4-(3-(2-(1H-imidazol-1-yl)éthyl)-5-fluoro-1H-indol-2-yl)-1-butyl-N,N-diméthylcyclohexanamine, citrate
(107) 4-(3-(2-(1H-imidazol-1-yl)éthyl)-5-fluoro-1H-indol-2-yl)-N,N-diméthyl-1-phénylcyclohexanamine, citrate
(108) 4-(3-(2-(1H-imidazol-1-yl)éthyl)-5-fluoro-1H-indol-2-yl)-N,N-diméthyl-1-phénylcyclohexanamine, citrate (2:3)
(109) 4-(3-(2-(1H-benzo[d]imidazol-1-yl)éthyl)-5-fluoro-1H-indol-2-yl)-1-benzyl-N,N-diméthylcyclohexanamine, citrate (4:3)
(110) 4-(3-(2-(1H-benzo[d]imidazol-1-yl)éthyl)-5-fluoro-1H-indol-2-yl)-1-benzyl-N,N-diméthylcyclohexanamine, citrate (2:3)
(111) 4-(3-(2-(1H-benzo[d]imidazol-1-yl)éthyl)-5-fluoro-1H-indol-2-yl)-1-butyl-N,N-diméthylcyclohexanamine, citrate (2:3)
(112) 9-(3-(2-(1H-1,2,3-triazol-1-yl)éthyl)-5-fluoro-1H-indol-2-yl)-1-benzyl-N,N-diméthylcyclohexanamine, citrate (4:1)
(113) 4-(3-(2-(1H-1,2,3-triazol-1-yl)éthyl)-5-fluoro-1H-indol-2-yl)-1-benzyl-N,N-diméthylcyclohexanamine, citrate
(114) 4-(3-(2-(1H-1,2,3-triazol-1-yl)éthyl)-5-fluoro-1H-indol-2-yl)-1-butyl-N,N-diméthylcyclohexanamine, citrate
(115) 4-(3-(2-(1H-1,2,3-triazol-1-yl)éthyl)-5-fluoro-1H-indol-2-yl)-1-butyl-N,N-diméthylcyclohexanamine, citrate
(116) 4-(3-(2-(1H-1,2,3-triazol-1-yl)éthyl)-5-fluoro-1H-indol-2-yl)-N,N-diméthyl-1-phénylcyclohexanamine, citrate
(117) 4-(3-(2-(1H-1,2,3-triazol-1-yl)éthyl)--5-fluoro-1H-indol-2-yl)-N,N-diméthyl-1-phénylcyclohexanamine, citrate
(118) 2-(4-(diméthylamino)-4-phénylcyclohexyl)-3-méthyl-1H-indol-5-ol
(119) 2-(4-(diméthylamino)-4-phénylcyclohexyl)-3-méthyl-1H-indol-5-ol
(120) 1-benzyl-N,N-diméthyl-4-(3-méthyl-5-(trifluorométhoxy)-1H-indol-2-yl)cyclohexanamine, citrate
(121) 1-butyl-N,N-diméthyl-4-(3-méthyl-5-(trifluorométhoxy)-1H-indol-2-yl)cyclohexanamine, citrate
(122) 1-butyl-N,N-diméthyl-4-(3-méthyl-5-(trifluorométhoxy)-1H-indol-2-yl)cyclohexanami.ne, citrate
(123) N,N-diméthyl-4-(3-méthyl-5-(trifluorométhoxy)-1H-indol-2-y1)-1-phénylcyclohexanamine, citrate
(124) N,N-diméthyl-4-(3-méthyl-5-(trifluorométhoxy)-1H-indol-2-yl)-1-phénylcyclohexanamine, citrate
(125) 1-butyl-4-(5-méthozy-3-méthyl-1H-indol-2-yl)-N,N-diméthylcyclohexanamine, citrate
(126) 1-butyl-4-(5-méthoxy-3-méthyl-1H-indol-2-yl)-N,N-diméthylcyclohexanamine, citrate
(127) 4-(5-méthoxy-3-méthyl-1H-indol-2-yl)-N,N-diméthyl-1-phénylcyclohexanamine, citrate
(128) 4-(5-méthoxy-3-méthyl-1H-indol-2-yl)-N,N-diméthyl-1-phénylcyclohexanamine, citrate (4:3)

(129) 1-benzyl-N,N-diméthyl-4-(3-méthyl-1H-indol-2-yl)cyclohexanamine, citrate
(130) 1-benzyl-N,N-diméthyl-4-(3-méthyl-1H-indol-2-yl)cyclohexanamine, citrate
(131) 1-butyl-N,N-diméthyl-4-(3-méthyl-1H-indol-2-yl)cyclohexanamine, citrate
(132) 1-butyl-N,N-diméthyl-4-(3-méthyl-1H-indol-2-yl)cyclohexanamine, citrate
(133) 1-benzyl-4-(3-cyclopropyl-1H-indol-2-yl)-N,N-diméthylcyclohexanamine, citrate
(134) 1-benzyl-4-(3-cyclopropyl-1H-indol-2-yl)-N,N-diméthylcyclohexanamine, citrate
(135) 1-butyl-4-(3-cyclopropyl-1H-indol-2-yl)-N,N-diméthylcyclohezanamine, chlorhydrate
(136) 4-(3-cyclopropyl-1H-indol-2-yl)-N,N-diméthyl-1-phénycyclohexanamine, citrate
(137) 2-(2-(9-(diméthylamino)-4-phénylcyclohexyl)-1H-indol-3-yl)acétate de méthyle, citrate
(138) 2-(2-(4-(diméthylamino)-4-phénylcyclohexyl)-1H-indol-3-yl)acétate de méthyle, citrate
(139) 1-benzyl-4-(3-(cyclohexylméthyl)-1H-indol-2-yl)-N,N-diméthylcyclohexanamine, citrate
(140) 1-benzyl-4-(3-(cyclohexylméthyl)-1H-indol-2-yl)-N,N-diméthylcyclohexanamine, citrate
(141) 1-benzyl-4-(3-benzyl-1H-indol-2-yl)-N,N-diméthylcyclohexanamine, citrate
(142) 1-benzyl-4-(3-benzyl-1H-indol-2-yl)-N,N-diméthylcyclohexanamine, citrate
(143) 1-butyl-4-(3-(cyclohexylméthyl)-1H-indol-2-yl)-N,N-diméthylcyclohexanamine, citrate
(144) 1-butyl-4-(3-(cyclohexylméthyl)-1H-indol-2-yl)-N,N-diméthylcyclohexanamine, chlorhydrate
(145) 4-(3-benzyl-1H-indol-2-yl)-1-butyl-N,N-diméthylcyclohezanamine, citrate
(146) 4-(3-benzyl-1H-indol-2-yl)-1-butyl-N,N-diméthylcyclohexanamine, citrate
(147) 4-(3-(cyclohexylméthyl)-1H-indol-2-yl)-N,N-diméthyl-1-phénylcyclobexanamine, citrate
(148) 4-(3-(cyclohexylméthyl)-1H-indol-2-yl)-N,N-diméthyl-1-phénylcyclohexanamine, citrate
(149) 4-(3-benzyl-1H-indol-2-yl)-N,N-diméthyl-1-phénylcyclohexanamine, citrate
(150) 4-(3-benzyl-1H-indol-2-yl)-N,N-diméthyl-1-phénylcyclohexanamine, citrate
(151) N,N-diméthyl-1-phényl-4-(3-(pyridin-2-ylméthyl)-1H-indol-2-yl)cyclohezanamine
(152) N,N-diméthyl-1-phényl-4-(3-(pyridin-2-ylméthyl)-1H-indol-2-yl)cyclohexanamine
(153) acide 3-(2-(4-(diméthylamino)-4-phénylcyclohexyl)-1H-indol-3-yl)propanoïque, chlorhydrate
(156) 1-benzyl-N,N-diméthyl-4-(3-propyl-1H-indol-2-yl)cyclohexanamine, citrate
(157) 1-butyl-N,N-diméthyl-4-(3-propyl-1H-indol-2-yl)cyclohexanamine, chlorhydrate
(158) 1-butyl-N,N-diméthyl-4-(3-propyl-1H-indol-2-yl)cyclohexanamine, citrate
(159) N,N-diméthyl-1-phényl-4-(3-propyl-1H-indol-2-yl)cyclohexanamine, citrate
(160) N,N-diméthyl-1-phényl-4-(3-propyl-1H-indol-2-yl)cyclohexanamine, citrate
(161) 1-benzyl-N,N-diméthyl-4-(3-(2-(pyridin-4-yl)éthyl)-1H-indol-2-yl)cyclohexanamine, citrate
(162) 1-benzyl-N,N-diméthyl-4-(3-(2-(pyridin-4-yl)éthyl)-1H-indol-2-yl)cyclohexanamine, citrate
(163) 1-butyl-N,N-diméthyl-4-(3-(2-(pyridin-4-yl)éthyl)-1H-indol-2-yl)cyclohexanamine, citrate
(164) 1-butyl-N,N-diméthyl-4-(3-(2-(pyridin-4-yl)éthyl)-1H-indol-2-yl)cyclohexanamine, citrate
(165) N,N-diméthyl-1-phényl-4-(3-(2-(pyridin-4-yl)éthyl)-1H-indol-2-yl)cyclohexanamine, citrate (1:4)
(166) N,N-diméthyl-1-phérlyl-4-(3-(2-(pyridin-4-yl)éthyl)-1H-indol-2-yl)cyclohexanamine, citrate
(167) N,N-diméthyl-4-(3-(2-(1-méthyl-1H-benzo[d]imidazol-2-yl)éthyl)-1H-indol-2-yl)-1-phénylcyclohexanamine, citrate
(168) N,N-diméthyl-4-(3-(2-(1-méthyl-1H-benzo[d]imidazol-2-yl)éthyl)-1H-indol-2-yl)-1-phénylcyclohexanamine, citrate
(169) N,N-diméthyl-1-phényl-4-(3-(2-(pyridin-2-yl)éthyl)-1H-indol-2-yl)cyclohexanamine, citrate
(170) N,N-diméthyl-1-phényl-4-(3-(2-(pyridin-2-yl)éthyl)-1H-indol-2-yl)cyclohexanamine, citrate (2:3)
(171) acide 4-(2-(4-benzyl-4-(diméthylamino)cyclohexyl)-1H-indol-3-yl)butanoïque, chlorhydrate
(172) acide 4-(2-(4-(diméthylamino)-4-phénylcyclohexyl)-1H-indol-3-yl)butanoïque, chlorhydrate
(173) 4-(2-(4-(diméthylamino)-4-phénylcyclohexyl)-1H-indol-3-yl)butan-1-ol, chlorhydrate
(174) acétate de 4-(2-(4-benzyl-4-(diméthylamino)cyclohexyl)-1H-indol-3-yl)butyle, chlorhydrate
(175) acétate de 4-(2-(4-benzyl-4-(diméthylamino)cyclohexyl)-1H-indol-3-yl)butyle, chlorhydrate
(176) acétate de 4-(2-(4-butyl-4-(diméthylamino)cyclohexyl)-1H-indol-3-yl)butyle, chlorhydrate
(177) acétate de 4-(2-(4-butyl-4-(diméthylamino)cyclohexyl)-1H-indol-3-yl)butyle, chlorhydrate
(178) acétate de 4-(2-(4-(diméthylamino)-4-phénylcyclohexyl)-1H-indol-3-yl)butyle, chlorhydrate
(179) acétate de 4-(2-(4-(diméthylamino)-4-phénylcyclohexyl)-1H-indol-3-yl)butyle, chlorhydrate
(180) 3-(2-(4-benzyl-4-(diméthylamino)cyclohexyl)-1H-indol-3-yl)propan-1-ol, citrate
(181) 3-(2-(4-butyl-4-(diméthylamino)cyclohexyl)-1H-indol-3-yl)propan-1-ol, chlorhydrate
(182) 3-(2-(4-butyl-4-(diméthylamino)cyclohexyl)-1H-indol-3-yl)propan-1-ol, chlorhydrate
(183) 3-(2-(4-(diméthylamino)-4-phénylcyclobexyl)-1H-indol-3-yl)propan-1-ol, chlorhydrate
(184) 3-(2-(4-(diméthylamino)-4-phénylcyclohexyl)-1H-indol-3-yl)propan-1-ol, chlorhydrate
(185) acétate de 3-(2-(4-(diméthylamino)-4-phénylcyclohexyl)-1H-indol-3-yl)propyle, chlorhydrate
(186) acétate de 3-(2-(4-(diméthylamino)-4-phénylcyclohexyl)-1H-indol-3-yl)propyle, chlorhydrate

(187) 1-(2-(2-(4-(diméthylamino)-4-phénylcyclohexyl)-1H-indol-3-yl)éthyl)pyrrolidine-2,5-dione
(188) 1-(2-(2-(4-(diméthylamino)-4-phénylcyclohexyl)-1H-indol-3-yl)éthyl)pyrrolidine-2,5-dione
(189) 4-(3-(2-(3,4-dihydroquinolin-1(2H)-yl)éthyl)-1H-indol-2-yl)-N,N-diméthyl-1-phénylcyclohexanamine, citrate (4:3)
(190) 4-(3-(2-(3,4-dihydroquinolin-1(2H)-y1)éthyl)-1H-indol-2-yl)-N,N-diméthyl-1-phénylcyclohexanamine, citrate
(191) 1-(2-(2-(4-(diméthylamino)-4-phénylcyclohexyl)-1H-indol-3-yl)éthyl)-1H-1,2,3-triazole-4-carboxylate de méthyle, citrate
(192) 1-(2-(2-(4-(diméthylamino)-4-phénylcyclohexyl)-1H-indol-3-yl)éthyl)-1H-1,2,3-triazole-4-carboxylate de méthyle, citrate
(193) 4-(3-(2-(isoindoléin-2-yl)éthyl)-1H-indol-2-yl)-N,N-diméthyl-1-phénylcyclohexanamine, citrate
(194) 4-(3-(2-(isoindoléin-2-yl)éthyl)-1H-indol-2-yl)-N,N-diméthyl-1-phénylcyclohexanamine, citrate
(195) 9-(3-(2-(3,4-dihydroisoquinolin-2(1H)-yl)éthyl)-1H-indol-2-yl)-N,N-diméthyl-l-phénylcyclohexanamine, citrate
(196) 4-(3-(2-(3,4-dihydroisoquinolin-2(1H)-yl)éthyl)-1H-indol-2-yl)-N,N-diméthyl-1-phénylcyclohexanamine, citrate (2:3)
(197) N,N-diméthyl-1-phényl-4-(3-(2-(pyrrolidin-1-yl)éthyl)-1H-indol-2-yl)cyclohexanamine, citrate
(198) N,N-diméthyl-1-phényl-4-(3-(2-(pyrrolidin-1-yl)éthyl)-1H-indol-2-yl)cyclohexanamine, citrate
(199) N,N-diméthyl-1-phényl-4-(3-(2-(pipéridin-1-yl)éthyl)-1H-indol-2-yl)cyclohexanamine, citrate
(200) N,N-diméthyl-4-(3-(2-(4-méthylpipérazin-1-yl)éthyl)-1H-indol-2-yl)-1-phénylcyclohexanamine, citrate
(201) N,N-diméthyl-4-(3-(2-morpholinoéthyl)-1H-indol-2-yl)-1-phénylcyclohexanamine, citrate
(202) N,N-diméthyl-4-(3-(2-morpholinoéthyl)-1H-indol-2-yl)-1-phénylayclohexanamine, citrate
(203) 4-(3-(2-(1H-benzo[d]imidazol-1-yl)éthyl)-1H-indol-2-yl)-N,N-diméthyl-1-phénylcyclohexanamine, citrate
(204) 1-(3-(2-(1H-benzo[d]imidazol-1-yl)éthyl)-1H-indol-2-yl)-N,N-diméthyl-1-phénylcyclohexanamine, citrate
(205) 4-(3-(2-(1H-imidazol-1-yl)éthyl)-1H-indol-2-yl)-N,N-diméthyl-1-phénylcyclohexanamine, citrate
(206) 4-(3-(2-(1H-imidazol-1-yl)éthyl)-1H-indol-2-yl)-N,N-diméthyl-1-phénylcyclohexanamine, citrate
(207) 4-(3-(2-(1H-1,2,4-triazol-1-yl)éthyl)-1H-indol-2-yl)-N,N-diméthyl-1-phénylcyclohexanamine, citrate
(208) 4-(3-(2-(1H-1,2,4-triazol-1-yl)éthyl)-1H-indol-2-yl)-N,N-diméthyl-1-phénylcyclohexanamine, citrate
(209) N,N-diméthyl-1-phényl-4-(3-(2-(thiazolidin-3-yl)éthyl)-1H-indol-2-yl)cyclohexanamine, citrate
(210) N,N-diméthyl-1-phényl-4-(3-(2-(thiazolidin-3-yl)éthyl)-1H-indol-2-yl)cyclohexanamine, citrate
(211) N,N-diméthyl-4-(3-(2-(5-méthyl-2H-tétrazol-2-yl)éthyl)-1H-indol-2-yl)-1-phénylcyclohexanamine, citrate
(212) N,N-diméthyl-4-(3-(2-(5-méthyl-2H-tétrizol-2-yl)éthyl)-1H-indol-2-yl)-1-phénylcyclohexanamine, citrate
(213) 4-(3-(2-(1H-pyrazol-1-yl)éthyl)-1H-indol-2-yl)-N,N-diméthyl-1-phénylcyclohexanamine, citrate
(214) 4-(3-(2-(1H-pyrazol-1-yl)éthyl)-1H-indol-2-yl)-N,N-diméthyl-1-phénylcyclohexanamine, citrate
(215) 4-(3-(2-(1H-1,2,3-triazol-1-yl)éthyl)-1H-indol-2-yl)-N,N-diméthyl-1-phénylcyclohexanamine, citrate
(216) 4-(3-(2-(1H-1,2,3-triazol-1-yl)éthyl)-1H-indol-2-yl)-N,N-diméthyl-1-phénylcyclohexanamine, citrate
(217) N,N-diméthyl-4-(3-(2-(5-méthyl-1H-tétrazol-1-yl)éthyl)-1H-indol-2-yl)-1-phénylcyclohexanamine, citrate
(218) N,N-diméthyl-4-(3-(2-(5-méthyl-1H-tétrazol-1-yl)éthyl)-1H-indol-2-yl)-1-phénylcyclohoxanamine, citrate
(219) 2-(2-(4-(diméthylamino)-4-phénylcyclohexyl)-1H-indol-3-yl)éthanol
(220) acétate de 2-(2-(4-(diméthylamino)-4-phénylcyclohexyl)-1H-indol-3-yl)éthyle
(221) N,N-diméthyl-4-(3-méthyl-1H-pyrrolo[3,2-c]pyridin-2-yl)-1-phénylcyclohexanamine, citrate
(222) N,N-diméthyl-4-(3-méthyl-1H-pyrrolo[3,2-c]pyridin-2-yl)-1-phénylcyclohexanamine, citrate
(223) N,N-diméthyl-4-(3-méthyl-1H-pyrrolo[2,3-b]pyridin-2-yl)-1-phénylcyclohexanamine, citrate
(224) N,N-diméthyl-4-(3-méthyl-1H-pyrrolo[2,3-b]pyridin-2-yl)-1-phénylcyclohexanamine, citrate
(225) N,N-diméthyl-4-(3-méthylbenzofuran-2-yl)-1-phénylcyclohexanamine, citrate
(226) N,N-diméthyl-4-(3-méthylbenzofuran-2-yl)-1-phénylcyclohexanamine, citrate
(227) 4-(1H-indol-3-yl)-N,N-diméthyl-1-phénylcyclohexanamine, citrate
(228) (±)-3-(4-(diméthylamino)-4-benzylcyclohex-1-ényl)-1H-pyrrolo[2,3-b]pyridine
(229) (±)-3-(4-(diméthylamino)-4-butylcyclohex-1-ényl)-1H-pyrrolo[2,3-b]pyridine
(230) (±)-3-(4-(diméthylamino)-4-phénylcyclohex-1-ényl)-1H-pyrrolo[2,3-b]pyridine, citrate
(231) (±)-4-(1H-indol-3-yl)-N,N-diméthyl-1-phénylcyclohex-3-énamine, citrate
(232) (±)-4-(1H-indol-3-yl)-N,N-diméthyl-1-phénylcyclohex-3-énamine
(233) (±)-2-(3-(4-(diméthylamino)-4-phénylcyclohex-1-ényl)benzo[b]thiophén-2-yl)éthanol, chlorhydrate
(234) (±)-2-(3-(4-(diméthylamino)-4-(pyridin-2-yl)cyclohex-1-ényl)benzo[b]thiophén-2-yl)éthanol, chlorhydrate
(235) 4-(1H-indol-2-yl)-N,N-diméthyl-1-phénylcyclohexanamine, citrate
(236) 4-(1H-indol-2-yl)-N,N-diméthyl-1-phénylcyclohexanamine, citrate
(237) 1-benzyl-N,N-diméthyl-4-(1H-pyrrolo[2,3-b]pyridin-3-yl)cyclohexanamine, chlorhydrate;
(238) 1-butyl-N,N-àiméthyl-4-(1H-pyrrolo[2,3-b]pyridin-3-yl)cyclohexanamine, chlorhydrate

(239) (±)-4-(benzofuran-2-yl)-1-benzyl-N,N-diméthylcyclohex-3-énamin, chlorhydrate
(240) (±)-N,N-diméthyl-4-(3-méthylbenzofuran-2-yl)-1-phénylcyclohex-3-énamin, citrate
(241) (±)-2-(2-(4-(diméthylamino)-4-phénylcyclohex-1-ényl)benzofuran-3-yl)éthanethiol, citrate
(242) (±)-N,N-diméthyl-4-(3-méthylbenzo[b]thiophén-2-yl)-1-phénylcyclohex-3-énamine, citrate
(243) N,N-diméthyl-4-(3-méthylbenzo[b]thiophén-2-yl)-1-phénylcyclohexanamine, citrate
(244) N,N-diméthyl-1-phényl-4-(1H-pyrrolo[2,3-b]pyridin-3-yl)cyclohexanamine, citrate
(246) 1-(diméthylamino)-3-(2-(4-(diméthylamino)-4-phénylcyclohexyl)-3-méthyl-1H-indol-1-yl)propan-2-ol
(247) N,N-diméthyl-4-(3-méthyl-1-(oxiran-2-ylméthyl)-1H-indol-2-yl)-1-phénylcyclohexanamine
(248) 4-(1,3-diméthyl-1H-indol-2-yl)-N,N-diméthyl-1-phénylcyclohexanamine
(249) 2-(9-butyl-4-(diméthylamino)cyclohexyl)-3-méthyl-1H-indol-5-ol, citrate
(250) 2-(4-butyl-4-(diméthylamino)cyclohexyl)-3-méthyl-1H-indol-5-ol, citrate
(251) (±)-2-(4-(diméthylamino)-4-(thiophén-2-yl)-cyclohex-1-ényl)-3-(2-(pyridin-4-yl)éthyl)-1H-indole
(252) (±)-2-(4-(diméthylamino)-4-(3-fluorophényl)-cyclohex-1-ényl)-3-(2-(pyridin-4-yl)éthyl)-1H-indole
(253) (±)-2-(4-butyl-4-(pyrrolidin-1-yl)cyclohex-1-ényl)-3-(2-(pyridin-4-yl)éthyl)-1H-indole
(254) (±)-2-(4-(méthylamino)-4-phénylcyclohex-1-ényl)-3-(2-(pyridin-4-yl)éthyl)-1H-indole
(255) (±)-2-(4-(diméthylamino)-4-(thiophén-2-yl)-cyclohex-1-ényl)-3-méthyl-1H-indole, citrate
(256) N,N-diméthyl-4-(3-méthyl-1H-inàdol-2-yl)-1-(thiophén-2-yl)cyclohexanamine, citrate
(257) N,N-diméthyl-4-(3-méthyl-1H-indol-2-yl)-1-(thiophén-2-yl)cyclohemanamine, citrate
(258) 2-(4-butyl-4-(pyrrolidin-1-yl)cyclohex-1-ényl)-3-méthyl-1H-indole
(259) N-méthyl-4-(3-méthyl-1H-indol-2-yl)-1-phénylcyclohexanamine, bromhydrate
(260) N-méthyl-4-(3-méthyl-1H-indol-2-yl)-1-phénylcyclohexanamine, citrate
(261) 1-(3-fluorophényl)-N,N-diméthyl-4-(3-méthyl-1H-indol-2-yl)cyclohexanamine, citrate
(262) 1-(3-fluorophényl)-N,N-diméthyl-4-(3-méthyl-1H-indol-2-yl)cyclohexanamine, citrate
(263) (±)-2-(4-(dimethylamino)-4-(3-fluorophényl) - cyclohex-1-ényl)-3-méthyl-1H-indole
(264) 2-(4-benzyl-4-(diméthylamino)cyclohexyl)-3-méthyl-1H-indol-5-ol, citrate
(265) 2-(4-benzyl-4-(diméthylamino)cyclohexyl)-3-méthyl-1H-indol-5-ol, citrate
(266) 2-(4-butyl-4-(pyrrolidin-1-yl)cyclohexyl)-3-(2-(pyridin-4-yl)éthyl)-1H-indole, citrate
(267) 2-(4-butyl-4-(pyrrolidin-1-yl)cyclohexyl)-3-(2-(pyridin-4-yl)éthyl)-1H-indole, citrate
(268) 2-(4-(azétidin-1-yl)-4-phénylcyclohexyl)-3-méthyl-1H-indole, citrate
(269) 2-(4-(azétidin-1-yl)-4-phénylcyclohexyl)-3-méthyl-1H-indole, citrate
(270) 2-(4-(azétidin-1-yl)-4-phénylcyclohexyl)-3-(2-(pyridin-4-yl)éthyl)-1H-indole, chlorhydrate
(271) 2-(4-(azétidin-1-yl)-4-phénylcyclohexyl)-3-(2-(pyridin-4-yl)éthyl)-1H-indole, chlorhydrate
(272) 2-(4-butyl-4-(pyrrolidin-1-yl)cyclohexyl)-3-méthyl-1H-indole, citrate
(273) 3-(2-(4-(diméthylamino)-4-phénylcyclohexyl)-1H-pyrrolo[2,3-b]pyridin-3-yl)propan-1-ol, citrate
(274) 1-(2-(4-(diméthylamino)-4-phénylcyclohexyl)-3-méthyl-1H-indol-1-yl)-3-(méthylamino)propan-2-ol, citrate
(275) 1-benzyl-3-(2-(2-(4-(diméthylamino)-4-phénylcyclohexyl)-1H-indol-3-yl)éthyl)urée, citrate
(276) 1-benzyl-3-(2-(2-(4-(diméthylamino)-4-phénylcyclohexyl)-1H-indol-3-yl)éthyl)urée, citrate
(277) 1-(2-(2-(4-(diméthylamino)-4-phénylcyclohexyl)-1H-indol-3-yl)éthyl)-3-phénylurée, citrate
(278) 1-(2-(2-(4-(diméthylamino)-4-phénylcyclohexyl)-1H-indol-3-yl)éthyl)-3-phénylurée, citrate
(279) 1-cyclopentyl-3-(2-(2-(4-(diméthylamino)-4-phénylcyclohexyl)-1H-indol-3-yl)éthyl)urée, citrate
(280) 1-cyclopentyl-3-(2-(2-(4-(diméthylamino)-4-phénylcyclohexyl)-1H-indol-3-yl)éthyl)urée, citrate
(281) N-(2-(2-(4-(diméthylamino)-4-phénylcyclohexyl)-1H-indol-3-yl)éthyl)cyclopentanesulfonamide, citrate
(282) N-(2-(2-(4-(diméthylamino)-4-phénylcyclohexyl)-1H-indol-3-yl)éthyl)cyclopentanesulfonamide, citrate
(283) N-(2-(2-(4-(diméthylamino)-4-phénylcyclohexyl)-1H-indol-3-yl)éthyl)benzènesulfonamide, citrate
(284) N-(2-(2-(4-(diméthylamino)-4-phénylcyclohexyl)-1H-indol-3-yl)éthyl)thiophéne-2-sulfonamide, citrate
(285) N-(2-(2-(4-(diméthylamino)-4-phénylcyclohexyl)-1H-indol-3-yl)éthyl)thiophène-2-sulfonamid, citrate
(286) N-(2-(2-(9-(diméthylamino)-4-phénylcyclohexyl)-1H-indol-3-yl)éthyl)nicotinamide, citrate
(287) N-(2-(2-(4-(diméthylamino)-4-phénylcyclohexyl)-1H-indol-3-yl)éthyl)nicotinamide, citrate
(288) N-(2-(2-(4-(diméthylamino)-4-phénylcyclohexyl)-1H-indol-3-yl)éthyl)benzamide, citrate
(289) 1-(3-fluorophényl)-N,N-diméthyl-4-(3-(2-(pyridin-4-yl)éthyl)-1H-indol-2-yl)cyclohexanamine
(290) 1-(3-fluorophényl)-N,N-diméthyl-4-(3-(2-(pyridin-4-yl)éthyl)-1H-indol-2-yl)cyclohexanamine, citrate
(291) N-méthyl-1-phényl-4-(3-(2-(pyridin-4-yl)éthyl)-1H-indol-2-yl)cyclohexanamine, citrate
(292) N-méthyl-1-phényl-4-(3-(2-(pyridin-4-yl)éthyl)-1H-indol-2-yl)cyclohexanamine, citrate
(293) 2-(4-butyl-4-(pyrrolidin-1-yl)cyclohexyl)-3-méthyl-1H-indole, citrate
(294) N,N-diméthyl-1-phényl-4-(1-(phénylsulfonyl)-1H-indol-2-yl)cyclohexanamine
(295) N,N-diméthyl-1-phényl-4-(1-(phénylsulfonyl)-1H-indol-2-yl)cyclohexanamine
(296) N,N-diméthyl-4-(3-méthyl-1-(oxiran-2-ylméthyl)-1H-indol-2-yl)-1-phénylcyclohex-3-énamine

(297) N,N-diméthyl-4-(3-méthyl-1-(oxiran-2-ylméthyl)-1H-indol-2-yl)-1-phénylcyclohexanamine, citrate
(298) 1-(diméthylamino)-3-(2-(4-(diméthylamino)-4-phénylcyclohex-1-ényl)-3-méthyl-1H-indol-1-yl)propan-2-ol, citrate
(299) 1-(diméthylamino)-3-(2-(4-(diméthylamino)-4-phénylcyclohexyl)-3-méthyl-1H-indol-1-yl)propan-2-ol, citrate
(300) 2-(3-(4-(diméthylamino)-4-phénylcyclohex-1-ényl)-1H-indol-1-yl)éthanol, chlorhydrate
(301) (±)-3-(4-(diméthylamino)-4-phénylcyclohex-1-ényl)-(1-(phénylsulfonyl)-1H-indole), chlorhydrate
(302) 1-benzyl-N,N-diméthyl-9-(1-méthyl-1H-indol-2-yl)cyclohex-3-énamine, chlorhydrate
(303) N,N-diméthyl-4-(3-(2-(pyridin-4-yl)éthyl)-1H-indol-2-yl)-1-(thiophén-2-yl)cyclohexanamine, chlorhydrate
(304) N,N-diméthyl-4-(3-(2-(pyridin-4-yl)éthyl)-1H-indol-2-yl)-1-(thiophén-2-yl)cyclohexanamine, chlorhydrate
(305) N-méthyl-4-(3-méthyl-1H-indol-2-yl)-1-(4-méthylthiazol-2-yl)cyclohexanamine
(306) N-méthyl-4-(3-méthyl-1H-indol-2-yl)-1-(4-méthylthiazol-2-yl)cyclohexanamine
(307) 2-(2-(4-(diméthylamino)-4-phénylcyclohexyl)-1H-indol-3-yl)éthanol,

sous la forme du racémat ; des énantiomères, des diastéréomères, de mélanges des énantiomères ou des diastéréomères, ou d'un énantiomère ou diastéréomère individuel ; des bases et/ou des sels d'acides physiologiquement compatibles.

**11.** Procédé de fabrication de dérivés hétéroaryliques substitués de formule Ic

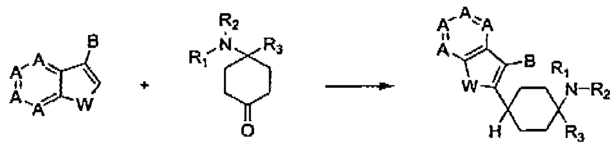

A B Ic

dans lequel des cétones de formule générale B sont mises en réaction avec des composés hétéroaromatiques de formule générale A dans des solvants ou des mélanges de solvants organiques, par exemple de l'acétate d'éthyle, du chloroforme, du dichlorométhane (DCM), du dichloroéthane (DCE), de l'éther diéthylique ($Et_2O$), de l'acétonitrile (MeCN) ou du nitrométhane, avec ajout d'un acide organique ou inorganique, par exemple HCl, HBr, l'acide trifluorométhanesulfonique, l'acide méthanesulfonique, l'acide acétique, l'acide trifluoroacétique, ou sans solvant dans un acide organique ou inorganique ou des mélanges d'acides, à des températures comprises entre 0 °C et 150 °C, éventuellement en utilisant un rayonnement micro-onde, puis mises en réaction avec ajout d'un réducteur organique ou inorganique, p. ex. du triéthylsilane ou de la poudre d'étain, à des températures comprises entre 0 °C et 150 °C, en utilisant éventuellement un rayonnement micro-onde.

**12.** Procédé de fabrication de dérivés hétéroaryliques substitués de formule générale Id ou Ie

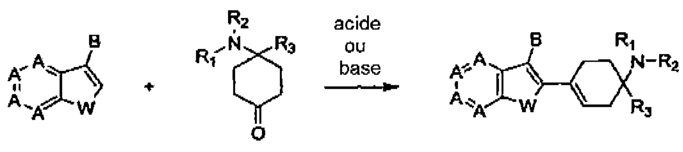


A B Id

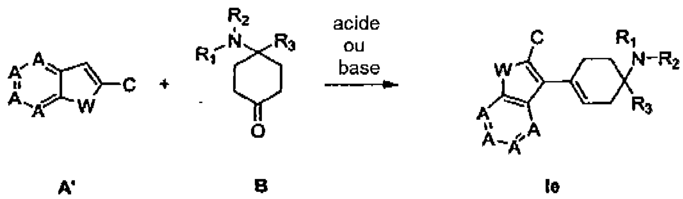


A' B Ie

dans lequel des composés hétéroaromatiques de formule générale A ou A' sont mis en réaction avec des cyclohexanones de formule générale B dans des solvants ou des mélanges de solvants organiques, par exemple du

chloroforme, du dichlorométhane (DCM), du dichloroéthane (DCE), de l'éther diéthylique ($Et_2O$), de l'acétonitrile (MeCN) ou du nitrométhane, avec ajout d'un acide organique ou inorganique, par exemple HCl, HBr, l'acide trifluorométhanesulfonique, l'acide méthanesulfonique, l'acide acétique, l'acide trifluoroacétique, à des températures comprises entre 0 °C et 150 °C, en utilisant éventuellement un rayonnement micro-onde,
ou des composés hétéroaromatiques de formule générale A ou A' sont mis en réaction avec des cyclohexanones de formule générale B en utilisant une base, par exemple KOH ou NaOH, dans un solvant organique, par exemple le méthanol, à des températures comprises entre 20 et 100 °C.

**13.** Procédé de fabrication de dérivés hétéroaryliques substitués de formule générale Ic ou If

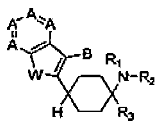

Ic

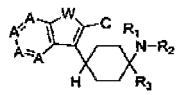

If

par réduction des composés Id ou Ie par de l'hydrogène sous la forme HBr/acide acétique glacial//Sn ou HCl/Sn (hydrogène naissant) ou $H_2$ en présence d'un catalyseur métallique tel que p. ex. du palladium sur du charbon, du platine sur du charbon, de l'oxyde de platine, du nickel, de Raney, du rhodium ou des complexes de ruthénium dans un solvant ou un mélange de solvants approprié, par, exemple le méthanol, l'éthanol, l'acétone, l'ester éthylique de l'acide acétique, HBr ou l'acide acétique, à des températures comprises entre 0 °C et 150 °C.

**14.** Médicament contenant au moins un dérivé hétéroarylique substitué selon l'une quelconque des revendications 1 à 10, éventuellement sous la forme de son racémat, des stéréoisomères purs, notamment les énantiomères et les diastéréomères, en un rapport de mélange quelconque ; sous la forme de ses acides ou de ses bases ou sous la forme de ses sels, notamment des sels physiologiquement compatibles ou des sels d'acides ou de cations physiologiquement compatibles ; ou sous la forme de ses solvates, notamment des hydrates, et contenant éventuellement des additifs et/ou adjuvants appropriés et/ou éventuellement d'autres substances actives.

**15.** Dérivé hétéroarylique substitué selon l'une quelconque des revendications 1 à 10, éventuellement sous la forme de son racémat, des stéréoisomères purs, notamment les énantiomères et les diastéréomères, en un rapport de mélange quelconque ; sous la forme de ses acides ou de ses bases ou sous la forme de ses sels, notamment des sels physiologiquement compatibles ou des sels d'acides ou de cations physiologiquement compatibles ; ou sous la forme de ses solvates, notamment des hydrates ; destiné à être utilisé pour le traitement de la douleur, notamment la douleur aiguë, neuropatique, chronique ou la douleur inflammatoire.

**16.** Dérivé hétéroarylique substitué selon l'une quelconque des revendications 1 à 10, destiné à être utilisé pour le traitement des états d'anxiété, du stress et des syndromes associés avec le stress, des dépressions, de l'épilepsie, de la maladie d'Alzheimer, de la démence sénile, de la catalepsie, des dysfonctionnements cognitifs généraux, des difficultés d'apprentissage et de mémorisation (en tant que nootropique), des phénomènes de sevrage, de l'abus de et/ou de la dépendance à l'alcool et/ou aux drogues et/ou aux médicaments, des dysfonctionnements sexuels, des maladies cardiovasculaires, de l'hypotension, de l'hypertension, des acouphènes, du prurit, de la migraine, de la surdité, du manque de motilité intestinale, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthésique ou pour la co-administration lors du traitement avec un analgésique opioïde ou avec un anesthésique, pour la diurèse ou l'antinatriurèse, l'anxiolyse, pour la modulation de l'activité motrice, pour la modulation de la distribution des neurotransmetteurs et le traitement des maladies neurodégénératives associées, pour le traitement des phénomènes de sevrage et/ou pour la réduction du potentiel d'accoutumance des opioides.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 02090317 A
- WO 9951576 A
- WO 2004043967 A
- WO 04043967 A
- WO 0290317 A
- US 4065573 A
- US 4065573 A1, Upjohn_Lednicer


### In der Beschreibung aufgeführte Nicht-Patentliteratur


- *Opioids: Introduction,* 127-150
- Further Opioid Receptors. Analgesics - From Chemistry and Pharmacology to Clinical Application. Wiley VCH, 2002, 455-476
- **MANABE et al.** *Nature,* 1997, vol. 394, 577-581
- **NISHI et al.** *EMBO J.,* 1997, vol. 16, 1858-1864
- **CALO et al.** *Br.J. Pharmacol.,* 2000, vol. 129, 1261-1283
- **LEDNICER et al.** *J.Med.Chem.,* 1980, vol. 23, 424-430
- **S. J. GARDEN ; R. B. DA SILVA ; A. C. PINTO.** *Tetrahedron,* 2002, vol. 58, 8399-8412
- **ARDATI et al.** *Mol. Pharmacol.,* 1997, vol. 51, 816-824
- **D'AMOUR ; SMITH.** *J. Pharm. Exp. Ther.,* 1941, vol. 72, 74-79